(19) Europäisches Patentamt
European Patent Office
Office européen des brevets

(11) EP 3 485 013 B1

(12) **EUROPEAN PATENT SPECIFICATION**

(45) Date of publication and mention of the grant of the patent:
**24.03.2021 Bulletin 2021/12**

(21) Application number: **18734715.8**

(22) Date of filing: **06.06.2018**

(51) Int Cl.:
***C12N 15/10** (2006.01)* ***C12N 15/73** (2006.01)*

(86) International application number:
**PCT/US2018/036333**

(87) International publication number:
**WO 2018/226880 (13.12.2018 Gazette 2018/50)**

(54) **A HTP GENOMIC ENGINEERING PLATFORM FOR IMPROVING ESCHERICHIA COLI**

GENOMISCHE HTP-ENGINEERING-PLATTFORM ZUR VERBESSERUNG VON ESCHERICHIA COLI

PLATE-FORME D'INGÉNIERIE GÉNOMIQUE HTP PERMETTANT D'AMÉLIORER ESCHERICHIA COLI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.06.2017 US 201762515870 P**

(43) Date of publication of application:
**22.05.2019 Bulletin 2019/21**

(73) Proprietor: **Zymergen, Inc.**
**Emeryville, California 94608 (US)**

(72) Inventors:
- **DAVIS, Matthew**
  **Emeryville, California 94608 (US)**
- **WISNEWSKI, Christy**
  **Emeryville, California 94608 (US)**
- **WESTFALL, Patrick**
  **Emeryville, California 94608 (US)**
- **SERBER, Zach**
  **Emeryville, California 94608 (US)**
- **DEAN, Erik Jedediah**
  **Emeryville, California 94608 (US)**
- **MANCHESTER, Shawn**
  **Emeryville, California 94608 (US)**
- **GORA, Katherine**
  **Emeryville, California 94608 (US)**
- **SHELLMAN, Erin**
  **Emeryville, California 94608 (US)**
- **KIMBALL, Aaron**
  **Emeryville, California 94608 (US)**
- **SZYJKA, Shawn**
  **Emeryville, California 94608 (US)**
- **FREWEN, Barbara**
  **Emeryville, California 94608 (US)**
- **TREYNOR, Thomas**
  **Emeryville, California 94608 (US)**
- **FLASHMAN, Michael**
  **Emeryville, California 94608 (US)**
- **HAUSHALTER, Robert**
  **Emeryville, California 94608 (US)**
- **MORGAN, Stacy-Anne**
  **Emeryville, California 94608 (US)**
- **BLAISSE, Michael**
  **Emeryville, California 94608 (US)**
- **RAMAKRISHNAN, Prabha**
  **Emeryville, California 94608 (US)**
- **ROTHSCHILD-MANCINELLI, Kyle**
  **Emeryville, California 94608 (US)**
- **KIM, Youngnyun**
  **Emeryville, California 94608 (US)**

(74) Representative: **Cooley (UK) LLP**
**Dashwood**
**69 Old Broad Street**
**London EC2M 1QS (GB)**

(56) References cited:

| | |
|---|---|
| **EP-A2- 2 657 250** | **WO-A1-00/04190** |
| **WO-A1-97/20078** | **WO-A1-97/35966** |
| **WO-A1-98/31837** | **WO-A1-2012/149470** |
| **WO-A1-2017/100377** | **WO-A1-2017/189784** |
| **WO-A2-02/29032** | **WO-A2-2010/002966** |
| **WO-A2-2012/142591** | **WO-A2-2017/100376** |
| **US-A1- 2006 141 578** | **US-A1- 2017 159 045** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ZHANG Y-X ET AL: "Genome shuffling leads to rapid phenotypic improvement in bacteria", NATURE, MACMILLAN JOURNALS LTD, LONDON, GB, vol. 415, no. 6872, 1 January 2002 (2002-01-01), pages 644-646, XP002228258, ISSN: 0028-0836, DOI: 10.1038/415644A
- WANG HARRIS H ET AL: "Programming cells by multiplex genome engineering and accelerated evolution.", NATURE 13 AUG 2009, vol. 460, no. 7257, 13 August 2009 (2009-08-13), pages 894-898, XP55111854, ISSN: 1476-4687
- LEE S Y ET AL: "Systems biotechnology for strain improvement", TRENDS IN BIOTECHNOLOGY, ELSEVIER PUBLICATIONS, CAMBRIDGE, GB, vol. 23, no. 7, 1 July 2005 (2005-07-01), pages 349-358, XP027778087, ISSN: 0167-7799 [retrieved on 2005-07-01]
- ALPER HAL ET AL: "Characterization of lycopene-overproducing E. coli strains by combining systematic and combinatorial gene knockout targets", NATURE BIOTECHNOLOGY MAY 2005, vol. 72, no. 5, October 2006 (2006-10), pages 612-616, XP2783819, ISSN: 0175-7598
- MAMIE Z LI ET AL: "MAGIC, an in vivo genetic method for the rapid construction of recombinant DNA molecules", NATURE GENETICS., vol. 37, no. 3, 30 January 2005 (2005-01-30), pages 311-319, XP55410043, NEW YORK, US ISSN: 1061-4036, DOI: 10.1038/ng1505
- ALPER HAL ET AL: "Tuning genetic control through promoter engineering", PROCEEDINGS OF THE NATIONAL ACADEMY OF SCIENCES, NATIONAL ACADEMY OF SCIENCES, US, vol. 102, no. 36, 6 September 2005 (2005-09-06), pages 12678-12683, XP002428722, ISSN: 0027-8424, DOI: 10.1073/PNAS.0504604102
- MASATO IKEDA ET AL: "A genome-based approach to create a minimally mutated Corynebacterium glutamicum strain for efficient l-lysine production", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 33, no. 7, 28 February 2006 (2006-02-28), pages 610-615, XP019388133, ISSN: 1476-5535, DOI: 10.1007/S10295-006-0104-5
- FOTINI A TRIKKA ET AL: "Iterative carotenogenic screens identify combinations of yeast gene deletions that enhance sclareol production", MICROBIAL CELL FACTORIES,, vol. 14, no. 1, 24 April 2015 (2015-04-24) , page 60, XP021214826, ISSN: 1475-2859, DOI: 10.1186/S12934-015-0246-0
- OHNISHI J ET AL: "A novel methodology employing Corynebacterium glutamicum genome information to generate a new L-lysine-producing mutant", APPLIED MICROBIOLOGY AND BIOTECHNOLOGY, SPRINGER, DE, vol. 58, no. 2, 1 February 2002 (2002-02-01), pages 217-223, XP002289984, ISSN: 0175-7598, DOI: 10.1007/S00253-001-0883-6
- LEE MICHAEL E ET AL: "A Highly Characterized Yeast Toolkit for Modular, Multipart Assembly.", ACS SYNTHETIC BIOLOGY 18 SEP 2015, vol. 4, no. 9, 18 September 2015 (2015-09-18), pages 975-986, XP002786200, ISSN: 2161-5063
- SNITKIN EVAN S ET AL: "Epistatic interaction maps relative to multiple metabolic phenotypes.", PLOS GENETICS 10 FEB 2011, vol. 7, no. 2, E1001294, 10 February 2011 (2011-02-10), pages 1-15, XP002786201, ISSN: 1553-7404
- RAINER KNAUS ET AL: "PL of coliphage lambda: an alternative solution for an efficient promoter", THE EMBO JOURNAL,, vol. 7, no. 9, 25 August 1988 (1988-08-25) , pages 2919-2923, XP001317078,
- KINCADE J M ET AL: "Bacteriophage lambda promoters pL and pR sequence determinants of in vivo activity and of sensitivity to the DNA gyrase inhibitor, coumermycin", GENE, ELSEVIER, AMSTERDAM, NL, vol. 97, no. 1, 1 January 1991 (1991-01-01), pages 7-12, XP023545045, ISSN: 0378-1119, DOI: 10.1016/0378-1119(91)90003-T [retrieved on 1991-01-01]
- WILLIAM R MCCLEARY: "Application of promoter swapping techniques to control expression of chromosomal genes", APPLIED MICROBIOLOGY AND BIOTECHNOLOGY, SPRINGER, BERLIN, DE, vol. 84, no. 4, 25 July 2009 (2009-07-25), pages 641-648, XP019737788, ISSN: 1432-0614, DOI: 10.1007/S00253-009-2137-Y
- PENGFEI GU ET AL: "One-step of tryptophan attenuator inactivation and promoter swapping to improve the production of L-tryptophan in Escherichia coli", MICROBIAL CELL FACTORIES,, vol. 11, no. 1, 2 March 2012 (2012-03-02), page 30, XP021118480, ISSN: 1475-2859, DOI: 10.1186/1475-2859-11-30
- RICE CHRISTOPHER D ET AL: "Employment of a promoter-swapping technique shows that PhoU modulates the activity of the PstSCAB2 ABC transporter in Escherichia coli.", APPLIED AND ENVIRONMENTAL MICROBIOLOGY FEB 2009, vol. 75, no. 3, February 2009 (2009-02), pages 573-582, XP002786202, ISSN: 1098-5336
- GUEGUEN ERWAN ET AL: "Promoter swapping unveils the role of the Citrobacter rodentium CTS1 type VI secretion system in interbacterial competition.", APPLIED AND ENVIRONMENTAL MICROBIOLOGY JAN 2013, vol. 79, no. 1, January 2013 (2013-01), pages 32-38, XP002786203, ISSN: 1098-5336

SEQUENCE LISTING

<110> Zymergen Inc.
Kimball, Aaron
Szyjka, Shawn
Frewen, Barbara
Treynor, Thomas
Serber, Zach
Dean, Erich Jedediah
Manchester, Shawn
Gora, Katherine
Flashman, Michael
Shellman, Erin
Haushalter, Robert
Morgan, Stacy-Anne
Blaisse, Michael
Ramakrishnan, Prabha
Rothschild-Mancinelli, Kyle
Kim, Youngnyun
Davis, Matthew
Wisnewski, Christy
Westfall, Patrick

<120> A HTP GENOMIC ENGINEERING PLATFORM FOR IMPROVING ESCHERICHIA COLI

<130> ZYMR-012/01WO 327574-2058

<150> US 62/515,870
<151> 2017-06-06

<160> 260

<170> PatentIn version 3.5

<210> 1
<211> 97
<212> DNA
<213> Unknown

<220>
<223> Expression promoter derived from Pcg0007_lib_39

<400> 1

tgccgtttct cgcgttgtgt gtggtactac gtggggacct aagcgtgtat tatggaaacg 60

tctgtatcgg ataagtagcg aggagtgttc gttaaaa 97

<210> 2
<211> 97
<212> DNA
<213> Unknown

<220>
<223> Expression promoter derived from Pcg0007

<400> 2

tgccgtttct cgcgttgtgt gtggtactac gtggggacct aagcgtgtaa gatggaaacg 60

tctgtatcgg ataagtagcg aggagtgttc gttaaaa 97

<210> 3
<211> 93
<212> DNA
<213> Unknown

<220>
<223> Expression promoter derived from Pcg1860

<400> 3

cttagctttg acctgcacaa atagttgcaa attgtcccac atacacataa agtagcttgc 60

gtatttaaaa ttatgaacct aaggggttta gca 93

<210> 4
<211> 98
<212> DNA
<213> Unknown

<220>
<223> Expression promoter derived from Pcg0755

<400> 4

aataaattta taccacacag tctattgcaa tagaccaagc tgttcagtag ggtgcatggg 60

agaagaattt cctaataaaa actcttaagg acctccaa 98

<210> 5
<211> 97
<212> DNA
<213> Unknown

<220>
<223> Expression promoter derived from Pcg0007_265

<400> 5

```
tgccgtttct cgcgttgtgt gtggtactac gtggggacct aagcgtgtac gctggaaacg      60
tctgtatcgg ataagtagcg aggagtgttc gttaaaa                               97
```

<210> 6
<211> 86
<212> DNA
<213> Unknown

<220>
<223> Expression promoter derived from Pcg3381

<400> 6

```
cgccggataa atgaattgat tattttaggc tcccagggat taagtctagg gtggaatgca      60
gaaatatttc ctacggaagg tccgtt                                           86
```

<210> 7
<211> 97
<212> DNA
<213> Unknown

<220>
<223> Expression promoter derived from Pcg0007_119

<400> 7

```
tgccgtttct cgcgttgtgt gtggtactac gtggggacct aagcgtgttg catggaaacg      60
tctgtatcgg ataagtagcg aggagtgttc gttaaaa                               97
```

<210> 8
<211> 87
<212> DNA
<213> Unknown

<220>
<223> Expression promoter derived from Pcg3121

<400> 8

```
gtggctaaaa cttttggaaa cttaagttac ctttaatcgg aaacttattg aattcgggtg      60
aggcaactgc aactctggac ttaaagc                                          87
```

<210> 9
<211> 25
<212> DNA
<213> Unknown

<220>

<223> cg0001 Terminator

<400> 9
gacccatctt cggatgggtc ttttt 25

<210> 10
<211> 30
<212> DNA
<213> Unknown

<220>
<223> cg0007 Terminator

<400> 10
cccgcccctg gaattctggg ggcgggtttt 30

<210> 11
<211> 24
<212> DNA
<213> Unknown

<220>
<223> cg0371 Terminator

<400> 11
ccggtaactt ttgtaagttg ccgg 24

<210> 12
<211> 27
<212> DNA
<213> Unknown

<220>
<223> cg0480 Terminator

<400> 12
cccctcagaa gcgattctga ggggttt 27

<210> 13
<211> 28
<212> DNA
<213> Unknown

<220>
<223> cg0494 Terminator

<400> 13
gcaccgcctt tcggggcggt gctttttt 28

<210> 14
<211> 28
<212> DNA
<213> Unknown

<220>
<223> cg0564 Terminator

<400> 14

ggccccatgc tttgcatggg gtcttttt 28

<210> 15
<211> 30
<212> DNA
<213> Unknown

<220>
<223> cg0610 Terminator

<400> 15
gcacttacct taactggtag gtgctttttt 30

<210> 16
<211> 24
<212> DNA
<213> Unknown

<220>
<223> cg0695 Terminator

<400> 16
acccggtcac cagaccgggt cttt 24

<210> 17
<211> 54
<212> DNA
<213> Unknown

<220>
<223> Cis1 encoding sequence

<400> 17
atgaaagcaa ttttcgtact gaaacatctt aatcatgcac aggagacttt ctaa 54

<210> 18
<211> 4
<212> PRT
<213> Unknown

<220>
<223> Serine-rich linker peptide

<400> 18

Gly Ser Gly Ser
1

<210> 19
<211> 5
<212> PRT
<213> Unknown

<220>
<223> Serine-rich linker Sequence

<400> 19

Gly Ser Gly Ser Gly
1 5

<210> 20
<211> 4
<212> PRT
<213> Unknown

<220>
<223> Serine-rich linker Sequence

<400> 20

Gly Ser Asn Gly
1

<210> 21
<211> 5
<212> PRT
<213> Unknown

<220>
<223> Alanine-rich linker Sequence

<400> 21

Thr Ser Ala Ala Ala
1 5

<210> 22
<211> 6
<212> PRT
<213> Unknown

<220>
<223> Linker Sequence

<400> 22

Ala Ala Ala Gly Gly Met
1 5

<210> 23
<211> 14
<212> PRT
<213> Unknown

<220>
<223> Linker Sequence

<400> 23

Ala Ala Ala Gly Gly Met Pro Pro Ala Ala Ala Gly Gly Met
1 5 10

<210> 24
<211> 6
<212> PRT

<213> Unknown

<220>
<223> Linker Sequence

<400> 24

Ala Ala Ala Gly Gly Met
1 5

<210> 25
<211> 9
<212> PRT
<213> Unknown

<220>
<223> Linker Sequence

<400> 25

Pro Pro Ala Ala Ala Gly Gly Met Met
1 5

<210> 26
<211> 87
<212> PRT
<213> Unknown

<220>
<223> Zif268

<400> 26

Pro Gly Glu Lys Pro Tyr Ala Cys Pro Val Glu Ser Cys Asp Arg Arg
1 5 10 15

Phe Ser Arg Ser Asp Glu Leu Thr Arg His Ile Arg Ile His Thr Gly
20 25 30

Gln Lys Pro Phe Gln Cys Arg Ile Cys Met Arg Asn Phe Ser Arg Ser
35 40 45

Asp His Leu Thr Thr His Ile Arg Thr His Thr Gly Glu Lys Pro Phe
50 55 60

Ala Cys Asp Ile Cys Gly Arg Lys Phe Ala Arg Ser Asp Glu Arg Lys
65 70 75 80

Arg His Thr Lys Ile His Thr
85

<210> 27
<211> 85
<212> PRT
<213> Unknown

<220>
<223> PBSII

<400> 27

```
Pro Gly Glu Lys Pro Tyr Ala Cys Pro Glu Cys Gly Lys Ser Phe Ser
1               5                   10                  15

Gln Arg Ala Asn Leu Arg Ala His Gln Arg Thr His Thr Gly Glu Lys
            20                  25                  30

Pro Tyr Lys Cys Pro Glu Cys Gly Lys Ser Phe Ser Arg Ser Asp His
        35                  40                  45

Leu Thr Thr His Gln Arg Thr His Thr Gly Glu Lys Pro Tyr Lys Cys
    50                  55                  60

Pro Glu Cys Gly Lys Ser Phe Ser Arg Ser Asp Val Leu Val Arg His
65                  70                  75                  80

Gln Arg Thr His Thr
                85
```

<210> 28
<211> 84
<212> PRT
<213> Unknown

<220>
<223> ZFa

<400> 28

```
Pro Gly Glu Arg Pro Phe Gln Cys Arg Ile Cys Met Arg Asn Phe Ser
1               5                   10                  15

Asp Ser Pro Thr Leu Arg Arg His Thr Arg Thr His Thr Gly Glu Lys
            20                  25                  30

Pro Phe Gln Cys Arg Ile Cys Met Arg Asn Phe Ser Val Arg His Asn
        35                  40                  45

Leu Thr Arg His Leu Arg Thr His Thr Gly Glu Lys Pro Phe Gln Cys
    50                  55                  60

Arg Ile Cys Met Arg Asn Phe Ser Asp Arg Thr Ser Leu Ala Arg His
65                  70                  75                  80

Leu Lys Thr His
```

<210> 29
<211> 84
<212> PRT
<213> Unknown

<220>
<223> ZFb

<400> 29

```
Pro Gly Glu Arg Pro Phe Gln Cys Arg Ile Cys Met Arg Asn Phe Ser
1               5                   10                  15

Lys Lys Asp His Leu His Arg His Thr Arg Thr His Thr Gly Glu Lys
            20                  25                  30

Pro Phe Gln Cys Arg Ile Cys Met Arg Asn Phe Ser Leu Ser Gln Thr
        35                  40                  45

Leu Lys Arg His Leu Arg Thr His Thr Gly Glu Lys Pro Phe Gln Cys
    50                  55                  60

Arg Ile Cys Met Arg Asn Phe Ser Arg Leu Asp Met Leu Ala Arg His
65                  70                  75                  80

Leu Lys Thr His
```

<210> 30
<211> 84
<212> PRT
<213> Unknown

<220>
<223> ZFc

<400> 30

```
Pro Gly Glu Arg Pro Phe Gln Cys Arg Ile Cys Met Arg Asn Phe Ser
1               5                   10                  15

Ser Pro Ser Lys Leu Ile Arg His Thr Arg Thr His Thr Gly Glu Lys
            20                  25                  30

Pro Phe Gln Cys Arg Ile Cys Met Arg Asn Phe Ser Asp Gly Ser Asn
        35                  40                  45

Leu Ala Arg His Leu Arg Thr His Thr Gly Glu Lys Pro Phe Gln Cys
    50                  55                  60

Arg Ile Cys Met Arg Asn Phe Ser Arg Val Asp Asn Leu Pro Arg His
65                  70                  75                  80

Leu Lys Thr His
```

<210> 31
<211> 83
<212> PRT
<213> Unknown

<220>
<223> Tyr123

<400> 31

```
Glu Lys Pro Tyr Lys Cys Pro Glu Cys Gly Lys Ser Phe Ser Asp Arg
1               5                   10                  15

Ser Asn Leu Thr Arg His Gln Arg Thr His Thr Gly Glu Lys Pro Tyr
            20                  25                  30

Lys Cys Pro Glu Cys Gly Lys Ser Phe Ser Thr Thr Ser Asn Leu Ala
        35                  40                  45

Arg His Gln Arg Thr His Thr Gly Glu Lys Pro Phe Lys Cys Pro Glu
    50                  55                  60

Cys Gly Lys Ser Phe Ser Arg Ser Asp Ala Leu Thr Arg His Gln Arg
65                  70                  75                  80

Thr His Thr
```

<210> 32
<211> 83
<212> PRT
<213> Unknown

<220>

<223> Tyr456

<400> 32

Glu Lys Pro Tyr Lys Cys Pro Glu Cys Gly Lys Ser Phe Ser Gln Ser
1               5                   10                  15

Ser Asn Leu Ala Arg His Gln Arg Thr His Thr Gly Glu Lys Pro Tyr
            20                  25                  30

Lys Cys Pro Glu Cys Gly Lys Ser Phe Ser Arg Ser Asp His Leu Thr
        35                  40                  45

Lys His Gln Arg Thr His Thr Gly Glu Lys Pro Phe Lys Cys Pro Glu
    50                  55                  60

Cys Gly Lys Ser Phe Ser Gln Ser Ser Asn Leu Ala Arg His Gln Arg
65                  70                  75                  80

Thr His Thr

<210> 33
<211> 94
<212> PRT
<213> Unknown

<220>
<223> Blues Zinc Finger

<400> 33

Ala Ser Asp Asp Arg Pro Tyr Ala Cys Pro Val Glu Ser Cys Asp Arg
1               5                   10                  15

Arg Phe Ser Arg Arg Asp Val Leu Met Asn His Ile Arg Ile His Thr
            20                  25                  30

Gly Gln Lys Pro Phe Gln Cys Arg Ile Cys Met Arg Asn Phe Ser Arg
        35                  40                  45

Ser Asp His Leu Thr Thr His Ile Arg Thr His Thr Gly Glu Lys Pro
    50                  55                  60

Phe Ala Cys Asp Ile Cys Gly Arg Lys Phe Ala Asn Arg Asp Thr Leu
65                  70                  75                  80

Thr Arg His Ser Lys Ile His Leu Arg Gln Asn Asp Leu Glu
                85                  90

<210> 34

<211> 94
<212> PRT
<213> Unknown

<220>
<223> Jazz Zinc Finger

<400> 34

```
Ala Ser Asp Asp Arg Pro Tyr Ala Cys Pro Val Glu Ser Cys Asp Arg
1               5                   10                  15

Arg Phe Ser Arg Ser Asp Glu Leu Thr Arg His Ile Arg Ile His Thr
            20                  25                  30

Gly Gln Lys Pro Phe Gln Cys Arg Ile Cys Met Arg Asn Phe Ser Ser
        35                  40                  45

Arg Asp Val Leu Arg Arg His Asn Arg Thr His Thr Gly Glu Lys Pro
    50                  55                  60

Phe Ala Cys Asp Ile Cys Gly Arg Lys Phe Ala Ser Arg Asp Val Leu
65                  70                  75                  80

Arg Arg His Asn Arg Ile His Leu Arg Gln Asn Asp Leu Glu
                85                  90
```

<210> 35
<211> 127
<212> PRT
<213> Unknown

<220>
<223> Bagly zinc finger

<400> 35

```
Glu Phe Met Thr Gly Asp Arg Pro Tyr Ala Cys Pro Val Glu Ser Cys
1               5                   10                  15

Asp Arg Arg Phe Ser Arg Ser Asp Glu Leu Thr Arg His Ile Arg Ile
            20                  25                  30

His Thr Gly Gln Lys Pro Phe Gln Cys Arg Ile Cys Met Arg Asn Phe
        35                  40                  45
```

Ser Ser Arg Asp Val Leu Arg Arg His Asn Arg Thr His Thr Gly Glu
50 55 60

Lys Pro Phe Ala Cys Asp Ile Cys Gly Arg Lys Phe Ala Ser Arg Asp
65 70 75 80

Val Leu Arg Arg His Asn Arg Ile His Leu Arg Gln Gly Arg Ser His
85 90 95

Val Cys Ala Glu Cys Gly Lys Ala Phe Val Glu Ser Ser Lys Leu Lys
100 105 110

Arg His Gln Leu Val His Thr Gly Glu Lys Pro Phe Gln Leu Glu
115 120 125

<210> 36
<211> 11
<212> DNA
<213> Unknown

<220>
<223> Bagly zinc finger binding site

<400> 36
cgggctgctg c 11

<210> 37
<211> 175
<212> PRT
<213> Unknown

<220>
<223> Gli1

<400> 37

Lys Arg Glu Pro Glu Ser Val Tyr Glu Thr Asp Cys Arg Trp Asp Gly
1 5 10 15

Cys Ser Gln Glu Phe Asp Ser Gln Glu Gln Leu Val His His Ile Asn
20 25 30

Ser Glu His Ile His Gly Glu Arg Lys Glu Phe Val Cys His Trp Gly
35 40 45

Gly Cys Ser Arg Glu Leu Arg Pro Phe Lys Ala Gln Tyr Met Leu Val
50 55 60

Val His Met Arg Arg His Thr Gly Glu Lys Pro His Lys Cys Thr Phe
65 70 75 80

```
Glu Gly Cys Arg Lys Ser Tyr Ser Arg Leu Glu Asn Leu Lys Thr His
                85                  90                  95

Leu Arg Ser His Thr Gly Glu Lys Pro Tyr Met Cys Glu His Glu Gly
            100                 105                 110

Cys Ser Lys Ala Phe Ser Asn Ala Ser Asp Arg Ala Lys His Gln Asn
        115                 120                 125

Arg Thr His Ser Asn Glu Lys Pro Tyr Val Cys Lys Leu Pro Gly Cys
    130                 135                 140

Thr Lys Arg Tyr Thr Asp Pro Ser Ser Leu Arg Lys His Val Lys Thr
145                 150                 155                 160

Val His Gly Pro Asp Ala His Val Thr Lys Arg His Arg Gly Asp
                165                 170                 175
```

<210> 38
<211> 15
<212> DNA
<213> Unknown

<220>
<223> Gli1 binding site

<400> 38
gaccacccaa gacga 15

<210> 39
<211> 84
<212> PRT
<213> Unknown

<220>
<223> HIVC zinc finger

<400> 39

Pro Phe Gln Cys Arg Ile Cys Met Arg Asn Phe Ser Leu Arg Thr Asp
1               5                   10                  15

Leu Asp Arg His Thr Arg Thr His Thr Gly Glu Lys Pro Phe Gln Cys
            20                  25                  30

Arg Ile Cys Met Arg Asn Phe Ser Leu Ser Gln Thr Leu Arg Arg His
        35                  40                  45

Leu Arg Thr His Thr Gly Glu Lys Pro Phe Gln Cys Arg Ile Cys Met
    50                  55                  60

Arg Asn Phe Ser Leu Arg Ser Asn Leu Gly Arg His Leu Lys Thr His

65                  70                  75                  80

Thr Gly Glu Lys

<210> 40
<211> 100
<212> PRT
<213> unknown

<220>
<223> B3 zinc finger

<400> 40

Ala Gln Ala Ala Leu Glu Pro Lys Glu Lys Pro Tyr Ala Cys Pro Glu
1               5                   10                  15

Cys Gly Lys Ser Phe Ser Asp Pro Gly Asn Leu Val Arg His Gln Arg
            20                  25                  30

Thr His Thr Gly Glu Lys Pro Tyr Lys Cys Pro Glu Cys Gly Lys Ser
        35                  40                  45

Phe Ser Arg Ser Asp Lys Leu Val Arg His Gln Arg Thr His Thr Gly
    50                  55                  60

Glu Lys Pro Tyr Lys Cys Pro Glu Cys Gly Lys Ser Phe Ser Gln Ser
65                  70                  75                  80

Ser His Leu Val Arg His Gln Arg Thr His Thr Gly Lys Lys Thr Ser
                85                  90                  95

Gly Gln Ala Gly
            100

<210> 41
<211> 100
<212> PRT
<213> unknown

<220>
<223> N1 Zinc Finger

<400> 41

```
Ala Gln Ala Ala Leu Glu Pro Lys Glu Lys Pro Tyr Ala Cys Pro Glu
1               5                   10                  15

Cys Gly Lys Ser Phe Ser Gln Ser Ser Ser Leu Val Arg His Gln Arg
            20                  25                  30

Thr His Thr Gly Glu Lys Pro Tyr Lys Cys Pro Glu Cys Gly Lys Ser
        35                  40                  45

Phe Ser Gln Ser Ser Asn Leu Val Arg His Gln Arg Thr His Thr Gly
    50                  55                  60

Glu Lys Pro Tyr Lys Cys Pro Glu Cys Gly Lys Ser Phe Ser Arg Ser
65                  70                  75                  80

Asp Lys Leu Val Arg His Gln Arg Thr His Thr Gly Lys Lys Thr Ser
                85                  90                  95

Gly Gln Ala Gly
            100
```

<210> 42
<211> 94
<212> PRT
<213> Unknown

<220>
<223> Sp-1 first zinc finger

<400> 42

```
Pro Gly Lys Lys Lys Gln His Ile Cys His Ile Gln Gly Cys Gly Lys
1               5                   10                  15

Val Tyr Gly Lys Thr Ser His Leu Arg Ala His Leu Arg Trp His Thr
            20                  25                  30

Gly Glu Arg Pro Phe Met Cys Thr Trp Ser Tyr Cys Gly Lys Arg Phe
        35                  40                  45

Thr Arg Ser Asp Glu Leu Gln Arg His Lys Arg Thr His Thr Gly Glu
    50                  55                  60

Lys Lys Phe Ala Cys Pro Glu Cys Pro Lys Arg Phe Met Arg Ser Asp
65                  70                  75                  80

His Leu Ser Lys His Ile Lys Thr His Gln Asn Lys Lys Gly
                85                  90
```

<210> 43
<211> 90
<212> PRT
<213> Unknown

<220>
<223> Sp-1 second finger

<400> 43

```
Pro Gly Lys Lys Lys Gln His Ala Cys Pro Glu Cys Gly Lys Ser Phe
1               5                   10                  15

Ser Lys Ser Ser His Leu Arg Ala His Gln Arg Thr His Thr Gly Glu
            20                  25                  30

Arg Pro Tyr Lys Cys Pro Glu Cys Gly Lys Ser Phe Ser Arg Ser Asp
        35                  40                  45

Glu Leu Gln Arg His Gln Arg Thr His Thr Gly Glu Lys Pro Tyr Lys
    50                  55                  60

Cys Pro Glu Cys Gly Lys Ser Phe Ser Arg Ser Asp His Leu Ser Lys
65                  70                  75                  80

His Gln Arg Thr His Gln Asn Lys Lys Gly
                85                  90
```

<210> 44
<211> 74
<212> PRT
<213> Unknown

<220>
<223> Class I SH3 protein binding domain

<400> 44

```
Glu Gly Tyr Gln Tyr Arg Ala Leu Tyr Asp Tyr Lys Lys Glu Arg Glu
1               5                   10                  15

Glu Asp Ile Asp Leu His Leu Gly Asp Ile Leu Thr Val Asn Lys Gly
            20                  25                  30

Ser Leu Val Ala Leu Gly Phe Ser Asp Gly Gln Glu Ala Arg Pro Glu
        35                  40                  45

Glu Ile Gly Trp Leu Asn Gly Tyr Asn Glu Thr Thr Gly Glu Arg Gly
    50                  55                  60

Asp Phe Pro Gly Thr Tyr Val Glu Tyr Ile
65                  70
```

<210> 45
<211> 54
<212> PRT
<213> Unknown

<220>
<223> Class II SH3 Protein Binding Site

<400> 45

```
Tyr Val Arg Ala Leu Phe Asp Phe Asn Gly Asn Asp Glu Glu Asp Leu
1               5                   10                  15

Pro Phe Lys Lys Gly Asp Ile Leu Arg Ile Arg Asp Lys Pro Glu Glu
            20                  25                  30

Gln Trp Trp Asn Ala Glu Asp Ser Glu Gly Lys Arg Gly Met Ile Pro
        35                  40                  45

Val Pro Tyr Val Glu Lys
    50
```

<210> 46
<211> 58
<212> PRT
<213> Unknown

<220>
<223> SH3 protein binding site

<400> 46

Met Ala Glu Tyr Val Arg Ala Leu Phe Asp Phe Asn Gly Asn Asp Glu
1               5                   10                  15

Glu Asp Leu Pro Phe Lys Lys Gly Asp Ile Leu Arg Ile Arg Asp Lys
            20                  25                  30

Pro Glu Glu Gln Trp Trp Asn Ala Glu Asp Ser Glu Gly Lys Arg Gly
        35                  40                  45

Met Ile Pro Val Pro Tyr Val Glu Lys Tyr
    50                  55

<210> 47
<211> 7
<212> PRT
<213> Unknown

<220>
<223> SH3 recruitment peptide

<400> 47

Arg Pro Leu Pro Val Ala Pro
1               5

<210> 48
<211> 13
<212> PRT
<213> Unknown

<220>
<223> SH3 recruitment Peptide

<400> 48

Pro Pro Pro Ala Leu Pro Pro Lys Arg Arg Arg Pro Gly
1               5                   10

<210> 49
<211> 10
<212> PRT
<213> Unknown

<220>
<223> SH3 recruitment peptide

<400> 49

Pro Pro Pro Ala Leu Pro Pro Lys Lys Arg
1               5                   10

<210> 50
<211> 84
<212> PRT
<213> Unknown

<220>
<223> PDZ protein binding domain

<400> 50

```
Glu Ile Thr Leu Glu Arg Gly Asn Ser Gly Leu Gly Phe Ser Ile Ala
1               5                   10                  15

Gly Gly Thr Asp Asn Pro His Ile Gly Asp Asp Pro Ser Ile Phe Ile
            20                  25                  30

Thr Lys Ile Ile Pro Gly Gly Ala Ala Ala Gln Asp Gly Arg Leu Arg
        35                  40                  45

Val Asn Asp Ser Ile Leu Phe Val Asn Glu Val Asp Val Arg Glu Val
    50                  55                  60

Thr His Ser Ala Ala Val Glu Ala Leu Lys Glu Ala Gly Ser Ile Val
65                  70                  75                  80

Arg Leu Tyr Val
```

<210> 51
<211> 87
<212> PRT
<213> Unknown

<220>
<223> PDZ protein binding domain

<400> 51

```
Val Met Glu Ile Lys Leu Ile Lys Gly Pro Lys Gly Leu Gly Phe Ser
1               5                   10                  15

Ile Ala Gly Gly Val Gly Asn Gln His Ile Pro Gly Asp Asn Ser Ile
            20                  25                  30

Tyr Val Thr Lys Ile Ile Glu Gly Gly Ala Ala His Lys Asp Gly Arg
        35                  40                  45

Leu Gln Ile Gly Asp Lys Ile Leu Ala Val Asn Ser Val Gly Leu Glu
    50                  55                  60

Asp Val Met His Glu Asp Ala Val Ala Ala Leu Lys Asn Thr Tyr Asp
65                  70                  75                  80

Val Val Tyr Leu Lys Val Ala
                85
```

<210> 52
<211> 79
<212> PRT
<213> Unknown

<220>
<223> PDZ protein binding domain

<400> 52

```
Arg Ile Val Ile His Arg Gly Ser Thr Gly Leu Gly Phe Asn Ile Val
1               5                   10                  15

Gly Gly Glu Asp Gly Glu Gly Ile Phe Ile Ser Phe Ile Leu Ala Gly
            20                  25                  30

Gly Pro Ala Asp Leu Ser Gly Glu Leu Arg Lys Gly Asp Gln Ile Leu
        35                  40                  45

Ser Val Asn Gly Val Asp Leu Arg Asn Ala Ser His Glu Gln Ala Ala
    50                  55                  60

Ile Ala Leu Lys Asn Ala Gly Gln Thr Val Thr Ile Ile Ala Gln
65                  70                  75
```

<210> 53
<211> 82
<212> PRT
<213> Unknown

<220>
<223> PDZ protein binding domain

<400> 53

```
Arg Arg Val Thr Val Arg Lys Ala Asp Ala Gly Gly Leu Gly Ile Ser
1               5                   10                  15

Ile Lys Gly Gly Arg Glu Asn Lys Met Pro Ile Leu Ile Ser Lys Ile
            20                  25                  30

Phe Lys Gly Leu Ala Ala Asp Gln Thr Glu Ala Leu Phe Val Gly Asp
        35                  40                  45

Ala Ile Leu Ser Val Asn Gly Glu Asp Leu Ser Ser Ala Thr His Asp
    50                  55                  60

Glu Ala Val Gln Ala Leu Lys Lys Thr Gly Lys Glu Val Val Leu Glu
65                  70                  75                  80

Val Lys
```

<210> 54
<211> 98
<212> PRT
<213> Unknown

<220>
<223> PDZ protein binding domain

<400> 54

Met Leu Gln Arg Arg Arg Val Thr Val Arg Lys Ala Asp Ala Gly Gly
1 5 10 15

Leu Gly Ile Ser Ile Lys Gly Gly Arg Glu Asn Lys Met Pro Ile Leu
20 25 30

Ile Ser Lys Ile Phe Lys Gly Leu Ala Ala Asp Gln Thr Glu Ala Leu
35 40 45

Phe Val Gly Asp Ala Ile Leu Ser Val Asn Gly Glu Asp Leu Ser Ser
50 55 60

Ala Thr His Asp Glu Ala Val Gln Ala Leu Lys Lys Thr Gly Lys Glu
65 70 75 80

Val Val Leu Glu Val Lys Tyr Met Lys Glu Val Ser Pro Tyr Phe Lys
85 90 95

Gly Ser

<210> 55
<211> 5
<212> PRT
<213> Unknown

<220>
<223> PDZ recruitment peptide

<400> 55

Ile Glu Ser Asp Val
1 5

<210> 56
<211> 6
<212> PRT
<213> Unknown

<220>
<223> PDZ recruitment peptide

<400> 56

Val Lys Glu Ser Leu Val
1 5

<210> 57
<211> 7
<212> PRT
<213> Unknown

<220>
<223> PDZ recruitment peptide

<400> 57

Gly Val Lys Glu Ser Leu Val
1 5

<210> 58
<211> 7
<212> PRT
<213> Unknown

<220>
<223> PDZ recruitment peptide

<400> 58

Gly Val Lys Gln Ser Leu Leu
1 5

<210> 59
<211> 7
<212> PRT
<213> Unknown

<220>
<223> PDZ recruitment peptide

<400> 59

Gly Val Lys Glu Ser Gly Ala
1 5

<210> 60
<211> 7
<212> PRT
<213> Unknown

<220>
<223> PDZ recruitment peptide

<400> 60

Tyr Val Lys Glu Ser Leu Val
1 5

<210> 61
<211> 5
<212> PRT

<213> Unknown

<220>
<223> PDZ recruitment peptide

<400> 61

```
Val Glu Thr Asp Val
1               5
```

<210> 62
<211> 43
<212> PRT
<213> Unknown

<220>
<223> GBD protein binding domain

<400> 62

```
Ala Asp Ile Gly Thr Pro Ser Asn Phe Gln His Ile Gly His Val Gly
1               5                   10                  15

Trp Asp Pro Asn Thr Gly Phe Asp Leu Asn Asn Leu Asp Pro Glu Leu
            20                  25                  30

Lys Asn Leu Phe Asp Met Cys Gly Ile Ser Glu
        35                  40
```

<210> 63
<211> 36
<212> PRT
<213> Unknown

<220>
<223> GBD protein binding domain

<400> 63

```
Lys Glu Arg Pro Glu Ile Ser Leu Pro Ser Asp Phe Glu His Thr Ile
1               5                   10                  15

His Val Gly Phe Asp Ala Val Thr Gly Glu Phe Thr Gly Met Pro Glu
            20                  25                  30

Gln Trp Ala Arg
        35
```

<210> 64
<211> 80
<212> PRT
<213> Unknown

<220>

<223> GBD protein binding domain

<400> 64

```
Met Thr Lys Ala Asp Ile Gly Thr Pro Ser Asn Phe Gln His Ile Gly
1               5                   10                  15

His Val Gly Trp Asp Pro Asn Thr Gly Phe Asp Leu Asn Asn Leu Asp
            20                  25                  30

Pro Glu Leu Lys Asn Leu Phe Asp Met Cys Gly Ile Ser Glu Ala Gln
        35                  40                  45

Leu Lys Asp Arg Glu Thr Ser Lys Val Ile Tyr Asp Phe Ile Glu Lys
    50                  55                  60

Thr Gly Gly Val Glu Ala Val Lys Asn Glu Leu Arg Arg Gln Ala Pro
65                  70                  75                  80
```

<210> 65
<211> 32
<212> PRT
<213> Unknown

<220>
<223> GBD recruitment peptide

<400> 65

```
Leu Val Gly Ala Leu Met His Val Met Gln Lys Arg Ser Arg Ala Ile
1               5                   10                  15

His Ser Ser Asp Glu Gly Glu Asp Gln Ala Gly Asp Glu Asp Glu Asp
            20                  25                  30
```

<210> 66
<211> 47
<212> PRT
<213> Unknown

<220>
<223> Leucine Zipper binding domain

<400> 66

```
Leu Glu Ile Glu Ala Ala Phe Leu Glu Arg Glu Asn Thr Ala Leu Glu
1               5                   10                  15

Thr Arg Val Ala Glu Leu Arg Gln Arg Val Gln Arg Leu Arg Asn Arg
            20                  25                  30

Val Ser Gln Tyr Arg Thr Arg Tyr Gly Pro Leu Gly Gly Gly Lys
        35                  40                  45
```

<210> 67
<211> 47
<212> PRT
<213> Unknown

<220>
<223> Leucine Zipper binding domain

<400> 67

```
Leu Glu Ile Glu Ala Ala Phe Leu Glu Arg Glu Asn Thr Ala Leu Glu
1               5                   10                  15

Thr Arg Val Ala Glu Leu Arg Gln Arg Val Gln Arg Leu Arg Asn Arg
            20                  25                  30

Val Ser Gln Tyr Arg Thr Arg Tyr Gly Pro Leu Gly Gly Gly Lys
        35                  40                  45
```

<210> 68
<211> 47
<212> PRT
<213> Unknown

<220>
<223> Leucine Zipper binding site

<400> 68

```
Leu Glu Ile Arg Ala Ala Phe Leu Arg Gln Arg Asn Thr Ala Leu Arg
1               5                   10                  15

Thr Glu Val Ala Glu Leu Glu Gln Glu Val Gln Arg Leu Glu Asn Glu
            20                  25                  30

Val Ser Gln Tyr Glu Thr Arg Tyr Gly Pro Leu Gly Gly Gly Lys
        35                  40                  45
```

<210> 69
<211> 88
<212> DNA
<213> Unknown

<220>
<223> Figure 43 Bicistronic Design (BCD) regulatory sequence

<400> 69

```
gggcccaagt tcacttaaaa aggagatcaa caatgaaagc aattttcgta ctgaaacatc      60

ttaatcatgc acaggagact ttctaatg                                         88
```

<210> 70
<211> 88

<212> DNA
<213> Unknown

<220>
<223> Figure 43 Bicistronic Design (BCD) regulatory sequence

<220>
<221> misc_feature
<222> (36)..(70)
<223> n is a, c, g, or t

<400> 70

```
gggcccaagt tcacttaaaa aggagatcaa caatgnnnnn nnnnnnnnnn nnnnnnnnnn    60

nnnnnnnnnn acaggagact ttctaatg                                        88
```

<210> 71
<211> 77
<212> DNA
<213> Escherichia coli

<400> 71

```
gatatgatct atatcaattt ctcatctata atgctttgtt agtatctcgt cgccgactta    60

ataaagagag agttagt                                                    77
```

<210> 72
<211> 78
<212> DNA
<213> Escherichia coli

<400> 72

```
atgtcggata tctggtggtg aaatacttta tgccatgata atttaatacg atgtatttat    60

tatatggagc acttaatt                                                   78
```

<210> 73
<211> 77
<212> DNA
<213> Escherichia coli

<400> 73

```
gaggctcttt gtgctaaact ggcccgccga atgtatagta cacttcggtt ggataggtaa    60

tttggcgaga taatacg                                                    77
```

<210> 74
<211> 80
<212> DNA
<213> Escherichia coli

<400> 74

cgtgtggatt gtgtcttgcg acgatgggca ctaaatgtta aaaggtgccc ctcaacaaaa 60

aagacacaca ggggaaaggc 80

<210> 75
<211> 70
<212> DNA
<213> Escherichia coli

<400> 75

caaactaatt aataaatagt taattaacgc tcatcattgt acaatgaact gtacaaaaga 60

ggagattgac 70

<210> 76
<211> 71
<212> DNA
<213> Escherichia coli

<400> 76

caaatctgtc aatttttcct ggaactggcg ttttcagtta tgattgtggg acttatcaaa 60

aaggagaggc c 71

<210> 77
<211> 78
<212> DNA
<213> Escherichia coli

<400> 77

tctttgtcac aaaggtggag gcaatgtcag tggtgtgtga caataagagt atcggcagga 60

cattaagagg aatgagcc 78

<210> 78
<211> 76
<212> DNA
<213> Escherichia coli

<400> 78

tttacgctgc gtcgatgcac agcctcatca ttttgcagta tccttaagat attccttata 60

tcttcaggag atcgtc 76

<210> 79
<211> 74

<212> DNA
<213> Escherichia coli

<400> 79

taatggaaac gcattagccg aatcggcaaa aattggttac cttacatctc atcgaaaaca 60

cggaggaagt atag 74

<210> 80
<211> 76
<212> DNA
<213> Escherichia coli

<400> 80

tgtttaaaaa tggcttgcca taattaacgt tgtatgtgat aacagatttc gggttaaacg 60

aggtacagtt ctgttt 76

<210> 81
<211> 72
<212> DNA
<213> Escherichia coli

<400> 81

aatcgtagct tcctgttgtc attaggttat tttacctgta taaataacca gtatattcaa 60

caggggggcta tt 72

<210> 82
<211> 77
<212> DNA
<213> Escherichia coli

<400> 82

aagacacatt ttatattgaa cgtccaatca ataaccgctt taatagataa acaccgctga 60

tgaatggagt ggcgaaa 77

<210> 83
<211> 81
<212> DNA
<213> Escherichia coli

<400> 83

atcacaaatc gcgaagagtt tcccattaat ttttgatata tttaaaactt aggacttatt 60

tgaatcacat ttgaggtggt t 81

<210> 84
<211> 79
<212> DNA
<213> Escherichia coli

<400> 84

aataaaccca tctatagatg gtaaaaatag gttgtggcaa ttatcattgc atcattccct 60

tttcgaatga gtttctatt 79

<210> 85
<211> 76
<212> DNA
<213> Escherichia coli

<400> 85

agaaataaaa aaacgtgaaa ttattatgcc gccaggcgta gtatcgcagc aggtaagatg 60

attcaggaga ttttaa 76

<210> 86
<211> 78
<212> DNA
<213> Escherichia coli

<400> 86

gacaccatcg aatggcgcaa aacctttcgc ggtatggcat gatagcgccc ggaagagagt 60

caattcaggg tggtgaat 78

<210> 87
<211> 80
<212> DNA
<213> Escherichia coli

<400> 87

tttgtgatcg ttatctcgat atttaaaaac aaataatttc attatatttt gaaatcgaaa 60

acaaacgaca ggatatgaaa 80

<210> 88
<211> 80
<212> DNA
<213> Escherichia coli

<400> 88

cgacgcgtcg tgattaggtg aaccccttct cgttatggca aaataagcca atacagaacc 60

agcattatct ggagaatttc 80

<210> 89
<211> 74
<212> DNA
<213> Escherichia coli

<400> 89

ttaggaatag ccgccgttca aaaatgtgtc actggtttac acttattcag gaatgcacaa 60

tgaacggagg tcct 74

<210> 90
<211> 73
<212> DNA
<213> Escherichia coli

<400> 90

gagtgcaaaa agtgctgtaa ctctgaaaaa gcgatggtag aatccatttt taagcaaacg 60

gtgattttga aaa 73

<210> 91
<211> 77
<212> DNA
<213> Escherichia coli

<400> 91

gcgacaactt tcgtaaaaca tccctaccct gcttcaggta tactatgccc ctcgattcca 60

caaacatcag gcagacc 77

<210> 92
<211> 75
<212> DNA
<213> Escherichia coli

<400> 92

tgtagcggag ttgtttttgt gtttacaaac aatggctcta cactgcaaac agacataaca 60

acattcgggg tgaat 75

<210> 93
<211> 70
<212> DNA
<213> Escherichia coli

<400> 93

actttttccac aggtagatcc caacgcgttc acagcgtaca atacgccact cttaataaag 60

gtggcggttt 70

<210> 94
<211> 78
<212> DNA
<213> Escherichia coli

<400> 94

ttaacaattg atgattttgc caacagccca catagcgcga tatactgaaa attctcgcag 60

caactgaatg ttaagcct 78

<210> 95
<211> 78
<212> DNA
<213> Escherichia coli

<400> 95

aaaagcctaa ctcacttttg attcattaaa aaagaaggct aaaatagaat gaatcatcaa 60

tccgcataag aaaatcct 78

<210> 96
<211> 75
<212> DNA
<213> Escherichia coli

<400> 96

cggctccagg ttacttcccg taggattctt gctttaatag tgggattaat ttccacatta 60

aaacagggat tgatc 75

<210> 97
<211> 79
<212> DNA
<213> Escherichia coli

<400> 97

tcacttaaca acaggcggta agcaacgcga aattctgcta ccatccacgc actctttatc 60

tgaataaatg gcagcgact 79

<210> 98
<211> 74
<212> DNA

<213> Escherichia coli

<400> 98

acggttctgt ctgcttgctt ttaatgccat accaaacgta ccattgagac acttgtttgc 60

acagaggatg gccc 74

<210> 99
<211> 81
<212> DNA
<213> Escherichia coli

<400> 99

atcagcgttt ttggctggcg gcgtagcgat gcgctggtta ctctgaaaac ggtctatgca 60

aattaacaaa agagaatagc t 81

<210> 100
<211> 82
<212> DNA
<213> Escherichia coli

<400> 100

atgctaaatc cttacttccg catattctct gagcgggtat gctacctgtt gtatcccaat 60

ttcatacagt taaggacagg cc 82

<210> 101
<211> 79
<212> DNA
<213> Escherichia coli

<400> 101

gatagcctga atcagtattg atctgctggc aagaacagac tactgtatat aaaaacagta 60

taacttcagg cagattatt 79

<210> 102
<211> 80
<212> DNA
<213> Escherichia coli

<400> 102

ctctcccgcg tgagaaatac gcttccccgt aagcgcatgg taaactatgc cttcaaatcg 60

ggcttatcgc gagtaaatct 80

<210> 103

<211> 80
<212> DNA
<213> Escherichia coli

<400> 103

aaaacacgcc accccttgaa ccaacgggcg ttttccgtaa cactgaaaga atgtaagcgt 60

ttacccacta aggtattttc 80

<210> 104
<211> 76
<212> DNA
<213> Escherichia coli

<400> 104

ttcagggctg actgtttgca taaaaattca tctgtatgca caataatgtt gtatcaacca 60

ccatatcggg tgactt 76

<210> 105
<211> 80
<212> DNA
<213> Escherichia coli

<400> 105

gttagcggct tacacgcggt cacattcaaa tgcgattctg ctacaatcct ccccccgttc 60

gaagattgag caatacacct 80

<210> 106
<211> 80
<212> DNA
<213> Escherichia coli

<400> 106

ttcgcttcag atcgttgaca gccgcactcc atgacgggta aaaagtggat aaaataattt 60

tacccaccgg atttttaccc 80

<210> 107
<211> 72
<212> DNA
<213> Escherichia coli

<400> 107

gcgtacaggc accgtagcat ttgtccgtta taacgcacaa gtgataaact tccgttttgc 60

cggaggagtc gc 72

<210> 108
<211> 76
<212> DNA
<213> Escherichia coli

<400> 108

tgttaacgcg ttatctcgtc gcgacctata agtttgggta atatgtgctg gaatttgccc 60

tgtctggaga atcgca 76

<210> 109
<211> 71
<212> DNA
<213> Escherichia coli

<400> 109

gttgacaaca ttttctgcta accctgtgac ctgcaatact gttttgcggg tgatcgacaa 60

ggagacttaa c 71

<210> 110
<211> 72
<212> DNA
<213> Escherichia coli

<400> 110

ttcagtgttc agttcgttaa ttcatctgtt ggggagtata attcctcaag ttaacttgag 60

gtaaagcgat tt 72

<210> 111
<211> 75
<212> DNA
<213> Escherichia coli

<400> 111

ctgattatga ttgtgtattt aattggttgt tatttgacta ctatcaactt gttttaattt 60

tatgataggt gcaag 75

<210> 112
<211> 81
<212> DNA
<213> Escherichia coli

<400> 112

acactataat ctgattttaa cgatgattcg tgcggggtaa aatagtaaaa acgatctatt 60

cacctgaaag agaaataaaa a 81

<210> 113
<211> 81
<212> DNA
<213> Escherichia coli

<400> 113

ccgttgctct ctgatttctc atttcatgct cacccaatat gatggcggcg ttttctaaaa 60

ctgttaaaga atgaggtaag t 81

<210> 114
<211> 72
<212> DNA
<213> Escherichia coli

<400> 114

ctgctatcag cgtagttagc cctctggtat gatgagtcca actttgtttt gctgtgttat 60

ggaaatctca ct 72

<210> 115
<211> 71
<212> DNA
<213> Escherichia coli

<400> 115

gttgttgtta tgcccccagg tatttacagt gtgagaaaga attattttga ctttagcgga 60

gcagttgaag a 71

<210> 116
<211> 82
<212> DNA
<213> Escherichia coli

<400> 116

tttttcgcct ttcatacttg caaaagcgga gaatcagcta tccttttccc tgaaacctca 60

tcaactcaaa gggagaatcg tg 82

<210> 117
<211> 75
<212> DNA
<213> Escherichia coli

<400> 117

cagactttac ccattgctga atgcacgggt aacgttaggc tcaaataatt aaacaacacg 60

ttacaggaca acagg 75

<210> 118
<211> 82
<212> DNA
<213> Escherichia coli

<400> 118

ctcctgaaca tccactcgat cttcgccttc ttccggttta ttgtgtttta accacctgcc 60

cgtaaacctg gagaaccatc gc 82

<210> 119
<211> 79
<212> DNA
<213> Escherichia coli

<400> 119

atataaaacc gttactcctt tcacgtccgt tataaatatg atggctatta gaaagtcatt 60

aaatttataa gggtgcgca 79

<210> 120
<211> 80
<212> DNA
<213> Escherichia coli

<400> 120

tatgaacagc ggcactggtc aggatgaacg gcttacggca gaatatgaac agatatgaac 60

agaatgagta aaccctctg 80

<210> 121
<211> 80
<212> DNA
<213> Escherichia coli

<400> 121

gggtaaagcg ccagggccag aagtcgatac gacctgtgct atgattcata aatcacaaca 60

ataacaacag actgaagcga 80

<210> 122
<211> 74

<212> DNA
<213> Escherichia coli

<400> 122

tgcgtttcca tttgagtatc ctgaaaacgg gcttttcagc atggaacgta catattaaat 60

agtaggagtg cata 74

<210> 123
<211> 81
<212> DNA
<213> Escherichia coli

<400> 123

gaatatagag aagtacttac ttaacatttt cccatttggt actatctaac cccttttcac 60

tattaagaag taatgcctac t 81

<210> 124
<211> 77
<212> DNA
<213> Escherichia coli

<400> 124

tagagcggca acgcgtaccc tgggtacgcg ttgtttgtct ggagtttcag tttaactagt 60

gacttgagga aaaccta 77

<210> 125
<211> 78
<212> DNA
<213> Escherichia coli

<400> 125

ggataatgaa cagcattgct tatcagagat atgcccccat atgttgaggc atatcctaac 60

gagaatctga caaccgtt 78

<210> 126
<211> 73
<212> DNA
<213> Escherichia coli

<400> 126

cacgatgaaa aaaatgtagt tttttcaagg tgaagcggtt taaattcgtt ctcaaattac 60

agtcaggacg cgt 73

<210> 127
<211> 77
<212> **DNA**
<213> Escherichia coli

<400> 127

**ataaaattta gtgctgtaca gagcgcgtta caacacggtt tactggcagc aaatacggtt 60**

**atcgcaggga tgaaaaa 77**

<210> 128
<211> 71
<212> DNA
<213> Escherichia coli

<400> 128

**aaagctataa ctgttaaaca caatacagtg aaaagtttta gactgaaggc tcactttgca 60**

**gagggaagcg t 71**

<210> 129
<211> 76
<212> DNA
<213> Escherichia coli

<400> 129

**gcaaatttta tcgtttgtca gcctgcgttg ttttttgtc caatatcatc aggttaatca 60**

**caggggaagg tgagat 76**

<210> 130
<211> 82
<212> DNA
<213> Escherichia coli

<400> 130

**tttgtttttc acgcgcttta cagcccgaaa aggccggaag atacttgccc gcaacgaaga 60**

**ttccttcata accgggtaag ca 82**

<210> 131
<211> 72
<212> DNA
<213> Escherichia coli

<400> 131

gcgcaagatt gttggttttt gcgtgatggt gaccgggcag cctaaaggct atccttaacc 60

agggagctga tt 72

<210> 132
<211> 63
<212> DNA
<213> Artificial Sequence

<220>
<223> pMB029 promoter

<400> 132

accgtgcgtg ttgactattt tacctctggc ggttataatg gttgcatgta ctaaggaggt 60

tgt 63

<210> 133
<211> 63
<212> DNA
<213> Artificial Sequence

<220>
<223> pMB023 promoter

<400> 133

accgtgcgtg ttgacaattt tacctctggc ggttataatg gttgcatgta ctaaggaggt 60

tgt 63

<210> 134
<211> 63
<212> DNA
<213> Artificial Sequence

<220>
<223> pMB025 promoter

<400> 134

accgtgcgtg ttgacaattt tacctctggc ggttagagtg gttgcatgta ctaaggaggt 60

tgt 63

<210> 135
<211> 63
<212> DNA
<213> Artificial Sequence

<220>
<223> pMB019 promoter

<400> 135

accgtgcgtg ttgacttaaa taccactggc ggttataatg gttgcatgta ctaaggaggt 60

tgt 63

<210> 136
<211> 63
<212> DNA
<213> Artificial Sequence

<220>
<223> pMB008 promoter

<400> 136

accgtgcgtg ttgacataaa taccactggc ggttataatg gttgcatgta ctaaggaggt 60

tgt 63

<210> 137
<211> 63
<212> DNA
<213> Artificial Sequence

<220>
<223> pMB020 promoter

<400> 137

accgtgcgtg ttgacataaa taccactggc ggttagagtg gttgcatgta ctaaggaggt 60

tgt 63

<210> 138
<211> 63
<212> DNA
<213> Artificial Sequence

<220>
<223> pMB022 promoter

<400> 138

accgtgcgtg ttgacataaa taccactggc ggtgataatg gttgcatgta ctaaggaggt 60

tgt 63

<210> 139
<211> 63
<212> DNA
<213> Artificial Sequence

<220>

<223> pMB089 promoter

<400> 139

```
accgtgcgtg ttgactattt tacctctggc ggttagagtg gttgcatgta ctaaggaggt        60

tgt                                                                       63
```

<210> 140
<211> 63
<212> DNA
<213> Artificial Sequence

<220>
<223> pMB001 promoter

<400> 140

```
accgtgcgtg ttgacataaa taccactggc ggttattttg gttgcatgta ctaaggaggt        60

tgt                                                                       63
```

<210> 141
<211> 63
<212> DNA
<213> Artificial Sequence

<220>
<223> pMB051 promoter

<400> **141**

```
accgtgcgtg ttgactattt tacctctggc ggttattttg gttgcatgta ctaaggaggt        60

tgt                                                                       63
```

<210> 142
<211> 63
<212> DNA
<213> Artificial Sequence

<220>
<223> pMB070 promoter

<400> 142

```
accgtgcgtg ttgacaattt tacctctggc ggtgatactg gttgcatgta ctaaggaggt        60

tgt                                                                       63
```

<210> 143
<211> 63
<212> DNA
<213> Artificial Sequence

<220>
<223> pMB074 promoter

<400> 143

accgtgcgtg tttacataaa taccactggc ggttataatg gttgcatgta ctaaggaggt 60

tgt 63

<210> 144
<211> 63
<212> DNA
<213> Artificial Sequence

<220>
<223> pMB046 promoter

<400> 144

accgtgcgtg ttgactattt tacctctggc ggtgataatg gttgcatgta ctaaggaggt 60

tgt 63

<210> 145
<211> 63
<212> DNA
<213> Artificial Sequence

<220>
<223> pMB071 promoter

<400> 145

accgtgcgtg ttgacttaaa taccactggc ggtgataatg gttgcatgta ctaaggaggt 60

tgt 63

<210> 146
<211> 63
<212> DNA
<213> Artificial Sequence

<220>
<223> pMB013 promoter

<400> 146

accgtgcgtg ttgacataaa taccactggc ggtgatactg gttgcatgta ctaaggaggt 60

tgt 63

<210> 147
<211> 63
<212> DNA

<213> Artificial Sequence

<220>
<223> pMB080 promoter

<400> 147

accgtgcgtg tagacttaaa taccactggc ggttataatg gttgcatgta ctaaggaggt 60

tgt 63

<210> 148
<211> 63
<212> DNA
<213> Artificial Sequence

<220>
<223> pMB038 promoter

<400> 148

accgtgcgtg ttgactattt tacctctggc ggtgatactg gttgcatgta ctaaggaggt 60

tgt 63

<210> 149
<211> 63
<212> DNA
<213> Artificial Sequence

<220>
<223> pMB060 promoter

<400> 149

accgtgcgtg tttacaattt tacctctggc ggttagagtg gttgcatgta ctaaggaggt 60

tgt 63

<210> 150
<211> 63
<212> DNA
<213> Artificial Sequence

<220>
<223> pMB064 promoter

<400> 150

accgtgcgtg ttgacttaaa taccactggc ggtgatactg gttgcatgta ctaaggaggt 60

tgt 63

<210> 151

<211> 63
<212> DNA
<213> Artificial Sequence

<220>
<223> pMB058 promoter

<400> 151

**accgtgcgtg tttacaattt tacctctggc ggttattttg gttgcatgta ctaaggaggt 60**

**tgt 63**

<210> 152
<211> 63
<212> DNA
<213> Artificial Sequence

<220>
<223> pMB085 promoter

<400> 152

**accgtgcgtg tttacaattt tacctctggc ggtgataatg gttgcatgta ctaaggaggt 60**

**tgt 63**

<210> 153
<211> 65
<212> DNA
<213> Artificial Sequence

<220>
<223> pMB081 promoter

<400> 153

**accgtgcgtg ttgacttaaa taccactggc ggttataatt aacatcctac aaggagaaca 60**

**aaagc 65**

<210> 154
<211> 65
<212> DNA
<213> Artificial Sequence

<220>
<223> pMB091 promoter

<400> 154

**accgtgcgtg ttgacaattt tacctctggc ggttagagtt aacatcctac aaggagaaca 60**

**aaagc 65**

<210> 155
<211> 65
<212> DNA
<213> Artificial Sequence

<220>
<223> pMB027 promoter

<400> 155

**accgtgcgtg ttgacataaa taccactggc ggttataatt aacatcctac aaggagaaca 60**

**aaagc 65**

<210> 156
<211> 65
<212> DNA
<213> Artificial Sequence

<220>
<223> pMB048 promoter

<400> 156

**accgtgcgtg ttgacataaa taccactggc ggttagagtt aacatcctac aaggagaaca 60**

**aaagc 65**

<210> 157
<211> 65
<212> DNA
<213> Artificial Sequence

<220>
<223> pMB055 promoter

<400> 157

**accgtgcgtg ttgacaattt tacctctggc ggttattttt aacatcctac aaggagaaca 60**

**aaagc 65**

<210> 158
<211> 65
<212> DNA
<213> Artificial Sequence

<220>
<223> pMB006 promoter

<400> 158

accgtgcgtg ttgactattt tacctctggc ggttataatt aacatcctac aaggagaaca 60

aaagc 65

<210> 159
<211> 63
<212> DNA
<213> Artificial Sequence

<220>
<223> pMB012 promoter

<400> 159

accgtgcgtg tttacataaa taccactggc ggtgataatg gttgcatgta ctaaggaggt 60

tgt 63

<210> 160
<211> 63
<212> DNA
<213> Artificial Sequence

<220>
<223> pMB014 promoter

<400> 160

accgtgcgtg tttacaattt tacctctggc ggtgatactg gttgcatgta ctaaggaggt 60

tgt 63

<210> 161
<211> 65
<212> DNA
<213> Artificial Sequence

<220>
<223> pMB028 promoter

<400> 161

accgtgcgtg ttgacaattt tacctctggc ggtgataatt aacatcctac aaggagaaca 60

aaagc 65

<210> 162
<211> 63
<212> DNA
<213> Artificial Sequence

<220>
<223> pMB059 promoter

<400> 162

accgtgcgtg tttacataaa taccactggc ggttattttg gttgcatgta ctaaggaggt 60

tgt 63

<210> 163
<211> 65
<212> DNA
<213> Artificial Sequence

<220>
<223> pMB061 promoter

<400> 163

accgtgcgtg ttgacaattt tacctctggc ggtgatactt aacatcctac aaggagaaca 60

aaagc 65

<210> 164
<211> 65
<212> DNA
<213> Artificial Sequence

<220>
<223> pMB043 promoter

<400> 164

accgtgcgtg ttgactattt tacctctggc ggttagagtt aacatcctac aaggagaaca 60

aaagc 65

<210> 165
<211> 63
<212> DNA
<213> Artificial Sequence

<220>
<223> pMB066 promoter

<400> 165

accgtgcgtg tagactattt tacctctggc ggttattttg gttgcatgta ctaaggaggt 60

tgt 63

<210> 166
<211> 65
<212> DNA
<213> Artificial Sequence

<220>

<223> pMB079 promoter

<400> 166

accgtgcgtg ttgacataaa taccactggc ggtgataatt aacatcctac aaggagaaca 60

aaagc 65

<210> 167
<211> 63
<212> DNA
<213> Artificial Sequence

<220>
<223> pMB032 promoter

<400> 167

accgtgcgtg tagacttaaa taccactggc ggttagagtg gttgcatgta ctaaggaggt 60

tgt 63

<210> 168
<211> 65
<212> DNA
<213> Artificial Sequence

<220>
<223> pMB068 promoter

<400> 168

accgtgcgtg tttacaattt tacctctggc ggttataatt aacatcctac aaggagaaca 60

aaagc 65

<210> 169
<211> 65
<212> DNA
<213> Artificial Sequence

<220>
<223> pMB082 promoter

<400> 169

accgtgcgtg ttgactattt tacctctggc ggttattttt aacatcctac aaggagaaca 60

aaagc 65

<210> 170
<211> 65
<212> DNA
<213> Artificial Sequence

<220>
<223> pMB030 promoter

<400> 170

```
accgtgcgtg tttacataaa taccactggc ggttataatt aacatcctac aaggagaaca        60

aaagc                                                                    65
```

<210> 171
<211> 65
<212> DNA
<213> Artificial Sequence

<220>
<223> pMB067 promoter

<400> 171

```
accgtgcgtg ttgacttaaa taccactggc ggttagagtt aacatcctac aaggagaaca        60

aaagc                                                                    65
```

<210> 172
<211> 65
<212> DNA
<213> Artificial Sequence

<220>
<223> pMB050 promoter

<400> 172

```
accgtgcgtg ttgacttaaa taccactggc ggttattttt aacatcctac aaggagaaca        60

aaagc                                                                    65
```

<210> 173
<211> 65
<212> DNA
<213> Artificial Sequence

<220>
<223> pMB069 promoter

<400> 173

```
accgtgcgtg ttgactattt tacctctggc ggtgataatt aacatcctac aaggagaaca        60

aaagc                                                                    65
```

<210> 174
<211> 63
<212> DNA

<213> Artificial Sequence

<220>
<223> pMB017 promoter

<400> 174

**accgtgcgtg taggctattt tacctctggc ggttataatg gttgcatgta ctaaggaggt 60**

**tgt 63**

<210> 175
<211> 63
<212> DNA
<213> Artificial Sequence

<220>
<223> pMB039 promoter

<400> 175

**accgtgcgtg tagacttaaa taccactggc ggttattttg gttgcatgta ctaaggaggt 60**

**tgt 63**

<210> 176
<211> 65
<212> DNA
<213> Artificial Sequence

<220>
<223> pMB011 promoter

<400> 176

**accgtgcgtg ttgacttaaa taccactggc ggtgataatt aacatcctac aaggagaaca 60**

**aaagc 65**

<210> 177
<211> 65
<212> DNA
<213> Artificial Sequence

<220>
<223> pMB072 promoter

<400> 177

**accgtgcgtg tagacttaaa taccactggc ggttataatt aacatcctac aaggagaaca 60**

**aaagc 65**

<210> 178

<211> 65
<212> DNA
<213> Artificial Sequence

<220>
<223> pMB016 promoter

<400> 178

accgtgcgtg ttgactattt tacctctggc ggtgatactt aacatcctac aaggagaaca 60

aaagc 65

<210> 179
<211> 65
<212> DNA
<213> Artificial Sequence

<220>
<223> pMB077 promoter

<400> 179

accgtgcgtg tagactattt tacctctggc ggttataatt aacatcctac aaggagaaca 60

aaagc 65

<210> 180
<211> 65
<212> DNA
<213> Artificial Sequence

<220>
<223> pMB047 promoter

<400> 180

accgtgcgtg ttgacttaaa taccactggc ggtgatactt aacatcctac aaggagaaca 60

aaagc 65

<210> 181
<211> 65
<212> DNA
<213> Artificial Sequence

<220>
<223> pMB052 promoter

<400> 181

accgtgcgtg tttacaattt tacctctggc ggttagagtt aacatcctac aaggagaaca 60

aaagc 65

<210> 182
<211> 65
<212> DNA
<213> Artificial Sequence

<220>
<223> pMB090 promoter

<400> 182

accgtgcgtg tttacaattt tacctctggc ggtgataatt aacatcctac aaggagaaca 60

aaagc 65

<210> 183
<211> 65
<212> DNA
<213> Artificial Sequence

<220>
<223> pMB035 promoter

<400> 183

accgtgcgtg tttacataaa taccactggc ggttagagtt aacatcctac aaggagaaca 60

aaagc 65

<210> 184
<211> 65
<212> DNA
<213> Artificial Sequence

<220>
<223> pMB073 promoter

<400> 184

accgtgcgtg tagactattt tacctctggc ggttagagtt aacatcctac aaggagaaca 60

aaagc 65

<210> 185
<211> 65
<212> DNA
<213> Artificial Sequence

<220>
<223> pMB004 promoter

<400> 185

accgtgcgtg tttacaattt tacctctggc ggtgatactt aacatcctac aaggagaaca 60

aaagc 65

<210> 186
<211> 65
<212> DNA
<213> Artificial Sequence

<220>
<223> pMB054 promoter

<400> 186

accgtgcgtg tagacttaaa taccactggc ggttagagtt aacatcctac aaggagaaca 60

aaagc 65

<210> 187
<211> 65
<212> DNA
<213> Artificial Sequence

<220>
<223> pMB024 promoter

<400> 187

accgtgcgtg tagactattt tacctctggc ggtgataatt aacatcctac aaggagaaca 60

aaagc 65

<210> 188
<211> 63
<212> DNA
<213> Artificial Sequence

<220>
<223> pMB007 promoter

<400> 188

accgtgcgtg taggctattt tacctctggc ggtgatactg gttgcatgta ctaaggaggt 60

tgt 63

<210> 189
<211> 65
<212> DNA
<213> Artificial Sequence

<220>
<223> pMB005 promoter

<400> 189

accgtgcgtg taggctattt tacctctggc ggttataatt aacatcctac aaggagaaca 60

aaagc 65

<210> 190
<211> 63
<212> DNA
<213> Artificial Sequence

<220>
<223> pMB003 promoter

<400> 190

accgtgcgtg taggctattt tacctctggc ggtgataatg gttgcatgta ctaaggaggt 60

tgt 63

<210> 191
<211> 65
<212> DNA
<213> Artificial Sequence

<220>
<223> pMB088 promoter

<400> 191

accgtgcgtg tttacataaa taccactggc ggtgataatt aacatcctac aaggagaaca 60

aaagc 65

<210> 192
<211> 65
<212> DNA
<213> Artificial Sequence

<220>
<223> pMB065 promoter

<400> 192

accgtgcgtg tagacttaaa taccactggc ggttattttt aacatcctac aaggagaaca 60

aaagc 65

<210> 193
<211> 65
<212> DNA
<213> Artificial Sequence

<220>

<223> pMB037 promoter

<400> 193

accgtgcgtg tagacttaaa taccactggc ggtgataatt aacatcctac aaggagaaca 60

aaagc 65

<210> 194
<211> 65
<212> DNA
<213> Artificial Sequence

<220>
<223> pMB009 promoter

<400> 194

accgtgcgtg tagacttaaa taccactggc ggtgatactt aacatcctac aaggagaaca 60

aaagc 65

<210> 195
<211> 65
<212> DNA
<213> Artificial Sequence

<220>
<223> pMB041 promoter

<400> 195

accgtgcgtg taggctattt tacctctggc ggttattttt aacatcctac aaggagaaca 60

aaagc 65

<210> 196
<211> 65
<212> DNA
<213> Artificial Sequence

<220>
<223> pMB036 promoter

<400> 196

accgtgcgtg taggctattt tacctctggc ggtgataatt aacatcctac aaggagaaca 60

aaagc 65

<210> 197
<211> 65
<212> DNA
<213> Artificial Sequence

<220>
<223> pMB049 promoter

<400> 197

accgtgcgtg taggcttaaa taccactggc ggttataatt aacatcctac aaggagaaca 60

aaagc 65

<210> 198
<211> 65
<212> DNA
<213> Artificial Sequence

<220>
<223> pMB044 promoter

<400> 198

accgtgcgtg tttacataaa taccactggc ggtgatactt aacatcctac aaggagaaca 60

aaagc 65

<210> 199
<211> 63
<212> DNA
<213> Artificial Sequence

<220>
<223> pMB042 promoter

<400> 199

accgtgcgtg taggcttaaa taccactggc ggtgataatg gttgcatgta ctaaggaggt 60

tgt 63

<210> 200
<211> 63
<212> DNA
<213> Artificial Sequence

<220>
<223> pMB086 promoter

<400> 200

accgtgcgtg taggcttaaa taccactggc ggttagagtg gttgcatgta ctaaggaggt 60

tgt 63

<210> 201
<211> 63
<212> DNA
<213> Artificial Sequence

<220>
<223> pMB053 promoter

<400> 201

```
accgtgcgtg taggcttaaa taccactggc ggttattttg gttgcatgta ctaaggaggt        60

tgt                                                                      63
```

<210> 202
<211> 65
<212> DNA
<213> Artificial Sequence

<220>
<223> pMB057 promoter

<400> 202

```
accgtgcgtg taggctattt tacctctggc ggtgatactt aacatcctac aaggagaaca        60

aaagc                                                                    65
```

<210> 203
<211> 65
<212> DNA
<213> Artificial Sequence

<220>
<223> pMB018 promoter

<400> 203

```
accgtgcgtg taggcttaaa taccactggc ggttattttt aacatcctac aaggagaaca        60

aaagc                                                                    65
```

<210> 204
<211> 65
<212> DNA
<213> Artificial Sequence

<220>
<223> pMB002 promoter

<400> 204

```
accgtgcgtg taggcttaaa taccactggc ggttagagtt aacatcctac aaggagaaca        60

aaagc                                                                    65
```

<210> 205
<211> 65
<212> DNA

<213> Artificial Sequence

<220>
<223> pMB015 promoter

<400> 205

accgtgcgtg taggcttaaa taccactggc ggtgataatt aacatcctac aaggagaaca 60

aaagc 65

<210> 206
<211> 65
<212> DNA
<213> Artificial Sequence

<220>
<223> pMB087 promoter

<400> 206

accgtgcgtg taggcttaaa taccactggc ggtgatactt aacatcctac aaggagaaca 60

aaagc 65

<210> 207
<211> 63
<212> DNA
<213> Artificial Sequence

<220>
<223> pMB063 promoter

<400> 207

accgtgcgtg taggcttaaa taccactggc ggtgatactg gttgcatgta ctaaggaggt 60

tgt 63

<210> 208
<211> 10
<212> DNA
<213> Lambda phage

<400> 208
accgtgcgtg 10

<210> 209
<211> 17
<212> DNA
<213> Lambda phage

<400> 209
attttacctc tggcggt 17

<210> 210
<211> 17
<212> DNA
<213> Lambda phage

<400> 210
taaataccac tggcggt 17

<210> 211
<211> 24
<212> DNA
<213> Lambda phage

<400> 211
ggttgcatgt actaaggagg ttgt 24

<210> 212
<211> 26
<212> DNA
<213> Escherichia coli

<400> 212
taacatccta caaggagaac aaaagc 26

<210> 213
<211> 6357
<212> DNA
<213> Artificial Sequence

<220>
<223> Ori_Plsmd27 vector

<400> 213

```
actacctagc tgcattttca ggaggaagcg atgggcggcc gcacaccttc ttaataagat      60
gatcttcttg agatcgtttt ggtctgcgcg taatctcttg ctctgaaaac gaaaaaaccg     120
ccttgcaggg cggtttttcg aaggttctct gagctaccaa ctctttgaac cgaggtaact     180
ggcttggagg agcgcagtca ccaaaacttg tcctttcagt ttagccttaa ccggcgcatg     240
acttcaagac taactcctct aaatcaatta ccagtggctg ctgccagtgg tgcttttgca     300
tgtctttccg ggttggactc aagacgatag ttaccggata aggcgcagcg gtcggactga     360
acggggggtt cgtgcataca gtccagcttg gagcgaactg cctacccgga actgagtgtc     420
aggcgtggaa tgagacaaac gcggccataa cagcggaatg acaccggtaa accgaaaggc     480
aggaacagga gagcgcacga gggagccgcc aggggaaacg cctggtatct ttatagtcct     540
gtcgggtttc gccaccactg atttgagcgt cagatttcgt gatgcttgtc aggggggcgg     600
agcctatgga aaaacggctt tgccgcggcc ctctcacttc cctgttaagt atcttcctgg     660
catcttccag gaaatctccg ccccgttcgt aagccatttc cgctcgccgc agtcgaacga     720
ccgagcgtag cgagtcagtg agcgaggaag cggaatatat cctgtatcac atattctgct     780
gacgcaccgg tgcagccttt tttctcctgc cacatgaagc acttcactga caccctcatc     840
agtgccaaca tagtaagcca gtatacactc cgctatgata atgggtgagt gagtgtgtgc     900
gtgtggggcg cgccagatgg gaacaaactt aatcgccttg cagcacatcc ccctttcgcc     960
agctggcgta atagcgaaga ggcccgcacc gatcgccctt cccaacagtt gcgcagcctg    1020
aatggcgaat ggcgataagc tagcttcacg ctgccgcaag cactcagggc gcaagggctg    1080
ctaaaggaag cggaacacgt agaaagccag tccgcagaaa cggtgctgac cccggatgaa    1140
tgtcagctac tgggctatct ggacaaggga aaacgcaagc gcaaagagaa agcaggtagc    1200
ttgcagtggg cttacatggc gatagctaga ctgggcggtt ttatggacag caagcgaacc    1260
ggaattgcca gctggggcgc cctctggtaa ggttgggaag ccctgcaaag taaactggat    1320
ggctttcttg ccgccaagga tctgatggcg caggggatca agatctgatc aagagacagg    1380
atgaggatcg tttcgcatga ttgaacaaga tggattgcac gcaggttctc cggccgcttg    1440
ggtggagagg ctattcggct atgactgggc acaacagaca atcggctgct ctgatgccgc    1500
cgtgttccgg ctgtcagcgc aggggcgccc ggttcttttt gtcaagaccg acctgtccgg    1560
tgccctgaat gaactccaag acgaggcagc gcggctatcg tggctggcca cgacgggcgt    1620
tccttgcgca gctgtgctcg acgttgtcac tgaagcggga agggactggc tgctattggg    1680
cgaagtgccg gggcaggatc tcctgtcatc tcaccttgct cctgccgaga aagtatccat    1740
catggctgat gcaatgcggc ggctgcatac gcttgatccg gctacctgcc cattcgacca    1800
```

ccaagcgaaa catcgcatcg agcgagcacg tactcggatg gaagccggtc ttgtcgatca 1860
ggatgatctg gacgaagagc atcaggggct cgcgccagcc gaactgttcg ccaggctcaa 1920
ggcgcggatg cccgacggcg aggatctcgt cgtgacccat ggcgatgcct gcttgccgaa 1980
tatcatggtg gaaaatggcc gcttttctgg attcatcgac tgtggccggc tgggtgtggc 2040
ggaccgctat caggacatag cgttggctac ccgtgatatt gctgaagagc ttggcggcga 2100
atgggctgac cgcttcctcg tgctttacgg tatcgccgct cccgattcgc agcgcatcgc 2160
cttctatcgc cttcttgacg agttcttctg agcgggactc tggggttcgc tagaggatcg 2220
atcctttta acccatcaca tatacctgcc gttcactatt atttagtgaa atgagatatt 2280
atgatatttt ctgaattgtg attaaaaagg caactttatg cccatgcaac agaaactata 2340
aaaaatacag agaatgaaaa gaaacagata gatttttag ttctttaggc ccgtagtctg 2400
caaatccttt tatgattttc tatcaaacaa aagaggaaaa tagaccagtt gcaatccaaa 2460
cgagagtcta atagaatgag gtcgaaaagt aaatcgcgcg ggtttgttac tgataaagca 2520
ggcaagacct aaaatgtgta aagggcaaag tgtatacttt ggcgtcaccc cttacatatt 2580
ttaggtcttt ttttattgtg cgtaactaac ttgccatctt caaacaggag ggctggaaga 2640
agcagaccgc taacacagta cataaaaaag gagacatgaa cgactccagt ctttctagaa 2700
gatggcaaac agctattatg ggtattatgg gtgctccccg aaaagtgcca cctgggtcct 2760
tttcatcacg tgctataaaa ataattataa tttaaatttt ttaatataaa tatataaatt 2820
aaaaatagaa agtaaaaaaa gaaattaaag aaaaaatagt ttttgttttc cgaagatgta 2880
aaagactcta gggggatcgc caacaaatac taccttttat cttgctcttc ctgctctcag 2940
gtattaatgc cgaattgttt catcttgtct gtgtagaaga ccacacacga aaatcctgtg 3000
attttacatt ttacttatcg ttaatcgaat gtatatctat ttaatctgct tttcttgtct 3060
aataaatata tatgtaaagt acgctttttg ttgaaatttt ttaaaccttt gtttattttt 3120
ttttcttcat tccgtaactc ttctaccttc tttatttact ttctaaaatc caaatacaaa 3180
acataaaaat aaataaacac agagtaaatt cccaaattat tccatcatta aaagatacga 3240
ggcgcgtgta agttacaggc aagcgatccg tcctaagaaa ccattattat catgacatta 3300
acctataaaa ataggcgtat cacgaggccc tttcgtctcg cgcgtttcgg tgatgacggt 3360
gaaaacctct gacacatgca gctcccggag acggtcacag cttgtctgta agcggatgcc 3420
gggagcagac aagcccgtca gggcgcgtca gcgggtgttg gcgggtgtcg gggctggctt 3480
aactatgcgg catcagagca gattgtactg agagtgcacc ataccacttt ttcgtgacgc 3540
gcggttttga aaacatagac aagtttttgg cgtcgttgtt aatttcgaag aggatgtcca 3600
atattttttt taaggaataa ggatacttca agactagatt ccccctgca ttcccatcag 3660

aaccgtaaac cttggcgctt tccttgggaa gtattcaaga agtgccttgt ccggtttctg 3720
tggctcacaa accagcgcgc ccgatatggc tttcttttca cttatgaatg taccagtacg 3780
ggacaattag aacgctcctg taacaatctc tttgcaaatg tggggttaca ttctaaccat 3840
gtcacactgc tgacgaaatt caaagtaaaa aaaaatggga ccacgtcttg agaacgatag 3900
attttcttta ttttacattg aacagtcgtt gtctcagcgc gctttatgtt ttcattcata 3960
cttcatatta taaaataaca aaagaagaat ttcatattca cgcccaagaa atcaggctgc 4020
tttccaaatg caattgacac ttcattagcc atcacacaaa actctttctt gctggagctt 4080
cttttaaaaa agacctcagt acaccaaaca cgttacccga cctcgttatt ttacgacaac 4140
tatgataaaa ttctgaagaa aaaataaaaa aattttcata cttcttgctt ttatttaaac 4200
cattgaatga tttcttttga acaaaactac ctgtttcacc aaaggaaata gaaagaaaaa 4260
atcaattaga agaaaacaaa aaacaaaatg tctgttatta atttcacagg tagttctggt 4320
ccattggtga aagtttgcgg cttgcagagc acagaggccg cagaatgtgc tctagattcc 4380
gatgctgact tgctgggtat tatatgtgtg cccaatagaa agagaacaat tgacccggtt 4440
attgcaagga aaatttcaag tcttgtaaaa gcatataaaa atagttcagg cactccgaaa 4500
tacttggttg gcgtgtttcg taatcaacct aaggaggatg ttttggctct ggtcaatgat 4560
tacggcattg atatcgtcca actgcatgga gatgagtcgt ggcaagaata ccaagagttc 4620
ctcggtttgc cagttattaa aagactcgta tttccaaaag actgcaacat actactcagt 4680
gcagcttcac agaaacctca ttcgtttatt cccttgtttg attcagaagc aggtgggaca 4740
ggtgaacttt tggattggaa ctcgatttct gactgggttg gaaggcaaga gagccccgaa 4800
agcttacatt ttatgttagc tggtggactg acgccagaaa atgttggtga tgcgcttaga 4860
ttaaatggcg ttattggtgt tgatgtaagc ggaggtgtgg agacaaatgg tgtaaaagac 4920
tctaacaaaa tagcaaattt cgtcaaaaat gctaagaaat aggttattac tgagtagtat 4980
ttatttaagt attgtttgtg cacttgcctg caggcctttt gaaaagcaag cataaaagat 5040
ctaaacataa aatctgtaaa ataacaagat gtaaagataa tgctaaatca tttggctttt 5100
tgattgattg tacaggactg ggtggaatcc cttctgcagc acctggatta ccctgttatc 5160
cctagtggcc aaaggcacgg ttcgctcgca ccggaagggt tcttcagggg aaccccctga 5220
aaccccggaa acatctgact tggttacgat ggactttgaa cacgccgagg aatgaacccc 5280
gaccgcgcac agtcatatga gaaagtcagc tgcatgagac catccacccg ccctgaggga 5340
cgctttgagc agctctggct accgctatgg ccactagcaa gcgacaacct ccgtgacggc 5400
atctaccgca cctcacgcga taaagcccta gacaagcgct atatcgaaac caacccggat 5460
gccatctcga atctcctggt cgtcgacatc gacaagcaag acgcactctt gcgttcgatg 5520
tgggacagag acggctggcg gcctaacgcc gtggttgaaa acccttaaac gggcacgcac 5580

acgccgtgtg ggcgctcgcg gagccattta cccgcaccga gtacgccagg cgcaagccac 5640
ctgctacgcg gccgctgtca ccgaaggact acgccgctct gtcgacggcg acaaaggcta 5700
ctccgggctg atcaccaaaa accccgagca cacagcctgg gacagccact gggtaaccga 5760
taagctctac acactcgatg aactgcgctt ttggctggaa gaaaccggct tcatgccgcc 5820
agagtcctgg aagaaaaccc gcagaaaatc gcccatcggg ctaggccgaa actgcgcgct 5880
cttcgagagc gcacgcagct gggcttatcg cgaaatacgc caccacttcg gcgaccccga 5940
cgggctagga cgctctatcc aagccaccgc ccaggcgctc aaccaggagc tgttttctga 6000
gccgctaccc gtggcagaag ttgaccaaat agccaggtca atccaccgat ggatcatcac 6060
caaatcacgc atgtggacag acggcccagc cgtctacgaa gccacattca ccacaatcca 6120
agccacacgc ggaaaacgaa gcgccgagca ccgctggggc accaccaacg cggaacgaat 6180
cgaaagattt atcaatgact aagcgcactc gtattccccg aaacggtaaa actatccgcg 6240
aagtagccga aggaactggc ctatcaactg cgacaatcga acgctggaca tctgcccctc 6300
gtaatgagta gtcctcatct ccctcaagca ggcgccggcg gtactgccat cctcgag 6357

<210> 214
<211> 6697
<212> DNA
<213> Artificial Sequence

<220>
<223> vector backbone 1

<400> 214

```
ctggggcgcc ctctggtaag gttgggaagc cctgcaaagt aaactggatg gctttcttgc      60
cgccaaggat ctgatggcgc aggggatcaa gatctgatca agagacagga tgaggatcgt     120
ttcgcatgat tgaacaagat ggattgcacg caggttctcc ggccgcttgg gtggagaggc     180
tattcggcta tgactgggca caacagacaa tcggctgctc tgatgccgcc gtgttccggc     240
tgtcagcgca ggggcgcccg gttctttttg tcaagaccga cctgtccggt gccctgaatg     300
aactccaaga cgaggcagcg cggctatcgt ggctggccac gacgggcgtt ccttgcgcag     360
ctgtgctcga cgttgtcact gaagcgggaa gggactggct gctattgggc gaagtgccgg     420
ggcaggatct cctgtcatct caccttgctc ctgccgagaa agtatccatc atggctgatg     480
caatgcggcg gctgcatacg cttgatccgg ctacctgccc attcgaccac caagcgaaac     540
atcgcatcga gcgagcacgt actcggatgg aagccggtct tgtcgatcag gatgatctgg     600
acgaagagca tcaggggctc gcgccagccg aactgttcgc caggctcaag gcgcggatgc     660
ccgacggcga ggatctcgtc gtgacccatg gcgatgcctg cttgccgaat atcatggtgg     720
aaaatggccg cttttctgga ttcatcgact gtggccggct gggtgtggcg gaccgctatc     780
```

aggacatagc gttggctacc cgtgatattg ctgaagagct tggcggcgaa tgggctgacc 840
gcttcctcgt gctttacggt atcgccgctc ccgattcgca gcgcatcgcc ttctatcgcc 900
ttcttgacga gttcttctga gcgggactct ggggttcgct agaggatcga tcctttttaa 960
cccatcacat atacctgccg ttcactatta tttagtgaaa tgagatatta tgatattttc 1020
tgaattgtga ttaaaaaggc aactttatgc ccatgcaaca gaaactataa aaaatacaga 1080
gaatgaaaag aaacagatag attttttagt tctttaggcc cgtagtctgc aaatcctttt 1140
atgattttct atcaaacaaa agaggaaaat agaccagttg caatccaaac gagagtctaa 1200
tagaatgagg tcgaaaagta aatcgcgcgg gtttgttact gataaagcag gcaagaccta 1260
aaatgtgtaa agggcaaagt gtatactttg gcgtcacccc ttacatattt taggtctttt 1320
tttattgtgc gtaactaact tgccatcttc aaacaggagg gctggaagaa gcagaccgct 1380
aacacagtac ataaaaaagg agacatgaac gtgagctgtt tacaattaat catcgtgtgg 1440
taccatgtgt ggaattggaa aggacatgaa catcaaaaag tttgcaaaac aagcaacagt 1500
attaaccttt actaccgcac tgctggcagg aggcgcaact caagcgtttg cgaaagaaac 1560
gaaccaaaag ccatataagg aaacatacgg catttcccat attacacgcc atgatatgct 1620
gcaaatccct gaacagcaaa aaaatgaaaa atatcaagtt tctgaatttg attcgtccac 1680
aattaaaaat atctcttctg caaaaggcct ggacgtttgg gacagctggc cattacaaaa 1740
cgctgacggc actgtcgcaa actatcacgg ctaccacatc gtctttgcat tagccggaga 1800
tcctaaaaat gcggatgaca catcgattta catgttctat caaaaagtcg gcgaaacttc 1860
tattgacagc tggaaaaacg ctggccgcgt ctttaaagac agcgacaaat tcgatgcaaa 1920
tgattctatc ctaaaagacc aaacacaaga atggtcaggt tcagccacat ttacatctga 1980
cggaaaaatc cgtttattct acactgattt ctccggtaaa cattacggca aacaaacact 2040
gacaactgca caagttaacg tatcagcatc agacagctct ttgaacatca acggtgtaga 2100
ggattataaa tcaatctttg acggtgacgg aaaaacgtat caaaatgtac agcagttcat 2160
cgatgaaggc aactacagct caggcgacaa ccatacgctg agagatcctc actacgtaga 2220
agataaaggc cacaaatact tagtatttga agcaaacact ggaactgaag atggctacca 2280
aggcgaagaa tctttattta acaaagcata ctatggcaaa agcacatcat tcttccgtca 2340
agaaagtcaa aaacttctgc aaagcgataa aaaacgcacg gctgagttag caaacggcgc 2400
tctcggtatg attgagctaa acgatgatta cacactgaaa aaagtgatga aaccgctgat 2460
tgcatctaac acagtaacag atgaaattga acgcgcgaac gtctttaaaa tgaacggcaa 2520
atggtacctg ttcactgact cccgcggatc aaaaatgacg attgacggca ttacgtctaa 2580
cgatatttac atgcttggtt atgtttctaa ttctttaact ggcccataca agccgctgaa 2640
caaaactggc cttgtgttaa aaatggatct tgatcctaac gatgtaacct ttacttactc 2700

acacttcgct gtacctcaag cgaaaggaaa caatgtcgtg attacaagct atatgacaaa 2760
cagaggattc tacgcagaca aacaatcaac gtttgcgccg agcttcctgc tgaacatcaa 2820
aggcaagaaa acatctgttg tcaaagacag catccttgaa caaggacaat taacagttaa 2880
caaataaaaa cgcaaaagaa aatgccgatg ggtaccgagc gaaatgaccg accaagcgac 2940
gcccaacctg ccatcacgag atttcgattc caccgccgcc ttctatgaaa ggttgggctt 3000
cggaatcgtt ttccgggacg ccctcgcgga cgtgctcata gtccaataac tacattgagc 3060
gaaatgccaa ccacatgtcc catgctttta ctaatgtggg gtcttagaag aaagcgacca 3120
atttaaggag agttgaatat gtctgaaatc cagctgacgg aggcatcatt gaacgaagca 3180
gccgatgctg caattaaagc gttcgatgga gcacaaaacc tcgatgaatt ggctgctctg 3240
cgacgagatc acctgggtga tgcggcacca atccctcagg cacgccgctc gcttggaacc 3300
attccaaaag atcagcgtaa ggatgccgga cgattcgtaa acatggcgct gggccgcgcg 3360
gaaaagcact tcgcccaggt taaggtggtt cttgaagaaa agcgaaacgc agaagtcctg 3420
gagctggagc gcgtggatgt taccgtccct accacacgtg aacaagtcgg cgcactgcac 3480
ccaattacga ttctcaacga acagatcgcg gacatctttg ttggcatggg ctgggagatc 3540
gcagagggcc cggaagttga agccgaatac ttcaatttcg atgcacttaa ctttctccca 3600
gaccacccag cccgcaccct gcaggatacc ttccacatcg cacctgaagg atcgcgccaa 3660
gtgttgcgca cccatacctc tcctgtccag gttcgcacga tgctgaatcg agaggtacct 3720
atctatatcg cctgtcctgg tcgcgtcttc cgcactgacg aattggatgc tacccacacc 3780
cctgtctttc accagatcga gggcctggct gtcgacaaag gcctgacaat ggctcacctt 3840
cgcggaactc tggatcactt ggctaaagaa ctgttcggac ctgagactaa aacccgcatg 3900
cgttcaaact acttcccatt ttctgagccc agcgcggaag ttgatgtctg gttcccaaat 3960
aagaagggcg gtgccggctg gatcgaatgg ggcggctgcg gcatggtcaa cccaaacgtg 4020
ctccgcgctg taggcgtcga cccggaagaa tacactggat tcggcttcgg tatgggtatt 4080
gaacgcacct tgcaattccg aaatggactc tcagatatgc gcgatatggt agaaggcgac 4140
attcgcttta ccctcccttt cggcattcag gcttaggcat ttttagtacg tgcaataacc 4200
actctggttt ttccagggtg gtttttttgat gccctttttg gagtcttcaa ctgagcctcg 4260
cagagcagga ttcccgttga gcaccgccag gtgcgaataa gggacagtga agaaggaaca 4320
cccgctcgcg ggtgggccta cttcacctat cctgcccggc tgacgccgtt ggatacacca 4380
aggaaagtct acacgaaccc tttggcaaaa tcctgtatat cgtgcgaaaa aggatggata 4440
taccgaaaaa atcgctataa tgaccccgaa gcagggttat gcagcggaaa agctccccga 4500
aaagtgccac ctgggtcctt ttcatcacgt gctataaaaa taattataat ttaaattttt 4560

taatataaat atataaatta aaaatagaaa gtaaaaaaag aaattaaaga aaaaatagtt 4620
tttgttttcc gaagatgtaa aagactctag ggggatcgcc aacaaatact accttttatc 4680
ttgctcttcc tgctctcagg tattaatgcc gaattgtttc atcttgtctg tgtagaagac 4740
cacacacgaa aatcctgtga ttttacattt tacttatcgt taatcgaatg tatatctatt 4800
taatctgctt ttcttgtcta ataaatatat atgtaaagta cgctttttgt tgaaattttt 4860
taaacctttg tttatttttt ttttcttcat tccgtaactc ttctaccttc tttatttact 4920
ttctaaaatc caaatacaaa acataaaaat aaataaacac agagtaaatt cccaaattat 4980
tccatcatta aaagatacga ggcgcgtgta agttacaggc aagcgatccg tctaagaaac 5040
cattattatc atgacattaa cctataaaaa taggcgtatc acgaggccct ttcgtctcgc 5100
gcgtttcggt gatgacggtg aaaacctctg acacatgcag ctcccggaga cggtcacagc 5160
ttgtctgtaa gcggatgccg ggagcagaca agcccgtcag ggcgcgtcag cgggtgttgg 5220
cgggtgtcgg ggctggctta actatgcggc atcagagcag attgtactga gagtgcacca 5280
taccaccttt tcaattcatc attttttttt tattcttttt tttgatttcg gtttctttga 5340
catttttttg attcggtaat ctccgaacag aaggaagaac gaaggaagga gcacagactt 5400
agattggtat atatacgcat atgtagtgtt gaagaaacat gaaattgccc agtattctta 5460
acccaactgc acagaacaaa aacctgcagg aaacgaagat aaatcatgtc gaaagctaca 5520
tataaggaac gtgctgctac tcatcctagt cctgttgctg ccaagctatt taatatcatg 5580
cacgaaaagc aaacaaactt gtgtgcttca ttggatgttc gtaccaccaa ggaattactg 5640
gagttagttg aagcattagg tcccaaaatt tgtttactaa aaacacatgt ggatatcttg 5700
actgattttt ccatggaggg cacagttaag ccgctaaagg cattatccgc caagtacaat 5760
tttttactct tcgaagacag aaaatttgct gacattggta atacagtcaa attgcagtac 5820
tctgcgggtg tatacagaat agcagaatgg gcagacatta cgaatgcaca cggtgtggtg 5880
ggcccaggta ttgttagcgg tttgaagcag gcggcagaag aagtaacaaa ggaacctaga 5940
ggccttttga tgttagcaga attgtcatgc aagggctccc tatctactgg agaatatact 6000
aagggtactg ttgacattgc gaagagcgac aaagattttg ttatcggctt tattgctcaa 6060
agagacatgg gtggaagaga tgaaggttac gattggttga ttatgacacc cggtgtgggt 6120
ttagatgaca agggagacgc attgggtcaa cagtatagaa ccgtggatga tgtggtctct 6180
acaggatctg acattattat tgttggaaga ggactatttg caaagggaag ggatgctaag 6240
gtagagggtg aacgttacag aaaagcaggc tgggaagcat atttgagaag atgcggccag 6300
caaaactaaa aaactgtatt ataagtaaat gcatgtatac taaactcaca aattagagct 6360
tcaatttaat tatatcagtt attacccccc tggcttgttg tccacaaccg ttaaacctta 6420
aaagctttaa aagccttata tattcttttt tttcttataa aacttaaaac cttagaggct 6480

```
atttaagttg ctgatttata ttaattttat tgttcaaaca tgagagctta gtacgtgaaa      6540
catgagagct tagtacgtta gccatgaggg tttagttcgt tagccatgag ggtttagttc      6600
gttaaacatg agagcttagt acgttaaaca tgagagctta gtacgtgaaa catgagagct      6660
tagtacgtac tatcaacagg ttgaactgct gatcttc                               6697
```

<210> 215
<211> 7810
<212> DNA
<213> Artificial Sequence

<220>
<223> vector backbone 2

<400> 215

gtttaaacaa ttttgtgtcg cccttaattg tgagcgctca caattccaca acggtttccc 60
tctagaaata attttgttta acttttcgag accttaggag gtaaacatat ggcgagcttg 120
gttaagaaag atatgtgtat taagatgacg atggaaggta ctgtgaacgg ttatcacttt 180
aagtgcgttg gcgagggtga aggcaagccg ttcgagggca cgcagaacat gcgcactcgt 240
gtcaccgagg gcggtccgct gccttttgca ttcgacatcc tggccccgtg ctgtatgtac 300
ggctctaaga ccttcattaa acacgtgagc ggtatcccgg attactttaa agagtccttt 360
ccagagggct tcacttggga acgtacccag atttttgagg acggtggtgt tctgaccgcg 420
caccaagaca ccagcctgga aggtaattgc ctgatctata aagtgaaggt tctgggtacc 480
aatttcccgg cgaatggtcc ggtgatgcaa aagaaaaccg cgggttggga gccgtgcgtc 540
gagatgctgt atccgcgtga cggcgtcttg tgtggtcaga gcttgatggc gctgaagtgc 600
accgatggca atcatctgac cagccacctg cgcacgacgt atcgtagccg taaaccgagc 660
aacgccgtta acatgccgga gttccatttt ggtgaccatc gcatcgaaat cctgaaagct 720
gagcagggca aattctacga acaatacgaa tcggctgtcg cacgttacag cgatgtgccg 780
gaaaaagcga cgtaatgaga attctgtaca ctcgagggtc tcaccccaag ggcgacaccc 840
cctaattagc ccgggcgaaa ggcccagtct ttcgactgag cctttcgttt tatttgagtt 900
taaacctggg gcgccctctg gtaaggttgg gaagccctgc aaagtaaact ggatgccagg 960
catcaaataa aacgaaaggc tcagtcgaaa gactgggcct ttcgttttat ctgttgtttg 1020
tcggtgaacg ctctctacta gagtcacact ggctcacctt cgggtgggcc tttctgcgtt 1080
tatagcacta ggacttgccg cggatactgc cccattacat gaattgcagc ctcagggacg 1140
tcagtagatc atggaggtag ggcatatgtc ctctgttgtt aaaatgtgag ttctcaacga 1200
agcacgaatc ggtcagaacc tacactaagg agatttggta ggtgcacggt ttctgtcgca 1260
tagaccagtt catttcagat gtctggcacg taagaggttc caactttcac cataatgaaa 1320

taagatcact accgggcgta ttttttgagt tatcgagatt ttcaggagct aaggaagcta 1380
aaatgattga acaagatgga ttgcacgcag gttctccggc cgcttgggtg gagaggctat 1440
tcggctatga ctgggcacaa cagacaatcg gctgctctga tgccgccgtg ttccggctgt 1500
cagcgcaggg gcgcccggtt ctttttgtca agaccgacct gtccggtgcc ctgaatgaac 1560
tccaagacga ggcagcgcgg ctatcgtggc tggccacgac gggcgttcct tgcgcagctg 1620
tgctcgacgt tgtcactgaa gcgggaaggg actggctgct attgggcgaa gtgccggggc 1680
aggatctcct gtcatctcac cttgctcctg ccgagaaagt atccatcatg gctgatgcaa 1740
tgcggcggct gcatacgctt gatccggcta cctgcccatt cgaccaccaa gcgaaacatc 1800
gcatcgagcg agcacgtact cggatggaag ccggtcttgt cgatcaggat gatctggacg 1860
aagagcatca ggggctcgcg ccagccgaac tgttcgccag gctcaaggcg cggatgcccg 1920
acggcgagga tctcgtcgtg acccatggcg atgcctgctt gccgaatatc atggtggaaa 1980
atggccgctt ttctggattc atcgactgtg gccggctggg tgtggcggac cgctatcagg 2040
acatagcgtt ggctacccgt gatattgctg aagagcttgg cggcgaatgg gctgaccgct 2100
tcctcgtgct ttacggtatc gccgctcccg attcgcagcg catcgccttc tatcgccttc 2160
ttgacgagtt cttctgatga tgaatggcaa ggcggcgcgt agcccccaa ccgaagttga 2220
ggggattttt ttgacaatta atcatccggc tcgtaattta tgtggatctt aatcatgcta 2280
aggaggtttt ctaatgatga acatcaaaaa gtttgcaaaa caagcaacag tattaacctt 2340
tactaccgca ctgctggcag gaggcgcaac tcaagcgttt gcgaaagaaa cgaaccaaaa 2400
gccatataag gaaacatacg gcatttccca tattacacgc catgatatgc tgcaaatccc 2460
tgaacagcaa aaaaatgaaa aatatcaagt ttctgaattt gattcgtcca caattaaaaa 2520
tatctcttct gcaaaaggcc tggacgtttg ggacagctgg ccattacaaa acgctgacgg 2580
cactgtcgca aactatcacg gctaccacat cgtctttgca ttagccggag atcctaaaaa 2640
tgcggatgac acatcgattt acatgttcta tcaaaaagtc ggcgaaactt ctattgacag 2700
ctggaaaaac gctggccgcg tctttaaaga cagcgacaaa ttcgatgcaa atgattctat 2760
cctaaaagac caaacacaag aatggtcagg ttcagccaca tttacatctg acggaaaaat 2820
ccgtttattc tacactgatt tctccggtaa acattacggc aaacaaacac tgacaactgc 2880
acaagttaac gtatcagcat cagacagctc tttgaacatc aacggtgtag aggattataa 2940
atcaatcttt gacggtgacg gaaaaacgta tcaaaatgta cagcagttca tcgatgaagg 3000
caactacagc tcaggcgaca accatacgct gagagatcct cactacgtag aagataaagg 3060
ccacaaatac ttagtatttg aagcaaacac tggaactgaa gatggctacc aaggcgaaga 3120
atctttattt aacaaagcat actatggcaa aagcacatca ttcttccgtc aagaaagtca 3180
aaaacttctg caaagcgata aaaaacgcac ggctgagtta gcaaacggcg ctctcggtat 3240

gattgagcta aacgatgatt acacactgaa aaaagtgatg aaaccgctga ttgcatctaa 3300
cacagtaaca gatgaaattg aacgcgcgaa cgtctttaaa atgaacggca aatggtacct 3360
gttcactgac tcccgcggat caaaaatgac gattgacggc attacgtcta acgatattta 3420
catgcttggt tatgtttcta attctttaac tggcccatac aagccgctga acaaaactgg 3480
ccttgtgtta aaaatggatc ttgatcctaa cgatgtaacc tttacttact cacacttcgc 3540
tgtacctcaa gcgaaaggaa acaatgtcgt gattacaagc tatatgacaa acagaggatt 3600
ctacgcagac aaacaatcaa cgtttgcgcc gagcttcctg ctgaacatca aaggcaagaa 3660
aacatctgtt gtcaaagaca gcatccttga acaaggacaa ttaacagtta acaaataaaa 3720
acgcaaaaga aaatgccgat gggtaccgag cgaaatgacc gaccaagcga cgcccaacct 3780
gccatcacga gatttcgatt ccaccgccgc cttctatgaa aggttgggct tcggaatcgt 3840
tttccgggac gccctcgcgg acgtgctcat agtccaggcg gtgttgacat aaataccact 3900
ggcggtgata ctgagcacat cagcaggtca cacaggaaag tactagatgt ctgaaatcca 3960
gctgacggag gcatcattga acgaagcagc cgatgctgca attaaagcgt tcgatggagc 4020
acaaaacctc gatgaattgg ctgctctgcg acgagatcac ctgggtgatg cggcaccaat 4080
ccctcaggca cgccgctcgc ttggaaccat tccaaaagat cagcgtaagg atgccggacg 4140
attcgtaaac atggcgctgg gccgcgcgga aaagcacttc gcccaggtta aggtggttct 4200
tgaagaaaag cgaaacgcag aagtcctgga gctggagcgc gtggatgtta ccgtccctac 4260
cacacgtgaa caagtcggcg cactgcaccc aattacgatt ctcaacgaac agatcgcgga 4320
catctttgtt ggcatgggct gggagatcgc agagggcccg gaagttgaag ccgaatactt 4380
caatttcgat gcacttaact ttctcccaga ccacccagcc cgcaccctgc aggatacctt 4440
ccacatcgca cctgaaggat cgcgccaagt gttgcgcacc catacctctc ctgtccaggt 4500
tcgcacgatg ctgaatcgag aggtacctat ctatatcgcc tgtcctggtc gcgtcttccg 4560
cactgacgaa ttggatgcta cccacacccc tgtctttcac cagatcgagg gcctggctgt 4620
cgacaaaggc ctgacaatgg ctcaccttcg cggaactctg gatcacttgg ctaaagaact 4680
gttcggacct gagactaaaa cccgcatgcg ttcaaactac ttcccatttt ctgagcccag 4740
cgcggaagtt gatgtctggt tcccaaataa gaagggcggt gccggctgga tcgaatgggg 4800
cggctgcggc atggtcaacc caaacgtgct ccgcgctgta ggcgtcgacc cggaagaata 4860
cactggattc ggcttcggta tgggtattga acgcaccttg caattccgaa atggactctc 4920
agatatgcgc gatatggtag aaggcgacat tcgctttacc ctccctttcg gcattcaggc 4980
ttaggcattt ttagtacgtg caataaccac tctggttttt ccagggtggt tttttgatgc 5040
cctttttgga gtcttcaact gagcctcgca gagcaggatt cccgttgagc accgccaggt 5100

gcgaataagg gacagtgaag aaggaacacc cgctcgcggg tgggcctact tcacctatcc 5160
tgcccggctg acgccgttgg atacaccaag gaaagtctac acgaaccctt tggcaaaatc 5220
ctgtatatcg tgcgaaaaag gatggatata ccgaaaaaat cgctataatg accccgaagc 5280
agggttatgc agcggaaaag ctccccgaaa agtgccacct gggtcctttt catcacgtgc 5340
tataaaaata attataattt aaatttttta atataaatat ataaattaaa aatagaaagt 5400
aaaaaaagaa attaaagaaa aaatagtttt tgttttccga agatgtaaaa gactctaggg 5460
ggatcgccaa caaatactac cttttatctt gctcttcctg ctctcaggta ttaatgccga 5520
attgtttcat cttgtctgtg tagaagacca cacacgaaaa tcctgtgatt ttacatttta 5580
cttatcgtta atcgaatgta tatctattta atctgctttt cttgtctaat aaatatatat 5640
gtaaagtacg ctttttgttg aaatttttta aacctttgtt tatttttttt ttcttcattc 5700
cgtaactctt ctaccttctt tatttacttt ctaaaatcca aatacaaaac ataaaaataa 5760
ataaacacag agtaaattcc caaattattc catcattaaa agatacgagg cgcgtgtaag 5820
ttacaggcaa gcgatccgtc taagaaacca ttattatcat gacattaacc tataaaaata 5880
ggcgtatcac gaggcccttt cgtctcgcgc gtttcggtga tgacggtgaa aacctctgac 5940
acatgcagct cccggagacg gtcacagctt gtctgtaagc ggatgccggg agcagacaag 6000
cccgtcaggg cgcgtcagcg ggtgttggcg ggtgtcgggg ctggcttaac tatgcggcat 6060
cagagcagat tgtactgaga gtgcaccata ccacctttc aattcatcat ttttttttta 6120
ttcttttttt tgatttcggt ttctttgaca tttttttgat tcggtaatct ccgaacagaa 6180
ggaagaacga aggaaggagc acagacttag attggtatat atacgcatat gtagtgttga 6240
agaaacatga aattgcccag tattcttaac ccaactgcac agaacaaaaa cctgcaggaa 6300
acgaagataa atcatgtcga aagctacata taaggaacgt gctgctactc atcctagtcc 6360
tgttgctgcc aagctattta atatcatgca cgaaaagcaa acaaacttgt gtgcttcatt 6420
ggatgttcgt accaccaagg aattactgga gttagttgaa gcattaggtc ccaaaatttg 6480
tttactaaaa acacatgtgg atatcttgac tgatttttcc atggagggca cagttaagcc 6540
gctaaaggca ttatccgcca agtacaattt tttactcttc gaagacagaa aatttgctga 6600
cattggtaat acagtcaaat tgcagtactc tgcgggtgta tacagaatag cagaatgggc 6660
agacattacg aatgcacacg gtgtggtggg cccaggtatt gttagcggtt tgaagcaggc 6720
ggcagaagaa gtaacaaagg aacctagagg ccttttgatg ttagcagaat tgtcatgcaa 6780
gggctcccta tctactggag aatatactaa gggtactgtt gacattgcga agagcgacaa 6840
agattttgtt atcggcttta ttgctcaaag agacatgggt ggaagagatg aaggttacga 6900
ttggttgatt atgacacccg gtgtgggttt agatgacaag ggagacgcat tgggtcaaca 6960
gtatagaacc gtggatgatg tggtctctac aggatctgac attattattg ttggaagagg 7020

actatttgca aagggaaggg atgctaaggt agagggtgaa cgttacagaa aagcaggctg 7080

ggaagcatat ttgagaagat gcggccagca aaactaaaaa actgtattat aagtaaatgc 7140

atgtatacta aactcacaaa ttagagcttc aatttaatta tatcagttat taccccctg 7200

gcttgttgtc cacaaccgtt aaaccttaaa agctttaaaa gccttatata ttctttttt 7260

tcttataaaa cttaaaacct tagaggctat ttaagttgct gatttatatt aattttattg 7320

ttcaaacatg agagcttagt acgtgaaaca tgagagctta gtacgttagc catgagggtt 7380

tagttcgtta gccatgaggg tttagttcgt taaacatgag agcttagtac gttaaacatg 7440

agagcttagt acgtgaaaca tgagagctta gtacgtacta tcaacaggtt gaactgctga 7500

tcttcggcat caaataaaac gaaaggctca gtcgaaagac tgggcctttc gttttatctg 7560

ttgtttgtcg gtgaacgctc tcctgagtag gacaaatccg ccgccctaga tattcacacg 7620

caatcaacag gcaggataat cgctggtaag gtcagtgctt tcttcaggta gtagagatac 7680

aatagttccc aacgataggt ggcagatttc actttacaga ccgactggtt cagaagcgta 7740

gataatagcc cgtgttttcc aataagggat agtgtaggta agtcaactcc tccgtcagag 7800

ccaaccgttt 7810

<210> 216
<211> 7756
<212> DNA
<213> Artificial Sequence

<220>
<223> vector backbone 3

<400> 216

```
gtttaaacaa ttttgtgtcg cccttaattg tgagcgctca caattccaca acggtttccc      60
tctagaaata attttgttta acttttcgag accttaggag gtaaacatat ggcgagcttg     120
gttaagaaag atatgtgtat taagatgacg atggaaggta ctgtgaacgg ttatcacttt     180
aagtgcgttg gcgagggtga aggcaagccg ttcgagggca cgcagaacat gcgcactcgt     240
gtcaccgagg gcggtccgct gccttttgca ttcgacatcc tggccccgtg ctgtatgtac     300
ggctctaaga ccttcattaa acacgtgagc ggtatcccgg attactttaa agagtccttt     360
ccagagggct tcacttggga acgtacccag atttttgagg acggtggtgt tctgaccgcg     420
caccaagaca ccagcctgga aggtaattgc ctgatctata aagtgaaggt tctgggtacc     480
aatttcccgg cgaatggtcc ggtgatgcaa aagaaaaccg cgggttggga gccgtgcgtc     540
gagatgctgt atccgcgtga cggcgtcttg tgtggtcaga gcttgatggc gctgaagtgc     600
accgatggca atcatctgac cagccacctg cgcacgacgt atcgtagccg taaaccgagc     660
aacgccgtta acatgccgga gttccatttt ggtgaccatc gcatcgaaat cctgaaagct     720
```

gagcagggca aattctacga acaatacgaa tcggctgtcg cacgttacag cgatgtgccg 780
gaaaaagcga cgtaatgaga attctgtaca ctcgagggtc tcaccccaag ggcgacaccc 840
cctaattagc ccgggcgaaa ggcccagtct ttcgactgag cctttcgttt tatttgagtt 900
taaacctggg gcgccctctg gtaaggttgg gaagccctgc aaagtaaact ggatgccagg 960
catcaaataa aacgaaaggc tcagtcgaaa gactgggcct ttcgttttat ctgttgtttg 1020
tcggtgaacg ctctctacta gagtcacact ggctcacctt cgggtgggcc tttctgcgtt 1080
tatagcacta ggacttgccg cggatactgc cccattacat gaattgcagc ctcagggacg 1140
tcagtagatc atggaggtag ggcatatgtc ctctgttgtt aaaatgtgag ttctcaacga 1200
agcacgaatc ggtcagaacc tacactaagg agatttggta ggtgcacggt ttctgtcgca 1260
tagaccagtt catttcagat gtctggcacg taagaggttc caactttcac cataatgaaa 1320
taagatcact accgggcgta ttttttgagt tatcgagatt ttcaggagct aaggaagcta 1380
aaatgattga acaagatgga ttgcacgcag gttctccggc cgcttgggtg gagaggctat 1440
tcggctatga ctgggcacaa cagacaatcg gctgctctga tgccgccgtg ttccggctgt 1500
cagcgcaggg gcgcccggtt ctttttgtca agaccgacct gtccggtgcc ctgaatgaac 1560
tccaagacga ggcagcgcgg ctatcgtggc tggccacgac gggcgttcct tgcgcagctg 1620
tgctcgacgt tgtcactgaa gcgggaaggg actggctgct attgggcgaa gtgccggggc 1680
aggatctcct gtcatctcac cttgctcctg ccgagaaagt atccatcatg gctgatgcaa 1740
tgcggcggct gcatacgctt gatccggcta cctgcccatt cgaccaccaa gcgaaacatc 1800
gcatcgagcg agcacgtact cggatggaag ccggtcttgt cgatcaggat gatctggacg 1860
aagagcatca ggggctcgcg ccagccgaac tgttcgccag gctcaaggcg cggatgcccg 1920
acggcgagga tctcgtcgtg acccatggcg atgcctgctt gccgaatatc atggtggaaa 1980
atggccgctt ttctggattc atcgactgtg gccggctggg tgtggcggac cgctatcagg 2040
acatagcgtt ggctacccgt gatattgctg aagagcttgg cggcgaatgg gctgaccgct 2100
tcctcgtgct ttacggtatc gccgctcccg attcgcagcg catcgccttc tatcgccttc 2160
ttgacgagtt cttctgatga tgaatggcaa ggcggcgcgt agcccccaa ccgaagttga 2220
ggggattttt ttgacaatta atcatccggc tcgtaattta tgtggatctt aatcatgcta 2280
aggaggtttt ctaatgatga acatcaaaaa gtttgcaaaa caagcaacag tattaacctt 2340
tactaccgca ctgctggcag gaggcgcaac tcaagcgttt gcgaaagaaa cgaaccaaaa 2400
gccatataag gaaacatacg gcatttccca tattacacgc catgatatgc tgcaaatccc 2460
tgaacagcaa aaaaatgaaa aatatcaagt ttctgaattt gattcgtcca caattaaaaa 2520
tatctcttct gcaaaaggcc tggacgtttg ggacagctgg ccattacaaa acgctgacgg 2580
cactgtcgca aactatcacg gctaccacat cgtctttgca ttagccggag atcctaaaaa 2640

tgcggatgac acatcgattt acatgttcta tcaaaaagtc ggcgaaactt ctattgacag 2700

ctggaaaaac gctggccgcg tctttaaaga cagcgacaaa ttcgatgcaa atgattctat 2760

cctaaaagac caaacacaag aatggtcagg ttcagccaca tttacatctg acggaaaaat 2820

ccgtttattc tacactgatt tctccggtaa acattacggc aaacaaacac tgacaactgc 2880

acaagttaac gtatcagcat cagacagctc tttgaacatc aacggtgtag aggattataa 2940

atcaatcttt gacggtgacg gaaaaacgta tcaaaatgta cagcagttca tcgatgaagg 3000

caactacagc tcaggcgaca accatacgct gagagatcct cactacgtag aagataaagg 3060

ccacaaatac ttagtatttg aagcaaacac tggaactgaa gatggctacc aaggcgaaga 3120

atctttattt aacaaagcat actatggcaa aagcacatca ttcttccgtc aagaaagtca 3180

aaaacttctg caaagcgata aaaaacgcac ggctgagtta gcaaacggcg ctctcggtat 3240

gattgagcta aacgatgatt acacactgaa aaaagtgatg aaaccgctga ttgcatctaa 3300

cacagtaaca gatgaaattg aacgcgcgaa cgtctttaaa atgaacggca aatggtacct 3360

gttcactgac tcccgcggat caaaaatgac gattgacggc attacgtcta acgatattta 3420

catgcttggt tatgtttcta attctttaac tggcccatac aagccgctga acaaaactgg 3480

ccttgtgtta aaaatggatc ttgatcctaa cgatgtaacc tttacttact cacacttcgc 3540

tgtacctcaa gcgaaaggaa acaatgtcgt gattacaagc tatatgacaa acagaggatt 3600

ctacgcagac aaacaatcaa cgtttgcgcc gagcttcctg ctgaacatca aaggcaagaa 3660

aacatctgtt gtcaaagaca gcatccttga acaaggacaa ttaacagtta acaaataaaa 3720

acgcaaaaga aaatgccgat gggtaccgag cgaaatgacc gaccaagcga cgcccaacct 3780

gccatcacga gatttcgatt ccaccgccgc cttctatgaa aggttgggct tcggaatcgt 3840

tttccgggac gccctcgcgg acgtgctcat agtccaggcg gtgttgacat aaataccact 3900

ggcggtgata ctgagcacat cagcaggtca cacaggaaag tactagatgt cgcatcttgc 3960

agaattagta gcttcagcga aggccgcgat ttctcaggcg agtgacgtcg cagcactgga 4020

taatgtacgt gttgagtacc tgggaaagaa gggacacctt actcttcaaa tgacaaccct 4080

gcgcgaactg ccgccggagg aacgccccgc agcaggagcg gtaatcaatg aggcaaagga 4140

gcaagtacaa caggcactga acgcccgtaa ggctgagttg gaatccgccg cattaaacgc 4200

gcgccttgct gcggaaacca ttgatgtctc gctgcccggg cgccgcattg agaatggagg 4260

cttacaccca gtgactcgta ccatcgaccg tatcgaatct ttctttggcg aacttggctt 4320

cactgtggca actggaccgg agattgagga cgactaccac aatttcgatg ccttgaacat 4380

tcccggtcat catcctgcac gcgccgatca tgatacattc tggtttgata ccacccgttt 4440

gcttcgtacc cagacaagcg gtgtccaaat ccgtacgatg aaggctcagc aaccaccgat 4500

ccgtatcatt gctccagggc gcgtgtaccg taacgattat gaccagacac atacaccgat 4560
gtttcaccaa atggaagggt tgattgtgga tacgaatatc tctttcacga atctgaaggg 4620
caccttacat gatttcttac gcaacttttt cgaggaggac cttcaaattc gctttcgtcc 4680
atcgtacttc ccttttgcag aaccttcggc tgaagtggat gtaatgggga aaaacggtaa 4740
gtggctggag gttttaggtt gcgggatggt tcatccaaat gtgcttcgca acgtcggcat 4800
cgaccccgaa gtctacagtg gattcggatt cgggatggga atggaacgtc tgactatgct 4860
tcgttacggc gtaacggatt tgcgctcctt ttttgagaac gatcttcgtt ttctgaagca 4920
attcaaataa gcatttttag tacgtgcaat aaccactctg gtttttccag ggtggttttt 4980
tgatgccctt tttggagtct tcaactgagc ctcgcagagc aggattcccg ttgagcaccg 5040
ccaggtgcga ataagggaca gtgaagaagg aacacccgct cgcgggtggg cctacttcac 5100
ctatcctgcc cggctgacgc cgttggatac accaaggaaa gtctacacga accctttggc 5160
aaaatcctgt atatcgtgcg aaaaaggatg gatataccga aaaaatcgct ataatgaccc 5220
cgaagcaggg ttatgcagcg gaaaagctcc ccgaaaagtg ccacctgggt ccttttcatc 5280
acgtgctata aaaataatta taatttaaat tttttaatat aaatatataa attaaaaata 5340
gaaagtaaaa aaagaaatta aagaaaaaat agtttttgtt ttccgaagat gtaaaagact 5400
ctagggggat cgccaacaaa tactaccttt tatcttgctc ttcctgctct caggtattaa 5460
tgccgaattg tttcatcttg tctgtgtaga agaccacaca cgaaaatcct gtgattttac 5520
attttactta tcgttaatcg aatgtatatc tatttaatct gcttttcttg tctaataaat 5580
atatatgtaa agtacgcttt ttgttgaaat tttttaaacc tttgtttatt tttttttct 5640
tcattccgta actcttctac cttctttatt tactttctaa aatccaaata caaaacataa 5700
aaataaataa acacagagta aattcccaaa ttattccatc attaaaagat acgaggcgcg 5760
tgtaagttac aggcaagcga tccgtctaag aaaccattat tatcatgaca ttaacctata 5820
aaaataggcg tatcacgagg ccctttcgtc tcgcgcgttt cggtgatgac ggtgaaaacc 5880
tctgacacat gcagctcccg gagacggtca cagcttgtct gtaagcggat gccgggagca 5940
gacaagcccg tcagggcgcg tcagcgggtg ttggcgggtg tcggggctgg cttaactatg 6000
cggcatcaga gcagattgta ctgagagtgc accataccac cttttcaatt catcattttt 6060
tttttattct ttttttgat ttcggtttct ttgacatttt tttgattcgg taatctccga 6120
acagaaggaa gaacgaagga aggagcacag acttagattg gtatatatac gcatatgtag 6180
tgttgaagaa acatgaaatt gcccagtatt cttaacccaa ctgcacagaa caaaaacctg 6240
caggaaacga agataaatca tgtcgaaagc tacatataag gaacgtgctg ctactcatcc 6300
tagtcctgtt gctgccaagc tatttaatat catgcacgaa aagcaaacaa acttgtgtgc 6360
ttcattggat gttcgtacca ccaaggaatt actggagtta gttgaagcat taggtcccaa 6420

aatttgttta ctaaaaacac atgtggatat cttgactgat ttttccatgg agggcacagt 6480

taagccgcta aaggcattat ccgccaagta caatttttta ctcttcgaag acagaaaatt 6540

tgctgacatt ggtaatacag tcaaattgca gtactctgcg ggtgtataca gaatagcaga 6600

atgggcagac attacgaatg cacacggtgt ggtgggccca ggtattgtta gcggtttgaa 6660

gcaggcggca gaagaagtaa caaaggaacc tagaggcctt ttgatgttag cagaattgtc 6720

atgcaagggc tccctatcta ctggagaata tactaagggt actgttgaca ttgcgaagag 6780

cgacaaagat tttgttatcg gctttattgc tcaaagagac atgggtggaa gagatgaagg 6840

ttacgattgg ttgattatga cacccggtgt gggtttagat gacaagggag acgcattggg 6900

tcaacagtat agaaccgtgg atgatgtggt ctctacagga tctgacatta ttattgttgg 6960

aagaggacta tttgcaaagg gaagggatgc taaggtagag ggtgaacgtt acagaaaagc 7020

aggctgggaa gcatatttga gaagatgcgg ccagcaaaac taaaaaactg tattataagt 7080

aaatgcatgt atactaaact cacaaattag agcttcaatt taattatatc agttattacc 7140

cccctggctt gttgtccaca accgttaaac cttaaaagct ttaaaagcct tatatattct 7200

tttttttctt ataaaactta aaaccttaga ggctatttaa gttgctgatt tatattaatt 7260

ttattgttca aacatgagag cttagtacgt gaaacatgag agcttagtac gttagccatg 7320

agggtttagt tcgttagcca tgagggttta gttcgttaaa catgagagct tagtacgtta 7380

aacatgagag cttagtacgt gaaacatgag agcttagtac gtactatcaa caggttgaac 7440

tgctgatctt cggcatcaaa taaaacgaaa ggctcagtcg aaagactggg cctttcgttt 7500

tatctgttgt ttgtcggtga acgctctcct gagtaggaca aatccgccgc cctagatatt 7560

cacacgcaat caacaggcag gataatcgct ggtaaggtca gtgctttctt caggtagtag 7620

agatacaata gttcccaacg ataggtggca gatttcactt tacagaccga ctggttcaga 7680

agcgtagata atagcccgtg ttttccaata agggatagtg taggtaagtc aactcctccg 7740

tcagagccaa ccgttt 7756

<210> 217
<211> 8067
<212> DNA
<213> Artificial Sequence

<220>
<223> vector backbone 4

<400> 217

gtttaaacaa ttttgtgtcg cccttaattg tgagcgctca caattccaca acggtttccc 60

tctagaaata attttgttta acttttcgag accttaggag gtaaacatat ggcgagcttg 120

gttaagaaag atatgtgtat taagatgacg atggaaggta ctgtgaacgg ttatcacttt 180

aagtgcgttg gcgagggtga aggcaagccg ttcgagggca cgcagaacat gcgcactcgt 240
gtcaccgagg gcggtccgct gccttttgca ttcgacatcc tggccccgtg ctgtatgtac 300
ggctctaaga ccttcattaa acacgtgagc ggtatcccgg attactttaa agagtccttt 360
ccagagggct tcacttggga acgtacccag atttttgagg acggtggtgt tctgaccgcg 420
caccaagaca ccagcctgga aggtaattgc ctgatctata aagtgaaggt tctgggtacc 480
aatttcccgg cgaatggtcc ggtgatgcaa aagaaaaccg cgggttggga gccgtgcgtc 540
gagatgctgt atccgcgtga cggcgtcttg tgtggtcaga gcttgatggc gctgaagtgc 600
accgatggca atcatctgac cagccacctg cgcacgacgt atcgtagccg taaaccgagc 660
aacgccgtta acatgccgga gttccatttt ggtgaccatc gcatcgaaat cctgaaagct 720
gagcagggca aattctacga acaatacgaa tcggctgtcg cacgttacag cgatgtgccg 780
gaaaaagcga cgtaatgaga attctgtaca ctcgagggtc tcaccccaag ggcgacaccc 840
cctaattagc ccgggcgaaa ggcccagtct ttcgactgag cctttcgttt tatttgagtt 900
taaacctggg gcgccctctg gtaaggttgg gaagccctgc aaagtaaact ggatgccagg 960
catcaaataa aacgaaaggc tcagtcgaaa gactgggcct ttcgttttat ctgttgtttg 1020
tcggtgaacg ctctctacta gagtcacact ggctcacctt cgggtgggcc tttctgcgtt 1080
tatagcacta ggacttgccg cggatactgc cccattacat gaattgcagc ctcagggacg 1140
tcagtagatc atggaggtag ggcatatgtc ctctgttgtt aaaatgtgag ttctcaacga 1200
agcacgaatc ggtcagaacc tacactaagg agatttggta ggtgcacggt ttctgtcgca 1260
tagaccagtt catttcagat gtctggcacg taagaggttc caactttcac cataatgaaa 1320
taagatcact accgggcgta tttttttgagt tatcgagatt ttcaggagct aaggaagcta 1380
aaatgattga acaagatgga ttgcacgcag gttctccggc cgcttgggtg gagaggctat 1440
tcggctatga ctgggcacaa cagacaatcg gctgctctga tgccgccgtg ttccggctgt 1500
cagcgcaggg gcgcccggtt ctttttgtca agaccgacct gtccggtgcc ctgaatgaac 1560
tccaagacga ggcagcgcgg ctatcgtggc tggccacgac gggcgttcct tgcgcagctg 1620
tgctcgacgt tgtcactgaa gcgggaaggg actggctgct attgggcgaa gtgccggggc 1680
aggatctcct gtcatctcac cttgctcctg ccgagaaagt atccatcatg gctgatgcaa 1740
tgcggcggct gcatacgctt gatccggcta cctgcccatt cgaccaccaa gcgaaacatc 1800
gcatcgagcg agcacgtact cggatggaag ccggtcttgt cgatcaggat gatctggacg 1860
aagagcatca ggggctcgcg ccagccgaac tgttcgccag gctcaaggcg cggatgcccg 1920
acggcgagga tctcgtcgtg acccatggcg atgcctgctt gccgaatatc atggtggaaa 1980
atggccgctt ttctggattc atcgactgtg gccggctggg tgtggcggac cgctatcagg 2040
acatagcgtt ggctacccgt gatattgctg aagagcttgg cggcgaatgg gctgaccgct 2100

tcctcgtgct ttacggtatc gccgctcccg attcgcagcg catcgccttc tatcgccttc 2160
ttgacgagtt cttctgatga tgaatggcaa ggcggcgcgt agccccccaa ccgaagttga 2220
ggggattttt ttgacaatta atcatccggc tcgtaattta tgtggatctt aatcatgcta 2280
aggaggtttt ctaatgatga acatcaaaaa gtttgcaaaa caagcaacag tattaacctt 2340
tactaccgca ctgctggcag gaggcgcaac tcaagcgttt gcgaaagaaa cgaaccaaaa 2400
gccatataag gaaacatacg gcatttccca tattacacgc catgatatgc tgcaaatccc 2460
tgaacagcaa aaaaatgaaa aatatcaagt ttctgaattt gattcgtcca caattaaaaa 2520
tatctcttct gcaaaaggcc tggacgtttg ggacagctgg ccattacaaa acgctgacgg 2580
cactgtcgca aactatcacg gctaccacat cgtctttgca ttagccggag atcctaaaaa 2640
tgcggatgac acatcgattt acatgttcta tcaaaaagtc ggcgaaactt ctattgacag 2700
ctggaaaaac gctggccgcg tctttaaaga cagcgacaaa ttcgatgcaa atgattctat 2760
cctaaaagac caaacacaag aatggtcagg ttcagccaca tttacatctg acggaaaaat 2820
ccgtttattc tacactgatt tctccggtaa acattacggc aaacaaacac tgacaactgc 2880
acaagttaac gtatcagcat cagacagctc tttgaacatc aacggtgtag aggattataa 2940
atcaatcttt gacggtgacg gaaaaacgta tcaaaatgta cagcagttca tcgatgaagg 3000
caactacagc tcaggcgaca accatacgct gagagatcct cactacgtag aagataaagg 3060
ccacaaatac ttagtatttg aagcaaacac tggaactgaa gatggctacc aaggcgaaga 3120
atctttattt aacaaagcat actatggcaa aagcacatca ttcttccgtc aagaaagtca 3180
aaaacttctg caaagcgata aaaaacgcac ggctgagtta gcaaacggcg ctctcggtat 3240
gattgagcta aacgatgatt acacactgaa aaaagtgatg aaaccgctga ttgcatctaa 3300
cacagtaaca gatgaaattg aacgcgcgaa cgtctttaaa atgaacggca aatggtacct 3360
gttcactgac tcccgcggat caaaaatgac gattgacggc attacgtcta acgatattta 3420
catgcttggt tatgtttcta attctttaac tggcccatac aagccgctga acaaaactgg 3480
ccttgtgtta aaaatggatc ttgatcctaa cgatgtaacc tttacttact cacacttcgc 3540
tgtacctcaa gcgaaaggaa acaatgtcgt gattacaagc tatatgacaa acagaggatt 3600
ctacgcagac aaacaatcaa cgtttgcgcc gagcttcctg ctgaacatca aaggcaagaa 3660
aacatctgtt gtcaaagaca gcatccttga acaaggacaa ttaacagtta acaaataaaa 3720
acgcaaaaga aaatgccgat gggtaccgag cgaaatgacc gaccaagcga cgcccaacct 3780
gccatcacga gatttcgatt ccaccgccgc cttctatgaa aggttgggct tcggaatcgt 3840
tttccgggac gccctcgcgg acgtgctcat agtccaggcg gtgttgacat aaataccact 3900
ggcggtgata ctgagcacat cagcaggtca cacaggaaag tactagatgt cgcatcttgc 3960

agaattagta gcttcagcga aggccgcgat ttctcaggcg agtgacgtcg cagcactgga 4020

taatgtacgt gttgagtacc tgggaaagaa gggacacctt actcttcaaa tgacaaccct 4080

gcgcgaactg ccgccggagg aacgccccgc agcaggagcg gtaatcaatg aggcaaagga 4140

gcaagtacaa caggcactga acgcccgtaa ggctgagttg gaatccgccg cattaaacgc 4200

gcgccttgct gcggaaacca ttgatgtctc gctgcccggg cgccgcattg agaatggagg 4260

cttacaccca gtgactcgta ccatcgaccg tatcgaatct ttctttggcg aacttggctt 4320

cactgtggca actggaccgg agattgagga cgactaccac aatttcgatg ccttgaacat 4380

tcccggtcat catcctgcac gcgccgatca tgatacattc tggtttgata ccacccgttt 4440

gcttcgtacc cagacaagcg gtgtccaaat ccgtacgatg aaggctcagc aaccaccgat 4500

ccgtatcatt gctccagggc gcgtgtaccg taacgattat gaccagacac atacaccgat 4560

gtttcaccaa atggaagggt tgattgtgga tacgaatatc tctttcacga atctgaaggg 4620

caccttacat gatttcttac gcaacttttt cgaggaggac cttcaaattc gctttcgtcc 4680

atcgtacttc ccttttgcag aaccttcggc tgaagtggat gtaatgggga aaaacggtaa 4740

gtggctggag gttttaggtt gcgggatggt tcatccaaat gtgcttcgca acgtcggcat 4800

cgaccccgaa gtctacagtg gattcggatt cgggatggga atggaacgtc tgactatgct 4860

tcgttacggc gtaacggatt tgcgctcctt ttttgagaac gatcttcgtt ttctgaagca 4920

attcaaataa gcatttttag tacgtgcaat aaccactctg gtttttccag ggtggttttt 4980

tgatgccctt tttggagtct tcaactgagc ctcgcagagc aggattcccg ttgagcaccg 5040

ccaggtgcga ataagggaca gtgaagaagg aacacccgct cgcgggtggg cctacttcac 5100

ctatcctgcc cggctgacgc cgttggatac accaaggaaa gtctacacga accctttggc 5160

aaaatcctgt atatcgtgcg aaaaaggatg gatataccga aaaaatcgct ataatgaccc 5220

cgaagcaggg ttatgcagcg gaaaagctcc ccgaaaagtg ccacctgggt ccttttcatc 5280

acgtgctata aaaataatta taatttaaat tttttaatat aaatatataa attaaaaata 5340

gaaagtaaaa aaagaaatta aagaaaaaat agtttttgtt ttccgaagat gtaaaagact 5400

ctagggggat cgccaacaaa tactaccttt tatcttgctc ttcctgctct caggtattaa 5460

tgccgaattg tttcatcttg tctgtgtaga agaccacaca cgaaaatcct gtgattttac 5520

attttactta tcgttaatcg aatgtatatc tatttaatct gcttttcttg tctaataaat 5580

atatatgtaa agtacgcttt ttgttgaaat tttttaaacc tttgtttatt ttttttttct 5640

tcattccgta actcttctac cttctttatt tactttctaa aatccaaata caaaacataa 5700

aaataaataa acacagagta aattcccaaa ttattccatc attaaaagat acgaggcgcg 5760

tgtaagttac aggcaagcga tccgtctaag aaaccattat tatcatgaca ttaacctata 5820

aaaataggcg tatcacgagg ccctttcgtc tcgcgcgttt cggtgatgac ggtgaaaacc 5880

tctgacacat gcagctcccg gagacggtca cagcttgtct gtaagcggat gccgggagca 5940
gacaagcccg tcagggcgcg tcagcgggtg ttggcgggtg tcggggctgg cttaactatg 6000
cggcatcaga gcagattgta ctgagagtgc accataccac agccggaaga ggagtaggga 6060
atattactgg ctgaaaataa gtcttgaatg aacgtatacg cgtatatttc taccaatctc 6120
tcaacactga gtaatggtag ttataagaaa gagaccgagt tagggacagt tagaggcggt 6180
ggagatattc cttatggcat gtctggcgat gataaaactt ttcaaacggc agccccgatc 6240
taaaagagct gacagggaaa tggtcagaaa aagaaacgtg cacccgcccg tctggacgcg 6300
ccgctcaccc gcacggcaga gaccaatcag taaaaatcaa cggttaacga cattactata 6360
tatataatat aggaagcatt taatagaaca gcatcgtaat atatgtgtac tttgcagtta 6420
tgacgccaga tggcagtagt ggaagatatt ctttattgaa aaatagcttg tcaccttacg 6480
tacaatcttg atccggagct tttctttttt tgccgattaa gaattcggtc gaaaaaagaa 6540
aaggagaggg ccaagaggga gggcattggt gactattgag cacgtgagta tacgtgatta 6600
agcacacaaa ggcagcttgg agtatgtctg ttattaattt cacaggtagt tctggtccat 6660
tggtgaaagt ttgcggcttg cagagcacag aggccgcaga atgtgctcta gattccgatg 6720
ctgacttgct gggtattata tgtgtgccca atagaaagag aacaattgac ccggttattg 6780
caaggaaaat ttcaagtctt gtaaaagcat ataaaaatag ttcaggcact ccgaaatact 6840
tggttggcgt gtttcgtaat caacctaagg aggatgtttt ggctctggtc aatgattacg 6900
gcattgatat cgtccaactg catggagatg agtcgtggca agaataccaa gagttcctcg 6960
gtttgccagt tattaaaaga ctcgtatttc caaaagactg caacatacta ctcagtgcag 7020
cttcacagaa acctcattcg tttattccct tgtttgattc agaagcaggt gggacaggtg 7080
aacttttgga ttggaactcg atttctgact gggttggaag gcaagagagc cccgaaagct 7140
tacattttat gttagctggt ggactgacgc cagaaaatgt tggtgatgcg cttagattaa 7200
atggcgttat tggtgttgat gtaagcggag gtgtggagac aaatggtgta aaagactcta 7260
acaaaatagc aaatttcgtc aaaaatgcta agaaataggt tattactgag tagtatttat 7320
ttaagtattg tttgtgcact tgcctgcagg ccttttgaaa agcaagcata aaagatctaa 7380
acataaaatc tgtaaaataa caagatgtaa agataatgct aaatcatttg gctttttgat 7440
tgattgtaca ggccctggct tgttgtccac aaccgttaaa ccttaaaagc tttaaaagcc 7500
ttatatattc tttttttct tataaaactt aaaaccttag aggctattta agttgctgat 7560
ttatattaat tttattgttc aaacatgaga gcttagtacg tgaaacatga gagcttagta 7620
cgttagccat gagggtttag ttcgttagcc atgagggttt agttcgttaa acatgagagc 7680
ttagtacgtt aaacatgaga gcttagtacg tgaaacatga gagcttagta cgtactatca 7740

acaggttgaa ctgctgatct tcggcatcaa ataaaacgaa aggctcagtc gaaagactgg 7800

gcctttcgtt ttatctgttg tttgtcggtg aacgctctcc tgagtaggac aaatccgccg 7860

ccctagatat tcacacgcaa tcaacaggca ggataatcgc tggtaaggtc agtgctttct 7920

tcaggtagta gagatacaat agttcccaac gataggtggc agatttcact ttacagaccg 7980

actggttcag aagcgtagat aatagcccgt gttttccaat aagggatagt gtaggtaagt 8040

caactcctcc gtcagagcca accgttt 8067

<210> 218
<211> 200
<212> DNA
<213> Artificial Sequence

<220>
<223> Insulator 1 vector element

<400> 218

tattcacacg caatcaacag gcaggataat cgctggtaag gtcagtgctt tcttcaggta 60

gtagagatac aatagttccc aacgataggt ggcagatttc actttacaga ccgactggtt 120

cagaagcgta gataatagcc cgtgttttcc aataagggat agtgtaggta agtcaactcc 180

tccgtcagag ccaaccgttt 200

<210> 219
<211> 200
<212> DNA
<213> Artificial Sequence

<220>
<223> Insulator 2 vector element

<400> 219

gcactaggac ttgccgcgga tactgcccca ttacatgaat tgcagcctca gggacgtcag 60

tagatcatgg aggtagggca tatgtcctct gttgttaaaa tgtgagttct caacgaagca 120

cgaatcggtc agaacctaca ctaaggagat ttggtaggtg cacggtttct gtcgcataga 180

ccagttcatt tcagatgtct 200

<210> 220
<211> 105
<212> DNA
<213> Artificial Sequence

<220>
<223> T1 vector element

<400> 220

ggcatcaaat aaaacgaaag gctcagtcga aagactgggc ctttcgtttt atctgttgtt 60

tgtcggtgaa cgctctcctg agtaggacaa atccgccgcc ctaga 105

<210> 221
<211> 129
<212> DNA
<213> Artificial Sequence

<220>
<223> B0015 vector element

<400> 221

ccaggcatca aataaaacga aaggctcagt cgaaagactg ggcctttcgt tttatctgtt 60

gtttgtcggt gaacgctctc tactagagtc acactggctc accttcgggt gggcctttct 120

gcgtttata 129

<210> 222
<211> 66
<212> DNA
<213> Artificial Sequence

<220>
<223> SacB promoter

<400> 222

ttgacaatta atcatccggc tcgtaattta tgtggatctt aatcatgcta aggaggtttt 60

ctaatg 66

<210> 223
<211> 1054
<212> DNA
<213> Artificial Sequence

<220>
<223> PheS promoter and sequence

<400> 223

ggcggtgttg acataaatac cactggcggt gatactgagc acatcagcag gtcacacagg 60
aaagtactag atgtcgcatc ttgcagaatt agtagcttca gcgaaggccg cgatttctca 120
ggcgagtgac gtcgcagcac tggataatgt acgtgttgag tacctgggaa agaagggaca 180
ccttactctt caaatgacaa ccctgcgcga actgccgccg gaggaacgcc ccgcagcagg 240
agcggtaatc aatgaggcaa aggagcaagt acaacaggca ctgaacgccc gtaaggctga 300
gttggaatcc gccgcattaa acgcgcgcct tgctgcggaa accattgatg tctcgctgcc 360
cgggcgccgc attgagaatg gaggcttaca cccagtgact cgtaccatcg accgtatcga 420
atctttcttt ggcgaacttg gcttcactgt ggcaactgga ccggagattg aggacgacta 480
ccacaatttc gatgccttga acattcccgg tcatcatcct gcacgcgccg atcatgatac 540
attctggttt gataccaccc gtttgcttcg tacccagaca agcggtgtcc aaatccgtac 600
gatgaaggct cagcaaccac cgatccgtat cattgctcca gggcgcgtgt accgtaacga 660
ttatgaccag acacatacac cgatgtttca ccaaatggaa gggttgattg tggatacgaa 720
tatctctttc acgaatctga agggcacctt acatgatttc ttacgcaact tttcgagga 780
ggaccttcaa attcgctttc gtccatcgta cttccctttt gcagaacctt cggctgaagt 840
ggatgtaatg ggaaaaacg gtaagtggct ggaggtttta ggttgcggga tggttcatcc 900
aaatgtgctt cgcaacgtcg gcatcgaccc cgaagtctac agtggattcg gattcgggat 960
gggaatggaa cgtctgacta tgcttcgtta cggcgtaacg gatttgcgct ccttttttga 1020
gaacgatctt cgttttctga agcaattcaa ataa 1054

<210> 224
<211> 77
<212> DNA
<213> Corynebacterium glutamicum

<400> 224

gcatttttag tacgtgcaat aaccactctg gtttttccag ggtggttttt tgatgccctt 60
tttggagtct tcaactg 77

<210> 225
<211> 90
<212> DNA
<213> Escherichia coli

<400> 225

ttcagccaaa aaacttaaga ccgccggtct tgtccactac cttgcagtaa tgcggtggac 60
aggatcggcg gttttctttt ctcttctcaa 90

<210> 226
<211> 52
<212> DNA
<213> Escherichia coli

<400> 226
gacgaacaat aaggcctccc aaatcggggg gccttttta ttgataacaa aa 52

<210> 227
<211> 42
<212> DNA
<213> Escherichia coli

<400> 227
gtaacaacgg aaaccggcca ttgcgccggt ttttttggc ct 42

<210> 228
<211> 41
<212> DNA
<213> Escherichia coli

<400> 228
aagcagagaa ccctggatga gagtccgggg tttttgtttt t 41

<210> 229
<211> 71
<212> DNA
<213> Escherichia coli

<400> 229

taaagtatgt atccccaaaa taattcgagt cattgcatct gtggctagaa gtatgaaggg 60

attaaccata a 71

<210> 230
<211> 57
<212> DNA
<213> Escherichia coli

<400> 230
ggaaacacag aaaaaagccc gcacctgaca gtgcgggctt ttttttcga ccaaagg 57

<210> 231
<211> 168
<212> DNA
<213> Streptococcus sp.

<400> 231

atgacgtata agttgatttt aaatggaaag accctgaaag gggagacgac cactgaagct 60

gtagatgcgg cgactgctga aaaagtcttt aagcaatatg caaacgacaa cggagttgat 120

ggtgaatgga cttatgatga cgccactaaa acgttcacag taaccgaa 168

<210> 232
<211> 207

<212> DNA
<213> Fasciola hepatica

<400> 232

atgccaagcg tacaggaagt ggagaagtta cttcatgtgt tggatcggaa cggagacgga 60

aaagtttcag ccgaagagtt aaaggccttt gcggacgact ctaagtgccc tctggattcc 120

aacaagataa aagcgtttat taaggagcat gataagaaca aggatggtaa acttgacctg 180

aaggagctgg tctccatact gtcgagc 207

<210> 233
<211> 228
<212> DNA
<213> Unknown

<220>
<223> Solubility Tag Ubiquitin (PST3)

<400> 233

atgcagatct ttgttaagac tttaacagga aaaacgataa cgctggaagt tgagtctagc 60

gacacaatag acaatgtcaa atcaaagatt caagataaag agggtatacc accggaccag 120

caacggttga ttttcgcagg taagcaatta gaggatggga gaacactgag tgactacaat 180

atccagaaag aatcgacgtt gcatttggtg cttagattgc gtggaggt 228

<210> 234
<211> 303
<212> DNA
<213> Homo sapiens

<400> 234

atgagtgatt ctgaggttaa tcaagaggcc aaaccggaag taaaaccaga agtgaagcct 60

gaaacgcata tcaatttgaa agtctcggac ggctcgtctg aaatattctt caagataaaa 120

aagacgacac cacttcggcg tctgatggag gcgtttgcca aaagacaagg aaaagaaatg 180

gactcgcttc gtttcttata cgacggtatc agaatacaag cggaccagac tccagaagat 240

cttgatatgg aggataatga cattatcgaa gcccatcgcg agcagatagg tggcgcgaca 300

tac 303

<210> 235
<211> 56
<212> PRT
<213> Streptococcus sp.

<400> 235

Met Thr Tyr Lys Leu Ile Leu Asn Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210> 236
<211> 69
<212> PRT
<213> Fasciola hepatica

<400> 236

Met Pro Ser Val Gln Glu Val Glu Lys Leu Leu His Val Leu Asp Arg
1               5                   10                  15

Asn Gly Asp Gly Lys Val Ser Ala Glu Glu Leu Lys Ala Phe Ala Asp
            20                  25                  30

Asp Ser Lys Cys Pro Leu Asp Ser Asn Lys Ile Lys Ala Phe Ile Lys
        35                  40                  45

Glu His Asp Lys Asn Lys Asp Gly Lys Leu Asp Leu Lys Glu Leu Val
    50                  55                  60

Ser Ile Leu Ser Ser
65

<210> 237
<211> 76
<212> PRT
<213> Unknown

<220>
<223> Solubility Tag Ubiquitin (PST3)

<400> 237

```
Met Gln Ile Phe Val Lys Thr Leu Thr Gly Lys Thr Ile Thr Leu Glu
1               5                   10                  15

Val Glu Ser Ser Asp Thr Ile Asp Asn Val Lys Ser Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly
65                  70                  75
```

<210> 238
<211> 101
<212> PRT
<213> Homo sapiens

<400> 238

```
Met Ser Asp Ser Glu Val Asn Gln Glu Ala Lys Pro Glu Val Lys Pro
1               5                   10                  15

Glu Val Lys Pro Glu Thr His Ile Asn Leu Lys Val Ser Asp Gly Ser
            20                  25                  30

Ser Glu Ile Phe Phe Lys Ile Lys Lys Thr Thr Pro Leu Arg Arg Leu
        35                  40                  45

Met Glu Ala Phe Ala Lys Arg Gln Gly Lys Glu Met Asp Ser Leu Arg
    50                  55                  60

Phe Leu Tyr Asp Gly Ile Arg Ile Gln Ala Asp Gln Thr Pro Glu Asp
65                  70                  75                  80

Leu Asp Met Glu Asp Asn Asp Ile Ile Glu Ala His Arg Glu Gln Ile
                85                  90                  95

Gly Gly Ala Thr Tyr
            100
```

<210> 239
<211> 36
<212> DNA
<213> Escherichia coli

<400> 239
gcagcaaatg acgagaatta tgccttggcg gcttaa 36

<210> 240
<211> 36
<212> DNA
<213> Escherichia coli

<400> 240
gctgcaaacg atgagaatta tgccctggta gcataa 36

<210> 241
<211> 36
<212> DNA
<213> Escherichia coli

<400> 241
gctgctaatg atgagaacta tgcagccgcg gtataa 36

<210> 242
<211> 36
<212> DNA
<213> Escherichia coli

<400> 242
gccgccaatg atgagaatta tgcagcgagt gtgtaa 36

<210> 243
<211> 30
<212> DNA
<213> Escherichia coli

<400> 243
cgcagtgagc agatttcgat ggagttttaa 30

<210> 244
<211> 18
<212> DNA
<213> Escherichia coli

<400> 244
tcattgttat ggtcctaa 18

<210> 245
<211> 63
<212> DNA
<213> Escherichia coli

<400> 245

**gcttcctcgc atgcgacccg tcaactgtca ggtctgaaaa ttcactcaaa cctttaccat 60**

**taa 63**

<210> 246
<211> 66
<212> DNA
<213> Escherichia coli

<400> 246

atgggacagc tgcgctcatt aaatggggag tggcgcttcg cgtggttccc agctcccgaa 60

gctgtg 66

<210> 247
<211> 11
<212> PRT
<213> Escherichia coli

<400> 247

Ala Ala Asn Asp Glu Asn Tyr Ala Leu Ala Ala
1 5 10

<210> 248
<211> 11
<212> PRT
<213> Escherichia coli

<400> 248

Ala Ala Asn Asp Glu Asn Tyr Ala Leu Val Ala
1 5 10

<210> 249
<211> 11
<212> PRT
<213> Escherichia coli

<400> 249

Ala Ala Asn Asp Glu Asn Tyr Ala Ala Ala Val
1 5 10

<210> 250
<211> 11
<212> **PRT**
<213> Escherichia coli

<400> 250

Ala Ala Asn Asp Glu Asn Tyr Ala Ala Ser Val
1 5 10

<210> 251
<211> 9
<212> PRT
<213> Escherichia coli

<400> 251

Arg Ser Glu Gln Ile Ser Met Glu Phe
1 5

<210> 252
<211> 5
<212> PRT

<213> Escherichia coli

<400> 252

Ser Leu Leu Trp Ser
1               5

<210> 253
<211> 20
<212> PRT
<213> Escherichia coli

<400> 253

Ala Ser Ser His Ala Thr Arg Gln Leu Ser Gly Leu Lys Ile His Ser
1               5                   10                  15

Asn Leu Tyr His
            20

<210> 254
<211> 22
<212> PRT
<213> Escherichia coli

<400> 254

Met Gly Gln Leu Arg Ser Leu Asn Gly Glu Trp Arg Phe Ala Trp Phe
1               5                   10                  15

Pro Ala Pro Glu Ala Val
            20

<210> 255
<211> 133
<212> DNA
<213> Artificial Sequence

<220>
<223> P3_BCD1

<400> 255

aaaaaattta tttgcttatt aattcatccg gctcgtataa tgtgtggagg gcccaagttc 60

acttaaaaag gagatcaaca atgaaagcaa ttttcgtact gaaacatctt aatcatgcac 120

aggagacttt cta 133

<210> 256
<211> 121
<212> DNA
<213> Artificial Sequence

<220>
<223> P4_BCD22

<400> 256

ttgacatcag gaaaattttt ctgtataatg tgtggagggc ccaagttcac ttaaaaagga 60

gatcaacaat gaaagcaatt ttcgtactga aacatcttaa tcatgcctag gaagttttct 120

a 121

<210> 257
<211> 132
<212> DNA
<213> Artificial Sequence

<220>
<223> P7_BCD19

<400> 257

aaaaaattta tttgctttcg catctttttg tacctataat gtgtggaggg cccaagttca 60

cttaaaaagg agatcaacaa tgaaagcaat tttcgtactg aaacatctta atcatgctat 120

ggaggttttc ta 132

<210> 258
<211> 121
<212> DNA
<213> Artificial Sequence

<220>
<223> P8_BCD15

<400> 258

ttcactttta atcatccggc tcgtataatg tgtggagggc ccaagttcac ttaaaaagga 60

gatcaacaat gaaagcaatt ttcgtactga aacatcttaa tcatgcgggg gagtctttct 120

a 121

<210> 259
<211> 121
<212> DNA
<213> Artificial Sequence

<220>
<223> P11_BCD17

<400> 259

ttgacaatta atcatccggc tcttagtgtt tgtggagggc ccaagttcac ttaaaaagga 60

gatcaacaat gaaagcaatt ttcgtactga aacatcttaa tcatgcggag gagggtttct 120

a 121

<210> 260
<211> 121

<212> DNA
<213> Artificial Sequence

<220>
<223> P13_BCD8

<400> 260

```
ttccctatta atcatccggc tcgtataatg tgtggagggc ccaagttcac ttaaaaagga      60

gatcaacaat gaaagcaatt ttcgtactga aacatcttaa tcatgcatcg gaccgtttct     120

a                                                                     121
```

## Description

### CROSS-REFERENCE TO RELATED APPLICATIONS

This application claims the benefit of priority to U.S. Provisional Application Serial No. 62/515,870, filed June 6, 2017.

### FIELD

The present disclosure is directed to high-throughput (HTP) microbial genomic engineering for *Escherichia coli.* The disclosed HTP genomic engineering platform is computationally driven and integrates molecular biology, automation, and advanced machine learning protocols. This integrative platform utilizes a suite of HTP molecular tool sets to create HTP genetic design libraries, which are derived from, *inter alia,* scientific insight and iterative pattern recognition.

### STATEMENT REGARDING SEQUENCE LISTING

The Sequence Listing associated with this application is provided in text format in lieu of a paper copy. The name of the text file containing the Sequence Listing is ZYMR_012_01WO_SeqList_ST25.txt. The text file is ≈ 127 KB, was created on June 6, 2018, and is being submitted electronically *via* EFS-Web.

### BACKGROUND

Humans have been harnessing the power of microbial cellular biosynthetic pathways for millennia to produce products of interest, the oldest examples of which include alcohol, vinegar, cheese, and yogurt. These products are still in large demand today and have also been accompanied by an ever increasing repertoire of products producible by microbes. The advent of genetic engineering technology has enabled scientists to design and program novel biosynthetic pathways into a variety of organisms to produce a broad range of industrial, medical, and consumer products. Indeed, microbial cellular cultures are now used to produce products ranging from small molecules, antibiotics, vaccines, insecticides, enzymes, fuels, and industrial chemicals.

Given the large number of products produced by modern industrial microbes, it comes as no surprise that engineers are under tremendous pressure to improve the speed and efficiency by which a given microorganism is able to produce a target product. A variety of approaches have been used to improve the economy of biologically-based industrial processes by "improving" the microorganism involved. For example, many pharmaceutical and chemical industries rely on microbial strain improvement programs in which the parent strains of a microbial culture are continuously mutated through exposure to chemicals or UV radiation and are subsequently screened for performance increases, such as in productivity, yield and titer. This mutagenesis process is extensively repeated until a strain demonstrates a suitable increase in product performance. The subsequent "improved" strain is then utilized in commercial production. The identification of improved industrial microbial strains through mutagenesis is time consuming and inefficient. The process, by its very nature, is haphazard and relies on stumbling upon a mutation that has a desirable outcome on product output. Not only are traditional microbial strain improvement programs inefficient, but the process can also lead to industrial strains with a high degree of detrimental mutagenic load. The accumulation of mutations in industrial strains subjected to these types of programs can become significant and may lead to an eventual stagnation in the rate of performance improvement.

Perhaps there is no better example of the stagnation resultant from trational strain improvement programs than with *E. coli,* which is one of the most engineered microbial host systems in existence. The microbe has been subjected to the aforementioned traditional methods of microbial strain improvement for decades. Despite the vast amount of effort that has been devoted to engineering *E. coli,* the microbe still possess an enormous amount of untapped potential. This is because *E. coli* presents unique challenges for researchers attempting to improve the microbe for production purposes. These challenges have hampered the field of genomic engineering in *E. coli* and prevented researchers from harnessing the full potential of this microbial system.

In particular, the industry has not yet developed a high-throughput system for genomic engineering in *E. coli.* It is clear that traditional methods of strain improvement have reached a plateau with respect to this organismal system, but yet researchers do not have the genomic engineering tools that are needed to traverse this plateau.

Lee Michael E et al., ASC Synthetic Biology, 18 September 2015, vol. 4, no. 9, pages 975 to 986, discloses a platform for engineering microbes (e.g. yeast) based on a modular assembly of multiple apart plasmids (e.g., selected from libraries of elements such as promoter or terminator part plasmids) to form a transcriptional unit that enables expression of single genes in microbes.

Kincade JM et al., Gene, vol. 97, no. 1, 1 January 1991, pages 7 to 12, discloses the bacteriophage lambda promoters pL and pR sequence determinanats of *in vivo* activity and of sensitivity to the DNA gyrase inhibitor, coumermycin.

Rainer Knaus et al., The EMBO Journal, vol. 7, no. 9, 25 August 1988, pages 2919-2923, discloses different chimeric promoter sequences comprising variant pL and pR promoter sequences, about 43 nucleotides in length and consisting of a distal portion of lambda phage pR promoter, variable -35 and -10 regions of lambda phage pL and pR promoters that are each six nucleotides in length, and core portions of lambda phage pL and pR.

EP 2 657 250 A2 describes a method for the enhancement of acetyl-coA synthetase activity through enhancement of gene expression by modification of nucleotide sequences of the promoter region and the 5'-UTR region of the asc gene.

US 2006/141578 A1 discloses a method of producing a desired protein by gene recombination. The vectors used comprise the 5'-UTR/Ribosomal Binding Site (RBS) portion of the promoter of the E. coli acs gene.

William R McCleray, Applied Microbiology and Biotechnology, vol. 84, no. 4, 25 July 2009, pages 641-648, is a review article discussing the application of promoter swapping techniques to control expression of chromosomal genes.

Pengfei GU et al., Microbial Cell Factories, vo. 11, no. 1, 2 March 2012, page 30, discloses the use of promoter swapping to improve the production of L-tryptophan in *Escherichia coli*.

Rice Christopher D et al., Applied and Enviromental Microbiology, vol. 75, no. 3, Februray 2009, pages 573-583, discloses that employment of a promoter-swapping technique shows that PhoU modulates the activity of the $PstSCAB_2$ ABC transporter in *Escherichia coli.* Gueguen Erwan et al., Applied and Environmental Microbiology, vol. 79, no. 1, January 2013, pages 32-38, discloses the use of promoter swapping to unveil the role of the *Citrobacter rodentium* CTS1 type VI secretion system in interbacterial competition.

Thus, there is a great need in the art for new methods of engineering *E. coli* for production purposes, which do not suffer from the aforementioned drawbacks inherent with traditional strain improvement programs. Specifically, a high-throughput system for discovering and consolidating beneficial mutations in *E. coli* would revolutionize the field and allow researchers to tap the full potential of this organism.

## SUMMARY OF THE DISCLOSURE

The present disclosure provides a high-throughput (HTP) genomic engineering platform for *E. coli* that does not suffer from the myriad of problems associated with traditional microbial strain improvement programs.

Further, the HTP platform taught herein is able to rehabilitate *E. coli* strains that have accumulated non-beneficial mutations through decades of random mutagenesis-based strain improvement programs.

The disclosure also provides for unique genomic engineering toolsets and procedures, which undergird the HTP platform's functionality in an *E. coli* system.

The disclosed HTP genomic engineering platform is computationally driven and integrates molecular biology, automation, and advanced machine learning protocols. This integrative platform utilizes a suite of HTP molecular tool sets to create HTP genetic design libraries, which are derived from, *inter alia,* scientific insight and iterative pattern recognition.

The taught HTP genetic design libraries function as drivers of the genomic engineering process, by providing libraries of particular genomic alterations for testing in *E. coli.* The microbes engineered utilizing a particular library, or combination of libraries, are efficiently screened in a HTP manner for a resultant outcome, e.g. production of a product of interest. This process of utilizing the HTP genetic design libraries to define particular genomic alterations for testing in a microbe and then subsequently screening host microbial genomes harboring the alterations is implemented in an efficient and iterative manner. In some examples, the iterative cycle or "rounds" of genomic engineering campaigns can be at least 1, 2, 3, 4, 5, 6, 7, 8, 9, 10, 20, 30, 40, 50, 60, 70, 80, 90, 100, or more iterations/cycles/rounds.

Thus, in some examples, the present disclosure teaches methods of conducting at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 125, 150, 175, 200, 225, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, 1000 or more "rounds" of HTP genetic engineering (e.g., rounds of SNP swap, PRO swap, STOP swap, or combinations thereof) in an *E. coli* host system.

In some examples, the present disclosure teaches a linear approach, in which each subsequent HTP genetic engineering round is based on genetic variation identified in the previous round of genetic engineering. In other examples the present disclosure teaches a non-linear approach, in which each subsequent HTP genetic engineering round is based on genetic variation identified in any previous round of genetic engineering, including previously conducted analysis, and separate HTP genetic engineering branches.

The data from these iterative cycles enables large scale data analytics and pattern recognition, which is utilized by the integrative platform to inform subsequent rounds of HTP genetic design library implementation. Consequently, the HTP genetic design libraries utilized in the taught platform are highly dynamic tools that benefit from large scale data pattern recognition algorithms and become more informative through each iterative round of microbial engineering. Such a system has never been developed for *E. coli* and is desperately needed in the art.

In some examples, the genetic design libraries of the present disclosure comprise at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 125, 150, 175, 200, 225, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, 1000 or more individual genetic changes (e.g., at least X number of promoter:gene combinations in the PRO swap library).

In some examples, the present disclosure teaches a high-throughput (HTP) method of genomic engineering to evolve an *E. coli* strain to acquire a desired phenotype, comprising: a) perturbing the genomes of an initial plurality of *E. coli* strains having the same strain background, to thereby create an initial HTP genetic design *E. coli* strain library comprising individual strains with unique genetic variations; b) screening and selecting individual strains of the initial HTP genetic design *E. coli* strain library for the desired phenotype; c) providing a subsequent plurality of *E. coli* microbes that each comprise a unique combination of genetic variation, said genetic variation selected from the genetic variation present in at least two individual *E. coli* strains screened in the preceding step, to thereby create a subsequent HTP genetic design *E. coli* strain library; d) screening and selecting individual *E. coli* strains of the subsequent HTP genetic design *E. coli* strain library for the desired phenotype; e) repeating steps c)-d) one or more times, in a linear or non-linear fashion, until an *E. coli* strain has acquired the desired phenotype, wherein each subsequent iteration creates a new HTP genetic design *E. coli* strain library comprising individual *E. coli* strains harboring unique genetic variations that are a combination of genetic variation selected from amongst at least two individual *E. coli* strains of a preceding HTP genetic design *E. coli* strain library.

In some examples, the present disclosure teaches that the initial HTP genetic design *E. coli* strain library is at least one selected from the group consisting of a promoter swap microbial strain library, SNP swap microbial strain library, start/stop codon microbial strain library, optimized sequence microbial strain library, a terminator swap microbial strain library, a protein solubility tag microbial strain library, a protein degradation tag microbial strain library or any combination thereof.

In some examples, the present disclosure teaches methods of making a subsequent plurality of *E. coli* strains that each comprise a unique combination of genetic variations, wherein each of the combined genetic variations is derived from the initial HTP genetic design *E. coli* strain library or the HTP genetic design *E. coli* strain library of the preceding step.

In some examples, the combination of genetic variations in the subsequent plurality of *E. coli* strains will comprise a subset of all the possible combinations of the genetic variations in the initial HTP genetic design *E. coli* strain library or the HTP genetic design *E. coli* strain library of the preceding step.

In some examples, the present disclosure teaches that the subsequent HTP genetic design *E. coli* strain library is a full combinatorial strain library derived from the genetic variations in the initial HTP genetic design *E. coli* strain library or the HTP genetic design *E. coli* strain library of the preceding step.

For example, if the prior HTP genetic design *E. coli* strain library only had genetic variations A, B, C, and D, then a partial combinatorial of said variations could include a subsequent HTP genetic design *E. coli* strain library comprising three strains with each comprising either the AB, AC, or AD unique combinations of genetic variations (order in which the mutations are represented is unimportant). A full combinatorial *E. coli* strain library derived from the genetic variations of the HTP genetic design library of the preceding step would include six microbes, each comprising either AB, AC, AD, BC, BD, or CD unique combinations of genetic variations.

In some examples, the methods of the present disclosure teach perturbing the genome of *E. coli* utilizing at least one method selected from the group consisting of: random mutagenesis, targeted sequence insertions, targeted sequence deletions, targeted sequence replacements, or any combination thereof.

In some examples of the presently disclosed methods, the initial plurality of *E. coli* comprise unique genetic variations derived from an industrial production *E. coli* strain.

In some examples of the presently disclosed methods, the initial plurality of *E. coli* comprise industrial production *E. coli* strains denoted $S_1Gen_1$ and any number of subsequent microbial generations derived therefrom denoted $S_nGen_n$.

In some examples, the present disclosure teaches a method for generating a SNP swap *E. coli* strain library, comprising the steps of: a) providing a reference *E. coli* strain and a second *E. coli* strain, wherein the second *E. coli* strain comprises a plurality of identified genetic variations selected from single nucleotide polymorphisms, DNA insertions, and DNA deletions, which are not present in the reference strain; b) perturbing the genome of either the reference strain, or the second strain, to thereby create an initial SNP swap *E. coli* strain library comprising a plurality of individual *E. coli* strains with unique genetic variations found within each strain of said plurality of individual strains, wherein each of said unique genetic variations corresponds to a single genetic variation selected from the plurality of identified genetic variations between the reference strain and the second strain.

In some examples of a SNP swap library, the genome of the reference *E. coli* strain is perturbed to add one or more of the identified single nucleotide polymorphisms, DNA insertions, or DNA deletions, which are found in the second *E. coli* strain.

In some examples of a SNP swap library, the genome of the second *E. coli* strain is perturbed to remove one or more of the identified single nucleotide polymorphisms, DNA insertions, or DNA deletions, which are not found in the reference *E. coli* strain.

In some examples, the genetic variations of the SNP swap library will comprise a subset of all the genetic variations identified between the reference *E. coli* strain and the second *E. coli* strain.

In some examples, the genetic variations of the SNP swap library will comprise all of the identified genetic variations identified between the reference *E. coli* strain and the second *E. coli* strain.

In some examples, the present disclosure teaches a method for rehabilitating and improving the phenotypic performance of an industrial *E. coli* strain, comprising the steps of: a) providing a parental lineage *E. coli* strain and an industrial *E. coli* strain derived therefrom, wherein the industrial strain comprises a plurality of identified genetic variations selected from single nucleotide polymorphisms, DNA insertions, and DNA deletions, not present in the parental lineage strain; b) perturbing the genome of either the parental lineage strain, or the industrial strain, to thereby create an initial SNP swap *E. coli* strain library comprising a plurality of individual strains with unique genetic variations found within each strain of said plurality of individual strains, wherein each of said unique genetic variations corresponds to a single genetic variation selected from the plurality of identified genetic variations between the parental lineage strain and the industrial strain; c) screening and selecting individual strains of the initial SNP swap *E. coli* strain library for phenotype performance improvements over a reference *E. coli* strain, thereby identifying unique genetic variations that confer said *E. coli* strains with phenotype performance improvements; d) providing a subsequent plurality of *E. coli* strains that each comprise a unique combination of genetic variation, said genetic variation selected from the genetic variation present in at least two individual strains screened in the preceding step, to thereby create a subsequent SNP swap *E. coli* strain library; e) screening and selecting individual strains of the subsequent SNP swap *E. coli* strain library for phenotype performance improvements over the reference strain, thereby identifying unique combinations of genetic variation that confer said *E. coli* strains with additional phenotype performance improvements; and f) repeating steps d)-e) one or more times, in a linear or non-linear fashion, until a strain exhibits a desired level of improved phenotype performance compared to the phenotype performance of the industrial *E. coli* strain, wherein each subsequent iteration creates a new SNP swap *E. coli* strain library comprising individual microbial strains harboring unique genetic variations that are a combination of genetic variation selected from amongst at least two individual microbial strains of a preceding SNP swap *E. coli* strain library.

In some examples, the present disclosure teaches methods for rehabilitating and improving the phenotypic performance of an industrial *E. coli* strain, wherein the genome of the parental lineage *E. coli* strain is perturbed to add one or more of the identified single nucleotide polymorphisms, DNA insertions, or DNA deletions, which are found in the industrial *E. coli* strain.

In some examples, the present disclosure teaches methods for rehabilitating and improving the phenotypic performance of an industrial *E. coli* strain, wherein the genome of the industrial *E. coli* strain is perturbed to remove one or more of the identified single nucleotide polymorphisms, DNA insertions, or DNA deletions, which are not found in the parental lineage *E. coli* strain.

In some examples, the present disclosure teaches a method for generating a promoter swap *E. coli* strain library, said method comprising the steps of: a) providing a plurality of target genes endogenous to a base *E. coli* strain, and a promoter ladder, wherein said promoter ladder comprises a plurality of promoters exhibiting different expression profiles in the base *E. coli* strain; b) engineering the genome of the base *E. coli* strain, to thereby create an initial promoter swap *E. coli* strain library comprising a plurality of individual *E. coli* strains with unique genetic variations found within each strain of said plurality of individual strains, wherein each of said unique genetic variations comprises one of the promoters from the promoter ladder operably linked to one of the target genes endogenous to the base *E. coli* strain.

In some examples, the present disclosure teaches a promoter swap method of genomic engineering to evolve an *E. coli* strain to acquire a desired phenotype, said method comprising the steps of: a) providing a plurality of target genes endogenous to a base *E. coli* strain, and a promoter ladder, wherein said promoter ladder comprises a plurality of promoters exhibiting different expression profiles in the base *E. coli* strain; b) engineering the genome of the base *E. coli* strain, to thereby create an initial promoter swap *E. coli* strain library comprising a plurality of individual *E. coli* strains with unique genetic variations found within each strain of said plurality of individual strains, wherein each of said unique genetic variations comprises one of the promoters from the promoter ladder operably linked to one of the target genes endogenous to the base *E. coli* strain; c) screening and selecting individual strains of the initial promoter swap *E. coli* strain library for the desired phenotype; d) providing a subsequent plurality of *E. coli* strains that each comprise a unique combination of genetic variation, said genetic variation selected from the genetic variation present in at least two individual strains screened in the preceding step, to thereby create a subsequent promoter swap *E. coli* strain library; e) screening and selecting individual strains of the subsequent promoter swap *E. coli* strain library for the desired phenotype; f) repeating steps d)-e) one or more times, in a linear or non-linear fashion, until a microbe has acquired the desired phenotype, wherein each subsequent iteration creates a new promoter swap *E. coli* strain library comprising individual strains harboring unique genetic variations that are a combination of genetic variation selected from amongst at least two individual strains of a preceding promoter swap *E. coli* strain library.

In some examples, the present disclosure teaches a method for generating a terminator swap *E. coli* strain library, said method comprising the steps of: a) providing a plurality of target genes endogenous to a base *E. coli* strain, and a terminator ladder, wherein said terminator ladder comprises a plurality of terminators exhibiting different expression profiles in the base *E. coli* strain; b) engineering the genome of the base *E. coli* strain, to thereby create an initial terminator swap *E. coli* strain library comprising a plurality of individual strains with unique genetic variations found within each strain of said plurality of individual strains, wherein each of said unique genetic variations comprises one of the target genes endogenous to the base *E. coli* strain operably linked to one or more of the terminators from the terminator ladder.

In some examples, the present disclosure teaches a terminator swap method of genomic engineering to evolve an *E. coli* strain to acquire a desired phenotype, said method comprising the steps of: a) providing a plurality of target genes endogenous to a base *E. coli* strain, and a terminator ladder, wherein said terminator ladder comprises a plurality of terminators exhibiting different expression profiles in the base *E. coli* strain; b) engineering the genome of the base *E. coli* strain, to thereby create an initial terminator swap *E. coli* strain library comprising a plurality of individual *E. coli* strains with unique genetic variations found within each strain of said plurality of individual strains, wherein each of said unique genetic variations comprises one of the target genes endogenous to the base *E. coli* strain operably linked to one or more of the terminators from the terminator ladder; c) screening and selecting individual microbial strains of the initial terminator swap *E. coli* strain library for the desired phenotype; d) providing a subsequent plurality of *E. coli* strains that each comprise a unique combination of genetic variation, said genetic variation selected from the genetic variation present in at least two individual strains screened in the preceding step, to thereby create a subsequent terminator swap *E. coli* strain library; e) screening and selecting individual strains of the subsequent terminator swap *E. coli* strain library for the desired phenotype; f) repeating steps d)-e) one or more times, in a linear or non-linear fashion, until a microbe has acquired the desired phenotype, wherein each subsequent iteration creates a new terminator swap *E. coli* strain library comprising individual strains harboring unique genetic variations that are a combination of genetic variation selected from amongst at least two individual strains of a preceding terminator swap *E. coli* strain library.

In some examples, the present disclosure teaches iteratively improving the design of candidate *E. coli* strains by (a) accessing a predictive model populated with a training set comprising (1) inputs representing genetic changes to one or more background *E. coli* strains and (2) corresponding performance measures; (b) applying test inputs to the predictive model that represent genetic changes, the test inputs corresponding to candidate *E. coli* strains incorporating those genetic changes; (c) predicting phenotypic performance of the candidate *E. coli* strains based at least in part upon the predictive model; (d) selecting a first subset of the candidate *E. coli* strains based at least in part upon their predicted performance; (e) obtaining measured phenotypic performance of the first subset of the candidate *E. coli* strains; (f) obtaining a selection of a second subset of the candidate *E. coli* strains based at least in part upon their measured phenotypic performance; (g) adding to the training set of the predictive model (1) inputs corresponding to the selected second subset of candidate *E. coli* strains, along with (2) corresponding measured performance of the selected second subset of candidate *E. coli* strains; and (h) repeating (b)-(g) until measured phenotypic performance of at least one candidate *E. coli* strain satisfies a performance metric. In some cases, during a first application of test inputs to the predictive model, the genetic changes represented by the test inputs comprise genetic changes to the one or more background *E. coli* strains; and during subsequent applications of test inputs, the genetic changes represented by the test inputs comprise genetic changes to candidate *E. coli* strains within a previously selected second subset of candidate *E. coli* strains.

In some examples, selection of the first subset may be based on epistatic effects. This may be achieved by: during a first selection of the first subset: determining degrees of dissimilarity between performance measures of the one or more background *E. coli* strains in response to application of a plurality of respective inputs representing genetic changes to the one or more background *E. coli* strains; and selecting for inclusion in the first subset at least two candidate *E. coli* strains based at least in part upon the degrees of dissimilarity in the performance measures of the one or more background *E. coli* strains in response to application of genetic changes incorporated into the at least two candidate *E. coli* strains.

In some examples, the present disclosure teaches applying epistatic effects in the iterative improvement of candidate *E. coli* strains, the method comprising: obtaining data representing measured performance in response to corresponding genetic changes made to at least one *E. coli* background strain; obtaining a selection of at least two genetic changes based at least in part upon a degree of dissimilarity between the corresponding responsive performance measures of the at least two genetic changes, wherein the degree of dissimilarity relates to the degree to which the at least two genetic changes affect their corresponding responsive performance measures through different biological pathways; and designing genetic changes to an *E. coli* background strain that include the selected genetic changes. In some cases, the *E. coli* background strain for which the at least two selected genetic changes are designed is the same as the at least one *E. coli* background strain for which data representing measured responsive performance was obtained.

In some examples, the present disclosure teaches HTP *E. coli* strain improvement methods utilizing only a single type of genetic library. For example, in some examples, the present disclosure teaches HTP *E. coli* strain improvement methods utilizing only SNP swap libraries. In other examples, the present disclosure teaches HTP *E. coli* strain improvement methods utilizing only PRO swap libraries. In some examples, the present disclosure teaches HTP *E. coli* strain improvement methods utilizing only STOP swap libraries. In some examples, the present disclosure teaches HTP *E. coli* strain improvement methods utilizing only Start/Stop Codon swap libraries.

In other examples, the present disclosure teaches HTP *E. coli* strain improvement methods utilizing two or more types of genetic libraries. For example, in some examples, the present disclosure teaches HTP *E. coli* strain improvement methods combining SNP swap and PRO swap libraries. In some examples, the present disclosure teaches HTP *E. coli* strain improvement methods combining SNP swap and STOP swap libraries. In some examples, the present disclosure teaches HTP *E. coli* strain improvement methods combining PRO swap and STOP swap libraries.

In other examples, the present disclosure teaches HTP *E. coli* strain improvement methods utilizing multiple types of genetic libraries (see, for example, Figure 5). In some examples, the genetic libraries are combined to produce combination mutations (e.g., promoter/terminator combination ladders applied to one or more genes). In yet other examples, the HTP *E. coli* strain improvement methods of the present disclosure can be combined with one or more traditional strain improvement methods.

In some examples, the HTP *E. coli* strain improvement methods of the present disclosure result in an improved *E. coli* host cell. That is, the present disclosure teaches methods of improving one or more *E. coli* host cell properties. In some examples the improved *E. coli* host cell property is selected from the group consisting of: volumetric productivity, specific productivity, yield or titre, of a product of interest produced by the *E. coli* host cell. In some examples, the improved *E. coli* host cell property is volumetric productivity. In some examples, the improved *E. coli* host cell property is specific productivity. In some examples, the improved *E. coli* host cell property is yield.

In some examples, the HTP *E. coli* strain improvement methods of the present disclosure result in an an *E. coli* host cell that exhibits a 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 150%, 200%, 250%, 300% or more of an improvement in at least one *E. coli* host cell property over a control *E. coli* host cell that is not subjected to the HTP strain improvements methods (e.g, an X% improvement in yield or productivity of a biomolecule of interest, incorporating any ranges and subranges therebetween). In some examples, the HTP *E. coli* strain improvement methods of the present disclosure are selected from the group consisting of SNP swap, PRO swap, STOP swap, SOLUBILITY TAG swap, DEGRADATION TAG swap, and combinations thereof.

Thus, in some examples, the SNP swap methods of the present disclosure result in an *E. coli* host cell that exhibits a 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 150%, 200%, 250%, 300% or more of an improvement in at least one *E. coli* host cell property over a control *E. coli* host cell that is not subjected to the SNP swap methods (e.g, an X% improvement in yield or productivity of a biomolecule of interest, incorporating any ranges and subranges therebetween).

Thus, in some examples, the PRO swap methods of the present disclosure result in an *E. coli* host cell that exhibits a 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 150%, 200%, 250%, 300% or more of an improvement in at least one *E. coli* host cell property over a control *E. coli* host cell that is not subjected to the PRO swap methods (e.g, an X% improvement in yield or productivity of a biomolecule of interest, incorporating any ranges and subranges therebetween).

Thus, in some examples, the TERMINATOR swap methods of the present disclosure result in an *E. coli* host cell that exhibits a 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%,

80%, 81%, 82%, 83%, 84%, 85%, 86%, 87%, 88%, 89%, 90%, 91%, 92%, 93%, 94%, 95%, 96%, 97%, 98%, 99%, 100%, 150%, 200%, 250%, 300% or more of an improvement in at least one *E. coli* host cell property over a control *E. coli* host cell that is not subjected to the TERMINATOR swap methods (e.g, an X% improvement in yield or productivity of a biomolecule of interest, incorporating any ranges and subranges therebetween).

Thus, in some examples, the SOLUBILITY TAG swap methods of the present disclosure result in an *E. coli* host cell that exhibits a 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 150%, 200%, 250%, 300% or more of an improvement in at least one *E. coli* host cell property over a control *E. coli* host cell that is not subjected to the SOLUBILITY TAG swap methods (e.g, an X% improvement in yield or productivity of a biomolecule of interest, incorporating any ranges and subranges therebetween).

Thus, in some examples, the DEGRADATION TAG swap methods of the present disclosure result in an *E. coli* host cell that exhibits a 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 150%, 200%, 250%, 300% or more of an improvement in at least one *E. coli* host cell property over a control *E. coli* host cell that is not subjected to the DEGRADATION TAG swap methods (e.g, an X% improvement in yield or productivity of a biomolecule of interest, incorporating any ranges and subranges therebetween).

In some examples, the present disclosure teaches a method for generating a protein solubility tag swap *E. coli* strain library, comprising the steps of: a. providing a plurality of target genes endogenous to a base *E. coli* strain, and a solubility tag ladder, wherein said solubility tag ladder comprises a plurality of solubility tags exhibiting different solubility profiles in the base *E. coli* strain; and b. engineering the genome of the base *E. coli* strain, to thereby create an initial solubility tag swap *E. coli* strain library comprising a plurality of individual *E. coli* strains with unique genetic variations found within each strain of said plurality of individual *E. coli* strains, wherein each of said unique genetic variations comprises one or more of the solubility tags from the solubility tag ladder operably linked to one of the target genes endogenous to the base *E. coli* strain.

In some examples, the present disclosure teaches a protein solubility tag swap method for improving the phenotypic performance of a production *E. coli* strain, comprising the steps of: providing a plurality of target genes endogenous to a base *E. coli* strain, and a solubility tag ladder, wherein said solubility tag ladder comprises a plurality of solubility tags exhibiting different expression profiles in the base *E. coli* strain; engineering the genome of the base *E. coli* strain, to thereby create an initial solubility tag swap *E. coli* strain library comprising a plurality of individual *E. coli* strains with unique genetic variations found within each strain of said plurality of individual *E. coli* strains, wherein each of said unique genetic variations comprises one or more of the solubility tags from the solubility tag ladder operably linked to one of the target genes endogenous to the base *E. coli* strain; screening and selecting individual *E. coli* strains of the initial solubility tag swap *E. coli* strain library for phenotypic performance improvements over a reference *E. coli* strain, thereby identifying unique genetic variations that confer phenotypic performance improvements; providing a subsequent plurality of *E. coli* microbes that each comprise a combination of unique genetic variations from the genetic variations present in at least two individual *E. coli* strains screened in the preceding step, to thereby create a subsequent solubility tag swap *E. coli* strain library; screening and selecting individual *E. coli* strains of the subsequent solubility tag swap *E. coli* strain library for phenotypic performance improvements over the reference *E. coli* strain, thereby identifying unique combinations of genetic variation that confer additional phenotypic performance improvements; and repeating steps d)-e) one or more times, in a linear or non-linear fashion, until an *E. coli* strain exhibits a desired level of improved phenotypic performance compared to the phenotypic performance of the production *E. coli* strain, wherein each subsequent iteration creates a new solubility tag swap *E. coli* strain library of microbial strains, where each strain in the new library comprises genetic variations that are a combination of genetic variations selected from amongst at least two individual *E. coli* strains of a preceding library.

In some examples, the subsequent solubility tag swap *E. coli* strain library is a full combinatorial library of the initial solubility tag *swap E. coli* strain library.

In some examples, the subsequent solubility tag swap *E. coli* strain library is a subset of a full combinatorial library of the initial solubility tag swap *E. coli* strain library.

In some examples, the subsequent solubility tag swap *E. coli* strain library is a full combinatorial library of a preceding solubility tag swap *E. coli* strain library.

In some examples, the subsequent solubility tag swap *E. coli* strain library is a subset of a full combinatorial library of a preceding solubility tag swap *E. coli* strain library.

In some examples, steps d)-e) are repeated until the phenotypic performance of an *E. coli* strain of a subsequent solubility tag swap *E. coli* strain library exhibits at least a 10% increase in a measured phenotypic variable compared to the phenotypic performance of the production *E. coli* strain.

In some examples, steps d)-e) are repeated until the phenotypic performance of an *E. coli* strain of a subsequent solubility tag swap *E. coli* strain library exhibits at least a one-fold increase in a measured phenotypic variable compared to the phenotypic performance of the production *E. coli* strain.

In some examples, the improved phenotypic performance of step f) is selected from the group consisting of: volumetric productivity of a product of interest, specific productivity of a product of interest, yield of a product of interest, titer of a product of interest, and combinations thereof.

In some examples, the improved phenotypic performance of step f) is: increased or more efficient production of a product of interest, said product of interest selected from the group consisting of: a small molecule, enzyme, peptide, amino acid, organic acid, synthetic compound, fuel, alcohol, primary extracellular metabolite, secondary extracellular metabolite, intracellular component molecule, and combinations thereof.

In some examples, the present disclosure teaches a method for generating a protein degradation tag swap *E. coli* strain library, comprising the steps of: a. providing a plurality of target genes endogenous to a base *E. coli* strain, and a degradation tag ladder, wherein said degradation tag ladder comprises a plurality of degradation tags exhibiting different solubility profiles in the base *E. coli* strain; and b. engineering the genome of the base *E. coli* strain, to thereby create an initial degradation tag swap *E. coli* strain library comprising a plurality of individual *E. coli* strains with unique genetic variations found within each strain of said plurality of individual *E. coli* strains, wherein each of said unique genetic variations comprises one or more of the degradation tags from the degradation tag ladder operably linked to one of the target genes endogenous to the base *E. coli* strain.

In some examples, the present disclosure teaches a protein degradation tag swap method for improving the phenotypic performance of a production *E. coli* strain, comprising the steps of: providing a plurality of target genes endogenous to a base *E. coli* strain, and a degradation tag ladder, wherein said degradation tag ladder comprises a plurality of degradation tags exhibiting different expression profiles in the base *E. coli* strain; engineering the genome of the base *E. coli* strain, to thereby create an initial degradation tag swap *E. coli* strain library comprising a plurality of individual *E. coli* strains with unique genetic variations found within each strain of said plurality of individual *E. coli* strains, wherein each of said unique genetic variations comprises one or more of the degradation tags from the degradation tag ladder operably linked to one of the target genes endogenous to the base *E. coli* strain; screening and selecting individual *E. coli* strains of the initial degradation tag swap *E. coli* strain library for phenotypic performance improvements over a reference *E. coli* strain, thereby identifying unique genetic variations that confer phenotypic performance improvements; providing a subsequent plurality of *E. coli* microbes that each comprise a combination of unique genetic variations from the genetic variations present in at least two individual *E. coli* strains screened in the preceding step, to thereby create a subsequent degradation tag swap *E. coli* strain library; screening and selecting individual *E. coli* strains of the subsequent degradation tag swap *E. coli* strain library for phenotypic performance improvements over the reference *E. coli* strain, thereby identifying unique combinations of genetic variation that confer additional phenotypic performance improvements; and repeating steps d)-e) one or more times, in a linear or non-linear fashion, until an *E. coli* strain exhibits a desired level of improved phenotypic performance compared to the phenotypic performance of the production *E. coli* strain, wherein each subsequent iteration creates a new degradation tag swap *E. coli* strain library of microbial strains, where each strain in the new library comprises genetic variations that are a combination of genetic variations selected from amongst at least two individual *E. coli* strains of a preceding library.

In some examples, the subsequent degradation tag swap *E. coli* strain library is a full combinatorial library of the initial degradation tag swap *E. coli* strain library.

In some examples, the subsequent degradation tag swap *E. coli* strain library is a subset of a full combinatorial library of the initial degradation tag swap *E. coli* strain library.

In some examples, the subsequent degradation tag swap *E. coli* strain library is a full combinatorial library of a preceding degradation tag swap *E. coli* strain library.

In some examples, the subsequent degradation tag swap *E. coli* strain library is a subset of a full combinatorial library of a preceding degradation tag swap *E. coli* strain library.

In some examples, steps d)-e) are repeated until the phenotypic performance of an *E. coli* strain of a subsequent degradation tag swap *E. coli* strain library exhibits at least a 10% increase in a measured phenotypic variable compared to the phenotypic performance of the production *E. coli* strain.

In some examples, steps d)-e) are repeated until the phenotypic performance of an *E. coli* strain of a subsequent degradation tag swap *E. coli* strain library exhibits at least a one-fold increase in a measured phenotypic variable compared to the phenotypic performance of the production *E. coli* strain.

In some examples, the improved phenotypic performance of step f) is selected from the group consisting of: volumetric productivity of a product of interest, specific productivity of a product of interest, yield of a product of interest, titer of a product of interest, and combinations thereof.

In some examples, the improved phenotypic performance of step f) is: increased or more efficient production of a product of interest, said product of interest selected from the group consisting of: a small molecule, enzyme, peptide, amino acid, organic acid, synthetic compound, fuel, alcohol, primary extracellular metabolite, secondary extracellular metabolite, intracellular component molecule, and combinations thereof.

In some examples, the present disclosure teaches a chimeric synthetic promoter operably linked to a heterologous gene for expression in a microbial host cell, wherein the chimeric synthetic promoter is 60-90 nucleotides in length and consists of a distal portion of lambda phage $p_R$ promoter, variable -35 and -10 regions of lambda phage $p_L$ and $p_R$ promoters that are each six nucleotides in length, core portions of lambda phage $p_L$ and $p_R$ promoters and a 5' UTR/Ribosomal Binding Site (RBS) portion of lambda phage $p_R$ promoter.

In some examples, nucleic acid sequences of the distal portion of the lambda phage $p_R$ promoter, the variable -35 and -10 regions of the lambda phage $p_L$ and $p_R$ promoters, the core portions of the the lambda phage $p_L$ and $p_R$ promoters and the 5' UTR/Ribosomal Binding Site (RBS) portion of the lambda phage $p_R$ promoter are selected from the nucleic acid sequences found in Table 1.5.

In some examples, the present disclosure teaches a chimeric synthetic promoter operably linked to a heterologous gene for expression in a microbial host cell, wherein the chimeric synthetic promoter is 60-90 nucleotides in length and consists of a distal portion of lambda phage $p_R$ promoter, variable -35 and -10 regions of lambda phage $p_L$ and $p_R$ promoters that are each six nucleotides in length, core portions of lambda phage $p_L$ and $p_R$ promoters and a 5' UTR/Ribosomal Binding Site (RBS) portion of the promoter of the *E. coli acs* gene.

In some examples, nucleic acid sequences of the distal portion of the lambda phage $p_R$ promoter, the variable -35 and -10 regions of the lambda phage $p_L$ and $p_R$ promoters, the core portions of the the lambda phage $p_L$ and $p_R$ promoters and the 5' UTR/Ribosomal Binding Site (RBS) portion of the promoter of the *E. coli acs* gene are selected from the nucleic acid sequences found in Table 1.5.

In some examples, the chimeric synthetic promoter consists of a nucleic acid sequence selected from SEQ ID NOs. 132-152, 159-160, 162, 165, 174-175, 188, 190, 199-201 or 207.

In some examples, the chimeric synthetic promoter consists of a nucleic acid sequence selected from SEQ ID NOs. 153-158, 161, 163-164, 166-173, 176-187, 189, 191-198 or 202-206.

In some examples, the microbial host cell is E. coli.

In some examples, the heterologous gene encodes a protein product of interest found in Table 2.

In some examples, the heterologous gene is a gene that is part of a lysine biosynthetic pathway.

In some examples, the heterologous gene is selected from the asd gene, the ask gene, the hom gene, the dapA gene, the dapB gene, the dapD gene, the ddh gene, the argD gene, the dapE gene, the dapF gene, the lysA gene, the lysE gene, the zwf gene, the pgi gene, the ktk gene, the fbp gene, the ppc gene, the pck gene, the ddx gene, the pyc gene or the icd gene.

In some examples, the heterologous gene is a gene that is part of a lycopene biosynthetic pathway.

In some examples, the heterologous gene is selected from the dxs gene, the ispC gene, the ispE gene, the ispD gene, the ispF gene, the ispG gene, the ispH gene, the idi gene, the ispA gene, the ispB gene, the crtE gene, the crtB gene, the crtI gene, the crtY gene, the ymgA gene, the dxr gene, the elbA gene, the gdhA gene, the appY gene, the elbB gene, or the ymgB gene.

In some examples, the heterologous gene encodes a biopharmaceutical or is a gene in a pathway for generating a biopharmaceutical.

In some examples, the biopharmaceutical is selected from humulin (rh insulin), intronA (interferon alpha2b), roferon (interferon alpha2a), humatrope (somatropin rh growth hormone), neupogen (filgrastim), detaferon (interferon beta-lb), lispro (fast-acting insulin), rapilysin (reteplase), infergen (interferon alfacon-1), glucagon, beromun (tasonermin), ontak (denileukin diftitox), lantus (long-acting insulin glargine), kineret (anakinra), natrecor (nesiritide), somavert (pegvisomant), calcitonin (recombinant calcitonin salmon), lucentis (ranibizumab), preotact (human parathyroid hormone), kyrstexxal (rh urate oxidase, PEGlyated), nivestim (filgrastim, rhGCSF), voraxaze (glucarpidase), or preos (parathyroid hormone).

In some examples, the present disclosure teaches a heterologous gene operably linked to a chimeric synthetic promoter with a nucleic acid sequence selected from SEQ ID NOs. 132-207.

In some examples, the heterologous gene encodes a protein product of interest found in Table 2.

In some examples, the heterologous gene is a gene that is part of a lysine biosynthetic pathway.

In some examples, the heterologous gene is selected from the asd gene, the ask gene, the hom gene, the dapA gene, the dapB gene, the dapD gene, the ddh gene, the argD gene, the dapE gene, the dapF gene, the lysA gene, the lysE gene, the zwf gene, the pgi gene, the ktk gene, the fbp gene, the ppc gene, the pck gene, the ddx gene, the pyc gene or the icd gene.

In some examples, the heterologous gene is a gene that is part of a lycopene biosynthetic pathway.

In some examples, the heterologous gene is selected from the dxs gene, the ispC gene, the ispE gene, the ispD gene, the ispF gene, the ispG gene, the ispH gene, the idi gene, the ispA gene, the ispB gene, the crtE gene, the crtB gene, the crtI gene, the crtY gene, the ymgA gene, the dxr gene, the elbA gene, the gdhA gene, the appY gene, the elbB gene, or the ymgB gene.

In some examples, the heterologous gene encodes a biopharmaceutical or is a gene in a pathway for generating a biopharmaceutical.

In some examples, the biopharmaceutical is selected from humulin (rh insulin), intronA (interferon alpha2b), roferon (interferon alpha2a), humatrope (somatropin rh growth hormone), neupogen (filgrastim), detaferon (interferon beta-lb), lispro (fast-acting insulin), rapilysin (reteplase), infergen (interferon alfacon-1), glucagon, beromun (tasonermin), ontak (denileukin diftitox), lantus (long-acting insulin glargine), kineret (anakinra), natrecor (nesiritide), somavert (pegvisomant), calcitonin (recombinant calcitonin salmon), lucentis (ranibizumab), preotact (human parathyroid hormone), kyrstexxal (rh urate oxidase, PEGlyated), nivestim (filgrastim, rhGCSF), voraxaze (glucarpidase), or preos (parathyroid hormone).

In a first aspect of the invention there is provided a method for generating a promoter swap *E. coli* strain library, comprising the steps of:

a. providing a plurality of target genes endogenous to a base *E. coli* strain, and a promoter ladder, wherein said promoter ladder comprises a plurality of promoters exhibiting different expression profiles in the base *E. coli* strain, wherein at least one of the plurality of promoters is a chimeric synthetic promoter, wherein the chimeric synthetic promoter is 60-90 nucleotides in length and consists of a distal portion of lambda phage $p_R$ promoter, variable -35 and -10 regions of lambda phage $p_L$ and $p_R$ promoters that are each six nucleotides in length, core portions of lambda phage $p_L$ and $p_R$ promoters and a 5' UTR/Ribosomal Binding Site (RBS) portion that is the 5' UTR/RBS portion of lambda phage $p_R$ promoter or the 5' UTR/Ribosomal Binding Site (RBS) portion of the promoter of the *E. coli acs* gene; and
b. engineering the genome of the base *E. coli* strain, to thereby create an initial promoter swap *E. coli* strain library comprising a plurality of individual *E. coli* strains with unique genetic variations found within each strain of said plurality of individual *E. coli* strains, wherein each of said unique genetic variations comprises one or more of the promoters from the promoter ladder operably linked to one of the target genes endogenous to the base *E. coli* strain.

In a second aspect of the invention there is provided a method for improving the phenotypic performance of a production *E. coli* strain, comprising the steps of:

c. providing a plurality of target genes endogenous to a base *E. coli* strain, and a promoter ladder, wherein said promoter ladder comprises a plurality of promoters exhibiting different expression profiles in the base *E. coli* strain, wherein at least one of the plurality of promoters is a chimeric synthetic promoter, wherein the chimeric synthetic promoter is 60-90 nucleotides in length and consists of a distal portion of lambda phage $p_R$ promoter, variable -35 and -10 regions of lambda phage $p_L$ and $p_R$ promoters that are each six nucleotides in length, core portions of lambda phage $p_L$ and $p_R$ promoters and a 5' UTR/Ribosomal Binding Site (RBS) portion that is the 5' UTR/RBS portion of lambda phage $p_R$ promoter or the 5' UTR/Ribosomal Binding Site (RBS) portion of the promoter of the *E. coli acs* gene;
d. engineering the genome of the base *E. coli* strain, to thereby create an initial promoter swap *E. coli* strain library comprising a plurality of individual *E. coli* strains with unique genetic variations found within each strain of said plurality of individual *E. coli* strains, wherein each of said unique genetic variations comprises one or more of the promoters from the promoter ladder operably linked to one of the target genes endogenous to the base *E. coli* strain;
e. screening and selecting individual *E. coli* strains of the initial promoter swap *E. coli* strain library for phenotypic performance improvements over a reference *E. coli* strain, thereby identifying unique genetic variations that confer phenotypic performance improvements;
f. providing a subsequent plurality of *E. coli* microbes that each comprise a combination of unique genetic variations from the genetic variations present in at least two individual *E. coli* strains screened in the preceding step, to thereby create a subsequent promoter swap *E. coli* strain library;
g. screening and selecting individual *E. coli* strains of the subsequent promoter swap *E. coli* strain library for phenotypic performance improvements over the reference *E. coli* strain, thereby identifying unique combinations of genetic variation that confer additional phenotypic performance improvements; and
h. repeating steps d)-e) one or more times, in a linear or non-linear fashion, until an *E. coli* strain exhibits a desired level of improved phenotypic performance compared to the phenotypic performance of the production *E. coli* strain, wherein each subsequent iteration creates a new promoter swap *E. coli* strain library of microbial strains, where each strain in the new library comprises genetic variations that are a combination of genetic variations selected from amongst at least two individual *E. coli* strains of a preceding library.

Preferred embodiments of the invention in any of its various aspects are as described below or as defined in the sub claims.

## BRIEF DESCRIPTION OF THE FIGURES

**FIGURE 1** depicts a DNA recombination method of the present disclosure for increasing variation in diversity pools. DNA sections, such as genome regions from related species, can be cut *via* physical or enzymatic/chemical means. The cut DNA regions are melted and allowed to reanneal, such that overlapping genetic regions prime polymerase extension reactions. Subsequent melting/extension reactions are carried out until products are reassembled into chimeric DNA, comprising elements from one or more starting sequences.

**FIGURE 2** outlines methods of the present disclosure for generating new host *E. coli* strains with selected sequence modifications (e.g., 100 SNPs to swap). Briefly, the method comprises (1) desired DNA inserts are designed and generated by combining one or more synthesized oligos in an assembly reaction, (2) DNA inserts are cloned into transformation plasmids, (3) completed plasmids are transferred into desired production strains, where they are integrated into the host strain genome, and (4) selection markers and other unwanted DNA elements are looped out of the host strain. Each DNA assembly step may involve additional quality control (QC) steps, such as cloning plasmids into *E. coli* bacteria for amplification and sequencing.

**FIGURE 3** depicts assembly of transformation plasmids of the present disclosure, and their integration into a host *E. coli* genome. The insert DNA is generated by combining one or more synthesized oligos in an assembly reaction. DNA inserts containing the desired sequence are flanked by regions of DNA homologous to the targeted region of the genome. These homologous regions facilitate genomic integration, and, once integrated, form direct repeat regions designed for looping out vector backbone DNA in subsequent steps. Assembled plasmids contain the insert DNA, and optionally, one or more selection markers.

**FIGURE 4** depicts a procedure for looping-out selected regions of DNA from host *E. coli* strains. Direct repeat regions of the inserted DNA and host genome can "loop out" in a recombination event. Cells counter selected for the selection marker contain deletions of the loop DNA flanked by the direct repeat regions.

**FIGURE 5** depicts an example of the *E. coli* strain improvement process of the present disclosure. Host strain sequences containing genetic modifications (Genetic Design) are tested for strain performance improvements in various strain backgrounds (Strain Build). Strains exhibiting beneficial mutations are analyzed (Hit ID and Analysis) and the data is stored in libraries for further analysis (e.g., SNP swap libraries, PRO swap libraries, and combinations thereof, among others). Selection rules of the present disclosure generate new proposed *E. coli* host strain sequences based on the predicted effect of combining elements from one or more libraries for additional iterative analysis.

**FIGURE 6A-B** depicts the DNA assembly, transformation, and *E. coli* strain screening steps of one of the examples of the present disclosure. **FIGURE 6A** depicts the steps for building DNA fragments, cloning said DNA fragments into vectors, transforming said vectors into host *E. coli* strains, and looping out selection sequences through counter selection. **FIGURE 6B** depicts the steps for high-throughput culturing, screening, and evaluation of selected *E. coli* host strains. This figure also depicts the optional steps of culturing, screening, and evaluating selected *E. coli* strains in culture tanks.

**FIGURE 7** depicts one example of the automated system of the present disclosure. The present disclosure teaches use of automated robotic systems with various modules capable of cloning, transforming, culturing, screening and/or sequencing host *E. coli.*

**FIGURE 8** depicts an overview of an example of the *E. coli* strain improvement program of the present disclosure.

**FIGURE 9** is a representation of the genome of *Corynebacterium glutamicum,* comprising around 3.2 million base pairs.

**FIGURE 10** depicts the results of a transformation experiment of the present disclosure. DNA inserts ranging from 0.5kb to 5.0kb were targeted for insertion into various regions (shown as relative positions 1-24) of the genome of *Corynebacterium glutamicum.* Light color indicates successful integration, while darker color indicates insertion failure.

**FIGURE 11** depicts the results of a second round HTP engineering PRO swap program. Top promoter: :gene combinations identified during the first PRO swap round were analyzed according to the methods of the present disclosure to identify combinations of said mutations that would be likely to exhibit additive or combinatorial beneficial effects on host performance. Second round PRO swap mutants thus comprised pair combinations of various promoter:: gene mutations. The resulting second round mutants were screened for differences in host cell yield of a selected biomolecule. A combination pair of mutations that had been predicted to exhibit beneficial effects is emphasized with a circle.

**FIGURE 12** depicts the results of an experiment testing successful plasmid assembly for plasmids transformed into *E. coli.* Picking four colonies is sufficient to achieve 13% failure rate for plasmids containing 1 and 2kb insertion sequences. Larger insertions may require additional colony screening to achieve consistent results.

**FIGURE 13** depicts results of an experiment testing successful transformation of *Corynebacterium glutamicum* with insertion vectors. DNA insert sizes of 2 and 5 kb exhibited high transformation rates with low assembly failure rates.

**FIGURE 14** depicts results of loop out selections in *Corynebacterium glutamicum.* Sucrose resistance of transformed bacteria indicates loop out of sacB selection marker. DNA insert size does not appear to impact loop out efficiency.

**FIGURE 15** is a similarity matrix computed using the correlation measure. The matrix is a representation of the functional similarity between SNP variants. The consolidation of SNPs with low functional similarity is expected to have a higher likelihood of improving strain performance, as opposed to the consolidation of SNPs with higher functional similarity.

**FIGURE 16A-B** depicts the results of an epistasis mapping experiment. Combination of SNPs and PRO swaps with low functional similarities yields improved strain performance. **FIGURE 16A** depicts a dendrogram clustered by functional similarity of all the SNPs/PRO swaps. **FIGURE 16B** depicts host strain performance of consolidated SNPs as measured by product yield. Greater cluster distance correlates with improved consolidation performance of the host strain.

**FIGURE 17A-B** depicts SNP differences among strain variants in the diversity pool. **FIGURE 17A** depicts the relationship among the strains of this experiment. Strain A is the wild-type host strain. Strain B is an intermediate engineered strain. Strain C is the industrial production strain. **FIGURE 17B** is a graph identifying the number of unique and shared SNPs in each strain.

**FIGURE 18** depicts a first-round SNP swapping experiment according to the methods of the present disclosure. (1) all the SNPs from C will be individually and/or combinatorially cloned into the base A strain ("wave up" A to C). (2) all the SNPs from C will be individually and/or combinatorially removed from the commercial strain C ("wave down" C to A). (3) all the SNPs from B will be individually and/or combinatorially cloned into the base A strain (wave up A to B). (4) all the SNPs from B will be individually and/or combinatorially removed from the commercial strain B (wave down B to A). (5) all the SNPs unique to C will be individually and/or combinatorially cloned into the commercial B strain (wave up B to C). (6) all the SNPs unique to C will be individually and/or combinatorially removed from the commercial strain C (wave down C to B).

**FIGURE 19** illustrates example gene targets to be utilized in a promoter swap process. The 4 underlined are diverting genes that can be targeted for downregulaton, while the remaining 19 on pathway genes can be targeted for overexpression.

**FIGURE 20** illustrates an exemplary promoter library that is being utilized to conduct a promoter swap process for the identified gene targets. Promoters utilized in the PRO swap (*i.e.* promoter swap) process are $P_1$-$P_8$, the sequences and identity of which can be found in Table 1.

**FIGURE 21** illustrates the different available approaches to promoter swapping depending on whether the targeted gene comprises its own promoter, or is part of an operon.

**FIGURE 22** depicts exemplary HTP promoter swapping data showing modifications that significantly affect performance on lysine yield. The X-axis represents different strains within the promoter swap genetic design microbial strain library, and the Y-axis includes relative lysine yield values for each strain. Each letter on the graph represents a PRO swap target gene. Each data point represents a replicate. The data demonstrates that a molecular tool adapted for HTP applications, as described herein (*i.e.* PRO swap), is able to efficiently create and optimize microbial strain performance for the production of a compound or molecule of interest. In this case, the compound of interest was lysine; however, the taught PRO swap molecular tool can be utilized to optimize and/or increase the production of any compound of interest. One of skill in the art would understand how to choose target genes, encoding the production of a desired compound, and then utilize the taught PRO swap procedure. One of skill in the art would readily appreciate that the demonstrated data exemplifying lysine yield increases taught herein, along with the detailed disclosure presented in the application, enables the PRO swap molecular tool to be a widely applicable advancement in HTP genomic engineering.

**FIGURE 23** illustrates the distribution of relative strain performances for the input data under consideration. A relative performance of zero indicates that the engineered strain performed equally well to the in-plate base strain. The processes described herein are designed to identify the strains that are likely to perform significantly above zero.

**FIGURE 24** illustrates the linear regression coefficient values, which depict the average change (increase or decrease) in relative strain performance associated with each genetic change incorporated into the depicted strains.

**FIGURE 25** illustrates the composition of changes for the top 100 predicted strain designs. The x-axis lists the pool of potential genetic changes (dss mutations are SNP swaps, and Pcg mutations are PRO swaps), and the y-axis shows the rank order. Black cells indicate the presence of a particular change in the candidate design, while white cells indicate the absence of that change. In this particular example, all of the top 100 designs contain the changes pcg3121_pgi, pcg1860_pyc, dss_339, and pcg0007_39_lysa. Additionally, the top candidate design contains the changes dss_034, dss_009.

**FIGURE 26** depicts the DNA assembly and transformation steps of one of the examples of the present disclosure.

The flow chart depicts the steps for building DNA fragments, cloning said DNA fragments into vectors, transforming said vectors into host *E. coli* strains, and looping out selection sequences through counter selection.

**FIGURE 27** depicts the steps for high-throughput culturing, screening, and evaluation of selected host *E. coli* strains. This figure also depicts the optional steps of culturing, screening, and evaluating selected *E. coli* strains in culture tanks.

**FIGURE 28** depicts expression profiles of illustrative promoters exhibiting a range of regulatory expression, according to the promoter ladders of the present disclosure. Promoter A expression peaks at the lag phase of bacterial cultures, while promoter B and C peak at the exponential and stationary phase, respectively.

**FIGURE 29** depicts expression profiles of illustrative promoters exhibiting a range of regulatory expression, according to the promoter ladders of the present disclosure. Promoter A expression peaks immediately upon addition of a selected substrate, but quickly returns to undetectable levels as the concentration of the substrate is reduced. Promoter B expression peaks immediately upon addition of the selected substrate and lowers slowly back to undetectable levels together with the corresponding reduction in substrate. Promoter C expression peaks upon addition of the selected substrate, and remains highly expressed throughout the culture, even after the substrate has dissipated.

**FIGURE 30** depicts expression profiles of illustrative promoters exhibiting a range of constitutive expression levels, according to the promoter ladders of the present disclosure. Promoter A exhibits the lowest expression, followed by increasing expression levels promoter B and C, respectively.

**FIGURE 31** diagrams an example of LIMS system of the present disclosure for *E. coli* strain improvement.

**FIGURE 32** diagrams a cloud computing implementation of examples of the LIMS system of the present disclosure.

**FIGURE 33** depicts an example of the iterative predictive strain design workflow of the present disclosure.

**FIGURE 34** diagrams an example of a computer system, according to examples of the present disclosure.

**FIGURE 35** depicts the workflow associated with the DNA assembly according to one example of the present disclosure. This process is divided up into 4 stages: parts generation, plasmid assembly, plasmid QC, and plasmid preparation for transformation. During parts generation, oligos designed by Laboratory Information Management System (LIMS) are ordered from an oligo sequencing vendor and used to amplify the target sequences from the host organism via PCR. These PCR parts are cleaned to remove contaminants and assessed for success by fragment analysis, *in silico* quality control comparison of observed to theoretical fragment sizes, and DNA quantification. The parts are transformed into yeast along with an assembly vector and assembled into plasmids via homologous recombination. Assembled plasmids are isolated from yeast and transformed into *E. coli* for subsequent assembly quality control and amplification. During plasmid assembly quality control, several replicates of each plasmid are isolated, amplified using Rolling Circle Amplification (RCA), and assessed for correct assembly by enzymatic digest and fragment analysis. Correctly assembled plasmids identified during the QC process are hit picked to generate permanent stocks and the plasmid DNA extracted and quantified prior to transformation into the target host organism.

**FIGURE 36** depicts the results of an experiment characterizing the effects of Terminators T1-T8 in two media over two time points. Conditions A and C represent the two time points for the BHI media, while the B and D points represent the two time points for the HTP test media.

**FIGURE 37** depicts the results of an experiment comparing the effectiveness of traditional strain improvement approaches such as UV mutagenesis against the HTP engineering methodologies of the present disclosure. The vast majority of UV mutations produced no noticeable increase in host cell performance. In contrast, PRO swap methodologies of the present disclosure produced a high proportion of mutants exhibiting 1.2 to 2 fold increases in host cell performance.

**FIGURE 38** depicts the results of a first round HTP engineering SNP swap program. 186 individual SNP mutations were identified and individually cloned onto a base strain. The resulting mutants were screened for differences in host cell yield of a selected biomolecule.

**FIGURE 39** depicts the results of a second round HTP engineering SNP swap program. 176 individual SNP mutations from a first round SNP swap program were individually cloned into a second round host cell strain containing a beneficial SNP identified during a first round SNP program. The resulting mutants thus represent the effect of two mutation combination pairs. Screening results for differences in host cell yield (Y-axis) and productivity (X-axis) for the selected biomolecule are shown.

**FIGURE 40** depicts the results of a tank fermentation validation experiment. The top mutation pairs from the second round of HTP SNP swap were cultured in fermentation tanks. Results for host cell yield and productivity for the selected biomolecule (*i.e.* lysine) are shown. As can be seen, in one round of genomic engineering the inventors utilized the PRO swap procedure to determine that a particular PRO swap mutant (zwf) exhibited increased yield of a selected biomolecule compared to base strain (*i.e.* compare base strain to base strain + zwf). Then, the inventors performed another round of genomic engineering, wherein a SNP swap procedure was used to determine beneficial SNP mutations that could affect yield of the biomolecule, when combined with said PRO swap mutant. The combination of the PRO swap procedure and SNP swap procedure created mutants with even higher yields than the previous PRO swap only mutants (*i.e.* compare base strain + zwf + SNP121 to the previously discussed base strain + zwf). This figure illustrates the dramatic improvements in yield that can be achieved by combining the PRO swap and SNP swap procedures of the disclosure. In examples, combining a PRO swap genomic engineering campaign with a SNP swap genomic engineering campaign can lead to increased yield and/or productivity of a biomolecule/product of interest by a factor of 1%, 2%, 3%, 4%, 5%, 6%, 7%, 8%, 9% 10%, 15%, 20%, 25%, 30%, 40%, 45%, 50%, or more, relative to a base strain.

**FIGURE 41** depicts the results of a first round HTP engineering PRO swap program. Selected genes believed to be associated with host performance were combined with a promoter ladder to create a first round PRO swap library, according to the methods of the present disclosure. The resulting mutants were screened for differences in host cell yield of a selected biomolecule (*i.e.* lysine).

**FIGURE 42** is a flowchart illustrating the consideration of epistatic effects in the selection of mutations for the design of a microbial strain, according to examples of the disclosure.

**FIGURE 43** depicts a Bicistronic Design (BCD) regulatory sequence, according to the present disclosure. In some examples, the present disclosure teaches that BCDs can be used in place of traditional promoters in order to improve expression consistency between different promoter: :target genes combinations in PRO-swaps. In some examples, BCDs comprise a promoter, a first ribosome binding site (SD1), a first cistronic sequence (Cis1), a second ribosome binding site (SD2), operably linked to a target gene of interest (Cis2). In some examples, the present disclosure teaches that Cis1 can be any peptide-coding sequence. Additional information about BCD design and use is provided in later sections of the specification.

**FIGURE 44** is an illustration of pathway enzyme co-localization via recombinant DNA binding domains. An engineered cell encodes pathway enzymes Enz1-3 with DNA binding domains. When expressed, these enzymes bind to a scaffold DNA or other target location, which comprises DNA motifs that are recognized by the recombinant DNA binding domains fused to the pathway enzymes. When the fused DNA binding domains attach to their cognate DNA motifs on the scaffold plasmid, the enzymes are constrained close to one another in space, which can improve productivity of the pathway.

**FIGURE 45** is a schematic diagram for incorporation of nucleotide sequences encoding DNA binding domains into pathway enzymes. GOI encodes a pathway enzyme. *E. coli* cells are transformed with a plasmid encoding a mutant version of GOI that includes a nucleotide sequence that encodes a DNA binding domain (denoted with a star). The plasmid also encodes an antibiotic resistance marker (Ab) that allows for selection of loop in cells, and a counterselection marker (Counter) that allows for subsequent counterselection of loop out cells. In the "Loop-in" step, the entire plasmid, including the mutant GOI, is incorporated into the genome via homologous recombination (HR). During the "Loop-out" step, some of the cells will revert to the native GOI sequence via HR, while others will undergo a HR event that leaves the mutant GOI in the genome.

**FIGURE 46** is a dot plot for the predicted performance vs measured performance of training data for a yield model of the present disclosure. The underlying model is a Kernel Ridge Regression model (with 4th order polynomial kernel). The model is trained on 1864 unique genetic constructs and associated phenotypic performance. The fitted model has an r2 value of 0.52.

**FIGURE 47** depicts the genetic makeup of candidate designs generated by the prediction algorithms of the present disclosure. These candidate designs were submitted for HTP build and analysis. Here the candidate design is defined as the combination of parent strain id and introduced mutation(s).

**FIGURE 48** is a dot plot of the predicted performance vs. measured performance of candidate designs generated by the prediction algorithms of the present disclosure, and built according the HTP build methods of the present disclosure. This figure demonstrates that the model may predict candidate strain performance within an acceptable degree of accuracy.

**FIGURE 49** is a box and whiskers plot depicting the yield percent change of candidate strains with respect to parent strains. On the y-axis, a value of 0.01 corresponds to 1%. This figure demonstrates that strains designed by a computer model (light gray) achieve measureable improvement over their corresponding parent strains. Additionally, the figure demonstrates that these model base strain improvements are comparable in magnitude to improvements achieved by human expert designed strains.

**FIGURE 50** illustrates the yield performance distribution for strains designed by the computer model (dark grey) and by a human expert (light grey). Computer-designed strains exhibited tighter distributions with higher median gains.

**FIGURE 51** is a box and whiskers plot depicting the absolute yield of candidate strains generated by the computer (light grey) or by a human expert (dark grey). Results are aggregated by parent strain.

**FIGURE 52** is a representation of the genome of *Escherichia coli,* comprising around 4.6 million base pairs.

**FIGURE 53** illustrates the effect of insulator and terminator parts in vector backbones on the efficiency of transformation and plasmid integration.

**FIGURE 54** illustrates the combinatorial design of synthetic promoter-5'UTR sequences from Table 1.4.

**FIGURE 55** depicts the plasmid map illustrating the components of the vector 1 backbone.

**FIGURE 56** depicts the plasmid map illustrating the components of the vector 2 backbone.

**FIGURE 57** depicts the plasmid map illustrating the components of the vector 3 backbone.

**FIGURE 58** depicts the plasmid map illustrating the components of the vector 4 backbone.

**FIGURE 59** depicts the E. coli lycopene biosynthetic pathway.

**FIGURE 60** depicts terminator edits at lycopene pathway targets *idi and ymgA.* The terminator TyjbE demonstrates decreased strain performance relative to the control, thus highlighting the utility of these library types for identifying critical pathway targets.

**FIGURE 61** depicts terminator edits at multiple lycopene pathway targets.

**FIGURE 62** depicts promoter (for comparison), degradation tag, and terminator swaps at lycopene pathway target *dxs.*The ssrA_LAA degradation tag demonstrates improved strain performance relative to the control. This is unexpected as this strain is a combination of a PROSWP with a degradation tag at a single pathway target. The initial PROSWP is expected to increase protein abundance, and the degradation tag is expected to decrease protein abundance, thus demonstrating the utility of combinations of library types for tuning optimal strain performance.

**FIGURE 63** depicts solubility tag, promoter, and terminator swaps at lycopene pathway target *gdhA.* The solubility tag FH8 demonstrates improved strain performance relative to the control, but the GB1 solubility tag does not, thus demonstrating the necessity for evaluating libraries of each modification type.

## DETAILED DESCRIPTION

*Definitions*

While the following terms are believed to be well understood by one of ordinary skill in the art, the following definitions are set forth to facilitate explanation of the presently disclosed subject matter.

The term "a" or "an" refers to one or more of that entity, *i.e.* can refer to a plural referents. As such, the terms "a" or "an", "one or more" and "at least one" are used interchangeably herein. In addition, reference to "an element" by the indefinite article "a" or "an" does not exclude the possibility that more than one of the elements is present, unless the context clearly requires that there is one and only one of the elements.

As used herein the terms "cellular organism" "microorganism" or "microbe" should be taken broadly. These terms are used interchangeably and include, but are not limited to, the two prokaryotic domains, Bacteria and Archaea, as well as certain eukaryotic fungi and protists. In some examples, the disclosure refers to the "microorganisms" or "cellular organisms" or "microbes" of lists/tables and figures present in the disclosure. This characterization can refer to not only the identified taxonomic genera of the tables and figures, but also the identified taxonomic species, as well as the various novel and newly identified or designed strains of any organism in said tables or figures. The same characterization holds true for the recitation of these terms in other parts of the Specification, such as in the Examples.

The term "prokaryotes" is art recognized and refers to cells which contain no nucleus or other cell organelles. The prokaryotes are generally classified in one of two domains, the Bacteria and the Archaea. The definitive difference between organisms of the Archaea and Bacteria domains is based on fundamental differences in the nucleotide base sequence in the 16S ribosomal RNA.

The term "Archaea" refers to a categorization of organisms of the division Mendosicutes, typically found in unusual environments and distinguished from the rest of the prokaryotes by several criteria, including the number of ribosomal proteins and the lack of muramic acid in cell walls. On the basis of ssrRNA analysis, the Archaea consist of two phylogenetically-distinct groups: Crenarchaeota and Euryarchaeota. On the basis of their physiology, the Archaea can be organized into three types: methanogens (prokaryotes that produce methane); extreme halophiles (prokaryotes that live at very high concentrations of salt (NaCl); and extreme (hyper) thermophilus (prokaryotes that live at very high temperatures). Besides the unifying archaeal features that distinguish them from Bacteria (*i.e.,* no murein in cell wall, ester-linked membrane lipids, etc.), these prokaryotes exhibit unique structural or biochemical attributes which adapt them to their particular habitats. The Crenarchaeota consists mainly of hyperthermophilic sulfur-dependent prokaryotes and the Euryarchaeota contains the methanogens and extreme halophiles.

"Bacteria" or "eubacteria" refers to a domain of prokaryotic organisms. Bacteria include at least 11 distinct groups as follows: (1) Gram-positive (gram+) bacteria, of which there are two major subdivisions: (1) high G+C group (*Actinomycetes, Mycobacteria, Micrococcus,* others) (2) low G+C group (*Bacillus, Clostridia, Lactobacillus, Staphylococci, Streptococci, Mycoplasmas*); (2) Proteobacteria, e.g., Purple photosynthetic+non-photosynthetic Gram-negative bacteria (includes most "common" Gram-negative bacteria); (3) Cyanobacteria, e.g., oxygenic phototrophs; (4) Spirochetes and related species; (5) Planctomyces; (6) *Bacteroides, Flavobacteria;* (7) *Chlamydia;* (8) Green sulfur bacteria; (9) Green non-sulfur bacteria (also anaerobic phototrophs); (10) Radioresistant micrococci and relatives; (11) *Thermotoga* and *Thermosipho thermophiles.*

A "eukaryote" is any organism whose cells contain a nucleus and other organelles enclosed within membranes.

Eukaryotes belong to the taxon Eukarya or Eukaryota. The defining feature that sets eukaryotic cells apart from prokaryotic cells (the aforementioned Bacteria and Archaea) is that they have membrane-bound organelles, especially the nucleus, which contains the genetic material, and is enclosed by the nuclear envelope.

The terms "genetically modified host cell," "recombinant host cell," and "recombinant strain" are used interchangeably herein and refer to host cells that have been genetically modified by the cloning and transformation methods of the present disclosure. Thus, the terms include a host cell (e.g., bacteria, yeast cell, fungal cell, CHO, human cell, etc.) that has been genetically altered, modified, or engineered, such that it exhibits an altered, modified, or different genotype and/or phenotype (e.g., when the genetic modification affects coding nucleic acid sequences of the microorganism), as compared to the naturally-occurring organism from which it was derived. It is understood that in some examples, the terms refer not only to the particular recombinant host cell in question, but also to the progeny or potential progeny of such a host cell

The term "wild-type microorganism" or "wild-type host cell" describes a cell that occurs in nature, *i.e.* a cell that has not been genetically modified.

The term "genetically engineered" may refer to any manipulation of a host cell's genome (*e.g.* by insertion, deletion, mutation, or replacement of nucleic acids).

The term "control" or "control host cell" refers to an appropriate comparator host cell for determining the effect of a genetic modification or experimental treatment. In some examples, the control host cell is a wild type cell. In other examples, a control host cell is genetically identical to the genetically modified host cell, save for the genetic modification(s) differentiating the treatment host cell. In some examples, the present disclosure teaches the use of parent strains as control host cells (e.g., the Si strain that was used as the basis for the strain improvement program). In other examples, a host cell may be a genetically identical cell that lacks a specific promoter or SNP being tested in the treatment host cell.

As used herein, the term "allele(s)" means any of one or more alternative forms of a gene, all of which alleles relate to at least one trait or characteristic. In a diploid cell, the two alleles of a given gene occupy corresponding loci on a pair of homologous chromosomes.

As used herein, the term "locus" (loci plural) means a specific place or places or a site on a chromosome where for example a gene or genetic marker is found.

As used herein, the term "genetically linked" refers to two or more traits that are co-inherited at a high rate during breeding such that they are difficult to separate through crossing.

A "recombination" or "recombination event" as used herein refers to a chromosomal crossing over or independent assortment.

As used herein, the term "phenotype" refers to the observable characteristics of an individual cell, cell culture, organism, or group of organisms which results from the interaction between that individual's genetic makeup (*i.e.,* genotype) and the environment.

As used herein, the term "chimeric" or "recombinant" when describing a nucleic acid sequence or a protein sequence refers to a nucleic acid, or a protein sequence, that links at least two heterologous polynucleotides, or two heterologous polypeptides, into a single macromolecule, or that re-arranges one or more elements of at least one natural nucleic acid or protein sequence. For example, the term "recombinant" can refer to an artificial combination of two otherwise separated segments of sequence, *e.g.*, by chemical synthesis or by the manipulation of isolated segments of nucleic acids by genetic engineering techniques.

As used herein, a "synthetic nucleotide sequence" or "synthetic polynucleotide sequence" is a nucleotide sequence that is not known to occur in nature or that is not naturally occurring. Generally, such a synthetic nucleotide sequence will comprise at least one nucleotide difference when compared to any other naturally occurring nucleotide sequence.

As used herein, the term "nucleic acid" refers to a polymeric form of nucleotides of any length, either ribonucleotides or deoxyribonucleotides, or analogs thereof. This term refers to the primary structure of the molecule, and thus includes double- and single-stranded DNA, as well as double- and single-stranded RNA. It also includes modified nucleic acids such as methylated and/or capped nucleic acids, nucleic acids containing modified bases, backbone modifications, and the like. The terms "nucleic acid" and "nucleotide sequence" are used interchangeably.

As used herein, the term "DNA scaffold" or "nucleic acid scaffold" refers to a nucleic acid scaffold that is either artificially produced or a naturally occurring sequence that is repurposed as a scaffold. In one example of the present disclosure, the nucleic acid scaffold is a synthetic deoxyribonucleic acid scaffold. The deoxyribonucleotides of the synthetic scaffold may comprise purine and pyrimidine bases or other natural, chemically or biochemically modified, non-natural, or derivatized deoxyribonucleotide bases. As described in more detail herein, the nucleic acid scaffold of the present disclosure is utilized to spatially and temporally assemble and immobilize two or more proteins involved in a biological pathway, i.e. biosynthetic enzymes, to create a functional complex. The assembly and immobilization of each biological pathway protein on the scaffold occurs via the binding interaction between one of the protein-binding sequences, i.e., protein docking sites, of the scaffold and a corresponding DNA-binding portion of a chimeric biosynthetic enzyme. Accordingly, the nucleic acid scaffold comprises one or more subunits, each subunit comprising two or more protein-binding sequences to accommodate the binding of two or more different chimeric biological pathway proteins.

As used herein, a "DNA binding sequence" or "DNA binding site" refers to a specific nucleic acid sequence that is recognized and bound by a DNA-binding domain portion of a chimeric biosynthetic gene (e.g., chimeric biosynthetic enzyme) encoded by modified genes of the present disclosure. Many DNA-binding domains and their cognate binding partner DNA recognition sites (i.e., DNA binding sites) are well known in the art. For example, numerous zinc finger binding domains and their corresponding DNA binding target sites are known in the art and suitable for use in the present disclosure. Other DNA binding domains include, without limitation, leucine zipper binding domains and their corresponding DNA binding sites, winged helix DNA binding domains and their corresponding DNA binding sites, winged helix-turn-helix DNA binding domains and their corresponding DNA binding sites, HMG-box DNA binding domains and their corresponding DNA binding sequences, helix-loop-helix DNA binding domains and their corresponding DNA binding sequences, and helix-turn-helix DNA binding domains and their corresponding DNA binding sequences. Other known DNA binding domains with known DNA binding sequences include the immunoglobulin DNA domain, B3 DNA binding domain, and TAL effector DNA binding domains. Nucleic acid scaffold subunits of the present disclosure may comprises any two or more of the aforementioned DNA binding sites.

As used herein, the term "gene" refers to any segment of DNA associated with a biological function. Thus, genes include, but are not limited to, coding sequences and/or the regulatory sequences required for their expression. Genes can also include non-expressed DNA segments that, for example, form recognition sequences for other proteins. Genes can be obtained from a variety of sources, including cloning from a source of interest or synthesizing from known or predicted sequence information, and may include sequences designed to have desired parameters.

As used herein, the term "homologous" or "homologue" or "ortholog" or "orthologue" is known in the art and refers to related sequences that share a common ancestor or family member and are determined based on the degree of sequence identity. The terms "homology," "homologous," "substantially similar" and "corresponding substantially" are used interchangeably herein. They refer to nucleic acid fragments wherein changes in one or more nucleotide bases do not affect the ability of the nucleic acid fragment to mediate gene expression or produce a certain phenotype. These terms also refer to modifications of the nucleic acid fragments of the instant disclosure such as deletion or insertion of one or more nucleotides that do not substantially alter the functional properties of the resulting nucleic acid fragment relative to the initial, unmodified fragment. It is therefore understood, as those skilled in the art will appreciate, that the disclosure encompasses more than the specific exemplary sequences. These terms describe the relationship between a gene found in one species, subspecies, variety, cultivar or strain and the corresponding or equivalent gene in another species, subspecies, variety, cultivar or strain. For purposes of this disclosure homologous sequences are compared. "Homologous sequences" or "homologues" or "orthologs" are thought, believed, or known to be functionally related. A functional relationship may be indicated in any one of a number of ways, including, but not limited to: (a) degree of sequence identity and/or (b) the same or similar biological function. Preferably, both (a) and (b) are indicated. Homology can be determined using software programs readily available in the art, such as those discussed in Current Protocols in Molecular Biology (F.M. Ausubel et al., eds., 1987) Supplement 30, section 7.718, Table 7.71. Some alignment programs are MacVector (Oxford Molecular Ltd, Oxford, U.K.), ALIGN Plus (Scientific and Educational Software, Pennsylvania) and AlignX (Vector NTI, Invitrogen, Carlsbad, CA). Another alignment program is Sequencher (Gene Codes, Ann Arbor, Michigan), using default parameters.

As used herein, the term "endogenous" or "endogenous gene," refers to the naturally occurring gene, in the location in which it is naturally found within the host cell genome. In the context of the present disclosure, operably linking a heterologous promoter to an endogenous gene means genetically inserting a heterologous promoter sequence in front of an existing gene, in the location where that gene is naturally present. An endogenous gene as described herein can include alleles of naturally occurring genes that have been mutated according to any of the methods of the present disclosure.

As used herein, the term "exogenous" is used interchangeably with the term "heterologous," and refers to a substance coming from some source other than its native source. For example, the terms "exogenous protein," or "exogenous gene" refer to a protein or gene from a non-native source or location, and that have been artificially supplied to a biological system.

As used herein, the term "nucleotide change" refers to, *e.g.,* nucleotide substitution, deletion, and/or insertion, as is well understood in the art. For example, mutations contain alterations that produce silent substitutions, additions, or deletions, but do not alter the properties or activities of the encoded protein or how the proteins are made.

As used herein, the term "protein modification" refers to, *e.g.,* amino acid substitution, amino acid modification, deletion, and/or insertion, as is well understood in the art.

As used herein, the term "at least a portion" or "fragment" of a nucleic acid or polypeptide means a portion having the minimal size characteristics of such sequences, or any larger fragment of the full length molecule, up to and including the full length molecule. A fragment of a polynucleotide of the disclosure may encode a biologically active portion of a genetic regulatory element. A biologically active portion of a genetic regulatory element can be prepared by isolating a portion of one of the polynucleotides of the disclosure that comprises the genetic regulatory element and assessing activity as described herein. Similarly, a portion of a polypeptide may be 4 amino acids, 5 amino acids, 6 amino acids, 7 amino acids, and so on, going up to the full length polypeptide. The length of the portion to be used will depend on the particular application. A portion of a nucleic acid useful as a hybridization probe may be as short as 12 nucleotides; in some examples, it is 20 nucleotides. A portion of a polypeptide useful as an epitope may be as short as 4 amino acids. A portion of a polypeptide that performs the function of the full-length polypeptide would generally be longer than 4 amino acids.

Variant polynucleotides also encompass sequences derived from a mutagenic and recombinogenic procedure such as DNA shuffling. Strategies for such DNA shuffling are known in the art. *See,* for example, Stemmer (1994) PNAS 91:10747-10751; Stemmer (1994) Nature 370:389-391; Crameri et al.(1997) Nature Biotech. 15:436-438; Moore et al.(1997) J. Mol. Biol. 272:336-347; Zhang et al.(1997) PNAS 94:4504-4509; Crameri et al.(1998) Nature 391:288-291; and U.S. Patent Nos. 5,605,793 and 5,837,458.

For PCR amplifications of the polynucleotides disclosed herein, oligonucleotide primers can be designed for use in PCR reactions to amplify corresponding DNA sequences from cDNA or genomic DNA extracted from any organism of interest. Methods for designing PCR primers and PCR cloning are generally known in the art and are disclosed in Sambrook et al.(2001) Molecular Cloning: A Laboratory Manual (3rd ed., Cold Spring Harbor Laboratory Press, Plainview, New York). See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Known methods of PCR include, but are not limited to, methods using paired primers, nested primers, single specific primers, degenerate primers, gene-specific primers, vector-specific primers, partially-mismatched primers, and the like.

The term "primer" as used herein refers to an oligonucleotide which is capable of annealing to the amplification target allowing a DNA polymerase to attach, thereby serving as a point of initiation of DNA synthesis when placed under conditions in which synthesis of primer extension product is induced, *i.e.,* in the presence of nucleotides and an agent for polymerization such as DNA polymerase and at a suitable temperature and pH. The (amplification) primer is preferably single stranded for maximum efficiency in amplification. Preferably, the primer is an oligodeoxyribonucleotide. The primer must be sufficiently long to prime the synthesis of extension products in the presence of the agent for polymerization. The exact lengths of the primers will depend on many factors, including temperature and composition (A/T vs. G/C content) of primer. A pair of bi-directional primers consists of one forward and one reverse primer as commonly used in the art of DNA amplification such as in PCR amplification.

As used herein, "promoter" refers to a DNA sequence capable of controlling the expression of a coding sequence or functional RNA. In some examples, the promoter sequence consists of proximal and more distal upstream elements, the latter elements often referred to as enhancers. Accordingly, an "enhancer" is a DNA sequence that can stimulate promoter activity, and may be an innate element of the promoter or a heterologous element inserted to enhance the level or tissue specificity of a promoter. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity.

As used herein, the phrases "recombinant construct", "expression construct", "chimeric construct", "construct", and "recombinant DNA construct" are used interchangeably herein. A recombinant construct comprises an artificial combination of nucleic acid fragments, e.g., regulatory and coding sequences that are not found together in nature. For example, a chimeric construct may comprise regulatory sequences and coding sequences that are derived from different sources, or regulatory sequences and coding sequences derived from the same source, but arranged in a manner different than that found in nature. Such construct may be used by itself or may be used in conjunction with a vector. If a vector is used then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells comprising any of the isolated nucleic acid fragments of the disclosure. The skilled artisan will also recognize that different independent transformation events will result in different levels and patterns of expression (Jones et al., (1985) EMBO J. 4:2411-2418; De Almeida et a/., (1989) Mol. Gen. Genetics 218:78-86), and thus that multiple events must be screened in order to obtain lines displaying the desired expression level and pattern. Such screening may be accomplished by Southern analysis of DNA, Northern analysis of mRNA expression, immunoblotting analysis of protein expression, or phenotypic analysis, among others. Vectors can be plasmids, viruses, bacteriophages, pro-viruses, phagemids, transposons, artificial chromosomes, and the like, that replicate autonomously or can integrate into a chromosome of a host cell. A vector can also be a naked RNA polynucleotide, a naked DNA polynucleotide, a polynucleotide composed of both DNA and RNA within the same strand, a poly-lysine-conjugated DNA or RNA, a peptide-conjugated DNA or RNA, a liposome-conjugated DNA, or the like, that is not autonomously replicating. As used herein, the term "expression" refers to the production of a functional end-product *e.g.,* an mRNA or a protein (precursor or mature).

"Operably linked" means in this context the sequential arrangement of the promoter polynucleotide according to the disclosure with a further oligo- or polynucleotide, resulting in transcription of said further polynucleotide.

The term "product of interest" or "biomolecule" as used herein refers to any product produced by microbes from feedstock. In some cases, the product of interest may be a small molecule, enzyme, peptide, amino acid, organic acid, synthetic compound, fuel, alcohol, etc. For example, the product of interest or biomolecule may be any primary or secondary extracellular metabolite. The primary metabolite may be, inter alia, ethanol, citric acid, lactic acid, glutamic acid, glutamate, lysine, threonine, tryptophan and other amino acids, vitamins, polysaccharides, etc. The secondary metabolite may be, inter alia, an antibiotic compound like penicillin, or an immunosuppressant like cyclosporin A, a plant hormone like gibberellin, a statin drug like lovastatin, a fungicide like griseofulvin, etc. The product of interest or biomolecule may also be any intracellular component produced by a microbe, such as: a microbial enzyme, including: catalase, amylase, protease, pectinase, glucose isomerase, cellulase, hemicellulase, lipase, lactase, streptokinase, and many others. The intracellular component may also include recombinant proteins, such as: insulin, hepatitis B vaccine, interferon, granulocyte colony-stimulating factor, streptokinase and others.

The term "carbon source" generally refers to a substance suitable to be used as a source of carbon for cell growth. Carbon sources include, but are not limited to, biomass hydrolysates, starch, sucrose, cellulose, hemicellulose, xylose, and lignin, as well as monomeric components of these substrates. Carbon sources can comprise various organic compounds in various forms, including, but not limited to polymers, carbohydrates, acids, alcohols, aldehydes, ketones, amino acids, peptides, etc. These include, for example, various monosaccharides such as glucose, dextrose (D-glucose), maltose, oligosaccharides, polysaccharides, saturated or unsaturated fatty acids, succinate, lactate, acetate, ethanol, etc., or mixtures thereof. Photosynthetic organisms can additionally produce a carbon source as a product of photosynthesis. In some examples, carbon sources may be selected from biomass hydrolysates and glucose.

The term "feedstock" is defined as a raw material or mixture of raw materials supplied to a microorganism or fermentation process from which other products can be made. For example, a carbon source, such as biomass or the carbon compounds derived from biomass are a feedstock for a microorganism that produces a product of interest (e.g. small molecule, peptide, synthetic compound, fuel, alcohol, etc.) in a fermentation process. However, a feedstock may contain nutrients other than a carbon source.

The term "volumetric productivity" or "production rate" is defined as the amount of product formed per volume of medium per unit of time. Volumetric productivity can be reported in gram per liter per hour (g/L/h).

The term "specific productivity" is defined as the rate of formation of the product. Specific productivity is herein further defined as the specific productivity in gram product per gram of cell dry weight (CDW) per hour (g/g CDW/h). Using the relation of CDW to $OD_{600}$ for the given microorganism specific productivity can also be expressed as gram product per liter culture medium per optical density of the culture broth at 600 nm (OD) per hour (g/L/h/OD).

The term "yield" is defined as the amount of product obtained per unit weight of raw material and may be expressed as g product per g substrate (g/g). Yield may be expressed as a percentage of the theoretical yield. "Theoretical yield" is defined as the maximum amount of product that can be generated per a given amount of substrate as dictated by the stoichiometry of the metabolic pathway used to make the product.

The term "titre" or "titer" is defined as the strength of a solution or the concentration of a substance in solution. For example, the titre of a product of interest (*e.g.* small molecule, peptide, synthetic compound, fuel, alcohol, etc.) in a fermentation broth is described as g of product of interest in solution per liter of fermentation broth (g/L).

The term "total titer" is defined as the sum of all product of interest produced in a process, including but not limited to the product of interest in solution, the product of interest in gas phase if applicable, and any product of interest removed from the process and recovered relative to the initial volume in the process or the operating volume in the process As used herein, the term "HTP genetic design library" or "library" refers to collections of genetic perturbations according to the present disclosure. In some examples, the libraries of the present disclosure may manifest as i) a collection of sequence information in a database or other computer file, ii) a collection of genetic constructs encoding for the aforementioned series of genetic elements, or iii) host cell strains comprising said genetic elements. In some examples, the libraries of the present disclosure may refer to collections of individual elements (e.g., collections of promoters for PRO swap libraries, collections of terminators for STOP swap libraries, collections of protein solubility tags for SOLUBILITY TAG swap libraries, or collections of protein degradation tags for DEGRADATION TAG swap libraries). In other examples, the libraries of the present disclosure may also refer to combinations of genetic elements, such as combinations of promoter:genes, gene:terminator, or even promoter:gene:terminators. In some examples, the libraries of the present disclosure may alos refer to combinations of promoters, terminators, protein solubility tags and/or protein degradation tags. In some examples, the libraries of the present disclosure further comprise meta data associated with the effects of applying each member of the library in host organisms. For example, a library as used herein can include a collection of promoter::gene sequence combinations, together with the resulting effect of those combinations on one or more phenotypes in a particular species, thus improving the future predictive value of using said combination in future promoter swaps.

As used herein, the term "SNP" refers to Small Nuclear Polymorphism(s). In some examples, SNPs of the present disclosure should be construed broadly, and include single nucleotide polymorphisms, sequence insertions, deletions, inversions, and other sequence replacements. As used herein, the term "non-synonymous" or non-synonymous SNPs" refers to mutations that lead to coding changes in host cell proteins
A "high-throughput (HTP)" method of genomic engineering may involve the utilization of at least one piece of automated equipment (e.g. a liquid handler or plate handler machine) to carry out at least one step of said method.

**Traditional Methods of Strain Improvement**

Traditional approaches to strain improvement can be broadly categorized into two types of approaches: directed strain engineering, and random mutagenesis.

Directed engineering methods of strain improvement involve the planned perturbation of a handful of genetic elements of a specific organism. These approaches are typically focused on modulating specific biosynthetic or developmental programs, and rely on prior knowledge of the genetic and metabolic factors affecting said pathways. In its simplest examples, directed engineering involves the transfer of a characterized trait (e.g., gene, promoter, or other genetic element capable of producing a measurable phenotype) from one organism to another organism of the same, or different species.

Random approaches to strain engineering involve the random mutagenesis of parent strains, coupled with extensive screening designed to identify performance improvements. Approaches to generating these random mutations include exposure to ultraviolet radiation, or mutagenic chemicals such as Ethyl methanesulfonate. Though random and largely unpredictable, this traditional approach to strain improvement had several advantages compared to more directed genetic manipulations. *First,* many industrial organisms were (and remain) poorly characterized in terms of their genetic and metabolic repertoires, rendering alternative directed improvement approaches difficult, if not impossible.

*Second,* even in relatively well characterized systems, genotypic changes that result in industrial performance improvements are difficult to predict, and sometimes only manifest themselves as epistatic phenotypes requiring cumulative mutations in many genes of known and unknown function.

*Additionally,* for many years, the genetic tools required for making directed genomic mutations in a given industrial organism were unavailable, or very slow and/or difficult to use.

The extended application of the traditional strain improvement programs, however, yield progressively reduced gains in a given strain lineage, and ultimately lead to exhausted possibilities for further strain efficiencies. Beneficial random mutations are relatively rare events, and require large screening pools and high mutation rates. This inevitably results in the inadvertent accumulation of many neutral and/or detrimental (or partly detrimental) mutations in "improved" strains, which ultimately create a drag on future efficiency gains.

Another limitation of traditional cumulative improvement approaches is that little to no information is known about any particular mutation’s effect on any strain metric. This fundamentally limits a researcher’s ability to combine and consolidate beneficial mutations, or to remove neutral or detrimental mutagenic "baggage."

Other approaches and technologies exist to randomly recombine mutations between strains within a mutagenic lineage. For example, some formats and examples for iterative sequence recombination, sometimes referred to as DNA shuffling, evolution, or molecular breeding, have been described in U.S. patent application Ser. No. 08/198,431, filed Feb. 17, 1994, Serial No. PCT/US95/02126, filed, Feb. 17, 1995, Ser. No. 08/425,684, filed Apr. 18, 1995, Ser. No. 08/537,874, filed Oct. 30, 1995, Ser. No. 08/564,955, filed Nov. 30, 1995, Ser. No. 08/621,859, filed. Mar. 25, 1996, Ser. No. 08/621,430, filed Mar. 25, 1996, Serial No. PCT/US96/05480, filed Apr. 18, 1996, Ser. No. 08/650,400, filed May 20, 1996, Ser. No. 08/675,502, filed Jul. 3, 1996, Ser. No. 08/721, 824, filed Sep. 27, 1996, and Ser. No. 08/722,660 filed Sep. 27, 1996; Stemmer, Science 270:1510 (1995); Stemmer et al., Gene164:49-53 (1995); Stemmer, Bio/Technology 13:549-553 (1995); Stemmer, Proc. Natl. Acad. Sci. U.S.A. 91:10747-10751 (1994); Stemmer, Nature370:389-391 (1994); Crameri et al., Nature Medicine 2(1):1-3 (1996); Crameri et al., Nature Biotechnology 14:315-319 (1996.

These include techniques such as protoplast fusion and whole genome shuffling that facilitate genomic recombination across mutated strains. For some industrial microorganisms such as yeast and filamentous fungi, natural mating cycles can also be exploited for pairwise genomic recombination. In this way, detrimental mutations can be removed by ’back-crossing’ mutants with parental strains and beneficial mutations consolidated. Moreover, beneficial mutations from two different strain lineages can potentially be combined, which creates additional improvement possibilities over what might be available from mutating a single strain lineage on its own. However, these approaches are subject to many limitations that are circumvented using the methods of the present disclosure.

For example, traditional recombinant approaches as described above are slow and rely on a relatively small number of random recombination crossover events to swap mutations, and are therefore limited in the number of combinations that can be attempted in any given cycle, or time period. In addition, although the natural recombination events in the prior art are essentially random, they are also subject to genome positional bias.

Most importantly, the traditional approaches also provide little information about the influence of individual mutations and due to the random distribution of recombined mutations many specific combinations cannot be generated and evaluated.

To overcome many of the aforementioned problems associated with traditional strain improvement programs, the present disclosure sets forth a unique HTP genomic engineering platform that is computationally driven and integrates molecular biology, automation, data analytics, and machine learning protocols. This integrative platform utilizes a suite of HTP molecular tool sets that are used to construct HTP genetic design libraries. These genetic design libraries will be elaborated upon below. Figure 8 depicts an overview of an example of the *E. coli* strain improvement program of the present disclosure.

The presently disclosed HTP platform and its unique microbial genetic design libraries fundamentally shift the paradigm of microbial strain development and evolution. For example, traditional mutagenesis-based methods of developing an industrial microbial strain will eventually lead to microbes burdened with a heavy mutagenic load that has been accumulated over years of random mutagenesis.

The ability to solve this issue (*i.e.* remove the genetic baggage accumulated by these microbes) has eluded microbial researchers for decades. However, utilizing the HTP platform disclosed herein, these industrial strains can be "rehabilitated," and the genetic mutations that are deleterious can be identified and removed. Congruently, the genetic mutations that are identified as beneficial can be kept, and in some cases improved upon. The resulting microbial strains demonstrate superior phenotypic traits (*e.g.*, improved production of a compound of interest), as compared to their parental strains.

Furthermore, the HTP platform taught herein is able to identify, characterize, and quantify the effect that individual mutations have on microbial strain performance. This information, *i.e.* what effect does a given genetic change *x* have on host cell phenotype *y* (*e.g.,* production of a compound or product of interest), is able to be generated and then stored in the microbial HTP genetic design libraries discussed below. That is, sequence information for each genetic permutation, and its effect on the host cell phenotype are stored in one or more databases, and are available for subsequent analysis (e.g., epistasis mapping, as discussed below). The present disclosure also teaches methods of physically saving/storing valuable genetic permutations in the form of genetic insertion constructs, or in the form of one or more host cell organisms containing said genetic permutation (e.g., see libraries discussed below.)

When one couples these HTP genetic design libraries into an iterative process that is integrated with a sophisticated data analytics and machine learning process a dramatically different methodology for improving host cells emerges. The taught platform is therefore fundamentally different from the previously discussed traditional methods of developing host cell strains. The taught HTP platform does not suffer from many of the drawbacks associated with the previous methods. These and other advantages will become apparent with reference to the HTP molecular tool sets and the derived genetic design libraries discussed below.

## Genetic Design & Microbial Engineering: A Systematic Combinatorial Approach to Strain Improvement Utilizing a Suite of HTP Molecular Tools and HTP Genetic Design Libraries

As aforementioned, the present disclosure provides a novel HTP platform and genetic design strategy for engineering microbial organisms through iterative systematic introduction and removal of genetic changes across strains. The platform is supported by a suite of molecular tools, which enable the creation of HTP genetic design libraries and allow for the efficient implementation of genetic alterations into a given host strain.

The HTP genetic design libraries of the disclosure serve as sources of possible genetic alterations that may be introduced into a particular microbial strain background. In this way, the HTP genetic design libraries are repositories of genetic diversity, or collections of genetic perturbations, which can be applied to the initial or further engineering of a given microbial strain. Techniques for programming genetic designs for implementation to host strains are described in pending US Patent Application, Serial No. 15/140,296, and pending International Patent Application Serial No. PCT/US17/29725, entitled "Microbial Strain Design System and Methods for Improved Large Scale Production of Engineered Nucleotide Sequences,".

The HTP molecular tool sets utilized in this platform may include, *inter alia*: (1) Promoter swaps (PRO Swap), (2) SNP swaps, (3) Start/Stop codon exchanges, (4) STOP swaps, (5) Sequence optimization, (6) SOLUBILITY TAG swaps and (7) DEGRADATION TAG swaps. The HTP methods of the present disclosure also teach methods for directing the consolidation/combinatorial use of HTP tool sets, including (8) Epistasis mapping protocols. As aforementioned, this suite of molecular tools, either in isolation or combination, enables the creation of HTP genetic design host cell libraries.

As will be demonstrated, utilization of the aforementioned HTP genetic design libraries in the context of the taught HTP microbial engineering platform enables the identification and consolidation of beneficial "causative" mutations or gene sections and also the identification and removal of passive or detrimental mutations or gene sections. This new approach allows rapid improvements in strain performance that could not be achieved by traditional random mutagenesis or directed genetic engineering. The removal of genetic burden or consolidation of beneficial changes into a strain with no genetic burden also provides a new, robust starting point for additional random mutagenesis that may enable further improvements.

In some examples, the present disclosure teaches that as orthogonal beneficial changes are identified across various, discrete branches of a mutagenic strain lineage, they can also be rapidly consolidated into better performing strains. These mutations can also be consolidated into strains that are not part of mutagenic lineages, such as strains with improvements gained by directed genetic engineering.

In some examples, the present disclosure differs from known strain improvement approaches in that it analyzes the genome-wide combinatorial effect of mutations across multiple disparate genomic regions, including expressed and non-expressed genetic elements, and uses gathered information (e.g., experimental results) to predict mutation combinations expected to produce strain enhancements.

In some examples, the present disclosure teaches: i) industrial microorganisms, and other host cells amenable to improvement *via* the disclosed disclosures, ii) generating diversity pools for downstream analysis, iii) methods and hardware for high-throughput screening and sequencing of large variant pools, iv) methods and hardware for machine learning computational analysis and prediction of synergistic effects of genome-wide mutations, and v) methods for high-throughput strain engineering.

The following molecular tools and libraries are discussed in terms of illustrative microbial examples. Persons having skill in the art will recognize that the HTP molecular tools of the present disclosure are compatible with any host cell, including eukaryotic cellular, and higher life forms. Furthermore, many of the illustrated examples are conducted in *Corynebacterium*; however, the same principles and process can be deployed in *Escherichia coli.*

Each of the identified HTP molecular tool sets-which enable the creation of the various HTP genetic design libraries utilized in the microbial engineering platform-will now be discussed.

### *1. Promoter Swaps: A Molecular Tool for the Derivation of Promoter Swap Microbial Strain Libraries*

In some examples, the present disclosure teaches methods of selecting promoters with optimal expression properties to produce beneficial effects on overall-host strain phenotype (e.g., yield or productivity).

For example, in some examples, the present disclosure teaches methods of identifying one or more promoters and/or generating variants of one or more promoters within a host cell, which exhibit a range of expression strengths (*e.g.* promoter ladders discussed *infra*), or superior regulatory properties (e.g.., tighter regulatory control for selected genes). A particular combination of these identified and/or generated promoters can be grouped together as a promoter ladder, which is explained in more detail below.

The promoter ladder in question is then associated with a given gene of interest. Thus, if one has promoters $P_1$-$P_8$ (representing eight promoters that have been identified and/or generated to exhibit a range of expression strengths) and associates the promoter ladder with a single gene of interest in a microbe (*i.e.* genetically engineer a microbe with a given promoter operably linked to a given target gene), then the effect of each combination of the eight promoters can be ascertained by characterizing each of the engineered strains resulting from each combinatorial effort, given that the engineered microbes have an otherwise identical genetic background except the particular promoter(s) associated with the target gene.

The resultant microbes that are engineered *via* this process form HTP genetic design libraries.

The HTP genetic design library can refer to the actual physical microbial strain collection that is formed *via* this process, with each member strain being representative of a given promoter operably linked to a particular target gene, in an otherwise identical genetic background, said library being termed a "promoter swap microbial strain library." In the specific context of *E. coli,* the library can be termed a "promoter swap *E. coli* strain library," but the terms can be used synonymously, as *E. coli* is a specific example of a microbe.

Furthermore, the HTP genetic design library can refer to the collection of genetic perturbations-in this case a given promoter *x* operably linked to a given gene y-said collection being termed a "promoter swap library."

Further, one can utilize the same promoter ladder comprising promoters $P_1$-$P_8$ to engineer microbes, wherein each of the 8 promoters is operably linked to 10 different gene targets. The result of this procedure would be 80 microbes that are otherwise assumed genetically identical, except for the particular promoters operably linked to a target gene of interest. These 80 microbes could be appropriately screened and characterized and give rise to another HTP genetic design library. The characterization of the microbial strains in the HTP genetic design library produces information and data that can be stored in any data storage construct, including a relational database, an object-oriented database or a highly distributed NoSQL database. This data/information could be, for example, a given promoter's (*e.g.* $P_1$-$P_8$) effect when operably linked to a given gene target. This data/information can also be the broader set of combinatorial effects that result from operably linking two or more of promoters $P_1$-$P_8$ to a given gene target.

The aforementioned examples of eight promoters and 10 target genes is merely illustrative, as the concept can be applied with any given number of promoters that have been grouped together based upon exhibition of a range of expression strengths and any given number of target genes. Persons having skill in the art will also recognize the ability to operably link two or more promoters in front of any gene target. Thus, in some examples, the present disclosure teaches promoter swap libraries in which 1, 2, 3 or more promoters from a promoter ladder are operably linked to one or more genes.

In summary, utilizing various promoters to drive expression of various genes in an organism is a powerful tool to optimize a trait of interest. The molecular tool of promoter swapping, developed by the inventors, uses a ladder of promoter sequences that have been demonstrated to vary expression of at least one locus under at least one condition. This ladder is then systematically applied to a group of genes in the organism using high-throughput genome engineering. This group of genes is determined to have a high likelihood of impacting the trait of interest based on any one of a number of methods. These could include selection based on known function, or impact on the trait of interest, or algorithmic selection based on previously determined beneficial genetic diversity. In some examples, the selection of genes can include all the genes in a given host. In other examples, the selection of genes can be a subset of all genes in a given host, chosen randomly.

The resultant HTP genetic design microbial strain library of organisms containing a promoter sequence linked to a gene is then assessed for performance in a high-throughput screening model, and promoter-gene linkages which lead to increased performance are determined and the information stored in a database. The collection of genetic perturbations (*i.e.* given promoter *x* operably linked to a given gene *y*) form a "promoter swap library," which can be utilized as a source of potential genetic alterations to be utilized in microbial engineering processing. Over time, as a greater set of genetic perturbations is implemented against a greater diversity of host cell backgrounds, each library becomes more powerful as a corpus of experimentally confirmed data that can be used to more precisely and predictably design targeted changes against any background of interest.

Transcription levels of genes in an organism are a key point of control for affecting organism behavior. Transcription is tightly coupled to translation (protein expression), and which proteins are expressed in what quantities determines organism behavior. Cells express thousands of different types of proteins, and these proteins interact in numerous complex ways to create function. By varying the expression levels of a set of proteins systematically, function can be altered in ways that, because of complexity, are difficult to predict. Some alterations may increase performance, and so, coupled to a mechanism for assessing performance, this technique allows for the generation of organisms with improved function.

In the context of a small molecule synthesis pathway, enzymes interact through their small molecule substrates and products in a linear or branched chain, starting with a substrate and ending with a small molecule of interest. Because these interactions are sequentially linked, this system exhibits distributed control, and increasing the expression of one enzyme can only increase pathway flux until another enzyme becomes rate limiting.

Metabolic Control Analysis (MCA) is a method for determining, from experimental data and first principles, which enzyme or enzymes are rate limiting. MCA is limited however, because it requires extensive experimentation after each expression level change to determine the new rate limiting enzyme. Promoter swapping is advantageous in this context, because through the application of a promoter ladder to each enzyme in a pathway, the limiting enzyme is found, and the same thing can be done in subsequent rounds to find new enzymes that become rate limiting. Further, because the read-out on function is better production of the small molecule of interest, the experiment to determine which enzyme is limiting is the same as the engineering to increase production, thus shortening development time. In some examples the present disclosure teaches the application of PRO swap to genes encoding individual subunits of multi-unit enzymes. In yet other examples, the present disclosure teaches methods of applying PRO swap techniques to genes responsible for regulating individual enzymes, or whole biosynthetic pathways.

In some examples, the promoter swap tool of the present disclosure is used to identify optimum expression of a selected gene target. In some examples, the goal of the promoter swap may be to increase expression of a target gene to reduce bottlenecks in a metabolic or genetic pathway. In other examples, the goal o the promoter swap may be to reduce the expression of the target gene to avoid unnecessary energy expenditures in the host cell, when expression of said target gene is not required.

In the context of other cellular systems like transcription, transport, or signaling, various rational methods can be used to try and find out, *a priori,* which proteins are targets for expression change and what that change should be. These rational methods reduce the number of perturbations that must be tested to find one that improves performance, but they do so at significant cost. Gene deletion studies identify proteins whose presence is critical for a particular function, and important genes can then be over-expressed. Due to the complexity of protein interactions, this is often ineffective at increasing performance. Different types of models have been developed that attempt to describe, from first principles, transcription or signaling behavior as a function of protein levels in the cell. These models often suggest targets where expression changes might lead to different or improved function. The assumptions that underlie these models are simplistic and the parameters difficult to measure, so the predictions they make are often incorrect, especially for non-model organisms. With both gene deletion and modeling, the experiments required to determine how to affect a certain gene are different than the subsequent work to make the change that improves performance. Promoter swapping sidesteps these challenges, because the constructed strain that highlights the importance of a particular perturbation is also, already, the improved strain.

Thus, in particular examples, promoter swapping is a multi-step process comprising:

1. Selecting a set of "x" promoters to act as a "ladder." Ideally these promoters have been shown to lead to highly variable expression across multiple genomic loci, but the only requirement is that they perturb gene expression in some way.

**2.** Selecting a set of "n" genes to target. This set can be every open reading frame (ORF) in a genome, or a subset of ORFs. The subset can be chosen using annotations on ORFs related to function, by relation to previously demonstrated beneficial perturbations (previous promoter swaps or previous SNP swaps), by algorithmic selection based on epistatic interactions between previously generated perturbations, other selection criteria based on hypotheses regarding beneficial ORF to target, or through random selection. In other examples, the "n" targeted genes can comprise non-protein coding genes, including non-coding RNAs.

3. High-throughput strain engineering to rapidly-and in some examples, in parallel-carry out the following genetic modifications: When a native promoter exists in front of target gene n and its sequence is known, replace the native promoter with each of the x promoters in the ladder. When the native promoter does not exist, or its sequence is unknown, insert each of the x promoters in the ladder in front of gene n *(see e.g.,* Figure 21). In this way a "library" (also referred to as a HTP genetic design library) of strains is constructed, wherein each member of the library is an instance of x promoter operably linked to n target, in an otherwise identical genetic context. As previously described combinations of promoters can be inserted, extending the range of combinatorial possibilities upon which the library is constructed.

4. High-throughput screening of the library of strains in a context where their performance against one or more metrics is indicative of the performance that is being optimized.

This foundational process can be extended to provide further improvements in strain performance by, *inter alia:* (1) Consolidating multiple beneficial perturbations into a single strain background, either one at a time in an interactive process, or as multiple changes in a single step. Multiple perturbations can be either a specific set of defined changes or a partly randomized, combinatorial library of changes. For example, if the set of targets is every gene in a pathway, then sequential regeneration of the library of perturbations into an improved member or members of the previous library of strains can optimize the expression level of each gene in a pathway regardless of which genes are rate limiting at any given iteration; (2) Feeding the performance data resulting from the individual and combinatorial generation of the library into an algorithm that uses that data to predict an optimum set of perturbations based on the interaction of each perturbation; and (3) Implementing a combination of the above two approaches (see Figure 20).

The molecular tool, or technique, discussed above is characterized as promoter swapping, but is not limited to promoters and can include other sequence changes that systematically vary the expression level of a set of targets. Other methods for varying the expression level of a set of genes could include: a) a ladder of ribosome binding sites (or Kozak sequences in eukaryotes); b) replacing the start codon of each target with each of the other start codons *(i.e* start/stop codon exchanges discussed *infra);* c) attachment of various mRNA stabilizing or destabilizing sequences to the 5' or 3' end, or at any other location, of a transcript, d) attachment of various protein stabilizing or destabilizing sequences at any location in the protein *(i.e.,* degradation or solubilization tag exchanges discussed *infra).*

The approach is exemplified in the present disclosure with industrial microorganisms, but is applicable to any organism where desired traits can be identified in a population of genetic mutants. For example, this could be used for improving the performance of CHO cells, yeast, insect cells, algae, as well as multi-cellular organisms, such as plants.

### *2. SNP Swaps: A Molecular Tool for the Derivation of SNP Swap Microbial Strain Libraries*

In certain examples, SNP swapping is not a random mutagenic approach to improving a microbial strain, but rather involves the systematic introduction or removal of individual Small Nuclear Polymorphism nucleotide mutations (i.e. SNPs) (hence the name "SNP swapping") across strains.

The resultant microbes that are engineered *via* this process form HTP genetic design libraries.

The HTP genetic design library can refer to the actual physical microbial strain collection that is formed *via* this process, with each member strain being representative of the presence or absence of a given SNP, in an otherwise identical genetic background, said library being termed a "SNP swap microbial strain library." In the specific context of *E. coli,* the library can be termed a "SNP swap *E. coli* strain library," but the terms can be used synonymously, as *E. coli* is a specific example of a microbe.

Furthermore, the HTP genetic design library can refer to the collection of genetic perturbations-in this case a given SNP being present or a given SNP being absent-said collection being termed a "SNP swap library."

In some examples, SNP swapping involves the reconstruction of host organisms with optimal combinations of target SNP "building blocks" with identified beneficial performance effects. Thus, in some examples, SNP swapping involves consolidating multiple beneficial mutations into a single strain background, either one at a time in an iterative process, or as multiple changes in a single step. Multiple changes can be either a specific set of defined changes or a partly randomized, combinatorial library of mutations.

In other examples, SNP swapping also involves removing multiple mutations identified as detrimental from a strain, either one at a time in an iterative process, or as multiple changes in a single step. Multiple changes can be either a specific set of defined changes or a partly randomized, combinatorial library of mutations. In some examples, the SNP swapping methods of the present disclosure include both the addition of beneficial SNPs, and removing detrimental and/or neutral mutations.

SNP swapping is a powerful tool to identify and exploit both beneficial and detrimental mutations in a lineage of strains subjected to mutagenesis and selection for an improved trait of interest. SNP swapping utilizes high-throughput genome engineering techniques to systematically determine the influence of individual mutations in a mutagenic lineage. Genome sequences are determined for strains across one or more generations of a mutagenic lineage with known performance improvements. High-throughput genome engineering is then used systematically to recapitulate mutations from improved strains in earlier lineage strains, and/or revert mutations in later strains to earlier strain sequences. The performance of these strains is then evaluated and the contribution of each individual mutation on the improved phenotype of interest can be determined. As aforementioned, the microbial strains that result from this process are analyzed/characterized and form the basis for the SNP swap genetic design libraries that can inform microbial strain improvement across host strains.

Removal of detrimental mutations can provide immediate performance improvements, and consolidation of beneficial mutations in a strain background not subject to mutagenic burden can rapidly and greatly improve strain performance. The various microbial strains produced *via* the SNP swapping process form the HTP genetic design SNP swapping libraries, which are microbial strains comprising the various added/deleted/or consolidated SNPs, but with otherwise identical genetic backgrounds.

As discussed previously, random mutagenesis and subsequent screening for performance improvements is a commonly used technique for industrial strain improvement, and many strains currently used for large scale manufacturing have been developed using this process iteratively over a period of many years, sometimes decades. Random approaches to generating genomic mutations such as exposure to UV radiation or chemical mutagens such as ethyl methanesulfonate were a preferred method for industrial strain improvements because: 1) industrial organisms may be poorly characterized genetically or metabolically, rendering target selection for directed improvement approaches difficult or impossible; 2) even in relatively well characterized systems, changes that result in industrial performance improvements are difficult to predict and may require perturbation of genes that have no known function, and 3) genetic tools for making directed genomic mutations in a given industrial organism may not be available or very slow and/or difficult to use.

However, despite the aforementioned benefits of this process, there are also a number of known disadvantages. Beneficial mutations are relatively rare events, and in order to find these mutations with a fixed screening capacity, mutations rates must be sufficiently high. This often results in unwanted neutral and partly detrimental mutations being incorporated into strains along with beneficial changes. Over time this 'mutagenic burden' builds up, resulting in strains with deficiencies in overall robustness and key traits such as growth rates. Eventually 'mutagenic burden' renders further improvements in performance through random mutagenesis increasingly difficult or impossible to obtain. Without suitable tools, it is impossible to consolidate beneficial mutations found in discrete and parallel branches of strain lineages.

SNP swapping is an approach to overcome these limitations by systematically recapitulating or reverting some or all mutations observed when comparing strains within a mutagenic lineage. In this way, both beneficial ('causative') mutations can be identified and consolidated, and/or detrimental mutations can be identified and removed. This allows rapid improvements in strain performance that could not be achieved by further random mutagenesis or targeted genetic engineering.

Removal of genetic burden or consolidation of beneficial changes into a strain with no genetic burden also provides a new, robust starting point for additional random mutagenesis that may enable further improvements.

In addition, as orthogonal beneficial changes are identified across various, discrete branches of a mutagenic strain lineage, they can be rapidly consolidated into better performing strains. These mutations can also be consolidated into strains that are not part of mutagenic lineages, such as strains with improvements gained by directed genetic engineering.

Other approaches and technologies exist to randomly recombine mutations between strains within a mutagenic lineage. These include techniques such as protoplast fusion and whole genome shuffling that facilitate genomic recombination across mutated strains. For some industrial microorganisms such as yeast and filamentous fungi, natural mating cycles can also be exploited for pairwise genomic recombination. In this way, detrimental mutations can be removed by 'backcrossing' mutants with parental strains and beneficial mutations consolidated. However, these approaches are subject to many limitations that are circumvented using the SNP swapping methods of the present disclosure.

For example, as these approaches rely on a relatively small number of random recombination crossover events to swap mutations, it may take many cycles of recombination and screening to optimize strain performance. In addition, although natural recombination events are essentially random, they are also subject to genome positional bias and some mutations may be difficult to address. These approaches also provide little information about the influence of individual mutations without additional genome sequencing and analysis. SNP swapping overcomes these fundamental limitations as it is not a random approach, but rather the systematic introduction or removal of individual mutations across strains.

In some examples, the present disclosure teaches methods for identifying the SNP sequence diversity present among the organisms of a diversity pool. A diversity pool can be a given number *n* of microbes utilized for analysis, with said microbes' genomes representing the "diversity pool."

In particular examples, a diversity pool may be an original parent strain (Si) with a "baseline" or "reference" genetic sequence at a particular time point ($S_1Gen_1$) and then any number of subsequent offspring strains ($S_{2-n}$) that were derived/developed from said $S_1$ strain and that have a different genome ($S_{2-n}Gen_{2-n}$), in relation to the baseline genome of $S_1$.

For example, in some examples, the present disclosure teaches sequencing the microbial genomes in a diversity pool to identify the SNPs present in each strain. In one example, the strains of the diversity pool are historical microbial production strains. Thus, a diversity pool of the present disclosure can include for example, an industrial reference strain, and one or more mutated industrial strains produced *via* traditional strain improvement programs.

In some examples, the SNPs within a diversity pool are determined with reference to a "reference strain." In some examples, the reference strain is a wild-type strain. In other examples, the reference strain is an original industrial strain prior to being subjected to any mutagenesis. The reference strain can be defined by the practitioner and does not have to be an original wild-type strain or original industrial strain. The base strain is merely representative of what will be considered the "base," "reference" or original genetic background, by which subsequent strains that were derived, or were developed from said reference strain, are to be compared.

Once all SNPS in the diversity pool are identified, the present disclosure teaches methods of SNP swapping and screening methods to delineate *(i.e.* quantify and characterize) the effects (e.g. creation of a phenotype of interest) of SNPs individually and/or in groups.

In some examples, the SNP swapping methods of the present disclosure comprise the step of introducing one or more SNPs identified in a mutated strain *(e.g.,* a strain from amongst $S_2$-$n$$Gen_{2-n}$) to a reference strain ($S_1Gen_1$) or wild-type strain ("wave up").

In other examples, the SNP swapping methods of the present disclosure comprise the step of removing one or more SNPs identified in a mutated strain *(e.g.,* a strain from amongst $S_{2-n}Gen_2$-n) ("wave down").

In some examples, each generated strain comprising one or more SNP changes (either introducing or removing) is cultured and analyzed under one or more criteria of the present disclosure (e.g., production of a chemical or product of interest). Data from each of the analyzed host strains is associated, or correlated, with the particular SNP, or group of SNPs present in the host strain, and is recorded for future use. Thus, the present disclosure enables the creation of large and highly annotated HTP genetic design microbial strain libraries that are able to identify the effect of a given SNP on any number of microbial genetic or phenotypic traits of interest. The information stored in these HTP genetic design libraries informs the machine learning algorithms of the HTP genomic engineering platform and directs future iterations of the process, which ultimately leads to evolved microbial organisms that possess highly desirable properties/traits.

### *3. Start/Stop Codon Exchanges: A Molecular Tool for the Derivation of Start/Stop Codon Microbial Strain Libraries*

In some examples, the present disclosure teaches methods of swapping start and stop codon variants. For example, typical stop codons for *S. cerevisiae* and mammals are TAA (UAA) and TGA (UGA), respectively. The typical stop codon for monocotyledonous plants is TGA (UGA), whereas insects and E. *coli* commonly use TAA (UAA) as the stop codon (Dalphin et al. (1996) Nucl. Acids Res. 24: 216-218). In other examples, the present disclosure teaches use of the TAG (UAG) stop codons.

The present disclosure similarly teaches swapping start codons. In some examples, the present disclosure teaches use of the ATG (AUG) start codon utilized by most organisms (especially eukaryotes). In some examples, the present disclosure teaches that prokaryotes use ATG (AUG) the most, followed by GTG (GUG) and TTG (UUG).

In other examples, the present disclosure teaches replacing ATG start codons with TTG. In some examples, the present disclosure teaches replacing ATG start codons with GTG. In some examples, the present disclosure teaches replacing GTG start codons with ATG. In some examples, the present disclosure teaches replacing GTG start codons with TTG. In some examples, the present disclosure teaches replacing TTG start codons with ATG. In some examples, the present disclosure teaches replacing TTG start codons with GTG.

In other examples, the present disclosure teaches replacing TAA stop codons with TAG. In some examples, the present disclosure teaches replacing TAA stop codons with TGA. In some examples, the present disclosure teaches replacing TGA stop codons with TAA. In some examples, the present disclosure teaches replacing TGA stop codons with TAG. In some examples, the present disclosure teaches replacing TAG stop codons with TAA. In some examples, the present disclosure teaches replacing TAG stop codons with TGA.

### *4. Stop swap: A Molecular Tool for the Derivation of STOP Swap Microbial Strain Libraries*

In some examples, the present disclosure teaches methods of improving host cell productivity through the optimization of cellular gene transcription. Gene transcription is the result of several distinct biological phenomena, including transcriptional initiation (RNAp recruitment and transcriptional complex formation), elongation (strand synthesis/extension), and transcriptional termination (RNAp detachment and termination). Although much attention has been devoted to the control of gene expression through the transcriptional modulation of genes *(e.g.,* by changing promoters, or inducing regulatory transcription factors), comparatively few efforts have been made towards the modulation of transcription via the modulation of gene terminator sequences.

The most obvious way that transcription impacts on gene expression levels is through the rate of Pol II initiation, which can be modulated by combinations of promoter or enhancer strength and trans-activating factors (Kadonaga, JT. 2004 "Regulation of RNA polymerase II transcription by sequence-specific DNA binding factors" Cell. 2004 Jan 23; 116(2):247-57). In eukaryotes, elongation rate may also determine gene expression patterns by influencing alternative splicing (Cramer P. et al., 1997 "Functional association between promoter structure and transcript alternative splicing." Proc Natl Acad Sci U S A. 1997 Oct 14; 94(21):11456-60). Failed termination on a gene can impair the expression of downstream genes by reducing the accessibility of the promoter to Pol II (Greger IH. et al., 2000 "Balancing transcriptional interference and initiation on the GAL7 promoter of Saccharomyces cerevisiae." Proc Natl Acad Sci USA. 2000 Jul 18; 97(15): 8415-20). This process, known as transcriptional interference, is particularly relevant in lower eukaryotes, as they often have closely spaced genes.

Termination sequences can also affect the expression of the genes to which the sequences belong. For example, studies show that inefficient transcriptional termination in eukaryotes results in an accumulation of unspliced pre-mRNA (see West, S., and Proudfoot, N.J., 2009 "Transcriptional Termination Enhances Protein Expression in Human Cells" Mol Cell. 2009 Feb 13; 33(3-9); 354-364). Other studies have also shown that 3' end processing, can be delayed by inefficient termination (West, S et al., 2008 "Molecular dissection of mammalian RNA polymerase II transcriptional termination." Mol Cell. 2008 Mar 14; 29(5):600-10.). Transcriptional termination can also affect mRNA stability by releasing transcripts from sites of synthesis. Further, strong termination sequences may increase mRNA stability, thus increasing protein abundance and overall pathway activity.

### *Termination of transcription mechanism in eukaryotes*

Transcriptional termination in eukaryotes operates through terminator signals that are recognized by protein factors associated with the RNA polymerase II. In some examples, the cleavage and polyadenylation specificity factor (CPSF) and cleavage stimulation factor (CstF) transfer from the carboxyl terminal domain of RNA polymerase II to the poly-A signal. In some examples, the CPSF and CstF factors also recruit other proteins to the termination site, which then cleave the transcript and free the mRNA from the transcription complex. Termination also triggers polyadenylation of mRNA transcripts. Illustrative examples of validated eukaryotic termination factors, and their conserved structures are discussed in later portions of this document.

### *Termination of transcription in prokaryotes*

In prokaryotes, two principal mechanisms, termed Rho-independent and Rho-dependent termination, mediate transcriptional termination. Rho-independent termination signals do not require an extrinsic transcription-termination factor, as formation of a stem-loop structure in the RNA transcribed from these sequences along with a series of Uridine (U) residues promotes release of the RNA chain from the transcription complex. Rho-dependent termination, on the other hand, requires a transcription-termination factor called Rho and cis-acting elements on the mRNA. The initial binding site for Rho, the Rho utilization (rut) site, is an extended (~70 nucleotides, sometimes 80-100 nucleotides) single-stranded region characterized by a high cytidine/low guanosine content and relatively little secondary structure in the RNA being synthesized, upstream of the actual terminator sequence. When a polymerase pause site is encountered, termination occurs, and the transcript is released by Rho's helicase activity.

### *Terminator Swapping (STOP swap)*

In some examples, the present disclosure teaches methods of selecting termination sequences ("terminators") with optimal expression properties to produce beneficial effects on overall-host strain productivity.

For example, in some examples, the present disclosure teaches methods of identifying one or more terminators and/or generating variants of one or more terminators within a host cell, which exhibit a range of expression strengths *(e.g.* terminator ladders discussed *infra).* A particular combination of these identified and/or generated terminators can be grouped together as a terminator ladder, which is explained in more detail below.

The terminator ladder in question is then associated with a given gene of interest. Thus, if one has terminators $T_1$-$T_8$ (representing eight terminators that have been identified and/or generated to exhibit a range of expression strengths when combined with one or more promoters) and associates the terminator ladder with a single gene of interest in a host cell *(i.e.* genetically engineer a host cell with a given terminator operably linked to the 3' end of to a given target gene), then the effect of each combination of the terminators can be ascertained by characterizing each of the engineered strains resulting from each combinatorial effort, given that the engineered host cells have an otherwise identical genetic background except the particular terminator(s) associated with the target gene. The resultant host cells that are engineered *via* this process form HTP genetic design libraries.

The HTP genetic design library can refer to the actual physical microbial strain collection that is formed *via* this process, with each member strain being representative of a given terminator operably linked to a particular target gene, in an otherwise identical genetic background, said library being termed a "terminator swap microbial strain library" or "STOP swap microbial strain library." In the specific context of *E. coli,* the library can be termed a "terminator swap *E. coli* strain library," or "STOP swap *E. coli* strain library," but the terms can be used synonymously, as *E. coli* is a specific example of a microbe.

Furthermore, the HTP genetic design library can refer to the collection of genetic perturbations-in this case a given terminator x operably linked to a given gene y-said collection being termed a "terminator swap library" or "STOP swap library."

Further, one can utilize the same terminator ladder comprising terminators $T_{1-}T_8$ to engineer microbes, wherein each of the eight terminators is operably linked to 10 different gene targets. The result of this procedure would be 80 host cell strains that are otherwise assumed genetically identical, except for the particular terminators operably linked to a target gene of interest. These 80 host cell strains could be appropriately screened and characterized and give rise to another HTP genetic design library. The characterization of the microbial strains in the HTP genetic design library produces information and data that can be stored in any database, including without limitation, a relational database, an object-oriented database or a highly distributed NoSQL database. This data/information could include, for example, a given terminators' (e.g., $TiT_8$) effect when operably linked to a given gene target. This data/information can also be the broader set of combinatorial effects that result from operably linking two or more promoters (e.g., $T_1$-$T_8$) to a given gene target.

The aforementioned examples of eight terminators and 10 target genes is merely illustrative, as the concept can be applied with any given number of terminators that have been grouped together based upon exhibition of a range of expression strengths and any given number of target genes. For example, another set of terminators that can be used in the methods provided herein (e.g., STOP Swapping) is the set of terminators found in Table 1.2 with nucleic acid SEQ ID Nos 225, 226, 227, 228, 229, or 230.

In summary, utilizing various terminators to modulate expression of various genes in an organism is a powerful tool to optimize a trait of interest. The molecular tool of terminator swapping, developed by the inventors, uses a ladder of terminator sequences that have been demonstrated to vary expression of at least one locus under at least one condition. This ladder is then systematically applied to a group of genes in the organism using high-throughput genome engineering. This group of genes is determined to have a high likelihood of impacting the trait of interest based on any one of a number of methods. These could include selection based on known function, or impact on the trait of interest, or algorithmic selection based on previously determined beneficial genetic diversity.

The resultant HTP genetic design microbial library of organisms containing a terminator sequence linked to a gene is then assessed for performance in a high-throughput screening model, and terminator-gene linkages which lead to increased performance are determined and the information stored in a database. The collection of genetic perturbations *(i.e.* given terminator *x* linked to a given gene y) form a "terminator swap library," which can be utilized as a source of potential genetic alterations to be utilized in microbial engineering processing. Over time, as a greater set of genetic perturbations is implemented against a greater diversity of microbial backgrounds, each library becomes more powerful as a corpus of experimentally confirmed data that can be used to more precisely and predictably design targeted changes against any background of interest. That is in some examples, the present disclosures teaches introduction of one or more genetic changes into a host cell based on previous experimental results embedded within the meta data associated with any of the genetic design libraries of the disclosure.

Thus, in particular examples, terminator swapping is a multi-step process comprising:

1. Selecting a set of "x" terminators to act as a "ladder." Ideally these terminators have been shown to lead to highly variable expression across multiple genomic loci, but the only requirement is that they perturb gene expression in some way.
2. Selecting a set of "n" genes to target. This set can be every ORF in a genome, or a subset of ORFs. The subset can be chosen using annotations on ORFs related to function, by relation to previously demonstrated beneficial perturbations (previous promoter swaps, STOP swaps, SOLUBILITY TAG swaps, DEGRADATION TAG swaps or SNP swaps), by algorithmic selection based on epistatic interactions between previously generated perturbations, other selection criteria based on hypotheses regarding beneficial ORF to target, or through random selection. In other examples, the "n" targeted genes can comprise non-protein coding genes, including non-coding RNAs.

3. High-throughput strain engineering to rapidly and in parallel carry out the following genetic modifications: When a native terminator exists at the 3' end of target gene n and its sequence is known, replace the native terminator with each of the x terminators in the ladder. When the native terminator does not exist, or its sequence is unknown, insert each of the x terminators in the ladder after the gene stop codon.

In this way a "library" (also referred to as a HTP genetic design library) of strains is constructed, wherein each member of the library is an instance of x terminator linked to n target, in an otherwise identical genetic context. As previously described, combinations of terminators can be inserted, extending the range of combinatorial possibilities upon which the library is constructed.

4. High-throughput screening of the library of strains in a context where their performance against one or more metrics is indicative of the performance that is being optimized.

This foundational process can be extended to provide further improvements in strain performance by, *inter alia:* (1) Consolidating multiple beneficial perturbations into a single strain background, either one at a time in an interactive process, or as multiple changes in a single step. Multiple perturbations can be either a specific set of defined changes or a partly randomized, combinatorial library of changes. For example, if the set of targets is every gene in a pathway, then sequential regeneration of the library of perturbations into an improved member or members of the previous library of strains can optimize the expression level of each gene in a pathway regardless of which genes are rate limiting at any given iteration; (2) Feeding the performance data resulting from the individual and combinatorial generation of the library into an algorithm that uses that data to predict an optimum set of perturbations based on the interaction of each perturbation; and (3) Implementing a combination of the above two approaches.

The approach is exemplified in the present disclosure with industrial microorganisms, but is applicable to any organism where desired traits can be identified in a population of genetic mutants. For example, this could be used for improving the performance of CHO cells, yeast, insect cells, algae, as well as multi-cellular organisms, such as plants.

### *5. Sequence Optimization: A Molecular Tool for the Derivation of Optimized Sequence Microbial Strain Libraries*

In one example, the methods of the disclosure comprise codon optimizing one or more genes expressed by the host organism. Methods for optimizing codons to improve expression in various hosts are known in the art and are described in the literature *(see* U.S. Pat. App. Pub. No. 2007/0292918). Optimized coding sequences containing codons preferred by a particular prokaryotic or eukaryotic host *(see* also, Murray et al. (1989) Nucl. Acids Res. 17:477-508) can be prepared, for example, to increase the rate of translation or to produce recombinant RNA transcripts having desirable properties, such as a longer half-life, as compared with transcripts produced from a non-optimized sequence.

Protein expression is governed by a host of factors including those that affect transcription, mRNA processing, and stability and initiation of translation. Optimization can thus address any of a number of sequence features of any particular gene. As a specific example, a rare codon induced translational pause can result in reduced protein expression. A rare codon induced translational pause includes the presence of codons in the polynucleotide of interest that are rarely used in the host organism may have a negative effect on protein translation due to their scarcity in the available tRNA pool.

Alternate translational initiation also can result in reduced heterologous protein expression. Alternate translational initiation can include a synthetic polynucleotide sequence inadvertently containing motifs capable of functioning as a ribosome binding site (RBS). These sites can result in initiating translation of a truncated protein from a gene-internal site. One method of reducing the possibility of producing a truncated protein, which can be difficult to remove during purification, includes eliminating putative internal RBS sequences from an optimized polynucleotide sequence.

Repeat-induced polymerase slippage can result in reduced heterologous protein expression. Repeat-induced polymerase slippage involves nucleotide sequence repeats that have been shown to cause slippage or stuttering of DNA polymerase which can result in frameshift mutations. Such repeats can also cause slippage of RNA polymerase. In an organism with a high G+C content bias, there can be a higher degree of repeats composed of G or C nucleotide repeats. Therefore, one method of reducing the possibility of inducing RNA polymerase slippage, includes altering extended repeats of G or C nucleotides.

Interfering secondary structures also can result in reduced heterologous protein expression. Secondary structures can sequester the RBS sequence or initiation codon and have been correlated to a reduction in protein expression. Stemloop structures can also be involved in transcriptional pausing and attenuation. An optimized polynucleotide sequence can contain minimal secondary structures in the RBS and gene coding regions of the nucleotide sequence to allow for improved transcription and translation.

For example, the optimization process can begin by identifying the desired amino acid sequence to be expressed by the host. From the amino acid sequence a candidate polynucleotide or DNA sequence can be designed. During the design of the synthetic DNA sequence, the frequency of codon usage can be compared to the codon usage of the host expression organism and rare host codons can be removed from the synthetic sequence. Additionally, the synthetic candidate DNA sequence can be modified in order to remove undesirable enzyme restriction sites and add or remove any desired signal sequences, linkers or untranslated regions. The synthetic DNA sequence can be analyzed for the presence of secondary structure that may interfere with the translation process, such as G/C repeats and stem-loop structures.

### *6. SOLUBILITY TAG swap: A Molecular Tool for the Derivation of SOLUBILITY TAG Swap Microbial Strain Libraries*

In some examples, the present disclosure teaches methods of improving host cell productivity through the optimization of post-translational mechanisms. Traditional strain improvement can often be accomplished through overexpression of pathway genes that produce some molecule of interest. Typically, known pathway genes can be duplicated, or strong promoters can be inserted to drive expression of these genes, and thus increase mRNA transcript levels with the goal of increasing pathway protein abundance to achieve improved rate, titer, or yield from a given pathway. This approach can be applied systematically at the whole-genome scale to identify all genes that can improve strain performance. Another frequently applied approach can be deletion of potentially competing pathway genes with the goal of completely eliminating protein products that may divert carbon from the desired pathway. However, these overexpression and/or deletion strain improvement approaches known in the art can have several limitations.

Beginning with pathway duplication or strong promoter insertion, the expected effect of increased mRNA transcript levels may not necessarily result in increased protein abundance. Various protein products may have various rate-limiting steps in their production, and this rate-limiting step may not be mRNA transcript levels. In scenarios where mRNA transcription is not the rate limiting step, it is possible that post-translational mechanisms may be impacting overall protein abundance. For example, the presence of protein solubility tags may be used to increase the abundance of correctly folded, active protein that can contribute to production of a target molecule, whereas simply increasing mRNA transcript levels may lead only to an increase in misfolded, inactive protein. Effects exerted protein solubility tags can also be made to be tunable depending on the sequence of the solubility tag that is used, enabling precise optimization of the target phenotype.

### *Protein Solubility Tag Swapping (SOLUBILITY TAG swap)*

In some examples, the present disclosure teaches methods of selecting protein solubility tag sequences ("solubility tags") with optimal protein solubility properties to produce beneficial effects on overall-host strain productivity.

For example, in some examples, the present disclosure teaches methods of identifying one or more protein solubility tags and/or generating variants of one or more protein solubility tags within a host cell, which exhibit a range of solubility strengths (e.g. protein solubility tags discussed *infra).* A particular combination of these identified and/or generated protein solubility tags can be grouped together as a protein solubility tag ladder, which is explained in more detail below.

The protein solubility tag ladder in question is then associated with a given gene of interest. Thus, if one has protein solubility tags $PST_1$-$PST_4$ *(see* Table 17) representing a subset of protein solubility tags that have been identified from Costa et al., Front Microbiol. 2014; 5: 63 to enhance protein solubility and also be smaller than 100 amino acids and associates the protein solubility tag ladder with a single gene of interest in a host cell *(i.e.* genetically engineer a host cell with a given protein solubility tag operably linked to a given target gene to generate a target protein tagged at either the N-terminus or the C-terminus). The effect of each combination of the protein solubility tag can be ascertained by characterizing each of the engineered strains resulting from each combinatorial effort, given that the engineered host cells have an otherwise identical genetic background except the particular solubility tag(s) associated with the target gene. The resultant host cells that are engineered *via* this process form HTP genetic design libraries.

The HTP genetic design library can refer to the actual physical microbial strain collection that is formed *via* this process, with each member strain being representative of a given protein solubility tag operably linked to a particular target protein, in an otherwise identical genetic background, said library being termed a "solubility tag swap microbial strain library" or "SOLUBILITY TAG swap microbial strain library." In the specific context of *E. coli,* the library can be termed a "SOLUBILITY TAG swap *E. coli* strain library," or "SOLUBILITY TAG swap *E. coli* strain library," but the terms can be used synonymously, as E. *coli* is a specific example of a microbe.

Furthermore, the HTP genetic design library can refer to the collection of genetic perturbations-in this case a given protein solubility tag x operably linked to a given gene y-said collection being termed a "protein solubility tag swap library" or "SOLUBILITY TAG swap library."

Further, one can utilize the same protein solubility tag ladder comprising protein solubility tag $PST_1$-$PST_4$ to

engineer microbes, wherein each of the four protein solubility tags is operably linked to 10 different gene targets. The result of this procedure would be 40 host cell strains that are otherwise assumed genetically identical, except for the particular protein solubility tags operably linked to a target gene of interest. These 40 host cell strains could be appropriately screened and characterized and give rise to another HTP genetic design library. The characterization of the microbial strains in the HTP genetic design library produces information and data that can be stored in any database, including without limitation, a relational database, an object-oriented database or a highly distributed NoSQL database. This data/information could include, for example, a given protein solubility tag (e.g., $PST_1$-$PST_4$) effect when operably linked to a given gene target. This data/information can also be the broader set of combinatorial effects that result from operably linking two or more solubility tags (e.g., $PST_1$-$PST_4$) to a given gene target.

The aforementioned examples of four protein solubility tags and 10 target genes is merely illustrative, as the concept can be applied with any given number of protein solubility tags that have been grouped together based upon exhibition of a range of solubility strengths and any given number of target genes.

In summary, utilizing various protein solubility tags to modulate solubility of various proteins in an organism is a powerful tool to optimize a trait of interest. The molecular tool of protein solubility tag swapping, developed by the inventors, uses a ladder of protein solubility tag sequences that have been demonstrated to vary solubility (e.g., enhance) of at least one protein under at least one condition. This ladder is then systematically applied to a group of genes in the organism using high-throughput genome engineering. This group of genes is determined to have a high likelihood of impacting the trait of interest based on any one of a number of methods. These could include selection based on known function, or impact on the trait of interest, or algorithmic selection based on previously determined beneficial genetic diversity.

The resultant HTP genetic design microbial library of organisms containing a protein solubility tag sequence linked to a gene is then assessed for performance in a high-throughput screening model, and protein solubility tag -gene linkages which lead to increased performance are determined and the information stored in a database. The collection of genetic perturbations *(i.e.* given protein solubility tag *x* linked to a given gene *y)* form a "protein solubility tag swap library," which can be utilized as a source of potential genetic alterations to be utilized in microbial engineering processing. Over time, as a greater set of genetic perturbations is implemented against a greater diversity of microbial backgrounds, each library becomes more powerful as a corpus of experimentally confirmed data that can be used to more precisely and predictably design targeted changes against any background of interest. That is in some examples, the present disclosures teaches introduction of one or more genetic changes into a host cell based on previous experimental results embedded within the meta data associated with any of the genetic design libraries of the disclosure.

Thus, in particular examples, protein solubility tag swapping is a multi-step process comprising:

**1.** Selecting a set of "x" protein solubility tags to act as a "ladder." Ideally these protein solubility tags have been shown to lead to enhanced protein solubility across multiple genomic loci, but the only requirement is that they perturb solubility in some way.

**2.** Selecting a set of "n" genes to target. This set can be every ORF in a genome, or a subset of ORFs. The subset can be chosen using annotations on ORFs related to function, by relation to previously demonstrated beneficial perturbations (previous promoter swaps, STOP swaps, DEGRADATION TAG swaps or SNP swaps), by algorithmic selection based on epistatic interactions between previously generated perturbations, other selection criteria based on hypotheses regarding beneficial ORF to target, or through random selection.

**3.** High-throughput strain engineering to rapidly and in parallel carry out the following genetic modifications: When a native protein solubility tag exists within a target gene n and its sequence is known, replace the native protein solubility tag with each of the x protein solubility tags in the ladder. When the native protein solubility tag does not exist, or its sequence is unknown, insert each of the x protein solubility tags in the ladder.

In this way a "library" (also referred to as a HTP genetic design library) of strains is constructed, wherein each member of the library is an instance of x protein solubility tag linked to n target, in an otherwise identical genetic context. As previously described, combinations of protein solubility tags can be inserted, extending the range of combinatorial possibilities upon which the library is constructed.

**4.** High-throughput screening of the library of strains in a context where their performance against one or more metrics is indicative of the performance that is being optimized.

This foundational process can be extended to provide further improvements in strain performance by, *inter alia:* (1) Consolidating multiple beneficial perturbations into a single strain background, either one at a time in an interactive process, or as multiple changes in a single step. Multiple perturbations can be either a specific set of defined changes or a partly randomized, combinatorial library of changes. For example, if the set of targets is every gene in a pathway, then sequential regeneration of the library of perturbations into an improved member or members of the previous library of strains can optimize the expression level of each gene in a pathway regardless of which genes are rate limiting at any given iteration; (2) Feeding the performance data resulting from the individual and combinatorial generation of the library into an algorithm that uses that data to predict an optimum set of perturbations based on the interaction of each perturbation; and (3) Implementing a combination of the above two approaches.

The approach is exemplified in the present disclosure with industrial microorganisms, but is applicable to any organism where desired traits can be identified in a population of genetic mutants. For example, this could be used for improving the performance of CHO cells, yeast, insect cells, algae, as well as multi-cellular organisms, such as plants.

### *7. DEGRADATION TAG swap: A Molecular Tool for the Derivation of DEGRADATION TAG Swap Microbial Strain Libraries*

Further to the above examples regarding methods for improving host cell productivity through the optimization of post-translational mechanisms, a strategy of gene deletion may also have drawbacks that can be addressed by the protein degradation tags (as well as terminators and protein solubility tags) of the present disclosure. Wholesale deletion of a gene and its corresponding protein product can in some cases impose a drastic modification to the cell. A more precise and tunable response may be achieved through libraries of protein degradation tags that target a protein for degradation at variable rates. This approach can also have the benefit of allowing modulation of protein products that may be essential for cell survival and would not be viable if completely deleted. As these degradation tags also function at a post-translational level, they may be able to address scenarios where altered mRNA transcript levels do not result in altered protein levels as described above.

### *Protein Degradation Tag Swapping (DEGRADATION TAG swap)*

In some examples, the present disclosure teaches methods of selecting protein degradation tag sequences ("degradation tags") with optimal protein degradation or protein level modulation properties to produce beneficial effects on overall-host strain productivity.

For example, in some examples, the present disclosure teaches methods of identifying one or more protein degradation tags and/or generating variants of one or more protein degradation tags within a host cell, which exhibit a range of degradation strengths or modulate the levels of target proteins *(e.g.* protein degradation tags discussed *infra).* A particular combination of these identified and/or generated protein degradation tags can be grouped together as a protein degradation tag ladder, which is explained in more detail below.

The protein degradation tag ladder in question is then associated with a given gene of interest. Thus, if one has protein degradation tags $PDT_1$-$PDT_8$ (*see* Table 18) representing a subset of protein degradation tags that have been identified from various sources as detailed in Table 18) and associates the protein degradation tag ladder with a single gene of interest in a host cell (*i.e.* genetically engineer a host cell with a given protein degradation tag operably linked to a given target gene), then the effect of each combination of the protein degradation tag can be ascertained by characterizing each of the engineered strains resulting from each combinatorial effort, given that the engineered host cells have an otherwise identical genetic background except the particular degradation tag(s) associated with the target gene. The resultant host cells that are engineered *via* this process form HTP genetic design libraries.

The HTP genetic design library can refer to the actual physical microbial strain collection that is formed *via* this process, with each member strain being representative of a given protein degradation tag operably linked to a particular target protein, in an otherwise identical genetic background, said library being termed a "degradation tag swap microbial strain library" or "DEGRADATION TAG swap microbial strain library." In the specific context of *E. coli,* the library can be termed a "DEGRADATION TAG swap *E. coli* strain library," or "DEGRADATION TAG swap *E. coli* strain library," but the terms can be used synonymously, as *E. coli* is a specific example of a microbe.

Furthermore, the HTP genetic design library can refer to the collection of genetic perturbations-in this case a given protein degradation tag *x* operably linked to a given gene *y*-said collection being termed a "protein degradation tag swap library" or "DEGRADATION TAG swap library."

Further, one can utilize the same protein degradation tag ladder comprising protein degradation tag $PDT_1$-$PDT_8$ to engineer microbes, wherein each of the eight protein degradation tags is operably linked to 10 different gene targets. The result of this procedure would be 80 host cell strains that are otherwise assumed genetically identical, except for the particular protein degradation tags operably linked to a target gene of interest. These 80 host cell strains could be appropriately screened and characterized and give rise to another HTP genetic design library. The characterization of the microbial strains in the HTP genetic design library produces information and data that can be stored in any database, including without limitation, a relational database, an object-oriented database or a highly distributed NoSQL database. This data/information could include, for example, a given protein degradation tag (e.g., $PDT_1$-$PDT_8$) effect when operably linked to a given gene target. This data/information can also be the broader set of combinatorial effects that result from operably linking two or more degradation tags (e.g., $PDT_1$-$PDT_8$) to a given gene target.

The aforementioned examples of eight protein degradation tags and 10 target genes is merely illustrative, as the concept can be applied with any given number of protein degradation tags that have been grouped together based upon exhibition of a range of degradation strengths and any given number of target genes.

In summary, utilizing various protein degradation tags to modulate degradation of various proteins in an organism is a powerful tool to optimize a trait of interest. The molecular tool of protein degradation tag swapping, developed by the inventors, uses a ladder of protein degradation tag sequences that have been demonstrated to vary degradation (e.g., enhance) of at least one protein under at least one condition. This ladder is then systematically applied to a group of genes in the organism using high-throughput genome engineering. This group of genes is determined to have a high likelihood of impacting the trait of interest based on any one of a number of methods. These could include selection based on known function, or impact on the trait of interest, or algorithmic selection based on previously determined beneficial genetic diversity.

The resultant HTP genetic design microbial library of organisms containing a protein degradation tag sequence linked to a gene is then assessed for performance in a high-throughput screening model, and protein degradation tag -gene linkages which lead to increased performance are determined and the information stored in a database. The collection of genetic perturbations *(i.e.* given protein degradation tag *x* linked to a given gene y) form a "protein degradation tag swap library," which can be utilized as a source of potential genetic alterations to be utilized in microbial engineering processing. Over time, as a greater set of genetic perturbations is implemented against a greater diversity of microbial backgrounds, each library becomes more powerful as a corpus of experimentally confirmed data that can be used to more precisely and predictably design targeted changes against any background of interest. That is in some examples, the present disclosures teaches introduction of one or more genetic changes into a host cell based on previous experimental results embedded within the meta data associated with any of the genetic design libraries of the disclosure.

Thus, in particular examples, protein degradation tag swapping is a multi-step process comprising:

1. Selecting a set of "x" protein degradation tags to act as a "ladder." Ideally these protein degradation tags have been shown to lead to enhanced protein degradation across multiple genomic loci, but the only requirement is that they perturb degradation in some way.
2. Selecting a set of "n" genes to target. This set can be every ORF in a genome, or a subset of ORFs. The subset can be chosen using annotations on ORFs related to function, by relation to previously demonstrated beneficial perturbations (previous promoter swaps, STOP swaps, SOLUBILITY TAG swaps or SNP swaps), by algorithmic selection based on epistatic interactions between previously generated perturbations, other selection criteria based on hypotheses regarding beneficial ORF to target, or through random selection.
3. High-throughput strain engineering to rapidly and in parallel carry out the following genetic modifications: When a native protein degradation tag exists within a target gene n and its sequence is known, replace the native protein degradation tag with each of the x protein degradation tags in the ladder. When the native protein degradation tag does not exist, or its sequence is unknown, insert each of the x protein degradation tags in the ladder.
In this way a "library" (also referred to as a HTP genetic design library) of strains is constructed, wherein each member of the library is an instance of x protein degradation tag linked to n target, in an otherwise identical genetic context. As previously described, combinations of protein degradation tags can be inserted, extending the range of combinatorial possibilities upon which the library is constructed.
**4.** High-throughput screening of the library of strains in a context where their performance against one or more metrics is indicative of the performance that is being optimized.

This foundational process can be extended to provide further improvements in strain performance by, *inter alia:* (1) Consolidating multiple beneficial perturbations into a single strain background, either one at a time in an interactive process, or as multiple changes in a single step. Multiple perturbations can be either a specific set of defined changes or a partly randomized, combinatorial library of changes. For example, if the set of targets is every gene in a pathway, then sequential regeneration of the library of perturbations into an improved member or members of the previous library of strains can optimize the expression level of each gene in a pathway regardless of which genes are rate limiting at any given iteration; (2) Feeding the performance data resulting from the individual and combinatorial generation of the library into an algorithm that uses that data to predict an optimum set of perturbations based on the interaction of each perturbation; and (3) Implementing a combination of the above two approaches.

The approach is exemplified in the present disclosure with industrial microorganisms, but is applicable to any organism where desired traits can be identified in a population of genetic mutants. For example, this could be used for improving the performance of CHO cells, yeast, insect cells, algae, as well as multi-cellular organisms, such as plants.

### *8. Epistasis Mapping - A Predictive Analytical Tool Enabling Beneficial Genetic Consolidations*

In some examples, the present disclosure teaches epistasis mapping methods for predicting and combining beneficial genetic alterations into a host cell. The genetic alterations may be created by any of the aforementioned HTP molecular tool sets (e.g., promoter swaps, SNP swaps, start/stop codon exchanges, sequence optimization, protein solubility tag swaps, protein degradation tag swaps and STOP swaps) and the effect of those genetic alterations would be known from the characterization of the derived HTP genetic design microbial strain libraries. Thus, as used herein, the term epistasis mapping includes methods of identifying combinations of genetic alterations (e.g., beneficial SNPs or beneficial promoter/target gene associations) that are likely to yield increases in host performance.

In examples, the epistasis mapping methods of the present disclosure are based on the idea that the combination of beneficial mutations from two different functional groups is more likely to improve host performance, as compared to a combination of mutations from the same functional group. *See, e.g.,* Costanzo, The Genetic Landscape of a Cell, Science, Vol. 327, Issue 5964, Jan. 22, 2010, pp. 425-431.

Mutations from the same functional group are more likely to operate by the same mechanism, and are thus more likely to exhibit negative or neutral epistasis on overall host performance. In contrast, mutations from different functional groups are more likely to operate by independent mechanisms, which can lead to improved host performance and in some instances synergistic effects. For example, referring to Figure 19, lysA and zwf are genes that operate in different pathways to achieve the production of lysine. Based upon the dissimilarity in the individual performance of those genes, genetic changes using those genes should result in additive consolidation effects. This was borne out in the actual measurement of the consolidated effects of the combination of lysA and zwf, as shown in Figure 16B and Example 6.

Thus, in some examples, the present disclosure teaches methods of analyzing SNP mutations to identify SNPs predicted to belong to different functional groups. In some examples, SNP functional group similarity is determined by computing the cosine similarity of mutation interaction profiles (similar to a correlation coefficient, *see* Figure 16A). The present disclosure also illustrates comparing SNPs *via* a mutation similarity matrix *(see* Figure 15 for example analysis conducted in Corynebacterium) or dendrogram *(see* Figure 16A for example analysis conducted in Corynebacterium).

Thus, the epistasis mapping procedure provides a method for grouping and/or ranking a diversity of genetic mutations applied in one or more genetic backgrounds for the purposes of efficient and effective consolidations of said mutations into one or more genetic backgrounds.

In examples, consolidation is performed with the objective of creating novel strains which are optimized for the production of target biomolecules. Through the taught epistasis mapping procedure, it is possible to identify functional groupings of mutations, and such functional groupings enable a consolidation strategy that minimizes undesirable epistatic effects.

As previously explained, the optimization of microbes for use in industrial fermentation is an important and difficult problem, with broad implications for the economy, society, and the natural world. Traditionally, microbial engineering has been performed through a slow and uncertain process of random mutagenesis. Such approaches leverage the natural evolutionary capacity of cells to adapt to artificially imposed selection pressure. Such approaches are also limited by the rarity of beneficial mutations, the ruggedness of the underlying fitness landscape, and more generally underutilize the state of the art in cellular and molecular biology.

Modern approaches leverage new understanding of cellular function at the mechanistic level and new molecular biology tools to perform targeted genetic manipulations to specific phenotypic ends. In practice, such rational approaches are confounded by the underlying complexity of biology. Causal mechanisms are poorly understood, particularly when attempting to combine two or more changes that each has an observed beneficial effect. Sometimes such consolidations of genetic changes yield positive outcomes (measured by increases in desired phenotypic activity), although the net positive outcome may be lower than expected and in some cases higher than expected. In other instances, such combinations produce either net neutral effect or a net negative effect. This phenomenon is referred to as epistasis, and is one of the fundamental challenges to microbial engineering (and genetic engineering generally).

As aforementioned, the present HTP genomic engineering platform solves many of the problems associated with traditional microbial engineering approaches. The present HTP platform uses automation technologies to perform hundreds or thousands of genetic mutations at once. In particular examples, unlike the rational approaches described above, the disclosed HTP platform enables the parallel construction of thousands of mutants to more effectively explore large subsets of the relevant genomic space, as disclosed in U.S. Application No. 15/140,296, entitled Microbial Strain Design System And Methods For Improved Large-Scale Production Of Engineered Nucleotide Sequences. By trying "everything," the present HTP platform sidesteps the difficulties induced by our limited biological understanding.

However, at the same time, the present HTP platform faces the problem of being fundamentally limited by the combinatorial explosive size of genomic space, and the effectiveness of computational techniques to interpret the generated data sets given the complexity of genetic interactions. Techniques are needed to explore subsets of vast combinatorial spaces in ways that maximize non-random selection of combinations that yield desired outcomes.

Somewhat similar HTP approaches have proved effective in the case of enzyme optimization. In this niche problem, a genomic sequence of interest (on the order of 1000 bases), encodes a protein chain with some complicated physical configuration. The precise configuration is determined by the collective electromagnetic interactions between its constituent atomic components. This combination of short genomic sequence and physically constrained folding problem lends itself specifically to greedy optimization strategies. That is, it is possible to individually mutate the sequence at every residue and shuffle the resulting mutants to effectively sample local sequence space at a resolution compatible with the Sequence Activity Response modeling.

However, for full genomic optimizations for biomolecules, such residue-centric approaches are insufficient for some important reasons. *First,* because of the exponential increase in relevant sequence space associated with genomic optimizations for biomolecules. *Second,* because of the added complexity of regulation, expression, and metabolic interactions in biomolecule synthesis. The present inventors have solved these problems *via* the taught epistasis mapping procedure.

The taught method for modeling epistatic interactions, between a collection of mutations for the purposes of more efficient and effective consolidation of said mutations into one or more genetic backgrounds, is groundbreaking and highly needed in the art.

When describing the epistasis mapping procedure, the terms "more efficient" and "more effective" refers to the avoidance of undesirable epistatic interactions among consolidation strains with respect to particular phenotypic objectives.

As the process has been generally elaborated upon above, a more specific workflow example will now be described.

*First,* one begins with a library of M mutations and one or more genetic backgrounds *(e.g.,* parent bacterial strains). Neither the choice of library nor the choice of genetic backgrounds is specific to the method described here. But in a particular implementation, a library of mutations may include exclusively, or in combination: SNP swap libraries, Promoter swap libraries, or any other mutation library described herein.

In one implementation, only a single genetic background is provided. In this case, a collection of distinct genetic backgrounds (microbial mutants) will first be generated from this single background. This may be achieved by applying the primary library of mutations (or some subset thereof) to the given background for example, application of a HTP genetic design library of particular SNPs or a HTP genetic design library of particular promoters to the given genetic background, to create a population (perhaps 100's or 1,000's) of microbial mutants with an identical genetic background except for the particular genetic alteration from the given HTP genetic design library incorporated therein. As detailed below, this example can lead to a combinatorial library or pairwise library.

In another implementation, a collection of distinct known genetic backgrounds may simply be given. As detailed below, this example can lead to a subset of a combinatorial library.

In a particular implementation, the number of genetic backgrounds and genetic diversity between these backgrounds (measured in number of mutations or sequence edit distance or the like) is determined to maximize the effectiveness of this method.

A genetic background may be a natural, native or wild-type strain or a mutated, engineered strain. N distinct background strains may be represented by a vector **b.** In one example, the background **b** may represent engineered backgrounds formed by applying N primary mutations $\mathbf{m_0}$ = ($m_1$, $m_2$, ... $m_N$) to a wild-type background strain $b_0$ to form the N mutated background strains **b** = $\mathbf{m_0}$ $b_0$ = ($m_1b_0$, $m_2b_0$, ... $m_N$ bo), where $m_ib_0$ represents the application of mutation $m_i$ to background strain $b_0$.

In either case (*i.e.* a single provided genetic background or a collection of genetic backgrounds), the result is a collection of N genetically distinct backgrounds. Relevant phenotypes are measured for each background.

*Second,* each mutation in a collection of M mutations $\mathbf{m_1}$ is applied to each background within the collection of N background strains **b** to form a collection of M x N mutants. In the implementation where the N backgrounds were themselves obtained by applying the primary set of mutations $\mathbf{m_0}$ (as described above), the resulting set of mutants will sometimes be referred to as a combinatorial library or a pairwise library. In another implementation, in which a collection of known backgrounds has been provided explicitly, the resulting set of mutants may be referred to as a subset of a combinatorial library. Similar to generation of engineered background vectors, in examples, the input interface 202 (see, Figure 31) receives the mutation vector $\mathbf{m_1}$ and the background vector **b**, and a specified operation such as cross product.

Continuing with the engineered background example above, forming the MxN combinatorial library may be represented by the matrix formed by $\mathbf{m}_1$ x $\mathbf{m}_0$ $b_0$, the cross product of $\mathbf{m}_1$ applied to the N backgrounds of **b** = $\mathbf{m}_0$ $\mathbf{b}_0$, where each mutation in $\mathbf{m}_1$ is applied to each background strain within **b**. Each ith row of the resulting MxN matrix represents the application of the ith mutation within $\mathbf{m}_1$ to all the strains within background collection **b.** In one example, $\mathbf{m}_1$ = $\mathbf{m_0}$ and the matrix represents the pairwise application of the same mutations to starting strain $b_0$. In that case, the matrix is symmetric about its diagonal (M=N), and the diagonal may be ignored in any analysis since it represents the application of the same mutation twice.

In examples, forming the MxN matrix may be achieved by inputting into the input interface 202 (see, Figure 31) the compound expression $\mathbf{m}_1$ x $\mathbf{m}_0b_0$. The component vectors of the expression may be input directly with their elements explicitly specified, via one or more DNA specifications, or as calls to the library 206 to enable retrieval of the vectors during interpretation by interpreter 204. As described in U.S. Patent Application, Serial No. 15/140,296, entitled "Microbial Strain Design System and Methods for Improved Large Scale Production of Engineered Nucleotide Sequences," via the interpreter 204, execution engine 207, order placement engine 208, and factory 210, the LIMS system 200 generates the microbial strains specified by the input expression.

*Third,* with reference to Figure 42, the analysis equipment 214 (see, Figure 31) measures phenotypic responses for each mutant within the MxN combinatorial library matrix (4202). As such, the collection of responses can be construed as an M x N Response Matrix R. Each element of **R** may be represented as $r_{ij} = y(m_i, m_j)$, where y represents the response (performance) of background strain bj within engineered collection **b** as mutated by mutation $m_i$. For simplicity, and practicality, we assume pairwise mutations where $\mathbf{m_1} = \mathbf{m_0}$**.** Where, as here, the set of mutations represents a pairwise mutation library, the resulting matrix may also be referred to as a gene interaction matrix or, more particularly, as a mutation interaction matrix.

Those skilled in the art will recognize that, in some examples, operations related to epistatic effects and predictive strain design may be performed entirely through automated means of the LIMS system 200, e.g., by the analysis equipment 214 (see, Figure 31), or by human implementation, or through a combination of automated and manual means. When an operation is not fully automated, the elements of the LIMS system 200, e.g., analysis equipment 214, may, for example, receive the results of the human performance of the operations rather than generate results through its own operational capabilities. As described elsewhere herein, components of the LIMS system 200, such as the analysis equipment 214, may be implemented wholly or partially by one or more computer systems. In some examples, in particular where operations related to predictive strain design are performed by a combination of automated and manual means, the analysis equipment 214 may include not only computer hardware, software or firmware (or a combination thereof), but also equipment operated by a human operator such as that listed in Table 5 below, e.g., the equipment listed under the category of "Evaluate performance."

*Fourth,* the analysis equipment 212 (see, Figure 31) normalizes the response matrix. Normalization consists of a manual and/or, in this example, automated processes of adjusting measured response values for the purpose of removing bias and/or isolating the relevant portions of the effect specific to this method. With respect to Figure 42, the first step 4202 may include obtaining normalized measured data. In general, in the claims directed to predictive strain design and epistasis mapping, the terms "performance measure" or "measured performance" or the like may be used to describe a metric that reflects measured data, whether raw or processed in some manner, e.g., normalized data. In a particular implementation, normalization may be performed by subtracting a previously measured background response from the measured response value. In that implementation, the resulting response elements may be formed as $r_{ij} = y(m_i, m_j) - y(m_j)$, where $y(m_j)$ is the response of the engineered background strain bj within engineered collection **b** caused by application of primary mutation $m_j$ to parent strain $\mathbf{b_0}$**.** Note that each row of the normalized response matrix is treated as a response profile for its corresponding mutation. That is, the ith row describes the relative effect of the corresponding mutation $m_i$ applied to all the background strains $b_j$ for j=1 to N.

With respect to the example of pairwise mutations, the combined performance/response of strains resulting from two mutations may be greater than, less than, or equal to the performance/response of the strain to each of the mutations individually. This effect is known as "epistasis," and may, in some examples, be represented as $e_{ij} = y(m_i, m_j) - (y(m_i) + y(m_j))$. Variations of this mathematical representation are possible, and may depend upon, for example, how the individual changes biologically interact. As noted above, mutations from the same functional group are more likely to operate by the same mechanism, and are thus more likely to exhibit negative or neutral epistasis on overall host performance. In contrast, mutations from different functional groups are more likely to operate by independent mechanisms, which can lead to improved host performance by reducing redundant mutative effects, for example. Thus, mutations that yield dissimilar responses are more likely to combine in an additive manner than mutations that yield similar responses. This leads to the computation of similarity in the next step.

*Fifth,* the analysis equipment 214 measures the similarity among the responses-in the pairwise mutation example, the similarity between the effects of the ith mutation and jth (e.g., primary) mutation within the response matrix (4204). Recall that the ith row of **R** represents the performance effects of the ith mutation $m_i$ on the N background strains, each of which may be itself the result of engineered mutations as described above. Thus, the similarity between the effects of the ith and jth mutations may be represented by the similarity $S_{ij}$ between the ith and jth rows, $\rho_i$ and $p_j$, respectively, to form a similarity matrix **S,** an example of which is illustrated in Figure 15. Similarity may be measured using many known techniques, such as cross-correlation or absolute cosine similarity, e.g., $S_{ij} = abs(cos(\rho_i, pj))$.

As an alternative or supplement to a metric like cosine similarity, response profiles may be clustered to determine degree of similarity. Clustering may be performed by use of a distance-based clustering algorithms (e.g. k-mean, hierarchical agglomerative, etc.) in conjunction with suitable distance measure (e.g. Euclidean, Hamming, etc). Alternatively, clustering may be performed using similarity based clustering algorithms (e.g. spectral, min-cut, etc.) with a suitable similarity measure (e.g. cosine, correlation, etc). Of course, distance measures may be mapped to similarity measures and vice-versa via any number of standard functional operations (e.g., the exponential function). In one implementation, hierarchical agglomerative clustering may be used in conjunction absolute cosine similarity. (See Figure 16A for example analysis conducted in *Corynebacterium*).

As an example of clustering, let C be a clustering of mutations $m_i$ into k distinct clusters. Let **C** be the cluster membership matrix, where $c_{ij}$ is the degree to which mutation i belongs to cluster j, a value between 0 and 1. The cluster-based similarity between mutations i and j is then given by $\mathbf{C}_i \times \mathbf{C}_j$ (the dot product of the ith and jth rows of **C**). In general, the cluster-based similarity matrix is given by $\mathbf{CC}^T$ (that is, **C** times **C**-transpose). In the case of hard-clustering (a mutation belongs to exactly one cluster), the similarity between two mutations is 1 if they belong to the same cluster and 0 if not.

As is described in Costanzo, The Genetic Landscape of a Cell, Science, Vol. 327, Issue 5964, Jan. 22, 2010, pp. 425-431, such a clustering of mutation response profiles relates to an approximate mapping of a cell's underlying functional organization. That is, mutations that cluster together tend to be related by an underlying biological process or metabolic pathway. Such mutations are referred to herein as a "functional group." The key observation of this method is that if two mutations operate by the same biological process or pathway, then observed effects (and notably observed benefits) may be redundant. Conversely, if two mutations operate by distant mechanism, then it is less likely that beneficial effects will be redundant.

*Sixth,* based on the epistatic effect, the analysis equipment 214 selects pairs of mutations that lead to dissimilar responses, e.g., their cosine similarity metric falls below a similarity threshold, or their responses fall within sufficiently separated clusters, (e.g., in Figure 15 and Figure 16A for example analyses conducted in *Corynebacterium)* as shown in Figure 42 (4206). Based on their dissimilarity, the selected pairs of mutations should consolidate into background strains better than similar pairs.

Based upon the selected pairs of mutations that lead to sufficiently dissimilar responses, the LIMS system (e.g., all of or some combination of interpreter 204, execution engine 207, order placer 208, and factory 210) may be used to design microbial strains having those selected mutations (4208). In examples, as described below and elsewhere herein, epistatic effects may be built into, or used in conjunction with the predictive model to weight or filter strain selection.

It is assumed that it is possible to estimate the performance (a.k.a. score) of a hypothetical strain obtained by consolidating a collection of mutations from the library into a particular background *via* some preferred predictive model. A representative predictive model utilized in the taught methods is provided in the below section entitled "Predictive Strain Design" that is found in the larger section of: "Computational Analysis and Prediction of Effects of Genome-Wide Genetic Design Criteria."

When employing a predictive strain design technique such as linear regression, the analysis equipment 214 may restrict the model to mutations having low similarity measures by, e.g., filtering the regression results to keep only sufficiently dissimilar mutations. Alternatively, the predictive model may be weighted with the similarity matrix. For example, some examples may employ a weighted least squares regression using the similarity matrix to characterize the interdependencies of the proposed mutations. As an example, weighting may be performed by applying the "kernel" trick to the regression model. (To the extent that the "kernel trick" is general to many machine learning modeling approaches, this re-weighting strategy is not restricted to linear regression.)

Such methods are known to one skilled in the art. In examples, the kernel is a matrix having elements 1 - w * $s_{ij}$ where 1 is an element of the identity matrix, and w is a real value between 0 and 1. When w = 0, this reduces to a standard regression model. In practice, the value of w will be tied to the accuracy ($r^2$ value or root mean square error (RMSE)) of the predictive model when evaluated against the pairwise combinatorial constructs and their associate effects $y(m_i, m_j)$. In one simple implementation, w is defined as $w = 1 - r^2$. In this case, when the model is fully predictive, $w=1-r^2=0$ and consolidation is based solely on the predictive model and epistatic mapping procedure plays no role. On the other hand, when the predictive model is not predictive at all, $w=1-r^2=1$ and consolidation is based solely on the epistatic mapping procedure. During each iteration, the accuracy can be assessed to determine whether model performance is improving.

It should be clear that the epistatic mapping procedure described herein does not depend on which model is used by the analysis equipment 214. Given such a predictive model, it is possible to score and rank all hypothetical strains accessible to the mutation library *via* combinatorial consolidation.

In some examples, to account for epistatic effects, the dissimilar mutation response profiles may be used by the analysis equipment 214 to augment the score and rank associated with each hypothetical strain from the predictive model. This procedure may be thought of broadly as a re-weighting of scores, so as to favor candidate strains with dissimilar response profiles (e.g., strains drawn from a diversity of clusters). In one simple implementation, a strain may have its score reduced by the number of constituent mutations that do not satisfy the dissimilarity threshold or that are drawn from the same cluster (with suitable weighting). In a particular implementation, a hypothetical strain's performance estimate may be reduced by the sum of terms in the similarity matrix associated with all pairs of constituent mutations associated with the hypothetical strain (again with suitable weighting). Hypothetical strains may be re-ranked using these augmented scores. In practice, such re-weighting calculations may be performed in conjunction with the initial scoring estimation.

The result is a collection of hypothetical strains with score and rank augmented to more effectively avoid confounding epistatic interactions. Hypothetical strains may be constructed at this time, or they may be passed to another computational method for subsequent analysis or use.

Those skilled in the art will recognize that epistasis mapping and iterative predictive strain design as described herein are not limited to employing only pairwise mutations, but may be expanded to the simultaneous application of many more mutations to a background strain. In another example, additional mutations may be applied sequentially to strains that have already been mutated using mutations selected according to the predictive methods described herein. In another example, epistatic effects are imputed by applying the same genetic mutation to a number of strain backgrounds that differ slightly from each other, and noting any significant differences in positive response profiles among the modified strain backgrounds.

**Organisms Amenable to Genetic Design**

The disclosed HTP genomic engineering platform is exemplified with industrial microbial cell cultures (e.g., *Corynebacterium),* but is applicable to any host cell organism where desired traits can be identified in a population of genetic mutants.

Further, as set forth in the introduction, the current disclosure provides for a HTP genomic engineering platform to improve host cell characteristics in *E. coli* systems and solves many problems that have previously prevented the development of such a system in *E. coli.*

Thus, as used herein, the term "microorganism" should be taken broadly. It includes, but is not limited to, the two prokaryotic domains, Bacteria and Archaea, as well as certain eukaryotic fungi and protists. However, in certain examples, "higher" eukaryotic organisms such as insects, plants, and animals can be utilized in the methods taught herein.

Suitable host cells include, but are not limited to: bacterial cells, algal cells, plant cells, fungal cells, insect cells, and mammalian cells. In one illustrative example, suitable host cells include E. *coli (e.g.,* SHuffle™ competent *E. coli* available from New England BioLabs in Ipswich, Mass.). The *E. coli* genome is 4,646,332 bp in size (see Figure 52).

Suitable host strains of the *E. coli* species comprise: Enterotoxigenic *E. coli* (ETEC), Enteropathogenic *E. coli* (EPEC), Enteroinvasive *E. coli* (EIEC), Enterohemorrhagic *E. coli* (EHEC), Uropathogenic E. *coli* (UPEC), Verotoxin-producing *E. coli, E. coli* O157:H7, *E. coli* O104:H4, *Escherichia coli* 0121, *Escherichia coli* O104:H21, *Escherichia coli* K1, and Escherichia coli NC101. In some examples, the present disclosure teaches genomic engineering of *E. coli* K12, *E. coli* B, and *E. coli C.*

In some examples, the present disclosure teaches genomic engineering of *E. coli* strains NCTC 12757, NCTC 12779, NCTC 12790, NCTC 12796, NCTC 12811, ATCC 11229, ATCC 25922, ATCC 8739, DSM 30083, BC 5849, BC 8265, BC 8267, BC 8268, BC 8270, BC 8271, BC 8272, BC 8273, BC 8276, BC 8277, BC 8278, BC 8279, BC 8312, BC 8317, BC 8319, BC 8320, BC 8321, BC 8322, BC 8326, BC 8327, BC 8331, BC 8335, BC 8338, BC 8341, BC 8344, BC 8345, BC 8346, BC 8347, BC 8348, BC 8863, and BC 8864.

In some examples, the present disclosure teaches verocytotoxigenic *E. coli* (VTEC), such as strains BC 4734 (O26:H11), BC 4735 (O157:H-), BC 4736 , BC 4737 (n.d.), BC 4738 (O157:H7), BC 4945 (O26:H-), BC 4946 (O157:H7), BC 4947 (O111:H-), BC 4948 (O157:H), BC 4949 (O5), BC 5579 (O157:H7), BC 5580 (O157:H7), BC 5582 (O3:H), BC 5643 (O2:H5), BC 5644 (0128), BC 5645 (O55:H-), BC 5646 (O69:H-), BC 5647 (O101:H9), BC 5648 (O103:H2), BC 5850 (O22:H8), BC 5851 (O55:H-), BC 5852 (O48:H21), BC 5853 (O26:H11), BC 5854 (O157:H7), BC 5855 (O157:H-), BC 5856 (O26:H-), BC 5857 (O103:H2), BC 5858 (O26:H11), BC 7832, BC 7833 (O raw form:H-), BC 7834 (ONT:H-), BC 7835 (O103:H2), BC 7836 (O57:H-), BC 7837 (ONT:H-), BC 7838, BC 7839 (O128:H2), BC 7840 (O157:H-), BC 7841 (O23:H-), BC 7842 (O157:H-), BC 7843, BC 7844 (O157:H-), BC 7845 (O103:H2), BC 7846 (O26:H11), BC 7847 (O145:H-), BC 7848 (O157:H-), BC 7849 (O156:H47), BC 7850, BC 7851 (O157:H-), BC 7852 (O157:H-), BC 7853 (O5:H-), BC 7854 (O157:H7), BC 7855 (O157:H7), BC 7856 (O26:H-), BC 7857, BC 7858, BC 7859 (ONT:H-), BC 7860 (O129:H-), BC 7861, BC 7862 (O103:H2), BC 7863, BC 7864 (O raw form:H-), BC 7865, BC 7866 (O26:H-), BC 7867 (O raw form:H-), BC 7868, BC 7869 (ONT:H-), BC 7870 (O113:H-), BC 7871 (ONT:H-), BC 7872 (ONT:H-), BC 7873, BC 7874 (O raw form:H-), BC 7875 (O157:H-), BC 7876 (O111:H-), BC 7877 (O146:H21), BC 7878 (O145:H-), BC 7879 (O22:H8), BC 7880 (O raw form:H-), BC 7881 (O145:H-), BC 8275 (O157:H7), BC 8318 (O55:K-:H-), BC 8325 (O157:H7), and BC 8332 (ONT), BC 8333.

In some examples, the present disclosure teaches enteroinvasive *E. coli* (EIEC), such as strains BC 8246 (O152:K-:H-), BC 8247 (O124:K(72):H3), BC 8248 (0124), BC 8249 (0112), BC 8250 (O136:K(78):H-), BC 8251 (O124:H-), BC 8252 (O144:K-:H-), BC 8253 (O143:K:H-), BC 8254 (0143), BC 8255 (0112), BC 8256 (O28a.e), BC 8257 (O124:H-), BC 8258 (0143), BC 8259 (O167:K-:H5), BC 8260 (O128a.c.:H35), BC 8261 (0164), BC 8262 (O164:K-:H-), BC 8263 (0164), and BC 8264 (0124).

In some examples, the present disclosure teaches enterotoxigenic *E. coli (ETEC),* such as strains BC 5581 (078:H11), BC 5583 (O2:K1), BC 8221 (0118), BC 8222 (O148:H-), BC 8223 (O111), BC 8224 (O110:H-), BC 8225 (0148), BC 8226 (0118), BC 8227 (O25:H42), BC 8229 (06), BC 8231 (O153:H45), BC 8232 (09), BC 8233 (0148), BC 8234 (0128), BC 8235 (0118), BC 8237 (O111), BC 8238 (O110:H17), BC 8240 (0148), BC 8241 (O6H16), BC 8243 (0153), BC 8244 (O15:H-), BC 8245 (020), BC 8269 (O125a.c:H-), BC 8313 (O6:H6), BC 8315 (O153:H-), BC 8329, BC 8334 (O118:H12), and BC 8339.

In some examples, the present disclosure teaches enteropathogenic *E. coli* (EPEC), such as strains BC 7567 (O86), BC 7568 (O128), BC 7571 (O114), BC 7572 (O119), BC 7573 (0125), BC 7574 (0124), BC 7576 (O127a), BC 7577 (0126), BC 7578 (0142), BC 7579 (O26), BC 7580 (OK26), BC 7581 (0142), BC 7582 (O55), BC 7583 (O158), BC 7584 (O-), BC 7585 (O-), BC 7586 (O-), BC 8330, BC 8550 (O26), BC 8551 (O55), BC 8552 (O158), BC 8553 (O26), BC 8554 (O158), BC 8555 (O86), BC 8556 (O128), BC 8557 (OK26), BC 8558 (O55), BC 8560 (O158), BC 8561 (O158), BC 8562 (O114), BC 8563 (O86), BC 8564 (O128), BC 8565 (O158), BC 8566 (O158), BC 8567 (O158), BC 8568 (O111), BC 8569 (O128), BC 8570 (O114), BC 8571 (O128), BC 8572 (O128), BC 8573 (O158), BC 8574 (O158), BC 8575 (O158), BC 8576 (O158), BC 8577 (O158), BC 8578 (O158), BC 8581 (O158), BC 8583 (O128), BC 8584 (O158), BC 8585 (O128), BC 8586 (O158), BC 8588 (O26), BC 8589 (O86), BC 8590 (0127), BC 8591 (O128), BC 8592 (O114), BC 8593 (O114), BC 8594 (O114), BC 8595 (0125), BC 8596 (O158), BC 8597 (O26), BC 8598 (O26), BC 8599 (O158), BC 8605 (O158), BC 8606 (O158), BC 8607 (O158), BC 8608 (O128), BC 8609 (O55), BC 8610 (O114), BC 8615 (O158), BC 8616 (O128), BC 8617 (O26), BC 8618 (O86), BC 8619, BC 8620, BC 8621, BC 8622, BC 8623, BC 8624 (O158), and BC 8625 (0158).

In some examples, the present disclosure also teaches methods for the engineering of *Shigella* organisms, including *Shigella flexneri, Shigella dysenteriae, Shigella boydii,* and *Shigella sonnei.*

**Generating Genetic Diversity Pools for Utilization in the Genetic Design & HTP Microbial Engineering Platform**

In some examples, the methods of the present disclosure are characterized as genetic design. As used herein, the term genetic design refers to the reconstruction or alteration of a host organism's genome through the identification and selection of the most optimum variants of a particular gene, portion of a gene, promoter, stop codon, 5'UTR, 3'UTR, or other DNA sequence to design and create new superior host cells.

In some examples, a first step in the genetic design methods of the present disclosure is to obtain an initial genetic diversity pool population with a plurality of sequence variations from which a new host genome may be reconstructed.

In some examples, a subsequent step in the genetic design methods taught herein is to use one or more of the aforementioned HTP molecular tool sets *(e.g.* SNP swapping, promoter swapping, terminator swapping, protein solubility tag swapping or protein degradation tag swapping) to construct HTP genetic design libraries, which then function as drivers of the genomic engineering process, by providing libraries of particular genomic alterations for testing in a host cell.

.***Harnessing Diversity Pools From Existing Wild-type Strains***

In some examples, the present disclosure teaches methods for identifying the sequence diversity present among microbes of a given wild-type population. Therefore, a diversity pool can be a given number *n* of wild-type microbes utilized for analysis, with said microbes' genomes representing the "diversity pool."

In some examples, the diversity pools can be the result of existing diversity present in the natural genetic variation among said wild-type microbes. This variation may result from strain variants of a given host cell or may be the result of the microbes being different species entirely. Genetic variations can include any differences in the genetic sequence of the strains, whether naturally occurring or not. In some examples, genetic variations can include SNPs swaps, PRO swaps, Start/Stop Codon swaps, SOLUBILITY TAG swaps, DEGRADATION TAG swaps or STOP swaps, among others.

*Harnessing Diversity Pools From Existing Industrial Strain Variants*

In other examples of the present disclosure, diversity pools are strain variants created during traditional strain improvement processes (e.g., one or more host organism strains generated *via* random mutation and selected for improved yields over the years). Thus, in some examples, the diversity pool or host organisms can comprise a collection of historical production strains.

In particular examples, a diversity pool may be an original parent microbial strain ($S_1$) with a "baseline" genetic sequence at a particular time point ($S_1Gen_1$) and then any number of subsequent offspring strains ($S_2$, $S_3$, $S_4$, $S_5$, etc., generalizable to $S_{2-n}$) that were derived/developed from said $S_1$ strain and that have a different genome ($S_{2-n}Gen_{2-n}$), in relation to the baseline genome of Si.

For example, in some examples, the present disclosure teaches sequencing the microbial genomes in a diversity pool to identify the SNP's present in each strain. In one example, the strains of the diversity pool are historical microbial production strains. Thus, a diversity pool of the present disclosure can include for example, an industrial base strain, and one or more mutated industrial strains produced *via* traditional strain improvement programs.

Once all SNPs in the diversity pool are identified, the present disclosure teaches methods of SNP swapping and screening methods to delineate *(i.e.* quantify and characterize) the effects *(e.g.* creation of a phenotype of interest) of SNPs individually and in groups. Thus, as aforementioned, an initial step in the taught platform can be to obtain an initial genetic diversity pool population with a plurality of sequence variations, e.g. SNPs. Then, a subsequent step in the taught platform can be to use one or more of the aforementioned HTP molecular tool sets *(e.g.* SNP swapping) to construct HTP genetic design libraries, which then function as drivers of the genomic engineering process, by providing libraries of particular genomic alterations for testing in a microbe.

In some examples, the SNP swapping methods of the present disclosure comprise the step of introducing one or more SNPs identified in a mutated strain (*e.g.,* a strain from amongst $S_{2\text{-}n}Gen_{2\text{-}n}$) to a base strain ($S_1Gen_1$) or wild-type strain.

In other examples, the SNP swapping methods of the present disclosure comprise the step of removing one or more SNPs identified in a mutated strain (*e.g.,* a strain from amongst $S_{2\text{-}n}Gen_{2\text{-}n}$).

### *Creating Diversity Pools via Mutagenesis*

In some examples, the mutations of interest in a given diversity pool population of cells can be artificially generated by any means for mutating strains, including mutagenic chemicals, or radiation. The term "mutagenizing" is used herein to refer to a method for inducing one or more genetic modifications in cellular nucleic acid material.

The term "genetic modification" refers to any alteration of DNA. Representative gene modifications include nucleotide insertions, deletions, substitutions, and combinations thereof, and can be as small as a single base or as large as tens of thousands of bases. Thus, the term "genetic modification" encompasses inversions of a nucleotide sequence and other chromosomal rearrangements, whereby the position or orientation of DNA comprising a region of a chromosome is altered. A chromosomal rearrangement can comprise an intrachromosomal rearrangement or an interchromosomal rearrangement.

In one example, the mutagenizing methods employed in the presently claimed subject matter are substantially random such that a genetic modification can occur at any available nucleotide position within the nucleic acid material to be mutagenized. Stated another way, in one example, the mutagenizing does not show a preference or increased frequency of occurrence at particular nucleotide sequences.

The methods of the disclosure can employ any mutagenic agent including, but not limited to: ultraviolet light, X-ray radiation, gamma radiation, N-ethyl-N-nitrosourea (ENU), methyinitrosourea (MNU), procarbazine (PRC), triethylene melamine (TEM), acrylamide monomer (AA), chlorambucil (CHL), melphalan (MLP), cyclophosphamide (CPP), diethyl sulfate (DES), ethyl methane sulfonate (EMS), methyl methane sulfonate (MMS), 6-mercaptopurine (6-MP), mitomycin-C (MMC), N-methyl-N'-nitro-N-nitrosoguanidine (MNNG), $^3H_2O$, and urethane (UR) (See *e.g.,* Rinchik, 1991; Marker *et al.,* 1997; and Russell, 1990). Additional mutagenic agents are well known to persons having skill in the art, including those described in http://www.iephb.nw.ru/~spirov/hazard/mutagen_lst.html.

The term "mutagenizing" also encompasses a method for altering (e.g., by targeted mutation) or modulating a cell function, to thereby enhance a rate, quality, or extent of mutagenesis. For example, a cell can be altered or modulated to thereby be dysfunctional or deficient in DNA repair, mutagen metabolism, mutagen sensitivity, genomic stability, or combinations thereof. Thus, disruption of gene functions that normally maintain genomic stability can be used to enhance mutagenesis. Representative targets of disruption include, but are not limited to DNA ligase I (Bentley *et al.,* 2002) and casein kinase I (U.S. Pat. No. 6,060,296).

In some examples, site-specific mutagenesis (e.g., primer-directed mutagenesis using a commercially available kit such as the Transformer Site Directed mutagenesis kit (Clontech)) is used to make a plurality of changes throughout a nucleic acid sequence in order to generate nucleic acid encoding a cleavage enzyme of the present disclosure.

The frequency of genetic modification upon exposure to one or more mutagenic agents can be modulated by varying dose and/or repetition of treatment, and can be tailored for a particular application.

Thus, in some examples, "mutagenesis" as used herein comprises all techniques known in the art for inducing mutations, including error-prone PCR mutagenesis, oligonucleotide-directed mutagenesis, site-directed mutagenesis, and iterative sequence recombination by any of the techniques described herein.

### *Single Locus Mutations to Generate Diversity*

In some examples, the present disclosure teaches mutating cell populations by introducing, deleting, or replacing selected portions of genomic DNA. Thus, in some examples, the present disclosure teaches methods for targeting mutations to a specific locus. In other examples, the present disclosure teaches the use of gene editing technologies such as ZFNs, TALENS, Lambda Red or CRISPR, to selectively edit target DNA regions.

In other examples, the present disclosure teaches mutating selected DNA regions outside of the host organism, and then inserting the mutated sequence back into the host organism. For example, in some examples, the present disclosure teaches mutating native or synthetic promoters to produce a range of promoter variants with various expression properties *(see* promoter ladder *infra).* In other examples, the present disclosure is compatible with single gene optimization techniques, such as ProSAR (Fox et al. 2007. "Improving catalytic function by ProSAR-driven enzyme evolution."

Nature Biotechnology Vol 25 (3) 338-343).

In some examples, the selected regions of DNA are produced *in vitro* via gene shuffling of natural variants, or shuffling with synthetic oligos, plasmid-plasmid recombination, virus plasmid recombination, virus-virus recombination. In other examples, the genomic regions are produced via error-prone PCR.

In some examples, generating mutations in selected genetic regions is accomplished by "reassembly PCR." Briefly, oligonucleotide primers (oligos) are synthesized for PCR amplification of segments of a nucleic acid sequence of interest, such that the sequences of the oligonucleotides overlap the junctions of two segments. The overlap region is typically about 10 to 100 nucleotides in length. Each of the segments is amplified with a set of such primers. The PCR products are then "reassembled" according to assembly protocols. In brief, in an assembly protocol, the PCR products are first purified away from the primers, by, for example, gel electrophoresis or size exclusion chromatography. Purified products are mixed together and subjected to about 1-10 cycles of denaturing, reannealing, and extension in the presence of polymerase and deoxynucleoside triphosphates (dNTP's) and appropriate buffer salts in the absence of additional primers ("self-priming"). Subsequent PCR with primers flanking the gene are used to amplify the yield of the fully reassembled and shuffled genes.

In some examples of the disclosure, mutated DNA regions, such as those discussed above, are enriched for mutant sequences so that the multiple mutant spectrum, i.e. possible combinations of mutations, is more efficiently sampled. In some examples, mutated sequences are identified via a mutS protein affinity matrix (Wagner et al., Nucleic Acids Res. 23(19):3944-3948 (1995); Su et al., Proc. Natl. Acad. Sci. (U.S.A.), 83:5057-5061(1986)) with a preferred step of amplifying the affinity-purified material in vitro prior to an assembly reaction. This amplified material is then put into an assembly or reassembly PCR reaction as described in later portions of this application.

### *Promoter Ladders*

Promoters regulate the rate at which genes are transcribed and can influence transcription in a variety of ways. Constitutive promoters, for example, direct the transcription of their associated genes at a constant rate regardless of the internal or external cellular conditions, while regulatable promoters increase or decrease the rate at which a gene is transcribed depending on the internal and/or the external cellular conditions, e.g. growth rate, temperature, responses to specific environmental chemicals, and the like. Promoters can be isolated from their normal cellular contexts and engineered to regulate the expression of virtually any gene, enabling the effective modification of cellular growth, product yield and/or other phenotypes of interest.

In some examples, the present disclosure teaches methods for producing promoter ladder libraries for use in downstream genetic design methods. For example, in some examples, the present disclosure teaches methods of identifying one or more promoters and/or generating variants of one or more promoters within a host cell, which exhibit a range of expression strengths, or superior regulatory properties. A particular combination of these identified and/or generated promoters can be grouped together as a promoter ladder, which is explained in more detail below.

In some examples, the present disclosure teaches the use of promoter ladders. In some examples, the promoter ladders of the present disclosure comprise promoters exhibiting a continuous range of expression profiles. For example, in some examples, promoter ladders are created by: identifying natural, native, or wild-type promoters that exhibit a range of expression strengths in response to a stimuli, or through constitutive expression *(see* e.g., Figure 20 and Figures 28-30). These identified promoters can be grouped together as a promoter ladder.

In other examples, the present disclosure teaches the creation of promoter ladders exhibiting a range of expression profiles across different conditions. For example, in some examples, the present disclosure teaches creating a ladder of promoters with expression peaks spread throughout the different stages of a fermentation *(see e.g.,* Figure 28). In other examples, the present disclosure teaches creating a ladder of promoters with different expression peak dynamics in response to a specific stimulus *(see e.g.,* Figure 29). Persons skilled in the art will recognize that the regulatory promoter ladders of the present disclosure can be representative of any one or more regulatory profiles.

In some examples, the promoter ladders of the present disclosure are designed to perturb gene expression in a predictable manner across a continuous range of responses. In some examples, the continuous nature of a promoter ladder confers strain improvement programs with additional predictive power. For example, in some examples, swapping promoters or termination sequences of a selected metabolic pathway can produce a host cell performance curve, which identifies the most optimum expression ratio or profile; producing a strain in which the targeted gene is no longer a limiting factor for a particular reaction or genetic cascade, while also avoiding unnecessary over expression or misexpression under inappropriate circumstances. In some examples, promoter ladders are created by: identifying natural, native, or wild-type promoters exhibiting the desired profiles. Examples of native promoters for use in the methods provided herein can be found in Table 1.4. In other examples, the promoter ladders are created by mutating naturally occurring promoters to derive multiple mutated promoter sequences. Each of these mutated promoters is tested for effect on target gene expression. In some examples, the edited promoters are tested for expression activity across a variety of conditions, such that each promoter variant's activity is documented/characterized/annotated and stored in a database. The resulting edited promoter variants are subsequently organized into promoter ladders arranged based on the strength of their expression (e.g., with highly expressing variants near the top, and attenuated expression near the bottom, therefore leading to the term "ladder"). Examples of synthetic promoters for use in the methods provided herein can be found in Table 1.4.

In some examples, the present disclosure teaches promoter ladders that are a combination of identified naturally occurring promoters and mutated variant promoters.

In some examples, the present disclosure teaches methods of identifying natural, native, or wild-type promoters that satisfied both of the following criteria: 1) represented a ladder of constitutive promoters; and 2) could be encoded by short DNA sequences, ideally less than 100 base pairs. In some examples, constitutive promoters of the present disclosure exhibit constant gene expression across two selected growth conditions (typically compared among conditions experienced during industrial cultivation). An example of examining gene expression using different promoters provided herein can be found in Example 12. In some examples, the promoters of the present disclosure will consist of a ~60 base pair core promoter, and a 5' UTR between 26-and 40 base pairs in length.

Native promoters for inclusion in promoter ladders for use in the PROSWP methods provided herein can be selected based on said native promoter showing minimal variation in an associated gene's expression. Further, the native promoters can be 60-90 bps in length, and can consist of sequence that is located 50 bp in front of a putative transcription start site, and, optionally, the sequence up to but not including a putative start codon. Examples of native promoters for use in the methods provided herein can be found in Table 1.4. In particular, the native promoters for use in the methods provided herein can be selected from nucleic acid SEQ ID NOs 71-131 from Table 1.4.

In some examples, one or more of the aforementioned identified naturally occurring promoter sequences are chosen for gene editing. In some examples, the natural promoters are edited *via* any of the mutation methods described *supra.* In other examples, the promoters of the present disclosure are edited by synthesizing new promoter variants with the desired sequence.

Synthetic promoters for inclusion in promoter ladders for use in the PROSWP methods provided herein can be chimeric sequences 60-90 bps in length. The synthetic promoter libraries for use herein can comprise a set or plurality of synthetic promoters that can be designed and constructed such that they are likely to express constitutively and/or represent a range of expression strengths in comparison to one another. Further, the synthetic promoters can be designed and constructed such that they are unlikely to bind any regulatory elements present in *E. coli* and therefore drive gene expression constitutively.

To achieve these design goals, the chimeric synthetic promoters can comprise all or a combination of the elements found in Table 1.5. In particular, relative to a transcription start site, the synthetic promoters can comprise or consist of a distal region, a -35 region, a core region, a - 10 region and a 5'UTR/ribosomal binding site (RBS) region , as shown in **Figure 54.** The distal region can be located just upstream of the -35 region, while the core region can be located between the -35 and -10 regions as shown **Figure 54.** Both the distal and the core regions can be important for binding regulatory elements *(see* Cox et al., Mol Syst Biol. 2007; 3: 145). Since the lambda phage $p_R$ promoter is expected to drive expression constitutively, distal and core regions from this promoter can be used in the design strategy. The core region of the lambda phage $p_L$ promoter also be included for the same reason, as well as to add additional variety to the library.

The -35 and -10 regions can be included because they are known to be particularly important in prokaryotes for binding the RNA polymerase and can therefore be critical for modulating the degree of expression. In one example, the -35 and -10 regions from the lambda phage $p_R$ promoter and $p_L$ promoter are used. The -35 and -10 regions from $p_R$ and $p_L$ can be used since they are expected to drive strong expression. Additionally, -35 and -10 regions found in many native *E. coli* promoters can be used, whereby said -35 and -10 regions represent small variations to $p_R$ and $p_L$ and can be expected to decrease the promoter strength in comparison with $p_R$ and $p_L$. The variable 6 bp sequence constituting the -35 and -10 regions can be selected from the -35 and -10 seqeunces found in Table 1.5.

In addition to the above elements, the chimeric synthetic promoters can comprise a 5' untranslated region (5'-UTR) that includes a ribosome binding site (RBS), which can be particularly important in prokaryotes for binding the ribosome and thus be critical to modulating the degree of protein expression. In one example, the 5'-UTR/RBS from the native *E. coli* gene *acs* can be used to add additional variety to the library. In another example, the 5'UTR/RBS from the lambda phage $p_R$ promoter can be used.

Examples of synthetic promoters for use in the methods provided herein can be found in Table 1.4. In particular, the synthetic promoters for use in the methods provided herein can be selected from nucleic acid SEQ ID NOs 132-207 from Table 1.4.

The entire disclosure of U.S. Patent Application No. 62/264,232, filed on December 07, 2015.

A non-exhaustive list of the promoters of the present disclosure is provided in the below Table 1 and/or Table 1.4. Each of the promoter sequences can be referred to as a heterologous promoter or heterologous promoter polynucleotide.

Table 1. Selected promoter sequences of the present disclosure.

| SEQ ID No. | Promoter Short Name | Promoter Name |
|---|---|---|
| 1 | P1 | Pcg0007_lib_39 |
| 2 | P2 | Pcg0007 |
| 3 | P3 | Pcg1860 |
| 4 | P4 | Pcg0755 |
| 5 | P5 | Pcg0007_265 |
| 6 | P6 | Pcg3381 |
| 7 | P7 | Pcg0007_119 |
| 8 | P8 | Pcg3121 |

**Table** 1.4 Additional promoter sequences of the present disclosure.

| Promoter name | SEQ ID NO. | Type* |
|---|---|---|
| b0904 _promoter | 71 | Native |
| b2405 _promoter | 72 | Native |
| b0096 _promoter | 73 | Native |
| b0576_promoter | 74 | Native |
| b2017_promoter | 75 | Native |
| b1278_promoter | 76 | Native |
| b4255 _promoter | 77 | Native |
| b0786_promoter | 78 | Native |
| b0605_promoter | 79 | Native |
| b1824_promoter | 80 | Native |
| b1061_promoter | 81 | Native |
| b0313_promoter | 82 | Native |
| b0814_promoter | 83 | Native |
| b4133_promoter | 84 | Native |
| b4268_promoter | 85 | Native |
| b0345_promoter | 86 | Native |
| b2096_promoter | 87 | Native |
| b1277_promoter | 88 | Native |
| b1646_promoter | 89 | Native |
| b4177_promoter | 90 | Native |
| b0369_promoter | 91 | Native |
| b1920_promoter | 92 | Native |
| b3742_promoter | 93 | Native |
| b3929_promoter | 94 | Native |
| b3743_promoter | 95 | Native |
| b1613_promoter | 96 | Native |

(continued)

| Promoter name | SEQ ID NO. | Type* |
|---|---|---|
| b1749_promoter | 97 | Native |
| b2478_promoter | 98 | Native |
| b0031_promoter | 99 | Native |
| b2414_promoter | 100 | Native |
| b1183_promoter | 101 | Native |
| b0159_promoter | 102 | Native |
| b2837_promoter | 103 | Native |
| b3237_promoter | 104 | Native |
| b3778_promoter | 105 | Native |
| b2349_promoter | 106 | Native |
| b1434_promoter | 107 | Native |
| b3617_promoter | 108 | Native |
| b0237_promoter | 109 | Native |
| b4063_promoter | 110 | Native |
| b0564_promoter | 111 | Native |
| b0019_promoter | 112 | Native |
| b2375_promoter | 113 | Native |
| b1187_promoter | 114 | Native |
| b2388_promoter | 115 | Native |
| b1051_promoter | 116 | Native |
| b4241_promoter | 117 | Native |
| b4054_promoter | 118 | Native |
| b2425_promoter | 119 | Native |
| b0995_promoter | 120 | Native |
| b1399_promoter | 121 | Native |
| b3298_promoter | 122 | Native |
| b2114_promoter | 123 | Native |
| b2779_promoter | 124 | Native |
| b1114_promoter | 125 | Native |
| b3730_promoter | 126 | Native |
| b3025_promoter | 127 | Native |
| b0850_promoter | 128 | Native |
| b2365_promoter | 129 | Native |
| b4117_promoter | 130 | Native |
| b2213_promoter | 131 | Native |
| pMB029_promoter | 132 | Synthetic |
| pMB023_promoter | 133 | Synthetic |
| pMB025_promoter | 134 | Synthetic |

(continued)

| Promoter name | SEQ ID NO. | Type* |
|---|---|---|
| pMB019_promoter | 135 | Synthetic |
| pMB008_promoter | 136 | Synthetic |
| pMB020_promoter | 137 | Synthetic |
| pMB022_promoter | 138 | Synthetic |
| pMB089_promoter | 139 | Synthetic |
| pMB001_promoter | 140 | Synthetic |
| pMB051_promoter | 141 | Synthetic |
| pMB070_promoter | 142 | Synthetic |
| pMB074_promoter | 143 | Synthetic |
| pMB046_promoter | 144 | Synthetic |
| pMB071_promoter | 145 | Synthetic |
| pMB013_promoter | 146 | Synthetic |
| pMB080_promoter | 147 | Synthetic |
| pMB03 8_promoter | 148 | Synthetic |
| pMB060_promoter | 149 | Synthetic |
| pMB064_promoter | 150 | Synthetic |
| pMB058_promoter | 151 | Synthetic |
| pMB085_promoter | 152 | Synthetic |
| pMB081_promoter | 153 | Synthetic |
| pMB091_promoter | 154 | Synthetic |
| pMB027_promoter | 155 | Synthetic |
| pMB048_promoter | 156 | Synthetic |
| pMB055_promoter | 157 | Synthetic |
| pMB006_promoter | 158 | Synthetic |
| pMB012_promoter | 159 | Synthetic |
| pMB014_promoter | 160 | Synthetic |
| pMB028_promoter | 161 | Synthetic |
| pMB059_promoter | 162 | Synthetic |
| pMB061_promoter | 163 | Synthetic |
| pMB043_promoter | 164 | Synthetic |
| pMB066_promoter | 165 | Synthetic |
| pMB079_promoter | 166 | Synthetic |
| pMB032_promoter | 167 | Synthetic |
| pMB068_promoter | 168 | Synthetic |
| pMB082_promoter | 169 | Synthetic |
| pMB030_promoter | 170 | Synthetic |
| pMB067_promoter | 171 | Synthetic |
| pMB050_promoter | 172 | Synthetic |

(continued)

| Promoter name | SEQ ID NO. | Type* |
|---|---|---|
| pMB069_promoter | 173 | Synthetic |
| pMB017_promoter | 174 | Synthetic |
| pMB039_promoter | 175 | Synthetic |
| pMB011_promoter | 176 | Synthetic |
| pMB072_promoter | 177 | Synthetic |
| pMB016_promoter | 178 | Synthetic |
| pMB077_promoter | 179 | Synthetic |
| pMB047_promoter | 180 | Synthetic |
| pMB052_promoter | 181 | Synthetic |
| pMB090_promoter | 182 | Synthetic |
| pMB035_promoter | 183 | Synthetic |
| pMB073_promoter | 184 | Synthetic |
| pMB004_promoter | 185 | Synthetic |
| pMB054_promoter | 186 | Synthetic |
| pMB024_promoter | 187 | Synthetic |
| pMB007_promoter | 188 | Synthetic |
| pMB005_promoter | 189 | Synthetic |
| pMB003_promoter | 190 | Synthetic |
| pMB088_promoter | 191 | Synthetic |
| pMB065_promoter | 192 | Synthetic |
| pMB037_promoter | 193 | Synthetic |
| pMB009_promoter | 194 | Synthetic |
| pMB041_promoter | 195 | Synthetic |
| pMB036_promoter | 196 | Synthetic |
| pMB049_promoter | 197 | Synthetic |
| pMB044_promoter | 198 | Synthetic |
| pMB042_promoter | 199 | Synthetic |
| pMB086_promoter | 200 | Synthetic |
| pMB053_promoter | 201 | Synthetic |
| pMB057_promoter | 202 | Synthetic |
| pMB018_promoter | 203 | Synthetic |
| pMB002_promoter | 204 | Synthetic |
| pMB015_promoter | 205 | Synthetic |
| pMB087_promoter | 206 | Synthetic |
| pMB063_promoter | 207 | Synthetic |
| *Native promoters from Escherichia coli | | |

**Table 1.5.** Sequence Parts used in combinatorial synthetic promoter-5'UTR library

| Part name | Part sequences | Origin |
|---|---|---|
| | | |
| distal | ACCGTGCGTG (SEQ ID NO. 208) | phage λ, $P_R$ promoter |
| | | |
| -35 | TTTACA | variation |
| | TTGACT | phage λ, $P_R$ promoter |
| | TTGACA | phage λ, $P_L$ promoter |
| | TAGGCT | variation |
| | TAGACT | variation |
| | | |
| Core | ATTTTACCTCTGGCGGT (SEQ ID NO. 209) | phage λ, $P_R$ promoter |
| | TAAATACCACTGGCGGT (SEQ ID NO. 210) | phage λ, $P_L$ promoter |
| | | |
| -10 | GATAAT | phage λ, $P_R$ promoter |
| | GATACT | phage λ, $P_L$ promoter |
| | TAGAGT | variation |
| | TATAAT | variation |
| | TATTTT | variation |
| | | |
| 5'-UTR/RBS | GGTTGCATGTACTAAGGAGGTTGT (SEQ ID NO. 211) | phage λ, $P_R$ promoter |
| | TAACATCCTACAAGGAGAACAAAAGC (SEQ ID NO. 212) | *E. coli,* promoter *of acs* gene |

In some examples, the promoters of the present disclosure exhibit at least 100%, 99%, 98%, 97%, 96%, 95%, 94%, 93%, 92%, 91%, 90%, 89%, 88%, 87%, 86%, 85%, 84%, 83%, 82%, 81 %, 80%, 79%, 78%, 77%, 76%, or 75% sequence identity with a promoter from Table 1 and/or Table 1.4.

***Bicistronic Regulatory Element Design***

On of the barriers to efficient and scalable HTP genetic design is the lack of standard parts that can be reused reliably in novel combinations. Many examples within *E. coli,* highlight how seemingly simple genetic functions behave differently in different settings. For example, in some examples, a prokaryotic ribosome-binding site (RBS) element that initiates translation for one coding sequence might not function at all with another coding sequence *(see* Salis, H.M., et al. "Automated design of synthetic ribosome binding sites to control protein expression" Nat. Biotechnol. Vol 27, 946-950 (2009)). If the genetic elements that encode control of central cellular processes such as transcription and translation cannot be reliably reused, then there is little chance that higher-order objects encoded from such basic elements will be reliable in larger-scale systems. In some examples, the methods of the present disclosure overcome these aforementioned challenges via the use of bicistronic design regulatory sequences.

Bicistronic designs of the present disclosure can, in some examples, greatly reduce the context dependent variability in expression strength of a given promoter for a variety of coding genes (Mutalik, et al. "Precise and reliable gene expression via standard transcription and translation initiation elements" Nat. Biotechnol. Vol 10 (4) pp 354-368 (2013)). In some examples, the present disclosure teaches that a bicistronic design (BCD) is a nucleotide sequence in which a promoter drives the expression of two coding sequences, where the first coding sequence (Cistron 1) terminates and the second coding sequences initiates at the same nucleotide base (Cistron 2/target gene). This strategy provides a means to avoid variability in expression strength of the second coding sequences due to unpredictable interactions between the promoter and the second coding sequence.

In some example, the promoters of the present disclosure are compound regulatory sequences following the bicistronic design. That is, in some examples, the promoters in the promoter ladders of the present disclosure are larger regulatory sequences comprising **i)** a promoter operably linked to **ii)** a first ribosome binding site (SD1), which is operably linked to **iii)** a first cistronic sequence (Cis1), wherein the Cis1 overlaps with **iv)** a second ribosome binding site (SD2), that is then operably linked to **v)** a target gene coding sequence (Cis2) *(See* Figure 43). In some examples, the present disclosure refers to the combination of elements i)-iv) as a "bicistronic design" or "bicistronic design regulatory sequence" (BCDs).

In some examples, the BCDs of the present disclosure can be operably linked to any target gene. Thus in some embodients, the BCDs of the present disclosure can be used in place of traditional promoters. In some examples, the present disclosure teaches that using BCDs in the PRO swap toolbox increases the consistency with which expressed transcripts are translated. Without wishing to be bound by any one theory, the present inventors believe that the presence of an SD1 and Cis1 leader sequences operably linked to the target gene recruit active ribosomal complexes, which are then able to consistenly reinitiate translation of the target gene via the SD2 ribosomal binding site.

A collection of promoters and bicistronic design elements have been reported that can be used for HTP genome engineering *(see* Mutalik, et al. "Precise and reliable gene expression via standard transcription and translation initiation elements" Nat. Biotechnol. Vol 10 (4) pp 354-368 (2013)). However, these reported sequences all contain identical DNA sequences in the first 35 nt of the 48nt of the regulatory bicistronic design sequence *(see* Mutalik state-of-the-art sequence in Figure 43).

In some examples, the present disclosure teaches that the BCD of Mutalik et al., could not be used to effectively engineer multiple target genes in a single organism. That is, in some examples, the present disclosure teaches against the multiple integration of the Mutalik BCD into the genome of a host cell. Without wishing to be bound by any one theory, the present inventors believe that repeated use of the Mutalik et al., BCD would result in increasing rates of undesired homologous recombination (HR) triggered by the presence of highly homologous sequences throughout the genome.

In some examples, the present disclosure solves this problem by describing novel BCDs with non-identical nucleotide sequences. These novel BCDs can be used for HTP genome engineering in *E. coli* to provide predictable changes in expression of multiple genes within a single genome, independent of the coding sequences of these genes, without inducing undesirable homologous recombination.

In some examples, the present disclosure teaches methods of expressing two target gene proteins in a host organism at relatively similar levels. Thus in some examples, the present disclosure teaches expression of two or more target gene proteins within 0.2, 0.4, 0.6, 0.8, 1, 1.2, 1.4, 1.6, 1.8, 2, 2.2, 2.4, 2.6, 2.8, or 3-fold of each other.

In some examples, the present disclosure teaches methods of expressing two target gene proteins in a host organism at similar levels, while reducing the risk of undesirable homologous recombination (HR) events triggered by the use of identical regulatory sequences. Thus in some examples, the present disclosure teaches methods of varying BCD sequence in a way that maintains expression levels, while reducing the risk of HR. That is, in some examples, the present disclosure teaches expression of two or more genes encoding for proteins via BCDs that are identical save for their Cis1 sequences.

### *BCD Promoters*

In some examples, the BCDs of the present disclosure comprise a promoter sequence. In some examples the promoters comprised in BCDs can be any promoter capable of expressing in the host cell. Thus, in some examples, the promoters can be any promoter disclosed in the specification. In some examples, the promoter can be any promoter known to function in *E. coli.* In other examples, the promoters can be any promoter disclosed in Table 1 and/or Table 1.4.

### *First and Second Ribosomal Binding Site (SD1 and SD2)*

In some examples, the BCDs of the present disclosure comprise a first and second ribosomal binding site, referred to as SD1 and SD2, respectively. In some examples, the sequences of SD1 and SD2 can be the same. In other examples the sequences of SD1 and SD2 can be different.

In some examples, the SD sequences can be any known ribosomal binding site functional in the host undering the HTP genomic engineering. In other examples, the present disclosure teaches an SD sequence of NNNGGANNN, wherein N refers to any nucleotide. In other examples, the present disclosure teaches SD sequences selected from the sequences disclosed in Table 1.1.

**Table 1.1-** Non-limiting list of Ribosomal Binding Sites, amenable for SD1 and SD2 use.

| SD # | Sequence | SD # | Sequence | SD # | Sequence |
|---|---|---|---|---|---|
| 1 | TGCGGAGGG | 50 | AGTGGAACC | 99 | GGAGGAGAC |

(continued)

| SD # | Sequence | SD # | Sequence | SD # | Sequence |
|---|---|---|---|---|---|
| 2 | CACGGAGGC | 51 | GCCGGAGTG | 100 | TAGGGAACG |
| 3 | TGGGGAGGG | 52 | GCGGGACAG | 101 | TGAGGACGT |
| 4 | AGGGGAGGC | 53 | CCGGGATAA | 102 | ATCGGAGGT |
| 5 | GGGGGAGGG | 54 | CCAGGAACG | 103 | CCGGGAGGG |
| 6 | TATGGAGGT | 55 | GACGGAGCA | 104 | ACGGGACGG |
| 7 | GACGGAGCG | 56 | AGCGGATTG | 105 | GTGGGAGAG |
| 8 | AGGGGAGTC | 57 | ATGGGATAT | 106 | CGAGGATAG |
| 9 | ACAGGAGGC | 58 | CTGGGAAGT | 107 | TGGGGAGCC |
| 10 | GGGGGAGCG | 59 | TGGGGAGCC | 108 | CGTGGAGTG |
| 11 | ACAGGAGTC | 60 | GTGGGACAT | 109 | TGGGGACTG |
| 12 | ACAGGAGAC | 61 | GCCGGAGCG | 110 | GGTGGAAGC |
| 13 | TGGGGAGGC | 62 | AATGGAGGC | 111 | GATGGACGG |
| 14 | TGGGGAGAT | 63 | GGAGGATTG | 112 | AATGGAGGT |
| 15 | GGGGGAGAA | 64 | GATGGACTC | 113 | GGAGGATCC |
| 16 | AGGGGAGGC | 65 | GAGGGATGG | 114 | ACAGGATCT |
| 17 | AGAGGAGTC | 66 | GAAGGACAG | 115 | CCCGGACAG |
| 18 | AGGGGATAT | 67 | TAGGGAAGG | 116 | ACGGGAAAC |
| 19 | CGTGGAGTG | 68 | TGGGGAACC | 117 | AGTGGACCG |
| 20 | GGGGGAAGG | 69 | TTAGGAGTC | 118 | CATGGATCA |
| 21 | AGGGGAATC | 70 | GGAGGAGGA | 119 | CTGGGATGT |
| 22 | CCCGGAGGT | 71 | CCGGGATCT | 120 | TTAGGATGG |
| 23 | AGGGGAGGG | 72 | GGGGGATGA | 121 | GTAGGATTC |
| 24 | TTTGGAGTC | 73 | CTGGGAGTG | 122 | TTTGGAGTT |
| 25 | AGGGGACAC | 74 | CAAGGAACC | 123 | CGCGGACTC |
| 26 | CGGGGAGAC | 75 | GCTGGAGGC | 124 | CTCGGACAG |
| 27 | GGGGGAGGG | 76 | ATGGGACCT | 125 | CAAGGAGTC |
| 28 | AAGGAGATC | 77 | GGAGGAGGG | 126 | CTTGGACAG |
| 29 | TAAGGAGGT | 78 | CTGGGATGC | 127 | GGCGGATCG |
| 30 | GGGGGAGTC | 79 | ACAGGATAC | 128 | GGGGGACAG |
| 31 | GGAGGATCG | 80 | GAGGGAAGG | 129 | ATTGGATGG |
| 32 | CTGGGATCG | 81 | AGTGGATCT | 130 | CCTGGATAT |
| 33 | ATCGGACCG | 82 | CCGGGAGTT | 131 | CTCGGATAC |
| 34 | GGGGGAGTG | 83 | GGTGGAGGC | 132 | GGGGGAGCC |
| 35 | TAGGGAGCA | 84 | CCAGGAAGA | 133 | TGGGGATTG |
| 36 | GGTGGAGGG | 85 | GGGGGATTT | 134 | CCAGGATCA |
| 37 | GCCGGAGGT | 86 | CGCGGAGTA | 135 | CGGGGACGG |
| 38 | TGAGGAAAG | 87 | CCAGGAATC | 136 | TCAGGACAA |
| 39 | CTTGGAGGG | 88 | ATTGGAGTG | 137 | GTAGGATGG |

(continued)

| SD # | Sequence | SD # | Sequence | SD # | Sequence |
|---|---|---|---|---|---|
| 40 | TGAGGAGAT | 89 | CTAGGAAGT | 138 | TCCGGACTA |
| 41 | CGTGGAGGG | 90 | GAAGGATAG | 139 | GTCGGATCA |
| 42 | GCGGGAGGG | 91 | AAAGGACAC | 140 | TGCGGAGTT |
| 43 | GGGGGATAG | 92 | CCGGGACGT | 141 | GGTGGACGG |
| 44 | GGGGGAGCG | 93 | ATGGGAGTG | 142 | CTTGGACGA |
| 45 | CCGGGAGCA | 94 | AGCGGACAG | 143 | CTGGGACTT |
| 46 | GCGGGAGTA | 95 | GGCGGATCT | 144 | TATGGAGTA |
| 47 | GTAGGACCG | 96 | ATAGGAGGG | 145 | ACAGGAGGC |
| 48 | CCGGGAAGG | 97 | TATGGAGGG | | |
| 49 | GACGGAGTC | 98 | GGTGGACTC | | |

It should be clear that the epistatic mapping procedure described herein does not depend on which model is used by the analysis equipment 214. Given such a predictive model, it is possible to score and rank all hypothetical strains accessible to the mutation library *via* combinatorial consolidation.

In some examples, the present disclosure teaches that varying individual SD sequences in the BCD will affect the overall expression of the BCD. Some SD sequences may serve to increase or decrease overall expression potential of the BCD. It is expected however, that each BCD will exhibit consistent expression results when combined over different target gene Cis2 sequences.

In some examples, the SD2 sequence is entirely embedded within the coding sequence of the first cistronic sequence. That is, in some examples, the SD2 sequence is integrated into the coding sequences of Cis2. Without wishing to be bound by any one theory, the present inventors believe that BCD arrangements in which the ribosomal binding site of the target gene (SD2) is entirely embedded in the coding sequence of the upstream gene (Cis1) results in the coupling of the translations of the Cis1 and Cis2 peptides. More specifically, the present inventors hypothesize that the intrinsic helicase activity of ribosomes arriving at the stop codon of an upstream Cis1 sequence eliminate inhibitory RNA structures that would otherwise disrupt translation initiation of the downstream Cis2 target gene.

***First Cistronic Sequence (Cis1)***

In some examples, the first cistronic sequence Cis1 of the present disclosure can be any sequence encoding a continuous peptide. For example, in some examples, the Cis1 sequence encodes for a peptide that is 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 350, 400, 450, 500, 550, 600, 650, 700, 750, 800, 850, 900, 950, 1000, or more amino acids long, including any ranges and subranges therein. In some examples, Cis1 does not need to encode a functional peptide.

In some examples, the Cis1 sequence encodes a 16-amino-acid leader peptide. In some examples the Cis1 nucleotide sequence is:
5' -ATGAAAGCAATTTTCGTACTGAAACATCTTAATCATGCACAGGAGACTTTCTAA-3' (SEQ ID No. 17).

In other examples, the present disclosure teaches that the Cis1 sequence can be 5'-ATGNNNNNNNNNNNNNNNNNNNNNNNNNNNNNNNNNNNNNNNNNNNNNNNNNN - 3' , where N can be any nucleic acid, so long as Cis1 encodes for a peptide

In some examples, the present disclosure teaches that the stop codon of Cis1 and the start codon of Cis2 must be in close proximity or overlapping. For example, in some examples, the stop codon of Cis1 must be within 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides of the start codon of Cis2, including all ranges and subranges therein.

In some examples, the Cis1 sequence overlaps with the Cis2 sequence by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100 or more nucleotides, including all ranges and subranges therein. In other examples, the BCD of the present disclosure is designed such that the Cis1 sequence overlaps by 1 nucleotide with the Cis2 target gene coding sequence, such that the last few nucleotides encode for both a stop and start codon via a -1 frame shift (*see* Figure 43). In some examples, the Cis1 and Cis 2 sequences must be coded on different open reading frames so as to prevent the formation of a chimeric protein combining the sequences of Cis1 and Cis2.

In some examples, the present disclosure teaches that the start codon of the Cis1 sequence can be any functional start codon. In some examples, the present disclosure teaches that prokaryotes use ATG (AUG) at the most common start codon, followed by GTG (GUG) and TTG (UUG).

In some examples, the present disclosure teaches that the Cis1 sequence does not have any premature stop codons. In other examples, the present disclosure teaches that rare codons in the Cis1 sequence can reduce the translation efficiency of Cis2. Thus, in some examples, Cis1 will encode for a peptide without any rare codons to achieve maximum expression. In other examples, Cis1 will encode for a peptide with one or more rare codons in order to modulate the expression of Cis2.

In other examples, the present disclosure teaches that multiple codons repeats in the Cis1 sequence can reduce the translation efficiency of Cis2. Thus, in some examples, Cis1 will encode for a peptide without any codon repeats to achieve maximum expression. In other examples, Cis1 will encode for a peptide with one or more codon repeats in order to modulate the expression of Cis2.

### *Second Cistronic Sequence (Cis2- Target Gene)*

In some examples, the present disclosure teaches that the BCDs of the present disclosure are operably linked to a Cis2 target gene sequence, in much the same way in which the promoters of the PRO-swap libraries are operably linked to target sequences. That is, in some examples, the BCDs of the present disclosure can take the place of traditional promoters in the PRO-swap libraries and methods of the present disclosure. The Cis2 sequences, can in some examples, be any sequence of interest.

The present disclosure teaches that, in some examples, target genes encoding for a polypeptide will be more effectively regulated by BCDs than by a promoter. That is, in some examples, BCDs will not modulate the expression of non-coding RNA more than would be possible by a promoter.

### *Terminator Ladders*

In some examples, the present disclosure teaches methods of improving genetically engineered host strains by providing one or more transcriptional termination sequences at a position 3' to the end of the RNA encoding element. In some examples, the present disclosure teaches that the addition of termination sequences improves the efficiency of RNA transcription of a selected gene in the genetically engineered host. In other examples, the present disclosure teaches that the addition of termination sequences reduces the efficiency of RNA transcription of a selected gene in the genetically engineered host. Thus in some examples, the terminator ladders of the present disclosure comprises a series of terminator sequences exhibiting a range of transcription efficiencies (e.g., one weak terminator, one average terminator, and one strong promoter).

A transcriptional termination sequence may be any nucleotide sequence, which when placed transcriptionally downstream of a nucleotide sequence encoding an open reading frame, causes the end of transcription of the open reading frame. Such sequences are known in the art and may be of prokaryotic, eukaryotic or phage origin. Examples of terminator sequences include, but are not limited to, PTH-terminator, pET-T7 terminator, T3-T$\varphi$ terminator, pBR322-P4 terminator, vesicular stomatitus virus terminator, rrnB-T1 terminator, rrnC terminator, TTadc transcriptional terminator, and yeast-recognized termination sequences, such as Mat$\alpha$ ($\alpha$-factor) transcription terminator, native $\alpha$-factor transcription termination sequence, ADR1 transcription termination sequence, ADH2transcription termination sequence, and GAPD transcription termination sequence. A non-exhaustive listing of transcriptional terminator sequences may be found in the iGEM registry, which is available at: http://partsregistry.org/Terminators/Catalog.

In some examples, transcriptional termination sequences may be polymerase-specific or nonspecific, however, transcriptional terminators selected for use in the present examples should form a 'functional combination' with the selected promoter, meaning that the terminator sequence should be capable of terminating transcription by the type of RNA polymerase initiating at the promoter. For example, in some examples, the present disclosure teaches a eukaryotic RNA pol II promoter and eukaryotic RNA pol II terminators, a T7 promoter and T7 terminators, a T3 promoter and T3 terminators, a yeast-recognized promoter and yeast-recognized termination sequences, etc., would generally form a functional combination. The identity of the transcriptional termination sequences used may also be selected based on the efficiency with which transcription is terminated from a given promoter. For example, a heterologous transcriptional terminator sequence may be provided transcriptionally downstream of the RNA encoding element to achieve a termination efficiency of at least 60%, at least 70%, at least 75%, at least 80%, at least 85%, at least 90%, at least 91%, at least 92%, at least 93%, at least 94%, at least 95%, at least 96%, at least 97%, at least 98%, or at least 99% from a given promoter.

In some examples, efficiency of RNA transcription from the engineered expression construct can be improved by providing nucleic acid sequence forms a secondary structure comprising two or more hairpins at a position 3' to the end of the RNA encoding element. Not wishing to be bound by a particular theory, the secondary structure destabilizes the transcription elongation complex and leads to the polymerase becoming dissociated from the DNA template, thereby minimizing unproductive transcription of non-functional sequence and increasing transcription of the desired RNA. Accordingly, a termination sequence may be provided that forms a secondary structure comprising two or more adjacent hairpins. Generally, a hairpin can be formed by a palindromic nucleotide sequence that can fold back on itself to form a paired stem region whose arms are connected by a single stranded loop. In some examples, the termination sequence comprises 2, 3, 4, 5, 6, 7, 8, 9, 10 or more adjacent hairpins. In some examples, the adjacent hairpins are separated by 0, 1, 2, 3, 4, 5, 6, 7, 8, 9, 10, 11, 12, 13, 14, or 15 unpaired nucleotides. In some examples, a hairpin stem comprises 4, 5, 6, 7, 8, 9, 10, 11, 12, 13, 14, 15, 16, 17, 18, 19, 20, 21, 22, 23, 24, 25, 26, 27, 28, 29, 30 or more base pairs in length. In certain examples, a hairpin stem is 12 to 30 base pairs in length. In certain examples, the termination sequence comprises two or more medium-sized hairpins having stem region comprising about 9 to 25 base pairs. In some examples, the hairpin comprises a loop-forming region of 1, 2, 3, 4, 5, 6, 7, 8, 9, or 10 nucleotides. In some examples, the loop-forming region comprises 4-8 nucleotides. Not wishing to be bound by a particular theory, stability of the secondary structure can be correlated with termination efficiency. Hairpin stability is determined by its length, the number of mismatches or bulges it contains and the base composition of the paired region. Pairings between guanine and cytosine have three hydrogen bonds and are more stable compared to adenine-thymine pairings, which have only two. The G/C content of a hairpin-forming palindromic nucleotide sequence can be at least 60%, at least 65%, at least 70%, at least 75%, at least 80%, at least 85%, at least 90% or more. In some examples, the G/C content of a hairpin-forming palindromic nucleotide sequence is at least 80%. In some examples, the termination sequence is derived from one or more transcriptional terminator sequences of prokaryotic, eukaryotic or phage origin. In some examples, a nucleotide sequence encoding a series of 4, 5, 6, 7, 8, 9, 10 or more adenines (A) are provided 3' to the termination sequence.

In some examples, the present disclosure teaches the use of a series of tandem termination sequences. In some examples, the first transcriptional terminator sequence of a series of 2, 3, 4, 5, 6, 7, or more may be placed directly 3' to the final nucleotide of the dsRNA encoding element or at a distance of at least 1-5, 5-10, 10-15, 15-20, 20-25, 25-30, 30-35, 35-40, 40-45, 45-50, 50-100, 100-150, 150-200, 200-300, 300-400, 400-500, 500-1,000 or more nucleotides 3' to the final nucleotide of the dsRNA encoding element. The number of nucleotides between tandem transcriptional terminator sequences may be varied, for example, transcriptional terminator sequences may be separated by 0, 1, 2, 3, 4, 5, 6, 7, 8, 9, 10, 10-15, 15-20, 20-25, 25-30, 30-35, 35-40, 40-45, 45-50 or more nucleotides. In some examples, the transcriptional terminator sequences may be selected based on their predicted secondary structure as determined by a structure prediction algorithm. Structural prediction programs are well known in the art and include, for example, CLC Main Workbench.

Persons having skill in the art will recognize that the methods of the present disclosure are compatible with any termination sequence. In some examples, the present disclosure teaches use of annotated *Corynebacterium glutamicum* terminators as disclosed in from Pfeifer-Sancar et al. 2013. "Comprehensive analysis of the Corynebacterium glutamicum transcriptome using an improved RNAseq technique" Pfeifer-Sancar et al. BMC Genomics 2013, 14:888). In other examples, the present disclosure teaches use of transcriptional terminator sequences found in the iGEM registry, which is available at: http://partsregistry.org/Terminators/Catalog. A non-exhaustive listing of transcriptional terminator sequences of the present disclosure is provided in Table 1.2 below.

**Table 1.2.** Non-exhaustive list of termination sequences of the present disclosure.

| *E. coli* | | | |
|---|---|---|---|
| **Name** | **Description** | **Direction** | **Length** |
| BBa_B0010 | T1 from *E. coli* rrnB | Forward | 80 |
| BBa_B0012 | TE from coliphageT7 | Forward | 41 |
| BBa_B0013 | TE from coliphage T7 (+/-) | Forward | 47 |
| BBa_B0015 | double terminator (B0010-B0012) | Forward | 129 |
| BBa_B0017 | double terminator (B0010-B0010) | Forward | 168 |
| BBa_B0053 | Terminator (His) | Forward | 72 |
| BBa_B0055 | -- No description -- | | 78 |
| BBa_B1002 | Terminator (artificial, small, %T~=85%) | Forward | 34 |
| BBa_B1003 | Terminator (artificial, small, %T~=80) | Forward | 34 |

(continued)

| *E. coli* | | | |
|---|---|---|---|
| **Name** | **Description** | **Direction** | **Length** |
| BBa_B1004 | Terminator (artificial, small, %T~=55) | Forward | 34 |
| BBa_B1005 | Terminator (artificial, small, %T~=25% | Forward | 34 |
| BBa_B1006 | Terminator (artificial, large, %T~>90) | Forward | 39 |
| BBa_B1010 | Terninator (artificial, large, %T~<10) | Forward | 40 |
| BBa_I11013 | Modification of biobricks part BBa_B0015 | | 129 |
| BBa_I51003 | -- No description -- | | 110 |
| BBa_J61048 | [rnpB-T1] Terminator | Forward | 113 |
| BBa_K1392970 | Terminator+Tetr Promoter+T4 Endolysin | | 623 |
| BBa_K1486001 | Arabinose promoter + CpxR | Forward | 1924 |
| BBa_K1486005 | Arabinose promoter + sfGFP-CpxR [Cterm] | Forward | 2668 |
| BBa_K1486009 | CxpR & Split IFP1.4 [Nterm + Nterm] | Forward | 3726 |
| BBa_K780000 | Terminator for Bacillus subtilis | | 54 |
| BBa_K864501 | T22, P22 late terminator | Forward | 42 |
| BBa_K864600 | T0 (21 imm) transcriptional terminator | Forward | 52 |
| BBa_K864601 | Lambda t1 transcriptional terminator | Forward | |
| BBa_B0011 | LuxICDABEG (+/-) | Bidirectional | 46 |
| BBa_B0014 | double terminator (B0012-B0011) | Bidirectional | 95 |
| BBa_B0021 | LuxICDABEG (+/-), reversed | Bidirectional | 46 |
| BBa_B0024 | double terminator (B0012-B0011), reversed | Bidirectional | 95 |
| BBa_B0050 | Terminator (pBR322, +/-) | Bidirectional | 33 |
| BBa_B0051 | Terminator (yciA/tonA, +/-) | Bidirectional | 35 |
| BBa_B1001 | Terminator (artifical, small, %T~=90) | Bidirectional | 34 |
| BBa_B1007 | Terminator (artificial, large, %T~=80) | Bidirectional | 40 |
| BBa_B1008 | Terminator (artificial, large, %T~=70) | Bidirectional | 40 |
| BBa_B1009 | Terminator (artificial, large, %T~=40%) | Bidirectional | 40 |
| BBa_K187025 | terminator in pAB, BioBytes plasmid | | 60 |
| BBa_K259006 | GFP- Terminator | Bidirectional | 823 |
| BBa_B0020 | Terminator (Reverse B0010) | Reverse | 82 |
| BBa_B0022 | TE from coliphageT7, reversed | Reverse | 41 |
| BBa_B0023 | TE from coliphage T7, reversed | Reverse | 47 |
| BBa_B0025 | double terminator (B0015), reversed | Reverse | 129 |
| BBa_B0052 | Terminator (rrnC) | Forward | 41 |
| BBa_B0060 | Terminator (Reverse B0050) | Bidirectional | 33 |
| BBa_B0061 | Terminator (Reverse B0051) | Bidirectional | 35 |
| BBa_B0063 | Terminator (Reverse B0053) | Reverse | 72 |
| Spy | Terminator (SEQ ID NO. 225) | | 90 |
| pheA | Terminator (SEQ ID NO. 226) | | 51 |

(continued)

| *E. coli* | | | | | |
|---|---|---|---|---|---|
| **Name** | **Description** | | | **Direction** | **Length** |
| osmE | Terminator (SEQ ID NO. 227) | | | | 42 |
| rpoH | Terminator (SEQ ID NO. 228) | | | | 41 |
| vibE | Terminator (SEQ ID NO. 229) | | | | 71 |
| Thr1_ABC | Terminator (SEQ ID NO. 230) | | | | 57 |
| ***Corynebacterium*** | | | | | |
| **Terminator** | **Termina tor Start** | **Terminator End** | **strand** | **Transcript End** | **DNA Sequence** |
| cg0001 T1 | 1628 | 1647 | + | loop | SEQ ID NO. 9 |
| cg0007 T2 | 7504 | 7529 | + | stem 1 | SEQ ID NO. 10 |
| cg0371 T3 | 322229 | 322252 | + | stem 1 | SEQ ID NO. 11 |
| cg0480 T4 | 421697 | 421720 | - | stem 1 | SEQ ID NO. 12 |
| cg0494 T5 | 436587 | 436608 | + | loop | SEQ ID NO. 13 |
| cg0564 T6 | 499895 | 499917 | + | stem 1 | SEQ ID NO. 14 |
| cg0610 T7 | 541016 | 541039 | + | stem 2 | SEQ ID NO. 15 |
| cg0695 T8 | 613847 | 613868 | - | loop | SEQ ID NO. 16 |

***Protein Solubility Tag Ladders***

In some examples, the present disclosure teaches methods of improving genetically engineered host strains by providing one or more protein solubility tag sequences operably linked with a target protein derived from a target gene. The solubility tags can be fusion partners operably linked with the target protein on either the N-terminus or the C-terminus of the target protein. In some examples, the present disclosure teaches that the addition of solubility tag sequences improves the solubility of a protein translated from a selected gene in the genetically engineered host. In other examples, the solubility tags may also be used to assist in the purification of the target protein.

Effective tags for use in protein solubility tag ladders of the present disclosure can be any solubility tags known in the art that form independent, well-folded domains, highly soluble domains. These domains may contribute to the solubility of their target protein through an additive effect, or when used as an N-terminal tag may quickly fold after emerging from the ribosome and sterically block the emerging amino acid chain of the target protein from interacting with other cellular components which may cause misfolding. Further, solubility tags for inclusion in solubility tag ladders may have properties in common such as being small, tightly-folded domains or being leader sequences of proteins known to be highly soluble. The protein solubility tag sequences can be any such tags known in the art such as, for example, the any of the tags found in Costa et al., Front Microbiol. 2014; 5: 63. In one example, solubility tag sequences include the tags found in Table 17.

In one example, the protein solubility tag is a fusion partner. The fusion partner coding gene can be present in any of the vectors (e.g., shuttle vectors) provided herein such that integration of the gene for a target protein in the vector operably links the fusion partner coding gene to the target gene. *E. coli* expression vectors comprising solubility tags for use herein can comprise a protease recognition sequence between the solubility tag fusion partner coding gene and the target protein coding gene that can allows for the tag removal as necessary. The choice of a fusion partner for use in the solubility swap methods provided herein can depend on:

(i) Purpose of the fusion: is it for solubility improvement or for affinity purification? A variety of fusion tags that render different purposes are available, and systems containing both solubility and affinity tags like, for instance, the dual hexahistine (His6)-MBP tag, can be designed in order to get a rapid "in one step" protein production. Some protein tags can also function in both affinity and solubility roles, as for instance, the MBP or glutathione-S-transferase (GST; Esposito and Chatterjee, Curr Opin Biotechnol. 2006 Aug;17(4):353-8. Epub 2006 Jun 15).

(ii) Amino acid composition and size: target proteins may require larger or smaller tags depending on their application. Larger tags can present a major diversity in the amino acid content, and may impose a metabolic burden in the host cell different from that imposed by small tags.

(iii) Required production levels: structural studies may require higher protein production levels that can be rapidly achieved with a larger fusion tag, which has strong translational initiation signals, whereas the study of physiological interactions may demand for lower production levels and small tags.

(iv) Tag location: fusion partners can promote different effects when located at the N-terminus or C-terminus of the target protein. N-terminal tags may often be advantageous over C-terminal tags because: (1) they provide a reliable context for efficient translation initiation, in which fusion proteins take advantage of efficient translation initiation sites on the tag; (2) they can be removed leaving none or few additional residues at the native N-terminal sequence of the target protein, since most of endoproteases cleave at or near the C-terminus of their recognition sites.

**Table 17.** Non-exhaustive list of protein solubility tag sequences of the present disclosure.

| Solubility Tag Name (Protein Solubility Tag #) | Description | Organism | Nucleic Acid SEQ ID NO. | Amino Acid SEQ ID NO. | Size (aa) |
|---|---|---|---|---|---|
| GB1 (PST1) | IgG domain B1 of Protein G | Streptococcus sp. | 231 | 235 | 56 |
| FH8 (PST2) | Fasciola hepatica 8-kDa antigen | F. hepatica | 232 | 236 | 69 |
| Ubiquitin (PST3) | | | 233 | 237 | |
| SUMO (PST4) | Small ubiquitin modified | Homo sapiens | 234 | 238 | ~100 |

***Protein Degradation Tag Ladders***

In some examples, the present disclosure teaches methods of improving genetically engineered host strains by providing one or more protein degradation tag sequences operably linked with a target protein derived from a target gene. The addition of a degradation tag sequence using the methods provided herien can mark the target protein for degradation. Marking the target protein for degradation may reduce or modulate the target protein abundance within a cell. By reducing or modulating the target protein levels or abundance in the cell, the addition of degradation tag sequences to a target protein may ulitmatley impact the overall phenotype of resultant strains.

Effective tags for use in protein degradation tag ladders of the present disclosure can be any degradation tags known in the art that are part of a known degradation pathway in the host organism (e.g., *E. coli*). For example, known degradation pathways in *E. coli* can include the clpXP/clpAP system, the Hf1B system, the ftsH System and the Ion system. Accordingly, degradation tags for use in the degradation tag swap methods provided herein can include any tags known to function in any of these *E. coli* protein degradation systems. In some cases, the degradation tags can be mutated in such a manner as to confer the ability of the resultant mutant tag to have its activity tuned. For example, the ssrA class of tags can be mutated such that the mutated ssrA degradation tags mark a tagged protein for degradation via the ClpXP degradation pathway with different degrees of efficiency. In one example,the ssrA tags can contain a single amino acid mutations in the last three residues of the AANDENYALAA consensus sequence such that target proteins comprising a C-terminal mutated ssrA tag can be degraded at varying levels of efficiency by certai intracellular tail specific proteases (e.g., Tsp protease) depending on which amino acid has been mutated in the last three residues of the ssrA tag consensus sequence (see Keiler K C, Sauer R T. Sequence determinants of C-terminal substrate recognition by the Tsp protease. J Biol Chem. 1996;271:2589-2593. Accordingly, using the degradation tag swap methods of the present disclosure, it is possible to obtain strains of host cells possessing target proteins of varying stability by constructing variants carrying C-terminal peptide tags with minor alterations in the Tsp consensus sequence. Examples of mutant ssrA tags for use in the methods herein can comprise amino acid SEQ ID NO: 248, 249 or 250.

Another example of mutated ssrA tags for use in the methods provided herein can be the DAS tags found in McGinness et al., "Engineering Controllable Protein Degradation" Mol. Cell, Vol 22(5), June 2006. In the DAS tags, two residues in the ssrA tag were replaced resulting in mutated ssrA tags that exhibited weakened ClpX binding without diminishing SspB recognition. As such, target proteins bearing the DAS tags can be degraded efficiently by ClpXP only when SspB is present, allowing intracellular degradation to be regulated by controlling SspB levels.

A non-exhaustive list of protein degradation tag sequence of the present disclosure can be found in Table 18.

**Table 18.** Non-exhaustive list of protein degradation tag sequences of the present disclosure.

| Degradation Tag Name (Protein Degradation Tag #) | Description | Organism | Nucleic Acid SEQ ID NO. | Amino Acid SEQ ID NO. | Source |
|---|---|---|---|---|---|
| ssrA_LAA (PDT1) | native | *E. coli* | 239 | 247 | Andersen et al., Appl Environ Microbiol. 1998 Jun; 64(6): 2240-2246. |
| ssrA_LVA (PDT2) | mutant | *E. coli* | 240 | 248 | Andersen et al., Appl Environ Microbiol. 1998 Jun; 64(6): 2240-2246. |
| ssrA_AAV (PDT3) | mutant | *E. coli* | 241 | 249 | Andersen et al., Appl Environ Microbiol. 1998 Jun; 64(6): 2240-2246. |
| ssrA_ASV (PDT4) | mutant | *E. coli* | 242 | 250 | Andersen et al., Appl Environ Microbiol. 1998 Jun; 64(6): 2240-2246. |
| ftsH-cII89-97 (PDT5) | native | *E. coli* | 243 | 251 | Kobiler O , Koby S, Teff D, Court D, Oppenheim AB. PNAS. 23 Oct 2002, 99(23): 14964-14969. |
| cI108 (PDT6) | native | *E. coli* | 244 | 252 | Herman et al., Genes Dev. 1998 May 1; 12(9): 1348-1355. |
| su120 (PDT7) | native | *E. coli* | 245 | 253 | Wohlever et al., Protein Eng Des Sel. 2013 Apr; 26(4): 299-305. |
| β20 (PDT8) | native | *E. coli* | 246 | 254 | Wohlever et al., Protein Eng Des Sel. 2013 Apr; 26(4): 299-305. |

The degradation tags can be fusion partners operably linked with the target protein on either the N-terminus or the C-terminus of the target protein. Accordingly, the fusion partner coding gene can be present in any of the vectors (e.g., shuttle vectors) provided herein such that integration of the gene for a target protein in the vector operably links the fusion partner coding gene to the target gene such that translation of the construct generates a fusion protein with the degradation tag present on either the N-terminus or C-terminus of the target protein as desired. In one example, placement of the degradation tags (or mutants thereof) at the N-terminus or C-terminus of a target protein can depend on the tag used. For example, degradation tags (or mutants thereof) associated with the clpXP/clpAP system, the Hf1B system, the ftsH System or the sul20 tag of the Ion system can be operably linked to a target protein at the C-terminus, while β20 degradation tags (or mutants thereof) of the the Ion system can be operably linked to a target protein at the N-terminus or internally. In one example, the degradation tag is the N-degron tag (Ntag) for *E. coli* as found in Sekar K, Gentile AM, Bostick JW, Tyo KEJ (2016) N-Terminal-Based Targeted, Inducible Protein Degradation in Escherichia coli. PLoS ONE 11(2): e0149746. The Ntag can be placed on the N-terminus of a target protein of interest and can serve to mark the target protein of interest for degradation in the E. coli host cell via the clpXP/clpAP system. In another example, the degradation tag is the RepA tag which can be located at the N-terminus of a target protein as described in Butz et al., Biochemistry, 2011, 50 (40), pp 8594-8602. The N-terminal RepA tag can serve to mark the target protein of interest for degradation in the E. coli host cell via the clpXP/clpAP system.

***Hypothesis-driven Diversity Pools and Hill Climbing***

The present disclosure teaches that the HTP genomic engineering methods of the present disclosure do not require prior genetic knowledge in order to achieve significant gains in host cell performance. Indeed, the present disclosure teaches methods of generating diversity pools (e.g., Figure 1) via several functionally agnostic approaches, including random mutagenesis, and identification of genetic diversity among pre-existing host cell variants (e.g., such as the comparison between a wild type host cell and an industrial variant).

In some examples however, the present disclosure also teaches hypothesis-driven methods of designing genetic diversity mutations that will be used for downstream HTP engineering. That is, in some examples, the present disclosure teaches the directed design of selected mutations. In some examples, the directed mutations are incorporated into the engineering libraries of the present disclosure (e.g., SNP swap, PRO swap, STOP swap, SOLUBILITY TAG swap, or DEGRADATION TAG swap).

In some examples, the present disclosure teaches the creation of directed mutations based on gene annotation, hypothesized (or confirmed) gene function, or location within a genome. The diversity pools of the present disclosure may include mutations in genes hypothesized to be involved in a specific metabolic or genetic pathway associated in the literature with increased performance of a host cell. In other examples, the diversity pool of the present disclosure may also include mutations to genes present in an operon associated with improved host performance. In yet other examples, the diversity pool of the present disclosure may also include mutations to genes based on algorithmic predicted function, or other gene annotation.

In some examples, the present disclosure teaches a "shell" based approach for prioritizing the targets of hypothesis-driven mutations. The shell metaphor for target prioritization is based on the hypothesis that only a handful of primary genes are responsible for most of a particular example of a host cell's performance (*e.g.*, production of a single biomolecule). These primary genes are located at the core of the shell, followed by secondary effect genes in the second layer, tertiary effects in the third shell, and... etc. For example, in one example the core of the shell might comprise genes encoding critical biosynthetic enzymes within a selected metabolic pathway (*e.g.*, production of citric acid). Genes located on the second shell might comprise genes encoding for other enzymes within the biosynthetic pathway responsible for product diversion or feedback signaling. Third tier genes under this illustrative metaphor would likely comprise regulatory genes responsible for modulating expression of the biosynthetic pathway, or for regulating general carbon flux within the host cell.

The present disclosure also teaches "hill climb" methods for optimizing performance gains from every identified mutation. In some examples, the present disclosure teaches that random, natural, or hypothesis-driven mutations in HTP diversity libraries can result in the identification of genes associated with host cell performance. For example, the present methods may identify one or more beneficial SNPs located on, or near, a gene coding sequence. This gene might be associated with host cell performance, and its identification can be analogized to the discovery of a performance "hill" in the combinatorial genetic mutation space of an organism.

In some examples, the present disclosure teaches methods of exploring the combinatorial space around the identified hill embodied in the SNP mutation. That is, in some examples, the present disclosure teaches the perturbation of the identified gene and associated regulatory sequences in order to optimize performance gains obtained from that gene node (*i.e.,* hill climbing). Thus, according to the methods of the present disclosure, a gene might first be identified in a diversity library sourced from random mutagenesis, but might be later improved for use in the strain improvement program through the directed mutation of another sequence within the same gene.

The concept of hill climbing can also be expanded beyond the exploration of the combinatorial space surrounding a single gene sequence. In some examples, a mutation in a specific gene might reveal the importance of a particular metabolic or genetic pathway to host cell performance. For example, in some examples, the discovery that a mutation in a single RNA degradation gene resulted in significant host performance gains could be used as a basis for mutating related RNA degradation genes as a means for extracting additional performance gains from the host organism. Persons having skill in the art will recognize variants of the above described shell and hill climb approaches to directed genetic design.

## Biosynthetic Pathway Scaffolding

In some examples, the present disclosure teaches that the productivity of some bioindustrial processes is limited by the random diffusion of substrates, intermediates, and biosynthetic enzymes within a host cell. In some examples, the present disclosure teaches that productivity of host cell cultures can be increased by co-localizing biosynthetic enzymes in a pathway. Thus, in some examples, the present disclosure teaches tethering biosynthetic enzymes to a scaffold, such as a DNA or protein scaffold.

In some examples, co-localization is achieved by recombinant fusions of DNA binding domains to the biosynthetic enzymes in the pathway, which then bind to a DNA scaffold region, thus constraining the pathway enzymes close to one another in the cell. In other examples, co-localization is achieved by recombinant fusions of protein beinding domains to the biosynthetic enzymes in the pathway, which then bind to a protein scaffold region, thus constraining the pathway enzymes close to one another in the cell. In some examples, co-localization increases the rate of production and decreases the concentration of pathway intermediates in the cell (*see* Figure 44).

In some examples, the present disclosure teaches a high-throughput method for engineering the genome of *Escherichia coli,* wherein nucleotide sequences encoding DNA binding or protein binding domains are inserted into genes encoding enzymes in a biosynthetic pathway, and a DNA scaffold plasmid or a scaffold protein is introduced into the cell. In accordance with one example of the disclosure, it is believed that the DNA or protein binding domains tethered to the biosynthetic genes will localize the recombinant pathway enzymes together onto the scaffold plasmid or peptide, thus leading to improved productivity of the target product.

In some examples, this disclosure solves the problem of diffusion-limited productivity of small molecules in *E. coli* cells with genomes engineered via high-throughput methods. Currently, the only reported examples of DNA scaffolding to localize biosynthetic enzymes have been low throughput processes in which the recombinant pathway enzymes are encoded on plasmids (Lee, et al. "Improved Production of L-Threonine in Escherichia coli by Use of a DNA Scaffold System" App. And Environ. Microbiol. Vol 79(3), pp. 774-782 (2013)). In some examples, the current disclosure provides a means to incorporate DNA binding domains into pathway enzymes that are chromosomally-encoded, in a high-throughput manner.

In some examples, the present disclosure teaches chimeric biosynthetic enzymes and scaffold DNAs and proteins. The various examples of this technology are discussed in more detail below.

### *DNA Binding Chimeric Proteins*

In some examples, the present disclosure teaches chimeric proteins comprising selected biosynthetic enzymes that are tethered to DNA binding domain. In accordance with these examples, it is expected that the chimeric biosynthetic enzymes will be recruited to a DNA scaffold by their DNA binding domains, thereby concentrating the various biosynthetic activities to an area of the host cell.

In some examples, the biosynthetic enzymes and the DNA binding domains are covalently linked. In some examples, the biosynthetic enzymes are translationally fused to the DNA binding domains. Thus, in some examples the chimeric biosynthetic enzymes are formed by coupling the DNA binding domain to the amino terminus, the carboxy terminus, or to an internal site within the biosynthetic pathway protein. Persons having skill in the art will recognize the need to ensure that the addition of the DNA binding domain does not substantially reduce the activity of the biosynthetic enzyme.

In some examples of the present disclosure, the biosynthetic enzyme is coupled to its DNA-binding domain via a short polypeptide linker sequence. Suitable linkers include peptides of between about 6 and about 40 amino acids in length. Preferred linker sequences include glycine-rich (e.g. G3-5), serine-rich (e.g. GSG, GSGS (SEQ ID NO. 18), GSGSG (SEQ ID NO. 19), GSNG (SEQ ID NO. 20), or alanine rich (e.g., TSAAA (SEQ ID NO. 21)) linker sequences. Other exemplary linker sequences have a combination of glycine, alanine, proline and methionine residues such as AAAGGM (SEQ ID NO. 22); AAAGGMPPAAAGGM (SEQ ID NO. 23); AAAGGM (SEQ ID NO. 24); and PPAAAGGMM (SEQ ID NO. 25). Linkers may have virtually any sequence that results in a generally flexible chimeric biological pathway protein.

In some examples, the methods of the present disclosure are compatible with any DNA binding domain capable of function in cis with the biosynthetic enzyme. In some examples, the DNA binding domains are preferably exogenous to the host organism. In other examples, the present disclosure teaches selection of DNA binding domains which are sufficiently selective to avoid excessive binding outside of the designed scaffold DNA.

Various DNA-binding domains are known in the art along with their corresponding nucleotide recognition sites in DNA (i.e., DNA binding sites) and are suitable for use in the system and methods of the present disclosure. For example, in one example of the present disclosure, the DNA binding portion of a chimeric biological pathway protein comprises a leucine zipper DNA binding domain wherein the scaffold comprises the corresponding leucine zipper DNA binding sequence. In another example of the present disclosure, the DNA binding portion of a chimeric biological pathway protein comprises a helix-loop-helix DNA binding domain wherein the scaffold comprises the corresponding helix-loop-helix DNA binding sequence. In another example, the DNA binding portion of a chimeric biological pathway protein comprises a winged helix DNA binding domain wherein the scaffold comprises the corresponding winged helix DNA binding sequence. In another example, the DNA binding portion of a chimeric biological pathway protein comprises a winged helix-turn-helix DNA binding domain wherein the scaffold comprises the corresponding winged helix-turn-helix DNA binding sequence. In another example, the DNA binding portion of a chimeric biological pathway protein comprises a helix-turn-helix DNA binding wherein the scaffold comprises the corresponding helix-turn-helix DNA binding sequence. In another example, the DNA binding portion of the chimeric biological pathway protein comprises a HMG-box DNA binding domain wherein the scaffold comprises the corresponding HMG-box DNA binding sequence. In another example, the DNA binding portion of the chimeric biological pathway protein comprises a custom designed TALE DNA binding domain wherein the scaffold comprises the corresponding designed TALE DNA binding sequence. In another example of the present disclosure, the DNA binding portion of a chimeric biological pathway protein comprises a zinc finger DNA binding domain wherein the scaffold comprises the corresponding zinc finger DNA binding sequence.

Exemplary zinc finger DNA binding domain sequences and corresponding DNA binding sites are provided in Table 1.3. Other zinc finger DNA binding domains and their corresponding target DNA binding sequences known in the art are also suitable for use in the present disclosure (see e.g., Greisman H A and Pabo C O, "A General Strategy for Selecting High-Affinity Zinc Finger Proteins for Diverse DNA Target Sites," Science 275:657-661 (1997), Rebar E J and Pabo C O, "Zinc Finger Phage: Affinity Selection of Fingers with New DNA-Binding Specificities," Science 263:671-673 (1994); Maeder et al., "Rapid "Open-Source" Engineering of Customized Zinc-Finger Nucleases for Highly Efficient Gene Modification," Mol. Cell. 31:294-301 (2008), Sander et al., "Selection-Free Zinc-Finger-Nuclease Engineering by Context-Dependent Assembly (CoDA)," Nat. Methods 8:67-69 (2011), U.S. Pat. No. 5,5789,538 to Rebar, U.S. Pat. No. 6,410,248 to Greisman, U.S. Pat. No. 7,605,140 to Rebar, U.S. Pat. No. 6,140,081 to Barbas, U.S. Pat. No. 7,067,617 to Barbas, U.S. Pat. No. 6,205,404 to Michaels, and U.S. Patent Application Publication No. 20070178454 to Joung.

Methods for optimizing the DNA binding specificities of zinc finger domains and methods of engineering synthetic DNA binding sites are also known in the art and can be utilized in the present disclosure to generate new zinc finger binding partners (see e.g., Bulyk et al., "Exploring the DNA-binding Specificities of Zinc Fingers with DNA Microarrays," Proc. Nat'l Acad. Sci. U.S.A 98(13): 7158-63 (2001) and "Hurt et al., "Highly Specific Zinc Finger Proteins Obtained by Directed Domain Shuffling and Cell-based Selection," Proc. Nat'l Acad. Sci. U.S.A. 100(21): 12271-6 (2003), U.S. Pat. No. 5,5789,538 to Rebar, U.S. Pat. No. 6,410,248 to Greisman, U.S. Pat. No. 7,605,140 to Rebar, U.S. Pat. No. 6,140,081 to Barbas, U.S. Pat. No. 7,067,617 to Barbas, U.S. Pat. No. 6,205,404 to Michaels, and U.S. Patent Application Publication No. 20070178454 to Joung.

**Table 1.3** - non-limiting list of DNA binding domains.

| Zinc Finger | DNA Binding Domain Sequence | DNA Binding Sequence (5'→-3') |
|---|---|---|
| Zif268 | PGEKPYACPVESCDRRFSRSDELTRHIRIHTGQKPFQC RICMRNFSRSDHLTTHIRTHTGEKPFACDICGRKFARS DERKRHTKIHT (SEQ ID NO. 26) | GCGTGGGCG<br>GCG GGG GCG |
| PBSII | PGEKPYACPECGKSFSQRANLRAHQRTHTGEKPYKC PECGKSFSRSDHLTTHQRTHTGEKPYKCPECGKSFSR SDVLVRHQRTHT (SEQ ID NO. 27) | GTGTGGAAA |
| ZFa | PGERPFQCRICMRNFSDSPTLRRHTRTHTGEKPFQCRI CMRNFSVRHNLTRHLRTHTGEKPFQCRICMRNFSDRT SLARHLKTH (SEQ ID NO. 28) | GTCGATGCC |
| ZFb | PGERPFQCRICMRNFSKKDHLHRHTRTHTGEKPFQCR ICMRNFSLSQTLKRHLRTHTGEKPFQCRICMRNFSRL DMLARHLKTH (SEQ ID NO. 29) | GCGGCTGGG |
| ZFc | PGERPFQCRICMRNFSSPSKLIRHTRTHTGEKPFQCRIC MRNFSDGSNLARHLRTHTGEKPFQCRICMRNFSRVD NLPRHLKTH (SEQ ID NO. 30) | GAGGACGGC |
| Tyr123 | EKPYKCPECGKSFSDRSNLTRHQRTHTGEKPYKCPEC GKSFSTTSNLARHQRTHTGEKPFKCPECGKSFSRSDA LTRHQRTHT (SEQ ID NO. 31) | GTGGATGAC |
| Tyr456 | EKPYKCPECGKSFSQSSNLARHQRTHTGEKPYKCPEC GKSFSRSDHLTKHQRTHTGEKPFKCPECGKSFSQSSN LARHQRTHT (SEQ ID NO. 32) | GAAGGGGAA |

(continued)

| Zinc Finger | DNA Binding Domain Sequence | DNA Binding Sequence (5'→-3') |
|---|---|---|
| Blues | ASDDRPYACPVESCDRRFSRRDVLMNHIRIHTGQKPFQCRICMRNFSRSDHLTTHIRTHTGEKPFACDICGRKFANRDTLTRHSKIHLRQNDLE (SEQ ID NO. 33) | GTTTGGATG |
| Jazz | ASDDRPYACPVESCDRRFSRSDELTRHIRIHTGQKPFQCRICMRNFSSRDVLRRHNRTHTGEKPFACDICGRKFASRDVLRRHNRIHLRQNDLE (SEQ ID NO. 34) | GCTGCTGCG |
| Bagly | EFMTGDRPYACPVESCDRRFSRSDELTRHIRIHTGQKPFQCRICMRNFSSRDVLRRHNRTHTGEKPFACDICGRKFASRDVLRRHNRIHLRQGRSHVCAECGKAFVESSKLKRHQLVHTGEKPFQLE (SEQ ID NO. 35) | CGGGCTGCTGC (SEQ ID NO. 36) |
| Gli1 | KREPESVYETDCRWDGCSQEFDSQEQLVHHINSEHIHGERKEFVCHWGGCSRELRPFKAQYMLVVHMRRHTGEKPHKCTFEGCRKSYSRLENLKTHLRSHTGEKPYMCEHEGCSKAFSNASDRAKHQNRTHSNEKPYVCKLPGCTKRYTDPSSLRKHVKTVHGPDAHVTKRHRGD (SEQ ID NO. 37) | GACCACCCAAGACGA (SEQ ID NO. 38) |
| HIVC | PFQCRICMRNFSLRTDLDRHTRTHTGEKPFQCRICMRNFSLSQTLRRHLRTHTGEKPFQCRICMRNFSLRSNLGRHLKTHTGEK (SEQ ID NO. 39) | GATGCTGCA |
| B3 | AQAALEPKEKPYACPECGKSFSDPGNLVRHQRTHTGEKPYKCPECGKSFSRSDKLVRHQRTHTGEKPYKCPECGKSFSQSSHLVRHQRTHTGKKTSGQAG (SEQ ID NO. 40) | GACGGGGG |
| N1 | AQAALEPKEKPYACPECGKSFSQSSSLVRHQRTHTGEKPYKCPECGKSFSQSSNLVRHQRTHTGEKPYKCPECGKSFSRSDKLVRHQRTHTGKKTSGQAG (SEQ ID NO. 41) | GTAGAAGGG |

(continued)

| Zinc Finger | DNA Binding Domain Sequence | DNA Binding Sequence (5'→-3') |
|---|---|---|
| Sp-1 | PGKKKQHICHIQGCGKVYGKTSHLRAHLRWHTGERP FMCTWSYCGKRFTRSDELQRHKRTHTGEKKFACPEC PKRFMRSDHLSKHIKTHQNKKG (SEQ ID NO. 42)<br>PGKKKQHACPECGKSFSKSSHLRAHQRTHTGERPYK CPECGKSFSRSDELQRHQRTHTGEKPYKCPECGKSFS RSDHLSKHQRTHQNKKG (SEQ ID NO. 43) | GGGGCGGGG |

*Nucleic Acid Scaffold Sequence*

In some examples, the present disclosure teaches a DNA scaffold comprising one or more of the DNA binding sequences corresponding to the DNA binding domains contained within the chimeric biosynthetic enzymes. In some examples the DNA scaffold is an extrachromosomal plasmid or other vector. In other examples, the DNA scaffold is encoded within the genome of a host cell.

Suitable nucleic acid vectors include, without limitation, plasmids, baculovirus vectors, bacteriophage vectors, phagemids, cosmids, fosmids, bacterial artificial chromosomes, viral vectors (for example, viral vectors based on vaccinia virus, poliovirus, adenovirus, adeno-associated virus, SV40, herpes simplex virus, and the like), artificial chromosomes, yeast plasmids, yeast artificial chromosomes, and other vectors. In some examples of the present disclosure, vectors suitable for use in prokaryotic host cells are preferred. Accordingly, exemplary vectors for use in prokaryotes such as *Escherichia coli* include, but are not limited to, pACYC184, pBeloBacll, pBR332, pBAD33, pBBR1MCS and its derivatives, pSC101, SuperCos (cosmid), pWE15 (cosmid), pTrc99A, pBAD24, vectors containing a ColE1 origin of replication and its derivatives, pUC, pBluescript, pGEM, Ori_Plsmd27 (SEQ ID NO. 213), vector backbone 1 (SEQ ID NO. 214), vector backbone 2 (SEQ ID NO. 215), vector backbone 3 (SEQ ID NO. 216), vector backbone 4 (SEQ ID NO. 217), and pTZ vectors.

In some examples, the present disclosure teaches that a nucleic acid scaffold subunit may comprise 2, 3, 4, 5, 6, 7, 8, 9, 10, 15, 20, 25, or more different DNA binding sites coupled together to facilitate the binding and immobilization of 2, 3, 4, 5, 6, 7, 8, 9, 10, 15, 20, 25, or more different biosynthetic pathway proteins. In some examples, the present disclosure teaches that the DNA scaffolds have a single DNA binding site for each corresponding chimeric biosynthetic protein.

In other examples, the nucleic acid scaffold may comprise two or more copies of the same DNA binding site. This architecture allows for optimizing the biological protein stiochiometry to be achieved. In accordance with this example of the present disclosure, the same DNA binding sites may be coupled together to create enzymatic centers for a particular chemical reaction. Thus in some examples, the DNA scaffold comprises groups of multiple DNA binding sites, each group corresponding to a specific chimeric biosynthetic gene/enzyme.

In some examples of the present disclosure, the method of assembling a synthetic biological pathway involves immobilizing at least a first chimeric biosynthetic gene (e.g., enzyme) and a second chimeric biosynthetic enzyme onto nucleic acid scaffold. The first chimeric biosynthetic enzyme produces a first product that is a substrate for the second chimeric biological pathway protein. The second chimeric biosynthetic enzyme is immobilized onto the scaffold construct such that it is positioned adjacent to or very close to the first chimeric biosynthetic enzyme. In this way, the effective concentration of the first product is high, and the second chimeric biosynthetic enzyme can act efficiently on the first product. As an example, a synthetic nucleic acid scaffold has immobilized thereon, in order from 3'→5' or 5'→3' of the scaffold construct a) the first chimeric biosynthetic enzyme, and b) the second chimeric biosynthetic enzyme to form a "scaffold subunit." The scaffold subunit can be repeated two or more times within the synthetic nucleic acid scaffold.

In accordance with this and all examples of the present disclosure, two or more copies (e.g., two, three, four, five, six, seven, eight, nine, ten, or more molecules) of each chimeric biosynthetic enzyme can be immobilized onto a scaffold subunit. For example, in some examples, a scaffold subunit has immobilized thereon, a) one molecule (copy) of the first chimeric biosynthetic enzyme and b) one molecule of the second chimeric biosynthetic enzyme. In other examples, a scaffold subunit has immobilized thereon, a) one molecule of the first chimeric biosynthetic enzyme and b) two or more molecules (e.g., two, three, four, five, six, or more molecules) of the second chimeric biosynthetic enzyme.

Accordingly, the ratio of any given protein in a biological pathway to any other protein in the pathway can be varied. By way of example only, the ratio of a first chimeric biological pathway protein to a second chimeric biological pathway protein can be varied from about 0.1:10 to about 10:0.1, e.g., from about 0.1:10 to about 0.5:10, from about 0.5:10 to about 1.0:10, from about 1.0:10 to about 2:10, from about 2:10 to about 5:10, from about 5:10 to about 7:10, from about 7:10 to about 10:10, from about 10:7 to about 10:5, from about 10:5 to about 10:2, from about 10:2 to about 10:1, from about 10:1 to about 10:0.5, or from about 10:0.5 to about 10:1.

In some examples, at least three chimeric biosynthetic enzymes are immobilized onto the synthetic nucleic acid scaffold to comprise a scaffold subunit. In accordance with this example of the present disclosure, the first chimeric biosynthetic enzyme produces a first product that is a substrate for the second chimeric biosynthetic enzyme, and the second chimeric biological pathway protein produces a second product that is a substrate for the third chimeric biosynthetic enzyme. In these examples, a scaffold subunit has immobilized thereon, in order from 3'→5' or 5'→3' of the scaffold a) the first chimeric biosynthetic enzyme, b) the second chimeric biosynthetic enzyme, and c) the third biosynthetic enzyme. The scaffold unit can be repeated two or more times in the nucleic acid construct as described supra.

In another example of the present disclosure, at least four chimeric biosynthetic enzymes are immobilized onto the nucleic acid scaffold. In another example of the present disclosure, at least five chimeric biosynthetic enzymes are immobilized onto the nucleic acid scaffold. It will be apparent from these examples that a sixth, seventh, eighth, ninth, tenth, etc., chimeric biosynthetic enzymes can be immobilized onto the nucleic acid scaffold, that the chimeric proteins are immobilized spatially in the order in which they function in a pathway, and that each protein can be immobilized onto the scaffold in one two, three, four, five, six, seven, eight, nine, ten, or more copies (or molecules).

In some examples, the present disclosure teaches spacing of each DNA binding site within the scaffold nucleic acid. In accordance with this example of the present disclosure, the two or more DNA binding sites are located adjacent to each other within a scaffold subunit, coupled to each other in tandem or separated by at least one spacer nucleotide. The two or more DNA binding sites may separated from each other by 2, 3, 4, 5, 6, 7, 8, 9, 10, 11, 12 13, 14, 15, 16, 17, 18, 19, 20, 25, 30, 35, 40, 45, 50, or more spacer nucleotides. The spacing between different DNA binding sites can vary within one scaffold unit (i.e., the spacing between a first and second DNA binding site may differ from the spacing between the second and third DNA binding site). Optimal spacing between different DNA binding sites within a scaffold subunit will vary depending on the biosynthetic enzyme requirements and the biological pathway being reconstructed, and should be optimized to achieve optimal biological pathway productivity.

## Peptide Scaffolding

In some examples, the scaffolding methods of the present disclosure can also be applied to protein/structural scaffolds within the cell. In some examples, the present disclosure teaches application of methods disclosed in U.S. Published Patent Application No. 20110008829.

## Protein-Binding Chimeric Proteins

In some examples, the present disclosure teaches chimeric proteins comprising selected biosynthetic enzymes that are tethered to one or more protein binding domain(s) capable of binding to a recruitment peptide. In accordance with these examples, it is expected that the chimeric biosynthetic enzymes will be recruited to a scaffold peptide by interacting with recruitment peptides contained within the scaffold peptide.

In some examples, the biosynthetic enzymes and the protein binding domains are covalently linked. In some examples, the biosynthetic enzymes are translationally fused to the protein binding domains. Thus, in some examples the chimeric biosynthetic enzymes are formed by coupling the protein binding domain to the amino terminus, the carboxy terminus, or to an internal site within the biosynthetic pathway protein. Persons having skill in the art will recognize the need to ensure that the addition of the protein binding domain does not substantially reduce the activity of the biosynthetic enzyme.

In some examples of the present disclosure, the biosynthetic enzyme is coupled to its protein-binding domain via a short polypeptide linker sequence as described in earlier portions of this disclosure.

Various protein-binding domains (PBD) are known in the art along with their corresponding recruitment peptides sequences and are suitable for use in the system and methods of the present disclosure. A non-limiting illustrative discussion of suitable PBD follows.

SH3

Suitable PBDs include SH3 domains. SH3 domains include Class I SH3 domains; Class II SH3 domains; and unconventional SH3 domains. Amino acid sequences of SH3 domains are known in the art. See, for example, amino acids 136-189 of the amino acid sequence provided in GenBank Accession No. NP.sub.--058431 (Homo sapiens Crk protein); amino acids 136-189 of the amino acid sequence provided in GenBank Accession No. AAH31149 (Mus musculus Crk protein); and amino acids 4-77 of the amino acid sequence provided in GenBank Accession No. P27986 (Homo sapiens p85 subunit of phosphatidylinositol 3-kinase).

In some examples, an SH3 domain is a Class I SH3 domain and comprises an amino acid sequence having at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence similarity to amino acid sequence: EGYQYRA LYDYKKEREE DIDLHLGDIL TVNKGSLVAL GFSDGQEARP EEIGWLNGYN ETTGERGDFP GTYVEYI (SEQ ID NO. 44), including all ranges and subranges there between.

In some examples, an SH3 domain is a Class II SH3 domain and comprises an amino acid sequence having at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence similarity to amino acid sequence: YVRALFDFNGNDEEDLPFKKGDILRIRDKPEEQWWNAEDSEGKRGMIPVPYVEK (SEQ ID NO. 45). As one non-limiting example, an SH3 domain comprises the amino acid sequence: MAEYVRALFDFNGNDEEDLPFKKGDILRIRDKPEEQWWNAEDSEGKRGMIPVPYVEKY (SEQ ID NO. 46), including all ranges and subranges there between.

An SH3 domain binds proline-rich peptides that form a left-handed poly-proline type II helix, where such peptides comprise the minimal consensus sequence Pro-X-X-Pro. In some examples, each Pro is preceded by an aliphatic residue. In some examples, a recruitment peptide is an SH3 domain ligand. An SH3 domain binds proline-rich peptides that form a left-handed poly-proline type II helix, where such peptides comprise the minimal consensus sequence Pro-X-X-Pro. In some examples, each Pro is preceded by an aliphatic residue. Exemplary, non-limiting examples of amino acid sequences of peptides comprising SH3 domain ligands include: RPLPVAP (SEQ ID NO. 47; bound by a Class I SH3 domain); PPPALPPKRRRPG (SEQ ID NO. 48); and PPPALPPKKR (SEQ ID NO. 49; bound by a Class II SH3 domain).

PDZ

Suitable PBD include PDZ domains. Amino acid sequences of PDZ domains are known in the art. See, for example, amino acids 108-191, amino acids 201-287, and amino acids 354-434 of the amino acid sequence provided in Gen Bank Accession No. AAC52113 (Homo sapiens post-synaptic density protein 95); and amino acids 80-161 of the amino acid sequence provided in GenBank Accession No. NP_033254 (Mus musculus syntrophin).

In some examples, a suitable PDZ domain comprises an amino acid sequence having at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence similarity to amino acid sequence: EITLERGNSGLGFSIAGGTDNPHIGDDPSIFIT KIIPGGAAAQDGRLRVNDSILFVNEVDVREVTHSAAVEALKEAGSIVRLYV (SEQ ID NO. 50), including all ranges and subranges there between.

In some examples, a suitable PDZ domain comprises an amino acid sequence having at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence similarity to amino acid sequence: VMEIKLIKGPKGLGFSIAGGVGNQHIPGDN SIYVTKIIEGGAAHKDGRLQ IGDKILAVNSVGLEDVMHEDAVAALKNTYDVVYLKVA (SEQ ID NO.51), including all ranges and subranges there between.

In some examples, a suitable PDZ domain comprises an amino acid sequence having at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence similarity to amino acid sequence: RIVIHRGSTGLGFNIVGGEDGEGIFISFILAGGPA DLSGELRKGDQILSVNGVDLRNASHEQAAIALKNAGQTVTIIAQ (SEQ ID NO. 52), including all ranges and subranges there between.

In some examples, a suitable PDZ domain comprises an amino acid sequence having at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence similarity to amino acid sequence: RRVTVRKADAGGLGISIKGGRENKMPILISK IFKGLAADQTEALFVGDAILSVNGEDLSSATHDEAVQALKKTGKEWLEVK (SEQ ID NO. 53), including all ranges and sub ranges there between. For example, a PDZ domain can comprise the amino acid sequence MLQRRRVTVRKADAGGLGISIKGGRENKMPILISKIFKGLAADQTEALFVGDAILSVNGE DLSS ATHDEAVQALKKTGKEVVLEVKYMKEVSPYFKGS (SEQ ID NO. 54).

In some examples, a recruitment peptide is a PDZ domain ligand. A PDZ domain binds to the C-terminal 4-5 residues of target proteins. In some examples, a consensus PDZ domain ligand comprises a hydrophobic residue, e.g., Val or Ile, at the carboxyl terminus. Exemplary, non-limiting examples of amino acid sequences of peptides comprising PDZ domain ligands include: IESDV (SEQ ID NO. 55); VKESLV (SEQ ID NO. 56); GVKESLV (SEQ ID NO. 57); GVKQSLL (SEQ ID NO. 58); GVKESGA (SEQ ID NO. 59); YVKESLV (SEQ ID NO. 60); and VETDV (SEQ ID NO. 61).

## GBD

Suitable PBD include GTPase-binding domains (GBD), also referred to in the art as CRIB (Cdc42/Rac-interactive binding) motifs. In some examples, a GBD binds a Cdc42p-like and/or a Rho-like small GTPase. Amino acid sequences of GBD are known in the art. See, e.g., amino acids 198-240 of the amino acid sequence provided in GenBank Accession No. NP.sub.--001103835 (Rattus norvegicus Wiskott-Aldrich syndrome-like protein (WASP)); amino acids 69-112 of the amino acid sequence provided in GenBank Accession No. Q13177 (Homo sapiens PAK-2); and amino acids 70-105 of the amino acid sequence provided in GenBank Accession No. P35465 (Rattus norvegicus PAK-1). See also the amino acid sequences PAK (75-111), ACK (504-549), and WASP (232-274), presented in FIG. 3A of Garrard et al. (2003) EMBO J. 22:1125. See also the amino acid sequences ACK (505-531), WASP (236-258), PAK1 (70-94), PAK2 (71-91), PAK-4 (6-30), presented in FIG. 1A of Bishop and Hall (2000) Biochem. J. 348:241.

In some examples, a suitable GBD comprises an amino acid sequence having at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence similarity to amino acid sequence: ADI GTPSNFQHIG HVGWDPNTGF DLNNLDPELK NLFDMCGISE (SEQ ID NO. 62), and all ranges and sub ranges there between.

In some examples, a suitable GBD comprises an amino acid sequence having at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence similarity to amino acid sequence: KERPEISLPSDFEHTIHVGFDAVTGEFTGMPEQWAR (SEQ ID NO. 63), and all ranges and sub ranges there between.

In some examples, a suitable GBD comprises an amino acid sequence having at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence similarity to amino acid sequence: MTKADIGTPSNFQHIGHVGWDPNTGFDLNNLDPELKNLFDMCGISEAQLKDRETSKVIY DFIEK TGGVEAVKNELRRQAP (SEQ ID NO. 64), and all ranges and subranges there between.

In some examples, an recruitment peptide is a GBD ligand. An exemplary, non-limiting GBD ligand comprises the amino acid sequence LVGALMHVMQKRSRAIHSSDEGEDQAGDEDED (SEQ ID NO. 65).

## Leucine Zipper Peptides

Suitable PBD include leucine zipper peptides. In some examples, leucine zipper peptides are peptides that interact via a coiled-coil domain. Amino acid sequences of leucine zipper domains are known in the art. Leucine zipper peptides include an EE12RR345L leucine zipper peptide; an RR12EE354L leucine zipper peptide; and the like.

An example of an amino acid sequence of a leucine zipper peptide is an EE12RR345L leucine zipper of the amino acid sequence: LEIEAAFLERENTALETRVAELRQRVQRLR NRVSQYRTRYGPLGGGK (SEQ ID NO. 66).

In some examples, a leucine zipper peptide comprises an amino acid sequence having at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% amino acid sequence similarity to amino acid sequence: LEIEAA FLERENTALETRVAELRQRVQRLRNRVSQYRTRYGPLGGGK (SEQ ID NO. 67), and all ranges and subranges there between. Such a leucine zipper peptide can serve as a PBD or as a recruitment peptide.

Another non-limiting example of an amino acid sequence of a leucine zipper peptide is an RR12EE345L leucine zipper peptide of the amino acid sequence: LEIRAAFLRQRNTALRT EVAELEQEVQRLENEVSQYETRYGPLGGGK (SEQ ID NO. 68).

The descriptions above have described the production of chimeric biosynthetic proteins comprising a protein binding domain designed to target (bind to) one or more recruitment peptides located on a scaffold polypeptide. Persons having skill in the art will recognize other compatible variations on this arrangement. For example, in some examples, the present disclosure teaches the production of chimeric biosynthetic proteins comprising recruitment peptides targeted by protein binding domains located on a scaffold polypeptide. In other examples, the protein binding domain and recruitment peptides are each incorporated into two or more chimeric biosynthetic proteins, such that the chimeric proteins form complexes (e.g., dimers or heterodimers). One illustrative example of complex formation is the use of compatible leucine zipper domains placed on chimeric biosynthetic proteins, such that two or more chimeric biosynthetic proteins are able to form a complex via the leucine zipper domains.

## Scaffold Polypeptide

In some examples, the present disclosure teaches a scaffold polypeptide that organizes biosynthetic pathway enzymes into a functional complex. In some examples, the scaffold polypeptides of the present disclosure comprise two or more recruitment peptides. That is, in some examples, the scaffold polypeptides of the present disclosure are capable of recruiting two more more chimeric biosynthetic proteins.

In some examples, the scaffold polypeptide of the present disclosure is an exogenous peptide introduced into a host cell (e.g., by the transformation of a DNA sequence encoding for the scaffold polypeptide, or direct introduction of the peptide). In other examples, the scaffold polypeptide is a naturally occurring structure within the host cell. That is, in some examples, the scaffold polypeptide is an organelle or membrane (e.g., the endoplasmid reticulum, or the golgi apartus). Thus, in some examples, the scaffold polypeptide of the presente disclosure includes host-cell structures composed of more than one peptide sequence.

In some examples, the recruitment peptide sequences within the scaffold polypeptide are organized so as to optimize target biosynthetic pathways whose enzymes are being recruited. In some examples, the organization of the scaffold polypeptide is similar to that described for DNA scaffolds above. Thus, in some examples, scaffold polypeptides contain groupings of recruitment peptides to regulate the order and ratios of various chimeric biosynthetic proteins.

***Cell Culture and Fermentation***

Cells of the present disclosure can be cultured in conventional nutrient media modified as appropriate for any desired biosynthetic reactions or selections. In some examples, the present disclosure teaches culture in inducing media for activating promoters. In some examples, the present disclosure teaches media with selection agents, including selection agents of transformants (*e.g.,* antibiotics), or selection of organisms suited to grow under inhibiting conditions (*e.g.,* high ethanol conditions). In some examples, the present disclosure teaches growing cell cultures in media optimized for cell growth. In other examples, the present disclosure teaches growing cell cultures in media optimized for product yield. In some examples, the present disclosure teaches growing cultures in media capable of inducing cell growth and also contains the necessary precursors for final product production (*e.g.*, high levels of sugars for ethanol production).

Culture conditions, such as temperature, pH and the like, are those suitable for use with the host cell selected for expression, and will be apparent to those skilled in the art. As noted, many references are available for the culture and production of many cells, including cells of bacterial, plant, animal (including mammalian) and archaebacterial origin. *See e.g.,* Sambrook, Ausubel (all supra), as well as Berger, Guide to Molecular Cloning Techniques, Methods in Enzymology volume 152 Academic Press, Inc., San Diego, CA; and Freshney (1994) Culture of Animal Cells, a Manual of Basic Technique, third edition, Wiley-Liss, New York and the references cited therein; Doyle and Griffiths (1997) Mammalian Cell Culture: Essential Techniques John Wiley and Sons, NY; Humason (1979) Animal Tissue Techniques, fourth edition W.H. Freeman and Company; and Ricciardelle et al., (1989) In Vitro Cell Dev. Biol. 25:1016-1024. For plant cell culture and regeneration, Payne et al. (1992) Plant Cell and Tissue Culture in Liquid Systems John Wiley & Sons, Inc. New York, N.Y.; Gamborg and Phillips (eds) (1995) Plant Cell, Tissue and Organ Culture*;* Fundamental Methods Springer Lab Manual, Springer-Verlag (Berlin Heidelberg N.Y.); Jones, ed. (1984) Plant Gene Transfer and Expression Protocols, Humana Press, Totowa, N.J. and Plant Molecular Biology (1993) R. R. D. Croy, Ed. Bios Scientific Publishers, Oxford, U.K. ISBN 0 12 198370 6. Cell culture media in general are set forth in Atlas and Parks (eds.) The Handbook of Microbiological Media (1993) CRC Press, Boca Raton, Fla.. Additional information for cell culture is found in available commercial literature such as the Life Science Research Cell Culture Catalogue from Sigma-Aldrich, Inc (St Louis, Mo.) ("Sigma-LSRCCC") and, for example, The Plant Culture Catalogue and supplement also from Sigma-Aldrich, Inc (St Louis, Mo.) ("Sigma-PCCS").

The culture medium to be used must in a suitable manner satisfy the demands of the respective strains. Descriptions of culture media for various microorganisms are present in the "Manual of Methods for General Bacteriology" of the American Society for Bacteriology (Washington D.C., USA, 1981).

The present disclosure furthermore provides a process for fermentative preparation of a product of interest, comprising the steps of: a) culturing a microorganism according to the present disclosure in a suitable medium, resulting in a fermentation broth; and b) concentrating the product of interest in the fermentation broth of a) and/or in the cells of the microorganism.

In some examples, the present disclosure teaches that the microorganisms produced may be cultured continuously-as described, for example, in WO 05/021772-or discontinuously in a batch process (batch cultivation) or in a fed-batch or repeated fed-batch process for the purpose of producing the desired organic-chemical compound. A summary of a general nature about known cultivation methods is available in the textbook by Chmiel (Bioprozeßtechnik. 1: Einführung in die Bioverfahrenstechnik (Gustav Fischer Verlag, Stuttgart, 1991)) or in the textbook by Storhas (Bioreaktoren and periphere Einrichtungen (Vieweg Verlag, Braunschweig/Wiesbaden, 1994)).

In some examples, the cells of the present disclosure are grown under batch or continuous fermentations conditions.

Classical batch fermentation is a closed system, wherein the compositions of the medium is set at the beginning of the fermentation and is not subject to artificial alternations during the fermentation. A variation of the batch system is a fed-batch fermentation which also finds use in the present disclosure. In this variation, the substrate is added in increments as the fermentation progresses. Fed-batch systems are useful when catabolite repression is likely to inhibit the metabolism of the cells and where it is desirable to have limited amounts of substrate in the medium. Batch and fed-batch fermentations are common and well known in the art.

Continuous fermentation is a system where a defined fermentation medium is added continuously to a bioreactor and an equal amount of conditioned medium is removed simultaneously for processing and harvesting of desired biomolecule products of interest. In some examples, continuous fermentation generally maintains the cultures at a constant high density where cells are primarily in log phase growth. In some examples, continuous fermentation generally maintains the cultures at a stationary or late log/stationary, phase growth. Continuous fermentation systems strive to maintain steady state growth conditions.

Methods for modulating nutrients and growth factors for continuous fermentation processes as well as techniques for maximizing the rate of product formation are well known in the art of industrial microbiology.

For example, a non-limiting list of carbon sources for the cultures of the present disclosure include, sugars and carbohydrates such as, for example, glucose, sucrose, lactose, fructose, maltose, molasses, sucrose-containing solutions from sugar beet or sugar cane processing, starch, starch hydrolysate, and cellulose; oils and fats such as, for example, soybean oil, sunflower oil, groundnut oil and coconut fat; fatty acids such as, for example, palmitic acid, stearic acid, and linoleic acid; alcohols such as, for example, glycerol, methanol, and ethanol; and organic acids such as, for example, acetic acid or lactic acid.

A non-limiting list of the nitrogen sources for the cultures of the present disclosure include, organic nitrogen-containing compounds such as peptones, yeast extract, meat extract, malt extract, corn steep liquor, soybean flour, and urea; or inorganic compounds such as ammonium sulfate, ammonium chloride, ammonium phosphate, ammonium carbonate, and ammonium nitrate. The nitrogen sources can be used individually or as a mixture.

A non-limiting list of the possible phosphorus sources for the cultures of the present disclosure include, phosphoric acid, potassium dihydrogen phosphate or dipotassium hydrogen phosphate or the corresponding sodium-containing salts.

The culture medium may additionally comprise salts, for example in the form of chlorides or sulfates of metals such as, for example, sodium, potassium, magnesium, calcium and iron, such as, for example, magnesium sulfate or iron sulfate, which are necessary for growth.

Finally, essential growth factors such as amino acids, for example homoserine and vitamins, for example thiamine, biotin or pantothenic acid, may be employed in addition to the abovementioned substances.

In some examples, the pH of the culture can be controlled by any acid or base, or buffer salt, including, but not limited to sodium hydroxide, potassium hydroxide, ammonia, or aqueous ammonia; or acidic compounds such as phosphoric acid or sulfuric acid in a suitable manner. In some examples, the pH is generally adjusted to a value of from 6.0 to 8.5, preferably 6.5 to 8.

In some examples, the cultures of the present disclosure may include an anti-foaming agent such as, for example, fatty acid polyglycol esters. In some examples the cultures of the present disclosure are modified to stabilize the plasmids of the cultures by adding suitable selective substances such as, for example, antibiotics.

In some examples, the culture is carried out under aerobic conditions. In order to maintain these conditions, oxygen or oxygen-containing gas mixtures such as, for example, air are introduced into the culture. It is likewise possible to use liquids enriched with hydrogen peroxide. The fermentation is carried out, where appropriate, at elevated pressure, for example at an elevated pressure of from 0.03 to 0.2 MPa. The temperature of the culture is normally from 20°C to 45°C and preferably from 25°C to 40°C, particularly preferably from 30°C to 37°C. In batch or fed-batch processes, the cultivation is preferably continued until an amount of the desired product of interest (*e.g.* an organic-chemical compound) sufficient for being recovered has formed. This aim can normally be achieved within 10 hours to 160 hours. In continuous processes, longer cultivation times are possible. The activity of the microorganisms results in a concentration (accumulation) of the product of interest in the fermentation medium and/or in the cells of said microorganisms.

In some examples, the culture is carried out under anaerobic conditions.

### *Screening*

In some examples, the present disclosure teaches high-throughput initial screenings. In other examples, the present disclosure also teaches robust tank-based validations of performance data (*see* Figure 6B).

In some examples, the high-throughput screening process is designed to predict performance of strains in bioreactors. As previously described, culture conditions are selected to be suitable for the organism and reflective of bioreactor conditions. Individual colonies are picked and transferred into 96 well plates and incubated for a suitable amount of time. Cells are subsequently transferred to new 96 well plates for additional seed cultures, or to production cultures. Cultures are incubated for varying lengths of time, where multiple measurements may be made. These may include measurements of product, biomass or other characteristics that predict performance of strains in bioreactors. High-throughput culture results are used to predict bioreactor performance.

In some examples, the tank-based performance validation is used to confirm performance of strains isolated by high-throughput screening. Candidate strains are screened using bench scale fermentation reactors (e.g., reactors disclosed in Table 5 of the present disclosure) for relevant strain performance characteristics such as productivity or yield.

**Product Recovery and Quantification**

Methods for screening for the production of products of interest are known to those of skill in the art and are discussed throughout the present specification. Such methods may be employed when screening the strains of the disclosure.

In some examples, the present disclosure teaches methods of improving strains designed to produce non-secreted intracellular products. For example, the present disclosure teaches methods of improving the robustness, yield, efficiency, or overall desirability of cell cultures producing intracellular enzymes, oils, pharmaceuticals, or other valuable small molecules or peptides. The recovery or isolation of non-secreted intracellular products can be achieved by lysis and recovery techniques that are well known in the art, including those described herein.

For example, in some examples, cells of the present disclosure can be harvested by centrifugation, filtration, settling, or other method. Harvested cells are then disrupted by any convenient method, including freeze-thaw cycling, sonication, mechanical disruption, or use of cell lysing agents, or other methods, which are well known to those skilled in the art.

The resulting product of interest, e.g. a polypeptide, may be recovered/isolated and optionally purified by any of a number of methods known in the art. For example, a product polypeptide may be isolated from the nutrient medium by conventional procedures including, but not limited to: centrifugation, filtration, extraction, spray-drying, evaporation, chromatography (*e.g.,* ion exchange, affinity, hydrophobic interaction, chromatofocusing, and size exclusion), or precipitation. Finally, high performance liquid chromatography (HPLC) can be employed in the final purification steps. (*See* for example Purification of intracellular protein as described in Parry et al., 2001, Biochem. J.353:117, and Hong et al., 2007, Appl. Microbiol. Biotechnol. 73:1331).

In addition to the references noted *supra,* a variety of purification methods are well known in the art, including, for example, those set forth in: Sandana (1997) Bioseparation of Proteins, Academic Press, Inc.; Bollag et al. (1996) Protein Methods, 2nd Edition, Wiley-Liss, NY; Walker (1996) The Protein Protocols Handbook Humana Press, NJ; Harris and Angal (1990) Protein Purification Applications: A Practical Approach, IRL Press at Oxford, Oxford, England; Harris and Angal Protein Purification Methods: A Practical Approach, IRL Press at Oxford, Oxford, England; Scopes (1993) Protein Purification: Principles and Practice 3rd Edition, Springer Verlag, NY; Janson and Ryden (1998) Protein Purification: Principles, High Resolution Methods and Applications, Second Edition, Wiley-VCH, NY; and Walker (1998) Protein Protocols on CD-ROM, Humana Press, NJ.

In some examples, the present disclosure teaches the methods of improving strains designed to produce secreted products. For example, the present disclosure teaches methods of improving the robustness, yield, efficiency, or overall desirability of cell cultures producing valuable small molecules or peptides.

In some examples, immunological methods may be used to detect and/or purify secreted or non-secreted products produced by the cells of the present disclosure. In one example approach, antibody raised against a product molecule (*e.g.*, against an insulin polypeptide or an immunogenic fragment thereof) using conventional methods is immobilized on beads, mixed with cell culture media under conditions in which the endoglucanase is bound, and precipitated. In some examples, the present disclosure teaches the use of enzyme-linked immunosorbent assays (ELISA).

In other related examples, immunochromatography is used, as disclosed in U.S. Pat. No. 5,591,645, U.S. Pat. No. 4,855,240, U.S. Pat. No. 4,435,504, U.S. Pat. No. 4,980,298, and Se-Hwan Paek, et al., "Development of rapid One-Step Immunochromatographic assay, Methods", 22, 53-60, 2000). A general immunochromatography detects a specimen by using two antibodies. A first antibody exists in a test solution or at a portion at an end of a test piece in an approximately rectangular shape made from a porous membrane, where the test solution is dropped. This antibody is labeled with latex particles or gold colloidal particles (this antibody will be called as a labeled antibody hereinafter). When the dropped test solution includes a specimen to be detected, the labeled antibody recognizes the specimen so as to be bonded with the specimen. A complex of the specimen and labeled antibody flows by capillarity toward an absorber, which is made from a filter paper and attached to an end opposite to the end having included the labeled antibody. During the flow, the complex of the specimen and labeled antibody is recognized and caught by a second antibody (it will be called as a tapping antibody hereinafter) existing at the middle of the porous membrane and, as a result of this, the complex appears at a detection part on the porous membrane as a visible signal and is detected.

In some examples, the screening methods of the present disclosure are based on photometric detection techniques (absorption, fluorescence). For example, in some examples, detection may be based on the presence of a fluorophore detector such as GFP bound to an antibody. In other examples, the photometric detection may be based on the accumulation on the desired product from the cell culture. In some examples, the product may be detectable via UV of the culture or extracts from said culture.

Persons having skill in the art will recognize that the methods of the present disclosure are compatible with host cells producing any desirable biomolecule product of interest. Table 2 below presents a non-limiting list of the product categories, biomolecules, and host cells, included within the scope of the present disclosure. These examples are provided for illustrative purposes, and are not meant to limit the applicability of the presently disclosed technology in any way.

**Table 2.** - A non-limiting list of the host cells and products of interest of the present disclosure.

| Product category | Products | Host category | Hosts |
|---|---|---|---|
| Amino acids | Lysine | Bacteria | *Escherichia coli* |
| Amino acids | Methionine | Bacteria | *Escherichia coli* |
| Amino acids | MSG | Bacteria | *Escherichia coli* |
| Amino acids | Threonine | Bacteria | *Escherichia coli* |
| Amino acids | Threonine | Bacteria | *Escherichia coli* |
| Amino acids | Tryptophan | Bacteria | *Escherichia coli* |
| Flavor & Fragrance | Agarwood | Bacteria | *Escherichia coli* |
| Flavor & Fragrance | Ambrox | Bacteria | *Escherichia coli* |
| Flavor & Fragrance | Nootkatone | Bacteria | *Escherichia coli* |
| Flavor & Fragrance | Patchouli oil | Bacteria | *Escherichia coli* |
| Flavor & Fragrance | Saffron | Bacteria | *Escherichia coli* |
| Flavor & Fragrance | Sandalwood oil | Bacteria | *Escherichia coli* |
| Flavor & | Valencene | Bacteria | *Escherichia coli* |
| Fragrance | | | |
| Flavor & Fragrance | Vanillin | Bacteria | *Escherichia coli* |
| Food | CoQ10/Ubiquinol | Bacteria | *Escherichia coli* |
| Food | Omega 3 fatty acids | Bacteria | *Escherichia coli* |
| Food | Omega 6 fatty acids | Bacteria | *Escherichia coli* |
| Food | Vitamin B12 | Bacteria | *Escherichia coli* |
| Food | Vitamin B2 | Bacteria | *Escherichia coli* |
| Food | Vitamin B2 | Bacteria | *Escherichia coli* |
| Food | Erythritol | Bacteria | *Escherichia coli* |
| Food | Erythritol | Bacteria | *Escherichia coli* |
| Food | Erythritol | Bacteria | *Escherichia coli* |
| Food | Steviol glycosides | Bacteria | *Escherichia coli* |
| Hydrocolloids | Diutan gum | Bacteria | *Escherichia coli* |
| Hydrocolloids | Gellan gum | Bacteria | *Escherichia coli* |
| Hydrocolloids | Xanthan gum | Bacteria | *Escherichia coli* |
| Intermediates | 1,3-PDO | Bacteria | *Escherichia coli* |
| Intermediates | 1,4-BDO | Bacteria | *Escherichia coli* |
| Intermediates | Butadiene | Bacteria | *Escherichia coli* |
| Intermediates | n-butanol | Bacteria | *Escherichia coli* |
| Organic acids | Citric acid | Bacteria | *Escherichia coli* |
| Organic acids | Citric acid | Bacteria | *Escherichia coli* |
| Organic acids | Gluconic acid | Bacteria | *Escherichia coli* |

(continued)

| Product category | Products | Host category | Hosts |
|---|---|---|---|
| Organic acids | Itaconic acid | Bacteria | *Escherichia coli* |
| Organic acids | Lactic acid | Bacteria | *Escherichia coli* |
| Organic acids | Lactic acid | Bacteria | *Escherichia coli* |
| Organic acids | LCDAs - DDDA | Bacteria | *Escherichia coli* |
| Polyketides/Ag | Spinosad | Bacteria | *Escherichia coli* |
| Polyketides/Ag | Spinetoram | Bacteria | *Escherichia coli* |

***Heterogolous Genes Of Interest***

In one example, provided herein are methods for expressing heterogolous genes in a microbial host cell. The heterogolous gene can be introduced into the microbial host cell using the methods provided herein and/or known in the art such that the microbial host cell uses the heterogolous gene to produce a product of interest. In one example, the microbial host cell is a strain of *E. coli.* The strain of *E. coli* can be any strain of *E. coli* known in the art and/or provided herein. The heterologous gene can be a wild-type verison of said gene or a mutant thereof. The heterologous gene can be operably linked to a promoter, terminator, protein solubility tag, protein degradation tag, or any combination thereof. Operably linking the heterologous gene to the promoter, terminator, protein solubility tag or protein degradation tag can be accomplished using the promoter swap, terminator swap, solubility tag swap and/or degradation swap methods provided throughout this disclosure.

In one example, the heterologous gene is operably linked to a promoter selected from Table 1. In one example, the heterologous gene is operably linked to a 60-90 bp chimeric synthetic promoter sequence, wherein the chimeric synthetic promoter consists of a distal portion of the lambda phage $p_R$ promoter, variable -35 and -10 regions of the lambda phage $p_L$ and $p_R$ promoters, core portions of the the lambda phage $p_L$ and $p_R$ promoters and either a 5' UTR/Ribosomal Binding Site (RBS) portion of the lambda phage $p_R$ promoter or a 5' UTR/Ribosomal Binding Site (RBS) portion of the promoter of the *E. coli acs* gene. The nucleic acid sequences of the distal portion of the lambda phage $p_R$ promoter, variable -35 and -10 regions of the lambda phage $p_L$ and $p_R$ promoters, core portions of the lambda phage $p_L$ and $p_R$ promoters and either a 5' UTR/Ribosomal Binding Site (RBS) portion of the lambda phage $p_R$ promoter or a 5' UTR/Ribosomal Binding Site (RBS) portion of the promoter of the *E. coli acs* gene for use in the chimeric synthetic promoter can be selected from the nucleic acid sequences found in Table 1.5. In one example, the heterologous gene can be operably linked to a chimeric synthetic promoter that has a nucleic acid sequence selected from SEQ ID NOs. 132-207 found in Table 1.4.

In one example, the heterologous gene is operably linked to a terminator selected from Table 1.2. In another example, the heterologous gene is operably linked to a terminator sequence selected from Table 19.

In one example, the heterologous gene is operably linked to a solubulity tag selected from Table 17.

In one example, the heterologous gene is operably linked to a degradation tag sequence selected from Table 18.

Further to the above examples, the heterologous gene can be any of the genes required to generate the products of interest found in Table 2 or any gene known in the art that can be expressed as a heterologous gene in the microbial host cell (e.g., *E. coli*) to produce a product of interest.

In one example, the heterogolous gene is a gene that is part of the lysine biosynthetic pathway as illustrated in Figure 19. Further to this example, the heterologous gene can be selected from the asd gene, the ask gene, the hom gene, the dapA gene, the dapB gene, the dapD gene, the ddh gene, the argD gene, the dapE gene, the dapF gene, the lysA gene, the lysE gene, the zwf gene, the pgi gene, the ktk gene, the fbp gene, the ppc gene, the pck gene, the ddx gene, the pyc gene or the icd gene. In one example, a heterologous gene that is part of the lysine pathway as provided herein is operably linked to a chimeric synthetic promoter with a nucleic acid sequence selected from SEQ ID NOs. 132-207.

In one example, the heterogolous gene is a gene that is part of the lycopene biosynthetic pathway as illustrated, for example, in Figure 59. Further to this example, the heterologous gene can be selected from the dxs gene, the ispC gene, the ispE gene, the ispD gene, the ispF gene, the ispG gene, the ispH gene, the idi gene, the ispA gene, the ispB gene, the crtE gene, the crtB gene, the crtI gene, the crtY gene, the ymgA gene, the dxr gene, the elbA gene, the gdhA gene, the appY gene, the elbB gene, or the ymgB gene. In one example, a heterologous gene that is part of the lycopene pathway as provided herein is operably linked to a chimeric synthetic promoter with a nucleic acid sequence selected from SEQ ID NOs. 132-207.

In one example, the heterogolous gene is a gene that encodes a biopharmaceutical or a gene in pathway for generating a biopharmaceutical. In one example, the microbial host cell is *E. coli* and the biopharmaceutical is any biopharmaceutical that has been shown to be produced in *E. coli.* The biopharmaceutical can be selected from humulin (rh insulin), intronA (interferon alpha2b), roferon (interferon alpha2a), humatrope (somatropin rh growth hormone), neupogen (filgrastim), detaferon (interferon beta-lb), lispro (fast-acting insulin), rapilysin (reteplase), infergen (interferon alfacon-1), glucagon, beromun (tasonermin), ontak (denileukin diftitox), lantus (long-acting insulin glargine), kineret (anakinra), natrecor (nesiritide), somavert (pegvisomant), calcitonin (recombinant calcitonin salmon), lucentis (ranibizumab), preotact (human parathyroid hormone), kyrstexxal (rh urate oxidase, PEGlyated), nivestim (filgrastim, rhGCSF), voraxaze (glucarpidase), or preos (parathyroid hormone). In one example, a heterologous gene that encodes a biopharmaceutical or a gene in a pathway that generates a biopharmaceutical as provided herein is operably linked to a chimeric synthetic promoter with a nucleic acid sequence selected from SEQ ID NOs. 132-207.

### *Selection Criteria and Goals*

The selection criteria applied to the methods of the present disclosure will vary with the specific goals of the strain improvement program. The present disclosure may be adapted to meet any program goals. For example, in some examples, the program goal may be to maximize single batch yields of reactions with no immediate time limits. In other examples, the program goal may be to rebalance biosynthetic yields to produce a specific product, or to produce a particular ratio of products. In other examples, the program goal may be to modify the chemical structure of a product, such as lengthening the carbon chain of a polymer. In some examples, the program goal may be to improve performance characteristics such as yield, titer, productivity, by-product elimination, tolerance to process excursions, optimal growth temperature and growth rate. In some examples, the program goal is improved host performance as measured by volumetric productivity, specific productivity, yield or titre, of a product of interest produced by a microbe.

In other examples, the program goal may be to optimize synthesis efficiency of a commercial strain in terms of final product yield per quantity of inputs (e.g., total amount of ethanol produced per pound of sucrose). In other examples, the program goal may be to optimize synthesis speed, as measured for example in terms of batch completion rates, or yield rates in continuous culturing systems. In other examples, the program goal may be to increase strain resistance to a particular phage, or otherwise increase strain vigor/robustness under culture conditions.

In some examples, strain improvement projects may be subject to more than one goal. In some examples, the goal of the strain project may hinge on quality, reliability, or overall profitability. In some examples, the present disclosure teaches methods of associated selected mutations or groups of mutations with one or more of the strain properties described above.

Persons having ordinary skill in the art will recognize how to tailor strain selection criteria to meet the particular project goal. For example, selections of a strain's single batch max yield at reaction saturation may be appropriate for identifying strains with high single batch yields. Selection based on consistency in yield across a range of temperatures and conditions may be appropriate for identifying strains with increased robustness and reliability.

In some examples, the selection criteria for the initial high-throughput phase and the tank-based validation will be identical. In other examples, tank-based selection may operate under additional and/or different selection criteria. For example, in some examples, high-throughput strain selection might be based on single batch reaction completion yields, while tank-based selection may be expanded to include selections based on yields for reaction speed.

### *Sequencing*

In some examples, the present disclosure teaches whole-genome sequencing of the organisms described herein. In other examples, the present disclosure also teaches sequencing of plasmids, PCR products, and other oligos as quality controls to the methods of the present disclosure. Sequencing methods for large and small projects are well known to those in the art.

In some examples, any high-throughput technique for sequencing nucleic acids can be used in the methods of the disclosure. In some examples, the present disclosure teaches whole genome sequencing. In other examples, the present disclosure teaches amplicon sequencing ultra deep sequencing to identify genetic variations. In some examples, the present disclosure also teaches novel methods for library preparation, including tagmentation (see WO/2016/073690). DNA sequencing techniques include classic dideoxy sequencing reactions (Sanger method) using labeled terminators or primers and gel separation in slab or capillary; sequencing by synthesis using reversibly terminated labeled nucleotides, pyrosequencing; 454 sequencing; allele specific hybridization to a library of labeled oligonucleotide probes; sequencing by synthesis using allele specific hybridization to a library of labeled clones that is followed by ligation; real time monitoring of the incorporation of labeled nucleotides during a polymerization step; polony sequencing; and SOLiD sequencing.

In one example of the disclosure, high-throughput methods of sequencing are employed that comprise a step of spatially isolating individual molecules on a solid surface where they are sequenced in parallel. Such solid surfaces may include nonporous surfaces (such as in Solexa sequencing, *e.g.* Bentley et al, Nature, 456: 53-59 (2008) or Complete Genomics sequencing, *e.g.* Drmanac et al, Science, 327: 78-81 (2010)), arrays of wells, which may include bead- or particle-bound templates (such as with 454, *e.g.* Margulies et al, Nature, 437: 376-380 (2005) or Ion Torrent sequencing, U.S. patent publication 2010/0137143 or 2010/0304982), micromachined membranes (such as with SMRT sequencing, *e.g.* Eid et al, Science, 323: 133-138 (2009)), or bead arrays (as with SOLiD sequencing or polony sequencing, *e.g.* Kim et al, Science, 316: 1481-1414 (2007)).

In another example, the methods of the present disclosure comprise amplifying the isolated molecules either before or after they are spatially isolated on a solid surface. Prior amplification may comprise emulsion-based amplification, such as emulsion PCR, or rolling circle amplification. Also taught is Solexa-based sequencing where individual template molecules are spatially isolated on a solid surface, after which they are amplified in parallel by bridge PCR to form separate clonal populations, or clusters, and then sequenced, as described in Bentley et al (cited above) and in manufacturer's instructions (*e.g.* TruSeq™ Sample Preparation Kit and Data Sheet, Illumina, Inc., San Diego, Calif., 2010); and further in the following references: U.S. Pat. Nos. 6,090,592; 6,300,070; 7,115,400; and EP0972081B1.

In one example, individual molecules disposed and amplified on a solid surface form clusters in a density of at least $10^5$ clusters per $cm^2$; or in a density of at least $5\times10^5$ per $cm^2$; or in a density of at least $10^6$ clusters per $cm^2$. In one example, sequencing chemistries are employed having relatively high error rates. In such examples, the average quality scores produced by such chemistries are monotonically declining functions of sequence read lengths. In one example, such decline corresponds to 0.5 percent of sequence reads have at least one error in positions 1-75; 1 percent of sequence reads have at least one error in positions 76-100; and 2 percent of sequence reads have at least one error in positions 101-125.

**Computational Analysis and Prediction of Effects of Genome-Wide Genetic Design Criteria**

In some examples, the present disclosure teaches methods of predicting the effects of particular genetic alterations being incorporated into a given host strain. In further examples, the disclosure provides methods for generating proposed genetic alterations that should be incorporated into a given host strain, in order for said host to possess a particular phenotypic trait or strain parameter. In given examples, the disclosure provides predictive models that can be utilized to design novel host strains.

In some examples, the present disclosure teaches methods of analyzing the performance results of each round of screening and methods for generating new proposed genome-wide sequence modifications predicted to enhance strain performance in the following round of screening.

In some examples, the present disclosure teaches that the system generates proposed sequence modifications to host strains based on previous screening results. In some examples, the recommendations of the present system are based on the results from the immediately preceding screening. In other examples, the recommendations of the present system are based on the cumulative results of one or more of the preceding screenings.

In some examples, the recommendations of the present system are based on previously developed HTP genetic design libraries. For example, in some examples, the present system is designed to save results from previous screenings, and apply those results to a different project, in the same or different host organisms.

In other examples, the recommendations of the present system are based on scientific insights. For example, in some examples, the recommendations are based on known properties of genes (from sources such as annotated gene databases and the relevant literature), codon optimization, transcriptional slippage, uORFs, or other hypothesis driven sequence and host optimizations.

In some examples, the proposed sequence modifications to a host strain recommended by the system, or predictive model, are carried out by the utilization of one or more of the disclosed molecular tools sets comprising: (1) Promoter swaps, (2) SNP swaps, (3) Start/Stop codon exchanges, (4) Sequence optimization, (5) Stop swaps, (6) Solubility Tag swaps, (7) Degradation Tag swaps and (8) Epistasis mapping.

The HTP genetic engineering platform described herein is agnostic with respect to any particular microbe or phenotypic trait (*e.g.* production of a particular compound). That is, the platform and methods taught herein can be utilized with *any* host cell to engineer said host cell to have *any* desired phenotypic trait. Furthermore, the lessons learned from a given HTP genetic engineering process used to create one novel host cell, can be applied to any number of other host cells, as a result of the storage, characterization, and analysis of a myriad of process parameters that occurs during the taught methods.

As alluded to in the epistatic mapping section, it is possible to estimate the performance (a.k.a. score) of a hypothetical strain obtained by consolidating a collection of mutations from a HTP genetic design library into a particular background *via* some preferred predictive model. Given such a predictive model, it is possible to score and rank all hypothetical strains accessible to the mutation library *via* combinatorial consolidation. The below section outlines particular models utilized in the present HTP platform.

## Predictive Strain Design

Described herein is an approach for predictive strain design, including: methods of describing genetic changes and strain performance, predicting strain performance based on the composition of changes in the strain, recommending candidate designs with high predicted performance, and filtering predictions to optimize for second-order considerations, *e.g.* similarity to existing strains, epistasis, or confidence in predictions.

## Inputs to Strain Design Model

In one example, for the sake of ease of illustration, input data may comprise two components: (1) sets of genetic changes and (2) relative strain performance. Those skilled in the art will recognize that this model can be readily extended to consider a wide variety of inputs, while keeping in mind the countervailing consideration of overfitting. In addition to genetic changes, some of the input parameters (independent variables) that can be adjusted are cell types (genus, species, strain, phylogenetic characterization, etc.) and process parameters (e.g., environmental conditions, handling equipment, modification techniques, etc.) under which fermentation is conducted with the cells.

The sets of genetic changes can come from the previously discussed collections of genetic perturbations termed HTP genetic design libraries. The relative strain performance can be assessed based upon any given parameter or phenotypic trait of interest (*e.g.* production of a compound, small molecule, or product of interest).

Cell types can be specified in general categories such as prokaryotic and eukaryotic systems, genus, species, strain, tissue cultures (vs. disperse cells), etc. Process parameters that can be adjusted include temperature, pressure, reactor configuration, and medium composition. Examples of reactor configuration include the volume of the reactor, whether the process is a batch or continuous, and, if continuous, the volumetric flow rate, etc. One can also specify the support structure, if any, on which the cells reside. Examples of medium composition include the concentrations of electrolytes, nutrients, waste products, acids, pH, and the like.

### *Sets of Genetic Changes From Selected HTP Genetic Design Libraries to be Utilized in the Initial Linear Regression Model that Subsequently is Used to Create the Predictive Strain Design Model*

An example, a set of entries from a table of genetic changes in *Corynebacterium* is shown below in Table 3. Each row indicates a genetic change in strain 7000051473, as well as metadata about the mechanism of change, *e.g.* promoter swap or SNP swap. aceE, zwf, and pyc are all related to the citric acid cycle.

In this case strain 7000051473 has a total of 7 changes. "Last change" means the change in this strain represents the most recent modification in this strain lineage. Thus, comparing this strain's performance to the performance of its parent represents a data point concerning the performance of the "last change" mutation.

**Table 3** - Strain design entry table for strain 7000051473

| strain | name | library | change | from | to | last_change |
|---|---|---|---|---|---|---|
| 7000051473 | dlc19_42 | proswp | pcg3121 | cg1144 | pcg3121_cg1144 | 1 |
| 7000051473 | dlc19_42 | scswp | acee atg>ttg | ttg | acee_atg | 0 |
| 7000051473 | dlc19_42 | snpswp | dss_033 | NA | na | 0 |
| 7000051473 | dlc19_42 | snpswp | dss_084 | NA | t | 0 |
| 7000051473 | dlc19_42 | snpswp | dss_316 | NA | na | 0 |
| 7000051473 | dlc19_42 | proswp | pcg0007_39 | zwf | pcg0007_39_zwf | 0 |
| 7000051473 | dlc19_42 | proswp | pcg1860 | pyc | pcg1860_pyc | 0 |

### *Built Strain Performance Assessment*

The goal of the taught model is to predict strain performance based on the composition of genetic changes introduced to the strain. To construct a standard for comparison, strain performance is computed relative to a common reference strain, by first calculating the median performance per strain, per assay plate. Relative performance is then computed as the difference in average performance between an engineered strain and the common reference strain within the same plate. Restricting the calculations to within-plate comparisons ensures that the samples under consideration all received the same experimental conditions.

Figure 23 shows the distribution of relative strain performances of *Corynebacterium* for the input data under consideration. A relative performance of zero indicates that the engineered strain performed equally well to the in-plate base or "reference" strain. Of interest is the ability of the predictive model to identify the strains that are likely to perform significantly above zero. Further, and more generally, of interest is whether any given strain outperforms its parent by some criteria. In practice, the criteria can be a product titer meeting or exceeding some threshold above the parent level, though having a statistically significant difference from the parent in the desired direction could also be used instead or in addition. The role of the base or "reference" strain is simply to serve as an added normalization factor for making comparisons within or between plates.

A concept to keep in mind is that of differences between: parent strain and reference strain. The parent strain is the background that was used for a current round of mutagenesis. The reference strain is a control strain run in every plate to facilitate comparisons, especially between plates, and is typically the "base strain" as referenced above. But since the base strain (*e.g.*, the wild-type or industrial strain being used to benchmark overall performance) is not necessarily a "base" in the sense of being a mutagenesis target in a given round of strain improvement, a more descriptive term is "reference strain."

In summary, a base/reference strain is used to benchmark the performance of built strains, generally, while the parent strain is used to benchmark the performance of a specific genetic change in the relevant genetic background.

***Ranking the Performance of Built Strains with Linear Regression***

The goal of the disclosed model is to rank the performance of built strains, by describing relative strain performance, as a function of the composition of genetic changes introduced into the built strains. As discussed throughout the disclosure, the various HTP genetic design libraries provide the repertoire of possible genetic changes (e.g., genetic perturbations/alterations) that are introduced into the engineered strains. Linear regression is the basis for the currently described exemplary predictive model.

The below table (i.e., Table 4) contains example input for regression-based modeling. The strain performances are ranked relative to a common base strain, as a function of the composition of the genetic changes contained in the strain.

Each column heading represents a genetic change, a "1" represents the presence of the change, whereas a "0" represents the absence of a change. "DSS" refers to SNP swaps from a particular library (first 3 columns after relative _perf). The last 3 columns are promoter swaps, where the pcgXXXX denotes the particular promoter, and the last 3 letters represent the gene the promoter is being applied to. The genes are related to central metabolism. The promoters are from *Corynebacterium glutamicum* (hence the "cg" notation). Further information on the utilized promoters can be found in Table 1, listing promoters P1-P8, and the sequence listing of the present application. Further, detailed information on each promoter P1-P8 can be found in U.S. Provisional Application No. 62/264,232, filed on December 07, 2015, and entitled "Promoters from *Corynebacterium glutamicum,".* For ease of reference, in the below table, pcg3121 = P8; pcg0755 = P4; and pcg1860 = P3.

**Table 4** - Summary of genetic changes and their effect on relative performance.

| relative_perf | dss_033 | dss_034 | dss_056 | pcg3121_pgi | pcg0755_zwf | pcg1860_pyc |
|---|---|---|---|---|---|---|
| 0.1358908 | 0 | 0 | 0 | 0 | 0 | 1 |
| -1.8946985 | 1 | 0 | 0 | 1 | 0 | 1 |
| -0.0222045 | 0 | 0 | 0 | 1 | 0 | 0 |
| 0.6342183 | 1 | 0 | 1 | 0 | 0 | 0 |
| -0.0803285 | 1 | 1 | 0 | 0 | 0 | 0 |
| 2.6468117 | 0 | 0 | 0 | 1 | 0 | 0 |

***Linear Regression to Characterize Built Strains***

Linear regression is an attractive method for the described HTP genomic engineering platform, because of the ease of implementation and interpretation. The resulting regression coefficients can be interpreted as the average increase or decrease in relative strain performance attributable to the presence of each genetic change.

For example, as seen in Figure 24, this technique allows us to conclude that changing the pgi promoter to pcg3121 improves relative strain performance by approximately 5 units on average and is thus a potentially highly desirable change, in the absence of any negative epistatic interactions (note: the input is a unit-less normalized value).

The taught method therefore uses linear regression models to describe/characterize and rank built strains, which have various genetic perturbations introduced into their genomes from the various taught libraries.

### *Predictive Design Modeling*

The linear regression model described above, which utilized data from constructed strains, can be used to make performance predictions for strains that haven't yet been built.

The procedure can be summarized as follows: generate *in silico* all possible configurations of genetic changes → use the regression model to predict relative strain performance → order the candidate strain designs by performance. Thus, by utilizing the regression model to predict the performance of as-yet-unbuilt strains, the method allows for the production of higher performing strains, while simultaneously conducting fewer experiments.

### *Generate Configurations*

When constructing a model to predict performance of as-yet-unbuilt strains, the first step is to produce a sequence of design candidates. This is done by fixing the total number of genetic changes in the strain, and then defining all possible combinations of genetic changes. For example, one can set the total number of potential genetic changes/perturbations to 29 (e.g. 29 possible SNPs, or 29 different promoters, or any combination thereof as long as the universe of genetic perturbations is 29) and then decide to design all possible 3-member combinations of the 29 potential genetic changes, which will result in 3,654 candidate strain designs.

To provide context to the aforementioned 3,654 candidate strains, consider that one can calculate the number of non-redundant groupings of size r from n possible members using n! / ((n - r )! * r! ). If r = 3, n = 29 gives 3,654. Thus, if one designs all possible 3-member combinations of 29 potential changes the results is 3,654 candidate strains. The 29 potential genetic changes are present in the x-axis of Figure 25.

### *Predict Performance of New Strain Designs*

Using the linear regression constructed above with the combinatorial configurations as input, one can then predict the expected relative performance of each candidate design. Figure 25 summarizes the composition of changes for the top 100 predicted strain designs for *Corynebacterium.* The x-axis lists the pool of potential genetic changes (29 possible genetic changes), and the y-axis shows the rank order. Black cells indicate the presence of a particular change in the candidate design, while white cells indicate the absence of that change. In this particular example, all of the top 100 designs contain the changes pcg3121_pgi, pcg1860_pyc, dss_339, and pcg0007_39_lysa. Additionally, the top candidate design contains the changes dss_034, dss_009.

Predictive accuracy should increase over time as new observations are used to iteratively retrain and refit the model. Results from a study by the inventors illustrate the methods by which the predictive model can be iteratively retrained and improved. Figure 46 compares model predictions with observed measurement values. The quality of model predictions can be assessed through several methods, including a correlation coefficient indicating the strength of association between the predicted and observed values, or the root-mean-square error, which is a measure of the average model error. Using a chosen metric for model evaluation, the system may define rules for when the model should be retrained.

A couple of unstated assumptions to the above model include: (1) there are no epistatic interactions; and (2) the genetic changes/perturbations utilized to build the predictive model (e.g. from built strain data as illustrated in Figure 24, or whatever data set is used as the reference to construct the model) were all made in the same *Corynebacterium* background, as the proposed combinations of genetic changes (e.g. as illustrated in Figure 25).

### *Filtering for Second-order Features*

The above illustrative example focused on linear regression predictions based on predicted host cell performance. In some examples, the present linear regression methods can also be applied to non-biomolecule factors, such as saturation biomass, resistance, or other measurable host cell features. Thus the methods of the present disclosure also teach in considering other features outside of predicted performance when prioritizing the candidates to build. Assuming there is additional relevant data, nonlinear terms are also included in the regression model.

### *Closeness with Existing Strains*

Predicted strains that are similar to ones that have already been built could result in time and cost savings despite not being a top predicted candidate

***Diversity of Changes***

When constructing the aforementioned models, one cannot be certain that genetic changes will truly be additive (as assumed by linear regression and mentioned as an assumption above) due to the presence of epistatic interactions. Therefore, knowledge of genetic change dissimilarity can be used to increase the likelihood of positive additivity. If one knows, for example, that the changes dss_034 and dss_009 (which are SNP swaps) from the top ranked strain above are on the same metabolic pathway and have similar performance characteristics, then that information could be used to select another top ranking strain with a dissimilar composition of changes. As described in the section above concerning epistasis mapping, the predicted best genetic changes may be filtered to restrict selection to mutations with sufficiently dissimilar response profiles. Alternatively, the linear regression may be a weighted least squares regression using the similarity matrix to weight predictions.

***Diversity of Predicted Performance***

Finally, one may choose to design strains with middling or poor predicted performance, in order to validate and subsequently improve the predictive models.

***Iterative strain design optimization***

As described for the example above, all of the top 100 strain designs contain the changes pcg3121_pgi, pcg1860_pyc, dss_339, and pcg0007_39_lysa. Additionally, the top candidate strain design contains the changes dss_034, dss_009.

In examples, the order placement engine 208 places a factory order to the factory 210 to manufacture microbial strains incorporating the top candidate mutations. In feedback-loop fashion, the results may be analyzed by the analysis equipment 214 to determine which microbes exhibit desired phenotypic properties (314). During the analysis phase, the modified strain cultures are evaluated to determine their performance, i.e., their expression of desired phenotypic properties, including the ability to be produced at industrial scale. For example, the analysis phase uses, among other things, image data of plates to measure microbial colony growth as an indicator of colony health. The analysis equipment 214 is used to correlate genetic changes with phenotypic performance, and save the resulting genotype-phenotype correlation data in libraries, which may be stored in library 206, to inform future microbial production.

In particular, the candidate changes that actually result in sufficiently high measured performance may be added as rows in the database to tables such as Table 4 above. In this manner, the best performing mutations are added to the predictive strain design model in a supervised machine learning fashion.

LIMS iterates the design/build/test/analyze cycle based on the correlations developed from previous factory runs. During a subsequent cycle, the analysis equipment 214 alone, or in conjunction with human operators, may select the best candidates as base strains for input back into input interface 202, using the correlation data to fine tune genetic modifications to achieve better phenotypic performance with finer granularity. In this manner, the laboratory information management system of examples of the disclosure implements a quality improvement feedback loop.

In sum, with reference to the flowchart of Figure 33 the iterative predictive strain design workflow may be described as follows:

- Generate a training set of input and output variables, e.g., genetic changes as inputs and performance features as outputs (3302). Generation may be performed by the analysis equipment 214 based upon previous genetic changes and the corresponding measured performance of the microbial strains incorporating those genetic changes.

- Develop an initial model (e.g., linear regression model) based upon training set (3304). This may be performed by the analysis equipment 214.

- Generate design candidate strains (3306)
  - In one example, the analysis equipment 214 may fix the number of genetic changes to be made to a background strain, in the form of combinations of changes. To represent these changes, the analysis equipment 214 may provide to the interpreter 204 one or more DNA specification expressions representing those combinations of changes. (These genetic changes or the microbial strains incorporating those changes may be referred to as "test inputs.") The interpreter 204 interprets the one or more DNA specifications, and the execution engine 207 executes the DNA specifications to populate the DNA specification with resolved outputs representing the individual candidate design strains for those changes.

- Based upon the model, the analysis equipment 214 predicts expected performance of each candidate design strain (3308).

- The analysis equipment 214 selects a limited number of candidate designs, e.g., 100, with highest predicted performance (3310).
  - As described elsewhere herein with respect to epistasis mapping, the analysis equipment 214 may account for second-order effects such as epistasis, by, e.g., filtering top designs for epistatic effects, or factoring epistasis into the predictive model.

- Build the filtered candidate strains (at the factory 210) based on the factory order generated by the order placement engine 208 (3312).

- The analysis equipment 214 measures the actual performance of the selected strains, selects a limited number of those selected strains based upon their superior actual performance (3314), and adds the design changes and their resulting performance to the predictive model (3316). In the linear regression example, add the sets of design changes and their associated performance as new rows in Table 4.

- The analysis equipment 214 then iterates back to generation of new design candidate strains (3306), and continues iterating until a stop condition is satisfied. The stop condition may comprise, for example, the measured performance of at least one microbial strain satisfying a performance metric, such as yield, growth rate, or titer.

In the example above, the iterative optimization of strain design employs feedback and linear regression to implement machine learning. In general, machine learning may be described as the optimization of performance criteria, e.g., parameters, techniques or other features, in the performance of an informational task (such as classification or regression) using a limited number of examples of labeled data, and then performing the same task on unknown data. In supervised machine learning such as that of the linear regression example above, the machine (e.g., a computing device) learns, for example, by identifying patterns, categories, statistical relationships, or other attributes, exhibited by training data. The result of the learning is then used to predict whether new data will exhibit the same patterns, categories, statistical relationships or other attributes.

Examples of the disclosure may employ other supervised machine learning techniques when training data is available. In the absence of training data, examples may employ unsupervised machine learning. Alternatively, examples may employ semi-supervised machine learning, using a small amount of labeled data and a large amount of unlabeled data. Examples may also employ feature selection to select the subset of the most relevant features to optimize performance of the machine learning model. Depending upon the type of machine learning approach selected, as alternatives or in addition to linear regression, examples may employ for example, logistic regression, neural networks, support vector machines (SVMs), decision trees, hidden Markov models, Bayesian networks, Gram Schmidt, reinforcement-based learning, cluster-based learning including hierarchical clustering, genetic algorithms, and any other suitable learning machines known in the art. In particular, examples may employ logistic regression to provide probabilities of classification (e.g., classification of genes into different functional groups) along with the classifications themselves. See, e.g., Shevade, A simple and efficient algorithm for gene selection using sparse logistic regression, Bioinformatics, Vol. 19, No. 17 2003, pp. 2246-2253, Leng, et al., Classification using functional data analysis for temporal gene expression data, Bioinformatics, Vol. 22, No. 1, Oxford University Press (2006), pp. 68-76.

Examples may employ graphics processing unit (GPU) accelerated architectures that have found increasing popularity in performing machine learning tasks, particularly in the form known as deep neural networks (DNN). Examples of the disclosure may employ GPU-based machine learning, such as that described in GPU-Based Deep Learning Inference: A Performance and Power Analysis, NVidia Whitepaper, November 2015, Dahl, et al., Multi-task Neural Networks for QSAR Predictions, Dept. of Computer Science, Univ. of Toronto, June 2014 (arXiv:1406.1231 [stat.ML]). Machine learning techniques applicable to examples of the disclosure may also be found in, among other references, Libbrecht, et al., Machine learning applications in genetics and genomics, Nature Reviews: Genetics, Vol. 16, June 2015, Kashyap, et al., Big Data Analytics in Bioinformatics: A Machine Learning Perspective, Journal of Latex Class Files, Vol. 13, No. 9, Sept. 2014, Prompramote, et al., Machine Learning in Bioinformatics, Chapter 5 of Bioinformatics Technologies, pp. 117-153, Springer Berlin Heidelberg 2005.

Iterative Predictive Strain Design: Example

The following provides an example application of the iterative predictive strain design workflow outlined above.

An initial set of training inputs and output variables was prepared. This set comprised 1864 unique engineered strains with defined genetic composition. Each strain contained between 5 and 15 engineered changes. A total of 336 unique genetic changes were present in the training.

An initial predictive computer model was developed. The implementation used a generalized linear model (Kernel Ridge Regression with 4th order polynomial kernel). The implementation models two distinct phenotypes (*yield* and *productivity*). These phenotypes were combined as weighted sum to obtain a single score for ranking, as shown below. Various model parameters, e.g. regularization factor, were tuned via k-fold cross validation over the designated training data.

The implementation does not incorporate any explicit analysis of interaction effects as described in the Epistasis Mapping section above. However, as those skilled in the art would understand, the implemented generalized linear model may capture interaction effects implicitly through the second, third and fourth order terms of the kernel.

The model was trained against the training set. The fitted model has an $R^2$ value (*coefficient of determination*) of 0.52 with respect to *yield* and an $R^2$ value of 0.67 with respect to *productivity.* Figure 46 demonstrates a significant quality fitting of the *yield* model to the training data.

Candidate strains were generated. This example includes a serial build constraint associated with the introduction of new genetic changes to a parent strain (in this example, only one new mutation was engineered into a strain at a time). Here, candidates are not considered simply as a function of the desired number of changes. Instead, the analysis equipment 214 selected, as a starting point, a collection of previously designed strains known to have high performance metrics ("seed strains"). The analysis equipment 214 individually applied genetic changes to each of the seed strains. The introduced genetic changes did not include those already present in the seed strain. For various technical, biological or other reasons, certain mutations were explicitly required, e.g., opca_4, or explicitly excluded, e.g., dss_422. Using 166 available seed strains and the 336 changes characterized by the model, 6239 novel candidate strains were designed.

Based upon the model, the analysis equipment 214 predicted the performance of candidate strain designs. The analysis equipment 214 ranked candidates from "best" to "worst" based on predicted performance with respect to two phenotypes of interest (*yield* and *productivity*). Specifically, the analysis equipment 214 used a weighted sum to score a candidate strain:

$$\text{Score} = 0.8 * \text{yield} / \max(\text{yields}) + 0.2 * \text{prod} / \max(\text{prods}),$$

where yield represents predicted yield for the candidate strain,
max(yields) represents the maximum yield over all candidate strains,
prod represents productivity for the candidate strain, and
max(prods) represents the maximum yield over all candidate strains.

The analysis equipment 214 generated a final set of recommendations from the ranked list of candidates by imposing both capacity constraints and operational constraints. In this example, the capacity limit was set at 48 computer-generated candidate design strains. Due to operational constraints, in this example only one seed strain was used per column of a 96-well plate. This means that after a seed strain was chosen, up to 8 changes to that strain could be built, but only 6 seed strains could be chosen in any given week.

The trained model (described above) was used to predict the expected performance (for yield and productivity) of each candidate strain. The analysis equipment 214 ranked the candidate strains using the scoring function given above. Capacity and operational constraints were applied to yield a filtered set of 48 candidate strains. This set of filtered candidate strains is depicted in Figure 47.

Filtered candidate strains were built (at the factory 210) based on a factory order generated by the order placement engine 208 (3312). The order was based upon DNA specifications corresponding to the candidate strains.

In practice, the build process has an expected failure rate whereby a random set of strains is not built. For this build cycle, roughly 20% of the candidate strains failed build, resulting in 37 built strains.

The analysis equipment 214 was used to measure the actual *yield* and *productivity* performance of the selected strains. The analysis equipment 214 evaluated the model and recommended strains based on three criteria: model accuracy; improvement in strain performance; and equivalence (or improvement) to human expert-generated designs.

The *yield* and *productivity* phenotypes were measured for recommended strains and compared to the values predicted by the model. As shown in Figure 48, the model demonstrates useful predictive utility. In particular, the predicted *yield* values for the recommended strains have a Pearson-r correlation coefficient of 0.59 with the corresponding observations.

Next, the analysis equipment 214 computed percentage performance change from the parent strain for each of the recommended strains. This data is shown in Figure 49 (in light gray). The inventors found that many of the predicted strains in fact exhibited the expected performance gains with respect to their immediate parents. In particular, the best predicted strain showed a 6% improvement in yield with respect to its immediate parent.

In parallel with the model-based strain design process described above, a collection of 48 strains was independently designed by a human expert. Of these strains, 37 were successfully built and tested. This data demonstrated that the model-based strain designs performed comparably to strains designed by human experts. These experts are highly-skilled (e.g., Ph.D.-level) scientists employed or otherwise engaged by the assignee of the present disclosure, and familiar with the examples of this disclosure. To compare the two methods, the inventors first inspected the performance distributions of each group (Figure 50). In this experiment, the mean yield of model-based strains showed a 1% increase with respect to human expert generated designs.

The inventors then compared human expert-designed and computer-model-designed strains grouped by background, *i.e.,* new strains with the same parent (Figure 51). Again, the inventors found that computer-generated designs perform comparably to, and in some cases better than, the human expert-generated designs, and further tend to produce less variability. Finally, the inventors compared the percentage change with respect to the parent strains of the human expert and model-designed strains (Figure 49). Again, these populations showed comparable gains.

See Table 4.1 for tabulated summary statistics.

**Table 4.1.** Measured performance statistics for strains designed by the predictive model and by a human expert reference.

| | | Yield [AU] | Yield change from parent [%] | Productivity [AU] | Productivity change from parent [%] |
|---|---|---|---|---|---|
| design method | | | | | |
| computer model | count | 37 | 37 | 37 | 37 |
| | mean | 1.058068108 | 0.3578340 | 0.737928919 | -2.5428848 |
| | std | 0.017811031 | 1.8293665 | 0.083619804 | 9.6743873 |
| | min | 1.015310000 | -4.5346677 | 0.572780000 | -23.3626353 |
| | median | 1.058710000 | 0.005007939 | 0.766870000 | -1.1824159 |
| | max | 1.093510000 | 6.0097309 | 0.872790000 | 26.6124119 |
| Human expert | count | 37 | 37 | 37 | 37 |
| | mean | 1.038804595 | -0.0005237 | 0.748320811 | -1.6126436 |
| | std | 0.032053625 | 1.9227716 | 0.120527468 | 9.8530758 |
| | min | 0.964910000 | -3.1043233 | 0.535980000 | -21.4589256 |
| | median | 1.045530000 | 0.0449168 | 0.760300000 | -1.9241048 |
| | max | 1.094790000 | 7.8487174 | 0.984110000 | 21.7335193 |

At the conclusion of each round of the prediction → build → test cycle, the inventors were interested in evaluating the quality of the model predictions and iteratively incorporating new data into the previous model. For the former-model evaluation-the inventors focused on measuring predictive accuracy by comparing model predictions with experimental measurements. Predictive accuracy can be assessed through several methods, including a correlation coefficient indicating the strength of association between the predicted and observed values, or the root-mean-square error, which is a measure of the average model error.

Over many rounds of experimentation, model predictions may drift, and new genetic changes may be added to the training inputs to improve predictive accuracy. For this example, design changes and their resulting performance were added to the predictive model (3316).

**Genomic design and engineering as a service**

In examples of the disclosure, the LIMS system software 3210 of Figure 32 may be implemented in a cloud computing system 3202 of Figure 32, to enable multiple users to design and build microbial strains according to examples of the present disclosure. Figure 32 illustrates a cloud computing environment 3204 according to examples of the present disclosure. Client computers 3206, such as those illustrated in Figure 32, access the LIMS system via a network 3208, such as the Internet. In examples, the LIMS system application software 3210 resides in the cloud computing system 3202. The LIMS system may employ one or more computing systems using one or more processors, of the type illustrated in Figure 32. The cloud computing system itself includes a network interface 3212 to interface the LIMS system applications 3210 to the client computers 3206 via the network 3208. The network interface 3212 may include an application programming interface (API) to enable client applications at the client computers 3206 to access the LIMS system software 3210. In particular, through the API, client computers 3206 may access components of the LIMS system 200, including without limitation the software running the input interface 202, the interpreter 204, the execution engine 207, the order placement engine 208, the factory 210, as well as test equipment 212 and analysis equipment 214. A software as a service (SaaS) software module 3214 offers the LIMS system software 3210 as a service to the client computers 3206. A cloud management module 3216 manages access to the LIMS system 3210 by the client computers 3206. The cloud management module 3216 may enable a cloud architecture that employs multitenant applications, virtualization or other architectures known in the art to serve multiple users.

## Genomic Automation

Automation of the methods of the present disclosure enables high-throughput phenotypic screening and identification of target products from multiple test strain variants simultaneously.

The aforementioned genomic engineering predictive modeling platform is premised upon the fact that hundreds and thousands of mutant strains are constructed in a high-throughput fashion. The robotic and computer systems described below are the structural mechanisms by which such a high-throughput process can be carried out.

In some examples, the present disclosure teaches methods of improving host cell productivities, or rehabilitating industrial strains. As part of this process, the present disclosure teaches methods of assembling DNA, building new strains, screening cultures in plates, and screening cultures in models for tank fermentation. In some examples, the present disclosure teaches that one or more of the aforementioned methods of creating and testing new host strains is aided by automated robotics.

In some examples, the present disclosure teaches a high-throughput strain engineering platform as depicted in Figure 6A-B or Figure 26.

### *HTP Robotic Systems*

In some examples, the automated methods of the disclosure comprise a robotic system. The systems outlined herein are generally directed to the use of 96- or 384-well microtiter plates, but as will be appreciated by those in the art, any number of different plates or configurations may be used. In addition, any or all of the steps outlined herein may be automated; thus, for example, the systems may be completely or partially automated.

In some examples, the automated systems of the present disclosure comprise one or more work modules. For example, in some examples, the automated system of the present disclosure comprises a DNA synthesis module, a vector cloning module, a strain transformation module, a screening module, and a sequencing module (see Figure 7).

As will be appreciated by those in the art, an automated system can include a wide variety of components, including, but not limited to: liquid handlers; one or more robotic arms; plate handlers for the positioning of microplates; plate sealers, plate piercers, automated lid handlers to remove and replace lids for wells on non-cross contamination plates; disposable tip assemblies for sample distribution with disposable tips; washable tip assemblies for sample distribution; 96 well loading blocks; integrated thermal cyclers; cooled reagent racks; microtiter plate pipette positions (optionally cooled); stacking towers for plates and tips; magnetic bead processing stations; filtrations systems; plate shakers; barcode readers and applicators; and computer systems.

In some examples, the robotic systems of the present disclosure include automated liquid and particle handling enabling high-throughput pipetting to perform all the steps in the process of gene targeting and recombination applications. This includes liquid and particle manipulations such as aspiration, dispensing, mixing, diluting, washing, accurate volumetric transfers; retrieving and discarding of pipette tips; and repetitive pipetting of identical volumes for multiple deliveries from a single sample aspiration. These manipulations are cross-contamination-free liquid, particle, cell, and organism transfers. The instruments perform automated replication of microplate samples to filters, membranes, and/or daughter plates, high-density transfers, full-plate serial dilutions, and high capacity operation.

In some examples, the customized automated liquid handling system of the disclosure is a TECAN machine (e.g. a customized TECAN Freedom Evo).

In some examples, the automated systems of the present disclosure are compatible with platforms for multi-well plates, deep-well plates, square well plates, reagent troughs, test tubes, mini tubes, microfuge tubes, cryovials, filters, micro array chips, optic fibers, beads, agarose and acrylamide gels, and other solid-phase matrices or platforms are accommodated on an upgradeable modular deck. In some examples, the automated systems of the present disclosure contain at least one modular deck for multi-position work surfaces for placing source and output samples, reagents, sample and reagent dilution, assay plates, sample and reagent reservoirs, pipette tips, and an active tip-washing station.

In some examples, the automated systems of the present disclosure include high-throughput electroporation systems. In some examples, the high-throughput electroporation systems are capable of transforming cells in 96 or 384- well plates. In some examples, the high-throughput electroporation systems include VWR® High-throughput Electroporation Systems, BTX™, Bio-Rad® Gene Pulser MXcell™ or other multi-well electroporation system.

In some examples, the integrated thermal cycler and/or thermal regulators are used for stabilizing the temperature of heat exchangers such as controlled blocks or platforms to provide accurate temperature control of incubating samples from 0°C to 100°C.

In some examples, the automated systems of the present disclosure are compatible with interchangeable machine-heads (single or multi-channel) with single or multiple magnetic probes, affinity probes, replicators or pipetters, capable of robotically manipulating liquid, particles, cells, and multi-cellular organisms. Multi-well or multi-tube magnetic separators and filtration stations manipulate liquid, particles, cells, and organisms in single or multiple sample formats.

In some examples, the automated systems of the present disclosure are compatible with camera vision and/or spectrometer systems. Thus, in some examples, the automated systems of the present disclosure are capable of detecting and logging color and absorption changes in ongoing cellular cultures.

In some examples, the automated system of the present disclosure is designed to be flexible and adaptable with multiple hardware add-ons to allow the system to carry out multiple applications. The software program modules allow creation, modification, and running of methods. The system's diagnostic modules allow setup, instrument alignment, and motor operations. The customized tools, labware, and liquid and particle transfer patterns allow different applications to be programmed and performed. The database allows method and parameter storage. Robotic and computer interfaces allow communication between instruments.

Thus, in some examples, the present disclosure teaches a high-throughput strain engineering platform, as depicted in Figure 26.

Persons having skill in the art will recognize the various robotic platforms capable of carrying out the HTP engineering methods of the present disclosure. Table 5 below provides a non-exclusive list of scientific equipment capable of carrying out each step of the HTP engineering steps of the present disclosure as described in Figure 26.

**Table 5** - Non-exclusive list of Scientific Equipment Compatible with the HTP engineering methods of the present disclosure.

| | Equipment Type | Operation(s) performed | Compatible Equipment Make/Model/Configuration |
|---|---|---|---|
| **Acquire and build DNA pieces** | liquid handlers | Hitpicking (combining by transferring) primers/templates for PCR amplification of DNA parts | Hamilton Microlab STAR, Labcyte Echo 550, Tecan EVO 200, Beckman Coulter Biomek FX, or equivalents |
| | Thermal cyclers | PCR amplification of DNA parts | Inheco Cycler, ABI 2720, ABI Proflex 384, ABI Veriti, or equivalents |
| **QC DNA parts** | Fragment analyzers (capillary electrophoresis) | gel electrophoresis to confirm PCR products of appropriate size | Agilent Bioanalyzer, AATI Fragment Analyzer, or equivalents |
| | Sequencer (sanger: Beckman) | Verifying sequence of parts/templates | Beckman Ceq-8000, Beckman GenomeLab™, or equivalents |
| | NGS (next generation sequencing) instrument | Verifying sequence of parts/templates | Illumina MiSeq series sequences, illumina Hi-Seq, Ion torrent, pac bio or other equivalents |

| | Equipment Type | Operation(s) performed | Compatible Equipment Make/Model/Configuration |
|---|---|---|---|
| | nanodrop/plate reader | assessing concentration of DNA samples | Molecular Devices SpectraMax M5, Tecan M1000, or equivalents. |
| Generate DNA assembly | liquid handlers | Hitpicking (combining by transferring) DNA parts for assembly along with cloning vector, addition of reagents for assembly reaction/process | Hamilton Microlab STAR, Labcyte Echo 550, Tecan EVO 200, Beckman Coulter Biomek FX, or equivalents |
| QC DNA assembly | Colony pickers | for inoculating colonies in liquid media | Scirobotics Pickolo, Molecular Devices QPix 420 |
| | liquid handlers | Hitpicking primers/templates, diluting samples | Hamilton Microlab STAR, Labcyte Echo 550, Tecan EVO 200, Beckman Coulter Biomek FX, or equivalents |
| | Fragment analyzers (capillary electrophoresis) | gel electrophoresis to confirm assembled products of appropriate size | Agilent Bioanalyzer, AATI Fragment Analyzer |
| | Sequencer (sanger: Beckman) | Verifying sequence of assembled plasmids | ABI3730 Thermo Fisher, Beckman Ceq-8000, Beckman GenomeLab™, or equivalents |

| | Equipment Type | Operation(s) performed | Compatible Equipment Make/Model/Configuration |
|---|---|---|---|
| | NGS (next generation sequencing) instrument | Verifying sequence of assembled plasmids | Illumina MiSeq series sequences, illumina Hi-Seq, Ion torrent, pac bio or other equivalents |
| **Prepare base strain and DNA assembly** | centrifuge | spinning / pelleting cells | Beckman Avanti floor centrifuge,<br>Hettich Centrifuge |
| **Transform DNA into base strain** | Electroporators | electroporative transformation of cells | BTX Gemini X2, BIO-RAD MicroPulser Electroporator |
| | Ballistic transformation | ballistic transformation of cells | BIO-RAD PDS1000 |
| | Incubators, thermal cyclers | for chemical transformation/heat shock | Inheco Cycler, ABI 2720, ABI Proflex 384, ABI Veriti, or equivalents |
| | Liquid handlers | for combining DNA, cells, buffer | Hamilton Microlab STAR, Labcyte Echo 550, Tecan EVO 200, Beckman Coulter Biomek FX, or equivalents |

| | Equipment Type | Operation(s) performed | Compatible Equipment Make/Model/Configuration |
|---|---|---|---|
| **Integrate DNA into genome of base strain** | Colony pickers | for inoculating colonies in liquid media | Scirobotics Pickolo, Molecular Devices QPix 420 |
| | Liquid handlers | For transferring cells onto Agar, transferring from culture plates to different culture plates (inoculation into other selective media) | Hamilton Microlab STAR, Labcyte Echo 550, Tecan EVO 200, Beckman Coulter Biomek FX, or equivalents |
| | Platform shaker-incubators | incubation with shaking of microtiter plate cultures | Kuhner Shaker ISF4-X, Infors-ht Multitron Pro |
| **QC transformed strain** | Colony pickers | for inoculating colonies in liquid media | Scirobotics Pickolo, Molecular Devices QPix 420 |
| | liquid handlers | Hitpicking primers/templates, diluting samples | Hamilton Microlab STAR, Labcyte Echo 550, Tecan EVO 200, Beckman Coulter Biomek FX, or equivalents |
| | Thermal cyclers | cPCR verification of strains | Inheco Cycler, ABI 2720, ABI Proflex 384, ABI Veriti, or equivalents |

| | Equipment Type | Operation(s) performed | Compatible Equipment Make/Model/Configuration |
|---|---|---|---|
| | Fragment analyzers (capillary electrophoresis) | gel electrophoresis to confirm cPCR products of appropriate size | Infors-ht Multitron Pro, Kuhner Shaker ISF4-X |
| | Sequencer (sanger: Beckman) | Sequence verification of introduced modification | Beckman Ceq-8000, Beckman GenomeLab™, or equivalents |
| | NGS (next generation sequencing) instrument | Sequence verification of introduced modification | Illumina MiSeq series sequences, illumina Hi-Seq, Ion torrent, pac bio or other equivalents |
| **Select and consolidate QC'd strains into test plate** | Liquid handlers | For transferring from culture plates to different culture plates (inoculation into production media) | Hamilton Microlab STAR, Labcyte Echo 550, Tecan EVO 200, Beckman Coulter Biomek FX, or equivalents |
| | Colony pickers | for inoculating colonies in liquid media | Scirobotics Pickolo, Molecular Devices QPix 420 |
| | Platform shaker-incubators | incubation with shaking of microtiter plate cultures | Kuhner Shaker ISF4-X, Infors-ht Multitron Pro |

| | Equipment Type | Operation(s) performed | Compatible Equipment Make/Model/Configuration |
|---|---|---|---|
| Culture strains in seed plates | Liquid handlers | For transferring from culture plates to different culture plates (inoculation into production media) | Hamilton Microlab STAR, Labcyte Echo 550, Tecan EVO 200, Beckman Coulter Biomek FX, or equivalents |
| | Platform shaker-incubators | incubation with shaking of microtiter plate cultures | Kuhner Shaker ISF4-X, Infors-ht Multitron Pro |
| | liquid dispensers | Dispense liquid culture media into microtiter plates | Well mate (Thermo), Benchcel2R (velocity 11), plateloc (velocity 11) |
| | microplate labeler | apply barcoders to plates | Microplate labeler (a2+ cab - agilent), benchcell 6R (velocity11) |
| Generate product from strain | Liquid handlers | For transferring from culture plates to different culture plates (inoculation into production media) | Hamilton Microlab STAR, Labcyte Echo 550, Tecan EVO 200, Beckman Coulter Biomek FX, or equivalents |
| | Platform shaker-incubators | incubation with shaking of microtiter plate cultures | Kuhner Shaker ISF4-X, Infors-ht Multitron Pro |

| | Equipment Type | Operation(s) performed | Compatible Equipment Make/Model/Configuration |
|---|---|---|---|
| | liquid dispensers | Dispense liquid culture media into multiple microtiter plates and seal plates | well mate (Thermo), Benchcel2R (velocity 11), plateloc (velocity 11) |
| | microplate labeler | Apply barcodes to plates | microplate labeler (a2+ cab - agilent), benchcell 6R (velocity11) |
| Evaluate performance | Liquid handlers | For processing culture broth for downstream analytical | Hamilton Microlab STAR, Labcyte Echo 550, Tecan EVO 200, Beckman Coulter Biomek FX, or equivalents |
| | UHPLC, HPLC | quantitative analysis of precursor and target compounds | Agilent 1290 Series UHPLC and 1200 Series HPLC with UV and RI detectors, or equivalent; also any LC/MS |
| | LC/MS | highly specific analysis of precursor and target compounds as well as side and degradation products | Agilent 6490 QQQ and 6550 QTOF coupled to 1290 Series UHPLC |
| | Spectrophotometer | Quantification of different compounds using spectrophotometer based assays | Tecan M1000, spectramax M5, Genesys 10S |

| | Equipment Type | Operation(s) performed | Compatible Equipment Make/Model/Configuration |
|---|---|---|---|
| **Culture strains in flasks** | Fermenters: | incubation with shaking | Sartorius, DASGIPs (Eppendorf), BIO-FLOs (Sartorius-stedim). Applikon |
| | Platform shakers | | innova 4900, or any equivalent |
| **Generate product from strain** | Fermenters: DASGIPs (Eppendorf), BIO-FLOs (Sartorius-stedim) | | |
| **Evaluate performance** | Liquid handlers | For transferring from culture plates to different culture plates (inoculation into production media) | Hamilton Microlab STAR, Labcyte Echo 550, Tecan EVO 200, Beckman Coulter Biomek FX, or equivalents |
| | UHPLC, HPLC | quantitative analysis of precursor and target compounds | Agilent 1290 Series UHPLC and 1200 Series HPLC with UV and RI detectors, or equivalent; also any LC/MS |
| | LC/MS | highly specific analysis of precursor and target compounds as well as side and degradation products | Agilent 6490 QQQ and 6550 QTOF coupled to 1290 Series UHPLC |

| | Equipment Type | Operation(s) performed | Compatible Equipment Make/Model/Configuration |
|---|---|---|---|
| | Flow cytometer | Characterize strain performance (measure viability) | BD Accuri, Millipore Guava |
| | Spectrophotometer | Characterize strain performance (measure biomass) | Tecan M1000, Spectramax M5, or other equivalents |

***Computer System Hardware***

Figure 34 illustrates an example of a computer system 800 that may be used to execute program code stored in a non-transitory computer readable medium (e.g., memory) in accordance with examples of the disclosure. The computer system includes an input/output subsystem 802, which may be used to interface with human users and/or other computer systems depending upon the application. The I/O subsystem 802 may include, e.g., a keyboard, mouse, graphical user interface, touchscreen, or other interfaces for input, and, e.g., an LED or other flat screen display, or other interfaces for output, including application program interfaces (APIs). Other elements of examples of the disclosure, such as the components of the LIMS system, may be implemented with a computer system like that of computer system 800.

Program code may be stored in non-transitory media such as persistent storage in secondary memory 810 or main memory 808 or both. Main memory 808 may include volatile memory such as random access memory (RAM) or non-volatile memory such as read only memory (ROM), as well as different levels of cache memory for faster access to instructions and data. Secondary memory may include persistent storage such as solid state drives, hard disk drives or optical disks. One or more processors 804 reads program code from one or more non-transitory media and executes the code to enable the computer system to accomplish the methods performed by the examples herein. Those skilled in the art will understand that the processor(s) may ingest source code, and interpret or compile the source code into machine code that is understandable at the hardware gate level of the processor(s) 804. The processor(s) 804 may include graphics processing units (GPUs) for handling computationally intensive tasks. Particularly in machine learning, one or more CPUs 804 may offload the processing of large quantities of data to one or more GPUs 804.

The processor(s) 804 may communicate with external networks via one or more communications interfaces 807, such as a network interface card, WiFi transceiver, etc. A bus 805 communicatively couples the I/O subsystem 802, the processor(s) 804, peripheral devices 806, communications interfaces 807, memory 808, and persistent storage 810. Examples of the disclosure are not limited to this representative architecture. Alternative examples may employ different arrangements and types of components, e.g., separate buses for input-output components and memory subsystems.

Those skilled in the art will understand that some or all of the elements of examples of the disclosure, and their accompanying operations, may be implemented wholly or partially by one or more computer systems including one or more processors and one or more memory systems like those of computer system 800. In particular, the elements of the LIMS system 200 and any robotics and other automated systems or devices described herein may be computer-implemented. Some elements and functionality may be implemented locally and others may be implemented in a distributed fashion over a network through different servers, e.g., in client-server fashion, for example. In particular, server-side operations may be made available to multiple clients in a software as a service (SaaS) fashion, as shown in Figure 32.

The term component in this context refers broadly to software, hardware, or firmware (or any combination thereof) component. Components are typically functional components that can generate useful data or other output using specified input(s). A component may or may not be self-contained. An application program (also called an "application") may include one or more components, or a component can include one or more application programs.

Some examples include some, all, or none of the components along with other modules or application components. Still yet, various examples may incorporate two or more of these components into a single module and/or associate a portion of the functionality of one or more of these components with a different component.

The term "memory" can be any device or mechanism used for storing information. In accordance with some examples of the present disclosure, memory is intended to encompass any type of, but is not limited to: volatile memory, nonvolatile memory, and dynamic memory. For example, memory can be random access memory, memory storage devices, optical memory devices, magnetic media, floppy disks, magnetic tapes, hard drives, SIMMs, SDRAM, DIMMs, RDRAM, DDR RAM, SODIMMS, erasable programmable read-only memories (EPROMs), electrically erasable programmable read-only memories (EEPROMs), compact disks, DVDs, and/or the like. In accordance with some examples, memory may include one or more disk drives, flash drives, databases, local cache memories, processor cache memories, relational databases, flat databases, servers, cloud based platforms, and/or the like. In addition, those of ordinary skill in the art will appreciate many additional devices and techniques for storing information can be used as memory.

Memory may be used to store instructions for running one or more applications or modules on a processor. For example, memory could be used in some examples to house all or some of the instructions needed to execute the functionality of one or more of the modules and/or applications disclosed in this application.

**HTP Microbial Strain Engineering Based Upon Genetic Design Predictions: An Example Workflow**

In some examples, the present disclosure teaches the directed engineering of new host organisms based on the recommendations of the computational analysis systems of the present disclosure.

In some examples, the present disclosure is compatible with all genetic design and cloning methods. That is, in some examples, the present disclosure teaches the use of traditional cloning techniques such as polymerase chain reaction, restriction enzyme digestions, ligation, homologous recombination, RT PCR, and others generally known in the art and are disclosed in for example: Sambrook et al. (2001) Molecular Cloning: A Laboratory Manual (3rd ed., Cold Spring Harbor Laboratory Press, Plainview, New York).

In some examples, the cloned sequences can include possibilities from any of the HTP genetic design libraries taught herein, for example: promoters from a promoter swap library, SNPs from a SNP swap library, start or stop codons from a start/stop codon exchange library, terminators from a STOP swap library, protein solubility tags from a SOLUBILITY TAG swap library, protein degradation tags from a DEGRADATION TAG swap library or sequence optimizations from a sequence optimization library.

Further, the exact sequence combinations that should be included in a particular construct can be informed by the epistatic mapping function.

In other examples, the cloned sequences can also include sequences based on rational design (hypothesis-driven) and/or sequences based on other sources, such as scientific publications.

In some examples, the present disclosure teaches methods of directed engineering, including the steps of i) generating custom-made SNP-specific DNA, ii) assembling SNP-specific plasmids, iii) transforming target host cells with SNP-specific DNA, and iv) looping out any selection markers (*See* Figure 2).

Figure 6A depicts the general workflow of the strain engineering methods of the present disclosure, including acquiring and assembling DNA, assembling vectors, transforming host cells and removing selection markers.

***Build Specific DNA Oligonucleotides***

In some examples, the present disclosure teaches inserting and/or replacing and/or altering and/or deleting a DNA segment of the host cell organism. In some examples, the methods taught herein involve building an oligonucleotide of interest *(i.e.* a target DNA segment), that will be incorporated into the genome of a host organism. In some examples, the target DNA segments of the present disclosure can be obtained *via* any method known in the art, including: copying or cutting from a known template, mutation, or DNA synthesis. In some examples, the present disclosure is compatible with commercially available gene synthesis products for producing target DNA sequences (*e.g.*, GeneArt™, GeneMaker™, GenScript™, Anagen™, Blue Heron™, Entelechon™, GeNOsys, Inc., or Qiagen™).

In some examples, the target DNA segment is designed to incorporate a SNP into a selected DNA region of the host organism (*e.g.*, adding a beneficial SNP). In other examples, the DNA segment is designed to remove a SNP from the DNA of the host organisms (*e.g.*, removing a detrimental or neutral SNP).

In some examples, the oligonucleotides used in the inventive methods can be synthesized using any of the methods of enzymatic or chemical synthesis known in the art. The oligonucleotides may be synthesized on solid supports such as controlled pore glass (CPG), polystyrene beads, or membranes composed of thermoplastic polymers that may contain CPG. Oligonucleotides can also be synthesized on arrays, on a parallel microscale using microfluidics (Tian et al., Mol. BioSyst., 5, 714-722 (2009)), or known technologies that offer combinations of both (see Jacobsen et al., U.S. Pat. App. No. 2011/0172127).

Synthesis on arrays or through microfluidics offers an advantage over conventional solid support synthesis by reducing costs through lower reagent use. The scale required for gene synthesis is low, so the scale of oligonucleotide product synthesized from arrays or through microfluidics is acceptable. However, the synthesized oligonucleotides are of lesser quality than when using solid support synthesis (See Tian infra.; see also Staehler et al., U.S. Pat. App. No. 2010/0216648).

A great number of advances have been achieved in the traditional four-step phosphoramidite chemistry since it was first described in the 1980s (see for example, Sierzchala, et al. J. Am. Chem. Soc., 125, 13427-13441 (2003) using peroxy anion deprotection; Hayakawa et al., U.S. Pat. No. 6,040,439 for alternative protecting groups; Azhayev et al, Tetrahedron 57, 4977-4986 (2001) for universal supports; Kozlov et al., Nucleosides, Nucleotides, and Nucleic Acids, 24 (5-7), 1037-1041 (2005) for improved synthesis of longer oligonucleotides through the use of large-pore CPG; and Damha et al., NAR, 18, 3813-3821 (1990) for improved derivatization).

Regardless of the type of synthesis, the resulting oligonucleotides may then form the smaller building blocks for longer oligonucleotides. In some examples, smaller oligonucleotides can be joined together using protocols known in the art, such as polymerase chain assembly (PCA), ligase chain reaction (LCR), and thermodynamically balanced inside-out synthesis (TBIO) (see Czar et al. Trends in Biotechnology, 27, 63-71 (2009)). In PCA, oligonucleotides spanning the entire length of the desired longer product are annealed and extended in multiple cycles (typically about 55 cycles) to eventually achieve full-length product. LCR uses ligase enzyme to join two oligonucleotides that are both annealed to a third oligonucleotide. TBIO synthesis starts at the center of the desired product and is progressively extended in both directions by using overlapping oligonucleotides that are homologous to the forward strand at the 5' end of the gene and against the reverse strand at the 3' end of the gene.

Another method of synthesizing a larger double stranded DNA fragment is to combine smaller oligonucleotides through top-strand PCR (TSP). In this method, a plurality of oligonucleotides spans the entire length of a desired product and contain overlapping regions to the adjacent oligonucleotide(s). Amplification can be performed with universal forward and reverse primers, and through multiple cycles of amplification a full-length double stranded DNA product is formed. This product can then undergo optional error correction and further amplification that results in the desired double stranded DNA fragment end product.

In one method of TSP, the set of smaller oligonucleotides that will be combined to form the full-length desired product are between 40-200 bases long and overlap each other by at least about 15-20 bases. For practical purposes, the overlap region should be at a minimum long enough to ensure specific annealing of oligonucleotides and have a high enough melting temperature ($T_m$) to anneal at the reaction temperature employed. The overlap can extend to the point where a given oligonucleotide is completely overlapped by adjacent oligonucleotides. The amount of overlap does not seem to have any effect on the quality of the final product. The first and last oligonucleotide building block in the assembly should contain binding sites for forward and reverse amplification primers. In one example, the terminal end sequence of the first and last oligonucleotide contain the same sequence of complementarity to allow for the use of universal primers.

***Assembling/Cloning Custom Plasmids***

In some examples, the present disclosure teaches methods for constructing vectors capable of inserting desired target DNA sections (e.g. containing a particular SNP) into the genome of host organisms. In some examples, the present disclosure teaches methods of cloning vectors comprising the target DNA, homology arms, and at least one selection marker *(see* Figure 3).

In some examples, the present disclosure is compatible with any vector suited for transformation into the host organism. In some examples, the present disclosure teaches use of shuttle vectors compatible with a host cell. In one example, a shuttle vector for use in the methods provided herein is a shuttle vector compatible with an *E. coli* and/or *Corynebacterium* host cell. Shuttle vectors for use in the methods provided herein can comprise markers for selection and/or counter-selection as described herein. The markers can be any markers known in the art and/or provided herein. The shuttle vectors can further comprise any regulatory sequence(s) and/or sequences useful in the assembly of said shuttle vectors as known in the art. The shuttle vectors can further comprise any origins of replication that may be needed for propagation in a host cell as provided herein such as, for example, *E. coli* or C. *glutamicum.* The regulatory sequence can be any regulatory sequence known in the art or provided herein such as, for example, a promoter, start, stop, insulator, signal, secretion and/or termination sequence used by the genetic machinery of the host cell. In certain instances, the target DNA can be inserted into vectors, constructs or plasmids obtainable from any repository or catalogue product, such as a commercial vector *(see e.g.,* DNA2.0 custom or GATEWAY® vectors). In certain instances, the target DNA can be inserted into vectors, constructs or plasmids obtainable from any repository or catalogue product, such as a commercial vector *(see e.g.,* DNA2.0 custom or GATEWAY® vectors).

In some examples, the assembly/cloning methods of the present disclosure may employ at least one of the following assembly strategies: **i)** type II conventional cloning, **ii)** type II S-mediated or "Golden Gate" cloning *(see, e.g.,* Engler, C., R. Kandzia, and S. Marillonnet. 2008 "A one pot, one step, precision cloning method with high-throughput capability". PLos One 3:e3647; Kotera, I., and T. Nagai. 2008 "A high-throughput and single-tube recombination of crude PCR products using a DNA polymerase inhibitor and type IIS restriction enzyme." J Biotechnol 137:1-7.; Weber, E., R.

Gruetzner, S. Werner, C. Engler, and S. Marillonnet. 2011 Assembly of Designer TAL Effectors by Golden Gate Cloning. PloS One 6:e1722), **iii)** GATEWAY® recombination, **iv)** TOPO® cloning, exonuclease-mediated assembly (Aslanidis and de Jong 1990. "Ligation-independent cloning of PCR products (LIC-PCR)." Nucleic Acids Research, Vol. 18, No. 20 6069), **v)** homologous recombination, **vi)** non-homologous end joining, vii) Gibson assembly (Gibson et al., 2009 "Enzymatic assembly of DNA molecules up to several hundred kilobases" Nature Methods 6, 343-345) or a combination thereof. Modular type IIS based assembly strategies are disclosed in PCT Publication WO 2011/154147.

In some examples, the present disclosure teaches cloning vectors with at least one selection marker. Various selection marker genes are known in the art often encoding antibiotic resistance function for selection in prokaryotic (*e.g*., against ampicillin, kanamycin, tetracycline, chloramphenicol, zeocin, spectinomycin/streptomycin) or eukaryotic cells (*e.g.* geneticin, neomycin, hygromycin, puromycin, blasticidin, zeocin) under selective pressure. Other marker systems allow for screening and identification of wanted or unwanted cells such as the well-known blue/white screening system used in bacteria to select positive clones in the presence of X-gal or fluorescent reporters such as green or red fluorescent proteins expressed in successfully transduced host cells. Another class of selection markers most of which are only functional in prokaryotic systems relates to counter selectable marker genes often also referred to as "death genes" which express toxic gene products that kill producer cells. Examples of such genes include sacB, rpsL(strA), tetAR, pheS, thyA, gata-1, or ccdB, the function of which is described in (Reyrat et al. 1998 "Counterselectable Markers: Untapped Tools for Bacterial Genetics and Pathogenesis." Infect Immun. 66(9): 4011-4017).

DNA Vector Assembly, Amplification, and Genome Editing

In some examples, the present disclosure teaches HTP genomic engineering steps specific for *E. coli.* In some examples, the present disclosure thus teaches methods of constructing and amplifying constructs in *E. coli,* as well as methods of engineering *E. coli.*

In some examples, the DNA vectors of the present disclosure comrpsie **i)** a conditional replication ori (R6K), **ii)** an antibiotic resistance gene, **iii)** one or more counter-selection gene(s) (e.g. sacb and/or PheS) and **iv)** a replication ori for *S. cerevisiae.*

In some examples, the present disclosure teaches methods of assembling DNA constructs in auxotrophic *S. cerevisiae.* Thus in, some examples, the vectors of the present disclosure comprise a replication origin for *S. cerevisiae.* This permits the vector to replicate in *S. cerevisiae* during assembly.

In some examples, the present disclosure teaches methods of propagating assembled DNA occurs in *E. coli* containing pir protein. Thus, in some examples, the vectors of the present disclosure comprise an R6K origin of replication. In some examples, the R6K replication origin is conditional on the presence of the pir protein. That is, in some examples, the presently disclosed vectors comprising the R6K replication origin will only be amplified in host cells comprising the pir gene. This allows researchers to amplify the vectors of the present disclosure during the vector construction and amplification steps, while also preventing extra chromosomal expression of the vectors during the host cell engineering steps.

In some examples, the vectors of the present disclosure comprise a PheS gene. *Escherichia coli* phenylalanyl-tRNA synthetase (PheS) can be useful as a counterselection marker, since its A294G variant misincorporates 4-chloro-phenylalanine (4CP) into cellular proteins during translation, thereby causing cell death. In some examples, the PheS gene is designed to temporarily incorporate into the genome of the host cell. In some examples, the present disclosure teaches counterselection methods comprising growing the host cells in minimal media with 4CP. Cells which still comprise the vector will incorporate 4CP into proteins and die. Cells which have looped out the PheS sequence survive.

In some examples, the present disclosure teaches methods of constructing, assembling and integrating vectors into host cells. In some examples, the present disclosure teaches that the homology arms (homL and homR) are amplified by PCR. In some examples, the desired genetic change (black bar between homL and homR) are present in the reverse primer of homL and the forward primer of homR (see Figure 3). In some examples, the forward primer of homL and the reverse primer of homR have sequence homology to the backbone plasmid. Further illustrations of the loop-in and loop-out process are depicted in Figure 45.

In some examples, the vectors of the present disclosure comprise one or more insulator sequences. The insulator sequence can be any insulator sequence known in the art. In one example, the insulator nucleic acid sequence is the insulator1 sequence (SEQ ID NO. 218), insulator 2 sequence (SEQ ID NO. 219) or both the insulator 1 and 2 sequences provided herein. In one example, the vectors of the present disclosure comprise insulator sequences flanking homology arms (homL and homR). In one example, the vectors of the present disclosure comprise insulator sequences flanking homology arms (homL and homR) and terminator sequence(s). The insulator sequences can be generated to be free of restriction endonuclease sequences.

In some examples, the vectors of the present disclosure comprise a combination of elements provided herein. In some cases, a vector for use in the methods provided herein can comprise an R6K origin of replication, a SacB gene, a PheS gene as a counterselection marker and a URA3 yeast auxotrophic marker such as, for example, vector 1 *(see* Figure 55), which has the nucleic acid sequence of SEQ ID NO. 214. In some cases, a vector for use in the methods provided herein can be an altered version of vector 1, such as, for example, vector 2 (see Figure 56), which has the nucleic acid sequence of SEQ ID NO. 215. In addition to the elements previously recited for vector 1, vector 2 can further comprise the elements found in Table 15. Additional vectors for use in the methods provided herein include vector 3 (nucleic acid SEQ ID NO. 216; Figure 57) and vector 4 (nucleic acid SEQ ID NO. 217; Figure 58). Both vectors 3 and 4 were built off of the vector 2 background. However, in vector 3, the promoter sequence of sacB was replaced with a promoter containing the P2-MCD2 promoter *(see* Mutalik et al, Nat Methods. 2013 Apr;10(4):354-60) and a codon optimized version of the native pheS gene containing the T251A/A294G mutations *(see* Miyazaki, K. Biotechniques. 2015 Feb 1;58(2):86-8), while vector 4 comprises the vector 3 background with the URA3 selection marker being replaced with the TRP1 marker.

**Table 15.** Select Sequence Elements of Vector 2

| Element name | Part sequence |
|---|---|
| Insulator1 | TATTCACACGCAATCAACAGGCAGGATAATCGCTGGTAAGGTCAGTGCTTTCTTCAGGTAGTAGAGATACAATAGTTCCCAACGATAGGTGGCAGATTTCACTTTACAGACCGACTGGTTCAGAAGCGTAGATAATAGCCCGTGTTTTCCAATAAGGGATAGTGTAGGTAAGTCAACTCCTCCGTCAGAGCCAACCGTTT (SEQ ID NO. 218) |
| Insulator2 | GCACTAGGACTTGCCGCGGATACTGCCCCATTACATGAATTGCAGCCTCAGGGACGTCAGTAGATCATGGAGGTAGGGCATATGTCCTCTGTTGTTAAAATGTGAGTTCTCAACGAAGCACGAATCGGTCAGAACCTACACTAAGGAGATTTGGTAGGTGCACGGTTTCTGTCGCATAGACCAGTTCATTTCAGATGTCT (SEQ ID NO. 219) |
| T1 | GGCATCAAATAAAACGAAAGGCTCAGTCGAAAGACTGGGCCTTTCGTTTTATCTGTTGTTTGTCGGTGAACGCTCTCCTGAGTAGGACAAATCCGCCGCCCTAGA (SEQ ID NO. 220) |
| B0015 | CCAGGCATCAAATAAAACGAAAGGCTCAGTCGAAAGACTGGGCCTTTCGTTTTATCTGTTGTTTGTCGGTGAACGCTCTCTACTAGAGTCACACTGGCTCACCTTCGGGTGGGCCTTTCTGCGTTTATA (SEQ ID NO. 221) |
| SacB promoter | TTGACAATTAATCATCCGGCTCGTAATTTATGTGGATCTTAATCATGCTAAGGAGGTTTTCTAATG (SEQ ID NO. 222) |

(continued)

| Element name | Part sequence |
|---|---|
| PheS promoter & sequence | GGCGGTGTTGACATAAATACCACTGGCGGTGATACTGAGCACATCAGC AGGTCACACAGGAAAGTACTAGATGTCGCATCTTGCAGAATTAGTAGC TTCAGCGAAGGCCGCGATTTCTCAGGCGAGTGACGTCGCAGCACTGGA TAATGTACGTGTTGAGTACCTGGGAAAGAAGGGACACCTTACTCTTCA AATGACAACCCTGCGCGAACTGCCGCCGGAGGAACGCCCCGCAGCAG GAGCGGTAATCAATGAGGCAAAGGAGCAAGTACAACAGGCACTGAAC GCCCGTAAGGCTGAGTTGGAATCCGCCGCATTAAACGCGCGCCTTGCT GCGGAAACCATTGATGTCTCGCTGCCCGGGCGCCGCATTGAGAATGGA GGCTTACACCCAGTGACTCGTACCATCGACCGTATCGAATCTTTCTTTG GCGAACTTGGCTTCACTGTGGCAACTGGACCGGAGATTGAGGACGACT ACCACAATTTCGATGCCTTGAACATTCCCGGTCATCATCCTGCACGCGC CGATCATGATACATTCTGGTTTGATACCACCCGTTTGCTTCGTACCCAG ACAAGCGGTGTCCAAATCCGTACGATGAAGGCTCAGCAACCACCGATC CGTATCATTGCTCCAGGGCGCGTGTACCGTAACGATTATGACCAGACA CATACACCGATGTTTCACCAAATGGAAGGGTTGATTGTGGATACGAAT ATCTCTTTCACGAATCTGAAGGGCACCTTACATGATTTCTTACGCAACT TTTTCGAGGAGGACCTTCAAATTCGCTTTCGTCCATCGTACTTCCCTTTT GCAGAACCTTCGGCTGAAGTGGATGTAATGGGGAAAAACGGTAAGTG GCTGGAGGTTTTAGGTTGCGGGATGGTTCATCCAAATGTGCTTCGCAAC GTCGGCATCGACCCCGAAGTCTACAGTGGATTCGGATTCGGGATGGGA ATGGAACGTCTGACTATGCTTCGTTACGGCGTAACGGATTTGCGCTCCT TTTTTGAGAACGATCTTCGTTTTCTGAAGCAATTCAAATAA (SEQ ID NO. 223) |

A general workflow associated with the DNA assembly according to one example of the present disclosure is shown in Figure 35.

In some examples, DNA assembly among the backbone and the inserts (homL and homR) is performed in yeast via yeast gap repair recombination. In some examples, auxotrophic marker (TRP or URA) is present in the backbone plasmid for the selection of assembled DNA in minimal media. The assembled plasmids are then extracted out of yeast culture.

In some examples, the extracted plasmids are next transformed into an *E. coli* strain containing pir gene to propagate the desired plasmids for the following transformation of the strain of interest. The transformants are selected for resistance to the given antibiotics. The transformants will be picked for sequencing to select the correctly assembled plasmid of interest.

In some embdoiments correctly assembled plasmids are transformed into host cells for genome engineering via electroporation. Since the target host cells do not contain pir protein, the colonies formed on the antibiotic selective media are expected to have integration of the plasmid at the desired locus in the genome. The correct integration of the plasmid can be verified by PCR with the primer outside the homL and homR, respectively, and the primer binding inside the plasmid.

In some examples, the present disclosure teaches loop-out methods of removing the backbone of the plasmid from the genome of the host. Thus, in some examples, the present disclosure teaches that subsequent selection on counter-selective media (sucrose) and/or 4CP can be used to isolate the clones that do not contain the backbone part of the plasmid. Thus, in some examples, isolated host cells comprising the correct integrant are inoculated in LB media and the culture is plated on LB agar plate containing sucrose and 4-p-chloro-phenylalanine (LB+suc+4CP). Due to the sensitivity of cells expressing sacB gene to sucrose, and the sensitivity of cells expressing PheS to 4CP, the colonies formed onto LB+suc+4CP agar plate are expected to have either the mutant or the wild-type of the gene of interest. PCR amplification of the target nucleotide(s) and sequencing of the PCR product allows us to isolate the new clones with the desired genome modification.

In some examples, the resulting clones are sequenced to find the clones with the desired nucleotide change(s).

In some examples, all the process above can be performed with the liquid handler.

### *Transformation of Host Cells*

In some examples, the vectors of the present disclosure may be introduced into the host cells using any of a variety of techniques, including transformation, transfection, transduction, viral infection, gene guns, or Ti-mediated gene transfer (see Christie, P.J., and Gordon, J.E., 2014 "The Agrobacterium Ti Plasmids" Microbiol SPectr. 2014; 2(6); 10.1128). Particular methods include calcium phosphate transfection, DEAE-Dextran mediated transfection, lipofection, or electroporation (Davis, L., Dibner, M., Battey, I., 1986 "Basic Methods in Molecular Biology"). Other methods of transformation include for example, lithium acetate transformation and electroporation See, e.g., Gietz et al., Nucleic Acids Res. 27:69-74 (1992); Ito et al., J. Bacterol. 153:163-168 (1983); and Becker and Guarente, Methods in Enzymology 194:182-187 (1991). In some examples, transformed host cells are referred to as recombinant host strains.

In some examples, the present disclosure teaches high-throughput transformation of cells using the 96-well plate robotics platform and liquid handling machines of the present disclosure.

In some examples, the present disclosure teaches screening transformed cells with one or more selection markers as described above. In one such example, cells transformed with a vector comprising a kanamycin resistance marker (KanR) are plated on media containing effective amounts of the kanamycin antibiotic. Colony forming units visible on kanamycin-laced media are presumed to have incorporated the vector cassette into their genome. Insertion of the desired sequences can be confirmed via PCR, restriction enzyme analysis, and/or sequencing of the relevant insertion site.

### *Looping Out of Selected Sequences*

In some examples, the present disclosure teaches methods of looping out selected regions of DNA from the host organisms. The looping out method can be as described in Nakashima et al. 2014 "Bacterial Cellular Engineering by Genome Editing and Gene Silencing." Int. J. Mol. Sci. 15(2), 2773-2793. In some examples, the present disclosure teaches looping out selection markers from positive transformants. Looping out deletion techniques are known in the art, and are described in (Tear et al. 2014 "Excision of Unstable Artificial Gene-Specific inverted Repeats Mediates Scar-Free Gene Deletions in Escherichia coli." Appl. Biochem. Biotech. 175:1858-1867). The looping out methods used in the methods provided herein can be performed using single-crossover homologous recombination or double-crossover homologous recombination. In one example, looping out of selected regions as described herein can entail using single-crossover homologous recombination as described herein.

First, loop out vectors are inserted into selected target regions within the genome of the host organism (e.g., via homologous recombination, CRISPR, lambda red-mediated recombineering or other gene editing technique). In one example, single-crossover homologous recombination is used between a circular plasmid or vector and the host cell genome in order to loop-in the circular plasmid or vector such as depicted in Figure 3. The inserted vector can be designed with a sequence which is a direct repeat of an existing or introduced nearby host sequence, such that the direct repeats flank the region of DNA slated for looping and deletion. Once inserted, cells containing the loop out plasmid or vector can be counter selected for deletion of the selection region *(e.g.,* see Figure 4; lack of resistance to the selection gene). Further illustrations of the loop-in and loop-out process are depicted in Figure 45.

Persons having skill in the art will recognize that the description of the loopout procedure represents but one illustrative method for deleting unwanted regions from a genome. Indeed the methods of the present disclosure are compatible with any method for genome deletions, including but not limited to gene editing via lambda red, CRISPR, TALENS, FOK, or other endonucleases. Persons skilled in the art will also recognize the ability to replace unwanted regions of the genome via homologous recombination techniques.

### *Lambda RED Mediated Gene Editing*

As provided herein, gene editing as described herein can be performed using Lambda Red-mediated homologous recombination as described by Datsenko and Wanner, PNAS USA 97:6640-6645 (2000).

The lambda red system is derived from the lambda red bacteriophage and its use as a genetic engineering tool can be called recombineering - short for homologous recombination-mediated genetic engineering. It can be used to make an assortment of modifications: insertion and deletion of selectable and non-selectable sequences, point mutations or other small base pair changes, and the addition of protein tags. It also has the flexibility to modify the *E. coli* chromosome, plasmid DNA or BAC DNA. To use the lambda red recombineering system to modify target DNA, a linear donor DNA substrate (either dsDNA or ssDNA - see below) can be electroporated into *E. coli* expressing the lambda red enzymes. These enzymes then catalyze the homologous recombination of the substrate with the target DNA sequence. This means cloning occurs in vivo, as compared to restriction enzyme cloning where the genetic changes occur in a test tube. The donor DNA substrate only requires ~50 nucleotides of homology to the target site for recombination.

The lambda red recombineering system has three components: 1) Exo, 2) Beta, and 3) Gam. All three are required for recombineering with a dsDNA substrate; however, only Beta is required when generating a modification with an ssDNA substrate.

Gam: Gam prevents both the endogenous RecBCD and SbcCD nucleases from digesting linear DNA introduced into a E. coli host cell.

Exo: Exo is a 5'→3' dsDNA-dependent exonuclease. Exo can degrade linear dsDNA starting from the 5' end and generate 2 possible products: 1) a partially dsDNA duplex with single-stranded 3' overhangs or 2) if the dsDNA was short enough, a ssDNA whose entire complementary strand was degraded.

Beta: Beta can protect the ssDNA created by Exo and promote its annealing to a complementary ssDNA target in the cell. Only Beta expression is required for recombineering with an ssDNA oligo substrate.

For use herein, a lambda red recombineering method can entail designing and generating substrate DNA; expressing lambda red recombination genes; transforming (e.g., electroporating) substrate DNA; growing transformants; and selecting and confirming recombinant clones.

**Substrate DNA Design and Generation**

Whether a linear dsDNA or ssDNA substrate is used can depend on the goal of the experiment. dsDNA substrate may be best for insertions or deletions greater than approximately 20 nucleotides, while ssDNA substrate may be best for point mutations or changes of only a few base pairs.

*dsDNA Substrate*

dsDNA inserts can be made by PCR using primers that amplify the DNA sequence of interest and flank it with 50 base pairs of homology to the targeted insert site. The primers can be ~70 nucleotides long (20 nucleotides that anneal to the DNA sequence of interest and 50 nucleotides of homology to regions flanking the target site). The dsDNA inserts can include: large insertions or deletions, including selectable DNA fragments, such as antibiotic resistance genes, as well as non-selectable DNA fragments, such as gene replacements and tags.

*ssDNA Substrate*

ssDNA substrates can be synthetic oligonucleotides or short PCR products. Either way, the substrate should be ~70-100 nucleotides long with the desired alteration(s) located in the center of the sequence. Since lambda red has a higher recombination frequency when the lagging strand of DNA is targeted, it's best to determine the direction of replication through a target region of interest and design an oligo that is complementary to the lagging strand. In some cases, oligos that target both strands are designed. One of the two oligos will recombine with a higher efficiency than the other which can aid in identifying the lagging strand.

ssDNA substrate can be more efficient than dsDNA with a recombination frequency between 0.1% to 1%, and can be increased to as high as 25-50% by designing oligos that avoid activating the methyl -directed mismatch repair (MMR) system. MMR's job is to correct DNA mismatches that occur during DNA replication. Activation of MMR can be avoided by: 1) using a strain of bacteria that has key MMR proteins knocked out or 2) specially design ssDNA oligos to avoid MMR: 1) *E. coli* with inactivated MMR: Using *E. coli* with inactive MMR is definitely the easier of the two options, but these cells are prone to mutations and can have more unintended changes to their genomes. 2) Designing ssDNA oligos that avoid MMR activation: In one example, a C/C mismatch at or within 6 base pairs of the edit site is introduced. In another example, the desired change is flanked with 4-5 silent changes in the wobble codons, i.e. make changes to the third base pair of the adjacent 4-5 codons that alter the nucleotide sequence but not the amino acid sequence of the translated protein. These changes can be 5' or 3' of the desired change.

**Expression of Lambda Red Recombination Genes**

The lambda red recombineering system can be expressed in a host cell by: 1) From a bacteria with integrated defective prophage 2) from a plasmid, 3) from mini-λ or 4) from the lambda red phage itself. Controlling expression of Red proteins is critical for minimizing the toxic effects of Gam expression and to limit spontaneous mutations that occur when Red is constitutively expressed. Which recombination system you use depends on what type of DNA you want to edit; however, BAC DNA can be modified with any of the below described approaches.

Bacterial Strain with Integrated Defective Prophage:

A number of *E. coli* strains exist that stably express lambda red recombineering genes due to integration of a defective lambda red phage. One such strain is DY380, which is derived from the DH10B *E. coli* strain. Several other bacterial strains commonly used for recombineering can be found in Thomason et al (Recombineering: Genetic Engineering in Bacteria Using Homologous Recombination. Current Protocols in Molecular Biology. 106:V:1.16:1.16.1-1.16.39) and Sharan et al (Recombineering: A Homologous Recombination-Based Method of Genetic Engineering. Nature protocols. 2009;4(2):206-223).

In some of these strains, expression of exo, beta and gam is tightly regulated by the endogenous phage promoter pL and repressor CI. For recombineering purposes, a temperature sensitive version of the repressor gene, CI857, is used. This mutant repressor prevents expression of the recombination genes at low temperatures (30-34°C). Shifting bacteria to 42°C for 15 minutes quickly inactivates the repressor and allows for expression of the recombination genes. After this, lowering the temperature allows the repressor to re-nature and again repress expression of exo, beta and gam. A major advantage of using this method for lambda red expression is that it doesn't require antibiotic selection to maintain expression of the recombineering system. This setup can be also be used to modify chromosomal genes. After the initial editing event, the defective prophage can be removed from the chromosome of the host *E. coli* by a second lambda red recombination event. Alternatively, if the modified allele is selectable, it can be transferred to a different genetic background via P1 transduction.

Plasmid:

Expressing lambda red genes from a plasmid allows for a mobile recombineering system, but tight regulation of expression is required for a successful experiment. Promoters commonly used to control expression of Red include the IPTG-inducible lac promoter, the arabinose-inducible pBAD promoter and the endogenous phage pL promoter. Plasmids that also express the repressors associated with these promoters (lacI, araC, cI857) can be used in some cases in order to limit leaky expression of the Red system. Using a plasmid to express the lambda recombineering system can be used for editing bacterial chromosomal DNA because it is easy to remove the recombineering system once recombinant clones are generated. A simple way to do this is to express lambda red genes from a plasmid with a heat sensitive origin of replication. Once the recombineering system is no longer needed, bacteria can be "cured" by growing them at 42°C.

Mini-λ:

A hybrid between using a plasmid and stable integration of a defective prophage is to use mini-λ, a defective non-replicating, circular piece of phage DNA, that when introduced into bacteria, integrates into the genome. Mini-λ uses the endogenous red promoter pL and the cI857 repressor to regulate expression. An antibiotic can be used to select for positive clones but, because mini-λ stably integrates, drug selection is not required for maintenance. A temperature shift to 42°C not only allows for activation of the red genes needed for recombineering, but also leads to expression of the int and xis genes which are responsible for excision of mini-λ from the host's chromosome. After this, mini-λ can readily be purified from bacteria just like a plasmid.

Phage:

Another option for expressing the Red system can be to use a lambda red phage, λTetR, that carries the tetracycline resistance gene and the lambda red repressor cI857. Once introduced, the prophage is stable and no longer requires drug selection. One drawback to this approach is that it requires the generation of bacteriophage, which is not a common molecular biology technique. However, an advantage of this method is that you can stably integrate the Red system into yoa strain of interest and P1 transduction could be used to move the modification into a different background, if needed. This approach is best suited for modifying plasmids or BACs because it results in stable integration of the phage into the E. coli genome.

**Selection and Confirmation of Recombinant Clones**

If a antibiotic resistance gene has been inserted, recombinants can first be selected via antibiotic resistance, but all clones should be further tested to confirm the presence of the desired modification. Colony PCR can be used to screen for positive clones in most cases, and restriction enzyme digest can be used to screen plasmids for the appropriate mutations. Point mutations and other subtle changes can be confirmed by sequencing, which can also be used for confirmation for all clones, regardless of what type of DNA is being targeted for modification: the *E. coli* chromosome, a plasmid, or a BAC.

***CRISPR Mediated Gene Editing***

In one example provided herein, the genome of a host cell can be modified by CRISPR. An exemplary example for utilizing the CRISPR/Cas9 system for gene editing in E. coli can be found in Examples 18 and 19.

The CRISPR/Cas system is a prokaryotic immune system that confers resistance to foreign genetic elements such as those present within plasmids and phages and that provides a form of acquired immunity. CRISPR stands for Clustered Regularly Interspaced Short Palindromic Repeat, and cas stands for CRISPR-associated system, and refers to the small cas genes associated with the CRISPR complex.

CRISPR-Cas systems are most broadly characterized as either Class 1 or Class 2 systems. The main distinguishing feature between these two systems is the nature of the Cas-effector module. Class 1 systems require assembly of multiple Cas proteins in a complex (referred to as a "Cascade complex") to mediate interference, while Class 2 systems use a large single Cas enzyme to mediate interference. Each of the Class 1 and Class 2 systems are further divided into multiple CRISPR-Cas types based on the presence of a specific Cas protein. For example, the Class 1 system is divided into the following three types: Type I systems, which contain the Cas3 protein; Type III systems, which contain the Cas10 protein; and the putative Type IV systems, which contain the Csf1 protein, a Cas8-like protein. Class 2 systems are generally less common than Class 1 systems and are further divided into the following three types: Type II systems, which contain the Cas9 protein; Type V systems, which contain Cas12a protein (previously known as Cpf1, and referred to as Cpf1 herein), Cas12b (previously known as C2c1), Cas12c (previously known as C2c3), Cas12d (previously known as CasY), and Cas12e (previously known as CasX); and Type VI systems, which contain Cas13a (previously known as C2c2), Cas13b, and Cas13c. Pyzocha et al., ACS Chemical Biology, Vol. 13 (2), pgs. 347-356. In one example, the CRISPR-Cas system for use in the methods provided herein is a Class 2 system. In one example, the CRISPR-Cas system for use in the methods provided herein is a Type II, Type V or Type VI Class 2 system. In one example, the CRISPR-Cas system for use in the methods provided herein is selected from Cas9, Cas12a, Cas12b, Cas12c, Cas12d, Cas12e, Cas13a, Cas13b, Cas13c or homologs, orthologs or paralogs thereof

CRISPR systems used in methods disclosed herein comprise a Cas effector module comprising one or more nucleic acid guided CRISPR-associated (Cas) nucleases, referred to herein as Cas effector proteins. In some examples, the Cas proteins can comprise one or multiple nuclease domains. A Cas effector protein can target single stranded or double stranded nucleic acid molecules (e.g. DNA or RNA nucleic acids) and can generate double strand or single strand breaks. In some examples, the Cas effector proteins are wild-type or naturally occurring Cas proteins. In some examples, the Cas effector proteins are mutant Cas proteins, wherein one or more mutations, insertions, or deletions are made in a WT or naturally occurring Cas protein *(e.g.,* a parental Cas protein) to produce a Cas protein with one or more altered characteristics compared to the parental Cas protein.

In some instances, the Cas protein is a wild-type (WT) nuclease. Non-limiting examples of suitable Cas proteins for use in the present disclosure include C2cl, C2c2, C2c3, Cas1, Cas1B, Cas2, Cas3, Cas4, Cas5, Cas6, Cas7, Cas8, Cas9 (also known as Csn1 and Csx12), Cas10, Cpf1, Csy1, Csy2, Csy3, Cse1, Cse2, Cscl, Csc2, Csa5, Csn2, Csm1, Csm2, Csm3, Csm4, Csm5, Csm6, Cmr1, Cmr3, Cmr4, Cmr5, Cmr6, Csb1, Csb2, Csb3, Csx17, Csx14, Csx100, Csx16, CsaX, Csx3, Csxl, Csx15, Csf1, Csf2, Csf3, Csf4, MAD1-20, SmCsm1, homologes thereof, orthologes thereof, variants thereof, mutants thereof, or modified versions thereof. Suitable nucleic acid guided nucleases (*e.g*., Cas 9) can be from an organism from a genus, which includes but is not limited to: *Thiomicrospira, Succinivibrio, Candidatus, Porphyromonas, Acidomonococcus, Prevotella, Smithella, Moraxella, Synergistes, Francisella, Leptospira, Catenibacterium, Kandleria, Clostridium, Dorea, Coprococcus, Enterococcus, Fructobacillus, Weissella, Pediococcus, Corynebacter, Sutterella, Legionella, Treponema, Roseburia, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma, Alicyclobacillus, Brevibacilus, Bacillus, Bacteroidetes, Brevibacilus, Carnobacterium, Clostridiaridium, Clostridium, Desulfonatronum, Desulfovibrio, Helcococcus, Leptotrichia, Listeria, Methanomethyophilus, Methylobacterium, Opitutaceae, Paludibacter, Rhodobacter, Sphaerochaeta, Tuberibacillus,* and *Campylobacter.* Species of organism of such a genus can be as otherwise herein discussed.

Suitable nucleic acid guided nucleases (e.g., Cas9) can be from an organism from a phylum, which includes but is not limited to: Firmicute, Actinobacteria, Bacteroidetes, Proteobacteria, Spirochates, and Tenericutes. Suitable nucleic acid guided nucleases can be from an organism from a class, which includes but is not limited to: Erysipelotrichia, Clostridia, Bacilli, Actinobacteria, Bacteroidetes, Flavobacteria, Alphaproteobacteria, Betaproteobacteria, Gammaproteobacteria, Deltaproteobacteria, Epsilonproteobacteria, Spirochaetes, and Mollicutes. Suitable nucleic acid guided nucleases can be from an organism from an order, which includes but is not limited to: Clostridiales, Lactobacillales, Actinomycetales, Bacteroidales, Flavobacteriales, Rhizobiales, Rhodospirillales, Burkholderiales, Neisseriales, Legionellales, Nautiliales, Campylobacterales, Spirochaetales, Mycoplasmatales, and Thiotrichales. Suitable nucleic acid guided nucleases can be from an organism from within a family, which includes but is not limited to: Lachnospiraceae, Enterococcaceae, Leuconostocaceae, Lactobacillaceae, Streptococcaceae, Peptostreptococcaceae, Staphylococcaceae, Eubacteriaceae, Corynebacterineae, Bacteroidaceae, Flavobacterium, Cryomoorphaceae, Rhodobiaceae, Rhodospirillaceae, Acetobacteraceae, Sutterellaceae, Neisseriaceae, Legionellaceae, Nautiliaceae, Campylobacteraceae, Spirochaetaceae, Mycoplasmataceae, and Francisellaceae.

Other nucleic acid guided nucleases (e.g., Cas9) suitable for use in the methods, systems, and compositions of the present disclosure include those derived from an organism such as, but not limited to: *Thiomicrospira sp.* XS5, *Eubacterium rectale, Succinivibrio dextrinosolvens, Candidatus Methanoplasma termitum, Candidatus Methanomethylophilus alvus, Porphyromonas crevioricanis, Flavobacterium branchiophilum, Acidomonococcus sp., Lachnospiraceae bacterium* COE1, *Prevotella brevis* ATCC 19188, *Smithella sp.* SCADC, *Moraxella bovoculi, Synergistes jonesii, Bacteroidetes* oral taxon 274, *Francisella tularensis, Leptospira inadai serovar* Lyme str. 10, *Acidomonococcus* sp. crystal structure (5B43) *S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii; Francisella tularensis l, Prevotella albensis, Lachnospiraceae bacterium* MC2017 1, *Butyrivibrio proteoclasticus, Peregrinibacteria bacterium* GW2011_GWA2_33_10, *Parcubacteria bacterium* GW2011_GWC2_ 44_17, *Smithella sp.* SCADC, *Microgenomates, Acidaminococcus sp.* BV3L6, *Lachnospiraceae bacterium* MA2020, *Candidatus Methanoplasma termitum, Eubacterium eligens, Moraxella bovoculi* 237, *Leptospira inadai, Lachnospiraceae bacterium* ND2006, *Porphyromonas crevioricanis* 3, *Prevotella disiens, Porphyromonas macacae, Catenibacterium sp.* CAG:290, *Kandleria vitulina,* Clostridiales bacterium KA00274, *Lachnospiraceae bacterium* 3-2, *Dorea longicatena, Coprococcus catus* GD/7, *Enterococcus columbae* DSM 7374, *Fructobacillus sp.* EFB-N1, *Weissella halotolerans, Pediococcus acidilactici, Lactobacillus curvatus, Streptococcus pyogenes, Lactobacillus versmoldensis,* and *Filifactor alocis* ATCC 35896. *See,* U.S. Pat. Nos. 8,697,359; 8,771,945; 8,795,965; 8,865,406; 8,871,445; 8,889,356; 8,895,308; 8,906,616; 8,932,814; 8,945,839; 8,993,233; 8,999,641; 9,822,372; 9,840,713; U.S. Pat. App. No. 13/842,859 (US 2014/0068797 A1); 9,260,723; 9,023,649; 9,834,791; 9,637,739; U.S. Pat. App. No. 14/683,443 (US 2015/0240261 A1); U.S. Pat. App. No. 14/743,764 (US 2015/0291961 A1); 9,790,490; 9,688,972; 9,580,701; 9,745,562; 9,816,081; 9,677,090; 9,738,687; U.S. App. No. 15/632,222 (US 2017/0369879 A1); U.S. App. No. 15/631,989; U.S. App. No. 15/632,001; and U.S. Pat. No. 9,896,696.

In some examples, a Cas effector protein comprises one or more of the following activities:

a nickase activity, i.e., the ability to cleave a single strand of a nucleic acid molecule;
a double stranded nuclease activity, i.e., the ability to cleave both strands of a double stranded nucleic acid and create a double stranded break;
an endonuclease activity;
an exonuclease activity; and/or
a helicase activity, i.e., the ability to unwind the helical structure of a double stranded nucleic acid.

In examples of the disclosure the term "guide nucleic acid" refers to a polynucleotide comprising 1) a guide sequence capable of hybridizing to a target sequence (referred to herein as a "targeting segment") and 2) a scaffold sequence capable of interacting with (either alone or in combination with a tracrRNA molecule) a nucleic acid guided nuclease as described herein (referred to herein as a "scaffold segment"). A guide nucleic acid can be DNA. A guide nucleic acid can be RNA. A guide nucleic acid can comprise both DNA and RNA. A guide nucleic acid can comprise modified non-naturally occurring nucleotides. In cases where the guide nucleic acid comprises RNA, the RNA guide nucleic acid can be encoded by a DNA sequence on a polynucleotide molecule such as a plasmid, linear construct, or editing cassette as disclosed herein.

In some examples, the guide nucleic acids described herein are RNA guide nucleic acids ("guide RNAs" or "gRNAs") and comprise a targeting segment and a scaffold segment. In some examples, the scaffold segment of a gRNA is comprised in one RNA molecule and the targeting segment is comprised in another separate RNA molecule. Such examples are referred to herein as "double-molecule gRNAs" or "two-molecule gRNA" or "dual gRNAs." In some examples, the gRNA is a single RNA molecule and is referred to herein as a "single-guide RNA" or an "sgRNA." The term "guide RNA" or "gRNA" is inclusive, referring both to two-molecule guide RNAs and sgRNAs.

The DNA-targeting segment of a gRNA comprises a nucleotide sequence that is complementary to a sequence in a target nucleic acid sequence. As such, the targeting segment of a gRNA interacts with a target nucleic acid in a sequence-specific manner via hybridization (i.e., base pairing), and the nucleotide sequence of the targeting segment determines the location within the target DNA that the gRNA will bind. The degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences. In some examples, a guide sequence is about or more than about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 75, or more nucleotides in length. In some examples, a guide sequence is less than about 75, 50, 45, 40, 35, 30, 25, 20 nucleotides in length. In examples, the guide sequence is 10-30 nucleotides long. The guide sequence can be 15-20 nucleotides in length. The guide sequence can be 15 nucleotides in length. The guide sequence can be 16 nucleotides in length. The guide sequence can be 17 nucleotides in length. The guide sequence can be 18 nucleotides in length. The guide sequence can be 19 nucleotides in length. The guide sequence can be 20 nucleotides in length.

The scaffold segment of a guide RNA interacts with a one or more Cas effector proteins to form a ribonucleoprotein complex (referred to herein as a CRISPR-RNP or a RNP-complex). The guide RNA directs the bound polypeptide to a specific nucleotide sequence within a target nucleic acid sequence via the above-described targeting segment. The scaffold segment of a guide RNA comprises two stretches of nucleotides that are complementary to one another and which form a double stranded RNA duplex. Sufficient sequence within the scaffold sequence to promote formation of a targetable nuclease complex may include a degree of complementarity along the length of two sequence regions within the scaffold sequence, such as one or two sequence regions involved in forming a secondary structure. In some cases, the one or two sequence regions are comprised or encoded on the same polynucleotide. In some cases, the one or two sequence regions are comprised or encoded on separate polynucleotides. Optimal alignment may be determined by any suitable alignment algorithm, and may further account for secondary structures, such as self-complementarity within either the one or two sequence regions. In some examples, the degree of complementarity between the one or two sequence regions along the length of the shorter of the two when optimally aligned is about or more than about 25%, 30%, 40%, 50%, 60%, 70%, 80%, 90%, 95%, 97.5%, 99%, or higher. In some examples, at least one of the two sequence regions is about or more than about 5, 6, 7, 8, 9, 10, 11, 12, 13, 14, 15, 16, 17, 18, 19, 20, 25, 30, 40, 50, or more nucleotides in length.

A scaffold sequence of a subject gRNA can comprise a secondary structure. A secondary structure can comprise a pseudoknot region or stem-loop structure. In some examples, the compatibility of a guide nucleic acid and nucleic acid guided nuclease is at least partially determined by sequence within or adjacent to the secondary structure region of the guide RNA. In some cases, binding kinetics of a guide nucleic acid to a nucleic acid guided nuclease is determined in part by secondary structures within the scaffold sequence. In some cases, binding kinetics of a guide nucleic acid to a nucleic acid guided nuclease is determined in part by nucleic acid sequence with the scaffold sequence.

A compatible scaffold sequence for a gRNA-Cas effector protein combination can be found by scanning sequences adjacent to a native Cas nuclease loci. In other words, native Cas nucleases can be encoded on a genome within proximity to a corresponding compatible guide nucleic acid or scaffold sequence.

Nucleic acid guided nucleases can be compatible with guide nucleic acids that are not found within the nucleases endogenous host. Such orthogonal guide nucleic acids can be determined by empirical testing. Orthogonal guide nucleic acids can come from different bacterial species or be synthetic or otherwise engineered to be non-naturally occurring. Orthogonal guide nucleic acids that are compatible with a common nucleic acid-guided nuclease can comprise one or more common features. Common features can include sequence outside a pseudoknot region. Common features can include a pseudoknot region. Common features can include a primary sequence or secondary structure

A guide nucleic acid can be engineered to target a desired target sequence by altering the guide sequence such that the guide sequence is complementary to the target sequence, thereby allowing hybridization between the guide sequence and the target sequence. A guide nucleic acid with an engineered guide sequence can be referred to as an engineered guide nucleic acid. Engineered guide nucleic acids are often non-naturally occurring and are not found in nature.

In some examples, the present disclosure provides a polynucleotide encoding a gRNA. In some examples, a gRNA-encoding nucleic acid is comprised in an expression vector, *e.g.,* a recombinant expression vector. In some examples, the present disclosure provides a polynucleotide encoding a site-directed modifying polypeptide. In some examples, the polynucleotide encoding a site-directed modifying polypeptide is comprised in an expression vector, *e.g.,* a recombinant expression vector.

## EXAMPLES

Examples 1-9 are demonstrations of the HTP genomic engineering platform in *Coynebacterium*. However, similar procedures have been customized for *E. coli* and are being successfully carried out by the inventors.

A brief table of contents is provided below solely for the purpose of assisting the reader. Nothing in this table of contents is meant to limit the scope of the examples or disclosure of the application.

**Table 5.1 - Table of Contents For Example Section**

| Example # | Title | Brief Description |
|---|---|---|
| **1** | HTP Transformation of *Corynebacterium* & Demonstration of SNP Library Creation | Describes examples of the **high-throughput genetic engineering** methods of the present disclosure. |
| **2** | HTP Genomic Engineering - Implementation of a SNP Library to Rehabilitate/Improve an Industrial Microbial Strain | Describes approaches for rehabilitating industrial organisms **through SNP swap** methods of the present disclosure. |
| **3** | HTP Genomic Engineering - Implementation of a SNP Swap Library to Improve Strain Performance in Lysine Production in *Corynebacterium.* | Describes an implementation of **SNP swap** techniques for **improving the performance of *Corynebacterium* strain producing lysine.** Also discloses selected **second and third order mutation consolidations.** |
| 4 | HTP Genomic Engineering - Implementation of a Promoter Swap Library to Improve an Industrial Microbial Strain | Describes methods for improving the strain performance of host organisms through **PRO swap** genetic design libraries of the present disclosure. |
| **5** | HTP Genomic Engineering - Implementation of a PRO Swap Library to Improve Strain Performance for Lysine Production | Describes an implementation of **PRO swap** techniques for **improving the performance of *Corynebacterium* strain producing lysine.** |
| **6** | Epistasis Mapping- An Algorithmic Tool for Predicting Beneficial Mutation Consolidations | Describes an example of the **automated tools/ algorithms** of the present disclosure for **predicting beneficial gene mutation consolidations.** |
| **7** | HTP Genomic Engineering - PRO Swap Mutation Consolidation and Multi-Factor Combinatorial Testing | Describes and illustrates the ability of the HTP methods of the present disclosure to effectively **explore the large solution space created by the combinatorial consolidation of multiple gene/genetic design library combinations.** |
| **8** | HTP Genomic Engineering - Implementation of a Terminator Library to Improve an Industrial Host Strain | Describes and illustrates an application of the **STOP swap** genetic design libraries of the present disclosure. |
| **9** | Comparing HTP Toolsets vs. Traditional UV Mutations | Provides experimental results **comparing the HTP genetic design** methods of the present disclosure **vs. traditional mutational strain improvement programs.** |
| **10** | HTP Genomic Engineering - Implementation of a Transposon Mutagenesis Library to Improve Strain Performance Escherichia coli | Describes examples of using a transposon mutagenesis library with the **high-throughput genetic engineering** methods of the present disclosure, **as applied to *E. coli* cells.** |
| **11** | HTP Genomic Engineering - Generation of Vector Backbones for use in HTP Genomic Engineering in *Escherichia coli* | Describes examples of generating vector backbones for use in the **high-throughput genetic engineering** methods of the present disclosure, **as applied to *E. coli* cells.** |
| **12** | HTP Genomic Engineering - Generation and testing of an additional Promoter Swap Library for use in improving an Industrial Microbial Strain | Describes methods for designing, generating and testing **PRO swap** genetic design libraries of the present disclosure. |

(continued)

| Example # | Title | Brief Description |
|---|---|---|
| 13 | HTP Genomic Engineering -Testing integration of Promoter Swap Library of Table 1.4 into the genome of E. coli using vector 2 backbone | Describes proof of concept of the use of the vector 2 backbone from Example 11 in combination with promoters form the promoter library of Table 1.4 to drive integration of a single copy of a heterologous promoter-gene construct into the genome of *E. coli* |
| 14 | HTP Genomic Engineering - Implementation of a PROSWP methods using promoter library derived from Table 1.4. | Describes methods for improving the strain performance of host organisms through **PRO swap** genetic design libraries derived from Table 1.4 of the present disclosure. |
| 15 | HTP Genomic Engineering - Implementation of a TERMINATOR Swap Library to Improve Strain Performance for Lycopene Production | Describes methods for improving the strain performance of host organisms through **terminator swap** genetic design libraries of the present disclosure. |
| 16 | HTP Genomic Engineering - Implementation of a TERMINATOR Swap Library or PRO Swap Library in combination with either a SOLUBILITY TAG swap Library or DEGRADATION Tag swap Library to Improve Strain Performance for Lycopene Production | Describes methods for improving the strain performance of host organisms through **terminator swap, solubility tag swap** and **degradation tag swap** genetic design libraries of the present disclosure. |

**Example 1: HTP Transformation of *Corynebacterium* & Demonstration of SNP Library Creation**

This example illustrates examples of the HTP genetic engineering methods of the present disclosure. Host cells are transformed with a variety of SNP sequences of different sizes, all targeting different areas of the genome. The results demonstrate that the methods of the present disclosure are able to generate rapid genetic changes of any kind, across the entire genome of a host cell.

***A. Cloning of Transformation Vectors***

A variety of SNPs were chosen at random from *Corynebacterium glutamicum* (ATCC21300) and were cloned into *Corynebacterium* cloning vectors using yeast homologous recombination cloning techniques to assemble a vector in which each SNP was flanked by direct repeat regions, as described *supra* in the "Assembling/Cloning Custom Plasmids" section, and as illustrated in Figure 3.

The SNP cassettes for this example were designed to include a range of homology direct repeat arm lengths ranging from 0.5Kb, 1Kb, 2Kb, and 5Kb. Moreover, SNP cassettes were designed for homologous recombination targeted to various distinct regions of the genome, as described in more detail below.

The C. *glutamicum* genome is 3,282,708 bp in size (see Figure 9). The genome was arbitrarily divided into 24 equal-sized genetic regions, and SNP cassettes were designed to target each of the 24 regions. Thus, a total of 96 distinct plasmids were cloned for this Example (4 different insert sizes x 24 distinct genomic regions).

Each DNA insert was produced by PCR amplification of homologous regions using commercially sourced oligos and the host strain genomic DNA described above as template. The SNP to be introduced into the genome was encoded in the oligo tails. PCR fragments were assembled into the vector backbone using homologous recombination in yeast.

Cloning of each SNP and homology arm into the vector was conducted according to the HTP engineering workflow described in Figure 6A-B, Figure 3, and Table 5.

**B. *Transformation of Assembled Clones into E. coli***

Vectors were initially transformed into *E. coli* using standard heat shock transformation techniques in order to identify correctly assembled clones, and to amplify vector DNA for *Corynebacterium* transformation.

For example, transformed *E. coli* bacteria were tested for assembly success. Four colonies from each *E. coli* transformation plate were cultured and tested for correct assembly via PCR. This process was repeated for each of the 24 transformation locations and for each of the 4 different insert sizes (i.e., for all 96 transformants of this example). Results from this experiment were represented as the number of correct colonies identified out of the four colonies that were tested for each treatment (insert size and genomic location) (see Figure 12). Longer 5kb inserts exhibited a decrease in assembly efficiency compared to shorter counterparts (n=96).

**C. *Transformation of Assembled Clones into Corynebacterium***

Validated clones were transformed into *Corynebacterium glutamicum* host cells via electroporation. For each transformation, the number of Colony Forming Units (CFUs) per μg of DNA was determined as a function of the insert size (see Figure 13). *Coryne* genome integration was also analyzed as a function of homology arm length, and the results showed that shorter arms had a lower efficiency (see Figure 13).

Genomic integration efficiency was also analyzed with respect to the targeted genome location in C. *glutamicum* transformants. Genomic positions 1 and 2 exhibited slightly lowered integration efficiency compared to the rest of the genome *(see* Figure 10).

**D. *Looping Out Selection Markers***

Cultures of *Corynebacterium* identified as having successful integrations of the insert cassette were cultured on media containing 5% sucrose to counter select for loop outs of the *sacb* selection gene. Sucrose resistance frequency for various homology direct repeat arms did not vary significantly with arm length *(see* Figure 14). These results suggested that loopout efficiencies remained steady across homology arm lengths of .5 kb to 5kb.

In order to further validate loop out events, colonies exhibiting sucrose resistance were cultured and analyzed via sequencing.

The results for the sequencing of the insert genomic regions are summarized in Table 6 below.

**Table** 6 - Loop-out Validation Frequency

| **Outcome** | **Frequency (sampling error 95% confidence)** |
|---|---|
| **Successful Loop out** | 13% (9%/20%) |
| **Loop Still present** | 42% (34%/50%) |
| **Mixed read** | 44% (36%/52%) |

Sequencing results showed a 10-20% efficiency in loop outs. Actual loop-out probably is somewhat dependent on insert sequence. However, picking 10-20 sucrose-resistant colonies leads to high success rates.

**E. *Summary***

Table 7 below provides a quantitative assessment of the efficiencies of the HTP genome engineering methods of the present disclosure. Construct assembly rates for yeast homology methodologies yielded expected DNA constructs in nearly 9 out of 10 tested colonies. *Coryne* transformations of SNP constructs with 2kb homology arms yielded an average of 51 colony forming units per micro gram of DNA (CFU/μg), with 98% of said colonies exhibiting correctly integrated SNP inserts (targeting efficiency). Loop out efficiencies remained at .2% of cells becoming resistant when exposed to sucrose, with 13% of these exhibiting correctly looped out sequences.

**Table** 7- Summary Results for *Corynebacterium glutamicum* Strain Engineering

| **QC Step** | **Results for 2 kb Homology Arms** |
|---|---|
| Construct Assembly Success | 87% |
| Coryne Transformation efficiency | 51 CFU/μg DNA (+/- 15) |
| Targeting efficiency | 98% |
| Loop out Efficiency | 0.2% (+/- 0.03%) |

**Example 2: HTP Genomic Engineering - Implementation of a SNP Library to Rehabilitate/Improve an Industrial Microbial Strain**

This example illustrates several examples of the SNP swap libraries of the HTP strain improvement programs of the present disclosure. Specifically, the example illustrates several envisioned approaches for rehabilitating currently existing industrial strains. This example describes the wave up and wave down approaches to exploring the phenotypic solution space created by the multiple genetic differences that may be present between "base," "intermediate," and industrial strains.

**A. Identification of SNPs in Diversity Pool**

An exemplary strain improvement program using the methods of the present disclosure was conducted on an industrial production microbial strain, herein referred to as "C." The diversity pool strains for this program are represented by A, B, and C. Strain A represented the original production host strain, prior to any mutagenesis. Strain C represented the current industrial strain, which has undergone many years of mutagenesis and selection *via* traditional strain improvement programs. Strain B represented a "middle ground" strain, which had undergone some mutagenesis, and had been the predecessor of strain C. *(see* Figure 17A).

Strains A, B, and C were sequenced and their genomes were analyzed for genetic differences between strains. A total of 332 non-synonymous SNPs were identified. Of these, 133 SNPs were unique to C, 153 were additionally shared by B and C, and 46 were unique to strain B *(see* Figure 17B). These SNPs will be used as the diversity pool for downstream strain improvement cycles.

**B. SNP Swapping Analysis**

SNPs identified from the diversity pool in Part A of Example 2 will be analyzed to determine their effect on host cell performance. The initial "learning" round of the strain performance will be broken down into six steps as described below, and diagramed in Figure 18.

*First,* all the SNPs from C will be individually and/or combinatorially cloned into the base A strain. This will represent a minimum of 286 individual transformants. The purpose of these transformants will be to identify beneficial SNPs.
*Second,* all the SNPs from C will be individually and/or combinatorially removed from the commercial strain C. This will represent a minimum of 286 individual transformants. The purpose of these transformants will be to identify neutral and detrimental SNPs. Additional optional steps 3-6 are also described below. The first and second steps of adding and subtracting SNPS from two genetic time points (base strain A, and industrial strain C) is herein referred to as "wave," which comprises a "wave up" (addition of SNPs to a base strain, first step), and a "wave down" (removal of SNPs from the industrial strain, second step). The wave concept extends to further additions/subtractions of SNPS.
*Third,* all the SNPs from B will be individually and/or combinatorially cloned into the base A strain. This will represent a minimum of 199 individual transformants. The purpose of these transformants will be to identify beneficial SNPs. Several of the transformants will also serve as validation data for transformants produced in the first step.
*Fourth,* all the SNPs from B will be individually and/or combinatorially removed from the commercial strain B. This will represent a minimum of 199 individual transformants. The purpose of these transformants will be to identify neutral and detrimental SNPs. Several of the transformants will also serve as validation data for transformants produced in the second step.
*Fifth,* all the SNPs *unique* to C (i.e., not also present in B) will be individually and/or combinatorially cloned into the commercial B strain. This will represent a minimum of 46 individual transformants. The purpose of these transformants will be to identify beneficial SNPs. Several of the transformants will also serve as validation data for transformants produced in the first and third steps.
*Sixth,* all the SNPs *unique* to C will be individually and/or combinatorially removed from the commercial strain C. This will represent a minimum of 46 individual transformants. The purpose of these transformants will be to identify neutral and detrimental SNPs. Several of the transformants will also serve as validation data for transformants produced in the second and fourth steps.

Data collected from each of these steps is used to classify each SNP as *prima facie* beneficial, neutral, or detrimental.

**C. Utilization of Epistatic Mapping to Determine Beneficial SNP Combinations**

Beneficial SNPs identified in Part B of Example 2 will be analyzed *via* the epistasis mapping methods of the present disclosure, in order to identify SNPs that are likely to improve host performance when combined.

New engineered strain variants will be created using the engineering methods of Example 1 to test SNP combinations according to epistasis mapping predictions. SNPs consolidation may take place sequentially, or may alternatively take place across multiple branches such that more than one improved strain may exist with a subset of beneficial SNPs. SNP consolidation will continue over multiple strain improvement rounds, until a final strain is produced containing the optimum combination of beneficial SNPs, without any of the neutral or detrimental SNP baggage

**Example** 3: **HTP Genomic Engineering** - **Implementation of a SNP Swap Library to Improve Strain Performance in Lysine Production in *Corynebacterium***

This example provides an illustrative implementation of a portion of the SNP Swap HTP design strain improvement program of Example 2 with the goal of producing yield and productivity improvements of lysine production in *Corynebacterium.*

Section B of this example further illustrates the mutation consolidation steps of the HTP strain improvement program of the present disclosure. The example thus provides experimental results for a first, second, and third round consolidation of the HTP strain improvement methods of the present disclosure.

Mutations for the second and third round consolidations are derived from separate genetic library swaps. These results thus also illustrate the ability for the HTP strain programs to be carried out multi-branch parallel tracks, and the "memory" of beneficial mutations that can be embedded into meta data associated with the various forms of the genetic design libraries of the present disclosure.

As described above, the genomes of a provided base reference strain (Strain A), and a second "engineered" strain (Strain C) were sequenced, and all genetic differences were identified. The base strain was a *Corynebacterium glutamicum* variant that had not undergone UV mutagenesis. The engineered strain was also a C. *glutamicum* strain that had been produced from the base strain after several rounds of traditional mutation improvement programs. This Example provides the SNP Swap results for 186 distinct non-synonymous SNP differences identified between strains A and C.

**A. HTP engineering and High-Throughput Screening**

Each of the 186 identified SNPs were individually added back into the base strain, according to the cloning and transformation methods of the present disclosure. Each newly created strain comprising a single SNP was tested for lysine yield in small scale cultures designed to assess product titer performance. Small scale cultures were conducted using media from industrial scale cultures. Product titer was optically measured at carbon exhaustion *(i.e.,* representative of single batch yield) with a standard colorimetric assay. Briefly, a concentrated assay mixture was prepared and was added to fermentation samples such that final concentrations of reagents were 160 mM sodium phosphate buffer, 0.2 mM Amplex Red, 0.2 U/mL Horseradish Peroxidase and 0.005 U/mL of lysine oxidase. Reactions were allowed to proceed to an end point and optical density measured using a Tecan M1000 plate spectrophotometer at a 560nm wavelength. The results of the experiment are summarized in Table 8 below, and depicted in Figure 38.

**Table 8** - Summary Results for SNP Swap Strain Engineering for Lysine Production

| SNP | N | Mean Lysine Yield (change in $A_{560}$ compared to reference strain) | Std Error | % Change over Reference | % Change error |
|---|---|---|---|---|---|
| DSS_ 033 | 4 | 0.1062 | 0.00888 | 11.54348 | 2.895652 |
| DSS_ 311 | 2 | 0.03603 | 0.01256 | 3.916304 | 4.095652 |
| DSS_ 350 | 1 | 0.03178 | 0.01777 | 3.454348 | 5.794565 |
| DSS_ 056 | 3 | 0.02684 | 0.01026 | 2.917391 | 3.345652 |
| DSS_ 014 | 4 | 0.02666 | 0.00888 | 2.897826 | 2.895652 |

(continued)

| SNP | N | Mean Lysine Yield (change in $A_{560}$ compared to reference strain) | Std Error | % Change over Reference | % Change error |
|---|---|---|---|---|---|
| DSS_ 338 | 3 | 0.02631 | 0.01026 | 2.859783 | 3.345652 |
| DSS_ 128 | 1 | 0.02584 | 0.01777 | 2.808696 | 5.794565 |
| DSS_ 038 | 4 | 0.02467 | 0.00888 | 2.681522 | 2.895652 |
| DSS_ 066 | 4 | 0.02276 | 0.00888 | 2.473913 | 2.895652 |
| DSS_ 108 | 2 | 0.02216 | 0.01256 | 2.408696 | 4.095652 |
| DSS_ 078 | 4 | 0.02169 | 0.00888 | 2.357609 | 2.895652 |
| DSS_ 017 | 3 | 0.02102 | 0.01026 | 2.284783 | 3.345652 |
| DSS_ 120 | 3 | 0.01996 | 0.01026 | 2.169565 | 3.345652 |
| DSS_ 064 | 4 | 0.01889 | 0.00888 | 2.053261 | 2.895652 |
| DSS_ 380 | 4 | 0.01888 | 0.00888 | 2.052174 | 2.895652 |
| DSS_ 105 | 3 | 0.0184 | 0.01026 | 2 | 3.345652 |
| DSS_ 407 | 1 | 0.01831 | 0.01777 | 1.990217 | 5.794565 |
| DSS_ 018 | 2 | 0.01825 | 0.01256 | 1.983696 | 4.095652 |
| DSS_ 408 | 3 | 0.01792 | 0.01026 | 1.947826 | 3.345652 |
| DSS_ 417 | 3 | 0.01725 | 0.01026 | 1.875 | 3.345652 |
| DSS_ 130 | 3 | 0.01724 | 0.01026 | 1.873913 | 3.345652 |
| DSS_ 113 | 4 | 0.0172 | 0.00888 | 1.869565 | 2.895652 |
| DSS_ 355 | 3 | 0.01713 | 0.01026 | 1.861957 | 3.345652 |
| DSS_ 121 | 3 | 0.01635 | 0.01026 | 1.777174 | 3.345652 |
| DSS_ 097 | 2 | 0.0162 | 0.01256 | 1.76087 | 4.095652 |
| DSS_ 107 | 3 | 0.01604 | 0.01026 | 1.743478 | 3.345652 |

(continued)

| SNP | N | Mean Lysine Yield (change in $A_{560}$ compared to reference strain) | Std Error | % Change over Reference | % Change error |
|---|---|---|---|---|---|
| DSS_ 110 | 2 | 0.01524 | 0.01256 | 1.656522 | 4.095652 |
| DSS_ 306 | 4 | 0.01501 | 0.00888 | 1.631522 | 2.895652 |
| DSS_ 316 | 1 | 0.01469 | 0.01777 | 1.596739 | 5.794565 |
| DSS_ 325 | 4 | 0.01436 | 0.00888 | 1.56087 | 2.895652 |
| DSS_ 016 | 4 | 0.01416 | 0.00888 | 1.53913 | 2.895652 |
| DSS_ 324 | 4 | 0.01402 | 0.00888 | 1.523913 | 2.895652 |
| DSS_ 297 | 4 | 0.01391 | 0.00888 | 1.511957 | 2.895652 |
| DSS_ 118 | 2 | 0.01371 | 0.01256 | 1.490217 | 4.095652 |
| DSS_ 100 | 2 | 0.01326 | 0.01256 | 1.441304 | 4.095652 |
| DSS_ 019 | 1 | 0.01277 | 0.01777 | 1.388043 | 5.794565 |
| DSS_ 131 | 3 | 0.01269 | 0.01026 | 1.379348 | 3.345652 |
| DSS_ 394 | 4 | 0.01219 | 0.00888 | 1.325 | 2.895652 |
| DSS_ 385 | 3 | 0.01192 | 0.01026 | 1.295652 | 3.345652 |
| DSS_ 395 | 1 | 0.01162 | 0.01777 | 1.263043 | 5.794565 |
| DSS_ 287 | 4 | 0.01117 | 0.00888 | 1.21413 | 2.895652 |
| DSS_ 418 | 2 | 0.01087 | 0.01256 | 1.181522 | 4.095652 |
| DSS_ 290 | 3 | 0.01059 | 0.01026 | 1.151087 | 3.345652 |
| DSS_ 314 | 2 | 0.01036 | 0.01256 | 1.126087 | 4.095652 |
| DSS_ 073 | 4 | 0.00986 | 0.00888 | 1.071739 | 2.895652 |
| DSS_ 040 | 4 | 0.00979 | 0.00888 | 1.06413 | 2.895652 |
| DSS_ 037 | 4 | 0.00977 | 0.00888 | 1.061957 | 2.895652 |

(continued)

| SNP | N | Mean Lysine Yield (change in $A_{560}$ compared to reference strain) | Std Error | % Change over Reference | % Change error |
|---|---|---|---|---|---|
| DSS_ 341 | 1 | 0.00977 | 0.01777 | 1.061957 | 5.794565 |
| DSS_ 302 | 4 | 0.00939 | 0.00888 | 1.020652 | 2.895652 |
| DSS_ 104 | 4 | 0.00937 | 0.00888 | 1.018478 | 2.895652 |
| DSS_ 273 | 2 | 0.00915 | 0.01256 | 0.994565 | 4.095652 |
| DSS_ 322 | 4 | 0.00906 | 0.00888 | 0.984783 | 2.895652 |
| DSS_ 271 | 3 | 0.00901 | 0.01026 | 0.979348 | 3.345652 |
| DSS_ 334 | 2 | 0.00898 | 0.01256 | 0.976087 | 4.095652 |
| DSS_ 353 | 4 | 0.00864 | 0.00888 | 0.93913 | 2.895652 |
| DSS_ 391 | 4 | 0.00764 | 0.00888 | 0.830435 | 2.895652 |
| DSS_ 372 | 1 | 0.00737 | 0.01777 | 0.801087 | 5.794565 |
| DSS_ 007 | 1 | 0.00729 | 0.01777 | 0.792391 | 5.794565 |
| DSS_ 333 | 2 | 0.0072 | 0.01256 | 0.782609 | 4.095652 |
| DSS_ 402 | 4 | 0.00718 | 0.00888 | 0.780435 | 2.895652 |
| DSS_ 084 | 1 | 0.0069 | 0.01777 | 0.75 | 5.794565 |
| DSS_ 103 | 3 | 0.00676 | 0.01026 | 0.734783 | 3.345652 |
| DSS_ 362 | 1 | 0.00635 | 0.01777 | 0.690217 | 5.794565 |
| DSS_ 012 | 2 | 0.00595 | 0.01256 | 0.646739 | 4.095652 |
| DSS_ 396 | 2 | 0.00574 | 0.01256 | 0.623913 | 4.095652 |
| DSS_ 133 | 3 | 0.00534 | 0.01026 | 0.580435 | 3.345652 |
| DSS_ 065 | 3 | 0.00485 | 0.01026 | 0.527174 | 3.345652 |
| DSS_ 284 | 2 | 0.00478 | 0.01256 | 0.519565 | 4.095652 |

(continued)

| SNP | N | Mean Lysine Yield (change in $A_{560}$ compared to reference strain) | Std Error | % Change over Reference | % Change error |
|---|---|---|---|---|---|
| DSS_ 301 | 3 | 0.00465 | 0.01026 | 0.505435 | 3.345652 |
| DSS_ 281 | 4 | 0.00461 | 0.00888 | 0.501087 | 2.895652 |
| DSS_ 405 | 2 | 0.00449 | 0.01256 | 0.488043 | 4.095652 |
| DSS_ 361 | 3 | 0.00438 | 0.01026 | 0.476087 | 3.345652 |
| DSS_ 342 | 4 | 0.00434 | 0.00888 | 0.471739 | 2.895652 |
| DSS_ 053 | 3 | 0.00422 | 0.01026 | 0.458696 | 3.345652 |
| DSS_ 074 | 4 | 0.00422 | 0.00888 | 0.458696 | 2.895652 |
| DSS_ 079 | 4 | 0.00375 | 0.00888 | 0.407609 | 2.895652 |
| DSS_ 381 | 3 | 0.0036 | 0.01026 | 0.391304 | 3.345652 |
| DSS_ 294 | 1 | 0.00336 | 0.01777 | 0.365217 | 5.794565 |
| DSS_ 313 | 2 | 0.00332 | 0.01256 | 0.36087 | 4.095652 |
| DSS_ 388 | 2 | 0.00305 | 0.01256 | 0.331522 | 4.095652 |
| DSS_ 392 | 4 | 0.00287 | 0.00888 | 0.311957 | 2.895652 |
| DSS_ 319 | 4 | 0.00282 | 0.00888 | 0.306522 | 2.895652 |
| DSS_ 310 | 4 | 0.00263 | 0.00888 | 0.28587 | 2.895652 |
| DSS_ 344 | 3 | 0.00259 | 0.01026 | 0.281522 | 3.345652 |
| DSS_ 025 | 4 | 0.00219 | 0.00888 | 0.238043 | 2.895652 |
| DSS_ 412 | 1 | 0.00204 | 0.01777 | 0.221739 | 5.794565 |
| DSS_ 300 | 3 | 0.00188 | 0.01026 | 0.204348 | 3.345652 |
| DSS_ 299 | 2 | 0.00185 | 0.01256 | 0.201087 | 4.095652 |
| DSS_ 343 | 4 | 0.00184 | 0.00888 | 0.2 | 2.895652 |

(continued)

| SNP | N | Mean Lysine Yield (change in $A_{560}$ compared to reference strain) | Std Error | % Change over Reference | % Change error |
|---|---|---|---|---|---|
| DSS_ 330 | 3 | 0.00153 | 0.01026 | 0.166304 | 3.345652 |
| DSS_ 416 | 4 | 0.00128 | 0.00888 | 0.13913 | 2.895652 |
| DSS_ 034 | 3 | 0.00128 | 0.01026 | 0.13913 | 3.345652 |
| DSS_ 291 | 2 | 0.00102 | 0.01256 | 0.11087 | 4.095652 |
| DSS_ 115 | 4 | 0.00063 | 0.00888 | 0.068478 | 2.895652 |
| DSS_ 288 | 4 | 0.00044 | 0.00888 | 0.047826 | 2.895652 |
| DSS_ 309 | 4 | 0.00008 | 0.00888 | 0.008696 | 2.895652 |
| DSS_ 125 | 3 | 0 | 0.01026 | 0 | 3.345652 |
| DSS_ 358 | 3 | -0.00015 | 0.01026 | -0.0163 | 3.345652 |
| DSS_ 099 | 2 | -0.00015 | 0.01256 | -0.0163 | 4.095652 |
| DSS_ 111 | 4 | -0.00017 | 0.00888 | -0.01848 | 2.895652 |
| DSS_ 359 | 3 | -0.00022 | 0.01026 | -0.02391 | 3.345652 |
| DSS_ 015 | 4 | -0.00043 | 0.00888 | -0.04674 | 2.895652 |
| DSS_ 060 | 3 | -0.0007 | 0.01026 | -0.07609 | 3.345652 |
| DSS_ 098 | 2 | -0.00088 | 0.01256 | -0.09565 | 4.095652 |
| DSS_ 379 | 4 | -0.00089 | 0.00888 | -0.09674 | 2.895652 |
| DSS_ 356 | 4 | -0.0009 | 0.00888 | -0.09783 | 2.895652 |
| DSS_ 278 | 4 | -0.00095 | 0.00888 | -0.10326 | 2.895652 |
| DSS_ 368 | 4 | -0.001 | 0.00888 | -0.1087 | 2.895652 |
| DSS_ 351 | 1 | -0.0015 | 0.01777 | -0.16304 | 5.794565 |
| DSS_ 296 | 1 | -0.0015 | 0.01777 | -0.16304 | 5.794565 |

(continued)

| SNP | N | Mean Lysine Yield (change in $A_{560}$ compared to reference strain) | Std Error | % Change over Reference | % Change error |
|---|---|---|---|---|---|
| DSS_119 | 3 | -0.00156 | 0.01026 | -0.16957 | 3.345652 |
| DSS_307 | 3 | -0.00163 | 0.01026 | -0.17717 | 3.345652 |
| DSS_077 | 4 | -0.00167 | 0.00888 | -0.18152 | 2.895652 |
| DSS_030 | 3 | -0.00188 | 0.01026 | -0.20435 | 3.345652 |
| DSS_370 | 2 | -0.00189 | 0.01256 | -0.20543 | 4.095652 |
| DSS_375 | 2 | -0.00212 | 0.01256 | -0.23043 | 4.095652 |
| DSS_280 | 3 | -0.00215 | 0.01026 | -0.2337 | 3.345652 |
| DSS_345 | 4 | -0.00225 | 0.00888 | -0.24457 | 2.895652 |
| DSS_419 | 1 | -0.00234 | 0.01777 | -0.25435 | 5.794565 |
| DSS_298 | 2 | -0.00249 | 0.01256 | -0.27065 | 4.095652 |
| DSS_367 | 3 | -0.0026 | 0.01026 | -0.28261 | 3.345652 |
| DSS_072 | 3 | -0.00268 | 0.01026 | -0.2913 | 3.345652 |
| DSS_366 | 4 | -0.00272 | 0.00888 | -0.29565 | 2.895652 |
| DSS_063 | 4 | -0.00283 | 0.00888 | -0.30761 | 2.895652 |
| DSS_092 | 3 | -0.00292 | 0.01026 | -0.31739 | 3.345652 |
| DSS_347 | 4 | -0.0033 | 0.00888 | -0.3587 | 2.895652 |
| DSS_114 | 4 | -0.0034 | 0.00888 | -0.36957 | 2.895652 |
| DSS_303 | 3 | -0.00396 | 0.01026 | -0.43043 | 3.345652 |
| DSS_276 | 4 | -0.00418 | 0.00888 | -0.45435 | 2.895652 |
| DSS_083 | 1 | -0.00446 | 0.01777 | -0.48478 | 5.794565 |
| DSS_031 | 2 | -0.00456 | 0.01256 | -0.49565 | 4.095652 |

(continued)

| SNP | N | Mean Lysine Yield (change in $A_{560}$ compared to reference strain) | Std Error | % Change over Reference | % Change error |
|---|---|---|---|---|---|
| DSS_ 328 | 3 | -0.00463 | 0.01026 | -0.50326 | 3.345652 |
| DSS_ 039 | 4 | -0.00475 | 0.00888 | -0.5163 | 2.895652 |
| DSS_ 331 | 4 | -0.00475 | 0.00888 | -0.5163 | 2.895652 |
| DSS_ 117 | 4 | -0.00485 | 0.00888 | -0.52717 | 2.895652 |
| DSS_ 382 | 4 | -0.00506 | 0.00888 | -0.55 | 2.895652 |
| DSS_ 323 | 4 | -0.00507 | 0.00888 | -0.55109 | 2.895652 |
| DSS_ 041 | 2 | -0.00527 | 0.01256 | -0.57283 | 4.095652 |
| DSS_ 069 | 4 | -0.00534 | 0.00888 | -0.58043 | 2.895652 |
| DSS_ 308 | 3 | -0.00534 | 0.01026 | -0.58043 | 3.345652 |
| DSS_ 365 | 3 | -0.00536 | 0.01026 | -0.58261 | 3.345652 |
| DSS_ 403 | 3 | -0.00594 | 0.01026 | -0.64565 | 3.345652 |
| DSS_ 376 | 1 | -0.00648 | 0.01777 | -0.70435 | 5.794565 |
| DSS_ 293 | 3 | -0.00652 | 0.01026 | -0.7087 | 3.345652 |
| DSS_ 286 | 1 | -0.00672 | 0.01777 | -0.73043 | 5.794565 |
| BS.2C | 139 | -0.00694 | 0.00151 | -0.75435 | 0.492391 |
| DSS_ 410 | 1 | -0.00724 | 0.01777 | -0.78696 | 5.794565 |
| DSS_ 312 | 2 | -0.00725 | 0.01256 | -0.78804 | 4.095652 |
| DSS_ 336 | 1 | -0.00747 | 0.01777 | -0.81196 | 5.794565 |
| DSS_ 327 | 2 | -0.00748 | 0.01256 | -0.81304 | 4.095652 |
| DSS_ 127 | 4 | -0.00801 | 0.00888 | -0.87065 | 2.895652 |
| DSS_ 332 | 3 | -0.0085 | 0.01026 | -0.92391 | 3.345652 |
| DSS_ 054 | 2 | -0.00887 | 0.01256 | -0.96413 | 4.095652 |

(continued)

| SNP | N | Mean Lysine Yield (change in $A_{560}$ compared to reference strain) | Std Error | % Change over Reference | % Change error |
|---|---|---|---|---|---|
| DSS_ 024 | 2 | -0.00902 | 0.01256 | -0.98043 | 4.095652 |
| DSS_ 106 | 3 | -0.0096 | 0.01026 | -1.04348 | 3.345652 |
| DSS_ 400 | 4 | -0.00964 | 0.00888 | -1.04783 | 2.895652 |
| DSS_ 346 | 3 | -0.00976 | 0.01026 | -1.06087 | 3.345652 |
| DSS_ 320 | 1 | -0.01063 | 0.01777 | -1.15543 | 5.794565 |
| DSS_ 275 | 4 | -0.01066 | 0.00888 | -1.1587 | 2.895652 |
| DSS_ 371 | 3 | -0.01111 | 0.01026 | -1.20761 | 3.345652 |
| DSS_ 277 | 1 | -0.01315 | 0.01777 | -1.42935 | 5.794565 |
| DSS_ 282 | 3 | -0.01326 | 0.01026 | -1.4413 | 3.345652 |
| DSS_ 393 | 3 | -0.01379 | 0.01026 | -1.49891 | 3.345652 |
| DSS_ 378 | 3 | -0.01461 | 0.01026 | -1.58804 | 3.345652 |
| DSS_ 289 | 3 | -0.01563 | 0.01026 | -1.69891 | 3.345652 |
| DSS_ 317 | 1 | -0.01565 | 0.01777 | -1.70109 | 5.794565 |
| DSS_ 062 | 4 | -0.01626 | 0.00888 | -1.76739 | 2.895652 |
| DSS_ 340 | 1 | -0.01657 | 0.01777 | -1.80109 | 5.794565 |
| DSS_ 109 | 2 | -0.01706 | 0.01256 | -1.85435 | 4.095652 |
| DSS_ 011 | 2 | -0.0178 | 0.01256 | -1.93478 | 4.095652 |
| DSS_ 089 | 4 | -0.01844 | 0.00888 | -2.00435 | 2.895652 |
| DSS_ 059 | 1 | -0.01848 | 0.01777 | -2.0087 | 5.794565 |
| DSS_ 112 | 2 | -0.01959 | 0.01256 | -2.12935 | 4.095652 |
| DSS_ 043 | 2 | -0.0213 | 0.01256 | -2.31522 | 4.095652 |

(continued)

| SNP | N | Mean Lysine Yield (change in $A_{560}$ compared to reference strain) | Std Error | % Change over Reference | % Change error |
|---|---|---|---|---|---|
| DSS_413 | 1 | -0.02217 | 0.01777 | -2.40978 | 5.794565 |
| DSS_305 | 4 | -0.0227 | 0.00888 | -2.46739 | 2.895652 |
| DSS_045 | 4 | -0.02289 | 0.00888 | -2.48804 | 2.895652 |
| DSS_082 | 2 | -0.0231 | 0.01256 | -2.51087 | 4.095652 |
| DSS_272 | 1 | -0.02311 | 0.01777 | -2.51196 | 5.794565 |
| DSS_390 | 4 | -0.02319 | 0.00888 | -2.52065 | 2.895652 |
| DSS_010 | 3 | -0.02424 | 0.01026 | -2.63478 | 3.345652 |
| DSS_357 | 2 | -0.02525 | 0.01256 | -2.74457 | 4.095652 |
| DSS_085 | 4 | -0.03062 | 0.00888 | -3.32826 | 2.895652 |
| DSS_044 | 3 | -0.04088 | 0.01026 | -4.44348 | 3.345652 |
| DSS_315 | 2 | -0.0501 | 0.01256 | -5.44565 | 4.095652 |
| DSS_080 | 2 | -0.13519 | 0.01256 | -14.6946 | 4.095652 |

**B. Second Round HTP engineering and High-Throughput Screening** - **Consolidation of SNP swap Library with Selected PRO swap Hits**

One of the strengths of the HTP methods of the present disclosure is their ability to store HTP genetic design libraries together with information associated with each SNP/Promoter/Terminator/Start Codon's effects on host cell phenotypes. The present inventors had previously conducted a promoter swap experiment that had identified several zwf promoter swaps in C. *glutamicum* with positive effects on biosynthetic yields (*see e.g.,* results for target "N" in Figure 22).

The present inventors modified the base strain A of this Example to also include one of the previously identified zwf promoter swaps from Example 5. The top 176 SNPs identified from the initial screen described above in Table 8 were re-introduced into this new base strain to create a new SNP swap genetic design microbial library. As with the previous step, each newly created strain comprising a single SNP was tested for lysine yield. Selected SNP mutant strains were also tested for a productivity proxy, by measuring lysine production at 24 hours using the colorimetric method described supra. The results from this step are summarized in Table 9 below, and are depicted in Figure 39.

**Table 9-** Second Round Screening for SNP Swap Strain Engineering for Lysine Production

| Strain ID | SNP | N for 24hr | N for 96hr | Mean 24hr ($A_{560}$) | Mean 96hr ($A_{560}$) | Std Error 24hr | Std Error 96hr |
|---|---|---|---|---|---|---|---|
| 7000006318 | BS2C_P0007_39zwf | 20 | 2 | 0.49 | 0.82 | 0.00 | 0.02 |
| 7000008538 | DSS_002 | 4 | 2 | 0.53 | 0.78 | 0.01 | 0.02 |

(continued)

| Strain ID | SNP | N for 24hr | N for 96hr | Mean 24hr ($A_{560}$) | Mean 96hr ($A_{560}$) | Std Error 24hr | Std Error 96hr |
|---|---|---|---|---|---|---|---|
| 7000008539 | DSS_003 | 4 | | 0.56 | | 0.01 | |
| 7000008541 | DSS_005 | 4 | | 0.27 | | 0.01 | |
| 7000008542 | DSS_006 | 4 | | 0.49 | | 0.01 | |
| 7000008547 | DSS_011 | 4 | | 0.55 | | 0.01 | |
| 7000008548 | DSS_012 | 4 | | 0.58 | | 0.01 | |
| 7000008549 | DSS_013 | 4 | | 0.56 | | 0.01 | |
| 7000008550 | DSS_014 | 4 | | 0.52 | | 0.01 | |
| 7000008551 | DSS_015 | 4 | | 0.54 | | 0.01 | |
| 7000008552 | DSS_016 | 4 | 2 | 0.50 | 0.84 | 0.01 | 0.02 |
| 7000008553 | DSS_017 | 4 | | 0.44 | | 0.01 | |
| 7000008555 | DSS_019 | 4 | 4 | 0.46 | 0.84 | 0.01 | 0.01 |
| 7000008557 | DSS_021 | 4 | 4 | 0.46 | 0.86 | 0.01 | 0.01 |
| 7000008559 | DSS_023 | 4 | 2 | 0.55 | 0.86 | 0.01 | 0.02 |
| 7000008561 | DSS_025 | 4 | | 0.54 | | 0.01 | |
| 7000008562 | DSS_026 | 2 | | 0.46 | | 0.01 | |
| 7000008564 | DSS_028 | 4 | | 0.51 | | 0.01 | |
| 7000008565 | DSS_029 | 4 | 4 | 0.48 | 0.87 | 0.01 | 0.01 |
| 7000008566 | DSS_030 | 4 | 4 | 0.47 | 0.85 | 0.01 | 0.01 |
| 7000008567 | DSS_031 | 4 | | 0.56 | | 0.01 | |
| 7000008569 | DSS_033 | 4 | 4 | 0.46 | 0.86 | 0.01 | 0.01 |
| 7000008570 | DSS_034 | 2 | 2 | 0.53 | 0.85 | 0.01 | 0.02 |
| 7000008573 | DSS_037 | 4 | | 0.54 | | 0.01 | |
| 7000008574 | DSS_038 | 4 | | 0.53 | | 0.01 | |
| 7000008575 | DSS_039 | 4 | | 0.55 | | 0.01 | |
| 7000008576 | DSS_040 | 4 | | 0.57 | | 0.01 | |
| 7000008577 | DSS_041 | 4 | | 0.45 | | 0.01 | |
| 7000008578 | DSS_042 | 4 | 4 | 0.52 | 0.87 | 0.01 | 0.01 |
| 7000008579 | DSS_043 | 4 | 4 | 0.45 | 0.87 | 0.01 | 0.01 |
| 7000008580 | DSS_044 | 4 | 2 | 0.50 | 0.85 | 0.01 | 0.02 |
| 7000008581 | DSS_045 | 4 | | 0.47 | | 0.01 | |
| 7000008582 | DSS_046 | 4 | 2 | 0.61 | 0.85 | 0.01 | 0.02 |
| 7000008583 | DSS_047 | 4 | 2 | 0.61 | 0.82 | 0.01 | 0.02 |
| 7000008586 | DSS_050 | 4 | | 0.57 | | 0.01 | |
| 7000008587 | DSS_051 | 4 | | 0.56 | | 0.01 | |
| 7000008588 | DSS_052 | 4 | 2 | 0.49 | 0.85 | 0.01 | 0.02 |
| 7000008589 | DSS_053 | 4 | 4 | 0.45 | 0.85 | 0.01 | 0.01 |
| 7000008590 | DSS_054 | 4 | 4 | 0.45 | 0.88 | 0.01 | 0.01 |

(continued)

| Strain ID | SNP | N for 24hr | N for 96hr | Mean 24hr ($A_{560}$) | Mean 96hr ($A_{560}$) | Std Error 24hr | Std Error 96hr |
|---|---|---|---|---|---|---|---|
| 7000008592 | DSS_056 | 4 | | 0.42 | | 0.01 | |
| 7000008596 | DSS_060 | 4 | 2 | 0.55 | 0.87 | 0.01 | 0.02 |
| 7000008597 | DSS_061 | 4 | 2 | 0.37 | 0.86 | 0.01 | 0.02 |
| 7000008598 | DSS_062 | 4 | 4 | 0.45 | 0.87 | 0.01 | 0.01 |
| 7000008601 | DSS_065 | 4 | 4 | 0.47 | 0.88 | 0.01 | 0.01 |
| 7000008602 | DSS_066 | 4 | | 0.47 | | 0.01 | |
| 7000008604 | DSS_068 | | 2 | | 0.51 | | 0.02 |
| 7000008605 | DSS_069 | 4 | 4 | 0.47 | 0.88 | 0.01 | 0.01 |
| 7000008606 | DSS_070 | 4 | | 0.55 | | 0.01 | |
| 7000008607 | DSS_071 | 4 | 2 | 0.56 | 0.84 | 0.01 | 0.02 |
| 7000008608 | DSS_072 | 4 | 2 | 0.54 | 0.83 | 0.01 | 0.02 |
| 7000008609 | DSS_073 | 4 | 2 | 0.47 | 0.84 | 0.01 | 0.02 |
| 7000008610 | DSS_074 | 4 | 2 | 0.51 | 0.83 | 0.01 | 0.02 |
| 7000008612 | DSS_076 | 4 | 4 | 0.48 | 0.76 | 0.01 | 0.01 |
| 7000008613 | DSS_077 | 4 | 4 | 0.46 | 0.87 | 0.01 | 0.01 |
| 7000008614 | DSS_078 | 4 | 2 | 0.44 | 0.87 | 0.01 | 0.02 |
| 7000008615 | DSS_079 | 4 | 2 | 0.47 | 0.90 | 0.01 | 0.02 |
| 7000008616 | DSS_080 | 4 | 2 | 0.48 | 0.81 | 0.01 | 0.02 |
| 7000008619 | DSS_083 | 4 | 2 | 0.59 | 0.86 | 0.01 | 0.02 |
| 7000008620 | DSS_084 | 4 | 2 | 0.70 | 0.89 | 0.01 | 0.02 |
| 7000008621 | DSS_085 | 4 | 4 | 0.49 | 0.89 | 0.01 | 0.01 |
| 7000008622 | DSS_086 | 4 | 2 | 0.48 | 0.82 | 0.01 | 0.02 |
| 7000008624 | DSS_088 | 4 | 2 | 0.47 | 0.88 | 0.01 | 0.02 |
| 7000008625 | DSS_089 | 4 | 4 | 0.45 | 0.89 | 0.01 | 0.01 |
| 7000008626 | DSS_090 | 4 | 4 | 0.47 | 0.87 | 0.01 | 0.01 |
| 7000008627 | DSS_091 | 4 | | 0.46 | | 0.01 | |
| 7000008629 | DSS_093 | 4 | 4 | 0.50 | 0.87 | 0.01 | 0.01 |
| 7000008630 | DSS_094 | 4 | 2 | 0.57 | 0.86 | 0.01 | 0.02 |
| 7000008634 | DSS_098 | 4 | 2 | 0.53 | 0.85 | 0.01 | 0.02 |
| 7000008636 | DSS_100 | 4 | | 0.52 | | 0.01 | |
| 7000008637 | DSS_101 | 4 | 2 | 0.49 | 0.85 | 0.01 | 0.02 |
| 7000008640 | DSS_104 | 4 | 2 | 0.51 | 0.84 | 0.01 | 0.02 |
| 7000008645 | DSS_109 | 4 | | 0.51 | | 0.01 | |
| 7000008646 | DSS_110 | 4 | 2 | 0.57 | 0.86 | 0.01 | 0.02 |
| 7000008648 | DSS_112 | 4 | 2 | 0.54 | 0.86 | 0.01 | 0.02 |
| 7000008651 | DSS_115 | 4 | | 0.49 | | 0.01 | |
| 7000008652 | DSS_116 | 4 | 2 | 0.52 | 0.82 | 0.01 | 0.02 |

(continued)

| Strain ID | SNP | N for 24hr | N for 96hr | Mean 24hr ($A_{560}$) | Mean 96hr ($A_{560}$) | Std Error 24hr | Std Error 96hr |
|---|---|---|---|---|---|---|---|
| 7000008653 | DSS_117 | 4 | 2 | 0.50 | 0.84 | 0.01 | 0.02 |
| 7000008657 | DSS_121 | 4 | 2 | 0.78 | 0.88 | 0.01 | 0.02 |
| 7000008659 | DSS_123 | 4 | | 0.54 | | 0.01 | |
| 7000008663 | DSS_127 | 4 | | 0.58 | | 0.01 | |
| 7000008665 | DSS_129 | 4 | | 0.48 | | 0.01 | |
| 7000008666 | DSS_130 | 4 | | 0.56 | | 0.01 | |
| 7000008669 | DSS_133 | 4 | | 0.50 | | 0.01 | |
| 7000008670 | DSS_271 | 4 | 2 | 0.52 | 0.86 | 0.01 | 0.02 |
| 7000008672 | DSS_273 | 4 | | 0.56 | | 0.01 | |
| 7000008677 | DSS_278 | 2 | | 0.46 | | 0.01 | |
| 7000008678 | DSS_279 | 4 | | 0.55 | | 0.01 | |
| 7000008681 | DSS_282 | 4 | | 0.51 | | 0.01 | |
| 7000008683 | DSS_284 | 4 | | 0.59 | | 0.01 | |
| 7000008684 | DSS_285 | 4 | | 0.51 | | 0.01 | |
| 7000008685 | DSS_286 | 4 | | 0.56 | | 0.01 | |
| 7000008687 | DSS_288 | 4 | | 0.46 | | 0.01 | |
| 7000008688 | DSS_289 | 4 | | 0.57 | | 0.01 | |
| 7000008689 | DSS_290 | 4 | | 0.47 | | 0.01 | |
| 7000008693 | DSS_294 | 4 | 2 | 0.52 | 0.63 | 0.01 | 0.02 |
| 7000008696 | DSS_297 | 4 | 2 | 0.52 | 0.86 | 0.01 | 0.02 |
| 7000008697 | DSS_298 | 4 | | 0.58 | | 0.01 | |
| 7000008699 | DSS_300 | 4 | | 0.48 | | 0.01 | |
| 7000008700 | DSS_301 | 4 | | 0.58 | | 0.01 | |
| 7000008701 | DSS_302 | 4 | | 0.47 | | 0.01 | |
| 7000008702 | DSS_303 | 3 | | 0.46 | | 0.01 | |
| 7000008703 | DSS_304 | 3 | | 0.48 | | 0.01 | |
| 7000008705 | DSS_306 | 4 | 2 | 0.53 | 0.80 | 0.01 | 0.02 |
| 7000008708 | DSS_309 | 4 | | 0.56 | | 0.01 | |
| 7000008709 | DSS_310 | 4 | | 0.56 | | 0.01 | |
| 7000008711 | DSS_312 | 4 | | 0.55 | | 0.01 | |
| 7000008712 | DSS_313 | 4 | | 0.51 | | 0.01 | |
| 7000008718 | DSS_319 | 4 | 2 | 0.50 | 0.82 | 0.01 | 0.02 |
| 7000008720 | DSS_321 | 4 | | 0.56 | | 0.01 | |
| 7000008722 | DSS_323 | 2 | 2 | 0.48 | 0.85 | 0.01 | 0.02 |
| 7000008723 | DSS_324 | 4 | | 0.55 | | 0.01 | |
| 7000008724 | DSS_325 | 4 | | 0.50 | | 0.01 | |
| 7000008725 | DSS_326 | 3 | | 0.46 | | 0.01 | |

(continued)

| Strain ID | SNP | N for 24hr | N for 96hr | Mean 24hr ($A_{560}$) | Mean 96hr ($A_{560}$) | Std Error 24hr | Std Error 96hr |
|---|---|---|---|---|---|---|---|
| 7000008726 | DSS_327 | 3 | | 0.47 | | 0.01 | |
| 7000008730 | DSS_331 | 4 | | 0.56 | | 0.01 | |
| 7000008731 | DSS_332 | 4 | 4 | 0.47 | 0.89 | 0.01 | 0.01 |
| 7000008732 | DSS_333 | 4 | 4 | 0.47 | 0.87 | 0.01 | 0.01 |
| 7000008733 | DSS_334 | 4 | | 0.45 | | 0.01 | |
| 7000008734 | DSS_335 | 2 | | 0.47 | | 0.01 | |
| 7000008735 | DSS_336 | 4 | | 0.47 | | 0.01 | |
| 7000008739 | DSS_340 | 4 | | 0.46 | | 0.01 | |
| 7000008740 | DSS_341 | 4 | 2 | 0.46 | 0.89 | 0.01 | 0.02 |
| 7000008741 | DSS_342 | 4 | | 0.56 | | 0.01 | |
| 7000008742 | DSS_343 | 4 | | 0.55 | | 0.01 | |
| 7000008743 | DSS_344 | 4 | 4 | 0.48 | 0.87 | 0.01 | 0.01 |
| 7000008746 | DSS_347 | 4 | 4 | 0.48 | 0.85 | 0.01 | 0.01 |
| 7000008747 | DSS_348 | 4 | 4 | 0.46 | 0.86 | 0.01 | 0.01 |
| 7000008749 | DSS_350 | 4 | 2 | 0.29 | 0.74 | 0.01 | 0.02 |
| 7000008752 | DSS_353 | 4 | 2 | 0.46 | 0.85 | 0.01 | 0.02 |
| 7000008753 | DSS_354 | 4 | 4 | 0.45 | 0.87 | 0.01 | 0.01 |
| 7000008755 | DSS_356 | 4 | 4 | 0.46 | 0.86 | 0.01 | 0.01 |
| 7000008756 | DSS_357 | 4 | 4 | 0.46 | 0.86 | 0.01 | 0.01 |
| 7000008758 | DSS_359 | 2 | 2 | 0.45 | 0.85 | 0.01 | 0.02 |
| 7000008760 | DSS_361 | 4 | 2 | 0.46 | 0.84 | 0.01 | 0.02 |
| 7000008761 | DSS_362 | 4 | | 0.44 | | 0.01 | |
| 7000008763 | DSS_364 | 4 | | 0.44 | | 0.01 | |
| 7000008764 | DSS_365 | 4 | | 0.46 | | 0.01 | |
| 7000008765 | DSS_366 | 4 | | 0.55 | | 0.01 | |
| 7000008766 | DSS_367 | 4 | | 0.55 | | 0.01 | |
| 7000008767 | DSS_368 | 4 | 2 | 0.44 | 0.86 | 0.01 | 0.02 |
| 7000008770 | DSS_371 | 4 | 2 | 0.47 | 0.88 | 0.01 | 0.02 |
| 7000008771 | DSS_372 | 4 | 2 | 0.46 | 0.83 | 0.01 | 0.02 |
| 7000008772 | DSS_373 | 4 | 2 | 0.46 | 0.88 | 0.01 | 0.02 |
| 7000008774 | DSS_375 | 4 | | 0.45 | | 0.01 | |
| 7000008776 | DSS_377 | 4 | | 0.45 | | 0.01 | |
| 7000008777 | DSS_378 | 4 | | 0.57 | | 0.01 | |
| 7000008778 | DSS_379 | 4 | | 0.54 | | 0.01 | |
| 7000008779 | DSS_380 | 4 | 2 | 0.46 | 0.87 | 0.01 | 0.02 |
| 7000008781 | DSS_382 | 4 | 2 | 0.46 | 0.84 | 0.01 | 0.02 |
| 7000008782 | DSS_383 | 4 | | 0.48 | | 0.01 | |

(continued)

| Strain ID | SNP | N for 24hr | N for 96hr | Mean 24hr ($A_{560}$) | Mean 96hr ($A_{560}$) | Std Error 24hr | Std Error 96hr |
|---|---|---|---|---|---|---|---|
| 7000008783 | DSS_384 | 4 | 2 | 0.47 | 0.82 | 0.01 | 0.02 |
| 7000008784 | DSS_385 | 4 | 2 | 0.46 | 0.83 | 0.01 | 0.02 |
| 7000008786 | DSS_387 | 3 | | 0.43 | | 0.01 | |
| 7000008787 | DSS_388 | 3 | | 0.47 | | 0.01 | |
| 7000008788 | DSS_389 | 4 | 2 | 0.46 | 0.89 | 0.01 | 0.02 |
| 7000008790 | DSS_391 | 4 | | 0.57 | | 0.01 | |
| 7000008791 | DSS_392 | 4 | | 0.44 | | 0.01 | |
| 7000008795 | DSS_396 | 4 | 2 | 0.46 | 0.82 | 0.01 | 0.02 |
| 7000008799 | DSS_400 | 4 | | 0.47 | | 0.01 | |
| 7000008800 | DSS_401 | 4 | 2 | 0.46 | 0.86 | 0.01 | 0.02 |
| 7000008801 | DSS_402 | 4 | | 0.54 | | 0.01 | |
| 7000008805 | DSS_406 | 4 | 2 | 0.47 | 0.85 | 0.01 | 0.02 |
| 7000008807 | DSS_408 | 4 | | 0.45 | | 0.01 | |
| 7000008810 | DSS_411 | 4 | 2 | 0.46 | 0.87 | 0.01 | 0.02 |
| 7000008812 | DSS_413 | 3 | | 0.47 | | 0.01 | |
| 7000008813 | DSS_414 | 4 | 2 | 0.45 | 0.84 | 0.01 | 0.02 |
| 7000008815 | DSS_416 | 4 | 2 | 0.45 | 0.87 | 0.01 | 0.02 |
| 7000008816 | DSS_417 | 4 | | 0.46 | | 0.01 | |
| 7000008818 | DSS_419 | 4 | 2 | 0.47 | 0.84 | 0.01 | 0.02 |
| 7000008820 | DSS_421 | 4 | 2 | 0.45 | 0.79 | 0.01 | 0.02 |
| 7000008821 | DSS_422 | 4 | | 0.44 | | 0.01 | |

The results from this second round of SNP swap identified several SNPs capable of increasing base strain yield *and* productivity of lysine in a base strain comprising the zwf promoter swap mutation (*see e.g.,* SNP 084 and SNP 121 on the upper right hand corner of Figure 39).

### C. Tank Culture Validation

Strains containing top SNPs identified during the HTP steps above were cultured into medium sized test fermentation tanks. Briefly, small 100ml cultures of each strain were grown over night, and were then used to inoculate 5 liter cultures in the test fermentation tanks with equal amounts of inoculate. The inoculate was normalized to contain the same cellular density following an OD600 measurement.

The resulting tank cultures were allowed to proceed for 3 days before harvest. Yield and productivity measurements were calculated from substrate and product titers in samples taken from the tank at various points throughout the fermentation. Samples were analyzed for particular small molecule concentrations by high pressure liquid chromatography using the appropriate standards. Results for this experiment are summarized in Table 10 below, and depicted in Figure 40.

**Table 10-** Tank Validation of SNP Swap Microbes

| Strain | N | Mean Yield (%)(g lysine produced / g glucose consumed) | Std Error | Mean Productivity (g/L/h) | Std Error |
|---|---|---|---|---|---|
| base strain | 1 | 41.1502 | 0.5940 1 | 3.29377 | 0.2450 8 |

(continued)

| Strain | N | Mean Yield (%)(g lysine produced / g glucose consumed) | Std Error | Mean Productivity (g/L/h) | Std Error |
|---|---|---|---|---|---|
| base strain + zwf | 7 | 48.2952 | 0.2245 1 | 2.73474 | 0.1000 5 |
| base strain + zwf + SNP121 | 2 | 50.325 | 0.4200 3 | 4.51397 | 0.1733 |
| base strain + zwf + pyc + lysA | 5 | 52.191 | 0.2656 5 | 4.15269 | 0.1225 4 |

As predicted by the small scale high-throughput cultures, larger tank cultures for strains comprising the combined zwf promoter swap and SNP 121 exhibited significant increases in yield and productivity over the base reference strain. Productivity of this strain for example, jumped to 4.5 g/L/h compared to the 3.29 g/L/h productivity of the base strain (a 37.0% increase in productivity in only 2 rounds of SNP Swap).

**Example 4: HTP Genomic Engineering - Implementation of a Promoter Swap Library to Improve an Industrial Microbial Strain**

Previous examples have demonstrated the power of the HTP strain improvement programs of the present disclosure for rehabilitating industrial strains. Examples 2 and 3 described the implementation of SNP swap techniques and libraries exploring the existing genetic diversity within various base, intermediate, and industrial strains

This example illustrates examples of the HTP strain improvement programs using the PRO swap techniques of the present disclosure. Unlike Example 3, this example teaches methods for the *de-novo* generation of mutations via PRO swap library generation.

**A. Identification of a Target for Promoter Swapping**

As aforementioned, promoter swapping is a multi-step process that comprises a step of: Selecting a set of "n" genes to target.

In this example, the inventors have identified a group of 23 potential pathway genes to modulate via the promoter ladder methods of the present disclosure (19 genes to overexpress and 4+ diverting genes to downregulate, in an exemplary metabolic pathway producing the molecule lysine). (*See,* Figure 19).

**B. Creation of Promoter Ladder**

Another step in the implementation of a promoter swap process is the selection of a set of "x" promoters to act as a "ladder". Ideally these promoters have been shown to lead to highly variable expression across multiple genomic loci, but the only requirement is that they perturb gene expression in some way.

These promoter ladders, in particular examples, are created by: identifying natural, native, or wild-type promoters associated with the target gene of interest and then mutating said promoter to derive multiple mutated promoter sequences. Each of these mutated promoters is tested for effect on target gene expression. In some examples, the edited promoters are tested for expression activity across a variety of conditions, such that each promoter variant's activity is documented/characterized/annotated and stored in a database. The resulting edited promoter variants are subsequently organized into "ladders" arranged based on the strength of their expression (*e.g.,* with highly expressing variants near the top, and attenuated expression near the bottom, therefore leading to the term "ladder").

In the present exemplary example, the inventors have created promoter ladder:ORF combinations for each of the target genes identified in Figure 19.

**C. Associating Promoters from the Ladder with Target Genes**

Another step in the implementation of a promoter swap process is the HTP engineering of various strains that comprise a given promoter from the promoter ladder associated with a particular target gene.

If a native promoter exists in front of target gene n and its sequence is known, then replacement of the native promoter with each of the x promoters in the ladder can be carried out. When the native promoter does not exist or its sequence is unknown, then insertion of each of the x promoters in the ladder in front of gene n can be carried out. In this way a library of strains is constructed, wherein each member of the library is an instance of x promoter operably linked to n target, in an otherwise identical genetic context (*see e.g.,* Figure 20).

**D. HTP Screening of the Strains**

A final step in the promoter swap process is the HTP screening of the strains in the aforementioned library. Each of the derived strains represents an instance of x promoter linked to n target, in an otherwise identical genetic background.

By implementing a HTP screening of each strain, in a scenario where their performance against one or more metrics is characterized, the inventors are able to determine what promoter/target gene association is most beneficial for a given metric (e.g. optimization of production of a molecule of interest). *See,* Figure 20 (promoters P1-P8 effect on gene of interest).

In the exemplary example illustrated in Figures 19-22, the inventors have utilized the promoter swap process to optimize the production of lysine. **An application of the Pro SWAP methods described above is described in Example 5, below.**

**Example 5: HTP Genomic Engineering - Implementation of a PRO Swap Library to Improve Strain Performance for Lysine Production.**

The section below provides an illustrative implementation of the PRO swap HTP design strain improvement program tools of the present disclosure, as described in Example 4. In this example, a *Corynebacterium* strain was subjected to the PRO swap methods of the present disclosure in order to increase host cell yield of lysine.

A. Promoter Swap

Promoter Swaps were conducted as described in Example 4. Selected genes from the Lysine biosynthetic pathway in Figure 19 were targeted for promoter swaps using promoters P1-P8.

B. HTP engineering and High-Throughput Screening

HTP engineering of the promoter swaps was conducted as described in Example 1 and 3. HTP screening of the resulting promoter swap strains was conducted as described in Example 3. In total 145 PRO swaps were conducted. The results of the experiment are summarized in Table 11 below, and are depicted in Figure 41.

**Table 11-** HTP Screening of Lysine PRO Swap Libraries

| Strain | promoter-target | N | Mean ($A_{560}$) | Std Error | % Yield Change From Base |
|---|---|---|---|---|---|
| 7000007713 | Pcg 1860-asd | 8 | 0.84595 | 0.00689 | 3.927615 |
| 7000007736 | Pcg0755-asd | 4 | 0.84036 | 0.00974 | 3.240866 |
| 7000007805 | Pcg0007_119-asd | 8 | 0.82493 | 0.00689 | 1.345242 |
| 7000007828 | Pcg3121-asd | 8 | 0.8246 | 0.00689 | 1.3047 |
| 7000007759 | Pcg0007_265-asd | 8 | 0.81155 | 0.00689 | -0.29853 |
| 7000007782 | Pcg3381-asd | 8 | 0.8102 | 0.00689 | -0.46438 |
| 7000007712 | Pcg 1860-ask | 8 | 0.83958 | 0.00689 | 3.14504 |
| 7000007735 | Pcg0755-ask | 8 | 0.81673 | 0.00689 | 0.337846 |
| 7000007827 | Pcg3121-ask | 8 | 0.81498 | 0.00689 | 0.122853 |
| 7000007804 | Pcg0007_119-ask | 8 | 0.81492 | 0.00689 | 0.115482 |
| 7000007758 | Pcg0007_265-ask | 8 | 0.80381 | 0.00689 | -1.24942 |
| 7000007781 | Pcg3381-ask | 8 | 0.80343 | 0.00689 | -1.2961 |
| 7000007780 | Pcg3381-aspB | 8 | 0.84072 | 0.00689 | 3.285093 |
| 7000007803 | Pcg0007_119-aspB | 8 | 0.82106 | 0.00689 | 0.8698 |

(continued)

| Strain | promoter-target | N | Mean ($A_{560}$) | Std Error | % Yield Change From Base |
|---|---|---|---|---|---|
| 7000007809 | Pcg0007_119-cg0931 | 8 | 0.83446 | 0.00689 | 2.516032 |
| 7000007717 | Pcg 1860-cg0931 | 4 | 0.83129 | 0.00974 | 2.126588 |
| 7000007763 | Pcg0007265-cg0931 | 4 | 0.82628 | 0.00974 | 1.511094 |
| 7000007671 | Pcg0007_39-cg0931 | 8 | 0.82554 | 0.00689 | 1.420182 |
| 7000007740 | Pcg0755-cg0931 | 8 | 0.81921 | 0.00689 | 0.642522 |
| 7000007694 | Pcg0007-cg0931 | 8 | 0.80444 | 0.00689 | -1.17202 |
| 7000007691 | Pcg0007-dapA | 8 | 0.8299 | 0.00689 | 1.955822 |
| 7000007783 | Pcg3381-dapA | 8 | 0.80951 | 0.00689 | -0.54915 |
| 7000007760 | Pcg0007_265-dapA | 8 | 0.76147 | 0.00689 | -6.45102 |
| 7000007806 | Pcg0007_119-dapA | 8 | 0.35394 | 0.00689 | -56.5174 |
| 7000007761 | Pcg0007_265-dapB | 8 | 0.84157 | 0.00689 | 3.389518 |
| 7000007738 | Pcg0755-dapB | 4 | 0.84082 | 0.00974 | 3.297378 |
| 7000007692 | Pcg0007-dapB | 8 | 0.83088 | 0.00689 | 2.076218 |
| 7000007784 | Pcg3381-dapB | 8 | 0.82474 | 0.00689 | 1.3219 |
| 7000007715 | Pcg 1860-dapB | 8 | 0.82232 | 0.00689 | 1.024595 |
| 7000007830 | Pcg3121-dapB | 8 | 0.81236 | 0.00689 | -0.19902 |
| 7000007807 | Pcg0007_119-dapB | 4 | 0.69622 | 0.00974 | -14.4672 |
| 7000007762 | Pcg0007_265-dapD | 8 | 0.84468 | 0.00689 | 3.771591 |
| 7000007808 | Pcg0007_119-dapD | 8 | 0.83869 | 0.00689 | 3.035701 |
| 7000007785 | Pcg3381-dapD | 8 | 0.83397 | 0.00689 | 2.455834 |
| 7000007670 | Pcg0007_39-dapD | 8 | 0.81698 | 0.00689 | 0.368559 |
| 7000007831 | Pcg3121-dapD | 4 | 0.8155 | 0.00974 | 0.186737 |
| 7000007693 | Pcg0007-dapD | 8 | 0.8117 | 0.00689 | -0.28011 |
| 7000007716 | Pcg 1860-dapD | 8 | 0.79044 | 0.00689 | -2.89196 |
| 7000007739 | Pcg0755-dapD | 8 | 0.78694 | 0.00689 | -3.32195 |
| 7000007787 | Pcg3381-dapE | 8 | 0.83814 | 0.00689 | 2.968132 |
| 7000007833 | Pcg3121-dapE | 8 | 0.83721 | 0.00689 | 2.853878 |
| 7000007741 | Pcg0755-dapE | 8 | 0.83263 | 0.00689 | 2.291211 |
| 7000007810 | Pcg0007_119-dapE | 8 | 0.83169 | 0.00689 | 2.175729 |
| 7000007718 | Pcg1860-dapE | 8 | 0.81855 | 0.00689 | 0.561439 |
| 7000007672 | Pcg0007_39-dapE | 8 | 0.80932 | 0.00689 | -0.5725 |
| 7000007765 | Pcg0007265-dapF | 8 | 0.8327 | 0.00689 | 2.299811 |
| 7000007788 | Pcg3381-dapF | 8 | 0.82942 | 0.00689 | 1.896853 |
| 7000007811 | Pcg0007_119-dapF | 8 | 0.82926 | 0.00689 | 1.877196 |
| 7000007696 | Pcg0007-dapF | 8 | 0.82099 | 0.00689 | 0.861201 |
| 7000007719 | Pcg 1860-dapF | 8 | 0.82067 | 0.00689 | 0.821888 |
| 7000007673 | Pcg0007_39-dapF | 8 | 0.82062 | 0.00689 | 0.815745 |
| 7000007789 | Pcg3381-ddh | 8 | 0.84817 | 0.00689 | 4.200349 |

(continued)

| Strain | promoter-target | N | Mean ($A_{560}$) | Std Error | % Yield Change From Base |
|---|---|---|---|---|---|
| 7000007835 | Pcg3121-ddh | 8 | 0.82141 | 0.00689 | 0.912799 |
| 7000007812 | Pcg0007_119-ddh | 8 | 0.82093 | 0.00689 | 0.853829 |
| 7000007674 | Pcg0007_39-ddh | 8 | 0.81494 | 0.00689 | 0.117939 |
| 7000007720 | Pcg 1860-ddh | 8 | 0.81473 | 0.00689 | 0.09214 |
| 7000007766 | Pcg0007265-ddh | 8 | 0.81427 | 0.00689 | 0.035627 |
| 7000007743 | Pcg0755-ddh | 8 | 0.80655 | 0.00689 | -0.9128 |
| 7000007697 | Pcg0007-ddh | 8 | 0.80621 | 0.00689 | -0.95457 |
| 7000007779 | Pcg3381-fbp | 8 | 0.85321 | 0.00689 | 4.819529 |
| 7000007802 | Pcg0007_119-fbp | 4 | 0.81425 | 0.00974 | 0.03317 |
| 7000007710 | Pcg1860-fbp | 4 | 0.40253 | 0.00974 | -50.5479 |
| 7000007687 | Pcg0007-fbp | 8 | 0.14881 | 0.00689 | -81.7182 |
| 7000007825 | Pcg3121-fbp | 4 | 0.12471 | 0.00974 | -84.679 |
| 7000007733 | Pcg0755-fbp | 4 | 0.08217 | 0.00974 | -89.9052 |
| 7000007746 | Pcg0755-hom | 8 | 0.81925 | 0.00689 | 0.647436 |
| 7000007792 | Pcg3381-hom | 4 | 0.77674 | 0.00974 | -4.57505 |
| 7000007723 | Pcg1860-hom | 8 | 0.71034 | 0.00689 | -12.7325 |
| 7000007838 | Pcg3121-hom | 8 | 0.559 | 0.00689 | -31.3251 |
| 7000007800 | Pcg0007_119-icd | 8 | 0.83236 | 0.00689 | 2.258041 |
| 7000007823 | Pcg3121-icd | 8 | 0.83155 | 0.00689 | 2.15853 |
| 7000007777 | Pcg3381-icd | 8 | 0.82844 | 0.00689 | 1.776456 |
| 7000007708 | Pcg1860-icd | 8 | 0.82384 | 0.00689 | 1.211332 |
| 7000007662 | Pcg0007_39-icd | 12 | 0.82008 | 0.00562 | 0.749404 |
| 7000007685 | Pcg0007-icd | 8 | 0.81257 | 0.00689 | -0.17322 |
| 7000007754 | Pcg0007_265-icd | 4 | 0.81172 | 0.00974 | -0.27765 |
| 7000007698 | Pcg0007-lysA | 4 | 0.8504 | 0.00974 | 4.474311 |
| 7000007675 | Pcg0007_39-lysA | 8 | 0.84414 | 0.00689 | 3.705251 |
| 7000007836 | Pcg3121-lysA | 4 | 0.83545 | 0.00974 | 2.637657 |
| 7000007767 | Pcg0007_265-lysA | 8 | 0.83249 | 0.00689 | 2.274012 |
| 7000007813 | Pcg0007_119-lysA | 8 | 0.83096 | 0.00689 | 2.086046 |
| 7000007790 | Pcg3381-lysA | 8 | 0.8118 | 0.00689 | -0.26782 |
| 7000007676 | Pcg0007 _39-lysE | 8 | 0.84394 | 0.00689 | 3.68068 |
| 7000007699 | Pcg0007-lysE | 4 | 0.83393 | 0.00974 | 2.45092 |
| 7000007768 | Pcg0007_265-lysE | 8 | 0.83338 | 0.00689 | 2.383351 |
| 7000007837 | Pcg3121-lysE | 4 | 0.83199 | 0.00974 | 2.212585 |
| 7000007791 | Pcg3381-lysE | 8 | 0.81476 | 0.00689 | 0.095825 |
| 7000007814 | Pcg0007_119-lysE | 8 | 0.81315 | 0.00689 | -0.10197 |
| 7000007775 | Pcg3381-odx | 8 | 0.82237 | 0.00689 | 1.030738 |
| 7000007752 | Pcg0007265-odx | 8 | 0.81118 | 0.00689 | -0.34399 |

(continued)

| Strain | promoter-target | N | Mean ($A_{560}$) | Std Error | % Yield Change From Base |
|---|---|---|---|---|---|
| 7000007729 | Pcg0755-odx | 8 | 0.81103 | 0.00689 | -0.36242 |
| 7000007683 | Pcg0007-odx | 8 | 0.80507 | 0.00689 | -1.09462 |
| 7000007706 | Pcg 1860-odx | 4 | 0.79332 | 0.00974 | -2.53815 |
| 7000007660 | Pcg0007_39-odx | 8 | 0.79149 | 0.00689 | -2.76297 |
| 7000007798 | Pcg0007_119-odx | 8 | 0.77075 | 0.00689 | -5.31094 |
| 7000007821 | Pcg3121-odx | 4 | 0.74788 | 0.00974 | -8.12059 |
| 7000007822 | Pcg3121-pck | 8 | 0.85544 | 0.00689 | 5.093491 |
| 7000007776 | Pcg3381-pck | 8 | 0.8419 | 0.00689 | 3.43006 |
| 7000007799 | Pcg0007_119-pck | 8 | 0.83851 | 0.00689 | 3.013588 |
| 7000007753 | Pcg0007_265-pck | 8 | 0.82738 | 0.00689 | 1.646232 |
| 7000007730 | Pcg0755-pck | 4 | 0.81785 | 0.00974 | 0.475442 |
| 7000007661 | Pcg0007 _39-pck | 8 | 0.80976 | 0.00689 | -0.51844 |
| 7000007684 | Pcg0007-pck | 8 | 0.79007 | 0.00689 | -2.93742 |
| 7000007707 | Pcg1860-pck | 8 | 0.71566 | 0.00689 | -12.0789 |
| 7000007840 | Pcg3121-pgi | 4 | 1.01046 | 0.00974 | 24.13819 |
| 7000007817 | Pcg0007 _119-pgi | 7 | 0.99238 | 0.00736 | 21.917 |
| 7000007794 | Pcg3381-pgi | 7 | 0.99008 | 0.00736 | 21.63444 |
| 7000007771 | Pcg0007 _265-pgi | 8 | 0.94665 | 0.00689 | 16.29893 |
| 7000007725 | Pcg1860-pgi | 8 | 0.85515 | 0.00689 | 5.057864 |
| 7000007702 | Pcg0007-pgi | 4 | 0.8056 | 0.00974 | -1.02951 |
| 7000007658 | Pcg0007 _39-ppc | 4 | 0.85221 | 0.00974 | 4.696676 |
| 7000007750 | Pcg0007265-ppc | 8 | 0.84486 | 0.00689 | 3.793705 |
| 7000007727 | Pcg0755-ppc | 8 | 0.84166 | 0.00689 | 3.400575 |
| 7000007773 | Pcg3381-ppc | 4 | 0.82883 | 0.00974 | 1.824369 |
| 7000007796 | Pcg0007_119-ppc | 8 | 0.82433 | 0.00689 | 1.27153 |
| 7000007704 | Pcg1860-ppc | 8 | 0.81736 | 0.00689 | 0.415244 |
| 7000007819 | Pcg3121-ppc | 8 | 0.79898 | 0.00689 | -1.8428 |
| 7000007732 | Pcg0755-ptsG | 8 | 0.84055 | 0.00689 | 3.264208 |
| 7000007709 | Pcg1860-ptsG | 8 | 0.81075 | 0.00689 | -0.39682 |
| 7000007663 | Pcg0007_39-ptsG | 8 | 0.80065 | 0.00689 | -1.63763 |
| 7000007778 | Pcg3381-ptsG | 8 | 0.23419 | 0.00689 | -71.229 |
| 7000007801 | Pcg0007_119-ptsG | 8 | 0.17295 | 0.00689 | -78.7525 |
| 7000007824 | Pcg3121-ptsG | 8 | 0.16035 | 0.00689 | -80.3005 |
| 7000007705 | Pcg1860-pyc | 8 | 0.85143 | 0.00689 | 4.60085 |
| 7000007728 | Pcg0755-pyc | 8 | 0.79803 | 0.00689 | -1.95951 |
| 7000007659 | Pcg0007_39-pyc | 8 | 0.75539 | 0.00689 | -7.19797 |
| 7000007751 | Pcg0007_265-pyc | 8 | 0.73664 | 0.00689 | -9.50146 |
| 7000007682 | Pcg0007-pyc | 4 | 0.73142 | 0.00974 | -10.1428 |

(continued)

| Strain | promoter-target | N | Mean ($A_{560}$) | Std Error | % Yield Change From Base |
|---|---|---|---|---|---|
| 7000007774 | Pcg3381-pyc | 4 | 0.66667 | 0.00974 | -18.0975 |
| 7000007797 | Pcg0007_119-pyc | 4 | 0.52498 | 0.00974 | -35.5046 |
| 7000007820 | Pcg3121-pyc | 8 | 0.52235 | 0.00689 | -35.8277 |
| 7000007841 | Pcg3121-tkt | 8 | 0.82565 | 0.00689 | 1.433696 |
| 7000007818 | Pcg0007_1 19-tkt | 8 | 0.81674 | 0.00689 | 0.339075 |
| 7000007749 | Pcg0755-tkt | 8 | 0.81496 | 0.00689 | 0.120396 |
| 7000007703 | Pcg0007-tkt | 4 | 0.76763 | 0.00974 | -5.69424 |
| 7000007795 | Pcg3381-tkt | 8 | 0.72213 | 0.00689 | -11.2841 |
| 7000007772 | Pcg0007_265-tkt | 8 | 0.68884 | 0.00689 | -15.3738 |
| 7000007701 | Pcg0007-zwf | 4 | 0.95061 | 0.00974 | 16.78542 |
| 7000007747 | Pcg0755-zwf | 8 | 0.92595 | 0.00689 | 13.75587 |
| 7000007770 | Pcg0007265-zwf | 8 | 0.9029 | 0.00689 | 10.9241 |
| 7000007724 | Pcg1860-zwf | 8 | 0.79309 | 0.00689 | -2.5664 |
| 7000007839 | Pcg3121-zwf | 4 | 0.13379 | 0.00974 | -83.5635 |
| 7000000017 | - | 11 6 | 0.92115 | 0.00181 | 13.16617 |
| 7000006284 | - | 12 8 | 0.81398 | 0.00172 | 0 |
| 7000005754 | - | 64 | 0.79489 | 0.00243 | -2.34527 |

When visualized, the results of the promoter swap library screening serve to identify gene targets that are most closely correlated with the performance metric being measured. In this case, gene targets pgi, zwf, ppc, pck, fbp, and ddh were identified as genes for which promoter swaps produce large gains in yield over base strains.

Selected strains from Table 11 were re-cultured in small plates and tested for lysine yield as describe above. The results from this secondary screening are provided in Figure 22.

**Example 6: Epistasis Mapping- An Algorithmic Tool for Predicting Beneficial Mutation Consolidations**

This example describes an example of the predictive modeling techniques utilized as part of the HTP strain improvement program of the present disclosure. After an initial identification of potentially beneficial mutations (through the use of genetic design libraries as described above), the present disclosure teaches methods of consolidating beneficial mutations in second, third, fourth, and additional subsequent rounds of HTP strain improvement. In some examples, the present disclosure teaches that mutation consolidations may be based on the individual performance of each of said mutations. In other examples, the present disclosure teaches methods for predicting the likelihood that two or more mutations will exhibit additive or synergistic effects if consolidated into a single host cell. The example below illustrates an example of the predicting tools of the present disclosure.

Selected mutations from the SNP swap and promoter swapping (PRO swap) libraries of Examples 3 and 5 were analyzed to identify SNP/PRO swap combinations that would be most likely to lead to strain host performance improvements.

SNP swapping library sequences were compared to each other using a cosine similarity matrix, as described in the "Epistasis Mapping" section of the present disclosure. The results of the analysis yielded functional similarity scores for each SNP/ PRO swap combination. A visual representation of the functional similarities among all SNPs/ PRO swaps is depicted in a heat map in Figure 15. The resulting functional similarity scores were also used to develop a dendrogram depicting the similarity distance between each of the SNPs/PRO swaps (Figure 16A).

Mutations from the same or similar functional group (*i.e.,* SNPs/PRO swaps with high functional similarity) are more likely to operate by the same mechanism, and are thus more likely to exhibit negative or neutral epistasis on overall host performance when combined. In contrast, mutations from different functional groups would be more likely to operate by independent mechanisms, and thus more likely to produce beneficial additive or combinatorial effects on host performance.

In order to illustrate the effects of biological pathways on epistasis, SNPs and PRO swaps exhibiting various functional similarities were combined and tested on host strains. Three SNP/PRO swap combinations were engineered into the genome of *Corynebacterium glutamicum* as described in Example 1: **i**) Pcg0007::zwf PRO swap + Pcg1860::pyc PRO swap, **ii**) Pcg0007::zwf PRO swap + SNP 309, and **iv**) Pcg0007::zwf PRO swap + Pcg0007::lysA PRO swap (see Figure 15 and 16A for functional similarity relationships).

The performance of each of the host cells containing the SNP/PRO swap combinations was tested as described in Example 3, and was compared to that of a control host cell containing only zwf PRO swap. Tables 12 and 13 below summarize the results of host cell yield (96hr measurements) and productivity (24hr measurements) of each of the strains.

**Table 12-** Lysine Accumulation for Epistasis Mapping Experiment at 24 hours.

| SNP/ PRO swap | Mean Lysine ($A_{560}$) | StDev |
|---|---|---|
| 6318 (zwf) | 0.51 | 0.03 |
| 8126 (zwf+ lysA) | 0.88 | 0.06 |
| 8156 (zwf+pyc) | 0.53 | 0.01 |
| 8708 (zwf+ SNP 309) | 0.56 | 0.00 |

**Table 13-** Lysine Accumulation for Epistasis Mapping Experiment at 96 hours.

| SNP/ PRO swap | Mean Lysine ($A_{560}$) | StDev |
|---|---|---|
| 6318 (zwf) | 0.83 | 0.01 |
| 8126 (zwf + lysA) | 0.94 | 0.02 |
| 8156 (zwf+ pyc) | 0.83 | 0.06 |

Host yield performance results for each SNP/PRO swap combination are also depicted in Figure 16B. Host strains combining SNPs/PRO swaps exhibiting lower functional similarity outperformed strains in which the combined SNPs had exhibited higher functional similarity at both 24, and 96 hour measurements.

Thus, the epistatic mapping procedure is useful for predicting/programming/informing effective and/or positive consolidations of designed genetic changes. The analytical insight from the epistatic mapping procedure allows for the creation of predictive rule sets that can guide subsequent rounds of microbial strain development. The predictive insight gained from the epistatic library may be used across microbial types and target molecule types.

**Example 7: HTP Genomic Engineering -Pro Swap Mutation Consolidation and Multi-Factor Combinatorial Testing**

Previous examples have illustrated methods for consolidating a small number of preselected PRO swap mutations with SNP swap libraries (Example 3). Other examples have illustrated the epistatic methods for selecting mutation consolidations that are most likely to yield additive or synergistic beneficial host cell properties (Example 6). This example illustrates the ability of the HTP methods of the present disclosure to effectively explore the large solution space created by the combinatorial consolidation of multiple gene/genetic design library combinations (e.g., PRO swap library x SNP Library or combinations within a PRO swap library).

In this illustrative application of the HTP strain improvement methods of the present disclosure, promoter swaps identified as having a positive effect on host performance in Example 5 are consolidated in second order combinations with the original PRO swap library. The decision to consolidate PRO swap mutations was based on each mutation's overall effect on yield or productivity, and the likelihood that the combination of the two mutations would produce an additive or synergistic effect.

For example, applicants refer to their choice of combining Pcg0007::zwf and Pcg0007:: lysA, based on the epistasis mapping results of Example 6.

**A. Consolidation Round for PRO Swap Strain Engineering**

Strains were transformed as described in previous Example 1. Briefly, strains already containing one desired PRO swap mutation were once again transformed with the second desired PRO swap mutation. In total, the 145 tested PRO swaps from Example 5 were consolidated into 53 second round consolidation strains, each comprising two PRO swap mutations expected to exhibit beneficial additive or synergistic effects.

The resulting second round strains were once again screened as described in Example 3. Results from this experiment are summarized in Table 14 below, and depicted in Figure 11.

**Table 14-** HTP Screening of Second Round Consolidated Lysine PRO Swap Libraries

| Strain ID | Number | PRO Swap 1 | PRO Swap 2 | Mean Yield ($A_{560}$) | Std Dev |
|---|---|---|---|---|---|
| 7000008489 | 4 | Pcg0007-lysA | Pcg3121-pgi | 1.17333 | 0.020121 |
| 7000008530 | 8 | Pcg1860-pyc | Pcg0007-zwf | 1.13144 | 0.030023 |
| 7000008491 | 7 | Pcg0007-lysA | Pcg0007-zwf | 1.09836 | 0.028609 |
| 7000008504 | 8 | Pcg3121-pck | Pcg0007-zwf | 1.09832 | 0.021939 |
| 7000008517 | 8 | Pcg000739-ppc | Pcg0007-zwf | 1.09502 | 0.030777 |
| 7000008502 | 4 | Pcg3121-pck | Pcg3121-pgi | 1.09366 | 0.075854 |
| 7000008478 | 4 | Pcg3381-ddh | Pcg0007-zwf | 1.08893 | 0.025505 |
| 7000008465 | 4 | Pcg0007 265-dapB | Pcg0007-zwf | 1.08617 | 0.025231 |
| 7000008535 | 8 | Pcg0007-zwf | Pcg3121-pgi | 1.06261 | 0.019757 |
| 7000008476 | 6 | Pcg3381-ddh | Pcg3121-pgi | 1.04808 | 0.084307 |
| 7000008510 | 8 | Pcg3121-pgi | Pcg 1860-pyc | 1.04112 | 0.021087 |
| 7000008525 | 8 | Pcg1860-pyc | Pcg0007 265-dapB | 1.0319 | 0.034045 |
| 7000008527 | 8 | Pcg1860-pyc | Pcg0007-lysA | 1.02278 | 0.043549 |
| 7000008452 | 5 | Pcg1860-asd | Pcg0007-zwf | 1.02029 | 0.051663 |
| 7000008463 | 4 | Pcg0007_265-dapB | Pcg3121-pgi | 1.00511 | 0.031604 |
| 7000008524 | 8 | Pcg1860-pyc | Pcg1860-asd | 1.00092 | 0.026355 |
| 7000008458 | 4 | Pcg3381-aspB | Pcg1860-pyc | 1.00043 | 0.020083 |
| 7000008484 | 8 | Pcg3381-fbp | Pcg1860-pyc | 0.99686 | 0.061364 |
| 7000008474 | 8 | Pcg3381-ddh | Pcg3381-fbp | 0.99628 | 0.019733 |
| 7000008522 | 8 | Pcg0755-ptsG | Pcg3121-pgi | 0.99298 | 0.066021 |
| 7000008528 | 8 | Pcg1860-pyc | Pcg3121-pck | 0.99129 | 0.021561 |
| 7000008450 | 4 | Pcg1860-asd | Pcg3121-pgi | 0.98262 | 0.003107 |
| 7000008448 | 8 | Pcg1860-asd | Pcg3381-fbp | 0.97814 | 0.022285 |
| 7000008494 | 8 | Pcg000739-lysE | Pcg3381-fbp | 0.97407 | 0.027018 |
| 7000008481 | 8 | Pcg3381-fbp | Pcg0007-lysA | 0.9694 | 0.029315 |
| 7000008497 | 8 | Pcg0007_39-lysE | Pcg 1860-pyc | 0.9678 | 0.028569 |
| 7000008507 | 8 | Pcg3121-pgi | Pcg3381-fbp | 0.96358 | 0.035078 |
| 7000008501 | 8 | Pcg3121-pck | Pcg0007-lysA | 0.96144 | 0.018665 |
| 7000008486 | 8 | Pcg0007-lysA | Pcg0007_265-dapB | 0.94523 | 0.017578 |
| 7000008459 | 8 | Pcg0007_265-dapB | Pcg1860-asd | 0.94462 | 0.023847 |
| 7000008506 | 2 | Pcg3121-pgi | Pcg0007_265-dapD | 0.94345 | 0.014014 |
| 7000008487 | 8 | Pcg0007-lysA | Pcg3381-ddh | 0.94249 | 0.009684 |
| 7000008498 | 8 | Pcg3121-pck | Pcg1860-asd | 0.94154 | 0.016802 |
| 7000008485 | 8 | Pcg0007-lysA | Pcg1860-asd | 0.94135 | 0.013578 |
| 7000008499 | 8 | Pcg3121-pck | Pcg0007_265-dapB | 0.93805 | 0.013317 |
| 7000008472 | 8 | Pcg3381-ddh | Pcg1860-asd | 0.93716 | 0.012472 |

(continued)

| Strain ID | Number | PRO Swap 1 | PRO Swap 2 | Mean Yield ($A_{560}$) | Std Dev |
|---|---|---|---|---|---|
| 7000008511 | 8 | Pcg000739-ppc | Pcg1860-asd | 0.93673 | 0.015697 |
| 7000008514 | 8 | Pcg000739-ppc | Pcg0007-lysA | 0.93668 | 0.027204 |
| 7000008473 | 8 | Pcg3381-ddh | Pcg0007_265-dapB | 0.93582 | 0.030377 |
| 7000008461 | 7 | Pcg0007_265-dapB | Pcg3381-fbp | 0.93498 | 0.037862 |
| 7000008512 | 8 | Pcg0007_39-ppc | Pcg0007_265-dapB | 0.93033 | 0.017521 |
| 7000008456 | 8 | Pcg3381-aspB | Pcg3121-pck | 0.92544 | 0.020075 |
| 7000008460 | 8 | Pcg0007_265-dapB | Pcg0007_265-dapD | 0.91723 | 0.009508 |
| 7000008492 | 8 | Pcg0007_39-lysE | Pcg3381-aspB | 0.91165 | 0.012988 |
| 7000008493 | 8 | Pcg0007_39-lysE | Pcg0007_265-dapD | 0.90609 | 0.031968 |
| 7000008453 | 8 | Pcg3381-aspB | Pcg0007_265-dapB | 0.90338 | 0.013228 |
| 7000008447 | 8 | Pcg1860-asd | Pcg0007_265-dapD | 0.89886 | 0.028896 |
| 7000008455 | 8 | Pcg3381-aspB | Pcg0007-lysA | 0.89531 | 0.027108 |
| 7000008454 | 6 | Pcg3381-aspB | Pcg3381-ddh | 0.87816 | 0.025807 |
| 7000008523 | 8 | Pcg0755-ptsG | Pcg 1860-pyc | 0.87693 | 0.030322 |
| 7000008520 | 8 | Pcg0755-ptsG | Pcg3381-fbp | 0.87656 | 0.018452 |
| 7000008533 | 4 | Pcg0007-zwf | Pcg3381-fbp | 0.84584 | 0.017012 |
| 7000008519 | 8 | Pcg0755-ptsG | Pcg0007_265-dapD | 0.84196 | 0.025747 |

As predicted by the epistasis model, the second round PRO swap strain comprising the Pcg0007:: zwf and Pcg0007:: lysA mutations exhibited one of the highest yield improvements, with a nearly 30% improvement in yield over Pcg0007::lysA alone, and a 35.5% improvement over the base strain (see circled data point on Figure 11).

The HTP methods for exploring solution space of single and double consolidated mutations, can also be applied to third, fourth, and subsequent mutation consolidations. Attention is also drawn, for example, to the disclosed 3-change consolidation strain corresponding to zwf, pyc, and lysa that was made from amongst the top hits of identified in the 2 change consolidations as shown in Table 14 above, and as identified by the epistatic methods of the present disclosure. This 3-change consolidation strain was further validated in tanks as being significantly improved as compared to the parent or parent + zwf (*see* Table 10 supra, and Figure 40).

**Example 8: HTP Genomic Engineering** - **Implementation of a Terminator Library to Improve an Industrial Host Strain**

The present example applies the HTP methods of the present disclosure to additional HTP genetic design libraries, including STOP swap. The example further illustrates the ability of the present disclosure to combine elements from basic genetic design libraries (e.g., PRO swap, SNP swap, STOP swap, etc.,) to create more complex genetic design libraries (e.g., PRO-STOP swap libraries, incorporating both a promoter and a terminator). In some examples, the present disclosure teaches any and all possible genetic design libraries, including those derived from combining any of the previously disclosed genetic design libraries.

In this example, a small scale experiment was conducted to demonstrate the effect of the STOP swap methods of the present disclosure on gene expression. Terminators T1-T8 of the present disclosure were paired with one of two native *Corynebacterium glutamicum* promoters as described below, and were analyzed for their ability to impact expression of a fluorescent protein.

***A. Assembly of DNA constructs***

Terminators T1-T8 were paired with one of two native *Corynebacterium glutamicum* promoters (e.g., Pcg0007 or Pcg0047) expressing a yellow fluorescence protein (YFP). To facilitate DNA amplification and assembly, the final promoter-YFP-terminator sequence was synthesized in two portions; the first portion encoded (from 5' to 3') **i**) the vector homology arm, **ii**) the selected promoter, **iii**) and 2/3 of the YFP gene. The second portion encoded (from 5' to 3') **iv**) the next 2/3 of the YFP gene, **v**) the selected terminator, and **vi**) the second vector homology arm. Each portion was amplified using synthetic oligonucleotides and gel purified. Gel purified amplicons were assembled with a vector backbone using yeast homologous recombination.

### *B. Transformation of Assembled Clones into E. coli*

Vectors containing the Promoter-YFP-terminator sequences were each individually transformed into *E. coli* in order to identify correctly assembled clones, and to amplify vector DNA for *Corynebacterium* transformation. Correctly assembled vectors were confirmed by restriction enzyme digest and Sanger sequencing. Positive clones were stored at -20°C for future use.

### *C. Transformation of Assembled Clones into Corynebacterium*

Verified vector clones were individually transformed into *Corynebacterium glutamicum* host cells via electroporation. Each vector was designed to integrate into a neutral integration site within the *Corynebacterium glutamicum* genome that was empirically determined to permit expression of heterologous yellow fluorescence protein but not be detrimental to the host cell. To facilitate integration, the expression vector further comprised about 2 kbp of sequence homologous (i.e., homology arm) to the desired integration site whereby each gene cassette described above was inserted downstream of the homology am. Integration into the genome occurred by single-crossover integration. Transformed *Corynebacterium* were then tested for correct integration via PCR. This process was repeated for each of the transformations conducted for each gene construct.

### *D. Evaluation of Individual terminator constructs in Corynebacterium*

The phenotype of each *Corynebacterium* transformant containing promoter-YFP-terminator constructs was then tested in two media types (brain heart infusion-BHI and HTP test media) at two time points in order to evaluate expression. Briefly, between four and six PCR-confirmed transformants were chosen and cultivated in selective media in a 96-well format. The initial cultures were then split into selective BHI media or selective seed media. At 48 hours, cultures in seed media were inoculated into selective HTP test media or BHI media and analyzed at two time points representing different portions of the growth curve. Time points for HTP test media cultures were 48 and 96 hours after inoculation. Cultures in the selective BHI media were analyzed at 48 and 72 hours after inoculation.

Analysis of the cultures was performed using a benchtop flow cytometer. Briefly, cultures were diluted 1:100 in 200 μl of phosphate buffered saline (PBS). For each culture, between 3000 and 5000 individual events (i.e., cells) were analyzed for yellow fluorescence. The benchtop flow cytometer plots a histogram of yellow fluorescence of each "event" and calculates the median fluorescence within each well. Figure 36 depicts the mean of the median fluorescence for each construct (across the 4-6 biological replicates). Error bars indicate the 95% confidence interval of each data point. Conditions A-D each refer to a single media and a single time point. Thus conditions A and B represent the two time points for the BHI media, while the C and D points represent the two time points for the HTP test media. Note that the arbitrary units (e.g., AU) represent the median fluorescence recorded by the benchtop flow cytometer.

The results show that terminators 1-8 of the STOP swap genetic design library result in a continuous range of YFP expression. These terminators thus form a terminator ladder that can be implemented into future genetic design libraries, according to the HTP methods of the present disclosure.

## Example 9: Comparing HTP Toolsets vs. Traditional UV Mutations.

This example demonstrates the benefits of the HTP genetic design libraries of the present disclosure over traditional mutational strain improvement programs. The experiments in this portion of the specification quantify the improved magnitude and speed of the phenotypical improvements achieved through the HTP methods of the present disclosure over traditional UV mutagenesis.

The present disclosure teaches new methods for accelerating the strain improvement programs of host cells. In some examples, the HTP strain improvement program of the present disclosure relies on the ability of the HTP toolsets to generate and identify genetic perturbations. The present inventors attempted to quantify the benefits of the HTP tool sets by conducting a small parallel track strain improvement program comparing the promoter swap techniques of the present disclosure against traditional UV mutations approaches.

A base reference strain producing a biochemical metabolite of interest was chosen as the starting point for both UV and promoter swap genetic perturbations.

A. UV mutations

Cultures of the base strain were grown in BHI medium in cultures that were OD normalized to $OD_{600}$ of 10. This culture was aliquoted into a sterile petri dish and agitated using a small magnetic stirrer bar. A UV trans illuminator at 254 nm wavelength was then inverted over the culture and aliquots taken at 5 and 9 minutes of UV exposure. These samples were serially diluted 10-fold and each dilution plated onto BHI medium Q-trays. From these Q-trays, approximately 2500 colonies from each UV exposure point were picked using an automated colony picking apparatus and the performance evaluated as below.

B. Promoter Swap

PRO swap constructs were generated in the base strain for 15 gene targets using either all or a subset of promoters selected from P1, P3, P4 and P8 described in Table 1. The final step in the biosynthesis of the product of interest is catalyzed by an O-methyltransferase enzyme that utilizes the potentially rate limiting cofactor S-adenosylmethionine. Gene targets for PRO swaps were therefore selected on the basis that they are directly involved in the biosynthesis of this cofactor or upstream metabolites.

C. UV and Promoter Swap Library Evaluation

The phenotype of each *Corynebacterium* strain developed for this example was tested for its ability to produce a selected biomolecule. Briefly, between four and six sequence confirmed colonies from each PRO swap strain, and single colonies for each UV strain were chosen and propagated in selective media in a 96-well format in production liquid media.

After biomass propagation in 96-well microwell plates, cell mass was added to fermentation media containing substrate in 96-well microwell plates and bioconversion was allowed to proceed for 24 hrs. Titers of product were determined for each strain using high-performance liquid chromatography from samples taken at 24 hrs. The titer results for each genetic perturbation (UV and PRO swap) was analyzed. Results for each replicate was averaged and assigned to represent the overall performance of said strain. Strains were then binned into categories based on each mutation's effect on measured yield expressed as a ratio over the yield of the base strain.

Figure 37 summarizes the results of this experiment, which are presented as the number of strains for each strain improvement technique that produced: i) no change in yield, ii) a 1.2 to 1.4 fold improvement to yield, iii) a 1.4 to 1.6 fold improvement to yield, iv) a 1.6 to 1.8 fold improvement to yield, or v) a 1.8 to 2 fold improvement to yield.

The results are illustrative of the benefits of the HTP toolsets of the present disclosure over traditional UV mutagenesis approaches. For example, the results of Figure 37 demonstrate that the PRO swap strains exhibited a higher rate of positive changes in yield, and were therefore more likely to provide mutations that could significantly improve the strain. Most striking, was the high incidence of high improvement strains showing 1.6, 1.8 and 2 fold increases in the PRO swap library, with little to no identified improvements in the UV library.

The results are also important because they highlight the accelerated rate of improvement of the PRO swap methods of the present disclosure. Indeed, results for the PRO swap library were based on less than 100 promoter::gene perturbations, whereas UV mutation results included the screening of over 4,000 distinct mutant strains. Thus the methods of the present disclosure drastically reduce the number of mutants that must be screened before identifying genetic perturbations capable of conferring strains with high gains in performance.

**Example 10 - HTP Genomic Engineering - Implementation of a Transposon Mutagenesis Library to Improve Strain Performance Escherichia coli**

Previous examples illustrate applications of HTP strain improvement programs on *Corynebacterium.* This example demonstrates the applicability of the same techniques to *E. coli* cells.

This example describes the application of transposon mutagenesis to generate *Escherichia coli* random strain libraries for the purpose of strain improvement. These strain libraries can be screened against a desired phenotype such as yield of tryptophan to identify variants with improved performance.

The present disclosure describes a method for generating a library of mutants through the application of the EZ-Tn5 transposon system (Epicenter Bio) in *Escherichia coli.* The EZ-Tn5 Transposase is incubated with payload DNA flanked by mosaic element sequences. Upon incubation, the Ez-Tn5 Transposase complexes with the DNA to form a transposome. The DNA/protein transposome complex is then transformed into *Escherichia coli* through electroporation. The EZ-Tn5 Transposase catalyzes the random integration of the payload DNA into the *Escherichia coli* genome thus giving rise to a random library of strain variants.

The specific sequence of the payload DNA can further be varied to bias toward either loss of function (LoF) or gain of function (GoF) effects of transposon insertion into the target genome.

LoF can be accomplished through inclusion of an antibiotic selection marker in the DNA payload. The antibiotic marker allows for the selection of cells with a productive transposon insertion. The insertion of the DNA payload may disrupt the function of DNA into which is has inserted in various ways including but not limited disruption of an open reading frame that prevents translation of the disrupted gene.

GoF can be accomplished through the inclusion of an antibiotic marker and a strong promoter in the DNA payload. The antibiotic marker allows for the selection of cells with a productive transposon insertion. The insertion of the DNA payload may increase the expression in genes proximal to the insertion site through the action of the strong promoter.

Either LoF of GoF DNA payloads may further contain a counterselection marker in addition to a selection marker to enable marker recycling and thus further rounds of engineering.

The library of strain variants generated through the transposon mutagenesis method described above can be screed against a desired phenotype. Stains can be cultivated and tested in high throughput to identify strains with an improved desired phenotype relative to the parent strain.

The improved strain variants can be subjected to additional rounds of cyclical engineering to further improve the desired phenotype such as yield of tryptophan. The additional rounds of engineering may consistent of transposon mutagenesis or other library types such as SNPSWP, PROSWP, or random mutagenesis. The improved strains may also be consolidated with other strain variants exhibiting an improved phenotype to produce a further improved strain through the additive effect of distinct beneficial mutations.

The methods described herein reduce the cost for building high quality libraries for screening in cyclical engineering. Application of transposon mutagenesis to *Escherichia coli* enables the production of thousands of genome wide of LoF or GoF mutants in a single reaction. An alternative method is to construct thousands of assigned plasmids to engineer strains through single crossover homologous recombination (SCHR). Another alternative method is to construct thousands of assigned linear fragments to engineer strains through lambda red recombineering. Both methods are costly because they require generating a unique DNA fragments for each mutant that contains the intended payload DNA and sequence homology that directs recombination to a specific location on the target genome. Transposon mutagenesis uses a single DNA payload and diversity is generated through random integration into the target genome.

### Example 11- HTP Genomic Engineering - Generation of Vector Backbones for use in HTP Genomic Engineering in *Escherichia coli*

This example describes the generation of vectors for use in the HTP genomic engineering for recombineering in *Escherichia coli* such that said vectors confer efficient transformation and plasmid integration.

Vector 1 (nucleic acid SEQ ID NO. 214) was generated and comprises an R6K origin of replication, a SacB gene, a PheS gene as a counterselection marker and a URA3 yeast auxotrophic marker. In order to improve the efficiency and single-crossover homologous recombination, the backbone of vector 1 was modified to contain the elements in Table 15, which resulted in vector 2 (nucleic acid SEQ ID NO. 215). The plasmid map shown in Figure 55 shows the general components of vector 1. In vector 2, random insulator sequences Insulator1 (SEQ ID NO. 218) and Insulator2 (SEQ ID NO. 219) were added flanking the homology arms, and terminator sequences T1 (nucleic acid SEQ ID NO. 220; see Orosz et al., Eur J Biochem. 1991 Nov 1;201 (3): 653-9) and B0015 (nucleic acid SEQ ID NO. 221) were added to eliminate transcriptional read-through at the site of genomic insertion. The plasmid map shown in Figure 56 shows the general components of vector 2.

The utility of the vectors or plasmids was tested in knock-out experiments. In summary, *E. coli* was inoculated into LB broth and grown for 8 hours at 37C with shaking. Subsequently, an aliquot of the overnight culture was then used to inoculate a larger volume of LB broth and grown for 16 hours at 18°C with shaking. For transformations, 100-400 ng of test plasmid was added to competent cells and transformation was carried out by electroporation. Cells were recovered in SOC media with incubation at 37C for 3 hours before plating on LB-agar with kanamycin. The plate was incubated at 37C to grow colonies with test plasmid.

The gene target to be knocked out was the *E. coli* aroA gene. As such, test plasmids of "aroA-KO in version 1" (i.e., vector 1) and "aroA-KO in version 2" (i.e., vector 2) were constructed by the insertion of homology arms to the *E. coli* aroA gene into the backbones of vector 1 (version 1) and vector 2 (version 2), respectively, such that the homology arms flanked a kanamycin resistance gene to allow single-crossover homology recombination in the *E. coli* host cell. Transformation of these test plasmids and selection on kanamycin verified that "aroA-KO in version 2" showed improved efficiency of transformation and plasmid integration (Figure 53).

Further modification of the vector 1 backbone in vector 2 allowed efficient counter-selection in media containing sucrose and 4-chlorophenylalanine by adding the PheS sequence (Table 15). It should be noted that the PheS promoter sequence in vector 2 consists of the phage λ PL promoter identified by Kincade and deHaseth (*see* Kinacade and deHaseth Gene. 1991 Jan 2;97(1):7-12) immediately followed by an RBS sequence called B0032 which came from iGEM. Further, in vector 2, the promoter sequence of sacB gene was replaced with a promoter containing the P5-MCD2 promoter (Mutalik et al, Nat Methods. 2013 Apr;10(4):354-60) and an additional ATG. This modification allowed efficient counter-selection in sucrose with strains integrated with the version 2 backbone. To generate vector 3, the promoter sequence and CDS of the C. glutamicum pheS* gene in backbone vector 1 were replaced with a new promoter sequence and CDS, specifically a codon-optimized version of the native *E. coli* pheS containing the requisite mutations (T251A/A294G, *see* Miyazaki, K. Biotechniques. 2015 Feb 1;58(2):86-8) (Table 15). This modification allowed improved counter-selection in 4 chlorophenylalanine with strains integrated with the vector 3 backbone. The plasmid map shown in Figure 57 shows the general components of vector 3.

The backbone used for HTP genomic engineering may contain various yeast selection markers for use in plasmid assembly. In the present disclosure, modification of the vector 3 backbone replaced the URA3 yeast selection marker with a TRP1 marker to give vector 4. The plasmid map shown in Figure 58 shows the general components of vector 4.

**Example 12** - **HTP Genomic Engineering - Generation and testing of an additional Promoter Swap Library for use in improving an Industrial Microbial Strain**

This example describes the generation of an additional PROSWP library for later use in the HTP genomic engineering methods provided herein for genetically engineering microbial host cells (e.g., *Escherichia coli*) in an effort to improve industrial strain performance.

In this example, a number of native *E. coli* promoters and synthetic promoters were compiled to generate the promoter swap library found in Table 1.4. For the native promoters, a set of promoter sequences 60-90 bp in length was selected from the genome of an *E. coli* K-12 strain (i.e., *E. coli* W3110). In particular, promoters were selected that showed minimal variation in the associated gene's expression, according to microarray-based expression data across multiple growth conditions (Lewis et al., Mol Syst Biol. 2010; 6: 390). The native promoter sequences consisted of 50bp in front of putative transcription start sites as well as the sequence up to but not including the putative start codon (see Table 1.4). Additionally, a set of chimeric synthetic promoter sequences was created consisting of portions of the known lambda phage promoters $p_L$ and $p_R$, the promoter in front of *E. coli* gene *acs,* and variable 6bp sequences constituting the -35 and -10 regions (Figure 54, Table 1.5). Each of the synthetic promoters were 60-90 bp in length.

In order to test the ability of each of the promoters found in Table 1.4 to drive expression of a gene operably linked thereto, a set of low-copy replicating plasmids was constructed, each containing the RFP gene driven by one of the promoters listed in Table 1.4. The low-copy replicating plasmid of choice was a plasmid called Ori_Plsmd27, which has nucleic sequence SEQ ID NO. 213. The vector was chosen because a replicating plasmid was desired in order to construct and evaluate the promoter library as rapidly as possible and a low copy plasmid such as Ori_Plsmd27 would more closely approximate the scenario in which only a single copy is integrated into the genome. Ori_Plsmd27 is low-copy because it possesses the *E. coli* origin of replication p15A. The p15A origin of replication typically results in approximately 10 copies of the plasmid in each cell. This is "low-copy" in comparison to other common plasmids which may maintain >20 or even several hundred plasmid copies per cell.

The plasmids were constructed using standard molecular biology techniques. Specifically, forward PCR primers were purchased consisting of a sequence to anneal to the RFP gene; the promoter sequence to be introduced; and a sequence overlapping with Ori_Plsmd27. A single reverse PCR primer was obtained consisting of a sequence to anneal to the ECK120033737 terminator (a native *E. coli* terminator) and a sequence overlapping with Ori_Plsmd27. The RFP gene was amplified by PCR with the forward primers and reverse primer to generate a set of PCR amplicons, each containing the RFP gene and one of the promoters listed in Table 1.4. The plasmids were constructing by digesting Ori_Plsmd27 with XhoI restriction enzyme and inserting the corresponding PCR amplicon using commercial DNA assembly enzyme mix. As a negative control, a construct comprising the *C. glutamicum* Tsod terminator (nucleic acid SEQ ID NO. 224 in Table 16) placed upstream of the RFP gene was generated.

The plasmids were transformed by electroporation into *E. coli* W3110. For each promoter to be evaluated, four colonies were picked and inoculated into 1 mL LB broth containing 25 μg/mL kanamycin in a 96-well culture plate. Cultures were grown at 37°C overnight with shaking at 1000 rpm. 10 μL of culture were used to inoculate 1 mL Media 1 (a rich medium containing glucose, yeast extract, salt, and phosphate buffer) containing 25 μg/mL kanamycin in a 96-well culture plate. Cultures were grown at 37°C for 24 hrs with shaking at 1000 rpm. The cultures were diluted in water in a black wall clear bottom 96-well plate and two measurements were taken on a spectrophotometer: $OD_{600}$ (optical density at 600 nm) and fluorescence (554 nm excitation, 590 nm emission). 10 μL of the cultures in Media 1 were used to inoculate 1 mL Media 2 (a rich media containing higher glucose than Media 1 but only a small amount of yeast extract, instead containing ammonium sulfate as nitrogen source along with trace elements) containing 25 μg/mL kanamycin in a 96-well culture plate. Cultures in Media 2 were likewise grown at 37°C overnight and measured after 24 hrs.

$OD_{600}$ measurements were corrected by subtracting the value of blank wells (wells containing only media). Table 16 shows the fluorescence measurements normalized for the corrected $OD_{600}$. As can be seen in Table 16, the resulting strains were effectively grown in two different media and enabled fluorescent protein expression over a ~5000-fold range.

**Table 16.** RFP Expression Levels for promoter-RFP constructs in (2) different media.

| Promoter name | SEQ ID NO. | Type* | Fluorescence in media 1 | Fluorescence in media 2 |
|---|---|---|---|---|
| Empty vector | | Control | 0.59310262 | 1.038945766 |
| No promoter | | Control | 0.372576311 | 0.759290158 |
| Terminator | 224 | Control | 0.41480568 | 0.638974345 |
| b0904_promoter | 71 | Native | 797.7465067 | 5344.268555 |
| b2405_promoter | 72 | Native | 181.125065 | 918.6698873 |
| b0096_promoter | 73 | Native | 124.2236508 | 404.6536222 |
| b0576_promoter | 74 | Native | 22.74750166 | 231.1578591 |
| b2017_promoter | 75 | Native | 43.07006732 | 214.1483559 |
| b1278_promoter | 76 | Native | 24.51965879 | 167.5502652 |
| b4255_promoter | 77 | Native | 35.05859344 | 159.6253961 |
| b0786_promoter | 78 | Native | 34.42969151 | 136.7168488 |
| b0605_promoter | 79 | Native | 19.80356197 | 116.9009802 |
| b1824_promoter | 80 | Native | 24.81069692 | 111.2950744 |
| b1061_promoter | 81 | Native | 23.595542 | 104.971453 |
| b0313_promoter | 82 | Native | 20.68806067 | 75.34101875 |
| b0814_promoter | 83 | Native | 16.46706854 | 70.36934251 |
| b4133_promoter | 84 | Native | 18.1034447 | 67.11707926 |
| b4268_promoter | 85 | Native | 12.80314528 | 60.91103948 |
| b0345_promoter | 86 | Native | 9.57124889 | 56.38701501 |
| b2096_promoter | 87 | Native | 9.852378505 | 44.58545057 |
| b1277_promoter | 88 | Native | 7.821796935 | 43.50459312 |
| b1646_promoter | 89 | Native | 18.43768693 | 33.66381279 |
| b4177_promoter | 90 | Native | 12.51676981 | 30.71734482 |
| b0369_promoter | 91 | Native | 6.614285367 | 28.3955664 |
| b1920_promoter | 92 | Native | 6.196134846 | 28.12617522 |
| b3742_promoter | 93 | Native | 5.646106239 | 27.85277232 |
| b3929_promoter | 94 | Native | 9.120314333 | 27.26180409 |
| b3743_promoter | 95 | Native | 5.416134854 | 26.11889953 |
| b1613_promoter | 96 | Native | 6.653798388 | 24.49553744 |
| b1749_promoter | 97 | Native | 4.14048349 | 24.04592462 |
| b2478_promoter | 98 | Native | 5.387025634 | 20.49940077 |
| b0031_promoter | 99 | Native | 3.227415659 | 18.8898903 |
| b2414_promoter | 100 | Native | 5.054709408 | 17.84254467 |
| b1183_promoter | 101 | Native | 5.109245812 | 14.42155472 |
| b0159_promoter | 102 | Native | 3.662900763 | 14.16361794 |
| b2837_promoter | 103 | Native | 3.427953441 | 13.22830567 |

(continued)

| Promoter name | SEQ ID NO. | Type* | Fluorescence in media 1 | Fluorescence in media 2 |
|---|---|---|---|---|
| b3237_promoter | 104 | Native | 3.238274954 | 11.27938911 |
| b3778_promoter | 105 | Native | 1.778132048 | 8.89448301 |
| b2349_promoter | 106 | Native | 1.918547188 | 8.854115649 |
| b1434_promoter | 107 | Native | 3.05979593 | 7.982575199 |
| b3617_promoter | 108 | Native | 1.631428047 | 7.872487968 |
| b0237_promoter | 109 | Native | 2.524629463 | 6.00416496 |
| b4063_promoter | 110 | Native | 1.75938278 | 5.338598994 |
| b0564_promoter | 111 | Native | 1.099810487 | 5.27271929 |
| b0019_promoter | 112 | Native | 1.407515599 | 5.174702317 |
| b2375_promoter | 113 | Native | 0.758754127 | 4.329319889 |
| b1187_promoter | 114 | Native | 1.042830698 | 4.119645144 |
| b2388_promoter | 115 | Native | 1.068375672 | 4.008318722 |
| b1051_promoter | 116 | Native | 2.595570861 | 3.770122087 |
| b4241_promoter | 117 | Native | 0.86986247 | 3.520447288 |
| b4054_promoter | 118 | Native | 1.166071321 | 3.009684331 |
| b2425_promoter | 119 | Native | 0.684355198 | 2.847357334 |
| b0995_promoter | 120 | Native | 0.783460564 | 2.701492023 |
| b1399_promoter | 121 | Native | 0.67920166 | 2.675743061 |
| b3298_promoter | 122 | Native | 0.693842895 | 2.248914103 |
| b2114_promoter | 123 | Native | 0.680005978 | 2.166569672 |
| b2779_promoter | 124 | Native | 0.516911046 | 1.708793442 |
| b1114_promoter | 125 | Native | 0.477678475 | 1.423398279 |
| b3730_promoter | 126 | Native | 0.527510171 | 1.40174991 |
| b3025_promoter | 127 | Native | 0.528069061 | 1.375386866 |
| b0850_promoter | 128 | Native | 0.580500872 | 1.365244168 |
| b2365_promoter | 129 | Native | 0.600099606 | 1.293746086 |
| b4117_promoter | 130 | Native | 0.465598493 | 1.292314074 |
| b2213_promoter | 131 | Native | 0.502863501 | 1.066458143 |
| pMB029_promoter | 132 | Synthetic | 3244.159758 | 6397.50355 |
| pMB023_promoter | 133 | Synthetic | 2716.103558 | 5324.355721 |
| pMB025_promoter | 134 | Synthetic | 2529.260134 | 5315.845766 |
| pMB019_promoter | 135 | Synthetic | 2632.199487 | 4730.881793 |
| pMB008_promoter | 136 | Synthetic | 3183.229982 | 4685.405472 |
| pMB020_promoter | 137 | Synthetic | 1719.381656 | 4331.834596 |
| pMB022_promoter | 138 | Synthetic | 1432.727416 | 4224.994617 |
| pMB089_promoter | 139 | Synthetic | 1588.496061 | 4173.815385 |
| pMB001_promoter | 140 | Synthetic | 1344.15431 | 3710.844718 |
| pMB051_promoter | 141 | Synthetic | 1485.624649 | 3614.713536 |

(continued)

| Promoter name | SEQ ID NO. | Type* | Fluorescence in media 1 | Fluorescence in media 2 |
|---|---|---|---|---|
| pMB070_promoter | 142 | Synthetic | 1148.371596 | 3494.100223 |
| pMB074_promoter | 143 | Synthetic | 1372.63931 | 3423.518954 |
| pMB046_promoter | 144 | Synthetic | 1070.100822 | 3325.635875 |
| pMB071_promoter | 145 | Synthetic | 829.6337908 | 2753.919683 |
| pMB013_promoter | 146 | Synthetic | 649.3261186 | 2255.268459 |
| pMB080_promoter | 147 | Synthetic | 584.6548742 | 2064.645666 |
| pMB03 8_promoter | 148 | Synthetic | 580.7846278 | 2043.19218 |
| pMB060_promoter | 149 | Synthetic | 575.1167059 | 1916.349295 |
| pMB064_promoter | 150 | Synthetic | 376.0013091 | 1390.767111 |
| pMB05 8_promoter | 151 | Synthetic | 292.4875916 | 1093.376645 |
| pMB085_promoter | 152 | Synthetic | 288.8178401 | 1085.653214 |
| pMB081_promoter | 153 | Synthetic | 278.7856475 | 670.8190328 |
| pMB091_promoter | 154 | Synthetic | 296.1836771 | 655.9453316 |
| pMB027_promoter | 155 | Synthetic | 262.5866089 | 601.1559107 |
| pMB048_promoter | 156 | Synthetic | 235.0853436 | 567.7578783 |
| pMB055_promoter | 157 | Synthetic | 189.6983907 | 499.3358783 |
| pMB006_promoter | 158 | Synthetic | 205.2252242 | 497.2257006 |
| pMB012_promoter | 159 | Synthetic | 118.144338 | 480.5859474 |
| pMB014_promoter | 160 | Synthetic | 119.9861606 | 474.4330596 |
| pMB028_promoter | 161 | Synthetic | 204.8853226 | 454.9894988 |
| pMB059_promoter | 162 | Synthetic | 101.7399612 | 387.0993978 |
| pMB061_promoter | 163 | Synthetic | 128.6404482 | 381.8073111 |
| pMB043_promoter | 164 | Synthetic | 139.0031576 | 380.0980602 |
| pMB066_promoter | 165 | Synthetic | 95.64407537 | 328.5708555 |
| pMB079_promoter | 166 | Synthetic | 105.9768914 | 312.5115498 |
| pMB032_promoter | 167 | Synthetic | 89.71910699 | 311.1818243 |
| pMB068_promoter | 168 | Synthetic | 97.56292371 | 276.8593245 |
| pMB082_promoter | 169 | Synthetic | 84.20594634 | 262.0127742 |
| pMB030_promoter | 170 | Synthetic | 92.28874802 | 255.1052708 |
| pMB067_promoter | 171 | Synthetic | 49.74078463 | 243.9642233 |
| pMB050_promoter | 172 | Synthetic | 69.03704925 | 223.6129199 |
| pMB069_promoter | 173 | Synthetic | 66.09043115 | 213.5258967 |
| pMB017_promoter | 174 | Synthetic | 50.43740198 | 213.0316985 |
| pMB039_promoter | 175 | Synthetic | 52.58639745 | 209.9432421 |
| pMB011_promoter | 176 | Synthetic | 63.05192023 | 194.8104082 |
| pMB072_promoter | 177 | Synthetic | 47.21948722 | 165.6730417 |
| pMB016_promoter | 178 | Synthetic | 47.20169015 | 163.6018648 |
| pMB077_promoter | 179 | Synthetic | 43.98192292 | 158.5866563 |

(continued)

| Promoter name | SEQ ID NO. | Type* | Fluorescence in media 1 | Fluorescence in media 2 |
|---|---|---|---|---|
| pMB047_promoter | 180 | Synthetic | 30.55918215 | 121.2753356 |
| pMB052_promoter | 181 | Synthetic | 34.86596074 | 114.4169675 |
| pMB090_promoter | 182 | Synthetic | 14.01229264 | 55.03412277 |
| pMB035_promoter | 183 | Synthetic | 14.7011564 | 50.95895734 |
| pMB073_promoter | 184 | Synthetic | 11.79654115 | 47.47608915 |
| pMB004_promoter | 185 | Synthetic | 8.701914436 | 33.9085268 |
| pMB054_promoter | 186 | Synthetic | 8.934971498 | 32.06510507 |
| pMB024_promoter | 187 | Synthetic | 6.760860897 | 27.59246488 |
| pMB007_promoter | 188 | Synthetic | 11.5557009 | 25.41738039 |
| pMB005_promoter | 189 | Synthetic | 5.354276069 | 23.77742451 |
| pMB003_promoter | 190 | Synthetic | 6.389447374 | 22.7246451 |
| pMB088_promoter | 191 | Synthetic | 5.873205603 | 22.42214302 |
| pMB065_promoter | 192 | Synthetic | 5.44173707 | 20.60442307 |
| pMB037_promoter | 193 | Synthetic | 4.809680789 | 19.26108105 |
| pMB009_promoter | 194 | Synthetic | 3.591881545 | 13.6498423 |
| pMB041_promoter | 195 | Synthetic | 4.78150005 | 13.03957332 |
| pMB036_promoter | 196 | Synthetic | 2.508122856 | 11.98560161 |
| pMB049_promoter | 197 | Synthetic | 2.916151498 | 11.02944113 |
| pMB044_promoter | 198 | Synthetic | 2.719080697 | 10.47251128 |
| pMB042_promoter | 199 | Synthetic | 2.661753833 | 8.883733663 |
| pMB086_promoter | 200 | Synthetic | 2.33212712 | 8.316649305 |
| pMB053_promoter | 201 | Synthetic | 2.336160735 | 7.262981543 |
| pMB057_promoter | 202 | Synthetic | 1.518801989 | 6.27420435 |
| pMB018_promoter | 203 | Synthetic | 1.261000991 | 4.714509042 |
| pMB002_promoter | 204 | Synthetic | 1.290587949 | 4.473711828 |
| pMB015_promoter | 205 | Synthetic | 0.994888783 | 4.328541658 |
| pMB087_promoter | 206 | Synthetic | 0.94621781 | 2.900396821 |
| pMB063_promoter | 207 | Synthetic | 0.425085793 | 1.459419227 |
| *Native promoters from Escherichia coli | | | | |

**Example 13- HTP Genomic Engineering -Testing integration of Promoter Swap Library of Table 1.4 into the genome of E. coli using vector 2 backbone**

This example describes a proof of concept of the use of the vector 2 backbone from Example 11 in combination with a subset of promoters form the promoter library of Table 1.4 to drive integration of a single copy of a heterologous promoter-gene construct into the genome of *E. coli.*

For this Example, a set of plasmids will be built to insert fluorescent genes RFP and GFP at two loci (*nupG* and *asl*) in *E. coli* with a subset of 14 promoters from the set in Table 1.4. This will allow those promoters to be evaluated as a single copy integrated into the genome, rather than on low-copy replicating plasmids (see Example 12).

The plasmids will be comprise homology arms flanking the RFP or GFP genes in order to facilitate integration into the genome of *E. coli* via "loop-in" as provided throughout this disclosure. The resulting strains will be tested for fluorescence, which will demonstrate that this subset of 14 promoters from Table 1.4 has been tested using a vector backbone described in Example 11 and can be used to insert a heterologous gene into the genome of *E. coli* using the methods described in this disclosure.

**Example 14- HTP Genomic Engineering - Implementation of a PROSWP methods using promoter library derived from of Table 1.4.**

The section below provides an illustrative implementation of the PRO swap HTP design strain improvement program tools of the present disclosure, as described in Examples 4 and 5. In this example, an *E. coli* strain was subjected to the PRO swap methods of the present disclosure in order to modulate the expression of genes in the *E. coli* genome. This example builds upon the results of Examples 12 and 13 in that this example illustrates the use of a promoter library comprising promoters from Table 1.4 in PROSWP methods of the present disclosure.

**A. Promoter Swap**

Promoter Swaps will be conducted as described in Example 4. Genes form the *E. coli* genome will be subjected to promoter swaps using the promoter library described in Example 13, which comprises a subset of 14 promoters from Table 1.4. The subset of 14 promoters to be used in this Example, will be selected based on their effect on gene expression as determined in Examples 12 and 13.

**B. HTP engineering and High-Throughput Screening**

HTP engineering of the promoter swaps will be conducted as described in Example 1 and 3. HTP screening of the resulting promoter swap strains will be conducted as described in Example 3. In total, 14 PRO swaps will be conducted. Finally, the impact of these modifications on production of products of interest will be tested.

**Example 15- HTP Genomic Engineering - Implementation of a TERMINATOR Swap Library to Improve Strain Performance for Lycopene Production**

The section below provides an illustrative implementation of the TERMINATOR swap HTP design strain improvement program tools of the present disclosure. In this example, an *E. coli* strain was subjected to the TERMINATOR swap methods of the present disclosure in order to affect host cell yield of lycopene.

Terminator swaps targeting genes in the lycopene biosynthetic pathway shown in Figure 59 were conducted using the terminator swap methods present throughout this disclosure. Constructs were designed as described below and recombination was mediated with CRISPR/Cas9 system. The terminators used for the terminator swaps in this example were the terminators found in Table 19.

**Table 19**-Terminators used in this Example for targeting genes in the lycopene biosynthetic pathway

| Name | Description | Length (bp) |
|---|---|---|
| Spy | Terminator (SEQ ID NO. 225) | 90 |
| pheA | Terminator (SEQ ID NO. 226) | 51 |
| osmE | Terminator (SEQ ID NO. 227) | 42 |
| rpoH | Terminator (SEQ ID NO. 228) | 41 |
| vibE | Terminator (SEQ ID NO. 229) | 71 |
| Thr1_ABC | Terminator (SEQ ID NO. 230) | 57 |

**Construct design**

A 20 base guide RNA near the target insertion sequence and adjacent to an NGG PAM sequence was identified to cut the genome at the desired position. The sequence intended to be inserted into the genome was flanked on both ends by 90 bases of homology, such that the homology would direct native sequence to be deleted or retained as desired. It should be noted that while recombination was facilitated by the CRISPR/Cas9 system in this Example, all strains can also be built by traditional single- and double-crossover homologous recombination methods as well as the Lambda Red system as described throughout the present disclosure. As such, each of the terminator swap library types is agnostic to the construction/recombination method.

**Inoculate seed culture**

A colony of editing base strain (W3110 pKD46-cas9 pLYC4) from a petri plate was picked and inoculated into larger volume of LB clin100 cmp25 and grew culture at $30^0$C with shaking for ~16 hours.

**Prepare competent cells and transform**

A 1:10 dilution of the overnight culture was prepared and measured the OD600 was measured. LB clin100 cmp25 was inoculated to an OD600 of 0.05 and grow at $30^0$C with shaking for ~2 hours.

After 2 hours, the OD600 was measured periodically until the induction target OD was reached, and upon reaching the target 20% arabinose was added to a final concentration of 0.2%.

Centrifuge the culture for 5 minutes at 5,000 x G at 4°C. Pour off the supernatant and resuspend to a final volume equivalent to the original culture volume.

Repeat step 7 to wash the cells for a third time.

After a 3 washes, the cells were pelleted and resuspended in 10% glycerol to ~1/250th the original culture volume. Prepare a 1:500 dilution of the resuspended cells was prepared and resuspended to a desired OD600 with an adequate volume for 40 uL of cells per transformation.

In a Framestar PCR plate (or microfuge tubes) 40 uL of cells were mixed with 100 ng of guide RNA plasmid, and ~4 uL of purified PCR product repair template. If using oligos for the repair template, the oligos were added to a final concentration of 2 uM.

The cells were electroporated and and immediately resuspended in LB and recover edin a deep well plate 1 hour at 30°C with shaking.

The recovered cells were diluted and plated on LB agar clin100 kan50 cmp25 and at 30°C 24 to 36 hours. Colonies were screened by colony PCR, sequencing, or phenotype screening. pKD46-cas9 plasmid can be cured by growth at 37°C or above, and pCRISPR2 can be cured by growth on 10% sucrose.

**HTP engineering and High-Throughput Screening**

HTP engineering of the swap combinations was conducted as described in Example 1 and 3 with the exception that CRISPR/Cas 9 was used to facilitate homologous recombination of constructs into the *E. coli* genome. HTP screening of the resulting promoter swap/terminator swap strains, promoter swap/degradation tag swap strains, promoter swap/solubility tag swap strains and promoter swap/terminator swap/degradation tag swap/solubility tag swap strains was conducted as described in Example 3. The results of the experiments are depicted in Figures 60 and 61.

As shown in Figure 60, terminator swaps at lycopene pathway targets *idi and ymgA* using the terminator TyjbE demonstrated decreased strain performance relative to the control, thus highlighting the utility of these library types for identifying critical pathway targets. This conclusion was further supported by the results shown in Figure 61, were terminator swaps were conducted on multiple lycopene pathway targets.

**Example 16- HTP Genomic Engineering - Implementation of a TERMINATOR Swap Library or PRO Swap Library in combination with either a SOLUBILITY TAG swap Library or DEGRADATION Tag swap Library to Improve Strain Performance for Lycopene Production**

The section below provides an illustrative implementation of the SOLUBILITY TAG swap and TERMINATOR swap HTP design strain improvement program tools of the present disclosure as well as a PRO swap and DEGRADATION TAG swap design strain improvement program tools of the present disclosure. In this example, an *E. coli* strain was subjected to the PRO swap in combination with DEGRADATION TAG swap methods of the present disclosure as well as SOLUBILITY TAG swap and TERMINATOR swap methods of the present disclosure in order to affect host cell yield of lycopene.

**Promoter Swap/Terminator Swap/Solubility Tag Swap/Degradation Tag Swap**

The Terminator swap was conducted as described in Example 15, while the Solubility Tag swap and Promoter Swap in combination with the degradation tag swaps were essentially conducted as described in Examples 4 and 5. Constructs were designed as described below and recombination was mediated with CRISPR/Cas9 system. The bicistronic promoters used for the promoter swaps in this example were from Mutalik et al., Nat Methods. 2013 Apr;10(4):354-60 and can be found in Table 20. It should be noted that any of the promoters provided herein could be used in the methods described below.

**Table 20**-Promoters used for promoter swap combinations in this example.

| Name | Length | SEQ ID NO. |
|---|---|---|
| P3_BCD1 | 133 | 255 |
| P4_BCD22 | 121 | 256 |
| P7_BCD19 | 132 | 257 |
| P8_BCD15 | 121 | 258 |
| P11_BCD17 | 121 | 259 |
| P13_BCD8 | 121 | 260 |

**Construct design**

A 20 base guide RNA near the target insertion sequence and adjacent to an NGG PAM sequence was identified to cut the genome at the desired position. The sequence intended to be inserted into the genome was flanked on both ends by 90 bases of homology, such that the homology would direct native sequence to be deleted or retained as desired. It should be noted that while recombination was facilitated by the CRISPR/Cas9 system in this Example, all strains can also be built by traditional single- and double-crossover homologous recombination methods as well as the Lambda Red system as described throughout the present disclosure. As such, each of these library types (promoter swap, protein solubility tag swap, protein degradation tag swap, and terminator swap) alone or in combination are agnostic to the construction/recombination method.

**Inoculate seed culture**

A colony of editing base strain (W3110 pKD46-cas9 pLYC4) from a petri plate was picked and inoculated into larger volume of LB clin100 cmp25 and grew culture at 30$^0$C with shaking for ~16 hours.

**Prepare competent cells and transform**

A 1:10 dilution of the overnight culture was prepared and measured the OD600 was measured. LB clin100 cmp25 was inoculated to an OD600 of 0.05 and grow at 30$^0$C with shaking for ~2 hours.
After 2 hours, the OD600 was measured periodically until the induction target OD was reached, and upon reaching the target 20% arabinose was added to a final concentration of 0.2%.
Centrifuge the culture for 5 minutes at 5,000 x G at 4°C. Pour off the supernatant and resuspend to a final volume equivalent to the original culture volume.
Repeat step 7 to wash the cells for a third time.
After a 3 washes, the cells were pelleted and resuspended in 10% glycerol to ~1/250th the original culture volume. Prepare a 1:500 dilution of the resuspended cells was prepared and resuspended to a desired OD600 with an adequate volume for 40 uL of cells per transformation.
In a Framestar PCR plate (or microfuge tubes) 40 uL of cells were mixed with 100 ng of guide RNA plasmid, and ~4 uL of purified PCR product repair template. If using oligos for the repair template, the oligos were added to a final concentration of 2 uM.
The cells were electroporated and and immediately resuspended in LB and recover edin a deep well plate 1 hour at 30°C with shaking.
The recovered cells were diluted and plated on LB agar clin100 kan50 cmp25 and at 30°C 24 to 36 hours. Colonies were screened by colony PCR, sequencing, or phenotype screening. pKD46-cas9 plasmid can be cured by growth at 37°C or above, and pCRISPR2 can be cured by growth on 10% sucrose.

**HTP engineering and High-Throughput Screening**

HTP engineering of the swap combinations was conducted as described in Example 1 and 3 with the exception that CRISPR/Cas 9 was used to facilitate homologous recombination of constructs into the *E. coli* genome. HTP screening of the resulting promoter swap/terminator swap strains, promoter swap/degradation tag swap strains, promoter swap/solubility tag swap strains and promoter swap/terminator swap/degradation tag swap/solubility tag swap strains was conducted as described in Example 3.

It should be noted that the promoter P3_BCD1 was used in all strains where modification at the dxs locus was under study, unless otherwise noted to be different, such as P4_BCD22. At any locus other than dxs, the native promoter sequence was used unless otherwise noted. This means that a strain described as ssrA_LAA at the dxs locus, for example, also contained P3_BCD1, but a strain described as ssrA_LAA at the gdhA locus used the native promoter sequence. The full contents of the strains tested in Table 21 below.

**Table 21**-Contents of strains generated in Example 16.

| **Modifications at dxs locus** | | | | | | |
|---|---|---|---|---|---|---|
| | ***Control -P** | **P4_BCD2 2** | **ssrA_AS V** | **ssrA_LA A** | **Tspy** | **TyjbE** |
| **Promoter** | P3_BCD 1 | P4_BCD2 2 | P3_BCD1 | P3_BCD1 | P3_BCD 1 | P3_BCD 1 |
| **Solubility tag** | none | none | none | none | none | none |
| **Degradation tag** | none | none | ssrA_AS V | ssrA_LAA | none | none |
| **Terminator** | native sequence | native sequence | native sequence | native sequence | Tspy | TyjbE |
| **Modifications at gdhA locus** | | | | | | |
| | *Control-P | FH8 | GB1 | P4_BCD2 2 | Tspy | Tyjbe |
| **Promoter** | native sequence | native sequence | native sequence | P4_BCD2 2 | native sequence | native sequence |
| **Solubility tag** | none | FH8 | GB1 | none | none | none |
| **Degradation tag** | none | none | none | none | none | none |
| **Terminator** | native sequence | native sequence | native sequence | native sequence | Tspy | Tyjbe |

The results of the experiments are summarized are depicted in Figures 62 and 63.

As shown in Figure 62, The ssrA_LAA degradation tag demonstrates improved strain performance relative to the control. This is unexpected as this strain is a combination of a PROSWP with a degradation tag at a single pathway target. The initial PROSWP is expected to increase protein abundance, and the degradation tag is expected to decrease protein abundance, thus demonstrating the utility of combinations of library types for tuning optimal strain performance. As shown in Figure 63, the solubility tag FH8 demonstrates improved strain performance relative to the control, but the GB1 solubility tag does not, thus demonstrating the necessity for evaluating libraries of each modification type.

Overall, what this Example demonstrates is that while components of the present disclosure may be useful individually for systematic strain improvement, they can also be used synergistically with other approaches. For example, after improving mRNA stability through terminator modification, a strong promoter may be inserted to further increase protein production beyond the level of either approach alone. Likewise, this new elevated protein production level may be further improved through a protein solubility tag in conjunction with other modifications. When employed together and with previous approaches, the components of the present disclosure may allow for more robust and effective strain improvement for the production of a target molecule.

**SEQUENCES OF THE DISCLOSURE WITH SEQ ID NO IDENTIFIERS**

| NAME (SHORT NAME) | SOURCE | NUCLEIC ACID SEQ ID NO. | AMINO ACID SEQ ID NO. | DESCRIPTION |
|---|---|---|---|---|
| Pcg0007_lib_39 (P1) | | 1 | | |
| Pcg0007 (P2) | | 2 | | |
| Pcg1860 (P3) | | 3 | | |
| Pcg0755 (P4) | | 4 | | |
| Pcg0007 _265 (P5) | | 5 | | |
| Pcg3381 (P6) | | 6 | | |

(continued)

| NAME (SHORT NAME) | SOURCE | NUCLEIC ACID SEQ ID NO. | AMINO ACID SEQ ID NO. | DESCRIPTION |
|---|---|---|---|---|
| Pcg0007_119 (P7) | | 7 | | |
| Pcg3121 (P8) | | 8 | | |
| cg0001 (T1) | | 9 | | |
| cg0007 (T2) | | 10 | | |
| cg0371 (T3) | | 11 | | |
| cg0480 (T4) | | 12 | | |
| cg0494 (T5) | | 13 | | |
| cg0564 (T6) | | 14 | | |
| cg0610 (T7) | | 15 | | |
| cg0695 (T8) | | 16 | | |
| Cisl nucleotide sequence | | 17 | | |
| Serine-rich linker | | 18 | | |
| Serine-rich linker | | 19 | | |
| Serine-rich linker | | 20 | | |
| Alanine-rich linker | | 21 | | |
| linker | | 22 | | |
| linker | | 23 | | |
| linker | | 24 | | |
| linker | | 25 | | |
| Zif268 | | 26 | | DNA Binding Domain Sequence |
| PBSII | | 27 | | DNA Binding Domain Sequence |
| ZFa | | 28 | | DNA Binding Domain Sequence |
| ZFb | | 29 | | DNA Binding Domain Sequence |
| ZFc | | 30 | | DNA Binding Domain Sequence |
| Tyr123 | | 31 | | DNA Binding Domain Sequence |
| Tyr456 | | 32 | | DNA Binding Domain Sequence |
| Blues Zinc Finger | | 33 | | DNA Binding Domain Sequence |
| Jazz Zinc Finger | | 34 | | DNA Binding Domain Sequence |
| Bagly Zinc Finger | | 35 | | DNA Binding Domain Sequence |
| Bagly Zinc Finger Binding Site | | 36 | | DNA Binding Sequence (5'$\rightarrow$3') |
| Glil | | 37 | | DNA Binding Domain Sequence |
| Glil binding site | | 38 | | DNA Binding Sequence (5'$\rightarrow$3') |
| HIVC zinc finger | | 39 | | DNA Binding Domain Sequence |
| B3 zinc finger | | 40 | | DNA Binding Domain Sequence |
| N1 zinc finger | | 41 | | DNA Binding Domain Sequence |
| Sp-1 first zinc finger | | 42 | | DNA Binding Domain Sequence |

(continued)

| NAME (SHORT NAME) | SOURCE | NUCLEIC ACID SEQ ID NO. | AMINO ACID SEQ ID NO. | DESCRIPTION |
|---|---|---|---|---|
| Sp-1 second finger | | 43 | | DNA Binding Domain Sequence |
| Class I SH3 protein binding domain | | | 44 | |
| Class II SH3 Protein Binding Site | | | 45 | |
| SH3 protein binding site | | | 46 | |
| SH3 recruitment peptide | | | 47 | |
| SH3 recruitment Peptide | | | 48 | |
| SH3 recruitment peptide | | | 49 | |
| PDZ protein binding domain | | | 50 | |
| PDZ protein binding domain | | | 51 | |
| PDZ protein binding domain | | | 52 | |
| PDZ protein binding domain | | | 53 | |
| PDZ protein binding domain | | | 54 | |
| PDZ recruitment peptide | | | 55 | |
| PDZ recruitment peptide | | | 56 | |
| PDZ recruitment peptide | | | 57 | |
| PDZ recruitment peptide | | | 58 | |
| PDZ recruitment peptide | | | 59 | |
| PDZ recruitment peptide | | | 60 | |
| PDZ recruitment peptide | | | 61 | |
| GBD protein binding domain | | | 62 | |
| GBD protein binding domain | | | 63 | |
| GBD protein binding domain | | | 64 | |
| GBD recruitment peptide | | | 65 | |
| Leucine Zipper binding domain | | | 66 | |
| Leucine Zipper binding domain | | | 67 | |
| Leucine Zipper binding domain | | | 68 | |
| Bicistronic Design (BCD) regulatory sequence | | | 69 | *see* Figure 43 |
| Bicistronic Design (BCD) regulatory sequence | | | 70 | *see* Figure 43 |
| b0904_promoter | *E. coli* | 71 | | |
| b2405_promoter | *E. coli* | 72 | | |
| b0096_promoter | *E. coli* | 73 | | |

(continued)

| NAME (SHORT NAME) | SOURCE | NUCLEIC ACID SEQ ID NO. | AMINO ACID SEQ ID NO. | DESCRIPTION |
|---|---|---|---|---|
| b0576_promoter | *E. coli* | 74 | | |
| b2017_promoter | *E. coli* | 75 | | |
| b1278_promoter | *E. coli* | 76 | | |
| b4255_promoter | *E. coli* | 77 | | |
| b0786_promoter | *E. coli* | 78 | | |
| b0605_promoter | *E. coli* | 79 | | |
| b1824_promoter | *E. coli* | 80 | | |
| b1061_promoter | *E. coli* | 81 | | |
| b0313_promoter | *E. coli* | 82 | | |
| b0814_promoter | *E. coli* | 83 | | |
| b4133_promoter | *E. coli* | 84 | | |
| b4268_promoter | *E. coli* | 85 | | |
| b0345_promoter | *E. coli* | 86 | | |
| b2096_promoter | *E. coli* | 87 | | |
| b1277_promoter | *E. coli* | 88 | | |
| b1646_promoter | *E. coli* | 89 | | |
| b4177_promoter | *E. coli* | 90 | | |
| b0369_promoter | *E. coli* | 91 | | |
| b1920_promoter | *E. coli* | 92 | | |
| b3742_promoter | *E. coli* | 93 | | |
| b3929_promoter | *E. coli* | 94 | | |
| b3743_promoter | *E. coli* | 95 | | |
| b1613_promoter | *E. coli* | 96 | | |
| b1749_promoter | *E. coli* | 97 | | |
| b2478_promoter | *E. coli* | 98 | | |
| b0031_promoter | *E. coli* | 99 | | |
| b2414_promoter | *E. coli* | 100 | | |
| b1183_promoter | *E. coli* | 101 | | |
| b0159_promoter | *E. coli* | 102 | | |
| b2837_promoter | *E. coli* | 103 | | |
| b3237_promoter | *E. coli* | 104 | | |
| b3778_promoter | *E. coli* | 105 | | |
| b2349_promoter | *E. coli* | 106 | | |
| b1434_promoter | *E. coli* | 107 | | |
| b3617_promoter | *E. coli* | 108 | | |
| b0237_promoter | *E. coli* | 109 | | |

(continued)

| NAME (SHORT NAME) | SOURCE | NUCLEIC ACID SEQ ID NO. | AMINO ACID SEQ ID NO. | DESCRIPTION |
|---|---|---|---|---|
| b4063_promoter | *E. coli* | 110 | | |
| b0564_promoter | *E. coli* | 111 | | |
| b0019_promoter | *E. coli* | 112 | | |
| b2375_promoter | *E. coli* | 113 | | |
| b1187_promoter | *E. coli* | 114 | | |
| b23 8 8_promoter | *E. coli* | 115 | | |
| b1051_promoter | *E. coli* | 116 | | |
| b4241_promoter | *E. coli* | 117 | | |
| b4054_promoter | *E. coli* | 118 | | |
| b2425_promoter | *E. coli* | 119 | | |
| b0995_promoter | *E. coli* | 120 | | |
| b1399_promoter | *E. coli* | 121 | | |
| b3298_promoter | *E. coli* | 122 | | |
| b2114_promoter | *E. coli* | 123 | | |
| b2779_promoter | *E. coli* | 124 | | |
| b1114_promoter | *E. coli* | 125 | | |
| b3 73 0_promoter | *E. coli* | 126 | | |
| b3025_promoter | *E. coli* | 127 | | |
| b0850_promoter | *E. coli* | 128 | | |
| b2365_promoter | *E. coli* | 129 | | |
| b4117_promoter | *E. coli* | 130 | | |
| b2213_promoter | *E. coli* | 131 | | |
| pMB029_promoter | Synthetic | 132 | | |
| pMB023_promoter | Synthetic | 133 | | |
| pMB025_promoter | Synthetic | 134 | | |
| pMB019_promoter | Synthetic | 135 | | |
| pMB008_promoter | Synthetic | 136 | | |
| pMB020_promoter | Synthetic | 137 | | |
| pMB022_promoter | Synthetic | 138 | | |
| pMB089_promoter | Synthetic | 139 | | |
| pMB001_promoter | Synthetic | 140 | | |
| pMB051_promoter | Synthetic | 141 | | |
| pMB070_promoter | Synthetic | 142 | | |
| pMB074_promoter | Synthetic | 143 | | |
| pMB046_promoter | Synthetic | 144 | | |
| pMB071_promoter | Synthetic | 145 | | |

(continued)

| NAME (SHORT NAME) | SOURCE | NUCLEIC ACID SEQ ID NO. | AMINO ACID SEQ ID NO. | DESCRIPTION |
|---|---|---|---|---|
| pMB013_promoter | Synthetic | 146 | | |
| pMB080_promoter | Synthetic | 147 | | |
| pMB03 8_promoter | Synthetic | 148 | | |
| pMB060_promoter | Synthetic | 149 | | |
| pMB064_promoter | Synthetic | 150 | | |
| pMB05 8_promoter | Synthetic | 151 | | |
| pMB085_promoter | Synthetic | 152 | | |
| pMB081_promoter | Synthetic | 153 | | |
| pMB091_promoter | Synthetic | 154 | | |
| pMB027_promoter | Synthetic | 155 | | |
| pMB048_promoter | Synthetic | 156 | | |
| pMB055_promoter | Synthetic | 157 | | |
| pMB006_promoter | Synthetic | 158 | | |
| pMB012_promoter | Synthetic | 159 | | |
| pMB014_promoter | Synthetic | 160 | | |
| pMB028_promoter | Synthetic | 161 | | |
| pMB059_promoter | Synthetic | 162 | | |
| pMB061_promoter | Synthetic | 163 | | |
| pMB043_promoter | Synthetic | 164 | | |
| pMB066_promoter | Synthetic | 165 | | |
| pMB079_promoter | Synthetic | 166 | | |
| pMB032_promoter | Synthetic | 167 | | |
| pMB068_promoter | Synthetic | 168 | | |
| pMB082_promoter | Synthetic | 169 | | |
| pMB03 0_promoter | Synthetic | 170 | | |
| pMB067_promoter | Synthetic | 171 | | |
| pMB050_promoter | Synthetic | 172 | | |
| pMB069_promoter | Synthetic | 173 | | |
| pMB017_promoter | Synthetic | 174 | | |
| pMB039_promoter | Synthetic | 175 | | |
| pMB011_promoter | Synthetic | 176 | | |
| pMB072_promoter | Synthetic | 177 | | |
| pMB016_promoter | Synthetic | 178 | | |
| pMB077_promoter | Synthetic | 179 | | |
| pMB047_promoter | Synthetic | 180 | | |
| pMB052_promoter | Synthetic | 181 | | |

(continued)

| NAME (SHORT NAME) | SOURCE | NUCLEIC ACID SEQ ID NO. | AMINO ACID SEQ ID NO. | DESCRIPTION |
|---|---|---|---|---|
| pMB090_promoter | Synthetic | 182 | | |
| pMB035_promoter | Synthetic | 183 | | |
| pMB073_promoter | Synthetic | 184 | | |
| pMB004_promoter | Synthetic | 185 | | |
| pMB054_promoter | Synthetic | 186 | | |
| pMB024_promoter | Synthetic | 187 | | |
| pMB007_promoter | Synthetic | 188 | | |
| pMB005_promoter | Synthetic | 189 | | |
| pMB003_promoter | Synthetic | 190 | | |
| pMB088_promoter | Synthetic | 191 | | |
| pMB065_promoter | Synthetic | 192 | | |
| pMB037_promoter | Synthetic | 193 | | |
| pMB009_promoter | Synthetic | 194 | | |
| pMB041_promoter | Synthetic | 195 | | |
| pMB036_promoter | Synthetic | 196 | | |
| pMB049_promoter | Synthetic | 197 | | |
| pMB044_promoter | Synthetic | 198 | | |
| pMB042_promoter | Synthetic | 199 | | |
| pMB086_promoter | Synthetic | 200 | | |
| pMB053_promoter | Synthetic | 201 | | |
| pMB057_promoter | Synthetic | 202 | | |
| pMB018_promoter | Synthetic | 203 | | |
| pMB002_promoter | Synthetic | 204 | | |
| pMB015_promoter | Synthetic | 205 | | |
| pMB087_promoter | Synthetic | 206 | | |
| pMB063_promoter | Synthetic | 207 | | |
| Distal portion of synthetic promoter | Phage λ | 208 | | $P_R$ promoter |
| Core portion of synthetic promoter | Phage λ | 209 | | $P_R$ promoter |
| Distal portion of synthetic promoter | Phage λ | 210 | | $P_L$ promoter |
| 5'UTR/RBS portion of synthetic promoter | Phage λ | 211 | | $P_R$ promoter |
| 5'UTR/RBS portion of synthetic promoter | *E. coli* | 212 | | promoter of *acs* gene |
| Ori_Plsmd27 | | 213 | | |
| vector backbone 1 | | 214 | | |

(continued)

| NAME (SHORT NAME) | SOURCE | NUCLEIC ACID SEQ ID NO. | AMINO ACID SEQ ID NO. | DESCRIPTION |
|---|---|---|---|---|
| vector backbone 2 | | 215 | | |
| vector backbone 3 | | 216 | | |
| vector backbone 4 | | 217 | | |
| Insulator 1 | | 218 | | see Table 15 |
| Insulator 2 | | 219 | | see Table 15 |
| T1 | | 220 | | from Orosz et al., Eur J Biochem. 1991 Nov 1;201(3):653-9; see Table 15 |
| B0015 | | 221 | | see Table 15 |
| SacB promoter | | 222 | | see Table 15 |
| PheS promoter & sequence | | 223 | | see Table 15 |
| Tsod terminator | *C. glutamicum* | 224 | | |
| Spy | *E. coli* | 225 | | Terminator Sequence |
| pheA | *E. coli* | 226 | | Terminator Sequence |
| osmE | *E. coli* | 227 | | Terminator Sequence |
| rpoH | *E. coli* | 228 | | Terminator Sequence |
| vibE | *E. coli* | 229 | | Terminator Sequence |
| Thr1_ABC | *E. coli* | 230 | | Terminator Sequence |
| GB1 (PST1) | Streptococcus sp. | 231 | 235 | IgG domain B1 of Protein G |
| FH8 (PST2) | F. hepatica | 232 | 236 | Fasciola hepatica 8-kDa antigen |
| Ubiquitin (PST3) | | 233 | 237 | |
| SUMO (PST4) | Homo sapiens | 234 | 238 | Small ubiquitin modified |
| ssrA_LAA (PDT1) | *E. coli* | 239 | 247 | native |
| ssrA_LVA (PDT2) | *E. coli* | 240 | 248 | mutant |
| ssrA_AAV (PDT3) | *E. coli* | 241 | 249 | mutant |
| ssrA_ASV (PDT4) | *E. coli* | 242 | 250 | mutant |
| ftsH-cII89-97 (PDT5) | *E. coli* | 243 | 251 | native |
| cI108 (PDT6) | *E. coli* | 244 | 252 | native |
| su120 (PDT7) | *E. coli* | 245 | 253 | native |
| β20 (PDT8) | *E. coli* | 246 | 254 | native |
| P3_BCD1 | Artificial | 255 | | |
| P4_BCD22 | Artificial | 256 | | |
| P7_BCD19 | Artificial | 257 | | |
| P8_BCD15 | Artificial | 258 | | |
| P11_BCD17 | Artificial | 259 | | |
| P13_BCD8 | Artificial | 260 | | |

## REFERENCES

The following are referred to: U.S. Application No. 15/396,230 (U.S. Pub. No. US 2017/0159045 A1) filed on December 30, 20016; PCT/US2016/065465 (WO 2017/100377 A1) filed on December 07, 2016; U.S. App. No. 15/140,296 (US 2017/0316353 A1) filed on April 27, 2016; PCT/US2017/029725 (WO 2017/189784 A1) filed on April 26, 2017; PCT/US2016/065464 (WO 2017/100376 A2) filed on December 07, 2016; U.S. Prov. App. No. 62/431,409 filed on December 07, 2016; U.S. Prov. App. No. 62/264,232 filed on December 07, 2015; and U.S. Prov. App. No. 62/368,786 filed on July 29, 2016.

## Claims

1. A method for generating a promoter swap *E. coli* strain library, comprising the steps of:

   a. providing a plurality of target genes endogenous to a base *E. coli* strain, and a promoter ladder, wherein said promoter ladder comprises a plurality of promoters exhibiting different expression profiles in the base *E. coli* strain, wherein at least one of the plurality of promoters is a chimeric synthetic promoter, wherein the chimeric synthetic promoter is 60-90 nucleotides in length and consists of a distal portion of lambda phage $p_R$ promoter, variable -35 and -10 regions of lambda phage $p_L$ and $p_R$ promoters that are each six nucleotides in length, core portions of lambda phage $p_L$ and $p_R$ promoters and a 5' UTR/Ribosomal Binding Site (RBS) portion that is the 5' UTR/RBS portion of lambda phage $p_R$ promoter or the 5' UTR/Ribosomal Binding Site (RBS) portion of the promoter of the *E. coli acs* gene; and
   b. engineering the genome of the base *E. coli* strain, to thereby create an initial promoter swap *E. coli* strain library comprising a plurality of individual *E. coli* strains with unique genetic variations found within each strain of said plurality of individual *E. coli* strains, wherein each of said unique genetic variations comprises one or more of the promoters from the promoter ladder operably linked to one of the target genes endogenous to the base *E. coli* strain.

2. The method for generating a promoter swap *E. coli* strain library according to claim 1, wherein at least one of the plurality of promoters comprises a bicistronic design (BCD) regulatory sequence.

3. A method for improving the phenotypic performance of a production *E. coli* strain, comprising the steps of:

   a. providing a plurality of target genes endogenous to a base *E. coli* strain, and a promoter ladder, wherein said promoter ladder comprises a plurality of promoters exhibiting different expression profiles in the base *E. coli* strain, wherein at least one of the plurality of promoters is a chimeric synthetic promoter, wherein the chimeric synthetic promoter is 60-90 nucleotides in length and consists of a distal portion of lambda phage $p_R$ promoter, variable -35 and -10 regions of lambda phage $p_L$ and $p_R$ promoters that are each six nucleotides in length, core portions of lambda phage $p_L$ and $p_R$ promoters and a 5' UTR/Ribosomal Binding Site (RBS) portion that is the 5' UTR/RBS portion of lambda phage $p_R$ promoter or the 5' UTR/Ribosomal Binding Site (RBS) portion of the promoter of the *E. coli acs* gene;
   b. engineering the genome of the base *E. coli* strain, to thereby create an initial promoter swap *E. coli* strain library comprising a plurality of individual *E. coli* strains with unique genetic variations found within each strain of said plurality of individual *E. coli* strains, wherein each of said unique genetic variations comprises one or more of the promoters from the promoter ladder operably linked to one of the target genes endogenous to the base *E. coli* strain;
   c. screening and selecting individual *E. coli* strains of the initial promoter swap *E. coli* strain library for phenotypic performance improvements over a reference *E. coli* strain, thereby identifying unique genetic variations that confer phenotypic performance improvements;
   d. providing a subsequent plurality of *E. coli* microbes that each comprise a combination of unique genetic variations from the genetic variations present in at least two individual *E. coli* strains screened in the preceding step, to thereby create a subsequent promoter swap *E. coli* strain library;
   e. screening and selecting individual *E. coli* strains of the subsequent promoter swap *E. coli* strain library for phenotypic performance improvements over the reference *E. coli* strain, thereby identifying unique combinations of genetic variation that confer additional phenotypic performance improvements; and
f. repeating steps d)-e) one or more times, in a linear or non-linear fashion, until an *E. coli* strain exhibits a desired level of improved phenotypic performance compared to the phenotypic performance of the production *E. coli* strain, wherein each subsequent iteration creates a new promoter swap *E. coli* strain library of microbial strains, where each strain in the new library comprises genetic variations that are a combination of genetic variations selected from amongst at least two individual *E. coli* strains of a preceding library.

**4.** The method for improving the phenotypic performance of a production *E. coli* strain according to claim 3, wherein the subsequent promoter swap *E. coli* strain library is a full combinatorial library of the initial promoter swap *E. coli* strain library.

**5.** The method for improving the phenotypic performance of a production *E. coli* strain according to claim 3, wherein the subsequent promoter swap *E. coli* strain library is a subset of a full combinatorial library of the initial promoter swap *E. coli* strain library.

**6.** The method for improving the phenotypic performance of a production *E. coli* strain according to claim 3, wherein the subsequent promoter swap *E. coli* strain library is a full combinatorial library of a preceding promoter swap *E. coli* strain library.

**7.** The method for improving the phenotypic performance of a production *E. coli* strain according to claim 3, wherein the subsequent promoter swap *E. coli* strain library is a subset of a full combinatorial library of a preceding promoter swap *E. coli* strain library.

**8.** The method for improving the phenotypic performance of a production *E. coli* strain according to any one of claims 3-7, wherein steps d)-e) are repeated until the phenotypic performance of an *E. coli* strain of a subsequent promoter swap *E. coli* strain library exhibits at least a 10% increase in a measured phenotypic variable compared to the phenotypic performance of the production *E. coli* strain.

**9.** The method for improving the phenotypic performance of a production *E. coli* strain according to any one of claims 3-7, wherein steps d)-e) are repeated until the phenotypic performance of an *E. coli* strain of a subsequent promoter swap *E. coli* strain library exhibits at least a one-fold increase in a measured phenotypic variable compared to the phenotypic performance of the production *E. coli* strain.

**10.** The method for improving the phenotypic performance of a production *E. coli* strain according to claim 3, wherein the improved phenotypic performance of step f) is selected from the group consisting of: volumetric productivity of a product of interest, specific productivity of a product of interest, yield of a product of interest, titer of a product of interest, and combinations thereof.

**11.** The method for improving the phenotypic performance of a production *E. coli* strain according to claim 3, wherein the improved phenotypic performance of step f) is: increased or more efficient production of a product of interest, said product of interest selected from the group consisting of: a small molecule, enzyme, peptide, amino acid, organic acid, synthetic compound, fuel, alcohol, primary extracellular metabolite, secondary extracellular metabolite, intracellular component molecule, and combinations thereof.

**12.** The method of claim 1 or 3, wherein the 5' UTR/Ribosomal Binding Site (RBS) portion is the 5' UTR/Ribosomal Binding Site (RBS) portion of the lambda phage $p_R$ promoter and nucleic acid sequences of the distal portion of the lambda phage $p_R$ promoter, the variable -35 and -10 regions of the lambda phage $p_L$ and $p_R$ promoters, the core portions of the lambda phage $p_L$ and $p_R$ promoters and the 5' UTR/Ribosomal Binding Site (RBS) portion of the lambda phage $p_R$ promoter are selected from the nucleic acid sequences found in Table 1.5.

**13.** The method of claim 1 or 3, wherein the 5' UTR/Ribosomal Binding Site (RBS) portion is the 5' UTR/Ribosomal Binding Site (RBS) portion of the promoter of the *E. coli acs* gene and nucleic acid sequences of the distal portion of the lambda phage $p_R$ promoter, the variable -35 and -10 regions of the lambda phage $p_L$ and $p_R$ promoters, the core portions of the lambda phage $p_L$ and $p_R$ promoters and the 5' UTR/Ribosomal Binding Site (RBS) portion of the promoter of the *E. coli acs* gene are selected from the nucleic acid sequences found in Table 1.5.

**Patentansprüche**

1. Verfahren zum Erzeugen einer Promotor-Swap-*E. coli*-Stammbibliothek, das die folgenden Schritte beinhaltet:

a. Bereitstellen einer Mehrzahl von Zielgenen, die endogen zu einem *E. coli*-Basisstamm sind, und einer Promotorleiter, wobei die genannte Promotorleiter eine Mehrzahl von Promotoren umfasst, die verschiedene Expressionsprofile in dem *E. coli*-Basisstamm aufweisen, wobei wenigstens einer aus der Mehrzahl von Promotoren ein chimärer synthetischer Promotor ist, wobei der chimäre synthetische Promotor eine Länge von 60-90 Nucleotiden hat und aus Folgendem besteht: einem distalen Abschnitt des Lambda-Phage-*pR*-Promotors, variablen -35 und -10 Regionen des Lambda-Phage-*pL*- und *pR*-Promotors, die jeweils eine Länge von sechs Nucleotiden haben, Kernabschnitten des Lambda-Phage-*pL*- und *pR*-Promotors und einem 5'-UTR/Ribosomenbindungsstellen-(RBS)-Abschnitt, der der 5'-UTR/RBS-Abschnitt des Lambda-Phage-*pR*-Promotors oder der 5'-UTR/Ribosomenbindungsstellen-(RBS)-Abschnitt des Promotors des *E. coli acs*-Gens ist; und
b. Konstruieren des Genoms des *E. coli*-Basisstamms, um dadurch eine anfängliche Promotor-Swap-*E. coli*-Stammbibliothek zu erzeugen, die eine Mehrzahl von individuellen *E. coli*-Stämmen mit eindeutigen genetischen Variationen umfasst, die in jedem Stamm der genannten Mehrzahl von individuellen *E. coli*-Stämmen zu finden sind, wobei jede der genannten eindeutigen genetischen Variationen einen oder mehrere der Promotoren aus der Promotorleiter umfasst, die mit einem der Zielgene operativ verknüpft sind, die endogen zu dem *E. coli*-Basisstamm sind.

2. Verfahren zum Erzeugen einer Promotor-Swap-*E. coli*-Stammbibliothek nach Anspruch 1, wobei wenigstens einer aus der Mehrzahl von Promotoren eine regulatorische Sequenz mit bicistronischem Design (BCD) umfasst.

3. Verfahren zum Verbessern der phänotypischen Leistung eines *E. coli*-Produktionsstamms, das die folgenden Schritte beinhaltet:

a. Bereitstellen einer Mehrzahl von Zielgenen, die endogen zu einem *E. coli*-Basisstamm sind, und einer Promotorleiter, wobei die genannte Promotorleiter eine Mehrzahl von Promotoren umfasst, die verschiedene Expressionsprofile in dem *E. coli*-Basisstamm aufweisen, wobei wenigstens einer aus der Mehrzahl von Promotoren ein chimärer synthetischer Promotor ist, wobei der chimäre synthetische Promotor eine Länge von 60-90 Nucleotiden hat und aus Folgendem besteht: einem distalen Abschnitt des Lambda-Phage-*pR*-Promotors, variablen -35 und -10 Regionen der Lambda-Phage-*pL*- und *pR*-Promotoren, die jeweils eine Länge von sechs Nucleotiden haben, Kernabschnitten der Lambda-Phage-*pL*- und *pR*-Promotoren und einem 5'-UTR/Ribosomenbindungsstellen-(RBS)-Abschnitt, der der 5'-UTR/RBS-Abschnitt des Lambda-Phage-*pR*-Promotors oder der 5'-UTR/Ribosomenbindungsstellen-(RBS)-Abschnitt des Promotors des *E. coli acs*-Gens ist;
b. Konstruieren des Genoms des *E. coli*-Basisstamms, um dadurch eine anfängliche Promotor-Swap-*E. coli*-Stammbibliothek zu erzeugen, die eine Mehrzahl von individuellen *E. coli*-Stämmen mit eindeutigen genetischen Variationen umfasst, die in jedem Stamm aus der genannten Mehrzahl von individuellen *E. coli*-Stämmen zu finden sind, wobei jede der genannten eindeutigen genetischen Variationen einen oder mehrere der Promotoren aus der Promotorleiter umfasst, die mit einem der Zielgene operativ verknüpft sind, die endogen zu dem *E. coli*-Basisstamm sind;
c. Screenen und Selektieren individueller *E. coli*-Stämme der anfänglichen Promotor-Swap-*E. coli*-Stammbibliothek in Bezug auf phänotypische Leistungsverbesserungen gegenüber einem Referenz-*E. coli*-Stamm, um dadurch eindeutige genetische Variationen zu identifizieren, die phänotypische Leistungsverbesserungen verleihen;
d. Bereitstellen einer nachfolgenden Mehrzahl von *E. coli*-Mikroben, die jeweils eine Kombination von eindeutigen genetischen Variationen aus den genetischen Variationen umfassen, die in wenigstens zwei individuellen *E. coli*-Stämmen vorhanden sind, die im vorhergehenden Schritt gescreent wurden, um dadurch eine nachfolgende Promotor-Swap-*E. coli*-Stammbibliothek zu erzeugen;
e. Screenen und Selektieren individueller *E. coli*-Stämme der nachfolgenden Promotor-Swap-*E. coli*-Stammbibliothek in Bezug auf phänotypische Leistungsverbesserungen gegenüber dem Referenz-*E. coli*-Stamm, um dadurch eindeutige Kombinationen genetischer Variationen zu identifizieren, die zusätzliche phänotypische Leistungsverbesserungen verleihen; und
f. einmaliges oder mehrmaliges Wiederholen der Schritte d)-e) auf lineare oder nichtlineare Weise, bis ein *E. coli*-Stamm einen gewünschten Grad an phänotypischer Leistungsverbesserung im Vergleich zur phänotypischen Leistung des *E. coli*-Produktionsstammes aufweist, wobei jede nachfolgende Iteration eine neue Promotor-Swap-*E. coli*-Stammbibliothek von mikrobiellen Stämmen erzeugt, wobei jeder Stamm in der neuen Bibliothek genetische Variationen umfasst, die eine Kombination genetischer Variationen sind, die aus wenigstens zwei individuellen *E. coli*-Stämmen einer vorhergehenden Bibliothek ausgewählt werden.

**4.** Verfahren zum Verbessern der phänotypischen Leistung eines *E. coli*-Produktionsstamms nach Anspruch 3, wobei die nachfolgende Promotor-Swap-*E. coli*-Stammbibliothek eine vollständige kombinatorische Bibliothek der anfänglichen Promotor-Swap-*E. coli*-Stammbibliothek ist.

**5.** Verfahren zum Verbessern der phänotypischen Leistung eines *E. coli*-Produktionsstamms nach Anspruch 3, wobei die nachfolgende Promotor-Swap-*E. coli*-Stammbibliothek eine Teilmenge einer vollständigen kombinatorischen Bibliothek der anfänglichen Promotor-Swap-*E. coli*-Stammbibliothek ist.

**6.** Verfahren zum Verbessern der phänotypischen Leistung eines *E. coli*-Produktionsstamms nach Anspruch 3, wobei die nachfolgende Promotor-Swap-*E. coli*-Stammbibliothek eine vollständige kombinatorische Bibliothek einer vorhergehenden Promotor-Swap-*E. coli*-Stammbibliothek ist.

**7.** Verfahren zum Verbessern der phänotypischen Leistung eines *E. coli*-Produktionsstamms nach Anspruch 3, wobei die nachfolgende Promotor-Swap-*E. coli*-Stammbibliothek eine Teilmenge einer vollständigen kombinatorischen Bibliothek einer vorhergehenden Promotor-Swap-*E. coli*-Stammbibliothek ist.

**8.** Verfahren zum Verbessern der phänotypischen Leistung eines *E. coli*-Produktionsstamms nach einem der Ansprüche 3-7, wobei die Schritte d)-e) wiederholt werden, bis die phänotypische Leistung eines *E. coli*-Stamms einer nachfolgenden Promotor-Swap-*E. coli*-Stammbibliothek wenigstens eine 10%ige Zunahme einer gemessenen phänotypischen Variablen im Vergleich zur phänotypischen Leistung des *E. coli*-Produktionsstammes aufweist.

**9.** Verfahren zum Verbessern der phänotypischen Leistung eines *E. coli*-Produktionsstamms nach einem der Ansprüche 3-7, wobei die Schritte d)-e) wiederholt werden, bis die phänotypische Leistung eines *E. coli*-Stamms einer nachfolgenden Promotor-Swap-*E. coli*-Stammbibliothek wenigstens eine einfache Zunahme einer gemessenen phänotypischen Variablen im Vergleich zur phänotypischen Leistung des *E. coli*-Produktionsstamms aufweist.

**10.** Verfahren zum Verbessern der phänotypischen Leistung eines *E. coli*-Produktionsstamms nach Anspruch 3, wobei die verbesserte phänotypische Leistung von Schritt f) ausgewählt wird aus der Gruppe bestehend aus volumetrischer Produktivität eines Produkts von Interesse, spezifischer Produktivität eines Produkts von Interesse, Ausbeute eines Produkts von Interesse, Titer eines Produkts von Interesse und Kombinationen davon.

**11.** Verfahren zum Verbessern der phänotypischen Leistung eines *E. coli*-Produktionsstamms nach Anspruch 3, wobei die verbesserte phänotypische Leistung von Schritt f) Folgendes ist: erhöhte oder effizientere Produktion eines Produkts von Interesse, wobei das genannte Produkt von Interesse ausgewählt wird aus der Gruppe bestehend aus: einem kleinen Molekül, Enzym, Peptid, Aminosäure, organischer Säure, synthetischer Verbindung, Brennstoff, Alkohol, primärem extrazellulärem Metaboliten, sekundärem extrazellulärem Metaboliten, intrazellulärem Komponentenmolekül und Kombinationen davon.

**12.** Verfahren nach Anspruch 1 oder 3, wobei der 5'-UTR/Ribosomenbindungsstellen-(RBS)-Abschnitt der 5'-UTR/Ribosomenbindungsstellen-(RBS)-Abschnitt des Lambda-Phage-*pR*-Promotors ist und Nucleinsäuresequenzen des distalen Abschnitts des Lambda-Phage-*pR*-Promotors, die variablen -35 und -10 Regionen der Lambda-Phage-*pL*- und - *pR*-Promotoren, die Kernabschnitte der Lambda-Phage-*pL*- und -*pR*-Promotoren und der 5'UTR/Ribosomenbindungsstellen-(RBS)-Abschnitt des Lambda-Phage-*pR*-Promotors aus den in Tabelle 1.5 aufgeführten Nucleinsäuresequenzen ausgewählt werden.

**13.** Verfahren nach Anspruch 1 oder 3, wobei der 5' UTR/Ribosomenbindungsstellen-(RBS)-Abschnitt der 5' UTR/Ribosomenbindungsstellen-(RBS)-Abschnitt des Promotors des *E. coli acs*-Gens ist und die Nucleinsäuresequenzen des distalen Abschnitts des Lambda-Phage-*pR*-Promotors, die variablen -35 und -10 Regionen der Lambda-Phage-*pL*- und - *pR*-Promotoren, die Kernabschnitte der Lambda-Phage-*pL*- und -*pR*-Promotoren und der 5'UTR/Ribosomenbindungsstellen-(RBS)-Abschnitt des Promotors des *E. coli acs*-Gens aus den in Tabelle 1.5 aufgeführten Nucleinsäuresequenzen ausgewählt sind.

## Revendications

**1.** Procédé de génération d'une bibliothèque de souches d'*E. coli* à échange de promoteurs, comprenant les étapes consistant à :

a) obtenir une pluralité de gènes cibles endogènes à une souche d'*E. coli* de base et une échelle de promoteurs, ladite échelle de promoteurs comprenant une pluralité de promoteurs qui présentent différents profils d'expression dans la souche d'*E. coli* de base, l'un au moins de la pluralité de promoteurs étant un promoteur chimérique de synthèse, lequel promoteur chimérique de synthèse a une longueur de 60 à 90 nucléotides et se compose d'une partie distale de promoteur $p_R$ de phage lambda, des régions variables -35 et -10 des promoteurs $p_L$ et $p_R$ de phage lambda, qui ont chacune une longueur de six nucléotides, des parties centrales des promoteurs $p_L$ et $p_R$ de phage lambda et d'une partie 5' UTR/RBS (site de liaison ribosomique) qui est la partie 5' UTR/RBS du promoteur $p_R$ de phage lambda ou la partie 5' UTR/RBS (site de liaison ribosomique) du promoteur du gène *acs d'E. coli ;* et
b) manipuler le génome de la souche d'*E. coli* de base afin de créer ainsi une bibliothèque initiale de souches d'*E. coli* à échange de promoteurs comprenant une pluralité de souches d'*E. coli* individuelles avec des variations génétiques uniques présentes dans chaque souche de ladite pluralité de souches d'*E. coli* individuelles, chacune desdites variations génétiques uniques comprenant un ou plusieurs des promoteurs de l'échelle de promoteurs lié(s) fonctionnellement à l'un des gènes cibles endogènes à la souche d'*E. coli* de base.

**2.** Procédé de génération d'une bibliothèque de souches d'*E. coli* à échange de promoteurs selon la revendication 1, dans lequel l'un au moins de la pluralité de promoteurs comprend une séquence régulatrice de structure bicistronique (BCD) .

**3.** Procédé d'amélioration de la performance phénotypique d'une souche d'*E. coli* de production, comprenant les étapes consistant à :

a) obtenir une pluralité de gènes cibles endogènes à une souche d'*E. coli* de base et une échelle de promoteurs, ladite échelle de promoteurs comprenant une pluralité de promoteurs qui présentent différents profils d'expression dans la souche d'*E. coli* de base, l'un au moins de la pluralité de promoteurs étant un promoteur chimérique de synthèse, lequel promoteur chimérique de synthèse a une longueur de 60 à 90 nucléotides et se compose d'une partie distale de promoteur $p_R$ de phage lambda, des régions variables -35 et -10 de promoteurs $p_L$ et $p_R$ de phage lambda ayant chacune une longueur de six nucléotides, des parties centrales des promoteurs $p_L$ et $p_R$ de phage lambda, et d'une partie 5' UTR/RBS (site de liaison ribosomique) qui est la partie 5' UTR/RBS du promoteur $p_R$ de phage lambda ou la partie 5' UTR/RBS (site de liaison ribosomique) du promoteur du gène *acs* d'*E. coli ;*
b) manipuler le génome de la souche d'*E. coli* de base afin de créer ainsi une bibliothèque initiale de souches d'*E. coli* à échange de promoteurs comprenant une pluralité de souches d'*E. coli* individuelles avec des variations génétiques uniques présentes dans chaque souche de ladite pluralité de souches d'*E. coli* individuelles, chacune desdites variations génétiques uniques comprenant un ou plusieurs des promoteurs de l'échelle de promoteurs lié(s) fonctionnellement à l'un des gènes cibles endogènes à la souche d'*E. coli* de base ;
c) cribler et sélectionner des souches d'*E. coli* individuelles de la bibliothèque initiale de souches d'*E. coli* à échange de promoteurs pour des améliorations de la performance phénotypique par rapport à une souche d'*E. coli* de référence afin d'identifier ainsi des variations génétiques uniques qui donnent des améliorations de la performance phénotypique ;
d) obtenir une pluralité suivante de microbes *E. coli* qui comprennent chacun une combinaison de variations génétiques uniques parmi les variations génétiques présentes dans au moins deux souches d'*E. coli* individuelles criblées dans l'étape précédente afin de créer ainsi une bibliothèque suivante de souches d'*E. coli* à échange de promoteurs ;
e) cribler et sélectionner des souches d'*E. coli* individuelles de la bibliothèque suivante de souches d'*E. coli* à échange de promoteurs pour des améliorations de la performance phénotypique par rapport à la souche d'*E. coli* de référence afin d'identifier ainsi des combinaisons uniques de variations génétiques qui donnent des améliorations supplémentaires de la performance phénotypique ; et
f) répéter les étapes d)-e) à une ou plusieurs reprises, de manière linéaire ou non linéaire, jusqu'à ce qu'une souche d'*E. coli* présente un niveau voulu de performance phénotypique améliorée comparée à la performance phénotypique de la souche d'*E. coli* de production, chaque itération suivante créant une nouvelle bibliothèque de souches *d'E. coli* à échange de promoteurs de souches microbiennes, chaque souche dans la nouvelle bibliothèque comprenant des variations génétiques qui sont une combinaison de variations génétiques choisies parmi au moins deux souches d'*E. coli* individuelles d'une bibliothèque précédente.

**4.** Procédé d'amélioration de la performance phénotypique d'une souche d'*E. coli* de production selon la revendication 3, dans lequel la bibliothèque suivante de souches d'*E. coli* à échange de promoteurs est une bibliothèque combinatoire complète de la bibliothèque initiale de souches d'*E. coli* à échange de promoteurs.

**5.** Procédé d'amélioration de la performance phénotypique d'une souche d'*E. coli* de production selon la revendication 3, dans lequel la bibliothèque suivante de souches d'*E. coli* à échange de promoteurs est un sous-ensemble d'une bibliothèque combinatoire complète de la bibliothèque initiale de souches d'*E. coli* à échange de promoteurs.

**6.** Procédé d'amélioration de la performance phénotypique d'une souche d'*E. coli* de production selon la revendication 3, dans lequel la bibliothèque suivante de souches d'*E. coli* à échange de promoteurs est une bibliothèque combinatoire complète d'une bibliothèque précédente de souches d'*E. coli* à échange de promoteurs.

**7.** Procédé d'amélioration de la performance phénotypique d'une souche d'*E. coli* de production selon la revendication 3, dans lequel la bibliothèque suivante de souches d'*E. coli* à échange de promoteurs est un sous-ensemble d'une bibliothèque combinatoire complète d'une bibliothèque précédente de souches d'*E. coli* à échange de promoteurs.

**8.** Procédé d'amélioration de la performance phénotypique d'une souche d'*E. coli* de production selon l'une quelconque des revendications 3 à 7, dans lequel les étapes d)-e) sont répétées jusqu'à ce que la performance phénotypique d'une souche d'*E. coli* d'une bibliothèque suivante de souches d'*E. coli* à échange de promoteurs présente au moins une augmentation de 10% d'une variable phénotypique mesurée comparée à la performance phénotypique de la souche d'*E. coli* de production.

**9.** Procédé d'amélioration de la performance phénotypique d'une souche d'*E. coli* de production selon l'une quelconque des revendications 3 à 7, dans lequel les étapes d)-e) sont répétées jusqu'à ce que la performance phénotypique d'une souche d'*E. coli* d'une bibliothèque suivante de souches d'*E. coli* à échange de promoteurs présente au moins une augmentation d'une fois d'une variable phénotypique mesurée comparée à la performance phénotypique de la souche d'*E. coli* de production.

**10.** Procédé d'amélioration de la performance phénotypique d'une souche d'*E. coli* de production selon la revendication 3, dans lequel la performance phénotypique améliorée de l'étape f) est choisie dans le groupe constitué par : la productivité volumétrique d'un produit d'intérêt, la productivité spécifique d'un produit d'intérêt, le rendement d'un produit d'intérêt, le titre d'un produit d'intérêt, et des combinaisons de ceux-ci.

**11.** Procédé d'amélioration de la performance phénotypique d'une souche d'*E. coli* de production selon la revendication 3, dans lequel la performance phénotypique améliorée de l'étape f) est une production augmentée ou plus efficace d'un produit d'intérêt, ledit produit d'intérêt étant choisi dans le groupe constitué par : une petite molécule, une enzyme, un peptide, un acide aminé, un acide organique, un composé synthétique, un combustible, un alcool, un métabolite extracellulaire primaire, un métabolite extracellulaire secondaire, une molécule de composant intracellulaire, et des combinaisons de ceux-ci.

**12.** Procédé selon la revendication 1 ou la revendication 3, dans lequel la partie 5' UTR/RBS (site de liaison ribosomique) est la partie 5' UTR/RBS (site de liaison ribosomique) du promoteur $p_R$ de phage lambda et des séquences d'acide nucléique de la partie distale du promoteur $p_R$ de phage lambda, des régions variables -35 et -10 des promoteurs $p_L$ et $p_R$ de phage lambda, des parties centrales des promoteurs $p_L$ et $p_R$ de phage lambda et de la partie 5' UTR/RBS (site de liaison ribosomique) du promoteur $p_R$ de phage lambda sont choisies parmi les séquences d'acide nucléique qui se trouvent dans le tableau 1.5.

**13.** Procédé selon la revendication 1 ou la revendication 3, dans lequel la partie 5' UTR/RBS (site de liaison ribosomique) est la partie 5' UTR/RBS (site de liaison ribosomique) du promoteur du gène *acs* d'*E. coli* et des séquences d'acide nucléique de la partie distale du promoteur $p_R$ de phage lambda, des régions variables -35 et -10 des promoteurs $p_L$ et $p_R$ de phage lambda, des parties centrales des promoteurs $p_L$ et $p_R$ de phage lambda et de la partie 5' UTR/RBS (site de liaison ribosomique) du promoteur du gène *acs* d'*E. coli* sont choisies parmi les séquences d'acide nucléique qui se trouvent dans le tableau 1.5.

**FIGURE 1**

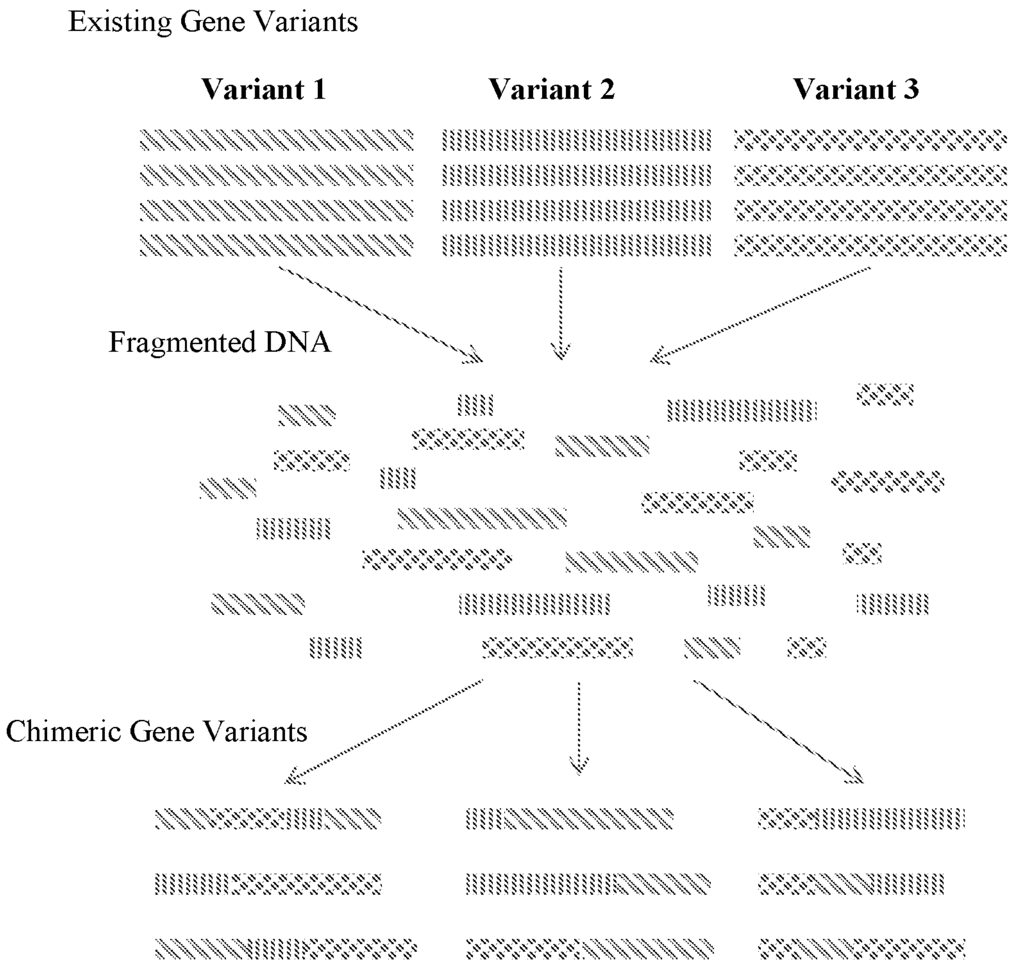
Existing Gene Variants
Variant 1
Variant 2
Variant 3
Fragmented DNA
Chimeric Gene Variants

**FIGURE 2**

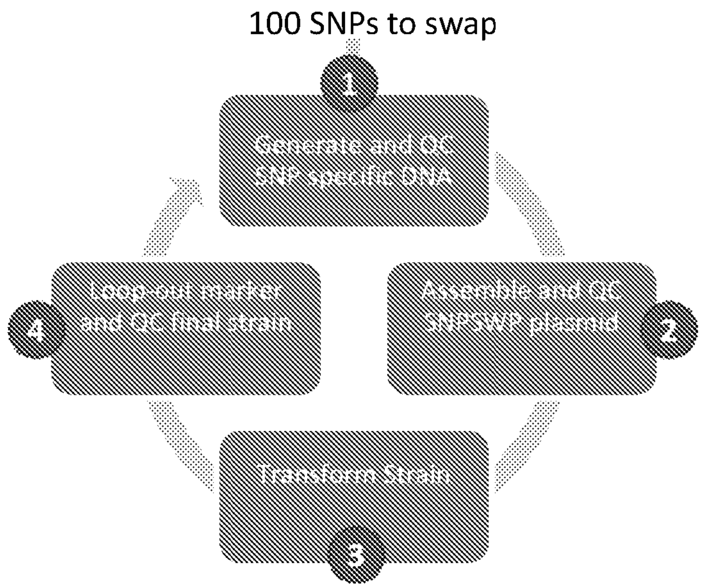
100 SNPs to swap
1
Generate and QC SNP specific DNA
2
Assemble and QC SNPSWP plasmid
3
Transform Strain
4
Loop-out marker and QC final strain

FIGURE 3

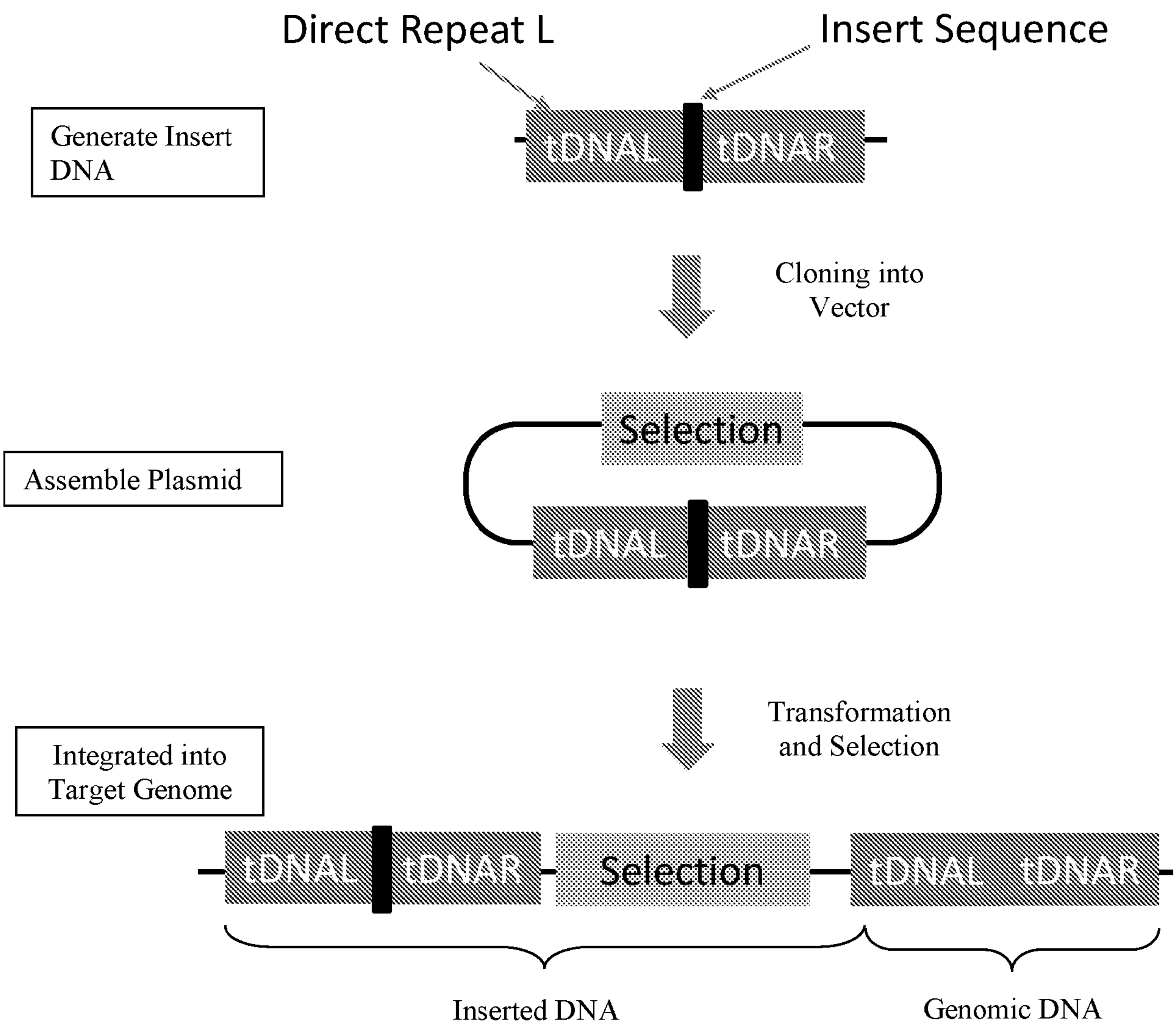
Direct Repeat L
Insert Sequence
Generate Insert DNA
tDNAL
tDNAR
Cloning into Vector
Assemble Plasmid
Selection
tDNAL
tDNAR
Transformation and Selection
Integrated into Target Genome
tDNAL
tDNAR
Selection
tDNAL
tDNAR
Inserted DNA
Genomic DNA

FIGURE 4

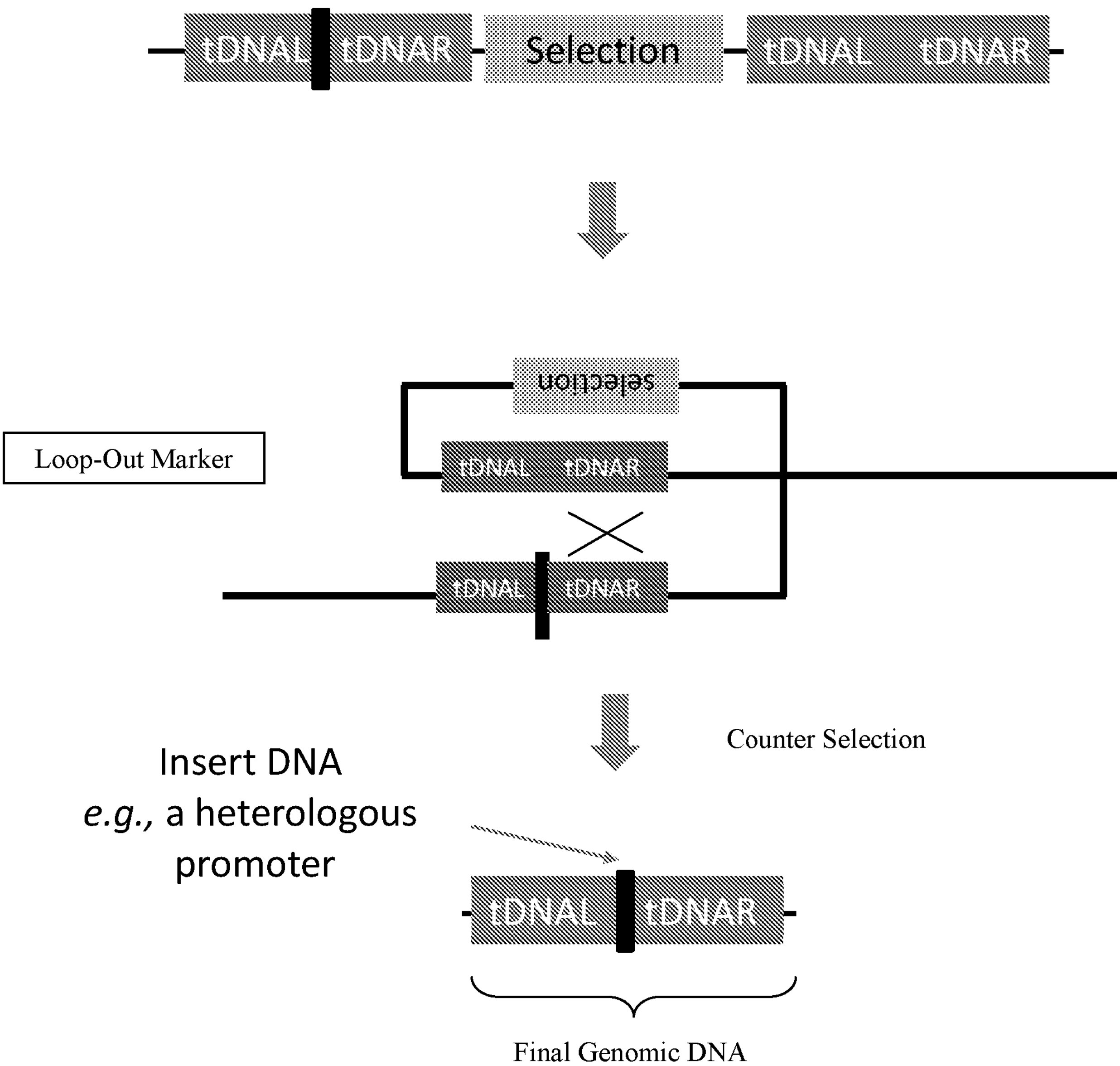
tDNAL
tDNAR
Selection
tDNAL
tDNAR
selection
Loop-Out Marker
tDNAL
tDNAR
tDNAL
tDNAR
Counter Selection
Insert DNA
e.g., a heterologous
promoter
tDNAL
tDNAR
Final Genomic DNA

**FIGURE 5**

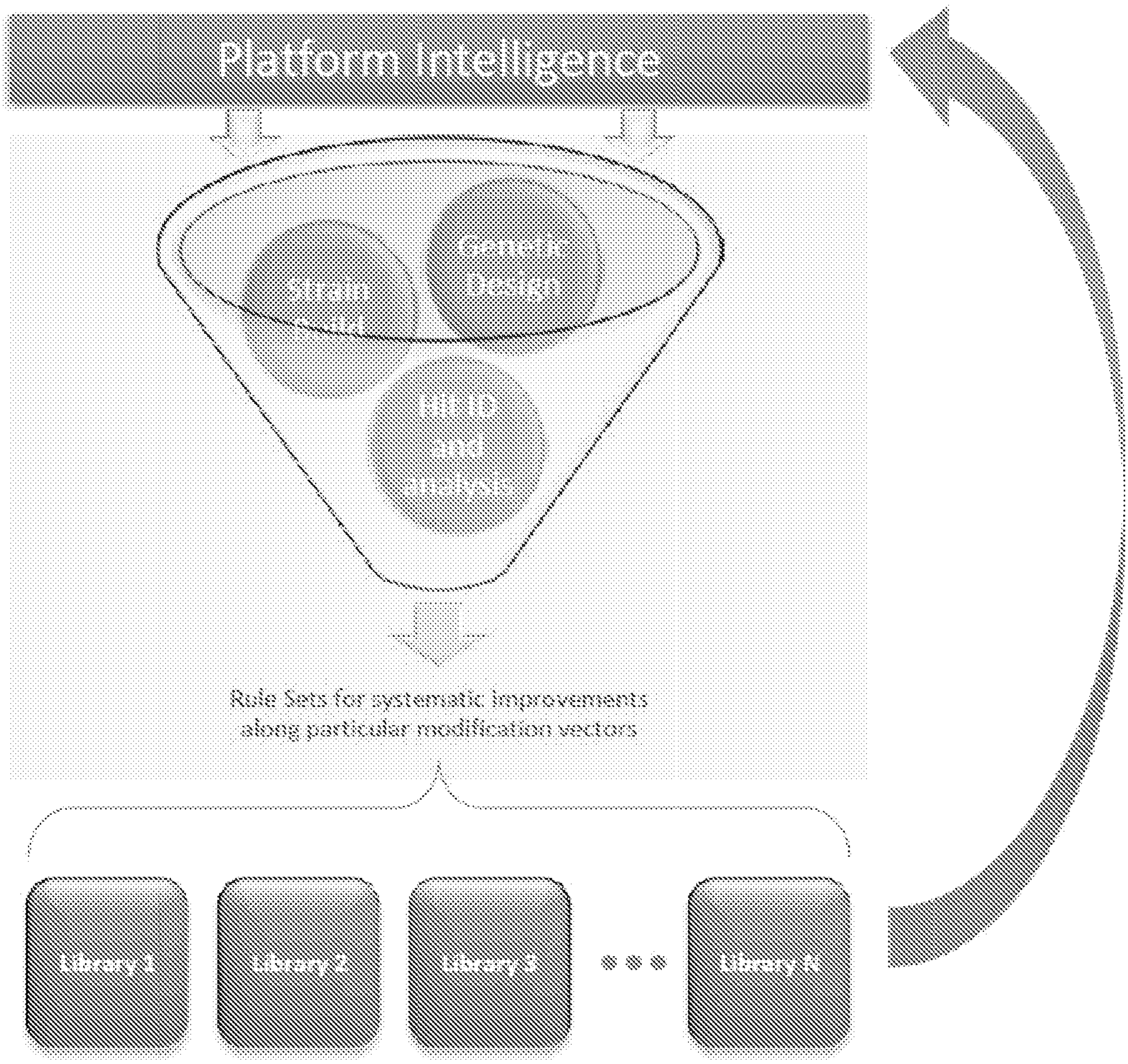
Platform Intelligence
Strain
Genetic Design
HT ID
and
Rule Sets for systematic improvements
along particular modification vectors
Library 1
Library 2
Library 3
Library n

**FIGURE 6A**

**Step 1 Build Vectors for Transformation- Instructions based on data from Step 3 or 4** — Go to 2

**Acquire and Build DNA**

- Source DNA fragments from vendors or internal storage.
- Alternatively synthesize DNA fragments in house.
- Join smaller DNA fragments into larger inserts for vector cloning.
- Evaluate joined DNA inserts prior to cloning.

**Assemble Vectors**

- Stitch together DNA inserts into vectors for genomic insertion into host organisms.
- Evaluate vectors prior to transformation.

**Step 2 Strain Transformation- Vectors from Step 1** — Go to 3

**Transform Vectors into Host Cell**

- Transform host cell with DNA Vectors.
- Select for transformed cells and evaluate cassette insertion into genomic DNA.

**Remove Selection Markers**

- Loop out selection markers through counter selection.
- Evaluate insertion site to confirm deletion of loop out regions.

**FIGURE 6B**

**Step 3 Strain Evaluation in Multi-Well Plate- Test Strains from Step 2** — Go to 4 or 1

| Culture Strains on Plate | Generate Product on Plate | Evaluate Performance |
|---|---|---|
| • Transfer strains onto culture plate with growth media.<br>• Allow cultures to grow to predetermined density (e.g., OD at 600nm). | • Transfer aliquots from culture plate to production plate based on actual culture concentrations.<br>• Production plates include media and conditions designed for product generation. | • Perform one or more assays to test performance (i.e. product concentration).<br>• Real time evaluation possible.<br>• Store selected cultures in cold storage. |

**Step 4 Strain Evaluation in Tank- Test Top Candidates from Step 3** — END or Back to 1-3

| Culture Strains on Flasks | Generate Product in Tank | Evaluate Performance |
|---|---|---|
| • Transfer strains into culture flask with growth media.<br>• Allow cultures to grow to predetermined density (e.g., OD at 600nm). | • Transfer aliquots from culture flask to production tank based on actual culture concentrations.<br>• Production tanks include media and conditions designed for product generation.<br>• Production tanks may also include initial biomass growth period. | • Perform one or more assays to test performance (i.e. product concentration).<br>• Evaluation typically at mid-point and end-point of cultures. |

**FIGURE 7**

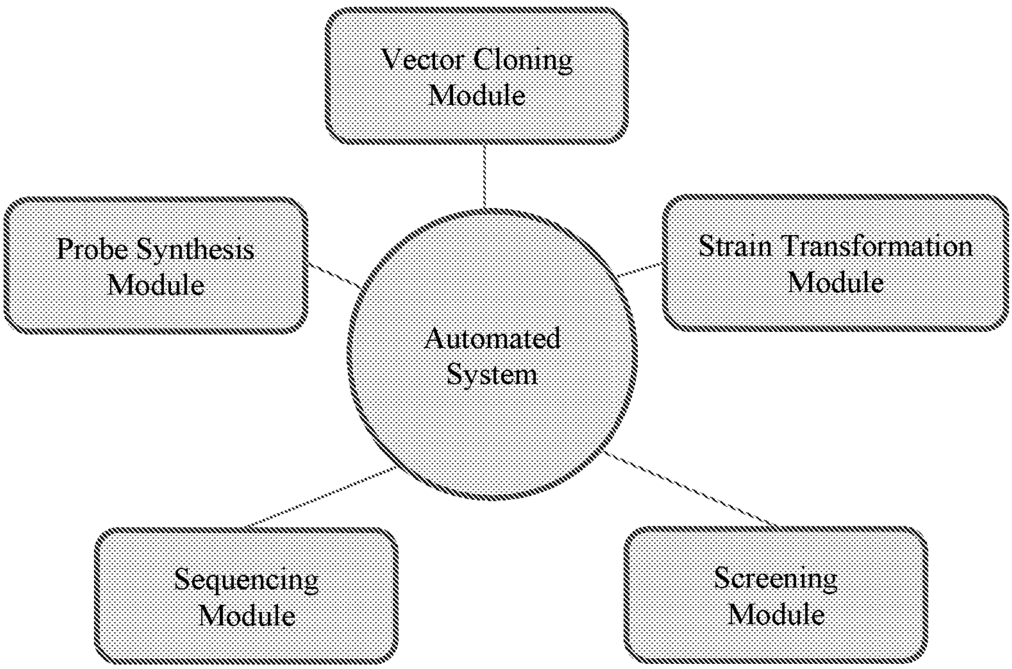
Vector Cloning Module
Probe Synthesis Module
Strain Transformation Module
Automated System
Sequencing Module
Screening Module

FIGURE 8

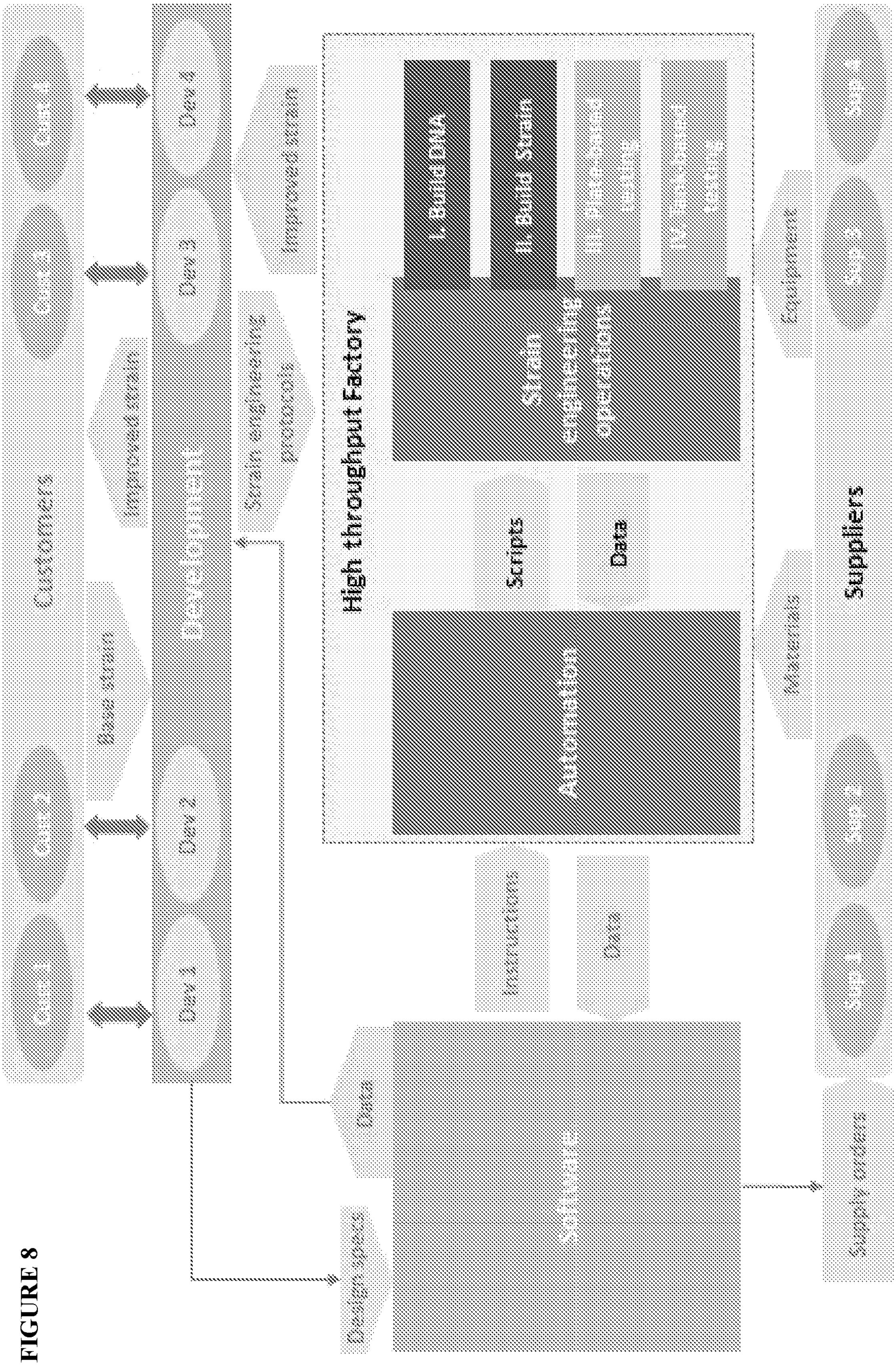
Customers
Cust 1
Cust 2
Cust 3
Cust 4
Base strain
Improved strain
Development
Dev 1
Dev 2
Dev 3
Dev 4
Strain engineering protocols
Improved strain
High throughput Factory
I. Build DNA
II. Build Strain
III. Plate-based testing
IV. Tank-based testing
Strain engineering operations
Scripts
Data
Automation
Instructions
Data
Data
Design specs
Software
Supply orders
Materials
Equipment
Suppliers
Sup 1
Sup 2
Sup 3
Sup 4

FIGURE 9

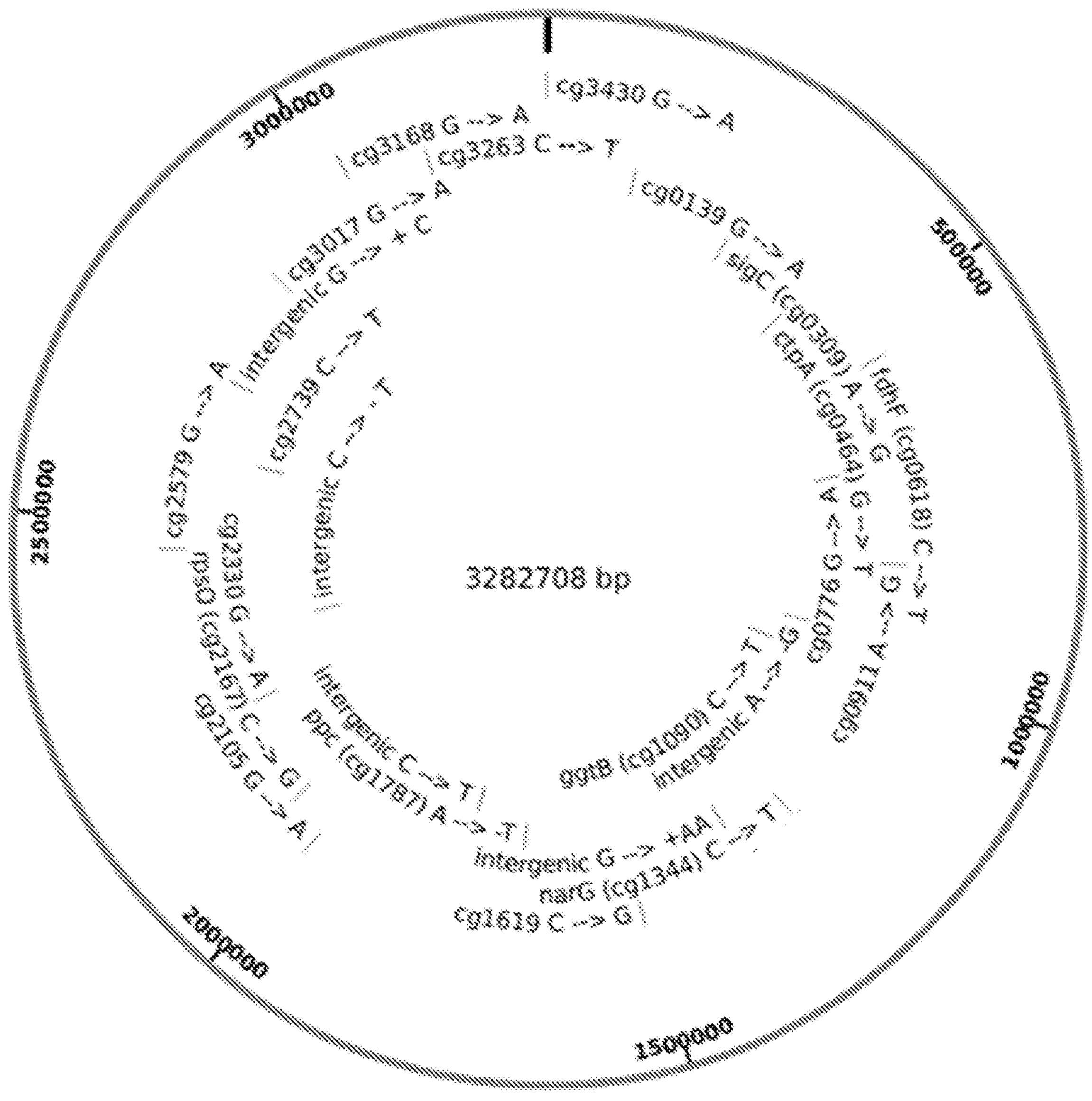
3282708 bp
500000
1000000
1500000
2000000
2500000
3000000
cg3430 G --> A
cg3168 G --> A
cg3263 C --> T
cg0139 G --> A
cg3017 G --> A
intergenic G --> + C
sigC (cg0309) A --> G
ctpA (cg0464) G --> T
fdhF (cg0618) C --> T
cg2739 C --> T
cg2579 G --> A
intergenic C --> - T
cg0776 G --> A
cg0911 A --> G
intergenic
cg2330 G --> A
rpsO (cg2167) C --> G
cg2105 G --> A
intergenic C --> T
ppc (cg1787) A --> -T
ggtB (cg1090) C --> T
intergenic A --> -G
intergenic G --> +AA
narG (cg1344) C --> T
cg1619 C --> G

FIGURE 10

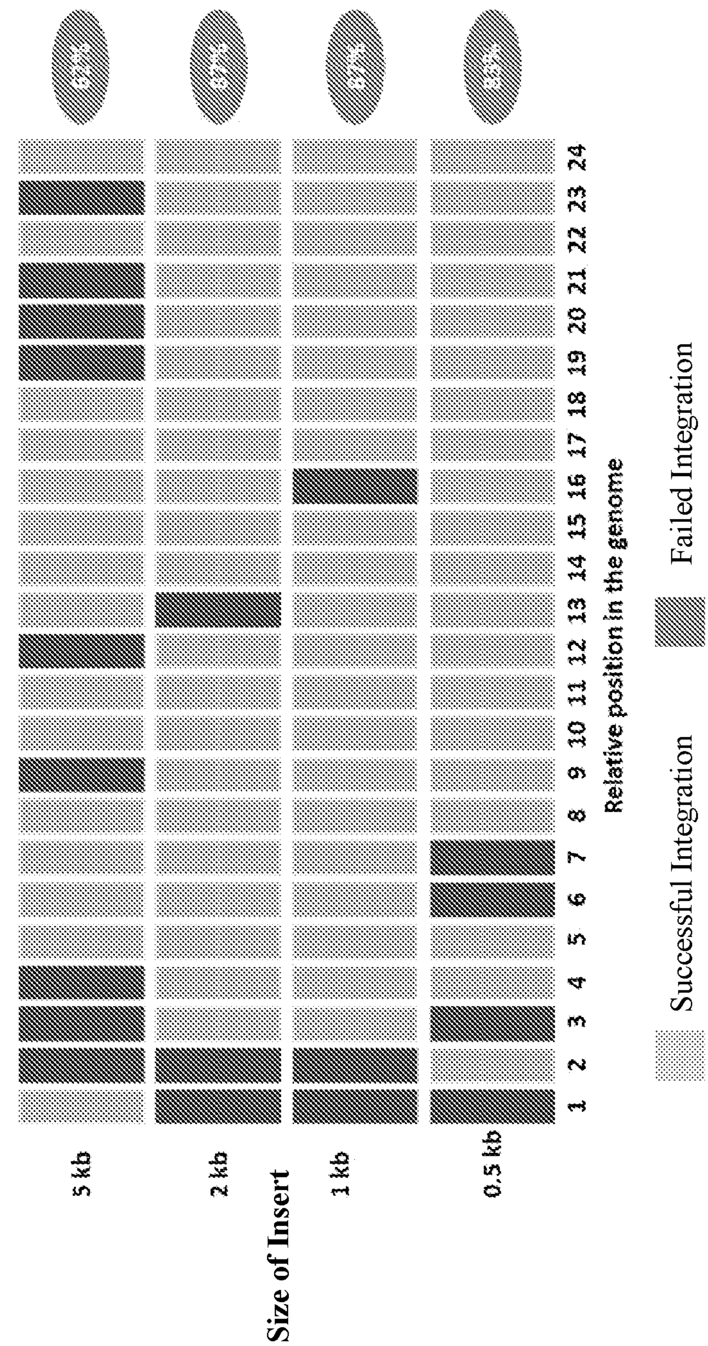
Size of Insert
5 kb
2 kb
1 kb
0.5 kb
1 2 3 4 5 6 7 8 9 10 11 12 13 14 15 16 17 18 19 20 21 22 23 24
Relative position in the genome
Successful Integration
Failed Integration

FIGURE 11

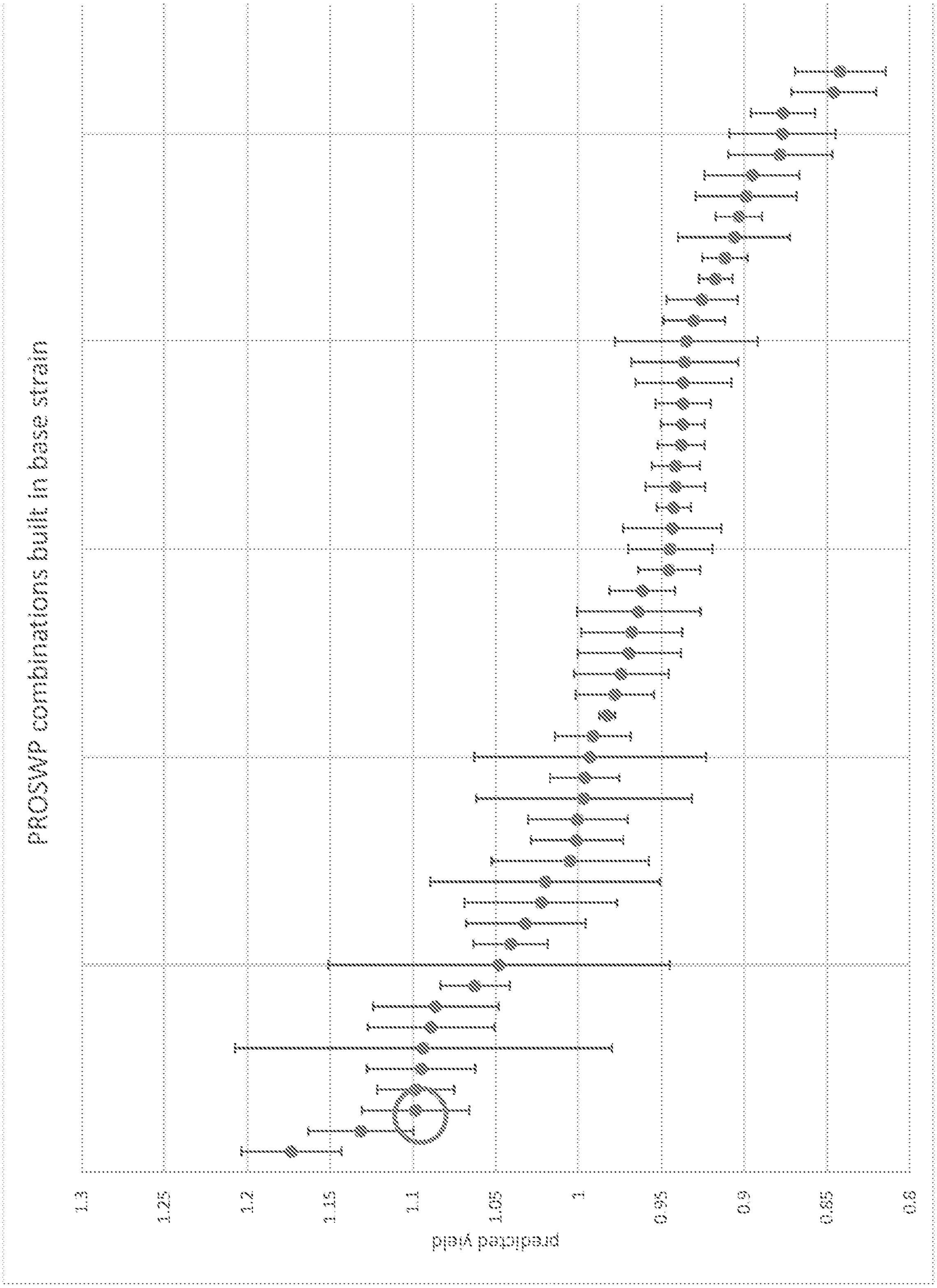
PROSWP combinations built in base strain
predicted yield
1.3
1.25
1.2
1.15
1.1
1.05
1
0.95
0.9
0.85
0.8

FIGURE 12

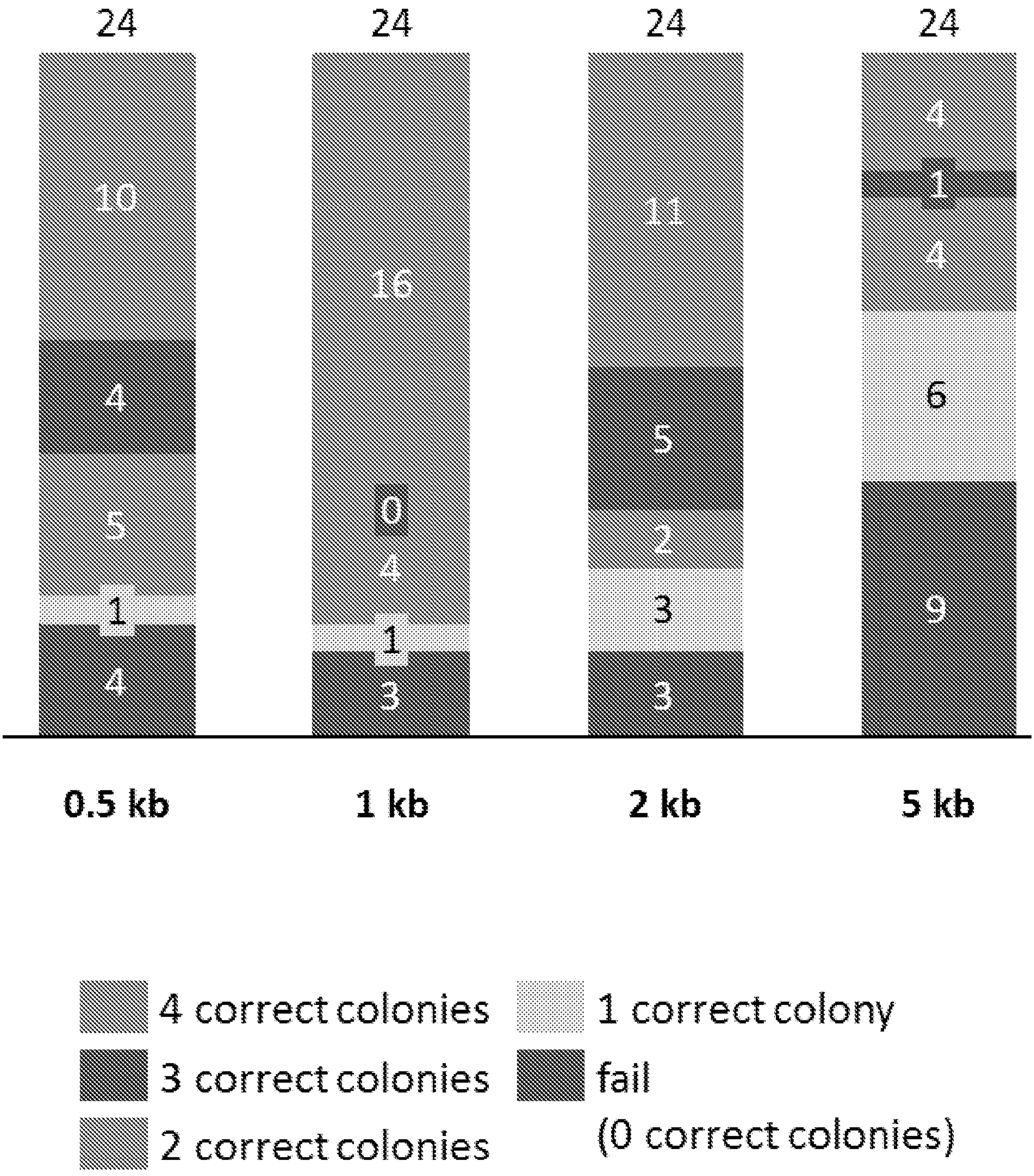
24
24
24
24
10
4
5
1
4
16
0
4
1
3
11
5
2
3
3
4
1
4
6
9
0.5 kb
1 kb
2 kb
5 kb
4 correct colonies
3 correct colonies
2 correct colonies
1 correct colony
fail
(0 correct colonies)

FIGURE 13

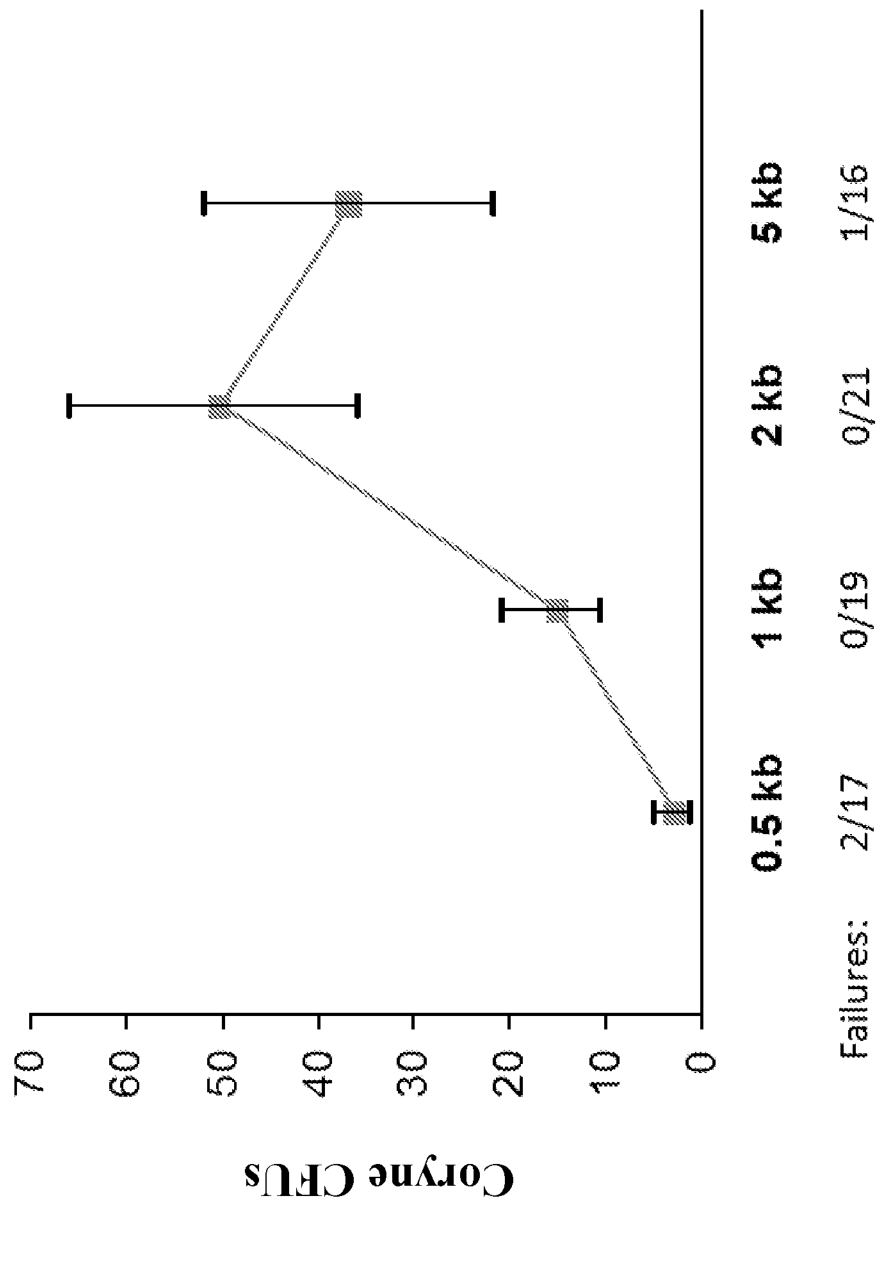
Coryne CFUs
70
60
50
40
30
20
10
0
0.5 kb
1 kb
2 kb
5 kb
Failures:
2/17
0/19
0/21
1/16

FIGURE 14

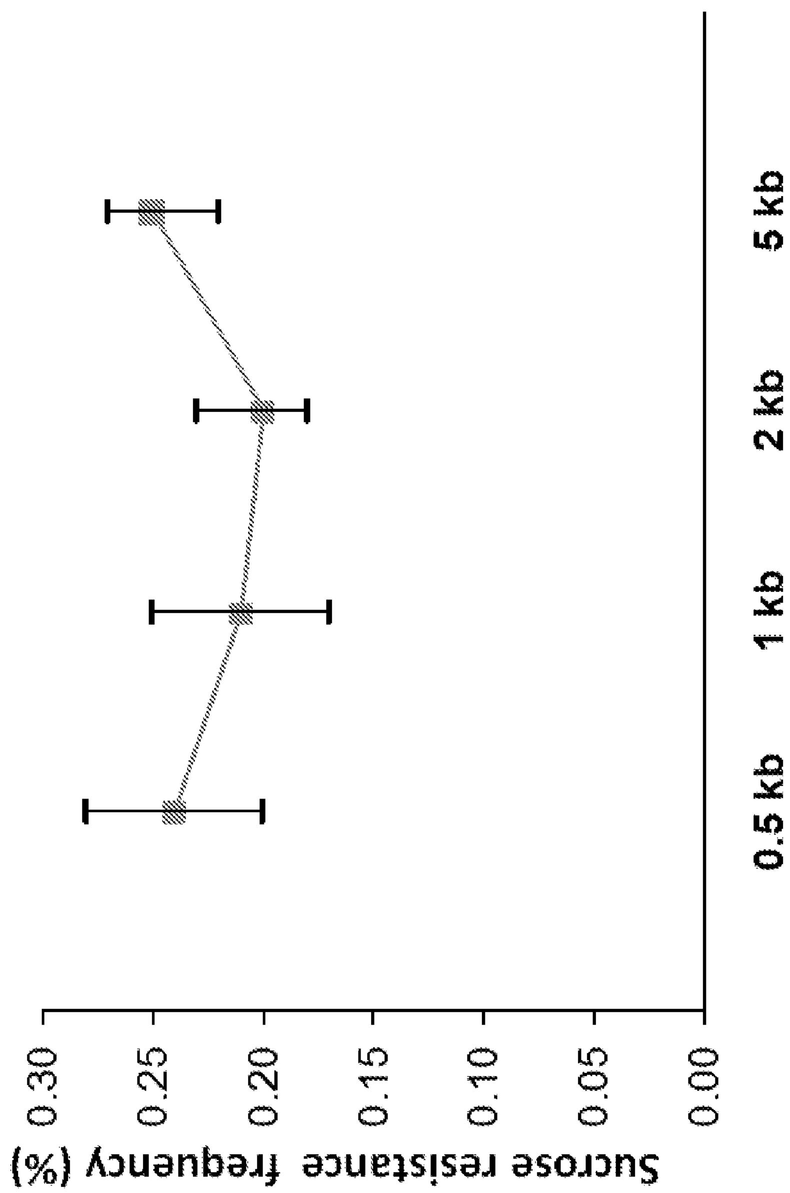
Sucrose resistance frequency (%)
0.30
0.25
0.20
0.15
0.10
0.05
0.00
0.5 kb
1 kb
2 kb
5 kb

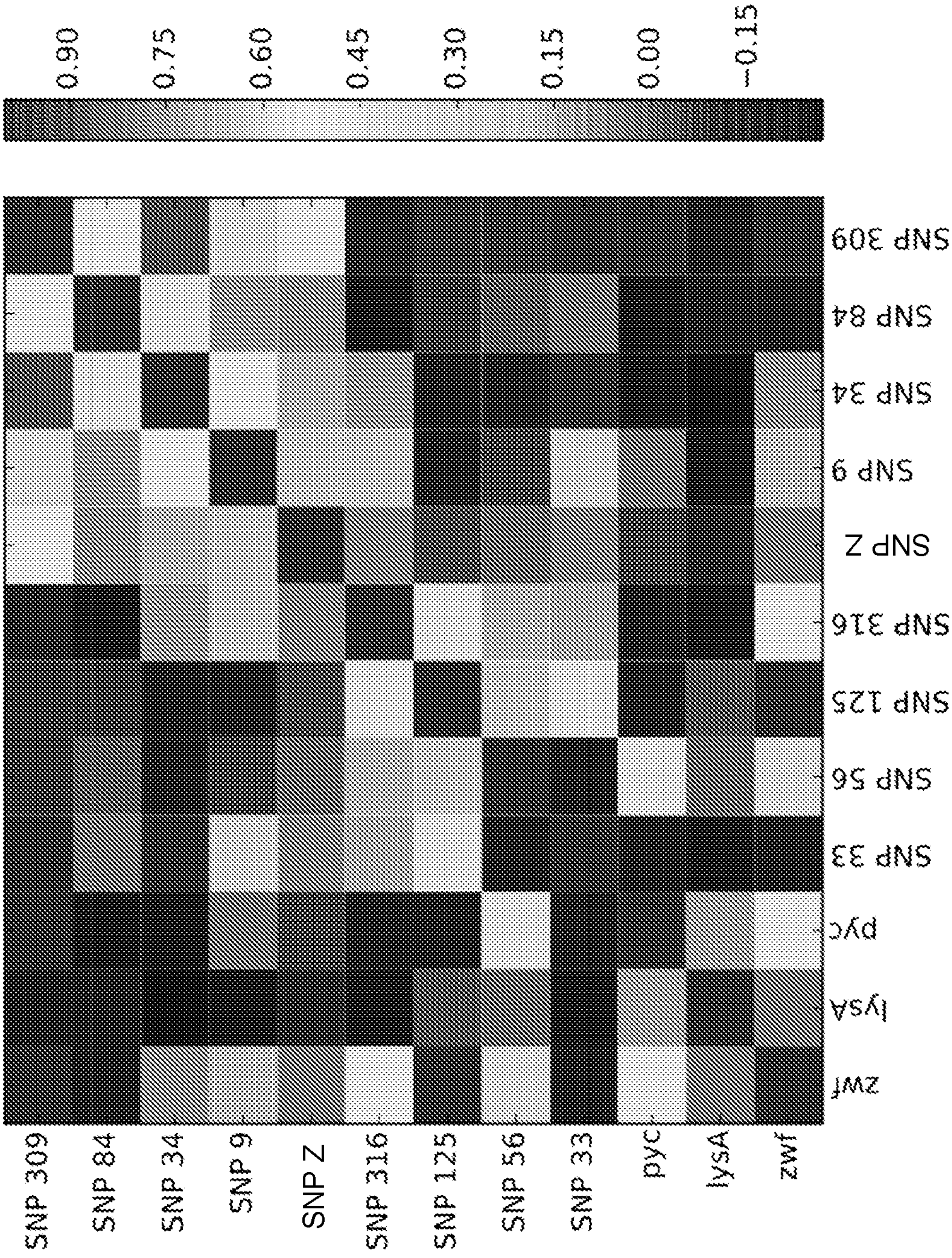
0.90
0.75
0.60
0.45
0.30
0.15
0.00
−0.15
SNP 309
SNP 84
SNP 34
SNP 9
SNP Z
SNP 316
SNP 125
SNP 56
SNP 33
pyc
lysA
zwf

FIGURE 15

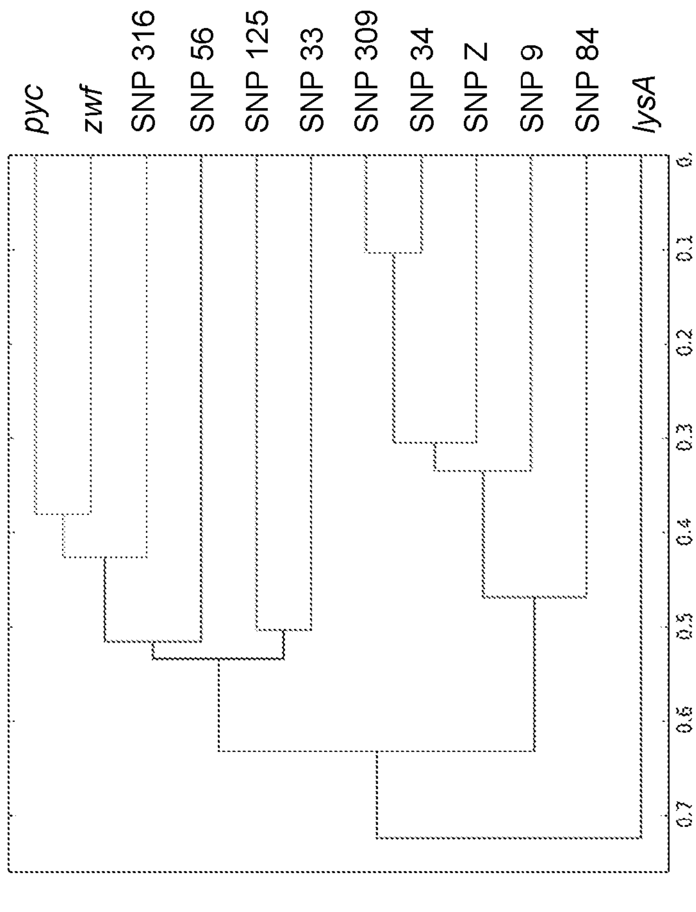


FIGURE 16 A

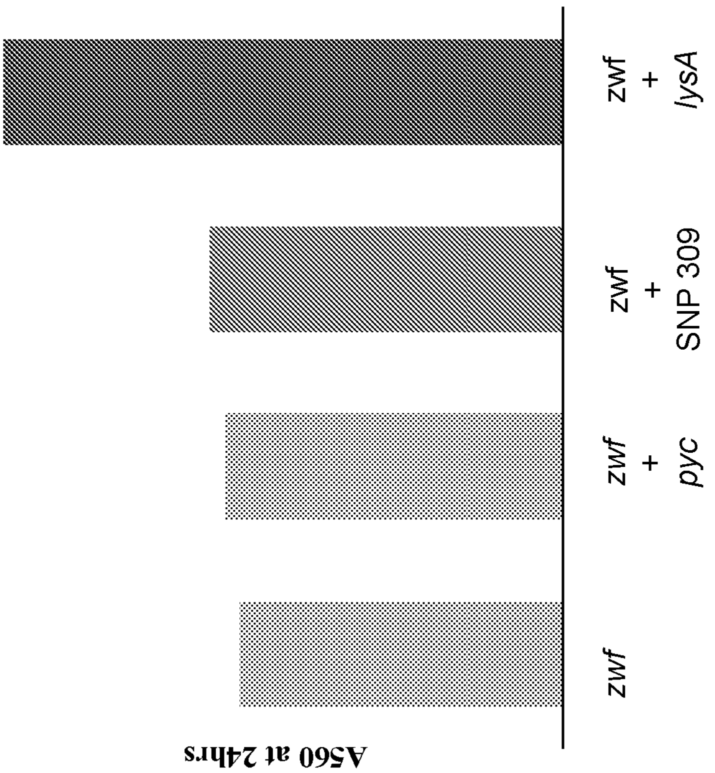


FIGURE 16 B

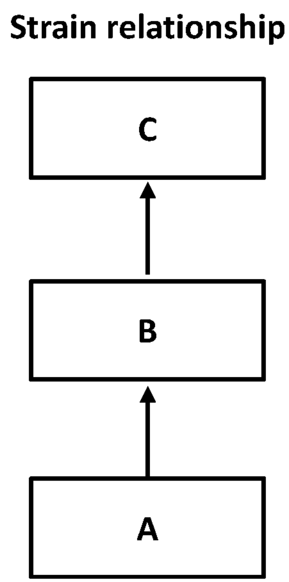


FIGURE 17 A

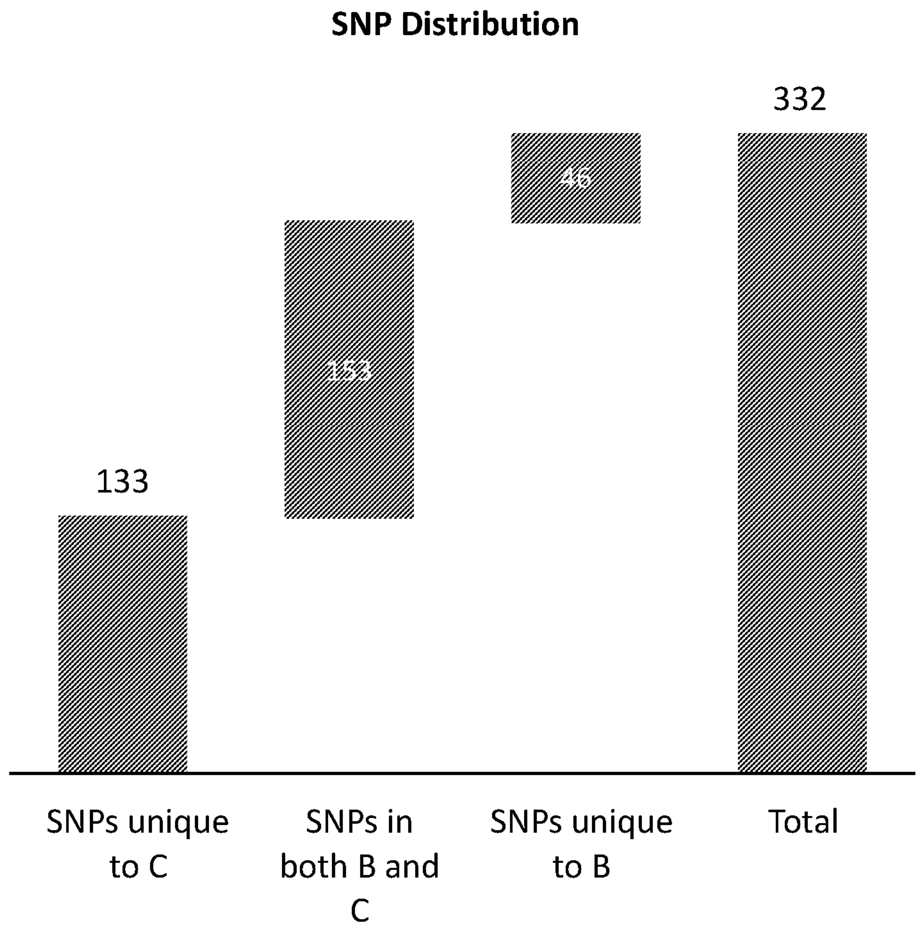


FIGURE 17 B

FIGURE 18

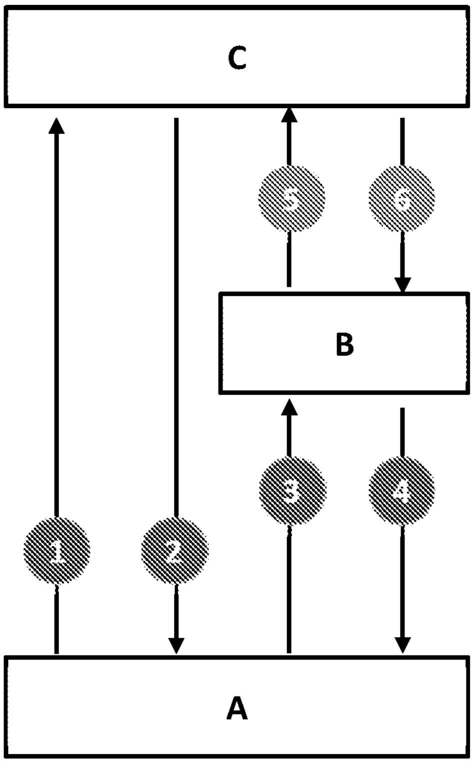

FIGURE 19

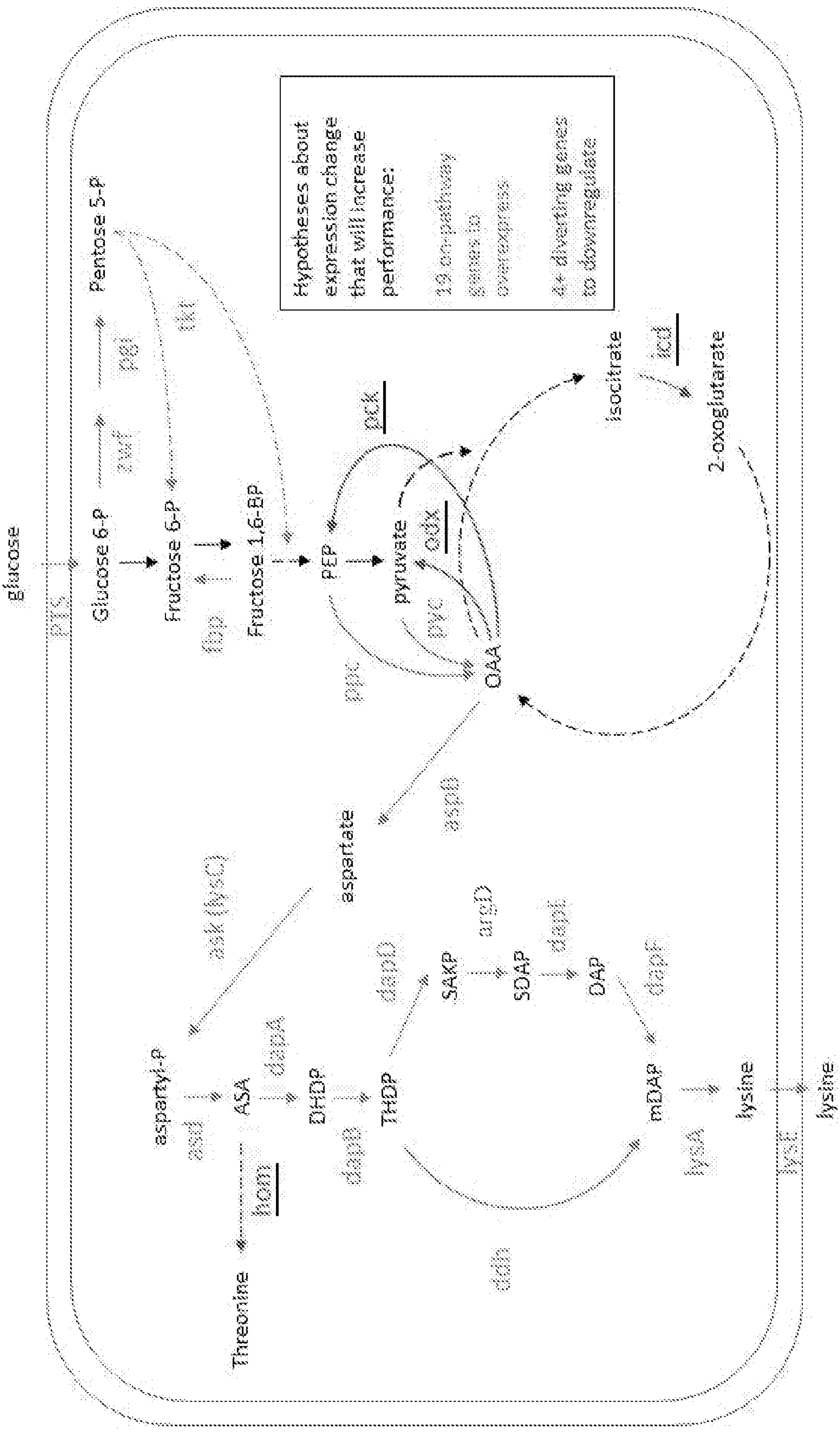
glucose
PTS
Glucose 6-P
zwf
pgl
Pentose 5-P
tkt
Fructose 6-P
fbp
Fructose 1,6-BP
PEP
pyruvate
ppc
pyc
odx
pck
OAA
Isocitrate
icd
2-oxoglutarate
aspB
aspartate
ask (lysC)
aspartyl-P
asd
ASA
hom
Threonine
dapA
DHDP
dapB
THDP
dapD
SAKP
argD
SDAP
dapE
DAP
dapF
mDAP
ddh
lysA
lysine
lysE
lysine
Hypotheses about expression change that will increase performance:
19 on-pathway genes to overexpress
4+ diverting genes to downregulate

FIGURE 20

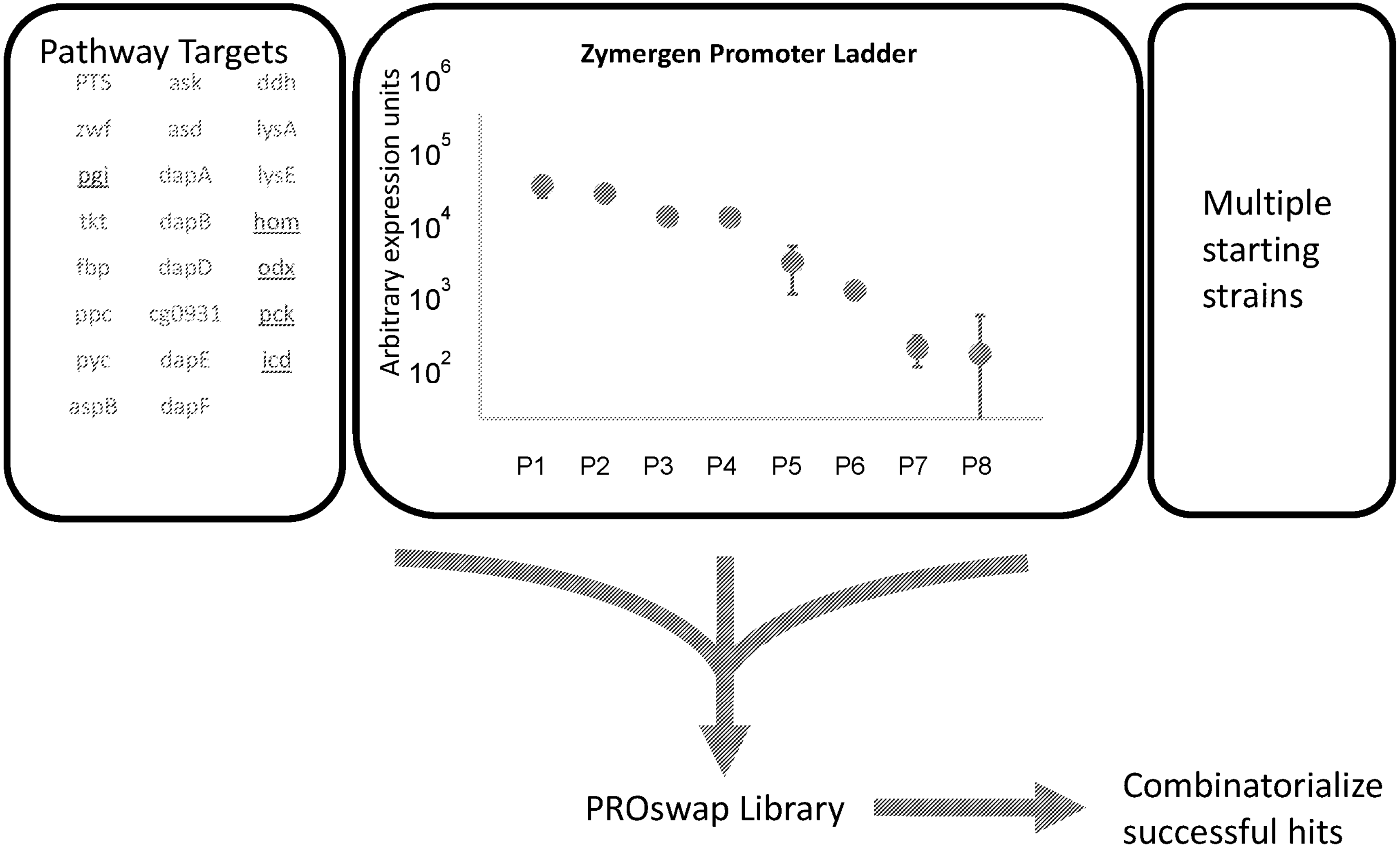
Pathway Targets
PTS ask ddh
zwf asd lysA
pgi dapA lysE
tkt dapB hom
fbp dapD odx
ppc cg0931 pck
pyc dapE icd
aspB dapF
Zymergen Promoter Ladder
Arbitrary expression units
$10^6$
$10^5$
$10^4$
$10^3$
$10^2$
P1 P2 P3 P4 P5 P6 P7 P8
Multiple starting strains
PROswap Library
Combinatorialize successful hits

FIGURE 21

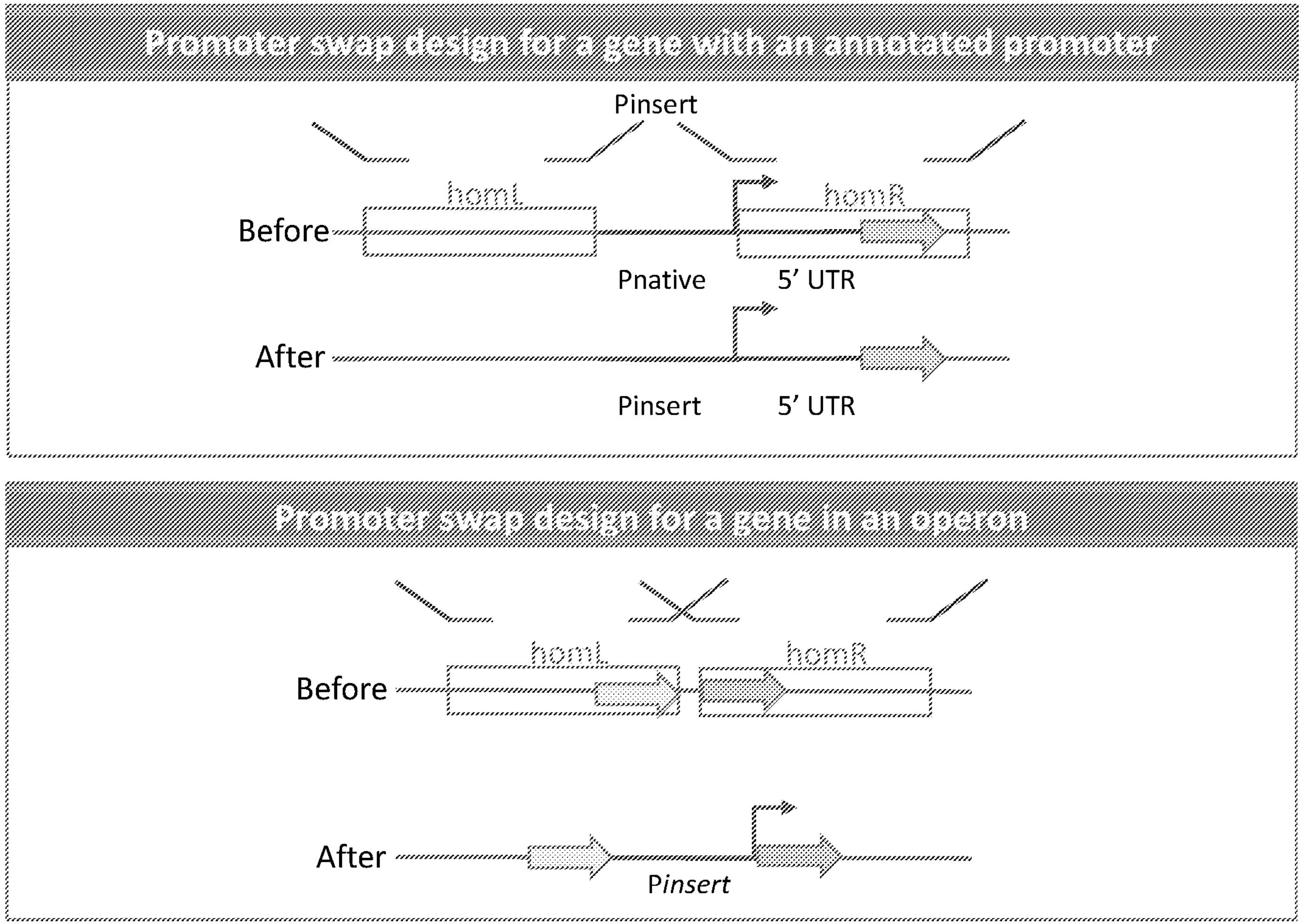
Promoter swap design for a gene with an annotated promoter
Pinsert
homL
homR
Before
Pnative
5' UTR
After
Pinsert
5' UTR
Promoter swap design for a gene in an operon
homL
homR
Before
After
Pinsert

FIGURE 22

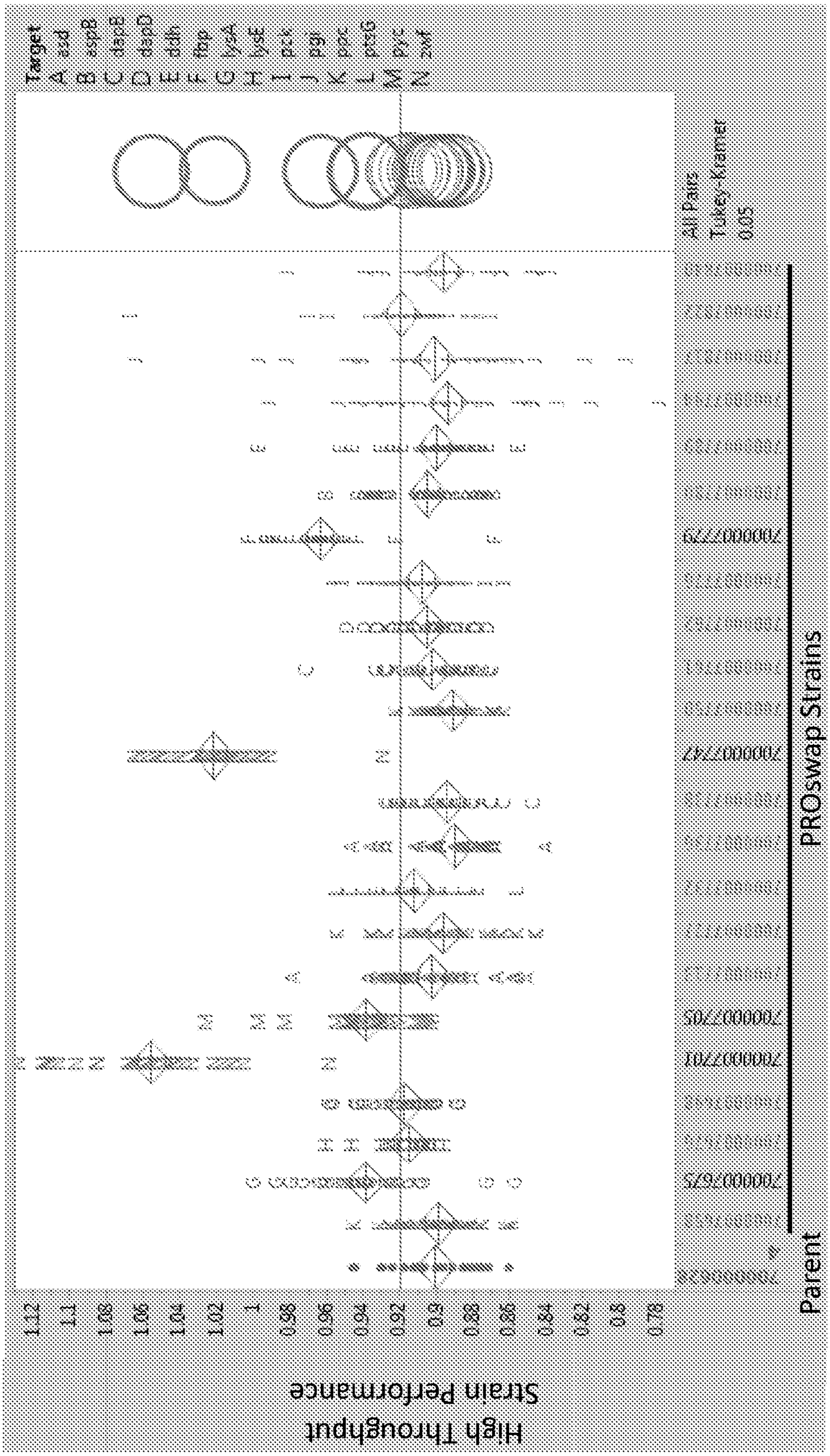
High Throughput Strain Performance
1.12
1.1
1.08
1.06
1.04
1.02
1
0.98
0.96
0.94
0.92
0.9
0.88
0.86
0.84
0.82
0.8
0.78
7000007625
7000007701
7000007705
7000007747
7000007779
Parent
PROswap Strains
All Pairs
Tukey-Kramer
0.05
Target
A asd
B aspB
C dapB
D dapD
E ddh
F fbp
G lysA
H lysE
I pck
J pgi
K ppc
L ptsG
M pyc
N zwf

FIGURE 23

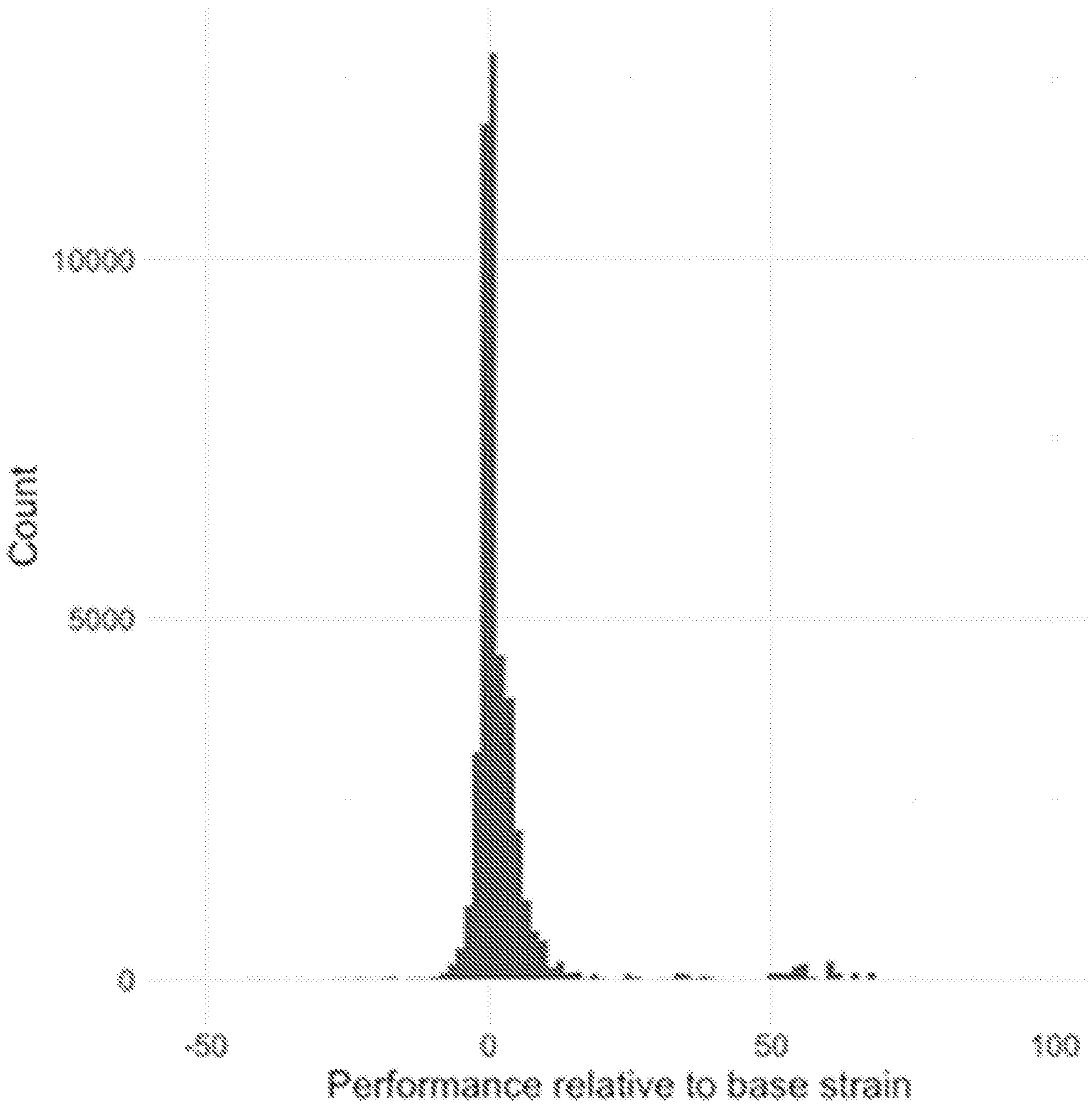
10000
5000
0
Count
-50
0
50
100
Performance relative to base strain

FIGURE 24

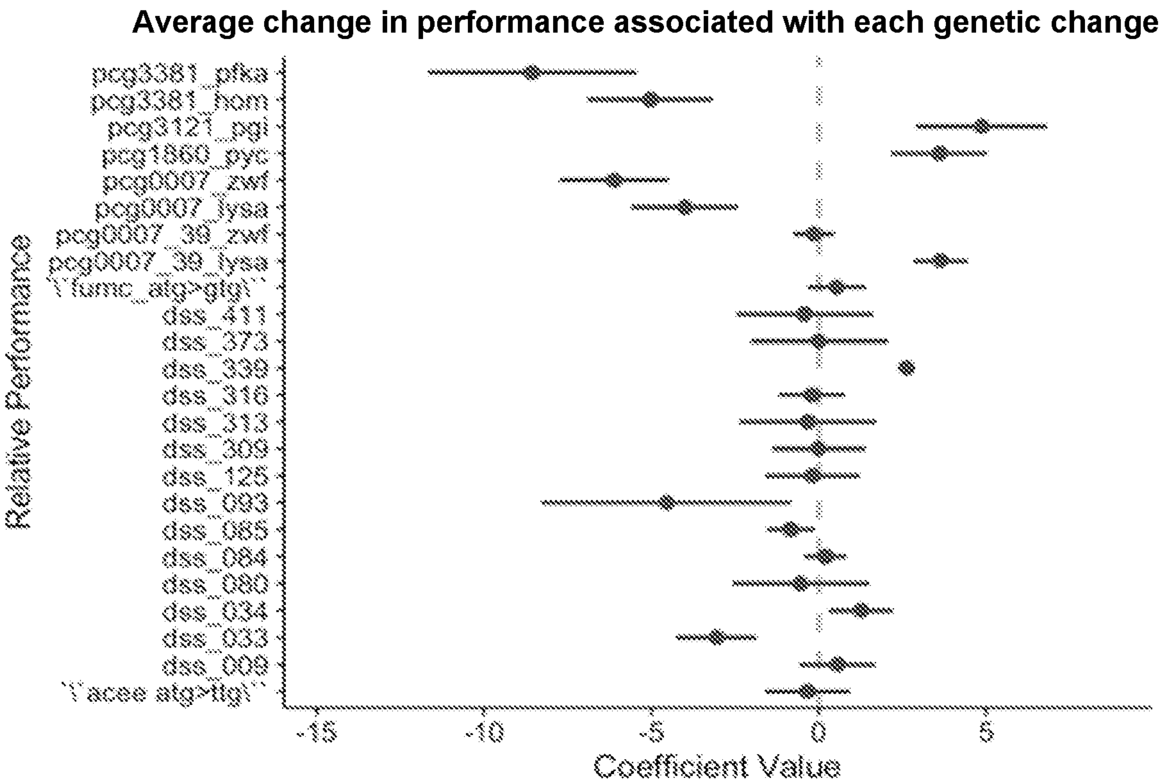
Average change in performance associated with each genetic change
pcg3381_pfka
pcg3381_hom
pcg3121_pgi
pcg1860_pyc
pcg0007_zwf
pcg0007_lysa
pcg0007_39_zwf
pcg0007_39_lysa
`\`fumc_atg>gtg\``
dss_411
dss_373
dss_339
dss_316
dss_313
dss_309
dss_125
dss_093
dss_085
dss_084
dss_080
dss_034
dss_033
dss_009
`\`acee atg>ttg\``
Relative Performance
-15
-10
-5
0
5
Coefficient Value

**FIGURE 25**

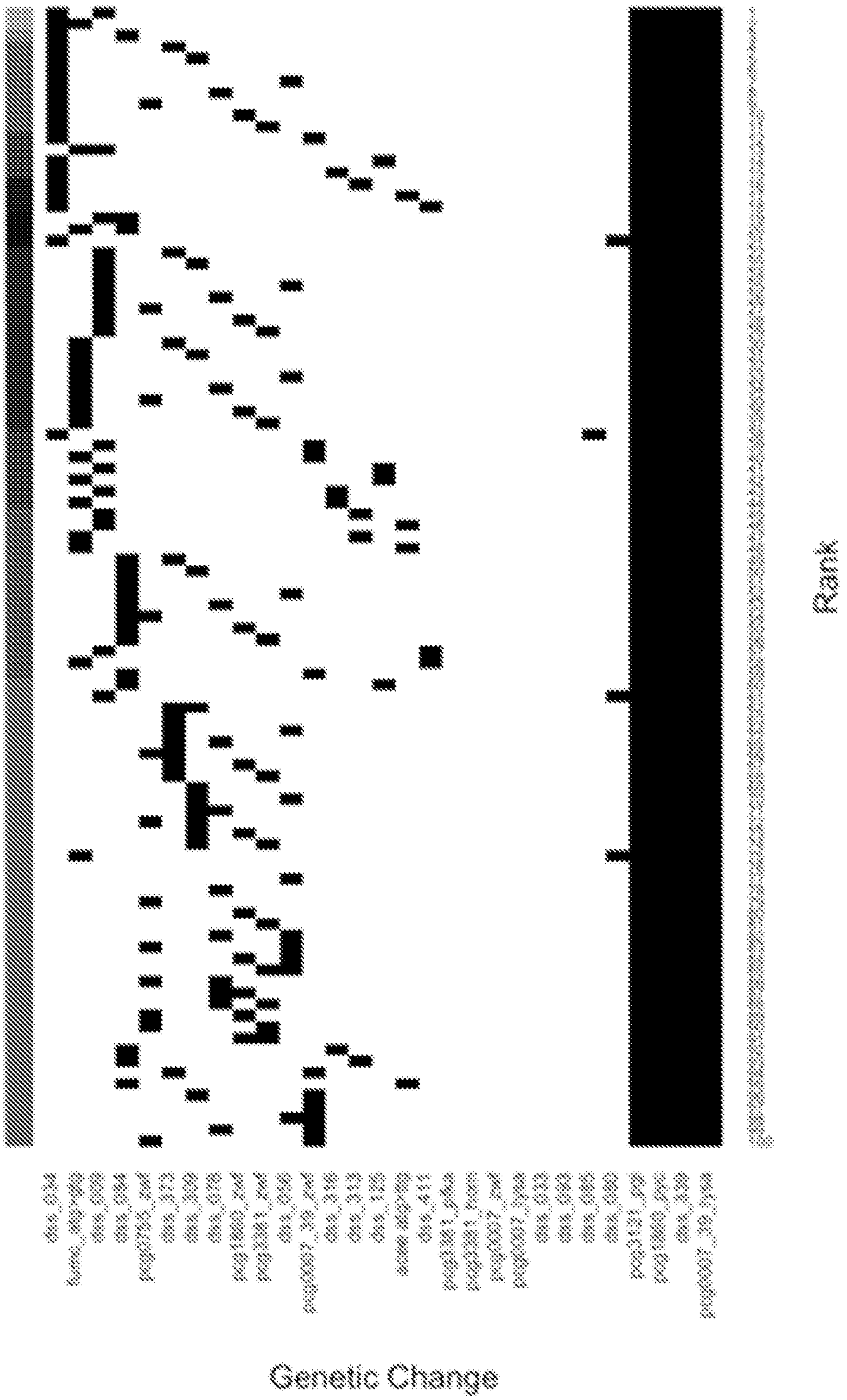

FIGURE 26

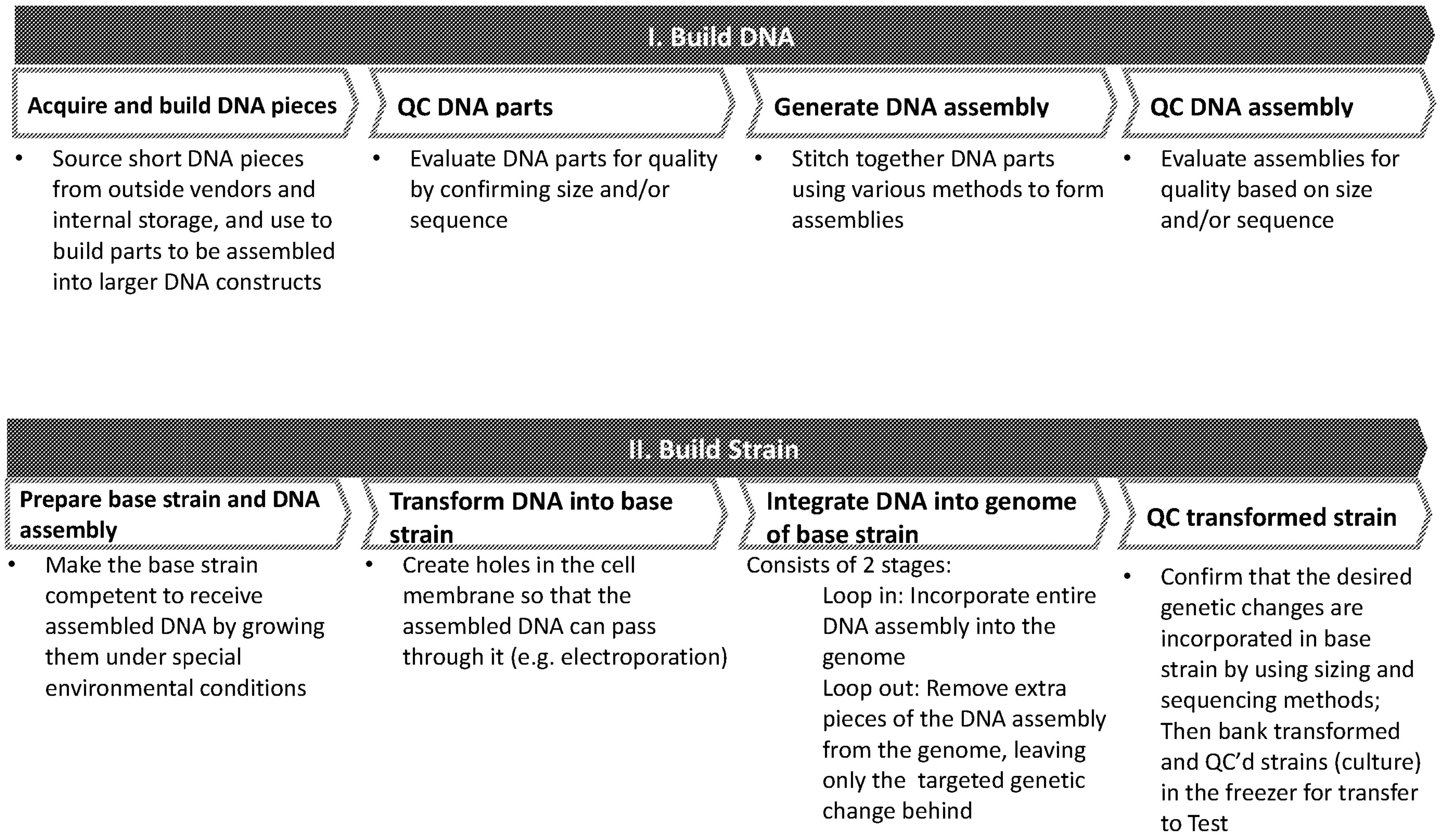
I. Build DNA
Acquire and build DNA pieces
• Source short DNA pieces from outside vendors and internal storage, and use to build parts to be assembled into larger DNA constructs
QC DNA parts
• Evaluate DNA parts for quality by confirming size and/or sequence
Generate DNA assembly
• Stitch together DNA parts using various methods to form assemblies
QC DNA assembly
• Evaluate assemblies for quality based on size and/or sequence
II. Build Strain
Prepare base strain and DNA assembly
• Make the base strain competent to receive assembled DNA by growing them under special environmental conditions
Transform DNA into base strain
• Create holes in the cell membrane so that the assembled DNA can pass through it (e.g. electroporation)
Integrate DNA into genome of base strain
Consists of 2 stages:
Loop in: Incorporate entire DNA assembly into the genome
Loop out: Remove extra pieces of the DNA assembly from the genome, leaving only the targeted genetic change behind
QC transformed strain
• Confirm that the desired genetic changes are incorporated in base strain by using sizing and sequencing methods; Then bank transformed and QC'd strains (culture) in the freezer for transfer to Test

FIGURE 27

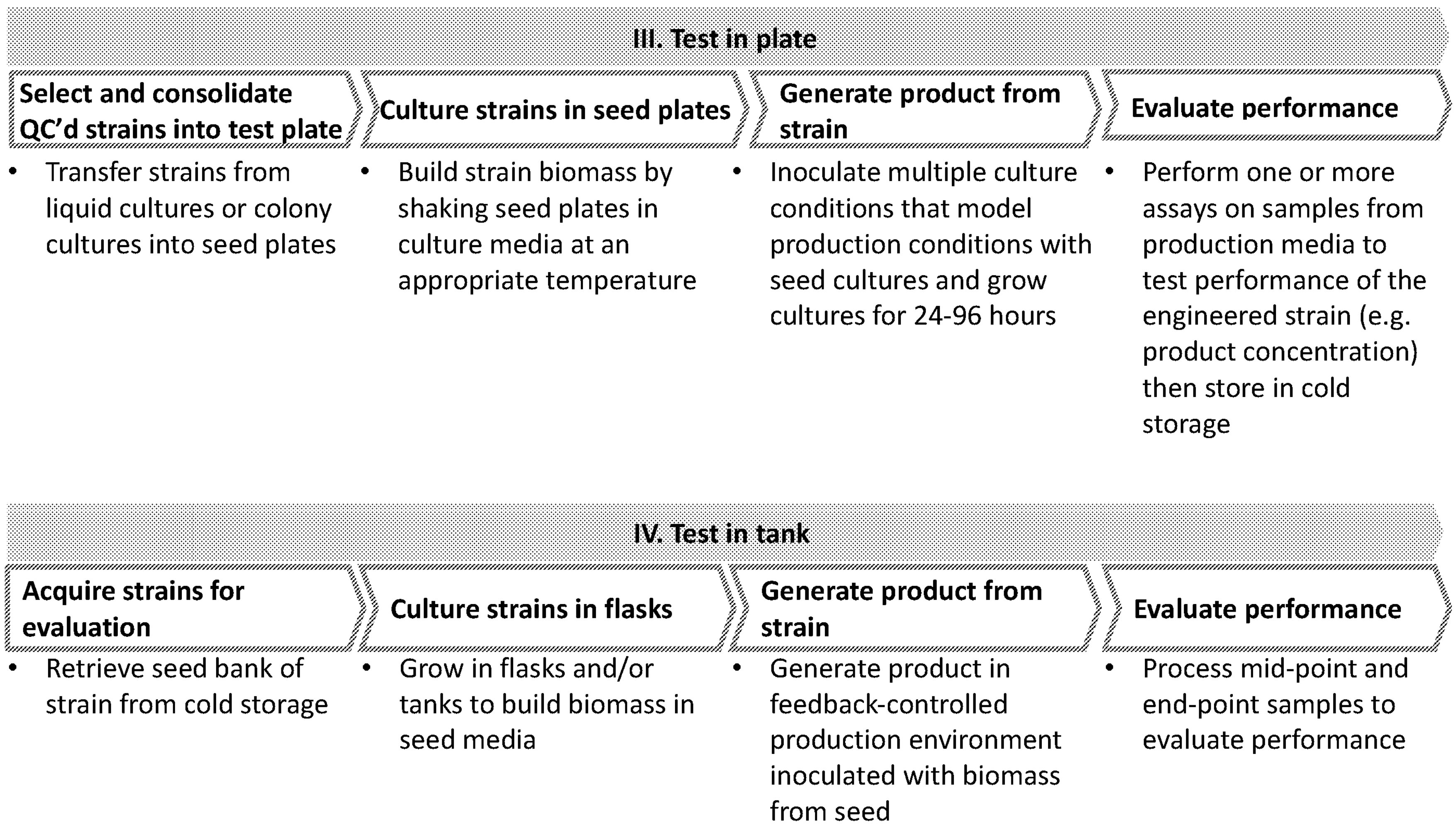
III. Test in plate
Select and consolidate QC'd strains into test plate
• Transfer strains from liquid cultures or colony cultures into seed plates
Culture strains in seed plates
• Build strain biomass by shaking seed plates in culture media at an appropriate temperature
Generate product from strain
• Inoculate multiple culture conditions that model production conditions with seed cultures and grow cultures for 24-96 hours
Evaluate performance
• Perform one or more assays on samples from production media to test performance of the engineered strain (e.g. product concentration) then store in cold storage
IV. Test in tank
Acquire strains for evaluation
• Retrieve seed bank of strain from cold storage
Culture strains in flasks
• Grow in flasks and/or tanks to build biomass in seed media
Generate product from strain
• Generate product in feedback-controlled production environment inoculated with biomass from seed
Evaluate performance
• Process mid-point and end-point samples to evaluate performance

FIGURE 28

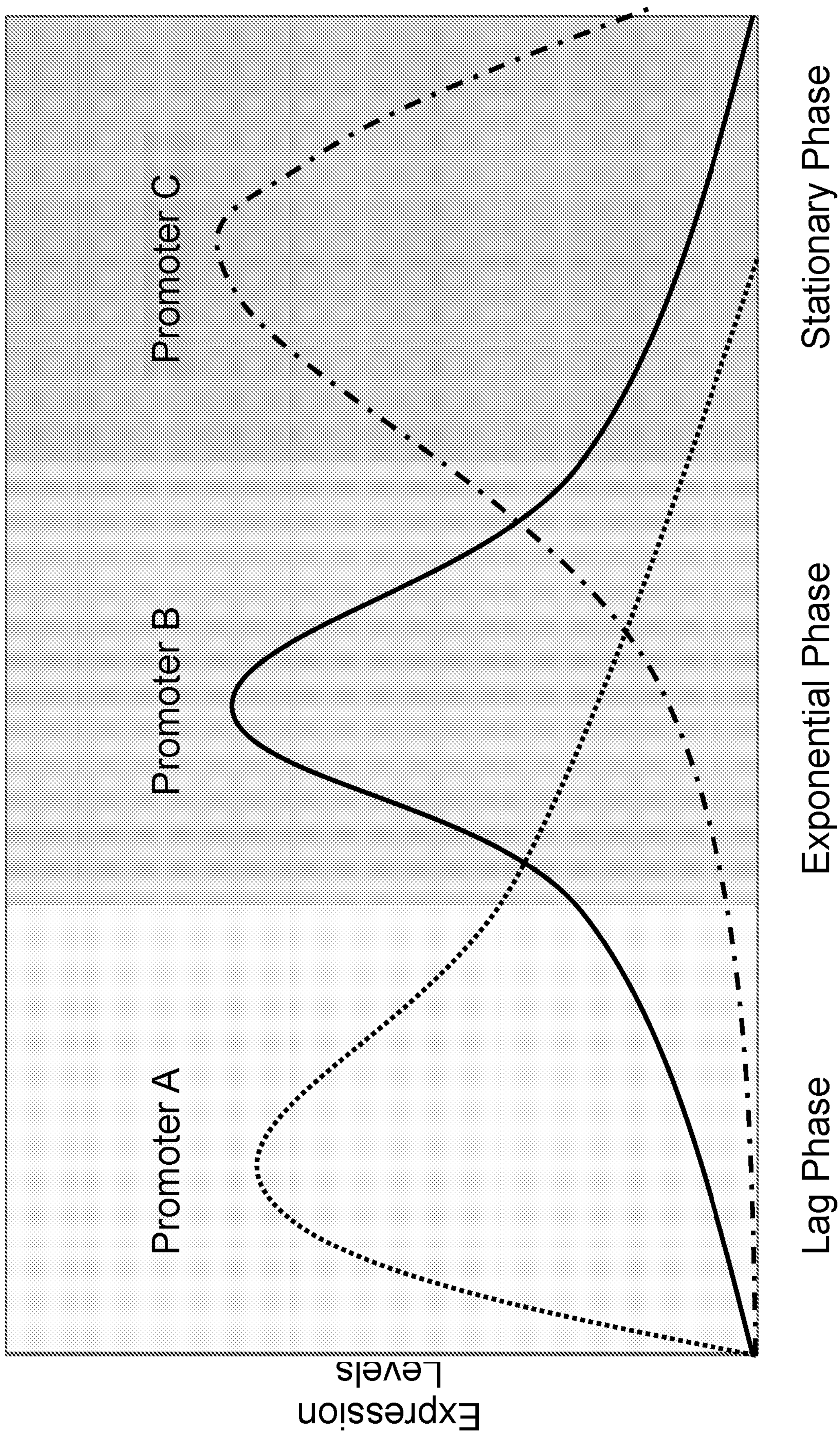
Expression Levels
Promoter A
Promoter B
Promoter C
Lag Phase
Exponential Phase
Stationary Phase

FIGURE 29

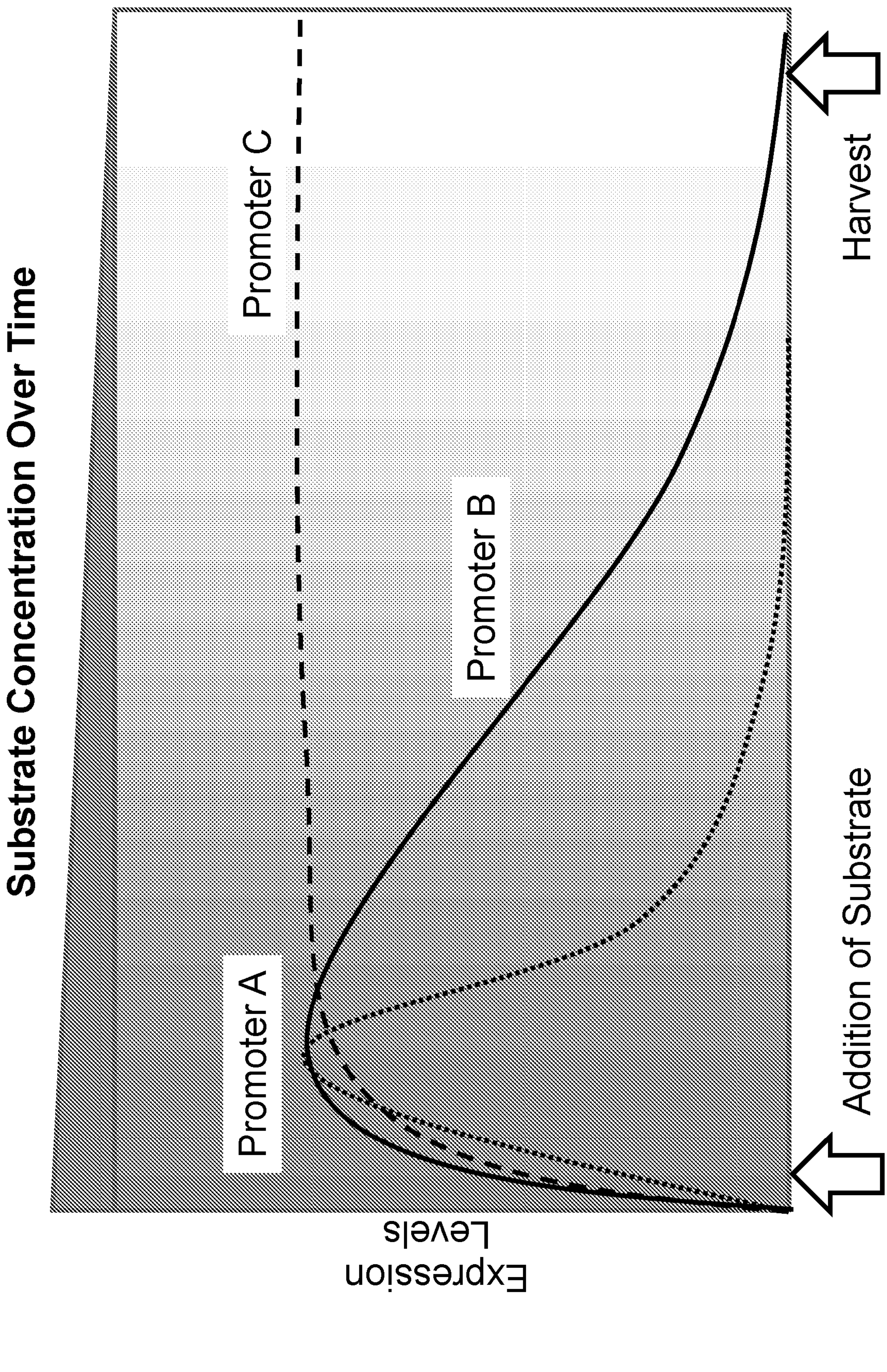
Substrate Concentration Over Time
Expression Levels
Promoter A
Promoter B
Promoter C
Addition of Substrate
Harvest

FIGURE 30

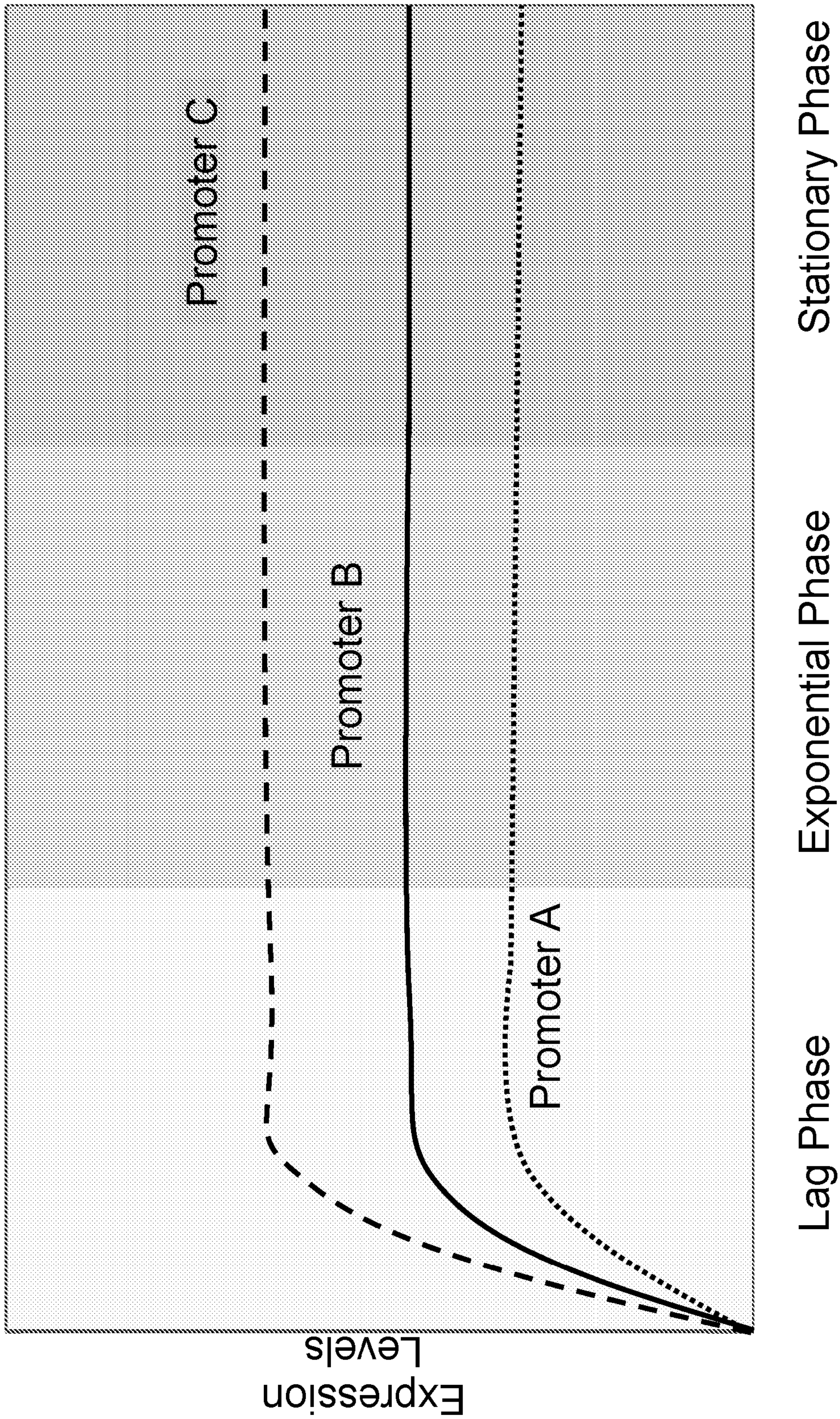
Expression Levels
Promoter A
Promoter B
Promoter C
Lag Phase
Exponential Phase
Stationary Phase

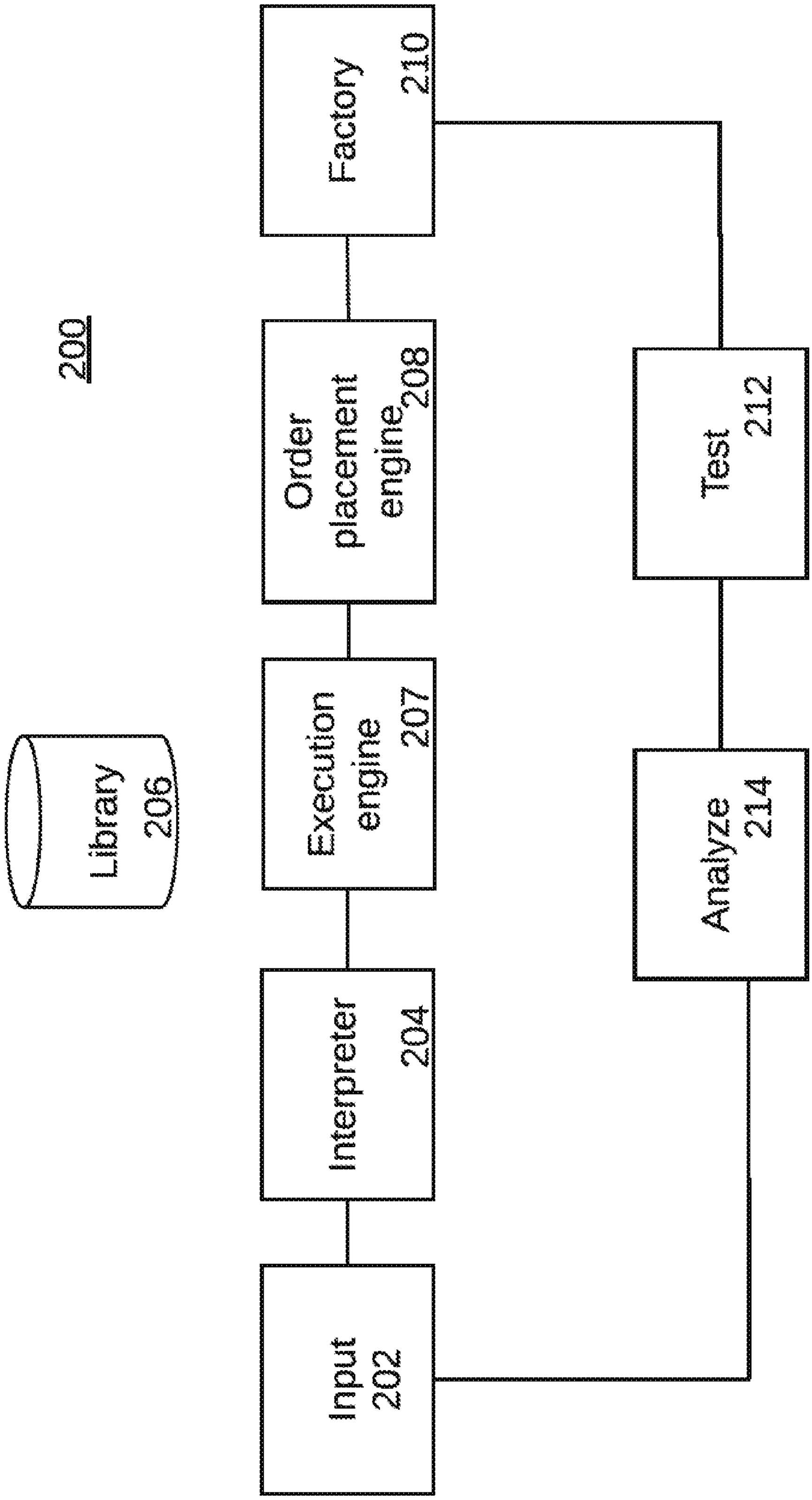
200
Input 202
Interpreter 204
Library 206
Execution engine 207
Order placement engine 208
Factory 210
Test 212
Analyze 214

FIGURE 31

FIGURE 32

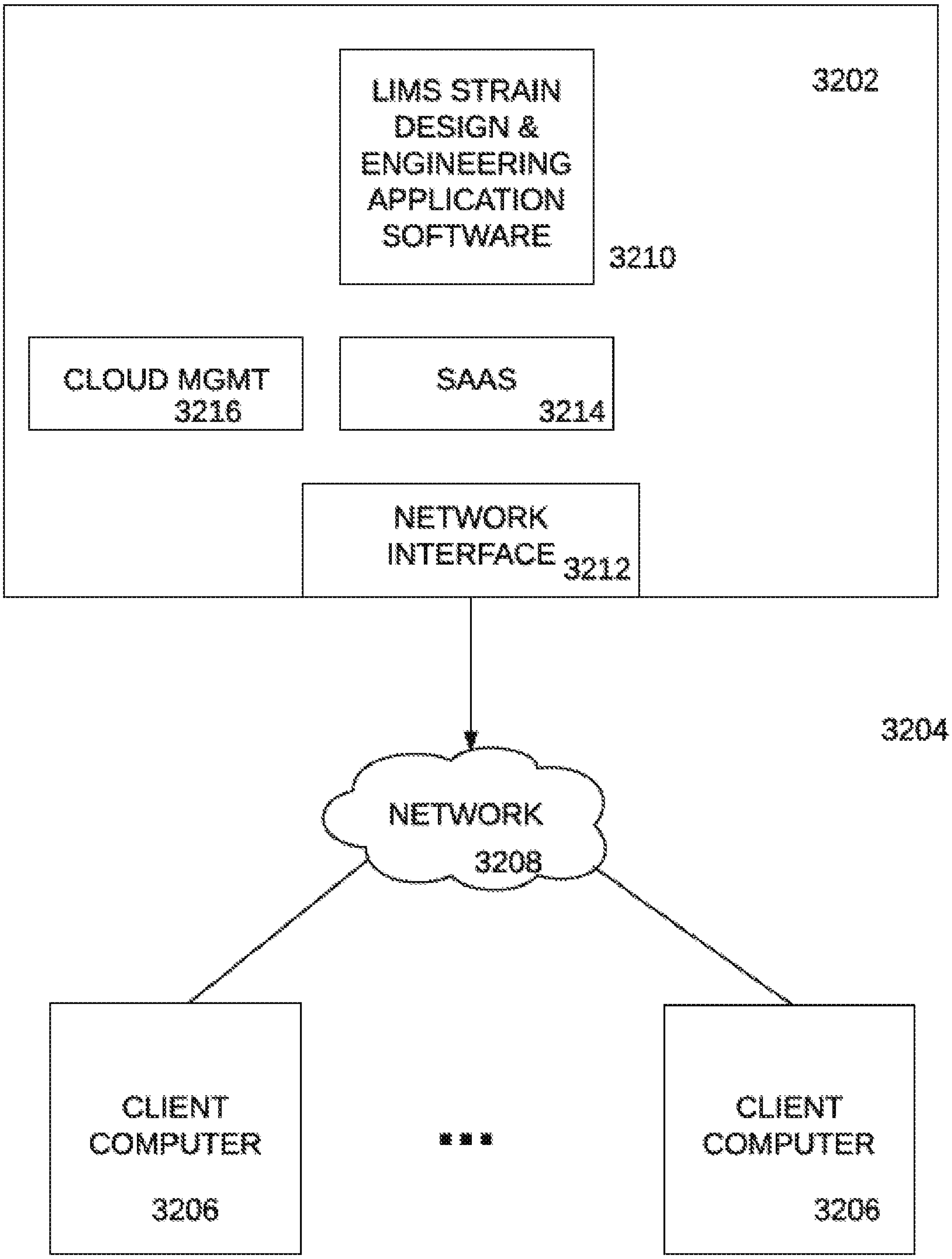
LIMS STRAIN DESIGN & ENGINEERING APPLICATION SOFTWARE
3210
3202
CLOUD MGMT
3216
SAAS
3214
NETWORK INTERFACE
3212
3204
NETWORK
3208
CLIENT COMPUTER
3206
CLIENT COMPUTER
3206

FIGURE 33

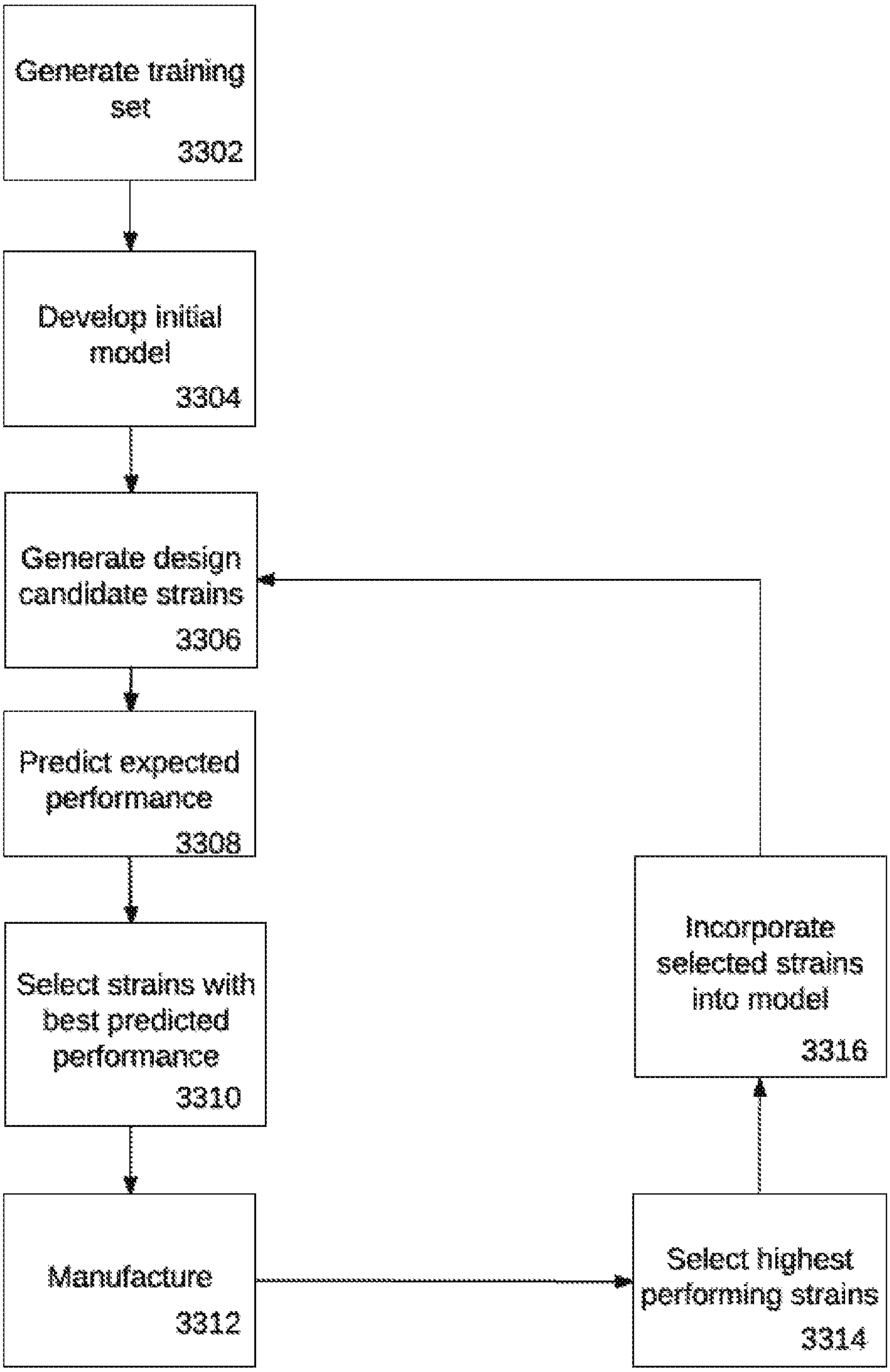
Generate training set
3302
Develop initial model
3304
Generate design candidate strains
3306
Predict expected performance
3308
Select strains with best predicted performance
3310
Manufacture
3312
Select highest performing strains
3314
Incorporate selected strains into model
3316

**FIGURE 34**

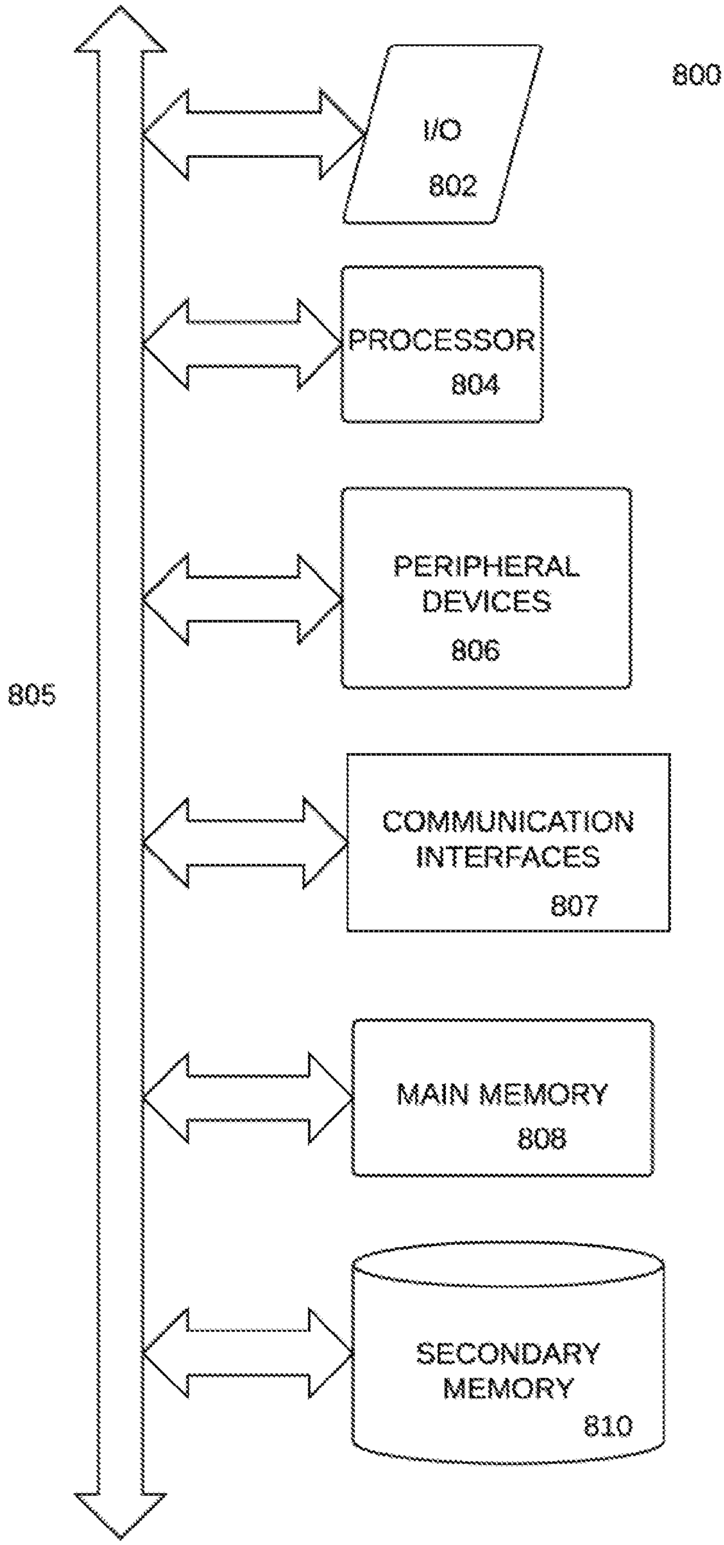
800
I/O
802
PROCESSOR
804
PERIPHERAL DEVICES
806
805
COMMUNICATION INTERFACES
807
MAIN MEMORY
808
SECONDARY MEMORY
810

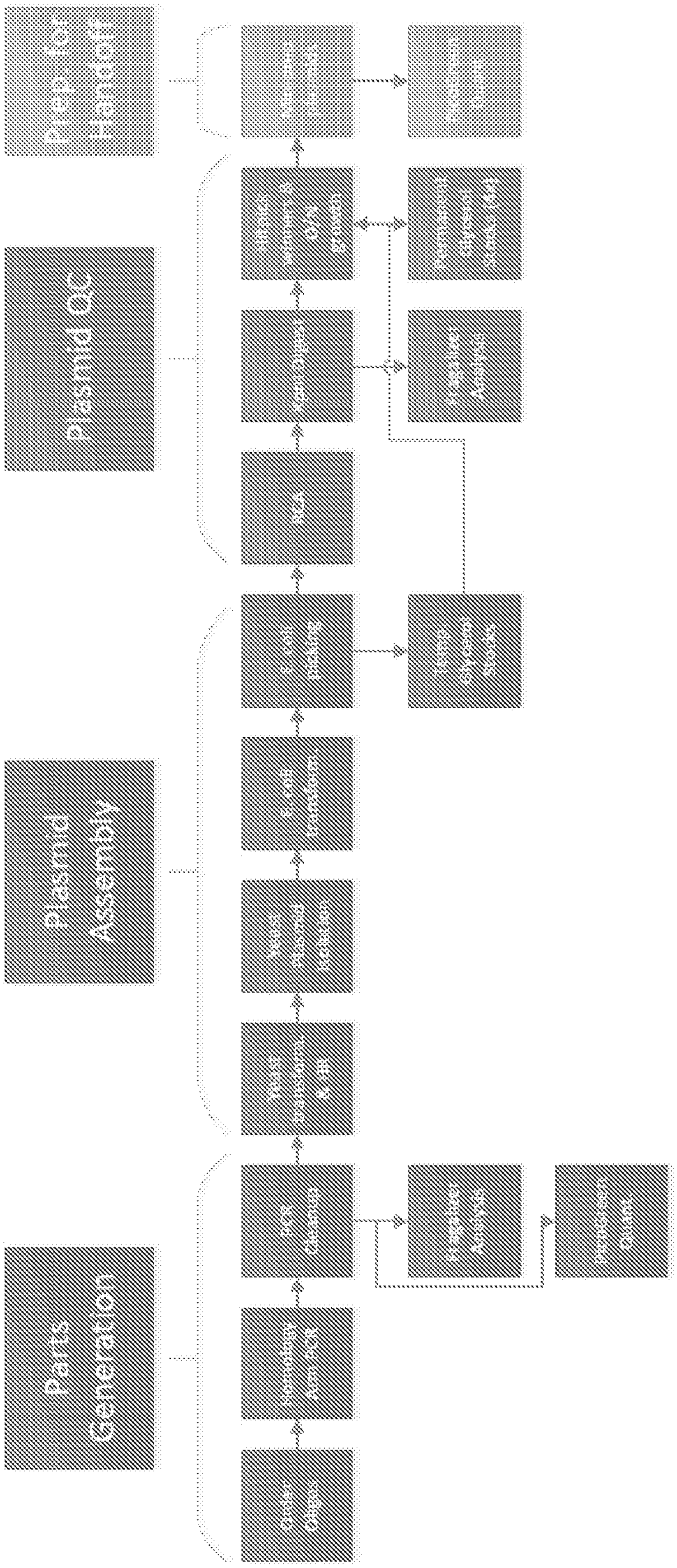
Parts Generation
Plasmid Assembly
Plasmid QC
Prep for Handoff

FIGURE 35

FIGURE 36

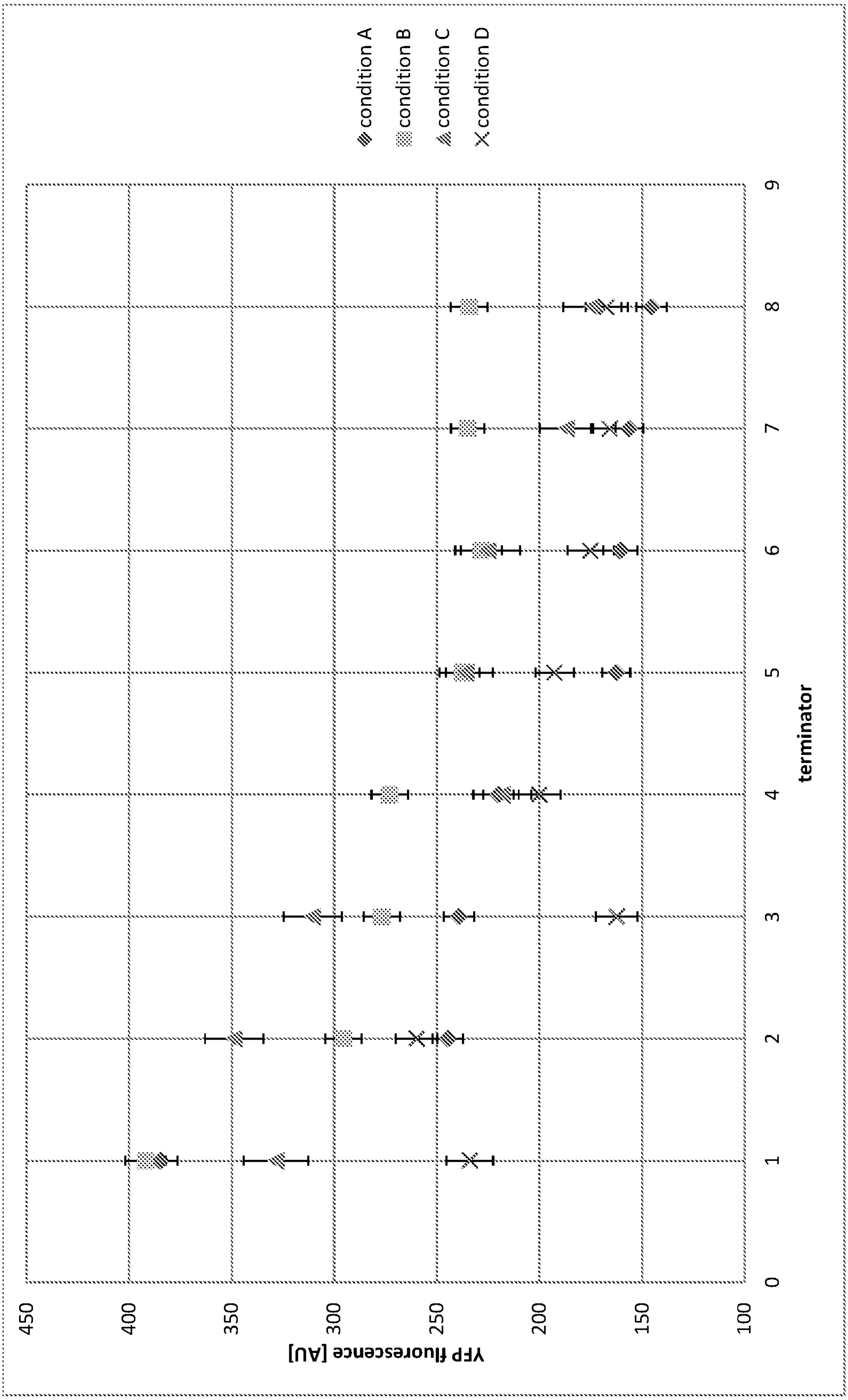

YFP fluorescence [AU]
450
400
350
300
250
200
150
100
0
1
2
3
4
5
6
7
8
9
terminator
condition A
condition B
condition C
condition D

FIGURE 37

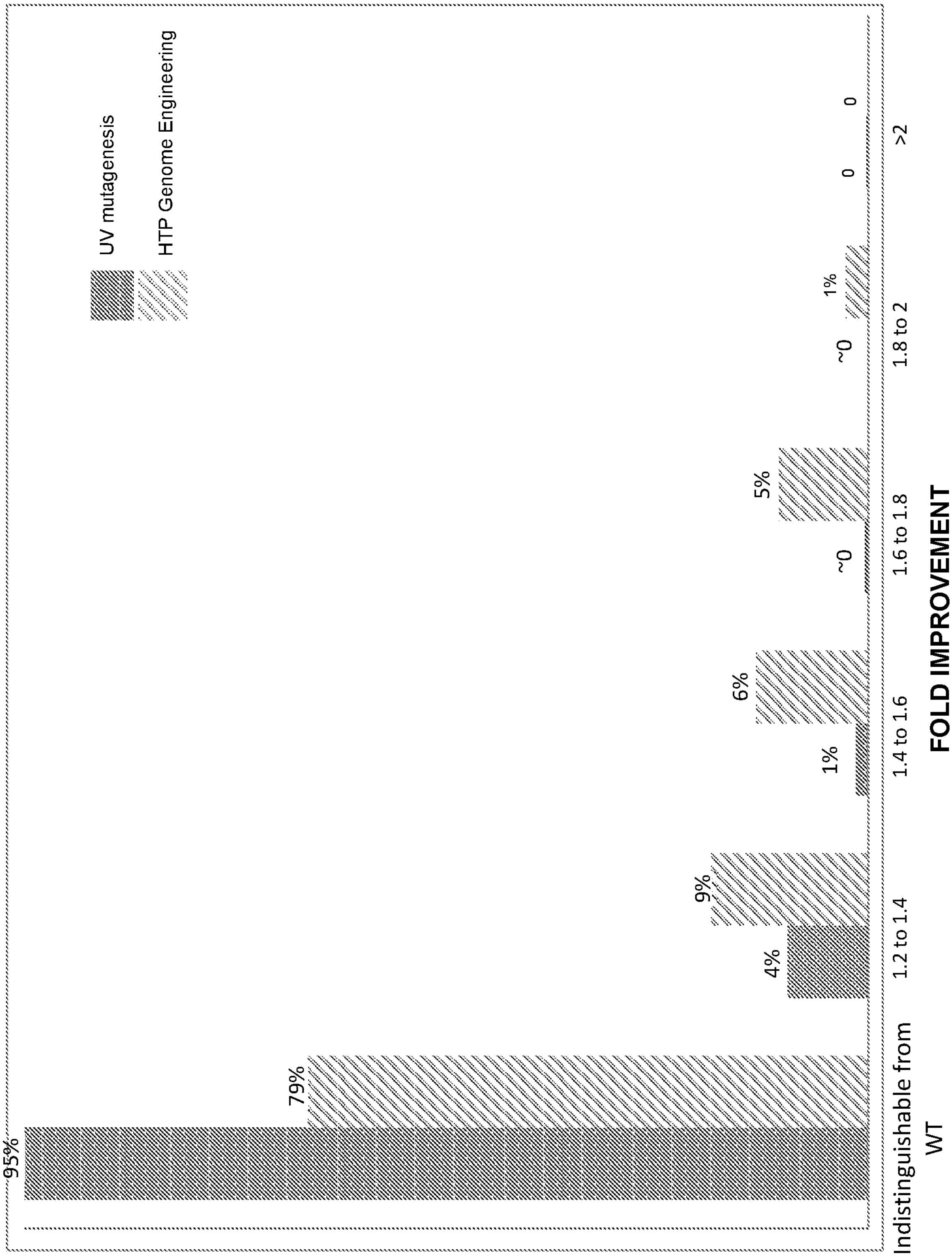

UV mutagenesis
HTP Genome Engineering
95%
79%
4%
9%
1%
6%
~0
5%
~0
1%
0
0
Indistinguishable from WT
1.2 to 1.4
1.4 to 1.6
1.6 to 1.8
1.8 to 2
>2
FOLD IMPROVEMENT

FIGURE 38

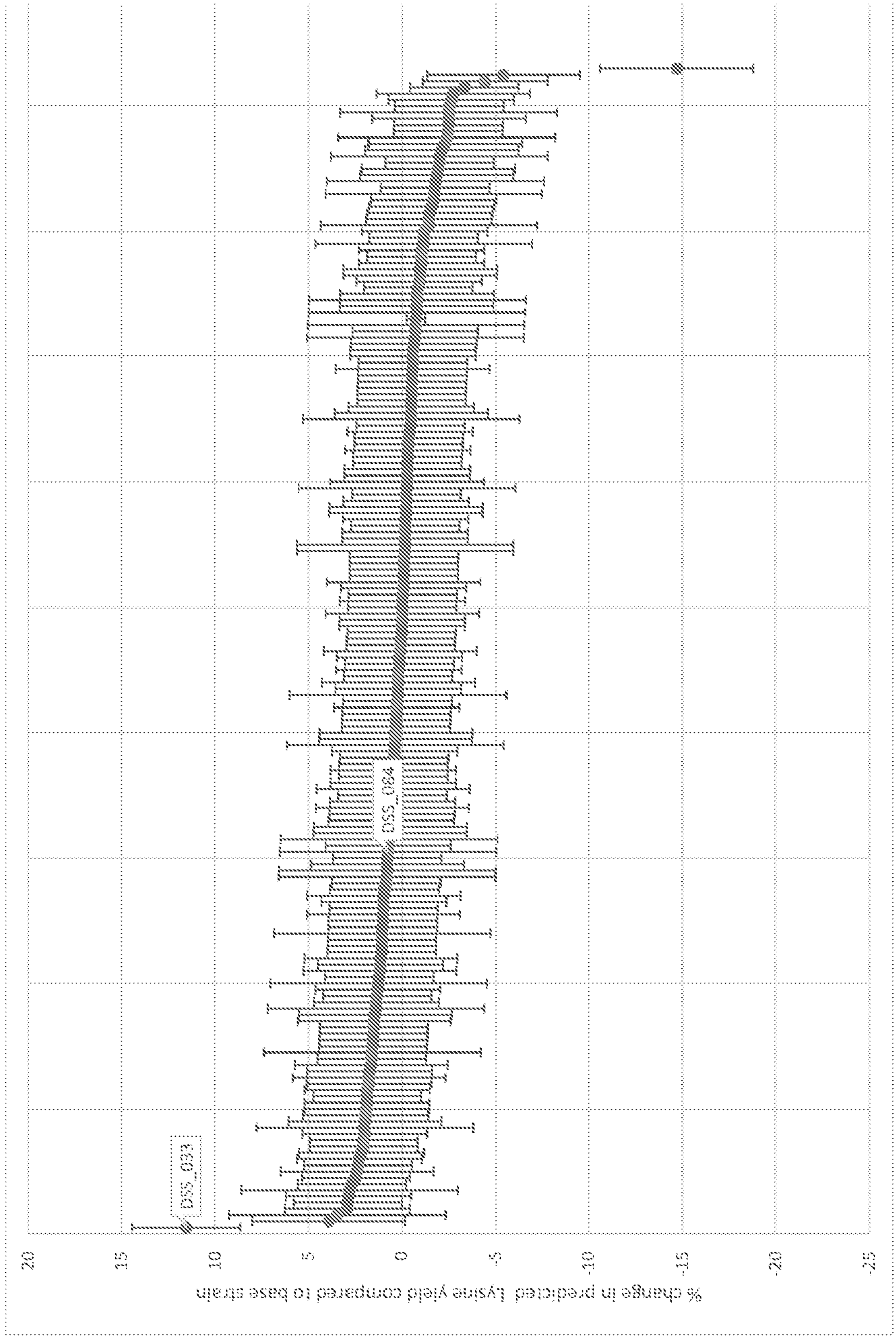

DSS_033
DSS_084
20
15
10
5
0
-5
-10
-15
-20
-25
% change in predicted Lysine yield compared to base strain

FIGURE 39

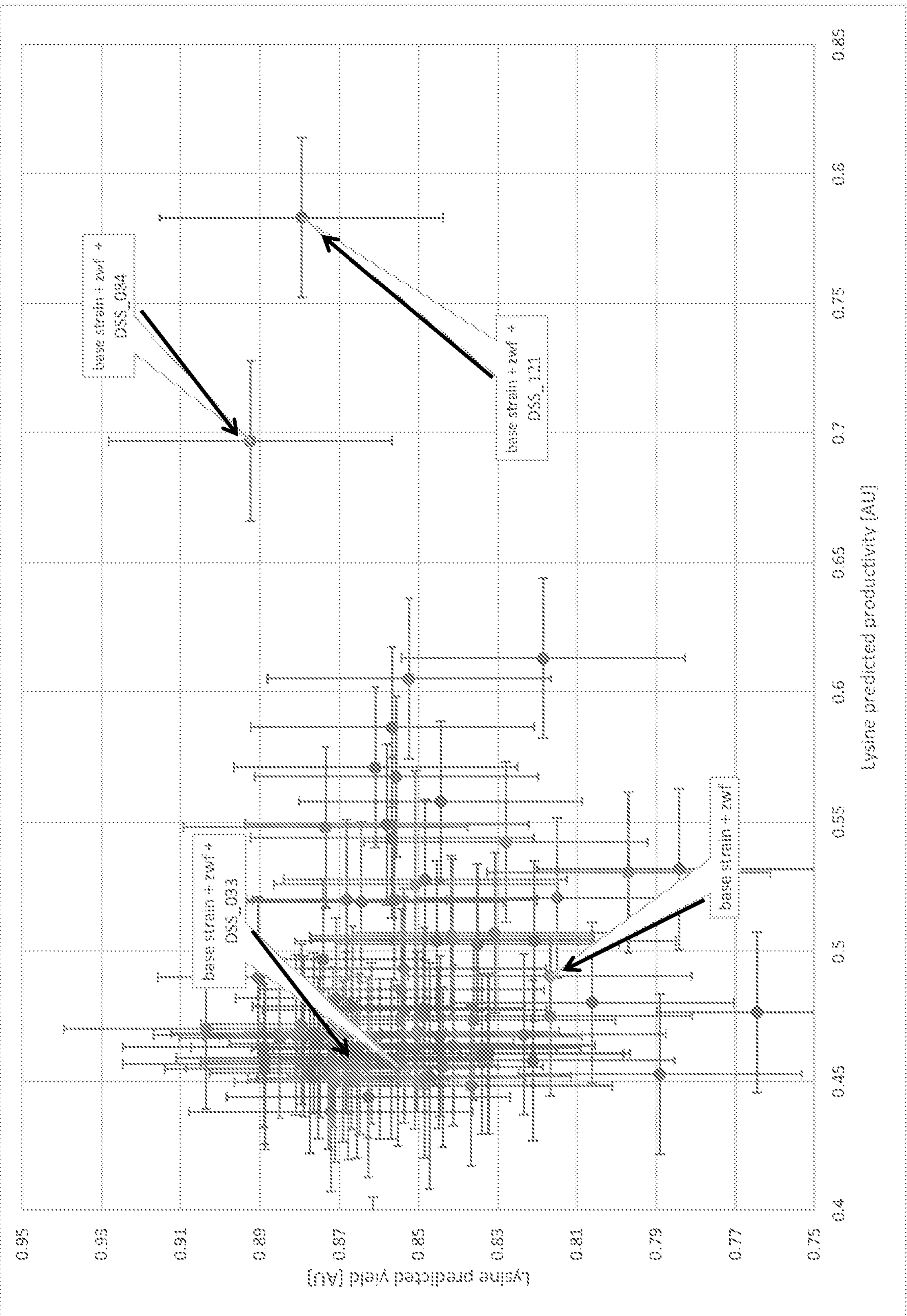

Lysine predicted yield [AU]
0.95
0.93
0.91
0.89
0.87
0.85
0.83
0.81
0.79
0.77
0.75
Lysine predicted productivity [AU]
0.4
0.45
0.5
0.55
0.6
0.65
0.7
0.75
0.8
0.85
base strain + zwf + DSS_033
base strain + zwf
base strain + zwf + DSS_084
base strain + zwf + DSS_121

FIGURE 40

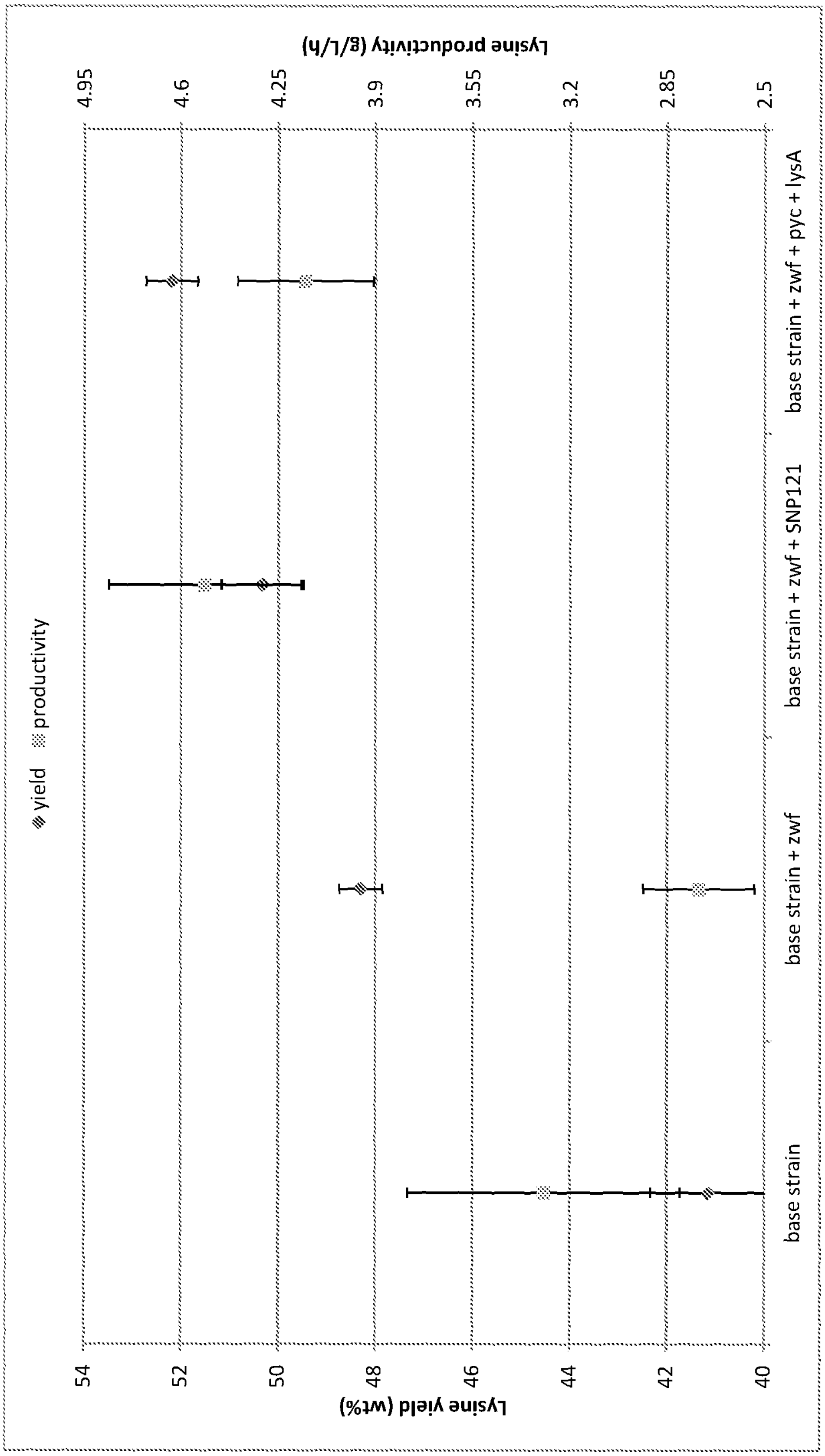
yield
productivity
Lysine yield (wt%)
Lysine productivity (g/L/h)
54
52
50
48
46
44
42
40
4.95
4.6
4.25
3.9
3.55
3.2
2.85
2.5
base strain
base strain + zwf
base strain + zwf + SNP121
base strain + zwf + pyc + lysA

FIGURE 41

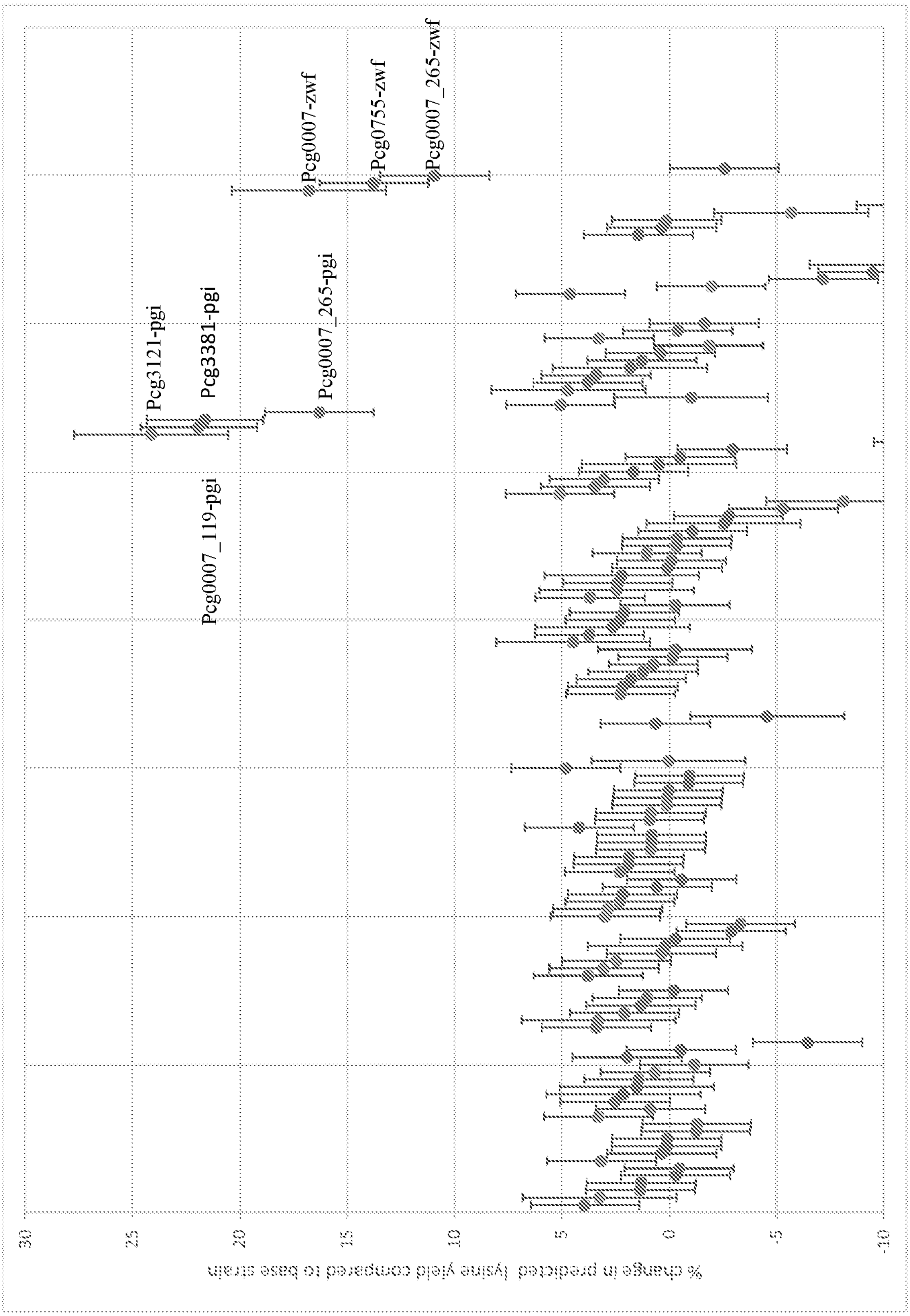
% change in predicted lysine yield compared to base strain
30
25
20
15
10
5
0
-5
-10
Pcg3121-pgi
Pcg3381-pgi
Pcg0007_265-pgi
Pcg0007_119-pgi
Pcg0007-zwf
Pcg0755-zwf
Pcg0007_265-zwf

FIGURE 42

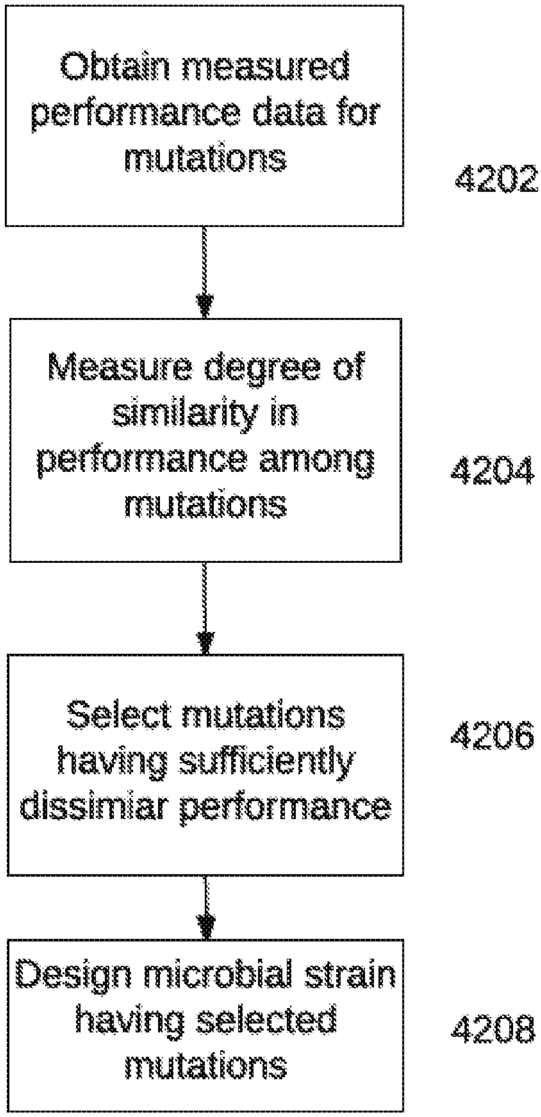
Obtain measured performance data for mutations
4202
Measure degree of similarity in performance among mutations
4204
Select mutations having sufficiently dissimiar performance
4206
Design microbial strain having selected mutations
4208

FIGURE 43

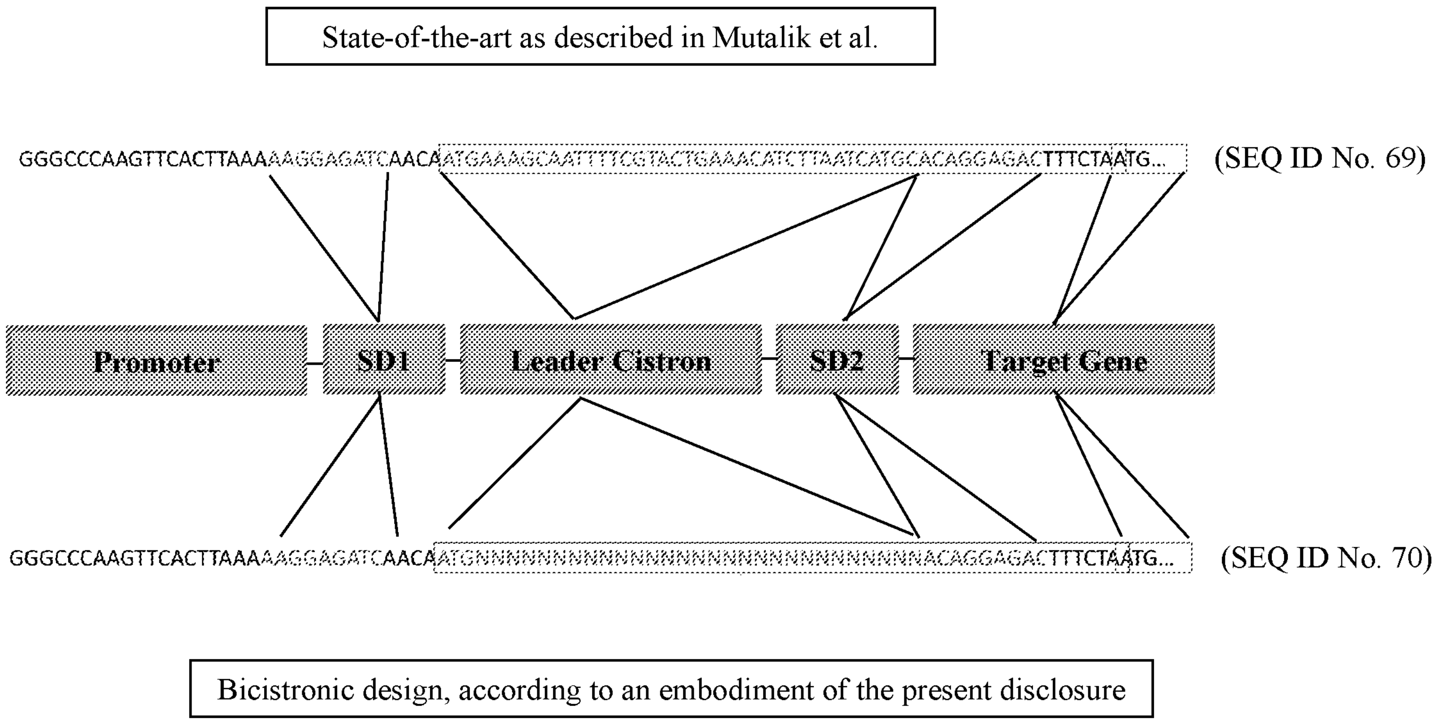
State-of-the-art as described in Mutalik et al.
GGGCCCAAGTTCACTTAAAAAGGAGATCAACAATGAAAGCAATTTTCGTACTGAAACATCTTAATCATGCACAGGAGACTTTCTAATG...
(SEQ ID No. 69)
Promoter
SD1
Leader Cistron
SD2
Target Gene
GGGCCCAAGTTCACTTAAAAAGGAGATCAACAATGNNNNNNNNNNNNNNNNNNNNNNNNNNNNNNNNNNNACAGGAGACTTTCTAATG...
(SEQ ID No. 70)
Bicistronic design, according to an embodiment of the present disclosure

FIGURE 44

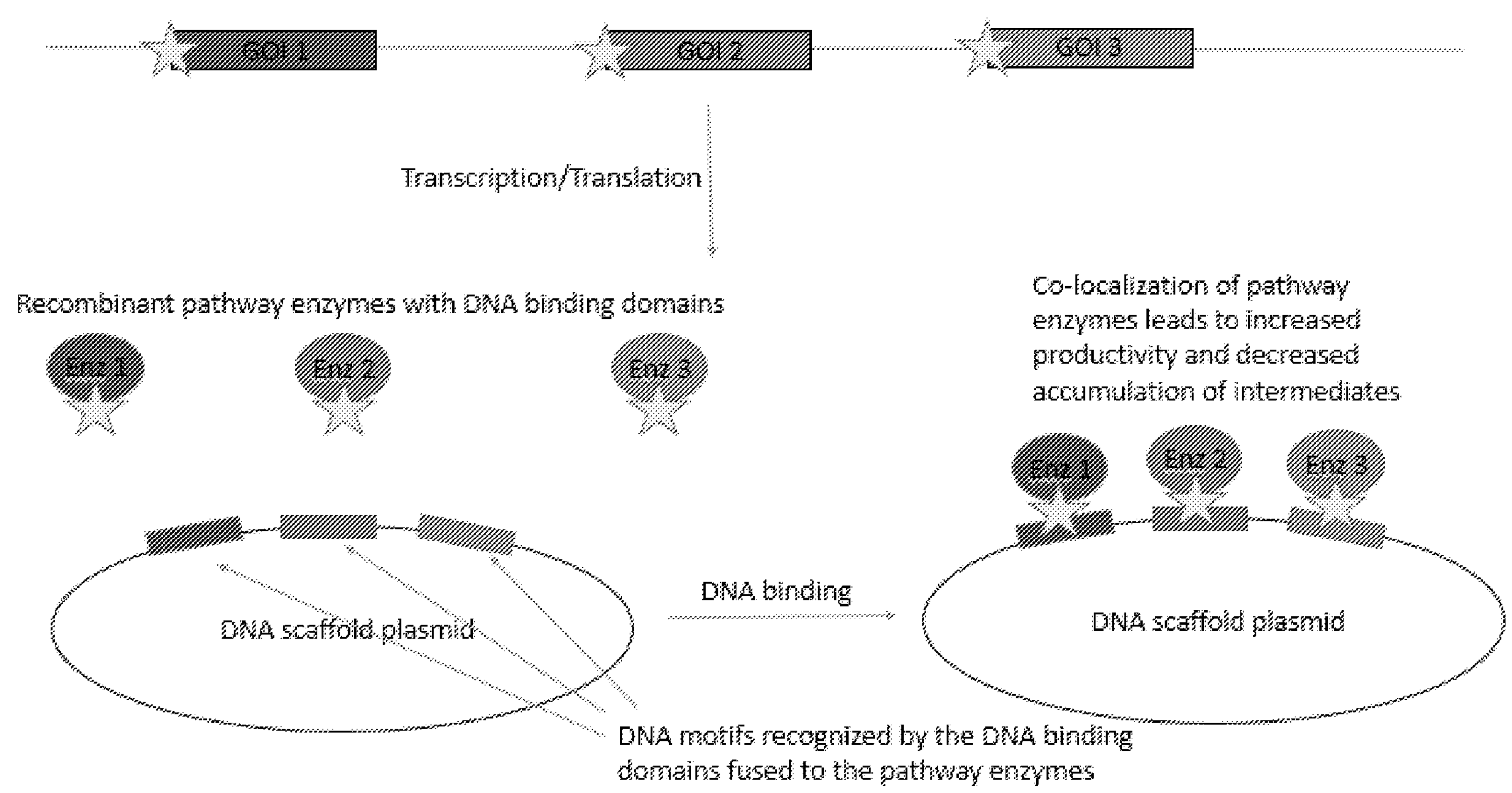
GOI 1
GOI 2
GOI 3
Transcription/Translation
Recombinant pathway enzymes with DNA binding domains
Enz 1
Enz 2
Enz 3
DNA scaffold plasmid
DNA binding
Co-localization of pathway enzymes leads to increased productivity and decreased accumulation of intermediates
Enz 1
Enz 2
Enz 3
DNA scaffold plasmid
DNA motifs recognized by the DNA binding domains fused to the pathway enzymes

FIGURE 45

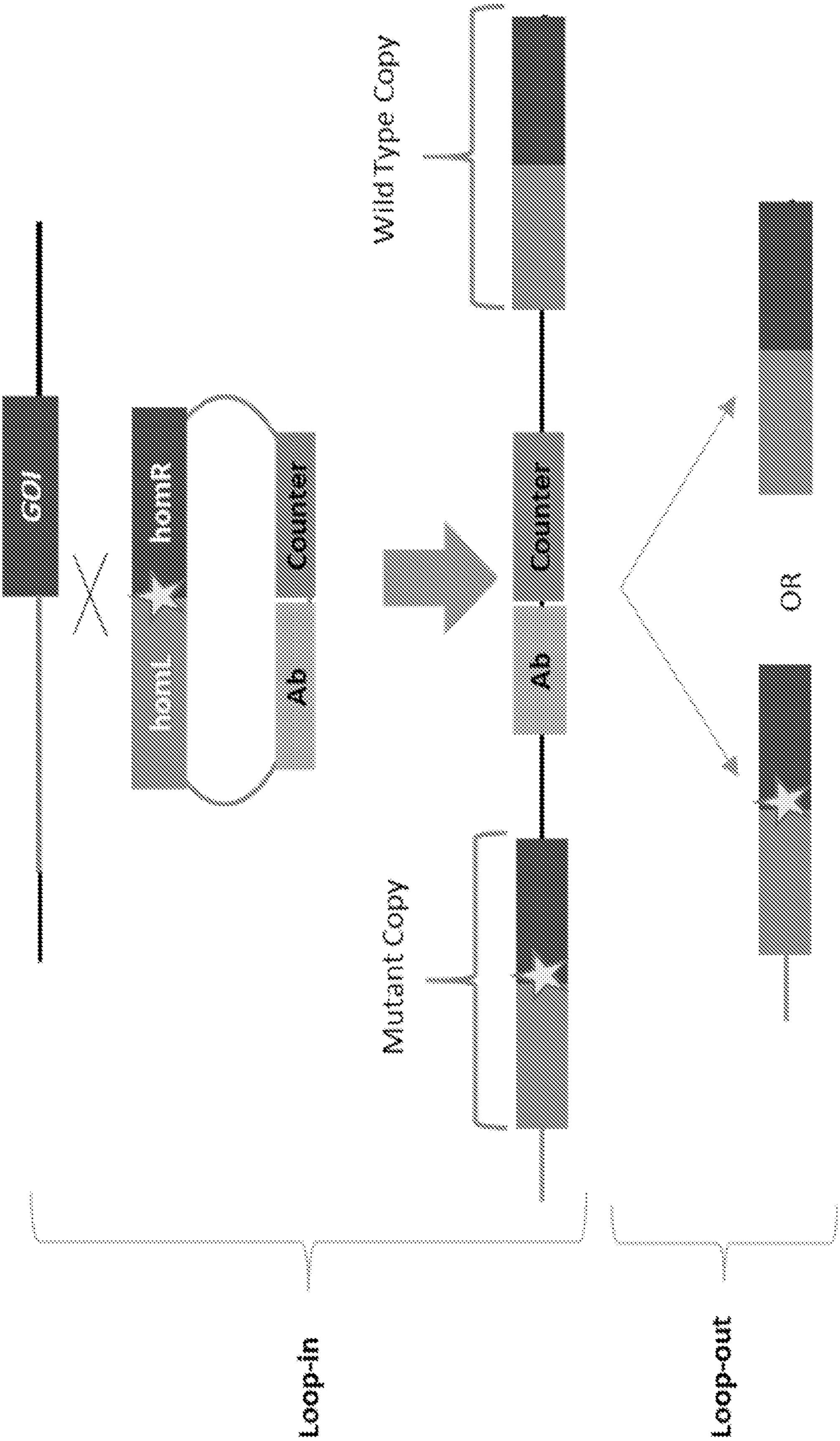
GOI
homL
homR
Ab
Counter
Wild Type Copy
Mutant Copy
OR
Loop-in
Loop-out

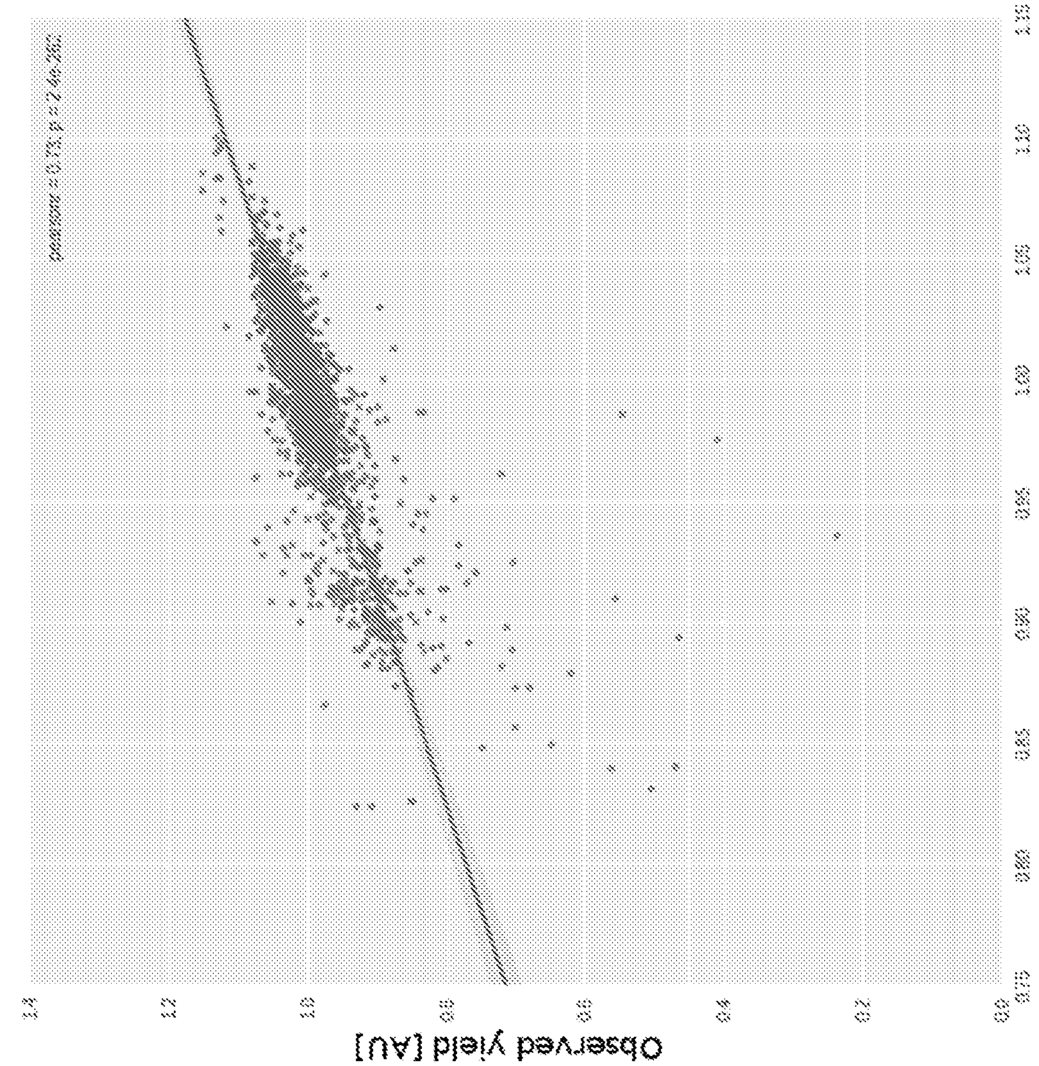


FIGURE 46

FIGURE 47

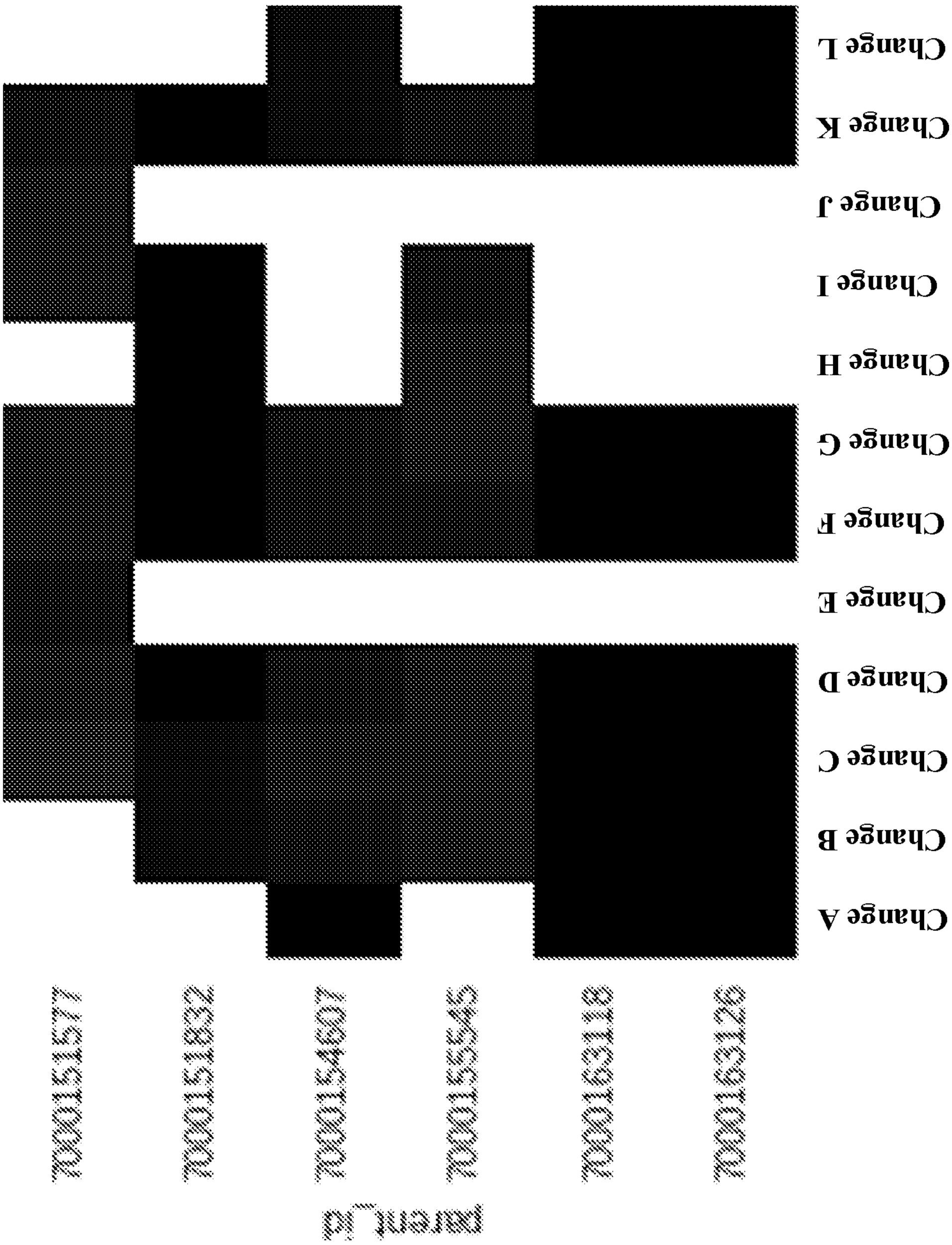
Change A
Change B
Change C
Change D
Change E
Change F
Change G
Change H
Change I
Change J
Change K
Change L
7000151577
7000151832
7000154607
7000155545
7000163118
7000163126
parent_id

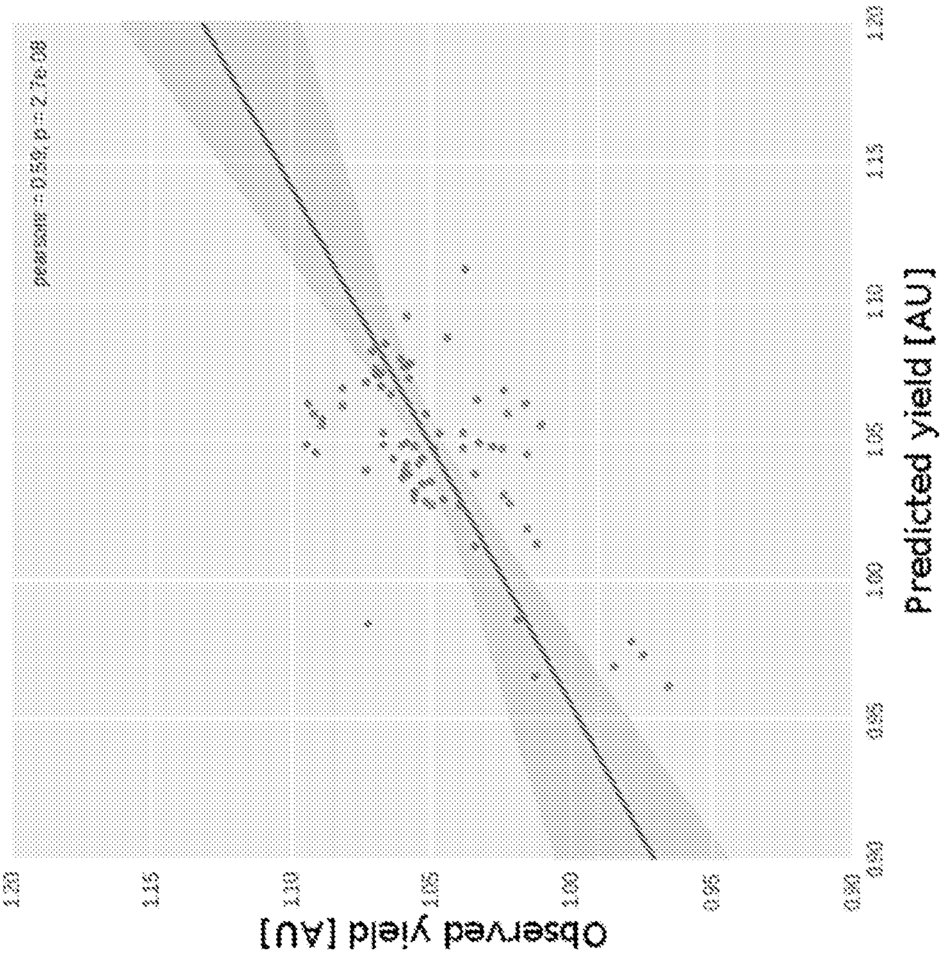


FIGURE 48

FIGURE 49

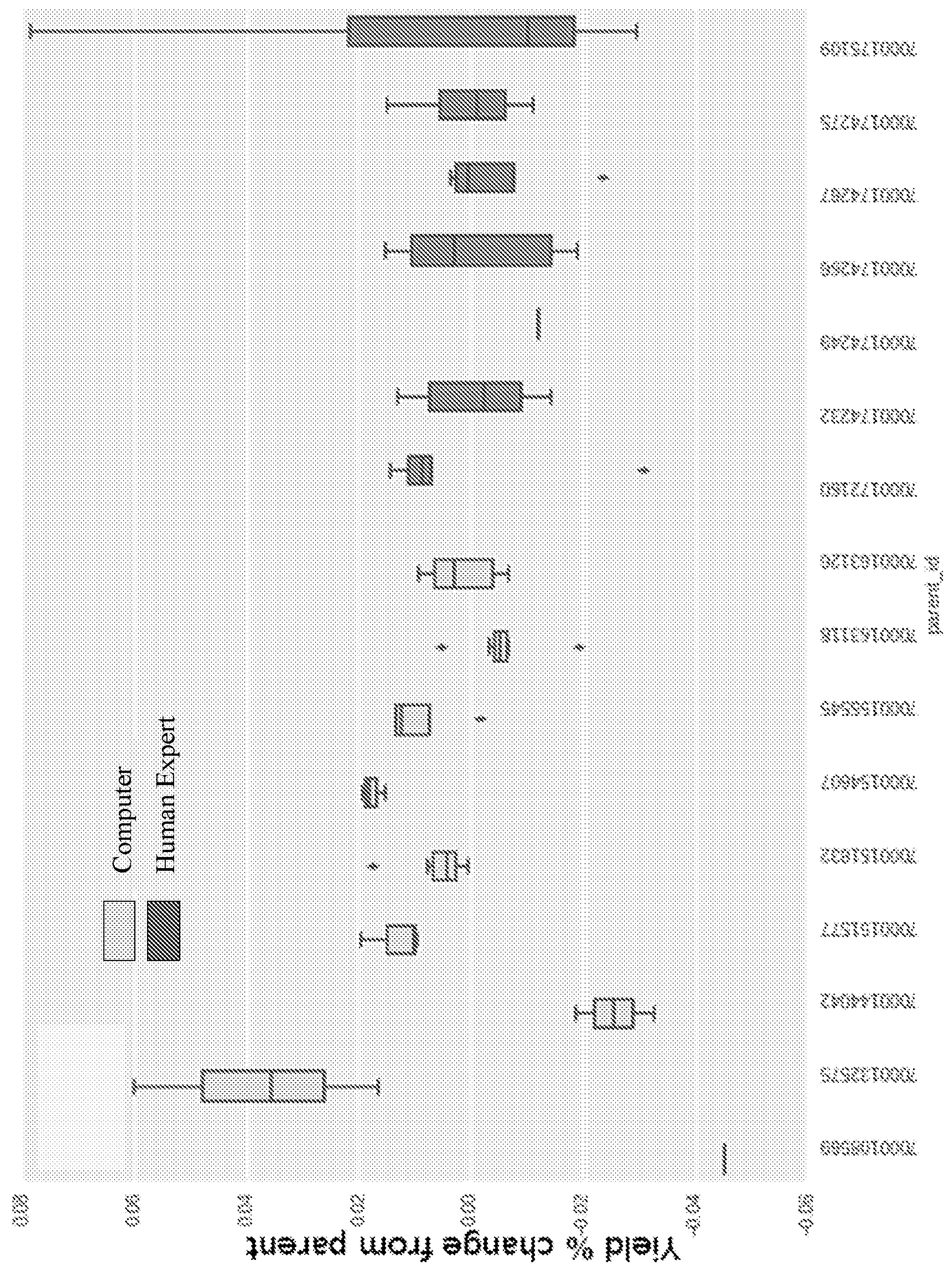
Computer
Human Expert
Yield % change from parent
0.08
0.06
0.04
0.02
0.00
-0.02
-0.04
-0.06

FIGURE 50

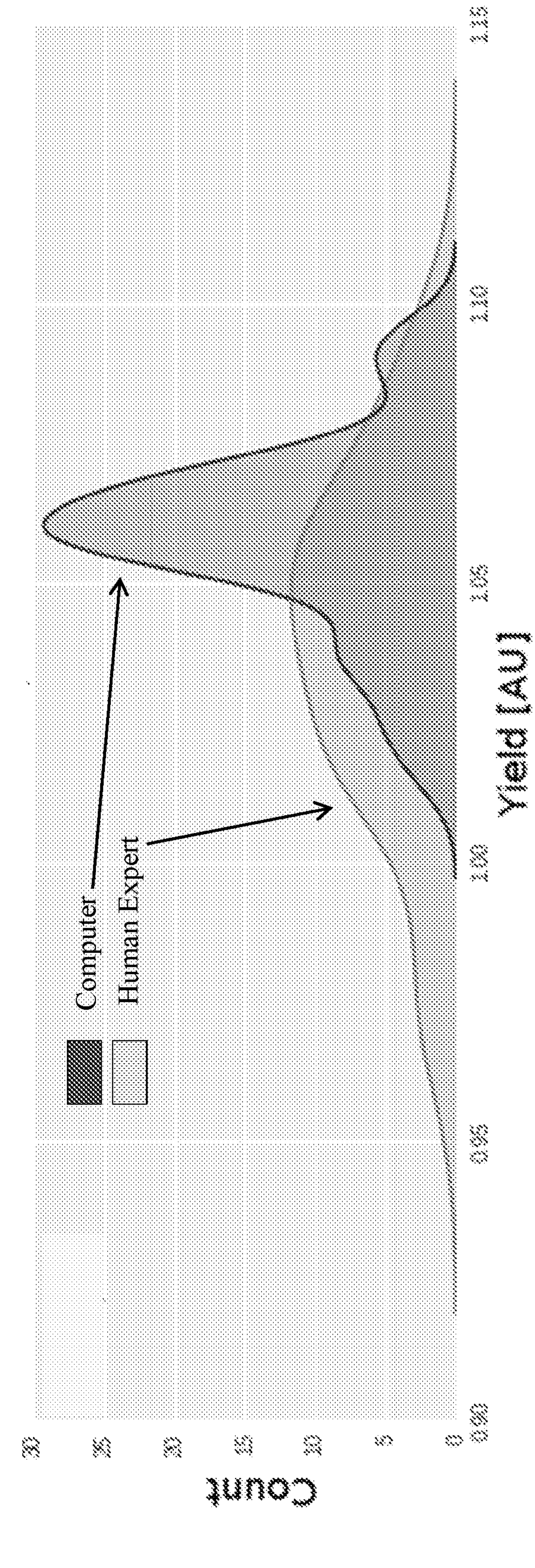
Count
Yield [AU]
Computer
Human Expert
0
5
10
15
20
25
30
0.90
0.95
1.00
1.05
1.10
1.15

FIGURE 51

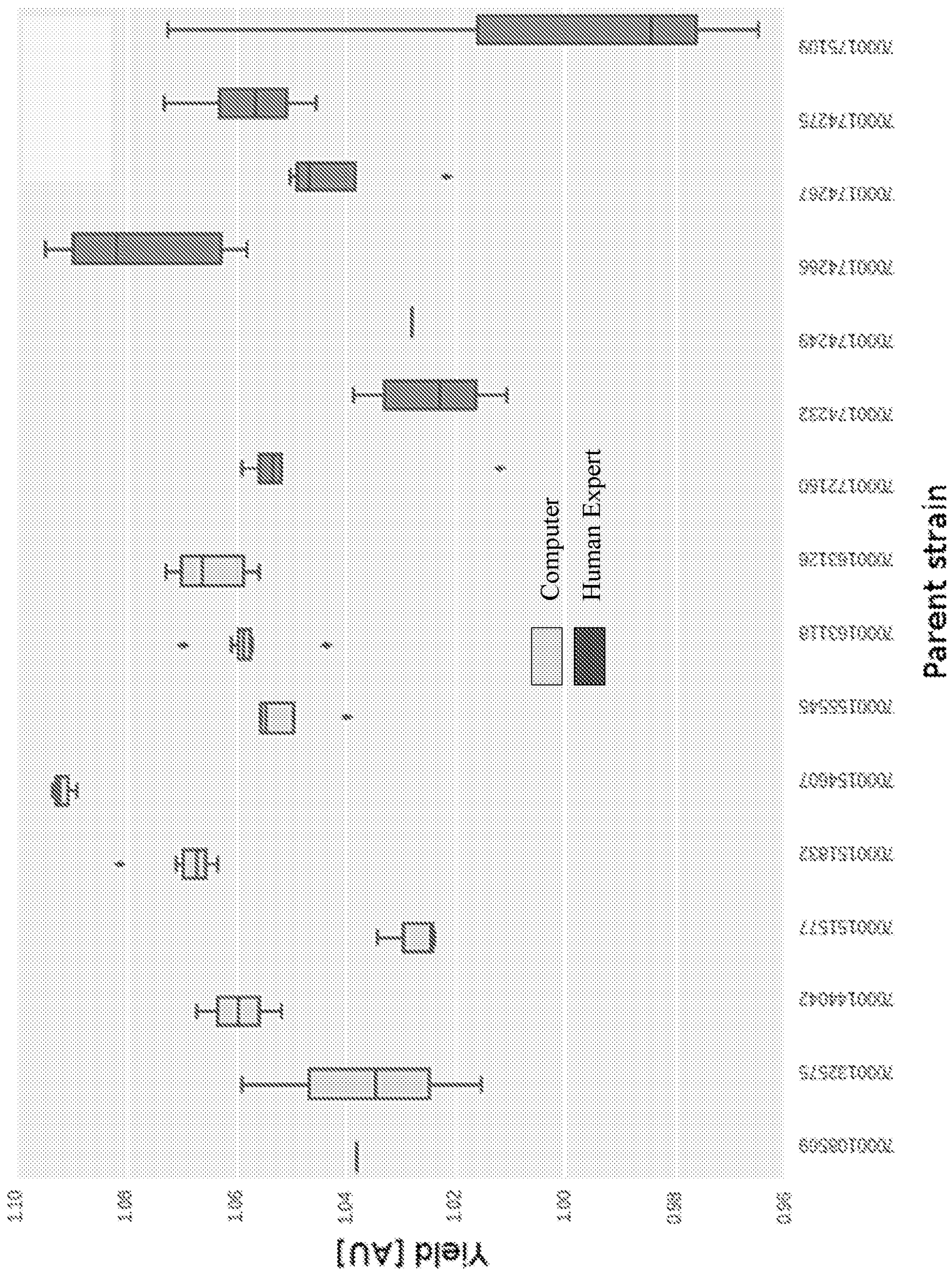
Yield [AU]
1.10
1.08
1.06
1.04
1.02
1.00
0.98
0.96
Computer
Human Expert
Parent strain

FIGURE 52

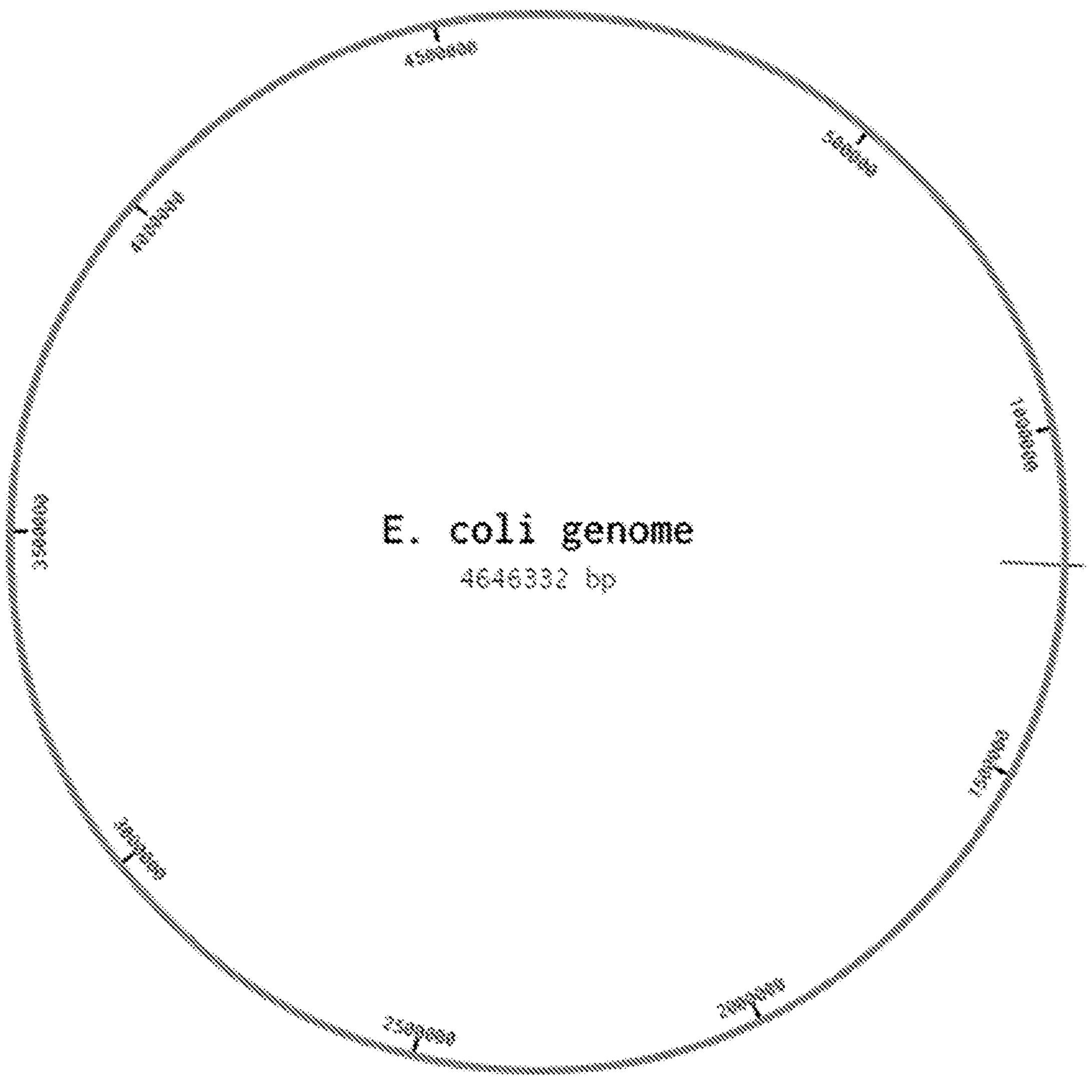
E. coli genome
4646332 bp
500000
1000000
1500000
2000000
2500000
3000000
3500000
4000000
4500000

FIGURE 53

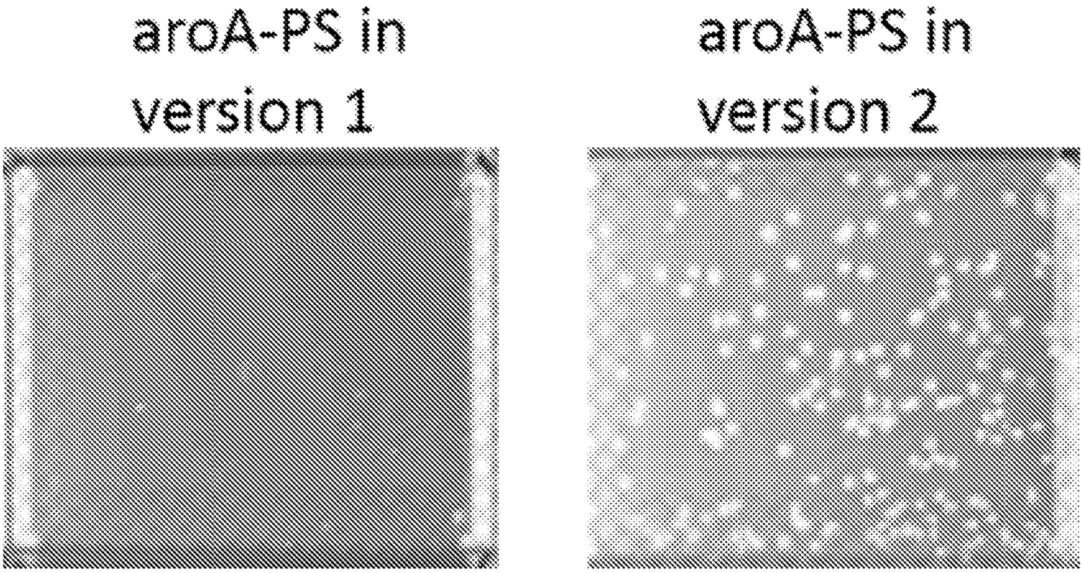

**FIGURE 54**

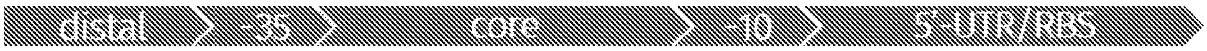


**FIGURE 55**

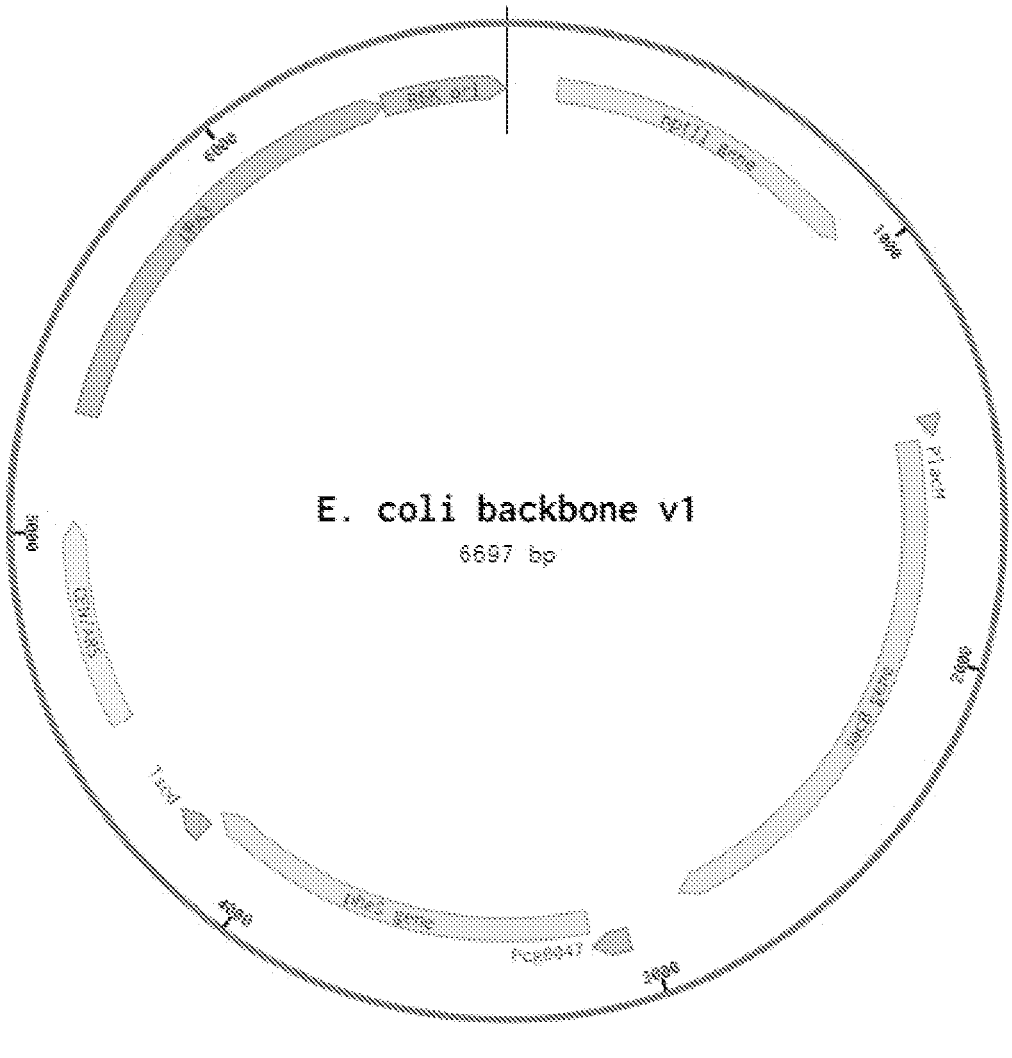

FIGURE 56

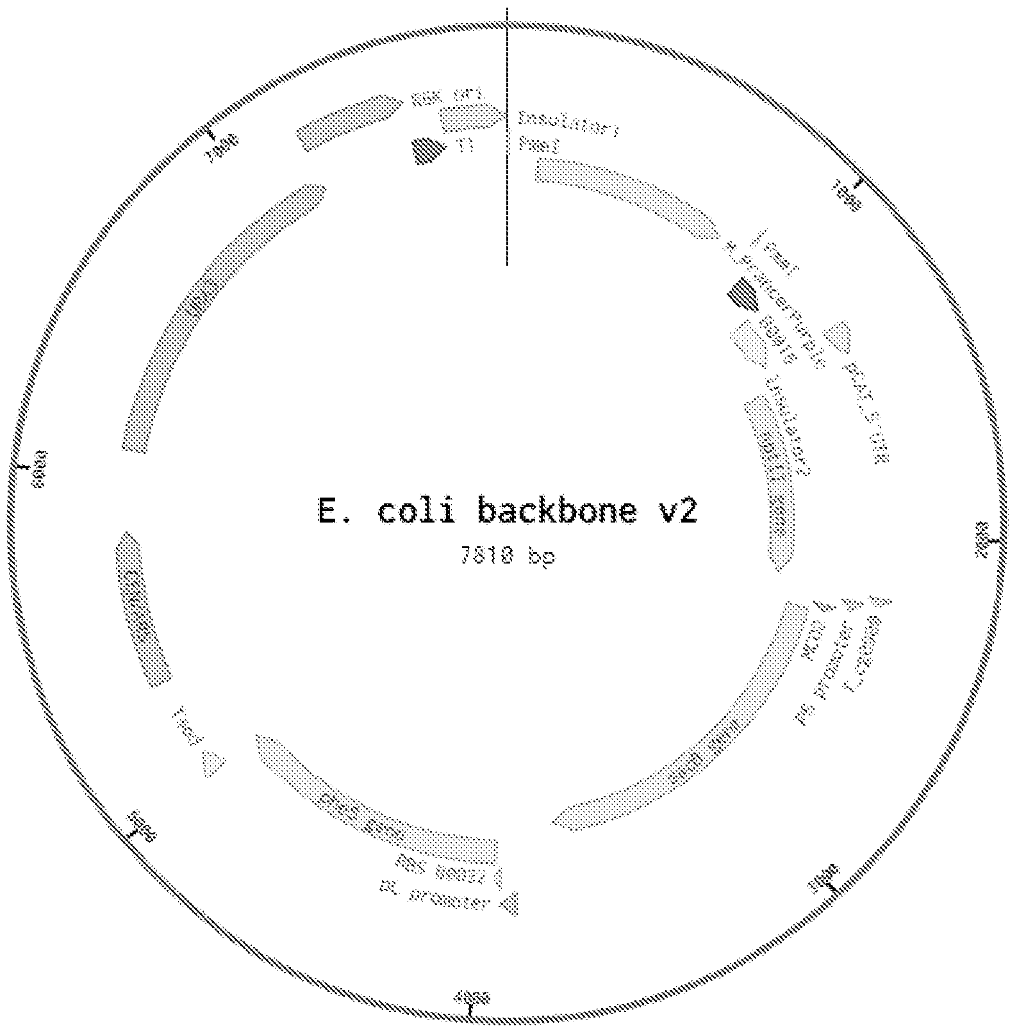
R6K ori
Insulator1
T1
PmeI
1000
2000
3000
4000
5000
6000
7000
E. coli backbone v2
7818 bp

FIGURE 57

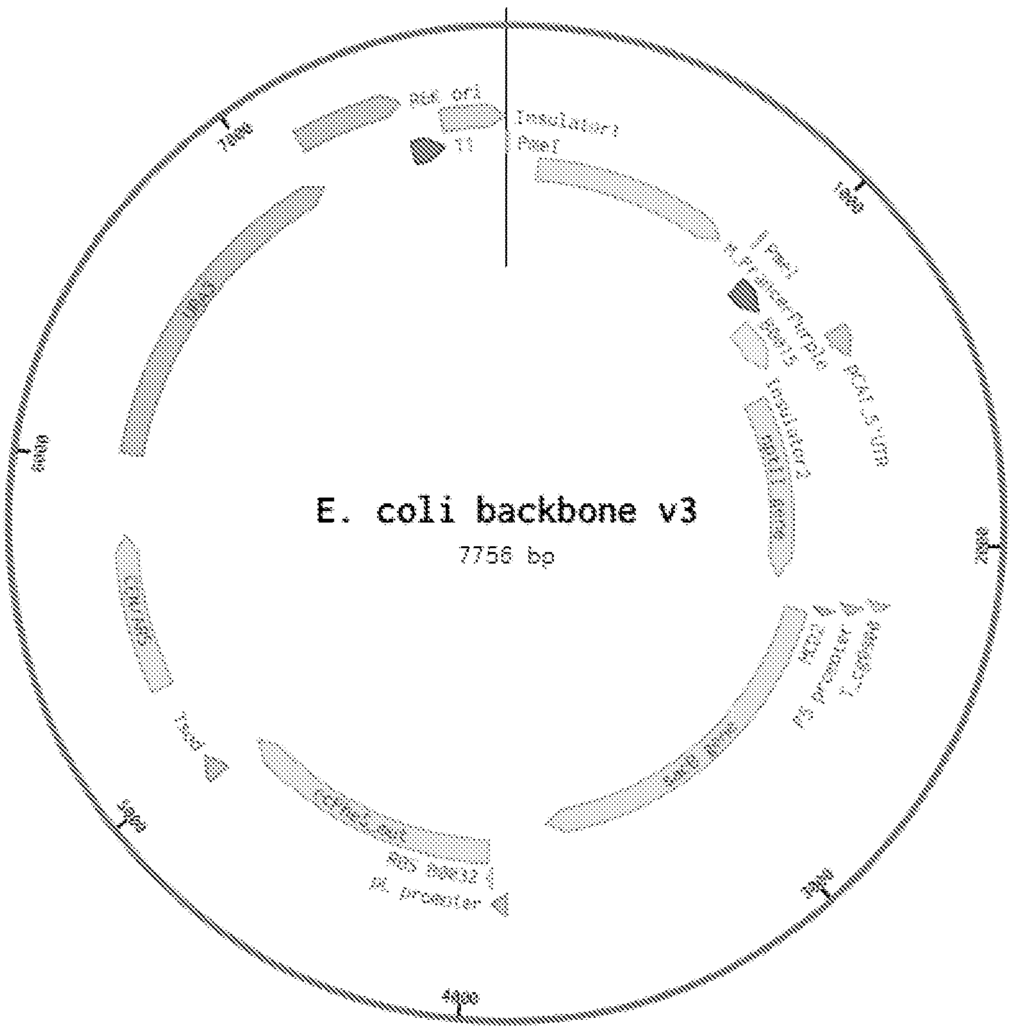
E. coli backbone v3
7756 bp
Insulator1
PmeI
T1
Insulator2
pL promoter
1000
2000
3000
4000
5000
6000
7000

FIGURE 58

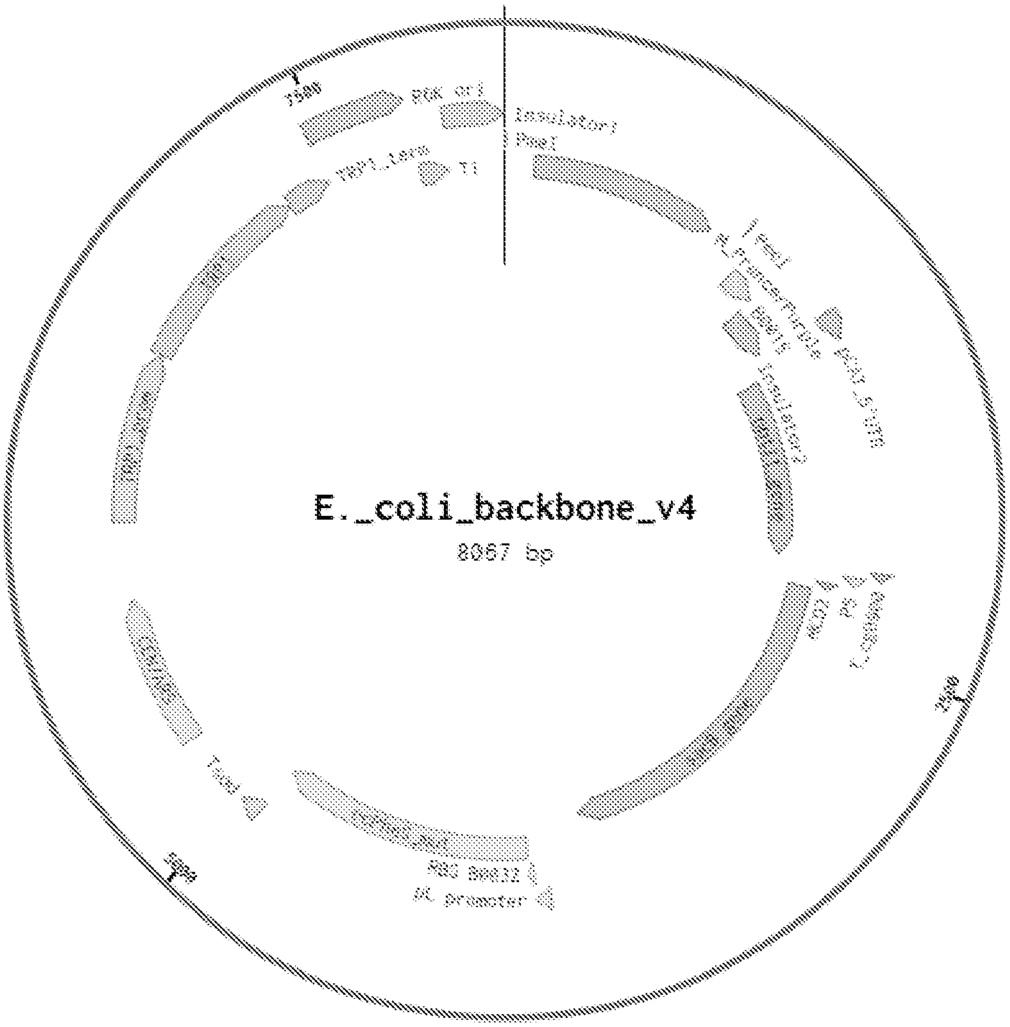
E._coli_backbone_v4
8067 bp
Insulator1
PmeI
T1
TRP1_term
RBS B0032
pL promoter
2500
5000
7500

**FIGURE 59**

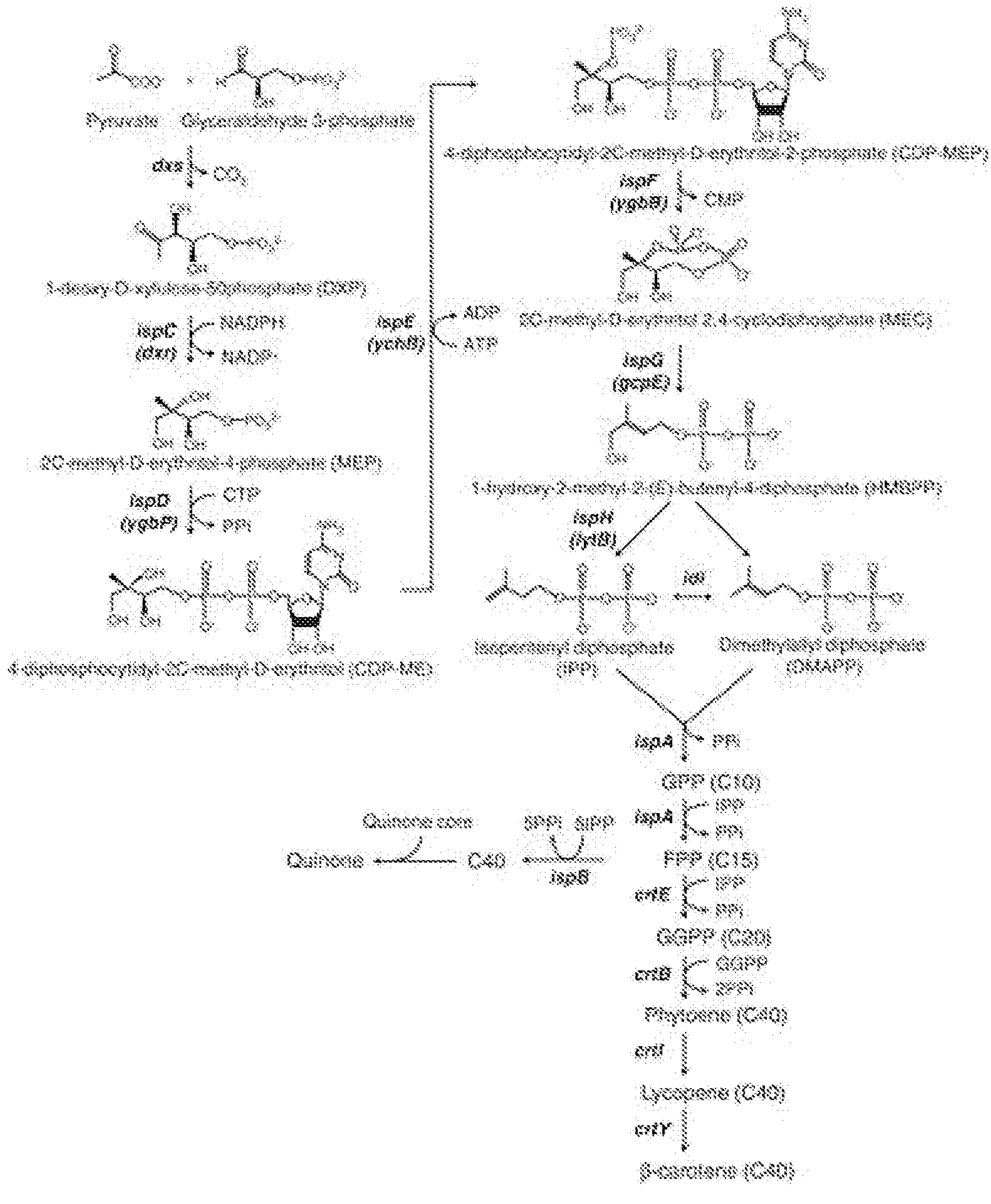
Pyruvate
Glyceraldehyde 3-phosphate
dxs
$CO_2$
1-deoxy-D-xylulose-5-phosphate (DXP)
ispC
(dxr)
NADPH
$NADP^+$
2C-methyl-D-erythritol-4-phosphate (MEP)
ispD
(ygbP)
CTP
PPi
4-diphosphocytidyl-2C-methyl-D-erythritol (CDP-ME)
ispE
(ychB)
ADP
ATP
4-diphosphocytidyl-2C-methyl-D-erythritol-2-phosphate (CDP-MEP)
ispF
(ygbB)
CMP
2C-methyl-D-erythritol 2,4-cyclodiphosphate (MEC)
ispG
(gcpE)
1-hydroxy-2-methyl-2-(E)-butenyl-4-diphosphate (HMBPP)
ispH
(lytB)
idi
Isopentenyl diphosphate
(IPP)
Dimethylallyl diphosphate
(DMAPP)
ispA
PPi
GPP (C10)
ispA
IPP
PPi
FPP (C15)
Quinone core
5PPi
5IPP
Quinone
C40
ispB
crtE
IPP
PPi
GGPP (C20)
crtB
GGPP
2PPi
Phytoene (C40)
crtI
Lycopene (C40)
crtY
β-carotene (C40)

FIGURE 60

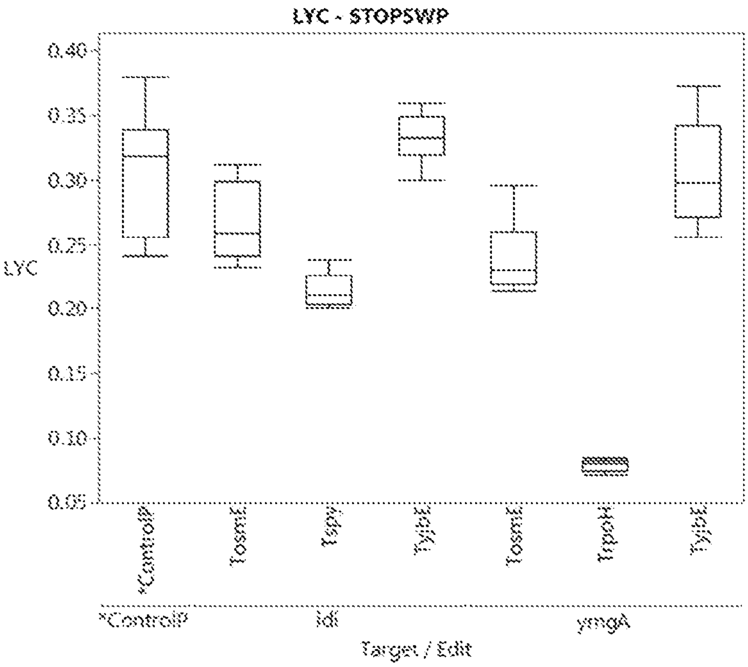
LYC - STOPSWP
0.40
0.35
0.30
0.25
LYC
0.20
0.15
0.10
0.05
*ControlP
TosmE
Tspy
TyjbE
TosmE
TrpoH
TyjbE
*ControlP
idi
ymgA
Target / Edit

**FIGURE 61**

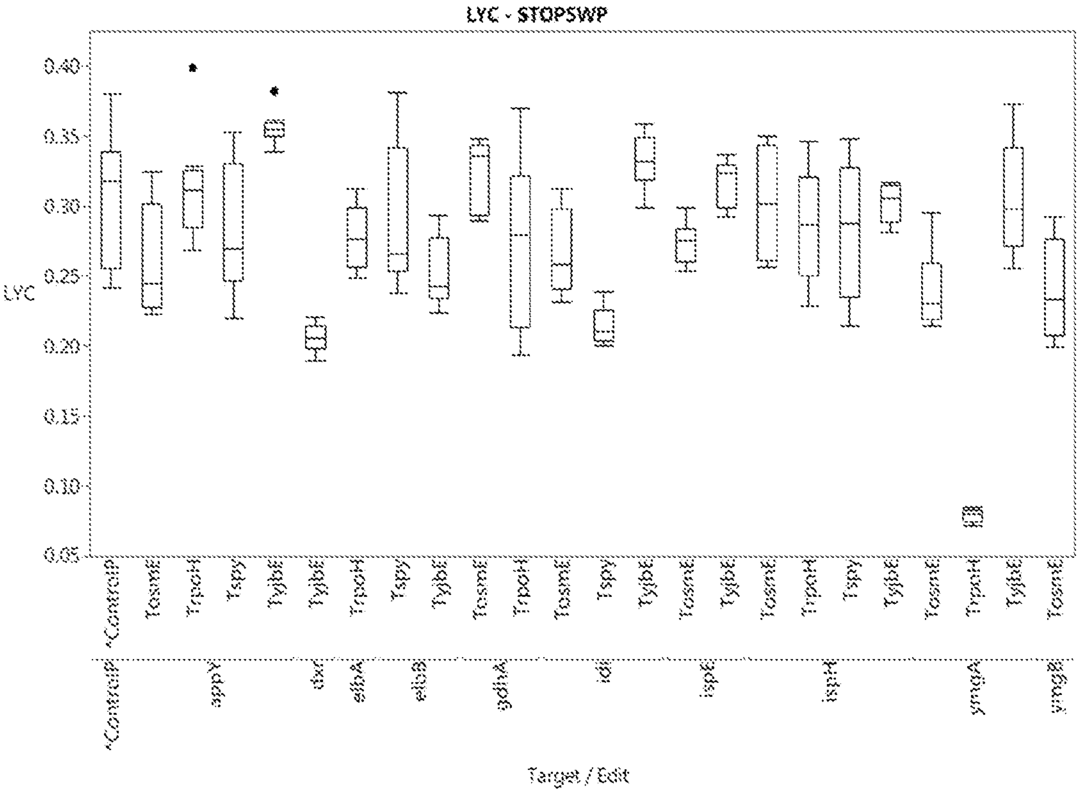

FIGURE 62

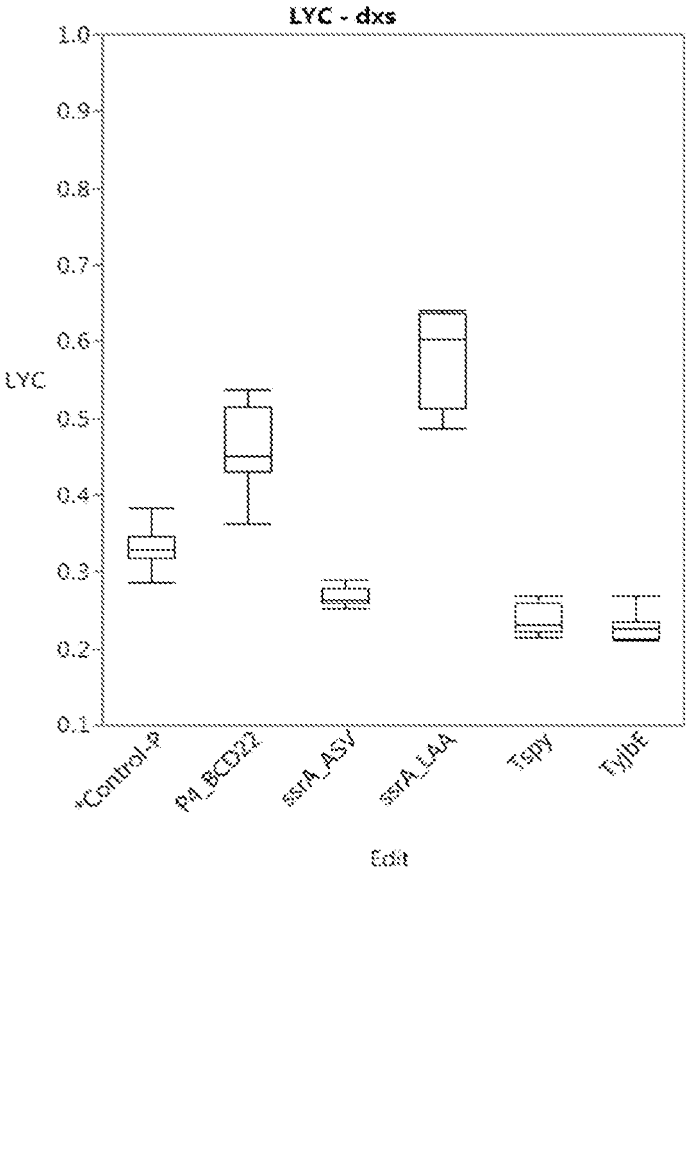
LYC - dxs
1.0
0.9
0.8
0.7
0.6
0.5
0.4
0.3
0.2
0.1
LYC
+Control-9
P4_BCD22
ssrA_ASV
ssrA_LAA
Tspy
TyjbE
Edit

FIGURE 63

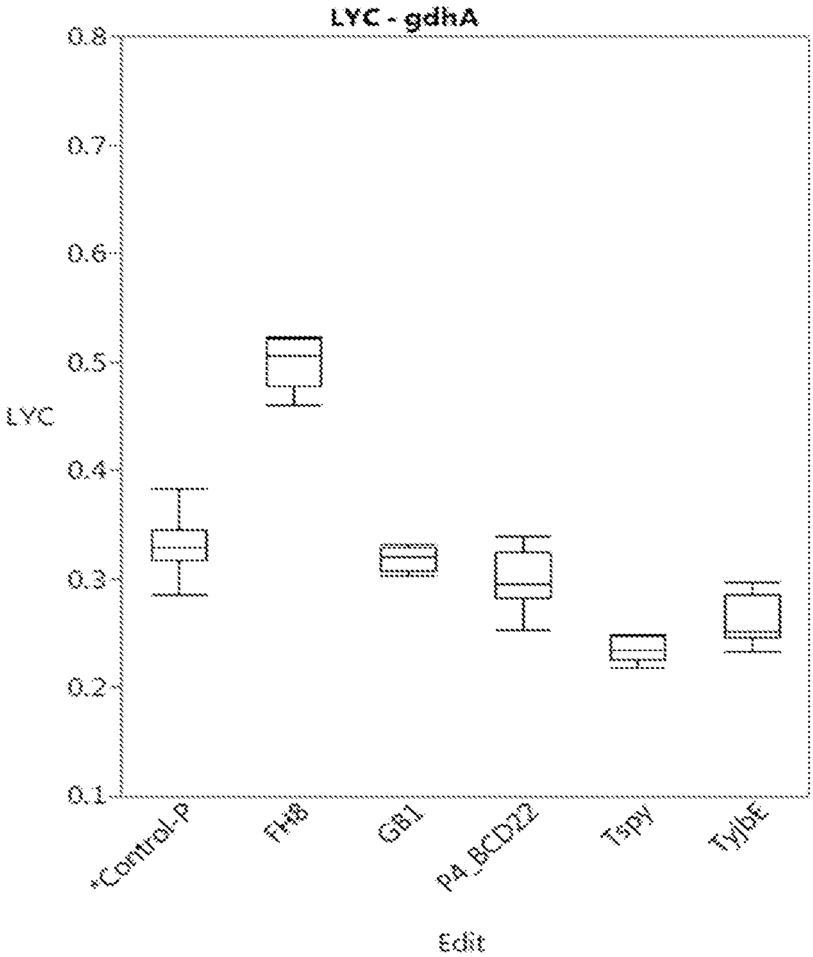
LYC - gdhA
0.8
0.7
0.6
0.5
0.4
0.3
0.2
0.1
LYC
+Control-P
FliB
GB1
P4_BCD22
Tspy
TybE
Edit

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62515870
- EP 2657250 A2
- US 2006141578 A1
- US 5605793 A
- US 5837458 A
- US 19843194
- US 9502126 W
- US 08425684 B
- US 08537874 B
- US 08564955 B
- US 08621859 B
- US 08621430 B
- US 9605480 W
- US 65040096 W
- US 08675502 B
- US 08721824 B
- US 08722660 B
- US 140296
- US 1729725 W
- US 20070292918
- US 6060296 A
- US 62264232
- US 55789538 B, Rebar
- US 6410248 B, Greisman
- US 7605140 B, Rebar
- US 6140081 A, Barbas
- US 7067617 B, Barbas
- US 6205404 B, Michaels
- US 20070178454, Joung
- US 20110008829 A
- WO 05021772 A
- US 5591645 A
- US 4855240 A
- US 4435504 A
- US 4980298 A
- WO 2016073690 A
- US 20100137143 A
- US 20100304982 A
- US 6090592 A
- US 6300070 B
- US 7115400 B
- EP 0972081 B1
- US 20110172127 A, Jacobsen
- US 20100216648 A, Staehler
- US 6040439 A, Hayakawa
- WO 2011154147 A
- US 8697359 B
- US 8771945 B
- US 8795965 B
- US 8865406 B
- US 8871445 B
- US 8889356 B
- US 8895308 B
- US 8906616 B
- US 8932814 B
- US 8945839 B
- US 8993233 B
- US 8999641 B
- US 9822372 B
- US 9840713 B
- US 842859
- US 20140068797 A1
- US 9260723 B
- US 9023649 B
- US 9834791 B
- US 9637739 B
- US 683443
- US 20150240261 A1
- US 743764
- US 20150291961 A1
- US 9790490 B
- US 9688972 B
- US 9580701 B
- US 9745562 B
- US 9816081 B
- US 9677090 B
- US 9738687 B
- US 632222
- US 20170369879 A1
- US 631989
- US 632001
- US 9896696 B
- US 396230
- US 20170159045 A1
- US 2016065465 W
- WO 2017100377 A1
- US 20170316353 A1
- US 2017029725 W
- WO 2017189784 A1
- US 2016065464 W
- WO 2017100376 A2
- US 62431409
- US 62368786
- US 62515870 B

## Non-patent literature cited in the description


- **LEE MICHAEL E et al.** *ASC Synthetic Biology,* 18 September 2015, vol. 4 (9), 975-986
- **KINCADE JM et al.** *Gene,* 01 January 1991, vol. 97 (1), 7-12
- **RAINER KNAUS et al.** *The EMBO Journal,* 25 August 1988, vol. 7 (9), 2919-2923
- **WILLIAM R MCCLERAY.** *Applied Microbiology and Biotechnology,* 25 July 2009, vol. 84 (4), 641-648
- **PENGFEI GU et al.** *Microbial Cell Factories,* 02 March 2012, vol. 11 (1), 30
- **RICE CHRISTOPHER D et al.** *Applied and Enviromental Microbiology,* February 2009, vol. 75 (3), 573-583
- **GUEGUEN ERWAN et al.** *Applied and Environmental Microbiology,* January 2013, vol. 79 (1), 32-38
- Current Protocols in Molecular Biology. 1987
- **STEMMER.** *PNAS,* 1994, vol. 91, 10747-10751
- **STEMMER.** *Nature,* 1994, vol. 370, 389-391
- **CRAMERI et al.** *Nature Biotech.,* 1997, vol. 15, 436-438
- **MOORE et al.** *J. Mol. Biol.,* 1997, vol. 272, 336-347
- **ZHANG et al.** *PNAS,* 1997, vol. 94, 4504-4509
- **CRAMERI et al.** *Nature,* 1998, vol. 391, 288-291
- **SAMBROOK et al.** Molecular Cloning: A Laboratory Manual. Cold Spring Harbor Laboratory Press, 2001
- PCR Protocols: A Guide to Methods and Applications. Academic Press, 1990
- PCR Strategies. Academic Press, 1995
- PCR Methods Manual. Academic Press, 1999
- **JONES et al.** *EMBO J.,* 1985, vol. 4, 2411-2418
- **DE ALMEIDA.** *Mol. Gen. Genetics,* 1989, vol. 218, 78-86
- **STEMMER.** *Science,* 1995, vol. 270, 1510
- **STEMMER et al.** *Gene,* 1995, vol. 164, 49-53
- **STEMMER.** *Bio/Technology,* 1995, vol. 13, 549-553
- **STEMMER.** *Proc. Natl. Acad. Sci. U.S.A.,* 1994, vol. 91, 10747-10751
- **CRAMERI et al.** *Nature Medicine,* 1996, vol. 2 (1), 1-3
- **CRAMERI et al.** *Nature Biotechnology,* 1996, vol. 14, 315-319
- **DALPHIN et al.** *Nucl. Acids Res.,* 1996, vol. 24, 216-218
- **KADONAGA, JT.** Regulation of RNA polymerase II transcription by sequence-specific DNA binding factors. *Cell,* 23 January 2004, vol. 116 (2), 247-57
- **CRAMER P. et al.** Functional association between promoter structure and transcript alternative splicing. *Proc Natl Acad Sci U S A.,* 14 October 1997, vol. 94 (21), 11456-60
- **GREGER IH. et al.** Balancing transcriptional interference and initiation on the GAL7 promoter of Saccharomyces cerevisiae. *Proc Natl Acad Sci USA.,* 18 July 2000, vol. 97 (15), 8415-20
- **WEST, S. ; PROUDFOOT, N.J.** Transcriptional Termination Enhances Protein Expression in Human Cells. *Mol Cell,* 13 February 2009, vol. 33 (3-9), 354-364
- **WEST, S et al.** Molecular dissection of mammalian RNA polymerase II transcriptional termination. *Mol Cell,* 14 March 2008, vol. 29 (5), 600-10
- **MURRAY et al.** *Nucl. Acids Res.,* 1989, vol. 17, 477-508
- **COSTA et al.** *Front Microbiol.,* 2014, vol. 5, 63
- **COSTANZO.** *The Genetic Landscape of a Cell, Science,* 22 January 2010, vol. 327 (5964), 425-431
- **COSTANZO.** The Genetic Landscape of a Cell. *Science,* 22 January 2010, vol. 327 (5964), 425-431
- **FOX et al.** Improving catalytic function by ProSAR-driven enzyme evolution. *Nature Biotechnology,* 2007, vol. 25 (3), 338-343
- **WAGNER et al.** *Nucleic Acids Res.,* 1995, vol. 23 (19), 3944-3948
- **SU et al.** *Proc. Natl. Acad. Sci. (U.S.A.),* 1986, vol. 83, 5057-5061
- **COX et al.** *Mol Syst Biol.,* 2007, vol. 3, 145
- **SALIS, H.M. et al.** Automated design of synthetic ribosome binding sites to control protein expression. *Nat. Biotechnol.,* 2009, vol. 27, 946-950
- **MUTALIK et al.** Precise and reliable gene expression via standard transcription and translation initiation elements. *Nat. Biotechnol.,* 2013, vol. 10 (4), 354-368
- **PFEIFER-SANCAR et al.** Comprehensive analysis of the Corynebacterium glutamicum transcriptome using an improved RNAseq technique. *BMC Genomics,* 2013, vol. 14, 888
- **ESPOSITO ; CHATTERJEE.** *Curr Opin Biotechnol.,* August 2006, vol. 17 (4), 353-8
- **KEILER K C ; SAUER R T.** Sequence determinants of C-terminal substrate recognition by the Tsp protease. *J Biol Chem.,* 1996, vol. 271, 2589-2593
- **MCGINNESS et al.** Engineering Controllable Protein Degradation. *Mol. Cell,* June 2006, vol. 22 (5
- **ANDERSEN et al.** *Appl Environ Microbiol.,* June 1998, vol. 64 (6), 2240-2246

- **KOBILER O ; KOBY S ; TEFF D ; COURT D ; OPPENHEIM AB.** *PNAS,* 23 October 2002, vol. 99 (23), 14964-14969
- **HERMAN et al.** *Genes Dev.,* 01 May 1998, vol. 12 (9), 1348-1355
- **WOHLEVER et al.** *Protein Eng Des Sel.,* April 2013, vol. 26 (4), 299-305
- **SEKAR K ; GENTILE AM ; BOSTICK JW ; TYO KEJ.** N-Terminal-Based Targeted, Inducible Protein Degradation in Escherichia coli. *PLoS ONE,* 2016, vol. 11 (2), e0149746
- **BUTZ et al.** *Biochemistry,* 2011, vol. 50 (40), 8594-8602
- **LEE et al.** Improved Production of L-Threonine in Escherichia coli by Use of a DNA Scaffold System. *App. And Environ. Microbiol.,* 2013, vol. 79 (3), 774-782
- **GREISMAN H A ; PABO C O.** A General Strategy for Selecting High-Affinity Zinc Finger Proteins for Diverse DNA Target Sites. *Science,* 1997, vol. 275, 657-661
- **REBAR E J ; PABO C O.** Zinc Finger Phage: Affinity Selection of Fingers with New DNA-Binding Specificities. *Science,* 1994, vol. 263, 671-673
- **MAEDER et al.** Rapid ''Open-Source'' Engineering of Customized Zinc-Finger Nucleases for Highly Efficient Gene Modification. *Mol. Cell,* 2008, vol. 31, 294-301
- **SANDER et al.** Selection-Free Zinc-Finger-Nuclease Engineering by Context-Dependent Assembly (CoDA). *Nat. Methods,* 2011, vol. 8, 67-69
- **BULYK et al.** Exploring the DNA-binding Specificities of Zinc Fingers with DNA Microarrays. *Proc. Nat'l Acad. Sci. U.S.A,* 2001, vol. 98 (13), 7158-63
- **HURT et al.** Highly Specific Zinc Finger Proteins Obtained by Directed Domain Shuffling and Cell-based Selection. *Proc. Nat'l Acad. Sci. U.S.A.,* 2003, vol. 100 (21), 12271-6
- **BISHOP ; HALL.** *Biochem. J.,* 2000, vol. 348, 241
- Guide to Molecular Cloning Techniques. **BERGER.** Methods in Enzymology. Academic Press, Inc, vol. 152
- **FRESHNEY.** Culture of Animal Cells, a Manual of Basic Technique. Wiley-Liss, 1994
- **DOYLE ; GRIFFITHS.** Mammalian Cell Culture: Essential Techniques. John Wiley and Sons, 1997
- **HUMASON.** Animal Tissue Techniques. W.H. Freeman and Company, 1979
- **RICCIARDELLE et al.** *In Vitro Cell Dev. Biol.,* 1989, vol. 25, 1016-1024
- **PAYNE et al.** Plant Cell and Tissue Culture. Liquid Systems John Wiley & Sons, Inc, 1992
- Plant Cell, Tissue and Organ Culture. 1995
- Fundamental Methods Springer Lab Manual. Springer-Verlag
- Plant Gene Transfer and Expression Protocols. Humana Press, 1984
- Plant Molecular Biology. Bios Scientific Publishers, 1993
- The Handbook of Microbiological Media. CRC Press, 1993
- Life Science Research Cell Culture Catalogue. Sigma-Aldrich, Inc
- The Plant Culture Catalogue and supplement. Sigma-Aldrich, Inc
- Manual of Methods for General Bacteriology. American Society for Bacteriology, 1981
- **STORHAS.** Bioreaktoren and periphere Einrichtungen. Vieweg Verlag, 1994
- **PARRY et al.** *Biochem. J.,* 2001, vol. 353, 117
- **HONG et al.** *Appl. Microbiol. Biotechnol,* 2007, vol. 73, 1331
- **SANDANA.** Bioseparation of Proteins. Academic Press, Inc, 1997
- **BOLLAG et al.** Protein Methods. Wiley-Liss, 1996
- **WALKER.** The Protein Protocols Handbook. Humana Press, 1996
- **HARRIS ; ANGAL.** Protein Purification Applications: A Practical Approach. IRL Press at Oxford, 1990
- **HARRIS ; ANGAL.** Protein Purification Methods: A Practical Approach. IRL Press at Oxford
- **SCOPES.** Protein Purification: Principles and Practice. Springer Verlag, 1993
- **JANSON ; RYDEN.** Protein Purification: Principles, High Resolution Methods and Applications. Wiley-VCH, 1998
- **WALKER.** Protein Protocols on CD-ROM. Humana Press, 1998
- **SE-HWAN PAEK et al.** *Development of rapid One-Step Immunochromatographic assay, Methods,* 2000, vol. 22, 53-60
- **BENTLEY et al.** *Nature,* 2008, vol. 456, 53-59
- **DRMANAC et al.** *Science,* 2010, vol. 327, 78-81
- **MARGULIES et al.** *Nature,* 2005, vol. 437, 376-380
- **EID et al.** *Science,* 2009, vol. 323, 133-138
- **KIM et al.** *Science,* 2007, vol. 316, 1481-1414
- TruSeq™ Sample Preparation Kit and Data Sheet. Illumina, Inc, 2010
- **SHEVADE.** A simple and efficient algorithm for gene selection using sparse logistic regression. *Bioinformatics,* 2003, vol. 19 (17), 2246-2253
- Classification using functional data analysis for temporal gene expression data. **LENG et al.** Bioinformatics. Oxford University Press, 2006, vol. 22, 68-76

- GPU-Based Deep Learning Inference: A Performance and Power Analysis, NVidia Whitepaper. **DAHL et al.** Multi-task Neural Networks for QSAR Predictions. Dept. of Computer Science, Univ. of Toronto, June 2014
- **LIBBRECHT et al.** Machine learning applications in genetics and genomics. *Nature Reviews: Genetics,* 16 June 2015
- **KASHYAP et al.** Big Data Analytics in Bioinformatics: A Machine Learning Perspective. *Journal of Latex Class Files,* September 2014, vol. 13
- Machine Learning in Bioinformatics. **PROMPRAMOTE et al.** Bioinformatics Technologies. Springer Berlin Heidelberg, 2005, 117-153
- **TIAN et al.** *Mol. BioSyst.,* 2009, vol. 5, 714-722
- **SIERZCHALA et al.** *J. Am. Chem. Soc.,* 2003, vol. 125, 13427-13441
- **AZHAYEV et al.** *Tetrahedron,* 2001, vol. 57, 4977-4986
- **KOZLOV et al.** *Nucleosides, Nucleotides, and Nucleic Acids,* 2005, vol. 24 (5-7), 1037-1041
- **DAMHA et al.** *NAR,* 1990, vol. 18, 3813-3821
- **CZAR et al.** *Trends in Biotechnology,* 2009, vol. 27, 63-71
- **ENGLER, C. ; R. KANDZIA ; S. MARILLONNET.** A one pot, one step, precision cloning method with high-throughput capability. *PLos One,* 2008, vol. 3, e3647
- **KOTERA, I. ; T. NAGAI.** A high-throughput and single-tube recombination of crude PCR products using a DNA polymerase inhibitor and type IIS restriction enzyme. *J Biotechnol,* 2008, vol. 137, 1-7
- **WEBER, E. ; R. GRUETZNER ; S. WERNER ; C. ENGLER ; S. MARILLONNET.** Assembly of Designer TAL Effectors by Golden Gate Cloning. *PloS One,* 2011, vol. 6, e1722
- **ASLANIDIS ; DE JONG.** Ligation-independent cloning of PCR products (LIC-PCR). *Nucleic Acids Research,* 1990, vol. 18 (20), 6069
- **GIBSON et al.** Enzymatic assembly of DNA molecules up to several hundred kilobases. *Nature Methods,* 2009, vol. 6, 343-345
- **REYRAT et al.** Counterselectable Markers: Untapped Tools for Bacterial Genetics and Pathogenesis. *Infect Immun.,* 1998, vol. 66 (9), 4011-4017
- **MUTALIK et al.** *Nat Methods.,* April 2013, vol. 10 (4), 354-60
- **MIYAZAKI, K.** *Biotechniques.,* 01 February 2015, vol. 58 (2), 86-8
- **CHRISTIE, P.J. ; GORDON, J.E.** The Agrobacterium Ti Plasmids. *Microbiol SPectr. 2014,* 2014, vol. 2 (6), 10.1128
- **DAVIS, L. ; DIBNER, M. ; BATTEY, I.** *Basic Methods in Molecular Biology,* 1986
- **GIETZ et al.** *Nucleic Acids Res.,* 1992, vol. 27, 69-74
- **ITO et al.** *J. Bacterol.,* 1983, vol. 153, 163-168
- **BECKER ; GUARENTE.** *Methods in Enzymology,* 1991, vol. 194, 182-187
- **NAKASHIMA et al.** Bacterial Cellular Engineering by Genome Editing and Gene Silencing. *Int. J. Mol. Sci.,* 2014, vol. 15 (2), 2773-2793
- **TEAR et al.** Excision of Unstable Artificial Gene-Specific inverted Repeats Mediates Scar-Free Gene Deletions in Escherichia coli. *Appl. Biochem. Biotech.,* 2014, vol. 175, 1858-1867
- **DATSENKO ; WANNER.** *PNAS USA,* 2000, vol. 97, 6640-6645
- **THOMASON et al.** Recombineering: Genetic Engineering in Bacteria Using Homologous Recombination. *Current Protocols in Molecular Biology,* vol. 106 (1.16), 1.16.1-1.16.39
- **SHARAN et al.** Recombineering: A Homologous Recombination-Based Method of Genetic Engineering. *Nature protocols,* 2009, vol. 4 (2), 206-223
- **PYZOCHA et al.** *ACS Chemical Biology,* vol. 13 (2), 347-356
- **OROSZ et al.** *Eur J Biochem.,* 01 November 1991, vol. 201 (3), 653-9
- **LEWIS et al.** *Mol Syst Biol.,* 2010, vol. 6, 390